(19) Europäisches Patentamt
European Patent Office
Office européen des brevets

(11) EP 3 296 404 A1

(12) EUROPEAN PATENT APPLICATION

(43) Date of publication:
**21.03.2018 Bulletin 2018/12**

(51) Int Cl.:
***C12P 13/12*** *(2006.01)*

(21) Application number: **16306172.4**

(22) Date of filing: **15.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Evonik Degussa GmbH**
**45128 Essen (DE)**

(72) Inventors:
- **VASSEUR, Perrine**
  **63270 Martres sur Morges (FR)**
- **DISCHERT, Wanda**
  **63270 Vic-le-Comte (FR)**
- **SEVENIER, Antoine**
  **63430 Pont du Château (FR)**

# (54) MODIFIED MICROORGANISM FOR PRODUCTION OF METHIONINE

(57) The present invention relates to recombinant microorganisms useful for the production of L-methionine and/or its derivatives and process for the preparation of L-methionine. More particularly the invention relates to recombinant microorganisms with enhanced production of methionine and attenuated production of at least one by-product chosen among isoleucine, phenylalanine or threonine compared to their non-modified counterparts. Moreover this invention relates to methods of generating such modified microorganisms.

EP 3 296 404 A1

## Description

## Prior art

Sulphur-containing compounds such as cysteine, homocysteine, methionine or S-adenosylmethionine are critical to cellular metabolism. In particular L-methionine, an essential amino acid, which cannot be synthesized by animals, plays an important role in many body functions. Most of the methionine produced industrially is widely used as an animal feed and food additive.

With the decreased use of animal-derived proteins as a result of BSE and chicken flu, the demand for pure methionine has increased. Commonly, D,L-methionine is produced chemically from acrolein, methyl mercaptan and hydrogen cyanide. However, the racemic mixture does not perform as well as pure L-methionine (Saunderson, 1985). Additionally, although pure L-methionine can be produced from racemic methionine, for example, through the acylase treatment of N-acetyl-D,L-methionine, this dramatically increases production costs. Accordingly, the increasing demand for pure L-methionine coupled with environmental concerns render microbial production of methionine an attractive prospect.

Other important amino acids, such as lysine, threonine and tryptophan are produced via fermentation for use in animal feed. Therefore, these amino acids can be made using glucose and other renewable resources as starting materials. Industrial one-step production of L-methionine via fermentation has not been successful yet, but the development of the technology is ongoing.

Because methionine biosynthesis involves incorporation of reduced sulfur atom and that is more complex than biosynthesis of other amino acids, combinations of genetic modifications leading to improved production of methionine and more particularly to commercially attractive levels of methionine are not obvious.

Different approaches for the optimization of L-methionine production in microorganisms have been described previously (see, for example, Patents or patent applications US 7,790,424, US 7,611,873, WO 2002/10209, WO 2005/059093 and WO 2006/008097); however, industrial production of L-methionine from microorganisms requires further improvements.

Optimization of L-methionine production in microorganisms may lie in genetic modifications of genes involved directly in the methionine backbone synthesis (including regulator of these genes) as disclosed for example in patent applications WO2005108561, US2010041108, WO2007077041 or WO2011073122, genes involved in the uptake or efflux of methionine as disclosed in patent application WO2015028675, in WO2015028674, genes involved in the availability of co-factors or substrate as disclosed in patent application WO2012055798 or WO2013001055, or genes involved in biosynthesis of by-products as disclosed in patent application WO20100047879.

All these different genes are either over-expressed or inactivated or down-expressed, depending on their function in the biosynthesis of L-methionine.

Genes involved directly in the methionine backbone synthesis are well disclosed in the prior art as in Dharmendra & Gomesthe (2005*) and Usudo* & *Kurahashi (2005).*

Moreover to improved production of methionine it could be advantageous to attenuate production of by-product as isoleucine, phenylalanine or threonine in order to facilitate the separation and recovering of methionine, as those three amino acids are likely to be by-produced from pathways linked to L-methionine production. Indeed, isoleucine and threonine are derived from aspartate pathway like L-methionine and phenylalanine is derived from the phosphoenolpyruvate (PEP) whose synthesis is enhanced for the production of L-methionine from glucose.

Pathways for the biosynthesis of isoleucine, phenylalanine and threonine are well-known by the man skilled in the art and are disclosed respectively in *Yin et al. (2012), Kennerknecht et al. (2002), Ding et al. (2016), Ikeda et al. (1993)* and Patent US5175107.

More precisely to synthesize isoleucine, first, pyruvate and 2-oxobutanoate are converted into (S)-2-aceto-2-hydroxybutanoate by acetohydroxy acid synthase I encoded by *ilvBN* genes, or by acetolactate synthase II encoded by *ilvGM* genes, or by acetolactate synthase III encoded by *ilvIH* genes. In *E. coli* MG1655 strain, the acetolactate synthase II is not functionnal as the *ilvG* gene encoding for this enzyme is truncated. Then the (S)-2-aceto-2-hydroxybutanoate is converted into (R)-2,3-dihydroxy-3-methylpentanoate by acetohydroxy acid isomeroreductase encoded by *ilvC* gene. Then the (R)-2,3-dihydroxy-3-methylpentanoate is converted into 3-methyl-2-oxobutanoate by dihydroxyacid dehydratase encoded by *ilvD* gene. Finally, the 3-methyl-2-oxobutanoate is converted into L-isoleucine by branched-chain amino-acid aminotransferase encoded by *ilvE* gene. The acetohydroxy acid isomeroreductase encoded by *ilvC* gene, the dihydroxyacid dehydratase encoded by *ilvD* gene and the branched-chain amino-acid aminotransferase encoded by *ilvE* gene are also involved in valine and leucine biosynthesis pathways.

To synthesize phenylalanine, first, prephenate is converted into 2-oxo-3-phenylpropanoate by chorismate mutase / prephenate dehydratase encoded by *pheA* gene. Then 2-oxo-3-phenylpropanoate is converted into L-phenylalanine by phenylalanine transaminase encoded by *ilvE* or *aspC* genes, or by the phenylalanine aminotransferase encoded by *tyrB* gene.

To synthesize threonine, first, L-homoserine is converted into O-phospho-L-homoserine by the homoserine kinase encoded by *thrB* gene. Then O-phospho-L-homoserine is converted into L-threonine by the threonine synthase encoded by *thrC* gene.
The inventors have found that abolishment of isoleucine and/or threonine and/or phenylalanine improve production of methionine and its recovering even with the obligatory medium complementation with threonine, phenylalanine, isoleucine, valine and leucine when needed due to auxotrophy of the recombinant microorganisms.

## Summary of the invention

The invention relates to a recombinant microorganism optimised for the production of methionine, wherein the biosynthesis of by-products chosen among isoleucine, phenylalanine and threonine are attenuated, preferably completely abolished. More preferentially the biosynthesis of the three by-products isoleucine, phenylalanine and threonine are all together abolished.
The recombinant microorganism may also comprise other genetic modifications such as:

- an increased expression of at least one of the following genes: *ptsG, pyc, pntAB, cysP, cysU, cysW*, *cysA, cysM, cysJ, cysI, cysH, gcvT, gcvH, gcvP, Ipd, serA, serB, serC, cysE, metF, metH, thrA, metA* allele encoding for an enzyme with reduced feed-back sensitivity to S-adenosylmethionine and/or methionine (*metA**)*, thrA,* or a *thrA* allele encoding for an enzyme with reduced feed-back inhibition to threonine (*thrA**) and/or

- an attenuated expression of one of the following genes: *metJ, pykA, pykF, purU, ybdL, metE or yncA.*

In a particular embodiment, the present invention is related to a microorganism wherein: a) the expression of the genes *metA*, metH, cysPUWAM, cysJIH, gcvTHP, metF, serA, serB, serC, cysE, thrA** and *pyc* are enhanced; and b) the expression of the genes *metJ, pykA, pykF, purU, metE* and *yncA* are attenuated.
The invention also relates to a method for the production of methionine or methionine derivatives in a fermentative process comprising the steps of: a) culturing the recombinant microorganism according to the invention in an appropriate culture medium comprising a fermentable source of carbon containing glucose and a source of sulphur and b) recovering methionine or methionine derivatives from the culture medium.
Preferably, the recombinant microorganism is *Escherichia coli* or *Corynebacterium glutomicum.*

## Detailed description of the invention

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.
All publications, patents and patent applications cited herein, whether supra or infra, are hereby incorporated by reference in their entirety.
Furthermore, the practice of the present invention employs, unless otherwise indicated, conventional microbiological and molecular biological techniques within the skill of the art. Such techniques are well known to the skilled worker, and are explained fully in the literature.
It must be noted that as used herein and in the appended claims, the singular forms "a", "an", and "the" include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.
In the claims that follow and in the consecutive description of the invention, except where the context requires otherwise due to express language or necessary implication, the word "comprise", "contain", "involve" or "include" or variations such as "comprises", "comprising", "containing", "involved", "includes", "including" are used in an inclusive sense, i.e. to specify the presence of the stated features but not to preclude the presence or addition of further features in various embodiments of the invention.
The term "methionine" and "L-methionine" designate the essential sulphur-containing amino-acid with chemical formula HO2CCH(NH2)CH2CH2SCH3 and CAS number 59-51-8 or 63-68-3 for the specific L-isomer.
"Derivatives of methionine" refers to molecules analogs to methionine which present the same chemical backbone but differ from methionine with at least one chemical group. In this invention, preferred methionine derivatives are N-acetyl methionine (NAM), S-adenosyl methionine (SAM) and hydroxy-methionine (or methionine hydroxy analog or MHA).

The term "microorganism", as used herein, refers to a bacterium, yeast or fungus which is not modified artificially. Preferentially, the microorganism is selected among *Enterobacteriaceae, Bacillaceae, Streptomycetaceae* and *Corynebacteriaceae.* More preferentially the microorganism is a species of *Escherichia, Klebsiella, Pontoea, Salmonella,* or *Corynebacterium.* Even more preferentially the microorganism of the invention is either the species *Escherichia coli* or *Corynebacterium glutomicum.*

The term "recombinant microorganism" or "genetically modified microorganism", as used herein, refers to a bacterium, yeast or fungus that is not found in nature and is genetically different from its equivalent found in nature. It means, it is modified either by introduction or by deletion or by modification of genetic elements. It can also be transformed by forcing the development and evolution of new metabolic pathways by combining directed mutagenesis and evolution under specific selection pressure (see, for example, WO2004076659 or WO2007011939).

A microorganism may be modified to express exogenous genes if these genes are introduced into the microorganism with all the elements allowing their expression in the host microorganism. The modification or "transformation" of microorganisms with exogenous DNA is a routine task for those skilled in the art.

A microorganism may be modified to modulate the expression level of an endogenous gene.

The term "endogenous gene" means that the gene was present in the microorganism before any genetic modification. Endogenous genes may be overexpressed by introducing heterologous sequences in addition to, or to replace endogenous regulatory elements, or by introducing one or more supplementary copies of the gene into the chromosome or a plasmid. Endogenous genes may also be modified to modulate their expression and activity of the corresponding encoded protein. For example, mutations may be introduced into the coding sequence to modify the gene product or heterologous sequences may be introduced in addition to or to replace endogenous regulatory elements. Modulation of an endogenous gene may result in the up-regulation and/or enhancement of the activity of the gene product, or alternatively, down regulate and/or lower the activity of the endogenous gene product.

Another way to modulate their expression is to exchange the endogenous promoter of a gene (e.g., wild type promoter) with a stronger or weaker promoter to up or down regulate expression of the endogenous gene. These promoters may be homologous or heterologous. It is well within the ability of the person skilled in the art to select appropriate promoters.

Contrariwise, "exogenous gene" means that the gene was introduced into a microorganism, by means well known by the man skilled in the art whereas this gene is not naturally occurring in the microorganism. Exogenous genes may be integrated into the host chromosome, or be expressed extra-chromosomally by plasmids or vectors. A variety of plasmids, which differ with respect to their origin of replication and their copy number in the cell, are well known in the art. These genes may be homologous.

In the context of the invention, the term "homologous gene" is not limited to designate genes having a theoretical common genetic ancestor, but includes genes which may be genetically unrelated that have, none the less, evolved to encode protein which perform similar functions and/or have similar structure. Therefore the term 'functional homologue" for the purpose of the present invention relates to the fact that a certain enzymatic activity may not only be provided by a specific protein of defined amino acid sequence, but also by proteins of similar sequence from other (un)related microorganisms.

Using the references given in Genbank for known genes, those skilled in the art are able to determine the equivalent genes in other organisms, bacterial strains, yeast, fungi, mammals, plants, etc. This routine work is advantageously done using consensus sequences that can be determined by carrying out sequence alignments with genes derived from other microorganisms and designing degenerate probes to clone the corresponding gene in another organism. These routine methods of molecular biology are well known to those skilled in the art.

The terms "improved methionine production", "enhanced methionine production" or "increased methionine production" and grammatical equivalents thereof, as used herein, refer to an increased methionine/carbon source yield (ratio of gram/mol methionine produced per gram/mol carbon source consumed that it can be expressed in percent). Methods for determining the amount of carbon source consumed and of methionine produced are well known to those in the art. The yield is higher in the recombinant microorganism compared to the corresponding non-modified microorganism.

The terms "microorganism optimized for the fermentative production of methionine" refers to microorganisms evolved and/or genetically modified to present an improved methionine production in comparison with the endogenous production of the corresponding wild-type microorganisms. Such microorganisms "optimized" for methionine production are well known in the art, and have been disclosed in particular in patent applications WO2005111202, WO2007077041, WO2009043803 WO2010020681, WO2011073738, WO2011080542, WO2011080301, WO2012055798, WO2013001055, WO2013190343, WO2015028675 and WO2015028674.

According to the invention the terms "fermentative production", "culture" or "fermentation" are used to denote the growth of bacteria. This growth is generally conducted in fermenters with an appropriate culture medium adapted to the microorganism being used and containing at least one simple carbon source, and if necessary co-substrates.

An "appropriate culture medium" designates a medium (e.g., a sterile, liquid media) comprising nutrients essential or beneficial to the maintenance and/or growth of the cell such as carbon sources or carbon substrates, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids and vitamins.

The term "carbon source" or "carbon substrate" or "source of carbon" according to the present invention denotes any source of carbon that can be used by those skilled in the art to support the normal growth of a microorganism, including monosaccharides (such as glucose, galactose, xylose, fructose or lactose), oligosaccharides, disaccharides (such as sucrose, cellobiose or maltose), molasses, starch or its derivatives, hemicelluloses and combinations thereof. An especially preferred simple carbon source is glucose. Another preferred simple carbon source is sucrose. The carbon source can be derived from renewable feed-stock. Renewable feed-stock is defined as raw material required for certain industrial processes that can be regenerated within a brief delay and in sufficient amount to permit its transformation into the desired product. Vegetal biomass treated or not, is an interesting renewable carbon source.

The term "source of sulphur" according to the invention refers to sulphate, thiosulfate, hydrogen sulphide, dithionate, dithionite, sulphite, methylmercaptan, dimethylsulfide and other methyl capped sulphides or a combination of the different sources. More preferentially, the sulphur source in the culture medium is sulphate or thiosulfate or a mixture thereof.

The terms "source of nitrogen" corresponds to either an ammonium salt or ammoniac gas. The nitrogen source is supplied in the form of ammonium or ammoniac.

The terms "attenuation" or "expression attenuated" mean in this context that the expression of a gene and/or the production of an enzyme is decreased or suppressed compared to the non-modified microorganism leading to a decrease in the intracellular concentration of a ribonucleic acid, a protein or an enzyme compared to the non-modified microorganism. The man skilled in the art knows different means and methods to measure ribonucleic acid concentration or protein concentration in the cell including for instance use of Reverse Transcription Polymerase Chain Reaction (RT-PCR) to determine ribonucleic acid concentration and use of specific antibody to determine concentration of specific protein.

Decrease or suppression of the production of an enzyme is obtained by the attenuation of the expression of gene encoding said enzyme.

Attenuation of genes may be achieved by means and methods known to the man skilled in the art. Generally, attenuation of gene expression may be achieved by:

- Mutating the coding region or the promoter region or,
- Deleting of all or a part of the promoter region necessary for the gene expression or,
- Deleting of all or a part of the coding region of the gene by homologous recombination or,
- Inserting an external element into coding region or into promoter region or,
- Expressing the gene under control of a weak promoter or an inducible promoter.

The man skilled in the art knows a variety of promoters which exhibit different strength and which promoter to use for a weak or an inducible genetic expression.

The term "activity" of an enzyme is used interchangeably with the term "function" and designates, in the context of the invention, the reaction that is catalyzed by the enzyme. The man skilled in the art knows how to measure the enzymatic activity of said enzyme.

The terms "attenuated activity" or "reduced activity" of an enzyme mean either a reduced specific catalytic activity of the protein obtained by mutation in the amino acids sequence and/or decreased concentrations of the protein in the cell obtained by mutation of the nucleotidic sequence or by deletion of the coding region of the gene.

The terms "enhanced activity" or "increased activity" of an enzyme designate either an increased specific catalytic activity of the enzyme, and/or an increased quantity/availability of the enzyme in the cell, obtained for example by overexpressing the gene encoding the enzyme.

The terms "increased expression", "enhanced expression" or "overexpression" and grammatical equivalents thereof, are used interchangeably in the text and have a similar meaning. These terms mean that the expression of a gene or the production of an enzyme is increased compared to the non-modified microorganism leading to an increase in the intracellular concentration of a ribonucleic acid, a protein or an enzyme compared to the non-modified microorganism. The man skilled in the art knows different means and methods to measure ribonucleic acid concentration or protein concentration in the cell including for instance use of Reverse Transcription Polymerase Chain Reaction (RT-PCR) to determine ribonucleic acid concentration and use of specific antibody to determine concentration of specific protein.

Increase production of an enzyme is obtained by increasing expression of the gene encoding said enzyme.

To increase the expression of a gene, the man skilled in the art knows different techniques such as:

- Increasing the number of copies of the gene in the microorganism. The gene is encoded chromosomally or extra-chromosomally. When the gene is located on the chromosome, several copies of the gene can be introduced on the chromosome by methods of recombination, known by the expert in the field (including gene replacement). When the gene is located extra-chromosomally, it may be carried by different types of plasmids that differ with respect to their origin of replication and thus their copy number in the cell. These plasmids are present in the microorganism in 1 to 5 copies, or about 20 copies, or up to 500 copies, depending on the nature of the plasmid : low copy number plasmids with tight replication (pSC101, RK2), low copy number plasmids (pACYC, pRSF1010) or high copy number plasmids (pSK bluescript II).

- Using a promoter leading to a high level of expression of the gene. The man skilled in the art knows which promoters are the most convenient, for example promoters Ptrc, Ptac, Plac, or the lambda promoters PR and PL are widely used. These promoters can be "inducible" by a particular compound or by specific external condition like temperature or light. These promoters may be homologous or heterologous. More preferentially Ptrc promoters and derivatives of Ptrc promoter are used.

- Attenuating the activity or the expression of a transcription repressor, specific or non-specific of the gene.

- Using elements stabilizing the corresponding messenger RNA (Carrier and Keasling, 1999) or elements stabilizing the protein (e.g., GST tags, GE Healthcare).

The terms "encoding" or "coding" refer to the process by which a polynucleotide, through the mechanisms of transcription and translation, produces an amino-acid sequence. The gene(s) encoding the enzyme(s) can be exogenous or endogenous.

The terms "feed-back sensitivity" or "feed-back inhibition" refer to a cellular mechanism control in which an or several enzyme that catalyze the production of a particular substance in the cell are inhibited or less active when that substance has accumulated to a certain level. So the terms "reduced feed-back sensitivity" or "reduced feed-back inhibition" mean that the activity of such a mechanism is decreased or suppressed compared to a non-modified microorganism. The man skilled in the art knows how to modify the enzyme to obtain this result. Such modifications have been described in the patent application WO2005111202 or in the patent US7611873.

The invention relates to a recombinant microorganism optimised for the production of methionine, wherein the biosynthesis of at least one by-products chosen among isoleucine, phenylalanine and threonine is attenuated. Preferably production of isoleucine, phenylalanine or threonine is completely abolished. Even more preferentially production of isoleucine and phenylalanine and threonine are abolished.

In a first aspect of the invention, isoleucine biosynthesis is attenuated by attenuating the expression of at least one gene involved in isoleucine biosynthesis pathway. Genes involved in biosynthesis of isoleucine are:

- *ilvBN* genes of SEQ ID N°5 and 7 respectively encoding acetohydroxy acid synthase I which converts pyruvate and 2-oxobutanoate are converted into (S)-2-aceto-2-hydroxybutanoate or,

- *ilvGM* genes of SEQ ID N°257 and 261 respectively encoding acetolactate synthase II which converts pyruvate and 2-oxobutanoate are converted into (S)-2-aceto-2-hydroxybutanoate or,

- *ilvIH* genes of SEQ ID N°9 and 11 respectively encoding acetolactate synthase III which converts pyruvate and 2-oxobutanoate are converted into (S)-2-aceto-2-hydroxybutanoate or,

- *ilvC* gene of SEQ ID N°3 encoding acetohydroxy acid isomeroreductase which converts (S)-2-aceto-2-hydroxybutanoate into (R)-2,3-dihydroxy-3-methylpentanoate or,

- *ilvD* gene of SEQ ID N°1 encoding dihydroxyacid dehydratase which converts (R)-2,3-dihydroxy-3-methylpentanoate into 3-methyl-2-oxobutanoate or,

- *ilvE* gene of SEQ ID N°307 encoding branched-chain amino-acid aminotransferase which converts 3-methyl-2-oxobutanoate into L-isoleucine.

In the microorganism of the invention, the expression of at least one gene chosen among *ilvBN, ilvGM, ilvIH, ilvC, ilvD* or *ilvE* is attenuated. Preferentially expression of *ilvD* or *ilvC* or combination of *ilvBN* and *ilvIH* genes are attenuated. The man skill in the art knows means and methods in order to attenuated the expression of a gene as, for instance the replacement of the wild promoter by a weak promoter.

More preferentially the expressions of the genes involved in isoleucine biosynthesis are completely abolished by deletion of the genes. Preferentially in the microorganism of the invention genes deleted are *ilvD* or *ilvC* or combination of *ilvBN* and *ilvIH.*

In a second aspect of the invention, phenylalanine biosynthesis is attenuated by attenuating the expression of at least one gene involved in phenylalanine biosynthesis pathway. Genes involved in biosynthesis of phenylalanine are:

- *pheA* gene of SEQ ID N°13 encoding chorismate mutase / prephenate dehydratase which converts prephenate into 2-oxo-3-phenylpropanoate or,

- *ilvE* or *aspC* genes of SEQ ID N°307 and 53 respectively encoding phenylalanine transaminase which converts 2-oxo-3-phenylpropanoate into L-phenylalanine or,

- *tyrB* gene of SEQ ID N°309 encoding phenylalanine aminotransferase which converts 2-oxo-3-phenylpropanoate into L-phenylalanine.

In the microorganism of the invention, the expression of at least one gene chosen among *pheA, ilvE, aspC* or *tyrB* is attenuated. Preferentially expression of *pheA* gene is attenuated. The man skill in the art knows means and methods in order to attenuated the expression of a gene as, for instance the replacement of the wild promoter by a weak promoter.

More preferentially the expressions of the genes involved in phenylalanine biosynthesis are completely abolished by deletion of the genes. Preferentially in the microorganism of the invention genes deleted are *pheA, ilvE, aspC* or *tyrB.* Even more preferentially only *pheA* is deleted in the microorganism of the invention in order to abolished phenylalanine biosynthesis.

In a third aspect of the invention, threonine biosynthesis is attenuated by attenuating the expression of at least one gene involved in threonine biosynthesis pathway. Genes involved in biosynthesis of threonine are:

- *thrB* gene of SEQ ID N°232 encoding homoserine kinase which converts L-homoserine into O-phospho-L-homoserine or,

- *thrC* gene of SEQ ID N°234 encoding threonine synthase which converts O-phospho-L-homoserine into L-threonine.

In the microorganism of the invention, the expression of *thrB* or *thrC* gene is attenuated. Preferentially expression of *thrB* and *thrC* gene are attenuated. The man skill in the art knows means and methods in order to attenuated the expression of a gene as, for instance the replacement of the wild promoter by a weak promoter.

More preferentially the expressions of the genes involved in threonine biosynthesis are completely abolished by deletion of the genes. Preferentially in the microorganism of the invention genes deleted are *thrB* and *thrC.*

In a fourth aspect of the invention, the microorganism is modified in a way that biosynthesis of isoleucine, phenylalanine and threonine are completely abolished by deletion of genes *ilvC* and *pheA* and *thrBC* or by deletion of genes *ilvD* and *pheA* and *thrBC* or by deletion of genes *ivlBN* and *ilvIH* and *pheA* and *thrBC.*

In a fifth aspect of the invention, the microorganism is modified in a way that biosynthesis of isoleucine and phenylalanine are completely abolished whereas biosynthesis of threonine is attenuated by deletion of genes *ilvC* and *pheA* and expression of *thrBC* genes under a weak promoter or by deletion of genes *ilvD* and *pheA* and expression of *thrBC* genes under a weak promoter or by deletion of genes *ivlBN* and *ilvIH* and *pheA* and expression of *thrBC* genes under a weak promoter.

These modifications are introduced in a recombinant microorganism already modified to optimize the fermentative production of methionine.

**Microorganism optimized for the fermentative production of methionine**

The recombinant microorganism according to the invention is modified for improving the production of methionine. Genes involved in methionine production are well known in the art, and comprise genes involved in the methionine specific biosynthesis pathway as well as genes involved in precursor-providing pathways and genes involved in methionine consuming pathways.

Efficient production of methionine requires the optimization of the methionine specific pathway and several precursor-providing pathways. Methionine producing strains have already been described, in particular in patent applications WO2005111202, WO2007077041 and WO2009043803. These applications are incorporated as reference into this application.

Except otherwise stated, all the genes mentioned below concerning optimization of methionine biosynthesis pathway are referring to those from *E. coli.*

In a specific embodiment of the invention, the recombinant microorganism is modified as described below: the expression of at least one gene chosen among *ptsG, pyc, pntAB, cysP, cysU, cysW*, *cysA, cysM, cysJ, cysI, cysH, gcvT, gcvH, gcvP, lpd, serA, serB, serC, cysE, metH, metF, metA, metA** allele encoding for an enzyme with reduced feed-back sensitivity to S-adenosylmethionine and/or methionine, *thrA,* and *thrA** allele encoding for an enzyme with reduced feed-back inhibition to threonine is increased.

- *ptsG* gene of SEQ ID N°171 encodes the PTS enzyme IICBGlc as described in patent application WO2013001055.

- *pyc* gene of SEQ ID N°145 encodes a pyruvate carboxylase as described in patent application WO2013001055. In a preferred embodiment, the *pyc* gene is heterologous and is chosen from *pyc* genes from *Rhizobium etli, Bacillus subtilis, Lactococcus lactis, Pseudomonas fluorescens* or *Corynebacterium species,*

- *pntAB* genes of SEQ ID N°190 and 192 respectively encode subunits of a membrane-bound transhydrogenase, such as described in patent application WO2012055798,

- *cysP* gene of SEQ ID N°57 encodes a periplasmic sulphate binding protein, as described in WO2007077041 and in WO2009043803,

- *cysU* gene of SEQ ID N°59 encodes a component of sulphate ABC transporter, as described in WO2007077041 and in WO2009043803,

- *cysW* gene of SEQ ID N°61 encodes a membrane bound sulphate transport protein, as described in WO2007077041 and in WO2009043803,

- *cysA* gene of SEQ ID N°63 encodes a sulphate permease, as described in WO2007077041 and in WO2009043803,

- *cysM* gene of SEQ ID N°65 encodes an O-acetyl serine sulfhydralase, as described in WO2007077041 and in WO2009043803,

- *cysI* and *cysJ* genes of SEQ ID N°69 and 67 respectively encode respectively the alpha and beta subunits of a sulfite reductase as described in WO2007077041 and in WO2009043803. Preferably *cysI* and *cysJ* are overexpressed together,

- *cysH* gene of SEQ ID N°71 encodes an adenylylsulfate reductase, as described in WO2007077041 and in WO2009043803,

- *metH* gene of SEQ ID N°25 encodes a methionine synthase, as described in patent application WO2015028674.

Increasing C1 metabolism is also a modification that leads to improved methionine production. It relates to the increase of the activity of at least one enzyme involved in the C1 metabolism chosen among GcvTHP, Lpd, or MetF. In a preferred embodiment of the invention, the one carbon metabolism is increased by enhancing the expression and/or the activity of at least one of the following:

- *gcvT, gcvH, gcvP,* and *lpd* genes of SEQ ID N°99, 101, 103 and 121 respectively coding for the glycine cleavage complex, as described in patent application WO 2007/077041. The glycine-cleavage complex (GCV) is a multienzyme complex that catalyzes the oxidation of glycine, yielding carbon dioxide, ammonia, methylene-THF and a reduced pyridine nucleotide. The GCV complex consists of four protein components, the glycine dehydrogenase said P-protein (GcvP), the lipoyl-GcvH-protein said H-protein (GcvH), the aminomethyltransferase said T-protein (GcvT), and the dihydrolipoamide dehydrogenase said L-protein (GcvL or Lpd). P-protein catalyzes the pyridoxal phosphate-dependent liberation of $CO_2$ from glycine, leaving a methylamine moiety. The methylamine moiety is transferred to the lipoic acid group of the H-protein, which is bound to the P-protein prior to decarboxylation of glycine. The T-protein catalyzes the release of NH3 from the methylamine group and transfers the remaining C1 unit to THF, forming methylene-THF. The L protein then oxidizes the lipoic acid component of the H-protein and transfers the electrons to NAD+, forming NADH;

- *metF* gene of SEQ ID N°27 encoding a methylenetetrahydrofolate reductase, as described in patent application WO2007077041;

The overexpression of at least one of the following genes involved in serine biosynthesis also reduces the production of the by-product isoleucine:

- *serA* gene of SEQ ID N°107 which encodes a phosphoglycerate dehydrogenase, as described in WO2007077041 and in WO2009043803,

- *serB* gene of SEQ ID N°109 which encodes a phosphoserine phosphatase, as described in WO2007077041 and in WO2009043803,

- *serC* gene of SEQ ID N°105 which encodes a phosphoserine aminotransferase, as described in WO2007077041 and in WO2009043803.

The overexpression of the following genes has already been shown to improve the production of methionine:

- *cysE* gene of SEQ ID N°55 encodes a serine acyltransferase; its overexpression allows an increase in methionine production, as described in WO2007077041;

- *metA* gene of SEQ ID N°17 encodes a homoserine succinyltransferase. The allele *metA** gene of SEQ ID N°19 codes for an enzyme with reduced feed-back sensitivity to S-adenosylmethionine and/or methionine. Preferentially, the allele *metA** described in the patent application WO2005111202 is used;

- *thrA* gene of SEQ ID N°21 encodes an aspartokinase /homoserine dehydrogenase; the allele *thrA** gene of SEQ ID N°23 codes for an enzyme with reduced feed-back inhibition to threonine, as described in WO2005111202.

In a specific embodiment of the invention, genes may be under control of an inducible promoter. In a preferred embodiment of the invention, at least one of these genes is under the control of a temperature inducible promoter. Preferably, the expression of at least one of the genes: *thrA, cysE, metA,* is under the control of an inducible promoter, directly or indirectly. More preferably, the genes *thrA, cysE* and *metA* are under control of an inducible promoter, directly or indirectly. In a preferred embodiment of the invention, expression of *thrA* gene is under direct control of an inducible promoter and expression of *cysE* gene is under polar effect of inducible expression of *thrA* gene. In another preferred embodiment of the invention, expression of *thrA* gene is under direct control of an inducible promoter and expressions of *cysE* and *metA* genes are under polar effect of inducible expression of *thrA* gene.

In a most preferred embodiment, the temperature inducible promoter belongs to the family of PR promoters. A methionine producing strain having genes under control of inducible promoters is described in patent application WO2011073122.

In another specific embodiment of the invention, the microorganism has been further modified, and the expression of at least one of the following genes is attenuated: *metJ, pykA, pykF, purU, ybdL, yncA, metE, dgsA* or *udhA.*

- the gene *metJ* of SEQ ID N°15 codes for the repressor protein MetJ (GenBank 1790373), responsible for the down-regulation of the methionine regulon as was suggested in patent application JP2000157267,

- the genes *pykA* and *pykF* of SEQ ID N°141 and 143 respectively code for the enzymes 'pyruvate kinase'. The attenuation of the expression of at least one or both of the pyruvate kinases decreases the consumption of phosphoenol pyruvate (PEP). Increased availability of PEP can increase the production of oxaloacetate, an important precursor of aspartate, which in turn is a precursor of methionine, as described in WO2007077041 and in WO2009043803,

- *purU* gene of SEQ ID N°111 codes for a formyltetrahydrofolate deformylase, an enzyme that catalyzes the formyl-THF deformylase reaction. The attenuation of the deformylase activity increases the production of methyl-THF that is required for methylation of homocysteine. Loss of C1 metabolites by deformylation leads to an increased production of homocysteine that cannot be transformed into methionine. Homocysteine can then be a substrate for the enzyme cystathionine gamma synthase (MetB) that can catalyze the reaction between O-succinylhomoserine and homocysteine resulting in the production of homolanthionine, as described in WO2007077041 and in WO2009043803,

- *ybdL* gene of SEQ ID N°317 encodes an aminotransferase as described in patent application WO2012090021,

- *yncA* gene of SEQ ID N°126, better known as *mnaT,* encodes a N-acyltransferase, as described in patent application WO2010020681,

- *metE* gene of SEQ ID N°29 encodes a cobalamin-independent methionine synthase, as described in patent application WO2013190343,

- *dgsA* gene of SEQ ID N°169, better known as *mlc,* encodes a transcriptional dual regulator that controls the expression of genes encoding enzymes of the phosphotransferase (PTS) and phosphoenolpyruvate (PEP) systems as described in patent application WO2013001055,

- *udhA* gene of SEQ ID N°194, better known as *sthA,* encodes soluble pyridine nucleotide transhydrogenase, as described in patent application WO2012055798.

**Culture conditions**

In a last aspect of the invention, a method is optimized for the fermentative production of methionine and/or its derivatives. It comprises the followings steps:

- Culturing a recombinant microorganism wherein isoleucine and/or phenylalanine and/or threonine biosynthesis are attenuated or completely abolished as disclosed previously, and,

- Recovering methionine and/or its derivatives from the culture medium.

In a particular embodiment, the method of the invention is a method optimized for the fermentative production of methionine and/or its derivatives comprising the followings steps:

- Culturing a recombinant microorganism wherein isoleucine and phenylalanine biosynthesis are completely abolished and threonine biosynthesis is attenuated as disclosed previously and,

- Recovering methionine and/or its derivatives from the culture medium.

Those skilled in the art are able to define the culture conditions for the microorganisms according to the invention. In particular the bacteria are fermented at a temperature between 20°C and 55°C, preferentially between 25°C and 40°C, and more specifically about 30°C for C. *glutamicum* and about 37°C for *E. coli.*

For *E. coli,* the culture medium can be of identical or similar composition to an M9 medium (Anderson, 1946), an M63 medium (Miller, 1992); or a medium such as defined by Schaefer et al., (1999).

For C. *glutamicum,* the culture medium can be of identical or similar composition to BMCG medium (Liebl et al., 1989) or to a medium such as described by Riedel et al., (2001).

The culture medium composition can be adjusted according to genetic modification of the microorganism grown:

- isoleucine, valine and leucine amino acids is added when *ilvD* or *ilvC* or *ilvBN* and ilvIH genes are deleted, as these genes are common to these three amino acid biosynthesis pathways,

- phenylalanine amino acid is added when pheA gene is deleted,

- threonine amino acid is added when thrBC genes are deleted.

The quantities to be added depend on the dry weight and are well known by the man skilled in the art.

In some embodiment of the invention, the growth of the recombinant microorganism is subjected to a limitation or starvation for one or several inorganic substrates, in particular phosphate and/or potassium, in the culture medium. It refers to condition under which growth of the microorganisms is governed by the quantity of an inorganic chemical supplied that still permits weak growth. Such limitation in microorganism growth has been described in the patent application WO2009043372. In a preferred embodiment of the invention, the culture is subjected to phosphate limitation.

In a particular embodiment of the method of the invention, the recombinant microorganism is from the bacterial family *Enterobacteriaceae* or *Corynebacteriaceae.* Preferentially, the recombinant microorganism is *Escherichia coli* or *Corynebacterium glutamicum,* and more preferentially the recombinant microorganism of the invention is *E. coli.*

The action of "recovering methionine and/or its derivatives from the culture medium" designates the action of recovering L-methionine and/or one of its derivatives, in particular N-acetyl methionine (NAM) and S-adenosyl methionine (SAM) and all other derivatives that may be useful such as hydroxy-methionine (or methionine hydroxy analogue or MHA). The action of "recovering methionine from the culture medium" designates the action of recovering methionine from the fermentation medium whatever its purity degree. "Recovering" means recovering the first product directly obtained from the fermentative process (fermentation must) which contains the product of interest (in this case methionine) and other co-products of the fermentation so with a more or less acceptable purity degree.

The "purifying" step consists of specifically purify the product of interest (in this case methionine) in order to obtain said product of interest with an improved purity degree.

Methionine might be recovered and purified by techniques and means well known by the man skilled in the art like distillation, ion-exchange chromatographic methods, precipitation, crystallization or complexation with salts and particularly with calcium salts or ammonium salts. Such methods for the recovery and purification of the produced compounds are in particular disclosed in WO2005007862 and in WO2005059155. Preferably, the step of recovering methionine and/or its derivatives comprises a step of concentration of methionine and/or its derivatives in the fermentation broth.

The amount of product in the fermentation medium can be determined using a number of methods known in the art, for example, high performance liquid chromatography (HPLC) or gas chromatography (GC). For example the quantity of methionine obtained in the medium is measured by HPLC after OPA/Fmoc derivatization using L-methionine (Fluka, Ref 64319) as a standard. The amount of NAM is determinated using refractometric HPLC using NAM (Sigma, Ref 01310) as a standard.

## EXAMPLES

The following experiments demonstrate how to facilitate the purification of L-methionine by producing directly L-methionine free of isoleucine, phenylalanine and threonine, which are the three amino acids the most difficult to separate from L-methionine, using an *E.coli* recombinant L-methionine producer strain as background.

In the examples given below, methods well known in the art were used to construct *E. coli* strains containing replicating vectors and/or various chromosomal insertions, deletions, and substitutions using homologous recombination well described by Datsenko & Wanner, (2000).

## PROTOCOLS

Several protocols have been used to construct methionine producing strains described in the following examples.

Protocol 1 (Chromosomal modifications by homologous recombination, selection of recombinants and antibiotic cassette excision) and protocol 2 (Transduction of phage P1) used in this invention have been fully described in patent application WO2013001055.

**Table 1:** Sequences cited in the followings examples

| SEQ ID N° | Sequence 5' → 3' |
|---|---|
| 240 | caataaatacaaaaaatgggacggcacgcaccgtcccatttacgagacag |
| 241 | gttacaggtaagcgatgccgaactggcggcgcgtcgtgaagcgcaggacg |
| 242 | caaacgcgaaccgaacaataagaagcacaacatcacgaggaatcaccatg |
| 243 | cgtattgctgttgcgggttaagtgcgcgctgatgccctcaccccgaccct |
| 244 | cgtattgctgttgcgggttaagtgcgcgctgatgccctcaccccgactct |
| 245 | agcgtctggagcagatgataagccaaatcgataagctggaagatgtcgtg |
| 246 | caattgcttaagcaagatcggacggttaatgtgttttacacattttttcc |
| 247 | gcgaaaattgtggaggttgctcgctctggtgtggtcggactttcgcgcgg |
| 248 | ctcccaaatcggggggcctttttattgataacaaaaaggcaacactatg |
| 249 | gtgcctgttgatccaacctgatgaaaaggtgccggatgatgtgaatcatc |

(continued)

| SEQ ID N° | Sequence 5' → 3' |
|---|---|
| 250 | cggcggtgacacctgttgatggtgcattgctcggagatgtagtcacggtt |
| 251 | gcagaacgtgctgatttacccttgctttcacataatctgcccgccgattt |
| 252 | tcacactggctcaccttcgggtgggcctttctgc |
| 253 | gagctgttggcgattaatcatccggctcgtatattgtgtggggttgcatgtactggagg acagacc |
| 254 | gtgtctttgctgatctgctacgtaccctctcatggaagttaggagtctga |
| 255 | atggttaaagtttatgccccggcttccagtgccaatatgagcgtcgggtt |

**EXAMPLE 1: Deletion of the isoleucine biosynthesis pathway in an *E.coli* L-methionine overproducer recombinant strain - Description of strain 1 and construction of strains 2 to 7.**

Description of Stain 1

The strain 9 described in patent application WO2015028674 will be named strain 1 in the current patent application. The genes of interest involved in the isoleucine, phenylalanine and threonine biosynthesis pathways were deleted or attenuated in strain 1.

Construction of Stains 2 to 7

The gene encoding the dihydroxy acid dehydratase, *ilvD* (SEQ ID N°1), or gene encoding the acetohydroxy acid isomeroreductase, *ilvC* (SEQ ID N°3), or genes encoding the acetohydroxy acid synthase I, *ilvBN* (SEQ ID N°5 and N°7), and genes encoding the acetolactate synthase III, *ilvIH* (SEQ ID N°9 and N°11), were deleted in *E.coli* recombinant L-methionine producer strain 1.

To achieve the deletion of *ilvD, ilvC, ilvBN* and *ilvIH,* the homologous recombination strategy described by Datsenko & Wanner, 2000 (according to Protocol 1) was used.

For each deletion, a fragment carrying a resistance marker flanked by DNA sequences homologous to upstream and downstream regions of the targeted gene was PCR amplified by the overlapping PCR technique (overlapping oligonucleotides). The sequences for recombination into upstream and downstream regions of the targeted genes are referred as SEQ ID N° 240 and 241, SEQ ID N° 242 and 243, SEQ ID N° 244 and 245 and SEQ ID N° 246 and 247 (listed in table 1), for *ilvD, ilvC, ilvBN* and *ilvIH* respectively. The corresponding PCR products obtained *"ΔilvD::Km", "ΔilvC::Km", "ΔilvBN::Km"* and *"ΔilvIH::Cm"* were then introduced by electroporation into the strain MG1655 *metA**11 (pKD46), separately. The antibiotic resistant transformants were selected and the deletions of *ilvD* or *ilvC* or *ilvBN* or *ilvIH* genes with the associated resistance cassette were verified by a PCR analysis with appropriate oligonucleotides. The strains retained were designated MG1655 *metA**11 *ΔilvD::Km,* MG1655 *metA**11 *ΔilvC::Km,* MG1655 *metA**11 *ΔilvBN::Km* and MG1655 *metA**11 *ΔilvIH::Cm.* Finally, the *ΔilvD::Km* and *ΔilvC::Km* were transferred by P1 phage transduction individually (according to Protocol 2) from MG1655 *metA**11 *ΔilvD::Km* and MG1655 *metA**11 *ΔilvC::Km* to strain 1. The *ΔilvBN::Km* and *ΔilvIH::Cm* deletions were both transferred by P1 phage transduction successively (according to Protocol 2) from the MG1655 *metA**11 *ΔilvBN::Km* and MG1655 *metA**11 *ΔilvIH::Cm* to strain 1. Chloramphenicol or kanamycin resistant transductants were selected and the presence of *ΔilvD::Km,* or *ΔilvC::Km,* or *ΔilvBN::Km* and *ΔilvIH::Cm* deletions was verified by a PCR analysis with appropriate oligonucleotides. The strains retained were named as follows:

- strain 2: strain 1 + *ΔilvD::Km,*
- strain 3: strain 1 + *ΔilvC::Km,*
- strain 4: strain 1 + *ΔilvBN::Km* + *ΔilvIH::Cm*

The antibiotic cassettes were removed from each locus *ilvD, ilvC* and both of *ilvBN* and *ilvIH* in strains 2, 3, and 4, using the Flp recombinase as described by Datsenko & Wanner, 2000 (according to Protocol 1). The kanamycin and chloramphenicol sensible transformants were selected and the absence of antibiotic cassette at loci of concerned was verified by a PCR analysis with appropriate oligonucleotides. The strains retained were named strain 5, strain 6 and strain 7, descendant of strain 2, stain 3 and stain 4, respectively.

As the large subunit of acetolactate synthase II encoded by *ilvG* (SEQ ID N°256) is not functional into strain 1, it was not necessary to delete the genes *ilvGM* encoding the acetolactate synthase II (*ilvM:* SEQ ID N°261)).

**EXAMPLE 2: Deletion of phenylalanine biosynthesis pathway in an *E.coli* L-methionine overproducer recombinant strain - Construction of strain 8.**

The gene encoding the chorismate mutase / prephenate dehydratase, *pheA* (SEQ ID N°13), was deleted in *E.coli* recombinant L-methionine producer strain 1.

To achieve the deletion of *pheA* gene, the homologous recombination strategy described by Datsenko & Wanner, 2000 (according to Protocol 1) was used.

For *pheA* deletion, a fragment carrying a resistance marker flanked by DNA sequences homologous to upstream and downstream regions of the targeted gene was PCR amplified by the overlapping PCR technique (overlapping oligonucleotides). The sequences for recombination into upstream and downstream regions of *pheA* gene are referred as SEQ ID N° 248 and 249 (listed in table 1). The PCR product obtained *"ΔpheA::Km"* was then introduced by electroporation into the strain MG1655 *metA**11 (pKD46). The antibiotic resistant transformants were selected and the deletion of *pheA* gene with the associated resistance cassette was verified by a PCR analysis with appropriate oligonucleotides. The strain retained was designated MG1655 *metA**11 *ΔpheA::Km.* Finally, the *ΔpheA::Km* deletion was transferred by P1 phage transduction (according to Protocol 2) from MG1655 *metA**11 *ΔpheA::Km* to strain 1. Kanamycin resistant transductants were selected and the presence of *ΔpheA::Km* deletion was verified by a PCR analysis with appropriate oligonucleotides. The strain retained was named strain 8.

**EXAMPLE 3: Deletion or attenuation of threonine biosynthesis pathway in an *E.coli* L-methionine overproducer recombinant strain - Construction of strains 9 and 10.**

The gene encoding the homoserine kinase, *thrB* (SEQ ID N°232), and the gene encoding the threonine synthase, *thrC* (SEQ ID N°234), were deleted or their expression was attenuated in *E.coli* recombinant L-methionine producer strain 1.

To achieve the deletion of *thrBC* genes, the homologous recombination strategy described by Datsenko & Wanner, 2000 (according to Protocol 1) was used.

For *thrBC* deletion, a fragment carrying a resistance marker flanked by DNA sequences homologous to upstream and downstream regions of the targeted genes was PCR amplified by the overlapping PCR technique (overlapping oligonucleotides). The sequences for recombination into upstream and downstream regions of *thrBC* genes are referred as SEQ ID N° 250 and 251 (listed in table 1). The PCR product obtained "*ΔthrBC::Cm*" was then introduced by electroporation into the strain MG1655 *metA**11 (pKD46). The antibiotic resistant transformants were selected and the deletion of *thrBC* gene with the associated resistance cassette was verified by a PCR analysis with appropriate oligonucleotides. The strain retained was designated MG1655 *metA**11 *ΔthrBC::Cm.* Finally, the *ΔthrBC::Cm* deletion was transferred by P1 phage transduction (according to Protocol 2) from MG1655 *metA**11 *ΔthrBC::Cm* to strain 1. Chloramphenicol resistant transductants were selected and the presence of *ΔthrBC::Cm* deletion was verified by a PCR analysis with appropriate oligonucleotides. The strain retained was named strain 9.

To achieve the attenuation of *thrBC genes,* the homologous recombination strategy described by Datsenko & Wanner, 2000 (according to Protocol 1) was used.

For attenuation of expression of *thrBC* genes, a fragment carrying a transcriptional terminator (SEQ ID N° 252, listed in table 1), a resistance marker and a weak promoter with a ribosome binding site (SEQ ID N° 253, listed in table 1), flanked by DNA sequence homologous to upstream region of the *thrB* gene and sequence homologous to the beginning of *thrB* gene was PCR amplified by the overlapping PCR technique (overlapping oligonucleotides). The sequences for recombination into upstream region of *thrB* gene and the beginning of *thrB* gene are referred as SEQ ID N° 254 and 255 (listed in table 1).

The PCR product obtained "Ptrc244-*thrBC::Cm*" was then introduced by electroporation into the strain MG1655 *metA**11 (pKD46). The antibiotic resistant transformants were selected and the attenuation Ptrc244-*thrBC* with the associated resistance cassette was verified by a PCR analysis with appropriate oligonucleotides. The strain retained was designated MG1655 *metA**11 Ptrc244-*thrBC::Cm.* Finally, the Ptrc244-*thrBC::Cm* attenuation was transferred by P1 phage transduction (according to Protocol 2) from MG1655 *metA**11 Ptrc244-*thrBC::Cm* to strain 1. Chloramphenicol resistant transductants were selected and the presence of Ptrc244-*thrBC::Cm* attenuation was verified by a PCR analysis with appropriate oligonucleotides. The strain retained was named strain 10.

**EXAMPLE 4: Deletion of isoleucine, phenylalanine and threonine biosynthesis pathways in an**

***E.coli* L-methionine overproducer recombinant strain - Construction of strains 11 to 13.**

To delete the isoleucine, phenylalanine and threonine biosynthesis pathways in *E.coli* recombinant L-methionine producer strain 1 as background, the Δ*pheA::Km* and Δ*thrBC::Cm* deletions were both successively transferred by P1 phage transduction (according to Protocol 2) from the MG1655 *metA**11 Δ*pheA::Km* and MG1655 *metA**11 Δ*thrBC::Cm* to strain 5 or to strain 6 or to strain 7, respectively. Chloramphenicol or kanamycin resistant transductants were selected and the presence of Δ*ilvD,* or Δ*ilvC,* or Δ*ilvBN,* Δ*pheA::Km* and Δ*thrBC::Cm* deletions was verified by a PCR analysis with appropriate oligonucleotides. The strains retained were named as follows:

- strain 11: strain 5 + Δ*pheA::Km* + Δ*thrBC::Cm,*
- strain 12: strain 6 + Δ*pheA::Km* + Δ*thrBC::Cm,*
- strain 13 : strain 7 + Δ*pheA::Km* + Δ*thrBC::Cm.*

**EXAMPLE 5: Deletion of isoleucine and phenylalanine biosynthesis pathways and attenuation of threonine biosynthesis pathway in an *E.coli* L-methionine overproducer recombinant strain - Construction of strains 14 to 16.**

To delete the isoleucine and phenylalanine biosynthesis pathways with the attenuation of the threonine biosynthesis pathway in *E.coli* recombinant L-methionine producer strain 1 as background, the Δ*pheA::Km* deletion and the Ptrc244-*threC::Cm* attenuation were both transferred by P1 phage transduction successively (according to Protocol 2) from the strains MG1655 *metA**11 Δ*pheA::Km* and MG1655 *metA**11 Ptrc244-*thrBC::Cm* to strain 5 or to strain 6 or to strain 7, respectively. Chloramphenicol or kanamycin resistant transductants were selected and the presences of Δ*ilvD,* or Δ*ilvC,* or Δ*ilvBN,* Δ*pheA::Km* deletions and Ptrc244-*thrBC::Cm* attenuation were verified by a PCR analysis with appropriate oligonucleotides. The strains retained were named as follows:

- strain 14: strain 5 + Δ*pheA::Km* + Ptrc244-*thrBC::Cm,*
- strain 15: strain 6 + Δ*pheA::Km* + Ptrc244-*thrBC::Cm,*
- strain 16 : strain 7 + Δ*pheA::Km* + Ptrc244-*thrBC::Cm.*

**EXAMPLE 6: Production of L-methionine free of isoleucine, phenylalanine and/or threonine by fermentation in shake flasks**

Production of strains 1 and 11 to 16 were assessed in small Erlenmeyer flasks. A 5.5 mL preculture was grown at 30°C for 21 hours in a mixed medium (10 % LB medium (Sigma 25 %) with 2.5 $g.L^{-1}$ glucose and 90 % minimal medium PC1). It was used to inoculate a 50 mL culture of PC1 medium (Table 2) to an $OD_{600}$ of 0.2. When necessary antibiotics were added at a concentration of 50 $mg.L^{-1}$ for kanamycin and spectinomycin, at a concentration of 30 $mg.L^{-1}$ for chloramphenicol and at a concentration of 10 $mg.L^{-1}$ for gentamycin. When necessary amino acids were added at a concentration of 1.4 mM for isoleucine, 2 mM for valine, 2.1 mM for leucine, 0.9 mM for phenylalanine, and at 1.2 mM for threonine. These concentrations are necessary for auxotrophic strains and sufficient to produce biomass, but not to accumulate at the end of the culture. Increase of 20% of these concentrations was also used in certain cultures in aim to evaluate strains without risk of limitation of amino acids of concerned. IPTG was added to the cultures at 0.02 mM. The growth temperature of the cultures was 37°C. When the culture had reached 5g/L of dry weight, extracellular amino acids were quantified by HPLC after OPA/Fmoc derivatization and other relevant metabolites were analyzed using HPLC with refractometric detection (organic acids and glucose) and GC-MS after silylation. For each strain, several repetitions were made.

The methionine yield was expressed as followed:

$$Y_{met} = \frac{methionine\ (g)}{consummed\ glu\cos e\ (g)} * 100$$

The methionine, isoleucine, phenylalanine and threonine titer was expressed as followed:

$$Titer_{methionine} = \frac{methionine\ (mg)}{volume\ (L)}$$

$$Titer_{isoleucine} = \frac{isoleucine\ (mg)}{volume\ (L)}$$

$$Titer_{phenylalanine} = \frac{phenylalanine\ (mg)}{volume\ (L)}$$

$$Titer_{threonine} = \frac{threonine\ (mg)}{volume\ (L)}$$

**Table 2:** Minimal medium composition (PC1).

| **Compound** | **Concentration ($g.L^{-1}$)** |
|---|---|
| $ZnSO_4.7H_2O$ | 0.0040 |
| $CuCl_2.2H_2O$ | 0.0020 |
| $MnSO_4.H_2O$ | 0.0200 |
| $CoCl_2.6H_2O$ | 0.0080 |
| $H_3BO_3$ | 0.0010 |
| $Na_2MoO_4.2H_2O$ | 0.0004 |
| $MgSO_4.7H_2O$ | 1.00 |
| Citric acid | 6.00 |
| $CaCl_2.2H_2O$ | 0.04 |
| $K_2HPO_4$ | 8.00 |
| $Na_2HPO_4$ | 2.00 |
| $(NH_4)_2HPO_4$ | 8.00 |
| $NH_4Cl$ | 0.13 |
| NaOH 4M | Adjusted to pH 6.8 |
| $FeSO_4.7H_2O$ | 0.04 |
| Thiamine | 0.01 |
| Glucose | 15.00 |
| Ammonium thiosulfate | 5.60 |
| Vitamin B12 | 0.01 |
| MOPS | 15.00 |

***Effect of the deletion or attenuation of isoleucine, phenylalanine and threonine pathways on the production of methionine in different strains***

**Table 3:** Yields of methionine ($Y_{Met}$) in % g of product /g of glucose and titer of methionine, isoleucine, phenylalanine and threonine (mM), produced in batch culture was evaluated for strains 1, 11 and 14, with addition according to the need of the strain of 1.4 mM for isoleucine, 2 mM for valine, 2.1 mM for leucine, 0.9 mM for phenylalanine, and at 1.2 mM for threonine. For the precise definition of the methionine/glucose yield see above. For each strain, two repetitions were made.

| Strain | $Y_{Met}$ (g/g) | Titer of methionine (mM) | Titer of isoleucine (mM) | Titer of phenylalanine (mM) | Titer of threonine (mM) |
|---|---|---|---|---|---|
| Strain 1 (Ref) | **16.5** n = 15 | 9.5 | 0.8 | 0.04 | 0.01 |
| Strain 11 | 16.8 | 9.9 | -0 | -0 | -0 |
| Strain 14 | 16.6 | 9.7 | -0 | -0 | 0- |

Table 3 shows that strains 11 and 14 are optimized for the fermentative production of L-methionine as deletion or attenuation of isoleucine, phenylalanine and threonine pathways allows to decrease the residual concentrations of these amino acids in culture medium. Results similar to those of strain 11 were obtained with strain 12 and stain 13; and results similar to those of strain 14 were obtained with strain 15 and stain 16 (data not shown).

The most interesting result presented in table 3 above is that upon deletion (strain 11) or attenuation (strain 14) of the three pathways isoleucine, phenylalanine and threonine, the L-methionine production is improved by two means; production performances (with higher titer and yield) and purity. Indeed, experiments have shown that in the liquid broth at the end of the fermentation, the amount of carbon compounds decrease to the benefit of the L-methionine concentration. Therefore, L-methionine is purer and easier to purify from those 3 amino acids which are recognized as to be hard to separate from L-methionine.

## REFERENCES

- Anderson, 1946, Proc. Natl. Acad. Sci. USA 32:120-128.
- Datsenko K.A., Wanner B.L., 2000, Proceedings of the National Academy of Sciences of the U S A, 97:6640-6645
- Ding et al. 2016, Sci Rep. 6:32208
- Ikeda et al. 1993, Applied microbiology and biotechnology. 39-3: 318-323.
- Kennerknecht et al. 2002, J. Bacteriol. 184: 3947-3956.
- Liebl et al., 1989, Appl. Microbiol. Biotechnol. 32: 205-210.
- Miller, 1992; "A Short Course in Bacterial Genetics: A Laboratory Manual and Handbook for Escherichia coli and Related Bacteria"; Cold Spring Harbor Laboratory Press, Cold Spring Harbor, New York.
- Riedel et al., 2001, J. Mol. Microbiol. Biotechnol. 3: 573-583.
- Schaefer et al. 1999, Anal. Biochem. 270: 88-96.
- Yin et al. 2012, Metab. Eng. 14: 542-550.

## Claims

1. A recombinant microorganism optimized for the fermentative production of methionine, wherein the biosynthesis of at least one by-product chosen among isoleucine, phenylalanine or threonine is attenuated in said microorganism.

2. The microorganism of claim 1, wherein the biosynthesis of at least one by-product chosen among isoleucine, phenylalanine or threonine is abolished.

3. The microorganism of claim 2, wherein the biosynthesis of isoleucine and phenylalanine and threonine are abolished.

4. The microorganism of claim 1 or 2, wherein biosynthesis of isoleucine is abolished by deletion of at least one gene chosen among *ilvD* of SEQ ID N°1, *ilvC* of SEQ ID N°3, *ilvB* of SEQ ID N°5, *ilvN* of SEQ ID N°7, *ilvI* of SEQ ID N°9 and *ilvH* of SEQ ID N°11.

5. The microorganism of claim 1 or 2, wherein biosynthesis of phenylalanine is abolished by deletion of at least one gene chosen among *pheA* of SEQ ID N°13.

6. The microorganism of claim 1 or 2, wherein biosynthesis of threonine is abolished by deletion of at least one gene chosen among *thrB* of SEQ ID N°232, *thrC* of SEQ ID N°234.

7. The microorganism of anyone of claims 1 to 6, wherein the expression of at least one of the following genes is increased: *ptsG* of SEQ ID N°171, *pyc* of SEQ ID N° 145, *pntAB* of SEQ ID N°190 and 192, *cysP* of SEQ ID N°57, *cysU* of SEQ ID N°59, *cysW* of SEQ ID N°61, *cysA* of SEQ ID N°63, *cysM* of SEQ ID N°65, *cysJ* of SEQ ID N°67, *cysI* of SEQ ID N°69, *cysH* of SEQ ID N°71, *gcvT* of SEQ ID N°99, *gcvH* of SEQ ID N°101, *gcvP* of SEQ ID N°103, *lpd* of SEQ ID N°121, *serA* of SEQ ID N°107, *serB* of SEQ ID N°109, *serC* of SEQ ID N°105, *cysE* of SEQ ID N°55, *metF* of SEQ ID N°27, *metH* of SEQ ID N°25, *metA* of SEQ ID N°17, or a *metA* allele encoding for an enzyme with reduced feed-back sensitivity to S-adenosylmethionine and/or methionine (*metA** of SEQ ID N°19), *thrA* of SEQ ID N°21, or a *thrA* allele encoding for an enzyme with reduced feed-back inhibition to threonine (*thrA** of of SEQ ID N°23).

**8.** The microorganism of claim 7, wherein at least one gene is under the control of an inducible promoter.

**9.** The microorganism of anyone of claims 1 to 8, wherein the expression of at least one of the following genes is attenuated: *metJ* of SEQ ID N°15, *pykA* of SEQ ID N°141, *pykF* of SEQ ID N°143, *purU* of SEQ ID N°111, *ybdL* of SEQ ID N°317, *metE* of SEQ ID N°29 or *yncA* of SEQ ID N°126.

**10.** The microorganism of anyone of claims 1 to 9, wherein:

a. the genes *ilvD* of SEQ ID N°1, *ilvC* of SEQ ID N°3, *ilvB* of SEQ ID N°5, *ilvN* of SEQ ID N°7, *ilvI* of SEQ ID N°9, *ilvH* of SEQ ID N°11, *pheA* of SEQ ID N°13, *thrB* of SEQ ID N°232 and *thrC* of SEQ ID N°234 are deleted
b. the expression of the genes *metA** of SEQ ID N°19, *metH* of SEQ ID N°25, *cysPUWAM* of SEQ ID N°57-59-61-63-65 respectively, *cysJIH* of SEQ ID N°67-69-71 respectively, *gcvTHP* of SEQ ID N°99-101-103 respectively, *metF* of SEQ ID N°27, *serA* of SEQ ID N°107, *serB* of SEQ ID N°109, *serC* of SEQ ID N°105, *cysE* of SEQ ID N°55, *thrA** of SEQ ID N°23 and *pyc* of SEQ ID N°145 are enhanced; and
c. the expression of the genes *metJ* of SEQ ID N°15, *pykA* of SEQ ID N°141, *pykF* of SEQ ID N°143, *purU* of SEQ ID N°111 and *yncA* of SEQ ID N°126 are attenuated.

**11.** The microorganism of anyone of claims 1 to 10, wherein said microorganism is from the bacterial family *Enterobacteriaceae* or *Corynebacteriaceae.*

**12.** The microorganism of anyone of claims 1 to 11, wherein said microorganism is *Escherichia coli.*

**13.** A method for the fermentative production of methionine comprising the steps of:

a. culturing a recombinant microorganism according to anyone of claims 1 to 12 in an appropriate culture medium comprising a fermentable source of carbon and a source of sulphur, and
b. recovering methionine or its derivatives from the culture medium.

**14.** The method of claim 13 wherein growth of the recombinant microorganism is subjected to limitation or deficiency for one or several inorganic substrate(s), in particular phosphate and/or potassium, in the culture medium.

Europäisches Patentamt
European Patent Office
Office européen des brevets

# EUROPEAN SEARCH REPORT

Application Number
EP 16 30 6172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | USUDA Y. ET AL: "Effects of Deregulation of Methionine Biosynthesis on Methionine Excretion in Escherichia coli", APPLIED AND ENVIRONMENTAL MICROBIOLOGY, vol. 71, no. 6, 1 June 2005 (2005-06-01), pages 3228-3234, XP055039220, ISSN: 0099-2240, DOI: 10.1128/AEM.71.6.3228-3234.2005<br>* the whole document *<br>----- | 1-4,7-14 | INV.<br>C12P13/12 |
| A | LI Y. ET AL: "Metabolic engineering of Corynebacterium glutamicum for methionine production by removing feedback inhibition and increasing NADPH level", ANTONIE VAN LEEUWENHOEK, vol. 109, no. 9, 2 June 2016 (2016-06-02), pages 1185-1197, XP036041196, ISSN: 0003-6072, DOI: 10.1007/S10482-016-0719-0<br>* the whole document *<br>----- | 1-4,7-14 | |
| | | | TECHNICAL FIELDS SEARCHED (IPC) |
| X,D | WO 2007/077041 A1 (METABOLIC EXPLORER SA [FR]; FIGGE RAINER [FR]; LUX FABIEN [FR]; RAYNAU) 12 July 2007 (2007-07-12)<br>* the whole document *<br>* page 8, line 10 - page 9, line 3 *<br>* claims 1-27 *<br>----- | 1,2,4, 7-9, 11-14 | C12P |
| X | US 2010/047879 A1 (FIGGE RAINER [FR] ET AL) 25 February 2010 (2010-02-25)<br>* the whole document *<br>* paragraph [0087] - paragraph [0088] *<br>* claims 1-19 *<br>-----<br>-/-- | 1,2,4, 7-9, 11-14 | |

~~The present search report has been drawn up for all claims~~

2

| Place of search | Date of completion of the search | Examiner |
|---|---|---|
| The Hague | 21 February 2017 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 16 30 6172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/082254 A2 (METABOLIC EXPLORER SA [FR]; FIGGE RAINER [FR]; BESTEL-CORRE GWENAELLE) 10 August 2006 (2006-08-10) * the whole document * * page 9, line 20 - page 10, line 7 * * claims 1-26 * ----- | 1,2,4, 7-9, 11-13 | |
| X,D | WO 2015/028674 A1 (METABOLIC EXPLORER SA [FR]) 5 March 2015 (2015-03-05) * the whole document * ----- | 1,7-9, 11-14 | |
| X,D | WO 2015/028675 A1 (METABOLIC EXPLORER SA [FR]) 5 March 2015 (2015-03-05) * the whole document * ----- | 1,7,9, 11-14 | |
| X,D | WO 2012/090021 A1 (METABOLIC EXPLORER SA [FR]; BESTEL-CORRE GWENAELLE [FR]; BOISART CEDRI) 5 July 2012 (2012-07-05) * the whole document * ----- | 1,7-9, 11-14 | |
| X,D | WO 2011/080301 A2 (METABOLIC EXPLORER SA [FR]; BOISART CEDRIC [FR]; BESTEL-CORRE GWENAELL) 7 July 2011 (2011-07-07) * the whole document * ----- | 1,7-9, 11-14 | TECHNICAL FIELDS SEARCHED (IPC) |
| X,D | WO 2009/043372 A1 (METABOLIC EXPLORER SA [FR]; FIGGE RAINER [FR]; SOUCAILLE PHILIPPE [FR]) 9 April 2009 (2009-04-09) * the whole document * ----- | 1,7-9, 11-14 | |
| A | HIRASAWA T. ET AL: "Recent advances in amino acid production by microbial cells", CURRENT OPINION IN BIOTECHNOLOGY, vol. 42, 2 May 2016 (2016-05-02), pages 133-146, XP029831144, ISSN: 0958-1669, DOI: 10.1016/J.COPBIO.2016.04.017 * the whole document * ----- | 1-4,7-14 | |

~~The present search report has been drawn up for all claims~~

2

| Place of search | Date of completion of the search | Examiner |
|---|---|---|
| The Hague | 21 February 2017 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

Europäisches Patentamt
European Patent Office
Office européen des brevets

Application Number
EP 16 30 6172

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

4(completely); 1-3, 7-14(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 16 30 6172

The Search Division considers that the present European patent application does not comply with the requirements of unity of invention and relates to several inventions or groups of inventions, namely:

1. claims: 4(completely); 1-3, 7-14(partially)

   A recombinant microorganism optimized for the fermentative production of methionine, wherein the biosynthesis of isoleucine is attenuated in said microorganism, preferably abolished, preferably wherein the biosynthesis of isoleucine, phenylalanine and threonine are abolished. A method for the fermentative production of methionine comprising culturing said recombinant microorganism.

   ---

2. claims: 5(completely); 1-3, 7-14(partially)

   A recombinant microorganism optimized for the fermentative production of methionine, wherein the biosynthesis of phenylalanine is attenuated in said microorganism, preferably abolished, preferably wherein the biosynthesis of isoleucine, phenylalanine and threonine are abolished. A method for the fermentative production of methionine comprising culturing said recombinant microorganism.

   ---

3. claims: 6(completely); 1-3, 7-14(partially)

   A recombinant microorganism optimized for the fermentative production of methionine, wherein the biosynthesis of threonine is attenuated in said microorganism, preferably abolished, preferably wherein the biosynthesis of isoleucine, phenylalanine and threonine are abolished. A method for the fermentative production of methionine comprising culturing said recombinant microorganism.

   ---

# ANNEX TO THE EUROPEAN SEARCH REPORT ON EUROPEAN PATENT APPLICATION NO. EP 16 30 6172

This annex lists the patent family members relating to the patent documents cited in the above-mentioned European search report. The members are as contained in the European Patent Office EDP file on The European Patent Office is in no way liable for these particulars which are merely given for the purpose of information.

21-02-2017

| Patent document cited in search report | Publication date | Patent family member(s) | Publication date |
|---|---|---|---|
| WO 2007077041 A1 | 12-07-2007 | AR 058914 A1 | 05-03-2008 |
| | | BR PI0620880 A2 | 18-09-2012 |
| | | CN 101351558 A | 21-01-2009 |
| | | DK 2314710 T3 | 13-06-2016 |
| | | EP 1969130 A1 | 17-09-2008 |
| | | EP 2314710 A2 | 27-04-2011 |
| | | ES 2459617 T3 | 12-05-2014 |
| | | JP 5172697 B2 | 27-03-2013 |
| | | JP 2009521939 A | 11-06-2009 |
| | | MY 148979 A | 28-06-2013 |
| | | US 2008311632 A1 | 18-12-2008 |
| | | US 2013183726 A1 | 18-07-2013 |
| | | WO 2007077041 A1 | 12-07-2007 |
| US 2010047879 A1 | 25-02-2010 | AR 073113 A1 | 13-10-2010 |
| | | BR PI0917192 A2 | 18-02-2015 |
| | | CN 102203270 A | 28-09-2011 |
| | | CN 104293845 A | 21-01-2015 |
| | | EP 2326724 A1 | 01-06-2011 |
| | | JP 5805533 B2 | 04-11-2015 |
| | | JP 2012500622 A | 12-01-2012 |
| | | KR 20110046542 A | 04-05-2011 |
| | | RU 2011108705 A | 27-09-2012 |
| | | US 2010047879 A1 | 25-02-2010 |
| | | WO 2010020290 A1 | 25-02-2010 |
| | | WO 2010020681 A1 | 25-02-2010 |
| WO 2006082254 A2 | 10-08-2006 | BR PI0607939 A2 | 30-11-2010 |
| | | CN 101374940 A | 25-02-2009 |
| | | EP 1846547 A2 | 24-10-2007 |
| | | JP 2008529485 A | 07-08-2008 |
| | | US 2008286840 A1 | 20-11-2008 |
| | | WO 2006082254 A2 | 10-08-2006 |
| WO 2015028674 A1 | 05-03-2015 | CA 2922597 A1 | 05-03-2015 |
| | | CN 105658803 A | 08-06-2016 |
| | | EP 3039153 A1 | 06-07-2016 |
| | | JP 2016529899 A | 29-09-2016 |
| | | KR 20160044037 A | 22-04-2016 |
| | | US 2016177352 A1 | 23-06-2016 |
| | | WO 2015028674 A1 | 05-03-2015 |
| WO 2015028675 A1 | 05-03-2015 | AU 2014314071 A1 | 10-03-2016 |
| | | CN 105658785 A | 08-06-2016 |
| | | EP 3039120 A1 | 06-07-2016 |
| | | JP 2016529900 A | 29-09-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 16 30 6172

This annex lists the patent family members relating to the patent documents cited in the above-mentioned European search report. The members are as contained in the European Patent Office EDP file on
The European Patent Office is in no way liable for these particulars which are merely given for the purpose of information.

21-02-2017

| Patent document cited in search report | Publication date | Patent family member(s) | Publication date |
|---|---|---|---|
| | | KR 20160044038 A | 22-04-2016 |
| | | US 2016177351 A1 | 23-06-2016 |
| | | WO 2015028675 A1 | 05-03-2015 |
| WO 2012090021 A1 | 05-07-2012 | AR 084595 A1 | 29-05-2013 |
| | | WO 2012090021 A1 | 05-07-2012 |
| WO 2011080301 A2 | 07-07-2011 | AR 079821 A1 | 22-02-2012 |
| | | BR 112012016908 A2 | 15-09-2015 |
| | | CN 102782137 A | 14-11-2012 |
| | | EP 2519637 A2 | 07-11-2012 |
| | | JP 5922036 B2 | 24-05-2016 |
| | | JP 2013516162 A | 13-05-2013 |
| | | KR 20120114325 A | 16-10-2012 |
| | | US 2012288904 A1 | 15-11-2012 |
| | | WO 2011080301 A2 | 07-07-2011 |
| WO 2009043372 A1 | 09-04-2009 | AR 068722 A1 | 02-12-2009 |
| | | BR PI0818528 A2 | 26-07-2016 |
| | | CN 101821401 A | 01-09-2010 |
| | | CN 103087971 A | 08-05-2013 |
| | | DK 2205754 T3 | 21-12-2015 |
| | | DK 2573189 T3 | 26-09-2016 |
| | | EP 2205754 A2 | 14-07-2010 |
| | | EP 2573189 A1 | 27-03-2013 |
| | | ES 2557487 T3 | 26-01-2016 |
| | | ES 2600036 T3 | 06-02-2017 |
| | | HR P20160075 T1 | 12-02-2016 |
| | | HU E026672 T2 | 28-07-2016 |
| | | HU E030032 T2 | 28-04-2017 |
| | | JP 5701604 B2 | 15-04-2015 |
| | | JP 2010539962 A | 24-12-2010 |
| | | KR 20100075587 A | 02-07-2010 |
| | | KR 20150038696 A | 08-04-2015 |
| | | PL 2573189 T3 | 31-01-2017 |
| | | RU 2010115225 A | 10-11-2011 |
| | | US 2010248311 A1 | 30-09-2010 |
| | | US 2016160249 A1 | 09-06-2016 |
| | | WO 2009043372 A1 | 09-04-2009 |
| | | WO 2009043803 A2 | 09-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7790424 B
- US 7611873 B
- WO 200210209 A
- WO 2005059093 A
- WO 2006008097 A
- WO 2005108561 A
- US 2010041108 A
- WO 2007077041 A
- WO 2011073122 A
- WO 2015028675 A
- WO 2015028674 A
- WO 2012055798 A
- WO 2013001055 A
- WO 20100047879 A
- US 5175107 A
- WO 2004076659 A
- WO 2007011939 A
- WO 2005111202 A
- WO 2009043803 A
- WO 2010020681 A
- WO 2011073738 A
- WO 2011080542 A
- WO 2011080301 A
- WO 2013190343 A
- JP 2000157267 B
- WO 2012090021 A
- WO 2009043372 A
- WO 2005007862 A
- WO 2005059155 A


### Non-patent literature cited in the description


- *Dharmendra & Gomesthe,* 2005
- **ANDERSON.** *Proc. Natl. Acad. Sci. USA,* 1946, vol. 32, 120-128
- **DATSENKO K.A. ; WANNER B.L.** *Proceedings of the National Academy of Sciences of the U S A,* 2000, vol. 97, 6640-6645
- **DING et al.** *Sci Rep.,* 2016, vol. 6, 32208
- **IKEDA et al.** *Applied microbiology and biotechnology.,* 1993, vol. 39-3, 318-323
- **KENNERKNECHT et al.** *J. Bacteriol.,* 2002, vol. 184, 3947-3956
- **LIEBL et al.** *Appl. Microbiol. Biotechnol.,* 1989, vol. 32, 205-210
- **MILLER.** A Short Course in Bacterial Genetics: A Laboratory Manual and Handbook for Escherichia coli and Related Bacteria''. Cold Spring Harbor Laboratory Press, 1992
- **RIEDEL et al.** *J. Mol. Microbiol. Biotechnol.,* 2001, vol. 3, 573-583
- **SCHAEFER et al.** *Anal. Biochem.,* 1999, vol. 270, 88-96
- **YIN et al.** *Metab. Eng.,* 2012, vol. 14, 542-550

SEQUENCE LISTING

<110> METABOLIC EXPLORER

<120> Modified microorganism for production of Methionine

<130> B71436D33946

<160> 317

<170> PatentIn version 3.5

<210> 1
<211> 1851
<212> DNA
<213> Escherichia coli

<400> 1
atgcctaagt accgttccgc caccaccact catggtcgta atatggcggg tgctcgtgcg 60

ctgtggcgcg ccaccggaat gaccgacgcc gatttcggta agccgattat cgcggttgtg 120

aactcgttca cccaatttgt accgggtcac gtccatctgc gcgatctcgg taaactggtc 180

gccgaacaaa ttgaagcggc tggcggcgtt gccaaagagt tcaacaccat tgcggtggat 240

gatgggattg ccatgggcca cggggggatg ctttattcac tgccatctcg cgaactgatc 300

gctgattccg ttgagtatat ggtcaacgcc cactgcgccg acgccatggt ctgcatctct 360

aactgcgaca aaatcacccc ggggatgctg atggcttccc tgcgcctgaa tattccggtg 420

atctttgttt ccggcggccc gatggaggcc gggaaaacca aactttccga tcagatcatc 480

aagctcgatc tggttgatgc gatgatccag ggcgcagacc cgaaagtatc tgactcccag 540

agcgatcagg ttgaacgttc cgcgtgtccg acctgcggtt cctgctccgg gatgtttacc 600

gctaactcaa tgaactgcct gaccgaagcg ctgggcctgt cgcagccggg caacggctcg 660

ctgctggcaa cccacgccga ccgtaagcag ctgttcctta atgctggtaa acgcattgtt 720

gaattgacca aacgttatta cgagcaaaac gacgaaagtg cactgccgcg taatatcgcc 780

agtaaggcgg cgtttgaaaa cgccatgacg ctggatatcg cgatgggtgg atcgactaac 840

accgtacttc acctgctggc ggcggcgcag gaagcggaaa tcgacttcac catgagtgat 900

atcgataagc tttcccgcaa ggttccacag ctgtgtaaag ttgcgccgag cacccagaaa 960

taccatatgg aagatgttca ccgtgctggt ggtgttatcg gtattctcgg cgaactggat 1020

cgcgcggggt tactgaaccg tgatgtgaaa aacgtacttg gcctgacgtt gccgcaaacg 1080

ctggaacaat acgacgttat gctgacccag gatgacgcgg taaaaaatat gttccgcgca 1140

ggtcctgcag gcattcgtac cacacaggca ttctcgcaag attgccgttg ggatacgctg 1200

gacgacgatc gcgccaatgg ctgtatccgc tcgctggaac acgcctacag caaagacggc 1260

ggcctggcgg tgctctacgg taactttgcg gaaaacggct gcatcgtgaa aacggcaggc 1320

gtcgatgaca gcatcctcaa attcaccggc ccggcgaaag tgtacgaaag ccaggacgat 1380

```
gcggtagaag cgattctcgg cggtaaagtt gtcgccggag atgtggtagt aattcgctat      1440
gaaggcccga aaggcggtcc ggggatgcag gaaatgctct acccaaccag cttcctgaaa      1500
tcaatgggtc tcggcaaagc ctgtgcgctg atcaccgacg gtcgtttctc tggtggcacc      1560
tctggtcttt ccatcggcca cgtctcaccg gaagcggcaa gcggcggcag cattggcctg      1620
attgaagatg gtgacctgat cgctatcgac atcccgaacc gtggcattca gttacaggta      1680
agcgatgccg aactggcggc gcgtcgtgaa gcgcaggacg ctcgaggtga caaagcctgg      1740
acgccgaaaa atcgtgaacg tcaggtctcc tttgccctgc gtgcttatgc cagcctggca      1800
accagcgccg acaaaggcgc ggtgcgcgat aaatcgaaac tgggggggtta a              1851
```

<210> 2
<211> 616
<212> PRT
<213> Escherichia coli

<400> 2

```
Met Pro Lys Tyr Arg Ser Ala Thr Thr Thr His Gly Arg Asn Met Ala
1               5                   10                  15

Gly Ala Arg Ala Leu Trp Arg Ala Thr Gly Met Thr Asp Ala Asp Phe
            20                  25                  30

Gly Lys Pro Ile Ile Ala Val Val Asn Ser Phe Thr Gln Phe Val Pro
        35                  40                  45

Gly His Val His Leu Arg Asp Leu Gly Lys Leu Val Ala Glu Gln Ile
    50                  55                  60

Glu Ala Ala Gly Gly Val Ala Lys Glu Phe Asn Thr Ile Ala Val Asp
65                  70                  75                  80

Asp Gly Ile Ala Met Gly His Gly Gly Met Leu Tyr Ser Leu Pro Ser
                85                  90                  95

Arg Glu Leu Ile Ala Asp Ser Val Glu Tyr Met Val Asn Ala His Cys
            100                 105                 110

Ala Asp Ala Met Val Cys Ile Ser Asn Cys Asp Lys Ile Thr Pro Gly
        115                 120                 125

Met Leu Met Ala Ser Leu Arg Leu Asn Ile Pro Val Ile Phe Val Ser
    130                 135                 140

Gly Gly Pro Met Glu Ala Gly Lys Thr Lys Leu Ser Asp Gln Ile Ile
145                 150                 155                 160
```

```
Lys Leu Asp Leu Val Asp Ala Met Ile Gln Gly Ala Asp Pro Lys Val
                165                 170                 175

Ser Asp Ser Gln Ser Asp Gln Val Glu Arg Ser Ala Cys Pro Thr Cys
            180                 185                 190

Gly Ser Cys Ser Gly Met Phe Thr Ala Asn Ser Met Asn Cys Leu Thr
        195                 200                 205

Glu Ala Leu Gly Leu Ser Gln Pro Gly Asn Gly Ser Leu Leu Ala Thr
    210                 215                 220

His Ala Asp Arg Lys Gln Leu Phe Leu Asn Ala Gly Lys Arg Ile Val
225                 230                 235                 240

Glu Leu Thr Lys Arg Tyr Tyr Glu Gln Asn Asp Glu Ser Ala Leu Pro
                245                 250                 255

Arg Asn Ile Ala Ser Lys Ala Ala Phe Glu Asn Ala Met Thr Leu Asp
            260                 265                 270

Ile Ala Met Gly Gly Ser Thr Asn Thr Val Leu His Leu Leu Ala Ala
        275                 280                 285

Ala Gln Glu Ala Glu Ile Asp Phe Thr Met Ser Asp Ile Asp Lys Leu
    290                 295                 300

Ser Arg Lys Val Pro Gln Leu Cys Lys Val Ala Pro Ser Thr Gln Lys
305                 310                 315                 320

Tyr His Met Glu Asp Val His Arg Ala Gly Gly Val Ile Gly Ile Leu
                325                 330                 335

Gly Glu Leu Asp Arg Ala Gly Leu Leu Asn Arg Asp Val Lys Asn Val
            340                 345                 350

Leu Gly Leu Thr Leu Pro Gln Thr Leu Glu Gln Tyr Asp Val Met Leu
        355                 360                 365

Thr Gln Asp Asp Ala Val Lys Asn Met Phe Arg Ala Gly Pro Ala Gly
    370                 375                 380

Ile Arg Thr Thr Gln Ala Phe Ser Gln Asp Cys Arg Trp Asp Thr Leu
385                 390                 395                 400

Asp Asp Asp Arg Ala Asn Gly Cys Ile Arg Ser Leu Glu His Ala Tyr
```

405 410 415

Ser Lys Asp Gly Gly Leu Ala Val Leu Tyr Gly Asn Phe Ala Glu Asn
420 425 430

Gly Cys Ile Val Lys Thr Ala Gly Val Asp Asp Ser Ile Leu Lys Phe
435 440 445

Thr Gly Pro Ala Lys Val Tyr Glu Ser Gln Asp Asp Ala Val Glu Ala
450 455 460

Ile Leu Gly Gly Lys Val Val Ala Gly Asp Val Val Val Ile Arg Tyr
465 470 475 480

Glu Gly Pro Lys Gly Gly Pro Gly Met Gln Glu Met Leu Tyr Pro Thr
485 490 495

Ser Phe Leu Lys Ser Met Gly Leu Gly Lys Ala Cys Ala Leu Ile Thr
500 505 510

Asp Gly Arg Phe Ser Gly Gly Thr Ser Gly Leu Ser Ile Gly His Val
515 520 525

Ser Pro Glu Ala Ala Ser Gly Gly Ser Ile Gly Leu Ile Glu Asp Gly
530 535 540

Asp Leu Ile Ala Ile Asp Ile Pro Asn Arg Gly Ile Gln Leu Gln Val
545 550 555 560

Ser Asp Ala Glu Leu Ala Ala Arg Arg Glu Ala Gln Asp Ala Arg Gly
565 570 575

Asp Lys Ala Trp Thr Pro Lys Asn Arg Glu Arg Gln Val Ser Phe Ala
580 585 590

Leu Arg Ala Tyr Ala Ser Leu Ala Thr Ser Ala Asp Lys Gly Ala Val
595 600 605

Arg Asp Lys Ser Lys Leu Gly Gly
610 615

<210> 3
<211> 1476
<212> DNA
<213> Escherichia coli

<400> 3
atggctaact acttcaatac actgaatctg cgccagcagc tggcacagct gggcaaatgt 60

```
cgctttatgg gccgcgatga attcgccgat ggcgcgagct accttcaggg taaaaaagta      120
gtcatcgtcg gctgtggcgc acagggtctg aaccagggcc tgaacatgcg tgattctggt      180
ctcgatatct cctacgctct gcgtaaagaa gcgattgccg agaagcgcgc gtcctggcgt      240
aaagcgaccg aaaatggttt taaagtgggt acttacgaag aactgatccc acaggcggat      300
ctggtgatta acctgacgcc ggacaagcag cactctgatg tagtgcgcac cgtacagcca      360
ctgatgaaag acggcgcggc gctgggctac tcgcacggtt tcaacatcgt cgaagtgggc      420
gagcagatcc gtaaagatat caccgtagtg atggttgcgc cgaaatgccc aggcaccgaa      480
gtgcgtgaag agtacaaacg tgggttcggc gtaccgacgc tgattgccgt tcacccggaa      540
aacgatccga aaggcgaagg catggcgatt gccaaagcct gggcggctgc aaccggtggt      600
caccgtgcgg gtgtgctgga atcgtccttc gttgcggaag tgaaatctga cctgatgggc      660
gagcaaacca tcctgtgcgg tatgttgcag gctggctctc tgctgtgctt cgacaagctg      720
gtggaagaag gtaccgatcc agcatacgca gaaaaactga ttcagttcgg ttgggaaacc      780
atcaccgaag cactgaaaca gggcggcatc accctgatga tggaccgtct ctctaacccg      840
gcgaaactgc gtgcttatgc gctttctgaa cagctgaaag agatcatggc acccctgttc      900
cagaaacata tggacgacat catctccggc gaattctctt ccggtatgat ggcggactgg      960
gccaacgatg ataagaaact gctgacctgg cgtgaagaga ccggcaaaac cgcgtttgaa     1020
accgcgccgc agtatgaagg caaaatcggc gagcaggagt acttcgataa aggcgtactg     1080
atgattgcga tggtgaaagc gggcgttgaa ctggcgttcg aaaccatggt cgattccggc     1140
atcattgaag agtctgcata ttatgaatca ctgcacgagc tgccgctgat tgccaacacc     1200
atcgcccgta agcgtctgta cgaaatgaac gtggttatct ctgataccgc tgagtacggt     1260
aactatctgt tctcttacgc ttgtgtgccg ttgctgaaac cgtttatggc agagctgcaa     1320
ccgggcgacc tgggtaaagc tattccggaa ggcgcggtag ataacgggca actgcgtgat     1380
gtgaacgaag cgattcgcag ccatgcgatt gagcaggtag gtaagaaact gcgcggctat     1440
atgacagata tgaaacgtat tgctgttgcg ggttaa                               1476
```

<210> 4
<211> 491
<212> PRT
<213> Escherichia coli

<400> 4

```
Met Ala Asn Tyr Phe Asn Thr Leu Asn Leu Arg Gln Gln Leu Ala Gln
1               5                   10                  15

Leu Gly Lys Cys Arg Phe Met Gly Arg Asp Glu Phe Ala Asp Gly Ala
            20                  25                  30
```

```
Ser Tyr Leu Gln Gly Lys Lys Val Val Ile Val Gly Cys Gly Ala Gln
        35                  40                  45

Gly Leu Asn Gln Gly Leu Asn Met Arg Asp Ser Gly Leu Asp Ile Ser
    50                  55                  60

Tyr Ala Leu Arg Lys Glu Ala Ile Ala Glu Lys Arg Ala Ser Trp Arg
65                  70                  75                  80

Lys Ala Thr Glu Asn Gly Phe Lys Val Gly Thr Tyr Glu Glu Leu Ile
                85                  90                  95

Pro Gln Ala Asp Leu Val Ile Asn Leu Thr Pro Asp Lys Gln His Ser
            100                 105                 110

Asp Val Val Arg Thr Val Gln Pro Leu Met Lys Asp Gly Ala Ala Leu
        115                 120                 125

Gly Tyr Ser His Gly Phe Asn Ile Val Glu Val Gly Glu Gln Ile Arg
    130                 135                 140

Lys Asp Ile Thr Val Val Met Val Ala Pro Lys Cys Pro Gly Thr Glu
145                 150                 155                 160

Val Arg Glu Glu Tyr Lys Arg Gly Phe Gly Val Pro Thr Leu Ile Ala
                165                 170                 175

Val His Pro Glu Asn Asp Pro Lys Gly Glu Gly Met Ala Ile Ala Lys
            180                 185                 190

Ala Trp Ala Ala Ala Thr Gly Gly His Arg Ala Gly Val Leu Glu Ser
        195                 200                 205

Ser Phe Val Ala Glu Val Lys Ser Asp Leu Met Gly Glu Gln Thr Ile
    210                 215                 220

Leu Cys Gly Met Leu Gln Ala Gly Ser Leu Leu Cys Phe Asp Lys Leu
225                 230                 235                 240

Val Glu Glu Gly Thr Asp Pro Ala Tyr Ala Glu Lys Leu Ile Gln Phe
                245                 250                 255

Gly Trp Glu Thr Ile Thr Glu Ala Leu Lys Gln Gly Gly Ile Thr Leu
            260                 265                 270

Met Met Asp Arg Leu Ser Asn Pro Ala Lys Leu Arg Ala Tyr Ala Leu
        275                 280                 285
```

```
Ser Glu Gln Leu Lys Glu Ile Met Ala Pro Leu Phe Gln Lys His Met
    290                 295                 300

Asp Asp Ile Ile Ser Gly Glu Phe Ser Ser Gly Met Met Ala Asp Trp
305                 310                 315                 320

Ala Asn Asp Asp Lys Lys Leu Leu Thr Trp Arg Glu Glu Thr Gly Lys
                325                 330                 335

Thr Ala Phe Glu Thr Ala Pro Gln Tyr Glu Gly Lys Ile Gly Glu Gln
            340                 345                 350

Glu Tyr Phe Asp Lys Gly Val Leu Met Ile Ala Met Val Lys Ala Gly
        355                 360                 365

Val Glu Leu Ala Phe Glu Thr Met Val Asp Ser Gly Ile Ile Glu Glu
    370                 375                 380

Ser Ala Tyr Tyr Glu Ser Leu His Glu Leu Pro Leu Ile Ala Asn Thr
385                 390                 395                 400

Ile Ala Arg Lys Arg Leu Tyr Glu Met Asn Val Val Ile Ser Asp Thr
                405                 410                 415

Ala Glu Tyr Gly Asn Tyr Leu Phe Ser Tyr Ala Cys Val Pro Leu Leu
            420                 425                 430

Lys Pro Phe Met Ala Glu Leu Gln Pro Gly Asp Leu Gly Lys Ala Ile
        435                 440                 445

Pro Glu Gly Ala Val Asp Asn Gly Gln Leu Arg Asp Val Asn Glu Ala
    450                 455                 460

Ile Arg Ser His Ala Ile Glu Gln Val Gly Lys Lys Leu Arg Gly Tyr
465                 470                 475                 480

Met Thr Asp Met Lys Arg Ile Ala Val Ala Gly
                485                 490


<210>  5
<211>  1689
<212>  DNA
<213>  Escherichia coli

<400>  5
atggcaagtt cgggcacaac atcgacgcgt aagcgcttta ccggcgcaga atttatcgtt        60

catttcctgg aacagcaggg cattaagatt gtgacaggca ttccgggcgg ttctatcctg       120
```

cctgtttacg atgccttaag ccaaagcacg caaatccgcc atattctggc ccgtcatgaa 180

cagggcgcgg gctttatcgc tcagggaatg gcgcgcaccg acggtaaacc ggcggtctgt 240

atggcctgta gcggaccggg tgcgactaac ctggtgaccg ccattgccga tgcgcggctg 300

gactccatcc cgctgatttg catcactggt caggttcccg cctcgatgat cggcaccgac 360

gccttccagg aagtggacac ctacggcatc tctatcccca tcaccaaaca caactatctg 420

gtcagacata tcgaagaact cccgcaggtc atgagcgatg ccttccgcat tgcgcaatca 480

ggccgcccag gcccggtgtg gatagacatt cctaaggatg tgcaaacggc agtttttgag 540

attgaaacac agcccgctat ggcagaaaaa gccgccgccc ccgcctttag cgaagaaagc 600

attcgtgacg cagcggcgat gattaacgct gccaaacgcc cggtgcttta tctgggcggc 660

ggtgtgatca atgcgcccgc acgggtgcgt gaactggcgg agaaagcgca actgcctacc 720

accatgactt taatggcgct gggcatgttg ccaaaagcgc atccgttgtc gctgggtatg 780

ctggggatgc acggcgtgcg cagcaccaac tatattttgc aggaggcgga tttgttgata 840

gtgctcggtg cgcgttttga tgaccgggcg attggcaaaa ccgagcagtt ctgtccgaat 900

gccaaaatca ttcatgtcga tatcgaccgt gcagagctgg gtaaaatcaa gcagccgcac 960

gtggcgattc aggcggatgt tgatgacgtg ctggcgcagt tgatcccgct ggtggaagcg 1020

caaccgcgtg cagagtggca ccagttggta gcggatttgc agcgtgagtt tccgtgtcca 1080

atcccgaaag cgtgcgatcc gttaagccat tacggcctga tcaacgccgt tgccgcctgt 1140

gtcgatgaca atgcaattat caccaccgac gttggtcagc atcagatgtg gaccgcgcaa 1200

gcttatccgc tcaatcgccc acgccagtgg ctgacctccg gtgggctggg cacgatgggt 1260

tttggcctgc ctgcggcgat tggcgctgcg ctggcgaacc cggatcgcaa agtgttgtgt 1320

ttctccggcg acggcagcct gatgatgaat attcaggaga tggcgaccgc cagtgaaaat 1380

cagctggatg tcaaaatcat tctgatgaac aacgaagcgc tggggctggt gcatcagcaa 1440

cagagtctgt tctacgagca aggcgttttt gccgccacct atccgggcaa aatcaacttt 1500

atgcagattg ccgccggatt cggcctcgaa acctgtgatt tgaataacga agccgatccg 1560

caggcttcat tgcaggaaat catcaatcgc cctggcccgg cgctgatcca tgtgcgcatt 1620

gatgccgaag aaaaagttta cccgatggtg ccgccaggtg cggcgaatac tgaaatggtg 1680

ggggaataa 1689

<210> 6
<211> 562
<212> PRT
<213> Escherichia coli

<400> 6

```
Met Ala Ser Ser Gly Thr Thr Ser Thr Arg Lys Arg Phe Thr Gly Ala
1               5                   10                  15

Glu Phe Ile Val His Phe Leu Glu Gln Gln Gly Ile Lys Ile Val Thr
            20                  25                  30

Gly Ile Pro Gly Gly Ser Ile Leu Pro Val Tyr Asp Ala Leu Ser Gln
        35                  40                  45

Ser Thr Gln Ile Arg His Ile Leu Ala Arg His Glu Gln Gly Ala Gly
    50                  55                  60

Phe Ile Ala Gln Gly Met Ala Arg Thr Asp Gly Lys Pro Ala Val Cys
65                  70                  75                  80

Met Ala Cys Ser Gly Pro Gly Ala Thr Asn Leu Val Thr Ala Ile Ala
                85                  90                  95

Asp Ala Arg Leu Asp Ser Ile Pro Leu Ile Cys Ile Thr Gly Gln Val
            100                 105                 110

Pro Ala Ser Met Ile Gly Thr Asp Ala Phe Gln Glu Val Asp Thr Tyr
        115                 120                 125

Gly Ile Ser Ile Pro Ile Thr Lys His Asn Tyr Leu Val Arg His Ile
    130                 135                 140

Glu Glu Leu Pro Gln Val Met Ser Asp Ala Phe Arg Ile Ala Gln Ser
145                 150                 155                 160

Gly Arg Pro Gly Pro Val Trp Ile Asp Ile Pro Lys Asp Val Gln Thr
                165                 170                 175

Ala Val Phe Glu Ile Glu Thr Gln Pro Ala Met Ala Glu Lys Ala Ala
            180                 185                 190

Ala Pro Ala Phe Ser Glu Glu Ser Ile Arg Asp Ala Ala Ala Met Ile
        195                 200                 205

Asn Ala Ala Lys Arg Pro Val Leu Tyr Leu Gly Gly Gly Val Ile Asn
    210                 215                 220

Ala Pro Ala Arg Val Arg Glu Leu Ala Glu Lys Ala Gln Leu Pro Thr
225                 230                 235                 240

Thr Met Thr Leu Met Ala Leu Gly Met Leu Pro Lys Ala His Pro Leu
                245                 250                 255
```

```
Ser Leu Gly Met Leu Gly Met His Gly Val Arg Ser Thr Asn Tyr Ile
            260                 265                 270

Leu Gln Glu Ala Asp Leu Leu Ile Val Leu Gly Ala Arg Phe Asp Asp
        275                 280                 285

Arg Ala Ile Gly Lys Thr Glu Gln Phe Cys Pro Asn Ala Lys Ile Ile
    290                 295                 300

His Val Asp Ile Asp Arg Ala Glu Leu Gly Lys Ile Lys Gln Pro His
305                 310                 315                 320

Val Ala Ile Gln Ala Asp Val Asp Asp Val Leu Ala Gln Leu Ile Pro
                325                 330                 335

Leu Val Glu Ala Gln Pro Arg Ala Glu Trp His Gln Leu Val Ala Asp
            340                 345                 350

Leu Gln Arg Glu Phe Pro Cys Pro Ile Pro Lys Ala Cys Asp Pro Leu
        355                 360                 365

Ser His Tyr Gly Leu Ile Asn Ala Val Ala Ala Cys Val Asp Asp Asn
    370                 375                 380

Ala Ile Ile Thr Thr Asp Val Gly Gln His Gln Met Trp Thr Ala Gln
385                 390                 395                 400

Ala Tyr Pro Leu Asn Arg Pro Arg Gln Trp Leu Thr Ser Gly Gly Leu
                405                 410                 415

Gly Thr Met Gly Phe Gly Leu Pro Ala Ala Ile Gly Ala Ala Leu Ala
            420                 425                 430

Asn Pro Asp Arg Lys Val Leu Cys Phe Ser Gly Asp Gly Ser Leu Met
        435                 440                 445

Met Asn Ile Gln Glu Met Ala Thr Ala Ser Glu Asn Gln Leu Asp Val
    450                 455                 460

Lys Ile Ile Leu Met Asn Asn Glu Ala Leu Gly Leu Val His Gln Gln
465                 470                 475                 480

Gln Ser Leu Phe Tyr Glu Gln Gly Val Phe Ala Ala Thr Tyr Pro Gly
                485                 490                 495

Lys Ile Asn Phe Met Gln Ile Ala Ala Gly Phe Gly Leu Glu Thr Cys
            500                 505                 510
```

Asp Leu Asn Asn Glu Ala Asp Pro Gln Ala Ser Leu Gln Glu Ile Ile
        515                 520                 525

Asn Arg Pro Gly Pro Ala Leu Ile His Val Arg Ile Asp Ala Glu Glu
    530                 535                 540

Lys Val Tyr Pro Met Val Pro Pro Gly Ala Ala Asn Thr Glu Met Val
545                 550                 555                 560

Gly Glu

<210> 7
<211> 291
<212> DNA
<213> Escherichia coli

<400> 7
atgcaaaaca caactcatga caacgtaatt ctggagctca ccgttcgcaa ccatccgggc 60

gtaatgaccc acgtttgtgg cctttttgcc cgccgcgctt ttaacgttga aggcattctt 120

tgtctgccga ttcaggacag cgacaaaagc catatctggc tactggtcaa tgacgaccag 180

cgtctggagc agatgataag ccaaatcgat aagctggaag atgtcgtgaa agtgcagcgt 240

aatcagtccg atccgacgat gtttaacaag atcgcggtgt tttttcagta a 291

<210> 8
<211> 96
<212> PRT
<213> Escherichia coli

<400> 8

Met Gln Asn Thr Thr His Asp Asn Val Ile Leu Glu Leu Thr Val Arg
1               5                   10                  15

Asn His Pro Gly Val Met Thr His Val Cys Gly Leu Phe Ala Arg Arg
            20                  25                  30

Ala Phe Asn Val Glu Gly Ile Leu Cys Leu Pro Ile Gln Asp Ser Asp
        35                  40                  45

Lys Ser His Ile Trp Leu Leu Val Asn Asp Asp Gln Arg Leu Glu Gln
    50                  55                  60

Met Ile Ser Gln Ile Asp Lys Leu Glu Asp Val Val Lys Val Gln Arg
65                  70                  75                  80

Asn Gln Ser Asp Pro Thr Met Phe Asn Lys Ile Ala Val Phe Phe Gln

85 90 95

<210> 9
<211> 1725
<212> DNA
<213> Escherichia coli

<400> 9
atggagatgt tgtctggagc cgagatggtc gtccgatcgc ttatcgatca gggcgttaaa 60

caagtattcg gttatcccgg aggcgcagtc cttgatattt atgatgcatt gcataccgtg 120

ggtggtattg atcatgtatt agttcgtcat gagcaggcgg cggtgcatat ggccgatggc 180

ctggcgcgcg cgaccgggga agtcggcgtc gtgctggtaa cgtcgggtcc aggggcgacc 240

aatgcgatta ctggcatcgc caccgcttat atggattcca ttccattagt tgtcctttcc 300

gggcaggtag cgacctcgtt gataggttac gatgcctttc aggagtgcga catggtgggg 360

atttcgcgac cggtggttaa acacagtttt ctggttaagc aaacggaaga cattccgcag 420

gtgctgaaaa aggctttctg gctggcggca agtggtcgcc caggaccagt agtcgttgat 480

ttaccgaaag atattcttaa tccggcgaac aaattaccct atgtctggcc ggagtcggtc 540

agtatgcgtt cttacaatcc cactactacc ggacataaag ggcaaattaa gcgtgctctg 600

caaacgctgg tagcggcaaa aaaaccggtt gtctacgtag gcggtggggc aatcacggcg 660

ggctgccatc agcagttgaa agaaacggtg gaggcgttga atctgcccgt tgtttgctca 720

ttgatggggc tgggggcgtt tccggcaacg catcgtcagg cactgggcat gctgggaatg 780

cacggtacct acgaagccaa tatgacgatg cataacgcgg atgtgatttt cgccgtcggg 840

gtacgatttg atgaccgaac gacgaacaat ctggcaaagt actgcccaaa tgccactgtt 900

ctgcatatcg atattgatcc tacttccatt tctaaaaccg tgactgcgga tatcccgatt 960

gtgggggatg ctcgccaggt cctcgaacaa atgcttgaac tcttgtcgca agaatccgcc 1020

catcaaccac tggatgagat ccgcgactgg tggcagcaaa ttgaacagtg gcgcgctcgt 1080

cagtgcctga aatatgacac tcacagtgaa aagattaaac cgcaggcggt gatcgagact 1140

ctttggcggt tgacgaaggg agacgcttac gtgacgtccg atgtcgggca gcaccagatg 1200

tttgctgcac tttattatcc attcgacaaa ccgcgtcgct ggatcaattc cggtggcctc 1260

ggcacgatgg gttttggttt acctgcggca ctgggcgtca aaatggcgtt gccagaagaa 1320

accgtggttt gcgtcactgg cgacggcagt attcagatga acatccagga actgtctacc 1380

gcgttgcaat acgagttgcc cgtactggtg gtgaatctca ataaccgcta tctggggatg 1440

gtgaagcagt ggcaggacat gatctattcc ggccgtcatt cacaatctta tatgcaatcg 1500

ctacccgatt tcgtccgtct ggcggaagcc tatgggcatg tcgggatcca gatttctcat 1560

ccgcatgagc tggaaagcaa acttagcgag gcgctggaac aggtgcgcaa taatcgcctg 1620

gtgtttgttg atgttaccgt cgatggcagc gagcacgtct acccgatgca gattcgcggg 1680

ggcggaatgg atgaaatgtg gttaagcaaa acggagagaa cctga 1725

<210> 10
<211> 574
<212> PRT
<213> Escherichia coli

<400> 10

Met Glu Met Leu Ser Gly Ala Glu Met Val Val Arg Ser Leu Ile Asp
1 5 10 15

Gln Gly Val Lys Gln Val Phe Gly Tyr Pro Gly Gly Ala Val Leu Asp
20 25 30

Ile Tyr Asp Ala Leu His Thr Val Gly Gly Ile Asp His Val Leu Val
35 40 45

Arg His Glu Gln Ala Ala Val His Met Ala Asp Gly Leu Ala Arg Ala
50 55 60

Thr Gly Glu Val Gly Val Val Leu Val Thr Ser Gly Pro Gly Ala Thr
65 70 75 80

Asn Ala Ile Thr Gly Ile Ala Thr Ala Tyr Met Asp Ser Ile Pro Leu
85 90 95

Val Val Leu Ser Gly Gln Val Ala Thr Ser Leu Ile Gly Tyr Asp Ala
100 105 110

Phe Gln Glu Cys Asp Met Val Gly Ile Ser Arg Pro Val Val Lys His
115 120 125

Ser Phe Leu Val Lys Gln Thr Glu Asp Ile Pro Gln Val Leu Lys Lys
130 135 140

Ala Phe Trp Leu Ala Ala Ser Gly Arg Pro Gly Pro Val Val Val Asp
145 150 155 160

Leu Pro Lys Asp Ile Leu Asn Pro Ala Asn Lys Leu Pro Tyr Val Trp
165 170 175

Pro Glu Ser Val Ser Met Arg Ser Tyr Asn Pro Thr Thr Thr Gly His
180 185 190

Lys Gly Gln Ile Lys Arg Ala Leu Gln Thr Leu Val Ala Ala Lys Lys
195 200 205

```
Pro Val Val Tyr Val Gly Gly Gly Ala Ile Thr Ala Gly Cys His Gln
    210                 215                 220

Gln Leu Lys Glu Thr Val Glu Ala Leu Asn Leu Pro Val Val Cys Ser
225                 230                 235                 240

Leu Met Gly Leu Gly Ala Phe Pro Ala Thr His Arg Gln Ala Leu Gly
                245                 250                 255

Met Leu Gly Met His Gly Thr Tyr Glu Ala Asn Met Thr Met His Asn
            260                 265                 270

Ala Asp Val Ile Phe Ala Val Gly Val Arg Phe Asp Asp Arg Thr Thr
        275                 280                 285

Asn Asn Leu Ala Lys Tyr Cys Pro Asn Ala Thr Val Leu His Ile Asp
    290                 295                 300

Ile Asp Pro Thr Ser Ile Ser Lys Thr Val Thr Ala Asp Ile Pro Ile
305                 310                 315                 320

Val Gly Asp Ala Arg Gln Val Leu Glu Gln Met Leu Glu Leu Leu Ser
                325                 330                 335

Gln Glu Ser Ala His Gln Pro Leu Asp Glu Ile Arg Asp Trp Trp Gln
            340                 345                 350

Gln Ile Glu Gln Trp Arg Ala Arg Gln Cys Leu Lys Tyr Asp Thr His
        355                 360                 365

Ser Glu Lys Ile Lys Pro Gln Ala Val Ile Glu Thr Leu Trp Arg Leu
    370                 375                 380

Thr Lys Gly Asp Ala Tyr Val Thr Ser Asp Val Gly Gln His Gln Met
385                 390                 395                 400

Phe Ala Ala Leu Tyr Tyr Pro Phe Asp Lys Pro Arg Arg Trp Ile Asn
                405                 410                 415

Ser Gly Gly Leu Gly Thr Met Gly Phe Gly Leu Pro Ala Ala Leu Gly
            420                 425                 430

Val Lys Met Ala Leu Pro Glu Glu Thr Val Val Cys Val Thr Gly Asp
        435                 440                 445

Gly Ser Ile Gln Met Asn Ile Gln Glu Leu Ser Thr Ala Leu Gln Tyr
    450                 455                 460
```

Glu Leu Pro Val Leu Val Val Asn Leu Asn Asn Arg Tyr Leu Gly Met
465 470 475 480

Val Lys Gln Trp Gln Asp Met Ile Tyr Ser Gly Arg His Ser Gln Ser
485 490 495

Tyr Met Gln Ser Leu Pro Asp Phe Val Arg Leu Ala Glu Ala Tyr Gly
500 505 510

His Val Gly Ile Gln Ile Ser His Pro His Glu Leu Glu Ser Lys Leu
515 520 525

Ser Glu Ala Leu Glu Gln Val Arg Asn Asn Arg Leu Val Phe Val Asp
530 535 540

Val Thr Val Asp Gly Ser Glu His Val Tyr Pro Met Gln Ile Arg Gly
545 550 555 560

Gly Gly Met Asp Glu Met Trp Leu Ser Lys Thr Glu Arg Thr
565 570

<210> 11
<211> 492
<212> DNA
<213> Escherichia coli

<400> 11
atgcgccgga tattatcagt cttactcgaa aatgaatcag gcgcgttatc ccgcgtgatt 60

ggcctttttt cccagcgtgg ctacaacatt gaaagcctga ccgttgcgcc aaccgacgat 120

ccgacattat cgcgtatgac catccagacc gtgggcgatg aaaaagtact tgagcagatc 180

gaaaagcaat tacacaaact ggtcgatgtc ttgcgcgtga gtgagttggg gcagggcgcg 240

catgttgagc gggaaatcat gctggtgaaa attcaggcca gcggttacgg gcgtgacgaa 300

gtgaaacgta atacggaaat attccgtggg caaattatcg atgtcacacc ctcgctttat 360

accgttcaat tagcaggcac cagcggtaag cttgatgcat ttttagcatc gattcgcgat 420

gtggcgaaaa ttgtggaggt tgctcgctct ggtgtggtcg gactttcgcg cggcgataaa 480

ataatgcgtt ga 492

<210> 12
<211> 163
<212> PRT
<213> Escherichia coli

<400> 12

Met Arg Arg Ile Leu Ser Val Leu Leu Glu Asn Glu Ser Gly Ala Leu

1 5 10 15

Ser Arg Val Ile Gly Leu Phe Ser Gln Arg Gly Tyr Asn Ile Glu Ser
20 25 30

Leu Thr Val Ala Pro Thr Asp Asp Pro Thr Leu Ser Arg Met Thr Ile
35 40 45

Gln Thr Val Gly Asp Glu Lys Val Leu Glu Gln Ile Glu Lys Gln Leu
50 55 60

His Lys Leu Val Asp Val Leu Arg Val Ser Glu Leu Gly Gln Gly Ala
65 70 75 80

His Val Glu Arg Glu Ile Met Leu Val Lys Ile Gln Ala Ser Gly Tyr
85 90 95

Gly Arg Asp Glu Val Lys Arg Asn Thr Glu Ile Phe Arg Gly Gln Ile
100 105 110

Ile Asp Val Thr Pro Ser Leu Tyr Thr Val Gln Leu Ala Gly Thr Ser
115 120 125

Gly Lys Leu Asp Ala Phe Leu Ala Ser Ile Arg Asp Val Ala Lys Ile
130 135 140

Val Glu Val Ala Arg Ser Gly Val Val Gly Leu Ser Arg Gly Asp Lys
145 150 155 160

Ile Met Arg

<210> 13
<211> 1161
<212> DNA
<213> Escherichia coli

<400> 13
atgacatcgg aaaacccgtt actggcgctg cgagagaaaa tcagcgcgct ggatgaaaaa 60

ttattagcgt tactggcaga acggcgcgaa ctggccgtcg aggtgggaaa agccaaactg 120

ctctcgcatc gcccggtacg tgatattgat cgtgaacgcg atttgctgga aagattaatt 180

acgctcggta aagcgcacca tctggacgcc cattacatta ctcgcctgtt ccagctcatc 240

attgaagatt ccgtattaac tcagcaggct ttgctccaac aacatctcaa taaaattaat 300

ccgcactcag cacgcatcgc ttttctcggc cccaaaggtt cttattccca tcttgcggcg 360

cgccagtatg ctgcccgtca ctttgagcaa ttcattgaaa gtggctgcgc caaatttgcc 420

gatattttta atcaggtgga aaccggccag gccgactatg ccgtcgtacc gattgaaaat 480

accagctccg gtgccataaa cgacgtttac gatctgctgc aacataccag cttgtcgatt 540

gttggcgaga tgacgttaac tatcgaccat tgtttgttgg tctccggcac tactgattta 600

tccaccatca atacggtcta cagccatccg cagccattcc agcaatgcag caaattcctt 660

aatcgttatc cgcactggaa gattgaatat accgaaagta cgtctgcggc aatggaaaag 720

gttgcacagg caaaatcacc gcatgttgct gcgttgggaa gcgaagctgg cggcactttg 780

tacggtttgc aggtactgga gcgtattgaa gcaaatcagc gacaaaactt cacccgattt 840

gtggtgttgg cgcgtaaagc cattaacgtg tctgatcagg ttccggcgaa aaccacgttg 900

ttaatggcga ccgggcaaca agccggtgcg ctggttgaag cgttgctggt actgcgcaac 960

cacaatctga ttatgacccg tctggaatca cgcccgattc acggtaatcc atgggaagag 1020

atgttctatc tggatattca ggccaatctt gaatcagcgg aaatgcaaaa agcattgaaa 1080

gagttagggg aaatcacccg ttcaatgaag gtattgggct gttacccaag tgagaacgta 1140

gtgcctgttg atccaacctg a 1161

<210> 14
<211> 386
<212> PRT
<213> Escherichia coli

<400> 14

Met Thr Ser Glu Asn Pro Leu Leu Ala Leu Arg Glu Lys Ile Ser Ala
1 5 10 15

Leu Asp Glu Lys Leu Leu Ala Leu Leu Ala Glu Arg Arg Glu Leu Ala
20 25 30

Val Glu Val Gly Lys Ala Lys Leu Leu Ser His Arg Pro Val Arg Asp
35 40 45

Ile Asp Arg Glu Arg Asp Leu Leu Glu Arg Leu Ile Thr Leu Gly Lys
50 55 60

Ala His His Leu Asp Ala His Tyr Ile Thr Arg Leu Phe Gln Leu Ile
65 70 75 80

Ile Glu Asp Ser Val Leu Thr Gln Gln Ala Leu Leu Gln Gln His Leu
85 90 95

Asn Lys Ile Asn Pro His Ser Ala Arg Ile Ala Phe Leu Gly Pro Lys
100 105 110

Gly Ser Tyr Ser His Leu Ala Ala Arg Gln Tyr Ala Ala Arg His Phe

115 120 125

Glu Gln Phe Ile Glu Ser Gly Cys Ala Lys Phe Ala Asp Ile Phe Asn
130 135 140

Gln Val Glu Thr Gly Gln Ala Asp Tyr Ala Val Val Pro Ile Glu Asn
145 150 155 160

Thr Ser Ser Gly Ala Ile Asn Asp Val Tyr Asp Leu Leu Gln His Thr
165 170 175

Ser Leu Ser Ile Val Gly Glu Met Thr Leu Thr Ile Asp His Cys Leu
180 185 190

Leu Val Ser Gly Thr Thr Asp Leu Ser Thr Ile Asn Thr Val Tyr Ser
195 200 205

His Pro Gln Pro Phe Gln Gln Cys Ser Lys Phe Leu Asn Arg Tyr Pro
210 215 220

His Trp Lys Ile Glu Tyr Thr Glu Ser Thr Ser Ala Ala Met Glu Lys
225 230 235 240

Val Ala Gln Ala Lys Ser Pro His Val Ala Ala Leu Gly Ser Glu Ala
245 250 255

Gly Gly Thr Leu Tyr Gly Leu Gln Val Leu Glu Arg Ile Glu Ala Asn
260 265 270

Gln Arg Gln Asn Phe Thr Arg Phe Val Val Leu Ala Arg Lys Ala Ile
275 280 285

Asn Val Ser Asp Gln Val Pro Ala Lys Thr Thr Leu Leu Met Ala Thr
290 295 300

Gly Gln Gln Ala Gly Ala Leu Val Glu Ala Leu Leu Val Leu Arg Asn
305 310 315 320

His Asn Leu Ile Met Thr Arg Leu Glu Ser Arg Pro Ile His Gly Asn
325 330 335

Pro Trp Glu Glu Met Phe Tyr Leu Asp Ile Gln Ala Asn Leu Glu Ser
340 345 350

Ala Glu Met Gln Lys Ala Leu Lys Glu Leu Gly Glu Ile Thr Arg Ser
355 360 365

Met Lys Val Leu Gly Cys Tyr Pro Ser Glu Asn Val Val Pro Val Asp
    370                 375                 380

Pro Thr
385

<210> 15
<211> 318
<212> DNA
<213> Escherichia coli

<400> 15
atggctgaat ggagcggcga atatatcagc ccatacgctg agcacggcaa gaagagtgaa 60

caagtcaaaa agattacggt ttccattcct cttaaggtgt taaaaatcct caccgatgaa 120

cgcacgcgtc gtcaggtgaa caacctgcgt cacgctacca acagcgagct gctgtgcgaa 180

gcgtttctgc atgcctttac cgggcaacct ttgccggatg atgccgatct gcgtaaagag 240

cgcagcgacg aaatcccgga agcggcaaaa gagatcatgc gtgagatggg gattaacccg 300

gagacgtggg aatactaa 318

<210> 16
<211> 105
<212> PRT
<213> Escherichia coli

<400> 16

Met Ala Glu Trp Ser Gly Glu Tyr Ile Ser Pro Tyr Ala Glu His Gly
1               5                   10                  15

Lys Lys Ser Glu Gln Val Lys Lys Ile Thr Val Ser Ile Pro Leu Lys
            20                  25                  30

Val Leu Lys Ile Leu Thr Asp Glu Arg Thr Arg Arg Gln Val Asn Asn
        35                  40                  45

Leu Arg His Ala Thr Asn Ser Glu Leu Leu Cys Glu Ala Phe Leu His
    50                  55                  60

Ala Phe Thr Gly Gln Pro Leu Pro Asp Asp Ala Asp Leu Arg Lys Glu
65                  70                  75                  80

Arg Ser Asp Glu Ile Pro Glu Ala Ala Lys Glu Ile Met Arg Glu Met
                85                  90                  95

Gly Ile Asn Pro Glu Thr Trp Glu Tyr
            100                 105

<210> 17

<211> 930
<212> DNA
<213> Escherichia coli

<400> 17
atgccgattc gtgtgccgga cgagctaccc gccgtcaatt tcttgcgtga agaaaacgtc 60

tttgtgatga caacttctcg tgcgtctggt caggaaattc gtccacttaa ggttctgatc 120

cttaacctga tgccgaagaa gattgaaact gaaaatcagt ttctgcgcct gctttcaaac 180

tcacctttgc aggtcgatat tcagctgttg cgcatcgatt cccgtgaatc gcgcaacacg 240

cccgcagagc atctgaacaa cttctactgt aactttgaag atattcagga tcagaacttt 300

gacggtttga ttgtaactgg tgcgccgctg ggcctggtgg agtttaatga tgtcgcttac 360

tggccgcaga tcaaacaggt gctggagtgg tcgaaagatc acgtcacctc gacgctgttt 420

gtctgctggg cggtacaggc cgcgctcaat atcctctacg gcattcctaa gcaaactcgc 480

accgaaaaac tctctggcgt ttacgagcat catattctcc atcctcatgc gcttctgacg 540

cgtggctttg atgattcatt cctggcaccg cattcgcgct atgctgactt tccggcagcg 600

ttgattcgtg attacaccga tctggaaatt ctggcagaga cggaagaagg ggatgcatat 660

ctgtttgcca gtaaagataa gcgcattgcc tttgtgacgg gccatcccga atatgatgcg 720

caaacgctgg cgcaggaatt tttccgcgat gtggaagccg gactagaccc ggatgtaccg 780

tataactatt tcccgcacaa tgatccgcaa aatacaccgc gagcgagctg gcgtagtcac 840

ggtaatttac tgtttaccaa ctggctcaac tattacgtct accagatcac gccatacgat 900

ctacggcaca tgaatccaac gctggattaa 930

<210> 18
<211> 309
<212> PRT
<213> Escherichia coli

<400> 18

Met Pro Ile Arg Val Pro Asp Glu Leu Pro Ala Val Asn Phe Leu Arg
1 5 10 15

Glu Glu Asn Val Phe Val Met Thr Thr Ser Arg Ala Ser Gly Gln Glu
20 25 30

Ile Arg Pro Leu Lys Val Leu Ile Leu Asn Leu Met Pro Lys Lys Ile
35 40 45

Glu Thr Glu Asn Gln Phe Leu Arg Leu Leu Ser Asn Ser Pro Leu Gln
50 55 60

Val Asp Ile Gln Leu Leu Arg Ile Asp Ser Arg Glu Ser Arg Asn Thr
65 70 75 80

```
Pro Ala Glu His Leu Asn Asn Phe Tyr Cys Asn Phe Glu Asp Ile Gln
                85                  90                  95

Asp Gln Asn Phe Asp Gly Leu Ile Val Thr Gly Ala Pro Leu Gly Leu
            100                 105                 110

Val Glu Phe Asn Asp Val Ala Tyr Trp Pro Gln Ile Lys Gln Val Leu
        115                 120                 125

Glu Trp Ser Lys Asp His Val Thr Ser Thr Leu Phe Val Cys Trp Ala
    130                 135                 140

Val Gln Ala Ala Leu Asn Ile Leu Tyr Gly Ile Pro Lys Gln Thr Arg
145                 150                 155                 160

Thr Glu Lys Leu Ser Gly Val Tyr Glu His His Ile Leu His Pro His
                165                 170                 175

Ala Leu Leu Thr Arg Gly Phe Asp Asp Ser Phe Leu Ala Pro His Ser
            180                 185                 190

Arg Tyr Ala Asp Phe Pro Ala Ala Leu Ile Arg Asp Tyr Thr Asp Leu
        195                 200                 205

Glu Ile Leu Ala Glu Thr Glu Glu Gly Asp Ala Tyr Leu Phe Ala Ser
    210                 215                 220

Lys Asp Lys Arg Ile Ala Phe Val Thr Gly His Pro Glu Tyr Asp Ala
225                 230                 235                 240

Gln Thr Leu Ala Gln Glu Phe Phe Arg Asp Val Glu Ala Gly Leu Asp
                245                 250                 255

Pro Asp Val Pro Tyr Asn Tyr Phe Pro His Asn Asp Pro Gln Asn Thr
            260                 265                 270

Pro Arg Ala Ser Trp Arg Ser His Gly Asn Leu Leu Phe Thr Asn Trp
        275                 280                 285

Leu Asn Tyr Tyr Val Tyr Gln Ile Thr Pro Tyr Asp Leu Arg His Met
    290                 295                 300

Asn Pro Thr Leu Asp
305

<210>  19
```

<211> 930
<212> DNA
<213> Escherichia coli

<400> 19

```
atgccgattc gtgtgccgga cgagctaccc gccgtcaatt tcttgcgtga agaaaacgtc      60
tttgtgatga caacttctcg tgcgtctggt caggaaattc gtccacttaa ggttctgatc     120
cttaacctga tgccgaagaa gattgaaact gaaaatcagt ttctgcgcct gctttcaaac     180
tcacctttgg aggtcgatat tcagctgttg cgcatcgatt cccgtgaatc gcgcaacacg     240
cccgcagagc atctgaacaa cttctactgt aactttgaag atattcagga tcagaacttt     300
gacggtttga ttgtaactgg tgcgccgctg ggcctggtgg agtttaatga tgtcgcttac     360
tggccgcaga tcaaacaggt gctggagtgg tcgaaagatc acgtcacctc gacgctgttt     420
gtctgctggg cggtacaggc cgcgctcaat atcctctacg gcattcctaa gcaaactcgc     480
accgaaaaac tctctggcgt ttacgagcat catattctcc atcctcatgc gcttctgacg     540
cgtggctttg atgattcatt cctggcaccg cattcgcgct atgctgactt tccggcagcg     600
ttgattcgtg attacaccga tctggaaatt ctggcagaga cggaagaagg ggatgcatat     660
ctgtttgcca gtaaagataa gcgcattgcc tttgtgacgg gccatcccga atatgatgcg     720
caaacgctgg cgcaggaatt tttccgcgat gtggaagccg gactagaccc ggatgtaccg     780
tataactatt tcccgcacaa tgatccgcaa aatacaccgc gagcgagctg gcgtagtcac     840
ggtaatttac tgtttaccaa ctggctcaac tattacgtct accagatcac gccatacgat     900
ctacggcaca tgaatccaac gctggattaa                                     930
```

<210> 20
<211> 309
<212> PRT
<213> Escherichia coli

<400> 20

```
Met Pro Ile Arg Val Pro Asp Glu Leu Pro Ala Val Asn Phe Leu Arg
1               5                   10                  15

Glu Glu Asn Val Phe Val Met Thr Thr Ser Arg Ala Ser Gly Gln Glu
            20                  25                  30

Ile Arg Pro Leu Lys Val Leu Ile Leu Asn Leu Met Pro Lys Lys Ile
        35                  40                  45

Glu Thr Glu Asn Gln Phe Leu Arg Leu Leu Ser Asn Ser Pro Leu Glu
    50                  55                  60

Val Asp Ile Gln Leu Leu Arg Ile Asp Ser Arg Glu Ser Arg Asn Thr
65                  70                  75                  80
```

```
Pro Ala Glu His Leu Asn Asn Phe Tyr Cys Asn Phe Glu Asp Ile Gln
                85                  90                  95

Asp Gln Asn Phe Asp Gly Leu Ile Val Thr Gly Ala Pro Leu Gly Leu
            100                 105                 110

Val Glu Phe Asn Asp Val Ala Tyr Trp Pro Gln Ile Lys Gln Val Leu
        115                 120                 125

Glu Trp Ser Lys Asp His Val Thr Ser Thr Leu Phe Val Cys Trp Ala
    130                 135                 140

Val Gln Ala Ala Leu Asn Ile Leu Tyr Gly Ile Pro Lys Gln Thr Arg
145                 150                 155                 160

Thr Glu Lys Leu Ser Gly Val Tyr Glu His His Ile Leu His Pro His
                165                 170                 175

Ala Leu Leu Thr Arg Gly Phe Asp Asp Ser Phe Leu Ala Pro His Ser
            180                 185                 190

Arg Tyr Ala Asp Phe Pro Ala Ala Leu Ile Arg Asp Tyr Thr Asp Leu
        195                 200                 205

Glu Ile Leu Ala Glu Thr Glu Glu Gly Asp Ala Tyr Leu Phe Ala Ser
    210                 215                 220

Lys Asp Lys Arg Ile Ala Phe Val Thr Gly His Pro Glu Tyr Asp Ala
225                 230                 235                 240

Gln Thr Leu Ala Gln Glu Phe Phe Arg Asp Val Glu Ala Gly Leu Asp
                245                 250                 255

Pro Asp Val Pro Tyr Asn Tyr Phe Pro His Asn Asp Pro Gln Asn Thr
            260                 265                 270

Pro Arg Ala Ser Trp Arg Ser His Gly Asn Leu Leu Phe Thr Asn Trp
        275                 280                 285

Leu Asn Tyr Tyr Val Tyr Gln Ile Thr Pro Tyr Asp Leu Arg His Met
    290                 295                 300

Asn Pro Thr Leu Asp
305
```

<210> 21

<211> 2463
<212> DNA
<213> Escherichia coli

<400> 21
atgcgagtgt tgaagttcgg cggtacatca gtggcaaatg cagaacgttt tctgcgtgtt 60

gccgatattc tggaaagcaa tgccaggcag ggcaggtgg ccaccgtcct ctctgccccc 120

gccaaaatca ccaaccacct ggtggcgatg attgaaaaaa ccattagcgg ccaggatgct 180

ttacccaata tcagcgatgc cgaacgtatt tttgccgaac ttttgacggg actcgccgcc 240

gcccagccgg ggttcccgct ggcgcaattg aaaactttcg tcgatcagga atttgcccaa 300

ataaaacatg tcctgcatgg cattagtttg ttggggcagt gcccggatag catcaacgct 360

gcgctgattt gccgtggcga gaaaatgtcg atcgccatta tggccggcgt attagaagcg 420

cgcggtcaca acgttactgt tatcgatccg gtcgaaaaac tgctggcagt ggggcattac 480

ctcgaatcta ccgtcgatat tgctgagtcc acccgccgta ttgcggcaag ccgcattccg 540

gctgatcaca tggtgctgat ggcaggtttc accgccggta atgaaaaagg cgaactggtg 600

gtgcttggac gcaacggttc cgactactct gctgcggtgc tggctgcctg tttacgcgcc 660

gattgttgcg agatttggac ggacgttgac ggggtctata cctgcgaccc gcgtcaggtg 720

cccgatgcga ggttgttgaa gtcgatgtcc taccaggaag cgatggagct ttcctacttc 780

ggcgctaaag ttcttcaccc ccgcaccatt acccccatcg cccagttcca gatcccttgc 840

ctgattaaaa ataccggaaa tcctcaagca ccaggtacgc tcattggtgc cagccgtgat 900

gaagacgaat taccggtcaa gggcatttcc aatctgaata acatggcaat gttcagcgtt 960

tctggtccgg ggatgaaagg gatggtcggc atggcggcgc gcgtctttgc agcgatgtca 1020

cgcgcccgta tttccgtggt gctgattacg caatcatctt ccgaatacag catcagtttc 1080

tgcgttccac aaagcgactg tgtgcgagct gaacgggcaa tgcaggaaga gttctacctg 1140

gaactgaaag aaggcttact ggagccgctg gcagtgacgg aacggctggc cattatctcg 1200

gtggtaggtg atggtatgcg caccttgcgt gggatctcgg cgaaattctt tgccgcactg 1260

gcccgcgcca atatcaacat tgtcgccatt gctcagggat cttctgaacg ctcaatctct 1320

gtcgtggtaa ataacgatga tgcgaccact ggcgtgcgcg ttactcatca gatgctgttc 1380

aataccgatc aggttatcga agtgtttgtg attggcgtcg gtggcgttgg cggtgcgctg 1440

ctggagcaac tgaagcgtca gcaaagctgg ctgaagaata aacatatcga cttacgtgtc 1500

tgcggtgttg ccaactcgaa ggctctgctc accaatgtac atggccttaa tctggaaaac 1560

tggcaggaag aactggcgca agccaaagag ccgtttaatc tcgggcgctt aattcgcctc 1620

gtgaaagaat atcatctgct gaacccggtc attgttgact gcacttccag ccaggcagtg 1680

gcggatcaat atgccgactt cctgcgcgaa ggtttccacg ttgtcacgcc gaacaaaaag 1740

gccaacacct cgtcgatgga ttactaccat cagttgcgtt atgcggcgga aaaatcgcgg 1800

cgtaaattcc tctatgacac caacgttggg gctggattac cggttattga gaacctgcaa 1860

aatctgctca atgcaggtga tgaattgatg aagttctccg gcattctttc tggttcgctt 1920

tcttatatct tcggcaagtt agacgaaggc atgagtttct ccgaggcgac cacgctggcg 1980

cgggaaatgg gttataccga accggacccg cgagatgatc tttctggtat ggatgtggcg 2040

cgtaaactat tgattctcgc tcgtgaaacg ggacgtgaac tggagctggc ggatattgaa 2100

attgaacctg tgctgcccgc agagtttaac gccgagggtg atgttgccgc ttttatggcg 2160

aatctgtcac aactcgacga tctctttgcc gcgcgcgtgg cgaaggcccg tgatgaagga 2220

aaagttttgc gctatgttgg caatattgat gaagatggcg tctgccgcgt gaagattgcc 2280

gaagtggatg gtaatgatcc gctgttcaaa gtgaaaaatg gcgaaaacgc cctggccttc 2340

tatagccact attatcagcc gctgccgttg gtactgcgcg gatatggtgc gggcaatgac 2400

gttacagctg ccggtgtctt tgctgatctg ctacgtaccc tctcatggaa gttaggagtc 2460

tga 2463

<210> 22
<211> 820
<212> PRT
<213> Escherichia coli

<400> 22

Met Arg Val Leu Lys Phe Gly Gly Thr Ser Val Ala Asn Ala Glu Arg
1 5 10 15

Phe Leu Arg Val Ala Asp Ile Leu Glu Ser Asn Ala Arg Gln Gly Gln
20 25 30

Val Ala Thr Val Leu Ser Ala Pro Ala Lys Ile Thr Asn His Leu Val
35 40 45

Ala Met Ile Glu Lys Thr Ile Ser Gly Gln Asp Ala Leu Pro Asn Ile
50 55 60

Ser Asp Ala Glu Arg Ile Phe Ala Glu Leu Leu Thr Gly Leu Ala Ala
65 70 75 80

Ala Gln Pro Gly Phe Pro Leu Ala Gln Leu Lys Thr Phe Val Asp Gln
85 90 95

Glu Phe Ala Gln Ile Lys His Val Leu His Gly Ile Ser Leu Leu Gly
100 105 110

Gln Cys Pro Asp Ser Ile Asn Ala Ala Leu Ile Cys Arg Gly Glu Lys

```
        115                 120                 125

Met Ser Ile Ala Ile Met Ala Gly Val Leu Glu Ala Arg Gly His Asn
    130                 135                 140

Val Thr Val Ile Asp Pro Val Glu Lys Leu Leu Ala Val Gly His Tyr
145                 150                 155                 160

Leu Glu Ser Thr Val Asp Ile Ala Glu Ser Thr Arg Arg Ile Ala Ala
                165                 170                 175

Ser Arg Ile Pro Ala Asp His Met Val Leu Met Ala Gly Phe Thr Ala
            180                 185                 190

Gly Asn Glu Lys Gly Glu Leu Val Val Leu Gly Arg Asn Gly Ser Asp
        195                 200                 205

Tyr Ser Ala Ala Val Leu Ala Ala Cys Leu Arg Ala Asp Cys Cys Glu
    210                 215                 220

Ile Trp Thr Asp Val Asp Gly Val Tyr Thr Cys Asp Pro Arg Gln Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Gly Asn Pro
        275                 280                 285

Gln Ala Pro Gly Thr Leu Ile Gly Ala Ser Arg Asp Glu Asp Glu Leu
    290                 295                 300

Pro Val Lys Gly Ile Ser Asn Leu Asn Asn Met Ala Met Phe Ser Val
305                 310                 315                 320

Ser Gly Pro Gly Met Lys Gly Met Val Gly Met Ala Ala Arg Val Phe
                325                 330                 335

Ala Ala Met Ser Arg Ala Arg Ile Ser Val Val Leu Ile Thr Gln Ser
            340                 345                 350

Ser Ser Glu Tyr Ser Ile Ser Phe Cys Val Pro Gln Ser Asp Cys Val
        355                 360                 365
```

Arg Ala Glu Arg Ala Met Gln Glu Glu Phe Tyr Leu Glu Leu Lys Glu
    370                 375                 380

Gly Leu Leu Glu Pro Leu Ala Val Thr Glu Arg Leu Ala Ile Ile Ser
385                 390                 395                 400

Val Val Gly Asp Gly Met Arg Thr Leu Arg Gly Ile Ser Ala Lys Phe
                405                 410                 415

Phe Ala Ala Leu Ala Arg Ala Asn Ile Asn Ile Val Ala Ile Ala Gln
            420                 425                 430

Gly Ser Ser Glu Arg Ser Ile Ser Val Val Val Asn Asn Asp Asp Ala
        435                 440                 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625 630 635 640

Ser Tyr Ile Phe Gly Lys Leu Asp Glu Gly Met Ser Phe Ser Glu Ala
645 650 655

Thr Thr Leu Ala Arg Glu Met Gly Tyr Thr Glu Pro Asp Pro Arg Asp
660 665 670

Asp Leu Ser Gly Met Asp Val Ala Arg Lys Leu Leu Ile Leu Ala Arg
675 680 685

Glu Thr Gly Arg Glu Leu Glu Leu Ala Asp Ile Glu Ile Glu Pro Val
690 695 700

Leu Pro Ala Glu Phe Asn Ala Glu Gly Asp Val Ala Ala Phe Met Ala
705 710 715 720

Asn Leu Ser Gln Leu Asp Asp Leu Phe Ala Ala Arg Val Ala Lys Ala
725 730 735

Arg Asp Glu Gly Lys Val Leu Arg Tyr Val Gly Asn Ile Asp Glu Asp
740 745 750

Gly Val Cys Arg Val Lys Ile Ala Glu Val Asp Gly Asn Asp Pro Leu
755 760 765

Phe Lys Val Lys Asn Gly Glu Asn Ala Leu Ala Phe Tyr Ser His Tyr
770 775 780

Tyr Gln Pro Leu Pro Leu Val Leu Arg Gly Tyr Gly Ala Gly Asn Asp
785 790 795 800

Val Thr Ala Ala Gly Val Phe Ala Asp Leu Leu Arg Thr Leu Ser Trp
805 810 815

Lys Leu Gly Val
820

<210> 23
<211> 2463
<212> DNA
<213> Escherichia coli

<400> 23
atgagagtgt tgaagttcgg cggtacatca gtggcaaatg cagaacgttt tctgcgtgtt 60

gccgatattc tggaaagcaa tgccaggcag ggcaggtgg ccaccgtcct ctctgccccc 120

gccaaaatca ccaaccacct ggtggcgatg attgaaaaaa ccattagcgg ccaggatgct 180

ttacccaata tcagcgatgc cgaacgtatt tttgccgaac ttttgacggg actcgccgcc 240

gcccagccgg ggttcccgct ggcgcaattg aaaactttcg tcgatcagga atttgcccaa 300

ataaaacatg tcctgcatgg cattagtttg ttggggcagt gcccggatag catcaacgct 360

gcgctgattt gccgtggcga gaaaatgtcg atcgccatta tggccggcgt attagaagcg 420

cgcggtcaca acgttactgt tatcgatccg gtcgaaaaac tgctggcagt ggggcattac 480

ctcgaatcta ccgtcgatat tgctgagtcc acccgccgta ttgcggcaag ccgcattccg 540

gctgatcaca tggtgctgat ggcaggtttc accgccggta atgaaaaagg cgaactggtg 600

gtgcttggac gcaacggttc cgactactct gctgcggtgc tggctgcctg tttacgcgcc 660

gattgttgcg agatttggac ggacgttgac ggggtctata cctgcgaccc gcgtcaggtg 720

cccgatgcga ggttgttgaa gtcgatgtcc taccaggaag cgatggagct ttcctacttc 780

ggcgctaaag ttcttcaccc ccgcaccatt acccccatcg cccagttcca gatcccttgc 840

ctgattaaaa ataccggaaa tcctcaagca ccaggtacgc tcattggtgc cagccgtgat 900

gaagacgaat taccggtcaa gggcatttcc aatctgaata acatggcaat gtccagcgtt 960

tctggtccgg ggatgaaagg gatggtcggc atggcggcgc gcgtctttgc agcgatgtca 1020

cgcgcccgta tttccgtggt gctgattacg caatcatctt ccgaatacag catcagtttc 1080

tgcgttccac aaagcgactg tgtgcgagct gaacgggcaa tgcaggaaga gttctacctg 1140

gaactgaaag aaggcttact ggagccgctg gcagtgacgg aacggctggc cattatctcg 1200

gtggtaggtg atggtatgcg caccttgcgt gggatctcgg cgaaattctt tgccgcactg 1260

gcccgcgcca atatcaacat tgtcgccatt gctcagggat cttctgaacg ctcaatctct 1320

gtcgtggtaa ataacgatga tgcgaccact ggcgtgcgcg ttactcatca gatgctgttc 1380

aataccgatc aggttatcga agtgtttgtg attggcgtcg gtggcgttgg cggtgcgctg 1440

ctggagcaac tgaagcgtca gcaaagctgg ctgaagaata aacatatcga cttacgtgtc 1500

tgcggtgttg ccaactcgaa ggctctgctc accaatgtac atggccttaa tctggaaaac 1560

tggcaggaag aactggcgca agccaaagag ccgtttaatc tcgggcgctt aattcgcctc 1620

gtgaaagaat atcatctgct gaacccggtc attgttgact gcacttccag ccaggcagtg 1680

gcggatcaat atgccgactt cctgcgcgaa ggtttccacg ttgtcacgcc gaacaaaaag 1740

gccaacacct cgtcgatgga ttactaccat cagttgcgtt atgcggcgga aaaatcgcgg 1800

cgtaaattcc tctatgacac caacgttggg gctggattac cggttattga gaacctgcaa 1860

aatctgctca atgcaggtga tgaattgatg aagttctccg gcattctttc tggttcgctt 1920

tcttatatct tcggcaagtt agacgaaggc atgagtttct ccgaggcgac cacgctggcg 1980

cgggaaatgg gttataccga accggacccg cgagatgatc tttctggtat ggatgtggcg 2040

cgtaaactat tgattctcgc tcgtgaaacg ggacgtgaac tggagctggc ggatattgaa 2100

attgaacctg tgctgcccgc agagtttaac gccgagggtg atgttgccgc ttttatggcg 2160

aatctgtcac aactcgacga tctctttgcc gcgcgcgtgg cgaaggcccg tgatgaagga 2220

aaagttttgc gctatgttgg caatattgat gaagatggcg tctgccgcgt gaagattgcc 2280

gaagtggatg gtaatgatcc gctgttcaaa gtgaaaaatg gcgaaaacgc cctggccttc 2340

tatagccact attatcagcc gctgccgttg gtactgcgcg gatatggtgc gggcaatgac 2400

gttacagctg ccggtgtctt tgctgatctg ctacgtaccc tctcatggaa gttaggagtc 2460

tga 2463

<210> 24
<211> 820
<212> PRT
<213> Escherichia coli

<400> 24

Met Arg Val Leu Lys Phe Gly Gly Thr Ser Val Ala Asn Ala Glu Arg
1 5 10 15

Phe Leu Arg Val Ala Asp Ile Leu Glu Ser Asn Ala Arg Gln Gly Gln
20 25 30

Val Ala Thr Val Leu Ser Ala Pro Ala Lys Ile Thr Asn His Leu Val
35 40 45

Ala Met Ile Glu Lys Thr Ile Ser Gly Gln Asp Ala Leu Pro Asn Ile
50 55 60

Ser Asp Ala Glu Arg Ile Phe Ala Glu Leu Leu Thr Gly Leu Ala Ala
65 70 75 80

Ala Gln Pro Gly Phe Pro Leu Ala Gln Leu Lys Thr Phe Val Asp Gln
85 90 95

Glu Phe Ala Gln Ile Lys His Val Leu His Gly Ile Ser Leu Leu Gly
100 105 110

Gln Cys Pro Asp Ser Ile Asn Ala Ala Leu Ile Cys Arg Gly Glu Lys
115 120 125

Met Ser Ile Ala Ile Met Ala Gly Val Leu Glu Ala Arg Gly His Asn
130 135 140

Val Thr Val Ile Asp Pro Val Glu Lys Leu Leu Ala Val Gly His Tyr
145 150 155 160

Leu Glu Ser Thr Val Asp Ile Ala Glu Ser Thr Arg Arg Ile Ala Ala
165 170 175

Ser Arg Ile Pro Ala Asp His Met Val Leu Met Ala Gly Phe Thr Ala
180 185 190

Gly Asn Glu Lys Gly Glu Leu Val Val Leu Gly Arg Asn Gly Ser Asp
195 200 205

Tyr Ser Ala Ala Val Leu Ala Ala Cys Leu Arg Ala Asp Cys Cys Glu
210 215 220

Ile Trp Thr Asp Val Asp Gly Val Tyr Thr Cys Asp Pro Arg Gln Val
225 230 235 240

Pro Asp Ala Arg Leu Leu Lys Ser Met Ser Tyr Gln Glu Ala Met Glu
245 250 255

Leu Ser Tyr Phe Gly Ala Lys Val Leu His Pro Arg Thr Ile Thr Pro
260 265 270

Ile Ala Gln Phe Gln Ile Pro Cys Leu Ile Lys Asn Thr Gly Asn Pro
275 280 285

Gln Ala Pro Gly Thr Leu Ile Gly Ala Ser Arg Asp Glu Asp Glu Leu
290 295 300

Pro Val Lys Gly Ile Ser Asn Leu Asn Asn Met Ala Met Ser Ser Val
305 310 315 320

Ser Gly Pro Gly Met Lys Gly Met Val Gly Met Ala Ala Arg Val Phe
325 330 335

Ala Ala Met Ser Arg Ala Arg Ile Ser Val Val Leu Ile Thr Gln Ser
340 345 350

Ser Ser Glu Tyr Ser Ile Ser Phe Cys Val Pro Gln Ser Asp Cys Val
355 360 365

Arg Ala Glu Arg Ala Met Gln Glu Glu Phe Tyr Leu Glu Leu Lys Glu
370 375 380

Gly Leu Leu Glu Pro Leu Ala Val Thr Glu Arg Leu Ala Ile Ile Ser
385 390 395 400

Val Val Gly Asp Gly Met Arg Thr Leu Arg Gly Ile Ser Ala Lys Phe

405 410 415

Phe Ala Ala Leu Ala Arg Ala Asn Ile Asn Ile Val Ala Ile Ala Gln
420 425 430

Gly Ser Ser Glu Arg Ser Ile Ser Val Val Val Asn Asn Asp Asp Ala
435 440 445

Thr Thr Gly Val Arg Val Thr His Gln Met Leu Phe Asn Thr Asp Gln
450 455 460

Val Ile Glu Val Phe Val Ile Gly Val Gly Gly Val Gly Gly Ala Leu
465 470 475 480

Leu Glu Gln Leu Lys Arg Gln Gln Ser Trp Leu Lys Asn Lys His Ile
485 490 495

Asp Leu Arg Val Cys Gly Val Ala Asn Ser Lys Ala Leu Leu Thr Asn
500 505 510

Val His Gly Leu Asn Leu Glu Asn Trp Gln Glu Glu Leu Ala Gln Ala
515 520 525

Lys Glu Pro Phe Asn Leu Gly Arg Leu Ile Arg Leu Val Lys Glu Tyr
530 535 540

His Leu Leu Asn Pro Val Ile Val Asp Cys Thr Ser Ser Gln Ala Val
545 550 555 560

Ala Asp Gln Tyr Ala Asp Phe Leu Arg Glu Gly Phe His Val Val Thr
565 570 575

Pro Asn Lys Lys Ala Asn Thr Ser Ser Met Asp Tyr Tyr His Gln Leu
580 585 590

Arg Tyr Ala Ala Glu Lys Ser Arg Arg Lys Phe Leu Tyr Asp Thr Asn
595 600 605

Val Gly Ala Gly Leu Pro Val Ile Glu Asn Leu Gln Asn Leu Leu Asn
610 615 620

Ala Gly Asp Glu Leu Met Lys Phe Ser Gly Ile Leu Ser Gly Ser Leu
625 630 635 640

Ser Tyr Ile Phe Gly Lys Leu Asp Glu Gly Met Ser Phe Ser Glu Ala
645 650 655

Thr Thr Leu Ala Arg Glu Met Gly Tyr Thr Glu Pro Asp Pro Arg Asp
660 665 670

Asp Leu Ser Gly Met Asp Val Ala Arg Lys Leu Leu Ile Leu Ala Arg
675 680 685

Glu Thr Gly Arg Glu Leu Glu Leu Ala Asp Ile Glu Ile Glu Pro Val
690 695 700

Leu Pro Ala Glu Phe Asn Ala Glu Gly Asp Val Ala Ala Phe Met Ala
705 710 715 720

Asn Leu Ser Gln Leu Asp Asp Leu Phe Ala Ala Arg Val Ala Lys Ala
725 730 735

Arg Asp Glu Gly Lys Val Leu Arg Tyr Val Gly Asn Ile Asp Glu Asp
740 745 750

Gly Val Cys Arg Val Lys Ile Ala Glu Val Asp Gly Asn Asp Pro Leu
755 760 765

Phe Lys Val Lys Asn Gly Glu Asn Ala Leu Ala Phe Tyr Ser His Tyr
770 775 780

Tyr Gln Pro Leu Pro Leu Val Leu Arg Gly Tyr Gly Ala Gly Asn Asp
785 790 795 800

Val Thr Ala Ala Gly Val Phe Ala Asp Leu Leu Arg Thr Leu Ser Trp
805 810 815

Lys Leu Gly Val
820

<210> 25
<211> 3684
<212> DNA
<213> Escherichia coli

<400> 25
gtgagcagca aagtggaaca actgcgtgcg cagttaaatg aacgtattct ggtgctggac 60

ggcggtatgg gcaccatgat ccagagttat cgactgaacg aagccgattt tcgtggtgaa 120

cgctttgccg actggccatg cgacctcaaa ggcaacaacg acctgctggt actcagtaaa 180

ccggaagtga tcgccgctat ccacaacgcc tactttgaag cgggcgcgga tatcatcgaa 240

accaacacct tcaactccac gaccattgcg atggcggatt accagatgga atccctgtcg 300

gcggaaatca actttgcggc ggcgaaactg gcgcgagctt gtgctgacga gtggaccgcg 360

cgcacgccag agaaaccgcg ctacgttgcc ggtgttctcg gcccgaccaa ccgcacggcg 420

```
tctatttctc cggacgtcaa cgatccggca tttcgtaata tcacttttga cgggctggtg      480
gcggcttatc gagagtccac caaagcgctg gtggaaggtg gcgcggatct gatcctgatt      540
gaaaccgttt tcgacacccct taacgccaaa gcggcggtat ttgcggtgaa aacggagttt      600
gaagcgctgg gcgttgagct gccgattatg atctccggca ccatcaccga cgcctccggg      660
cgcacgctct ccgggcagac caccgaagca ttttacaact cattgcgcca cgccgaagct      720
ctgacctttg gcctgaactg tgcgctgggg cccgatgaac tgcgccagta cgtgcaggag      780
ctgtcacgga ttgcggaatg ctacgtcacc gcgcacccga acgccgggct acccaacgcc      840
tttggtgagt acgatctcga cgccgacacg atggcaaaac agatacgtga atgggcgcaa      900
gcgggttttc tcaatatcgt cggcggctgc tgtggcacca cgccacaaca tattgcagcg      960
atgagtcgtg cagtagaagg attagcgccg cgcaaactgc cggaaattcc cgtagcctgc     1020
cgtttgtccg gcctggagcc gctgaacatt ggcgaagata gcctgtttgt gaacgtgggt     1080
gaacgcacca acgtcaccgg ttccgctaag ttcaagcgcc tgatcaaaga agagaaatac     1140
agcgaggcgc tggatgtcgc gcgtcaacag gtggaaaacg gcgcgcagat tatcgatatc     1200
aacatggatg aagggatgct cgatgccgaa gcggcgatgg tgcgttttct caatctgatt     1260
gccggtgaac cggatatcgc tcgcgtgccg attatgatcg actcctcaaa atgggacgtc     1320
attgaaaaag gtctgaagtg tatccagggc aaaggcattg ttaactctat ctcgatgaaa     1380
gagggcgtcg atgcctttat ccatcacgcg aaattgttgc gtcgctacgg tgcggcagtg     1440
gtggtaatgg cctttgacga acagggacag gccgatactc gcgcacggaa aatcgagatt     1500
tgccgtcggg cgtacaaaat cctcaccgaa gaggttggct tcccgccaga agatatcatc     1560
ttcgacccaa acatcttcgc ggtcgcaact ggcattgaag agcacaacaa ctacgcgcag     1620
gactttatcg gcgcgtgtga agacatcaaa cgcgaactgc cgcacgcgct gatttccggc     1680
ggcgtatcta acgtttcttt ctcgttccgt ggcaacgatc cggtgcgcga agccattcac     1740
gcagtgttcc tctactacgc tattcgcaat ggcatggata tggggatcgt caacgccggg     1800
caactggcga tttacgacga cctacccgct gaactgcgcg acgcggtgga agatgtgatt     1860
cttaatcgtc gcgacgatgg caccgagcgt ttactggagc ttgccgagaa atatcgcggc     1920
agcaaaaccg acgacaccgc caacgcccag caggcggagt ggcgctcgtg ggaagtgaat     1980
aaacgtctgg aatactcgct ggtcaaaggc attaccgagt ttatcgagca ggataccgaa     2040
gaagcccgcc agcaggctac gcgcccgatt gaagtgattg aaggcccgtt gatggacggc     2100
atgaatgtgg tcggcgacct gtttggcgaa gggaaaatgt tcctgccaca ggtggtcaaa     2160
tcggcgcgcg tcatgaaaca ggcggtggcc tacctcgaac cgtttattga agccagcaaa     2220
gagcagggca aaaccaacgg caagatggtg atcgccaccg tgaagggcga cgtccacgac     2280
```

atcggtaaaa atatcgttgg tgtggtgctg caatgtaaca actacgaaat tgtcgatctc 2340

ggcgttatgg tgcctgcgga aaaaattctc cgtaccgcta aagaagtgaa tgctgatctg 2400

attggccttt cggggcttat cacgccgtcg ctggacgaga tggttaacgt ggcgaaagag 2460

atggagcgtc agggcttcac tattccgtta ctgattggcg gcgcgacgac ctcaaaagcg 2520

cacacggcgg tgaaaatcga gcagaactac agcggcccga cggtgtatgt gcagaatgcc 2580

tcgcgtaccg ttggtgtggt ggcggcgctg ctttccgata cccagcgtga tgattttgtc 2640

gctcgtaccc gcaaggagta cgaaaccgta cgtattcagc acgggcgcaa gaaaccgcgc 2700

acaccaccgg tcacgctgga agcggcgcgc gataacgatt tcgcttttga ctggcaggct 2760

tacacgccgc cggtggcgca ccgtctcggc gtgcaggaag tcgaagccag catcgaaacg 2820

ctgcgtaatt acatcgactg gacaccgttc tttatgacct ggtcgctggc cgggaagtat 2880

ccgcgcattc tggaagatga agtggtgggc gttgaggcgc agcggctgtt taaagacgcc 2940

aacgacatgc tggataaatt aagcgccgag aaaacgctga atccgcgtgg cgtggtgggc 3000

ctgttcccgg caaaccgtgt gggcgatgac attgaaatct accgtgacga aacgcgtacc 3060

catgtgatca acgtcagcca ccatctgcgt caacagaccg aaaaaacagg cttcgctaac 3120

tactgtctcg ctgacttcgt tgcgccgaag ctttctggta aagcagatta catcggcgca 3180

tttgccgtga ctggcgggct ggaagaggac gcactggctg atgcctttga agcgcagcac 3240

gatgattaca acaaaatcat ggtgaaagcg cttgccgacc gtttagccga agcctttgcg 3300

gagtatctcc atgagcgtgt gcgtaaagtc tactggggct atgcgccgaa cgagaacctc 3360

agcaacgaag agctgatccg cgaaaactac cagggcatcc gtccggcacc gggctatccg 3420

gcctgcccgg aacatacgga aaaagccacc atctgggagc tgctggaagt ggaaaaacac 3480

actggcatga aactcacaga atctttcgcc atgtggcccg gtgcatcggt ttcgggttgg 3540

tacttcagcc acccggacag caagtactac gctgtagcac aaattcagcg cgatcaggtt 3600

gaagattatg cccgccgtaa aggtatgagc gttaccgaag ttgagcgctg gctggcaccg 3660

aatctggggt atgacgcgga ctga 3684

<210> 26
<211> 1227
<212> PRT
<213> Escherichia coli

<400> 26

Met Ser Ser Lys Val Glu Gln Leu Arg Ala Gln Leu Asn Glu Arg Ile
1 5 10 15

Leu Val Leu Asp Gly Gly Met Gly Thr Met Ile Gln Ser Tyr Arg Leu
20 25 30

```
Asn Glu Ala Asp Phe Arg Gly Glu Arg Phe Ala Asp Trp Pro Cys Asp
        35                  40                  45

Leu Lys Gly Asn Asn Asp Leu Leu Val Leu Ser Lys Pro Glu Val Ile
    50                  55                  60

Ala Ala Ile His Asn Ala Tyr Phe Glu Ala Gly Ala Asp Ile Ile Glu
65                  70                  75                  80

Thr Asn Thr Phe Asn Ser Thr Thr Ile Ala Met Ala Asp Tyr Gln Met
                85                  90                  95

Glu Ser Leu Ser Ala Glu Ile Asn Phe Ala Ala Ala Lys Leu Ala Arg
            100                 105                 110

Ala Cys Ala Asp Glu Trp Thr Ala Arg Thr Pro Glu Lys Pro Arg Tyr
        115                 120                 125

Val Ala Gly Val Leu Gly Pro Thr Asn Arg Thr Ala Ser Ile Ser Pro
    130                 135                 140

Asp Val Asn Asp Pro Ala Phe Arg Asn Ile Thr Phe Asp Gly Leu Val
145                 150                 155                 160

Ala Ala Tyr Arg Glu Ser Thr Lys Ala Leu Val Glu Gly Gly Ala Asp
                165                 170                 175

Leu Ile Leu Ile Glu Thr Val Phe Asp Thr Leu Asn Ala Lys Ala Ala
            180                 185                 190

Val Phe Ala Val Lys Thr Glu Phe Glu Ala Leu Gly Val Glu Leu Pro
        195                 200                 205

Ile Met Ile Ser Gly Thr Ile Thr Asp Ala Ser Gly Arg Thr Leu Ser
    210                 215                 220

Gly Gln Thr Thr Glu Ala Phe Tyr Asn Ser Leu Arg His Ala Glu Ala
225                 230                 235                 240

Leu Thr Phe Gly Leu Asn Cys Ala Leu Gly Pro Asp Glu Leu Arg Gln
                245                 250                 255

Tyr Val Gln Glu Leu Ser Arg Ile Ala Glu Cys Tyr Val Thr Ala His
            260                 265                 270

Pro Asn Ala Gly Leu Pro Asn Ala Phe Gly Glu Tyr Asp Leu Asp Ala
        275                 280                 285
```

```
Asp Thr Met Ala Lys Gln Ile Arg Glu Trp Ala Gln Ala Gly Phe Leu
    290                 295                 300

Asn Ile Val Gly Gly Cys Cys Gly Thr Thr Pro Gln His Ile Ala Ala
305                 310                 315                 320

Met Ser Arg Ala Val Glu Gly Leu Ala Pro Arg Lys Leu Pro Glu Ile
                325                 330                 335

Pro Val Ala Cys Arg Leu Ser Gly Leu Glu Pro Leu Asn Ile Gly Glu
            340                 345                 350

Asp Ser Leu Phe Val Asn Val Gly Glu Arg Thr Asn Val Thr Gly Ser
        355                 360                 365

Ala Lys Phe Lys Arg Leu Ile Lys Glu Glu Lys Tyr Ser Glu Ala Leu
    370                 375                 380

Asp Val Ala Arg Gln Gln Val Glu Asn Gly Ala Gln Ile Ile Asp Ile
385                 390                 395                 400

Asn Met Asp Glu Gly Met Leu Asp Ala Glu Ala Ala Met Val Arg Phe
                405                 410                 415

Leu Asn Leu Ile Ala Gly Glu Pro Asp Ile Ala Arg Val Pro Ile Met
            420                 425                 430

Ile Asp Ser Ser Lys Trp Asp Val Ile Glu Lys Gly Leu Lys Cys Ile
        435                 440                 445

Gln Gly Lys Gly Ile Val Asn Ser Ile Ser Met Lys Glu Gly Val Asp
    450                 455                 460

Ala Phe Ile His His Ala Lys Leu Leu Arg Arg Tyr Gly Ala Ala Val
465                 470                 475                 480

Val Val Met Ala Phe Asp Glu Gln Gly Gln Ala Asp Thr Arg Ala Arg
                485                 490                 495

Lys Ile Glu Ile Cys Arg Arg Ala Tyr Lys Ile Leu Thr Glu Glu Val
            500                 505                 510

Gly Phe Pro Pro Glu Asp Ile Ile Phe Asp Pro Asn Ile Phe Ala Val
        515                 520                 525

Ala Thr Gly Ile Glu Glu His Asn Asn Tyr Ala Gln Asp Phe Ile Gly
```

530 535 540

Ala Cys Glu Asp Ile Lys Arg Glu Leu Pro His Ala Leu Ile Ser Gly
545 550 555 560

Gly Val Ser Asn Val Ser Phe Ser Phe Arg Gly Asn Asp Pro Val Arg
565 570 575

Glu Ala Ile His Ala Val Phe Leu Tyr Tyr Ala Ile Arg Asn Gly Met
580 585 590

Asp Met Gly Ile Val Asn Ala Gly Gln Leu Ala Ile Tyr Asp Asp Leu
595 600 605

Pro Ala Glu Leu Arg Asp Ala Val Glu Asp Val Ile Leu Asn Arg Arg
610 615 620

Asp Asp Gly Thr Glu Arg Leu Leu Glu Leu Ala Glu Lys Tyr Arg Gly
625 630 635 640

Ser Lys Thr Asp Asp Thr Ala Asn Ala Gln Gln Ala Glu Trp Arg Ser
645 650 655

Trp Glu Val Asn Lys Arg Leu Glu Tyr Ser Leu Val Lys Gly Ile Thr
660 665 670

Glu Phe Ile Glu Gln Asp Thr Glu Glu Ala Arg Gln Gln Ala Thr Arg
675 680 685

Pro Ile Glu Val Ile Glu Gly Pro Leu Met Asp Gly Met Asn Val Val
690 695 700

Gly Asp Leu Phe Gly Glu Gly Lys Met Phe Leu Pro Gln Val Val Lys
705 710 715 720

Ser Ala Arg Val Met Lys Gln Ala Val Ala Tyr Leu Glu Pro Phe Ile
725 730 735

Glu Ala Ser Lys Glu Gln Gly Lys Thr Asn Gly Lys Met Val Ile Ala
740 745 750

Thr Val Lys Gly Asp Val His Asp Ile Gly Lys Asn Ile Val Gly Val
755 760 765

Val Leu Gln Cys Asn Asn Tyr Glu Ile Val Asp Leu Gly Val Met Val
770 775 780

Pro Ala Glu Lys Ile Leu Arg Thr Ala Lys Glu Val Asn Ala Asp Leu
785 790 795 800

Ile Gly Leu Ser Gly Leu Ile Thr Pro Ser Leu Asp Glu Met Val Asn
805 810 815

Val Ala Lys Glu Met Glu Arg Gln Gly Phe Thr Ile Pro Leu Leu Ile
820 825 830

Gly Gly Ala Thr Thr Ser Lys Ala His Thr Ala Val Lys Ile Glu Gln
835 840 845

Asn Tyr Ser Gly Pro Thr Val Tyr Val Gln Asn Ala Ser Arg Thr Val
850 855 860

Gly Val Val Ala Ala Leu Leu Ser Asp Thr Gln Arg Asp Asp Phe Val
865 870 875 880

Ala Arg Thr Arg Lys Glu Tyr Glu Thr Val Arg Ile Gln His Gly Arg
885 890 895

Lys Lys Pro Arg Thr Pro Pro Val Thr Leu Glu Ala Ala Arg Asp Asn
900 905 910

Asp Phe Ala Phe Asp Trp Gln Ala Tyr Thr Pro Pro Val Ala His Arg
915 920 925

Leu Gly Val Gln Glu Val Glu Ala Ser Ile Glu Thr Leu Arg Asn Tyr
930 935 940

Ile Asp Trp Thr Pro Phe Phe Met Thr Trp Ser Leu Ala Gly Lys Tyr
945 950 955 960

Pro Arg Ile Leu Glu Asp Glu Val Val Gly Val Glu Ala Gln Arg Leu
965 970 975

Phe Lys Asp Ala Asn Asp Met Leu Asp Lys Leu Ser Ala Glu Lys Thr
980 985 990

Leu Asn Pro Arg Gly Val Val Gly Leu Phe Pro Ala Asn Arg Val Gly
995 1000 1005

Asp Asp Ile Glu Ile Tyr Arg Asp Glu Thr Arg Thr His Val Ile
1010 1015 1020

Asn Val Ser His His Leu Arg Gln Gln Thr Glu Lys Thr Gly Phe
1025 1030 1035

```
Ala Asn  Tyr Cys Leu Ala Asp  Phe Val Ala Pro Lys  Leu Ser Gly
    1040                 1045                 1050

Lys Ala  Asp Tyr Ile Gly Ala  Phe Ala Val Thr Gly  Gly Leu Glu
    1055                 1060                 1065

Glu Asp  Ala Leu Ala Asp Ala  Phe Glu Ala Gln His  Asp Asp Tyr
    1070                 1075                 1080

Asn Lys  Ile Met Val Lys Ala  Leu Ala Asp Arg Leu  Ala Glu Ala
    1085                 1090                 1095

Phe Ala  Glu Tyr Leu His Glu  Arg Val Arg Lys Val  Tyr Trp Gly
    1100                 1105                 1110

Tyr Ala  Pro Asn Glu Asn Leu  Ser Asn Glu Glu Leu  Ile Arg Glu
    1115                 1120                 1125

Asn Tyr  Gln Gly Ile Arg Pro  Ala Pro Gly Tyr Pro  Ala Cys Pro
    1130                 1135                 1140

Glu His  Thr Glu Lys Ala Thr  Ile Trp Glu Leu Leu  Glu Val Glu
    1145                 1150                 1155

Lys His  Thr Gly Met Lys Leu  Thr Glu Ser Phe Ala  Met Trp Pro
    1160                 1165                 1170

Gly Ala  Ser Val Ser Gly Trp  Tyr Phe Ser His Pro  Asp Ser Lys
    1175                 1180                 1185

Tyr Tyr  Ala Val Ala Gln Ile  Gln Arg Asp Gln Val  Glu Asp Tyr
    1190                 1195                 1200

Ala Arg  Arg Lys Gly Met Ser  Val Thr Glu Val Glu  Arg Trp Leu
    1205                 1210                 1215

Ala Pro  Asn Leu Gly Tyr Asp  Ala Asp
    1220                 1225
```

<210> 27
<211> 891
<212> DNA
<213> Escherichia coli

<400> 27

```
atgagctttt ttcacgccag ccagcgggat gccctgaatc agagcctggc agaagtccag        60

gggcagatta acgtttcgtt cgagtttttc ccgccgcgta ccagtgaaat ggagcagacc       120
```

ctgtggaact ccatcgatcg ccttagcagc ctgaaaccga agtttgtatc ggtgacctat 180

ggcgcgaact ccggcgagcg cgaccgtacg cacagcatta ttaaaggcat taaagatcgc 240

actggtctgg aagcggcacc gcatcttact tgcattgatg cgacgcccga cgagctgcgc 300

accattgcac gcgactactg gaataacggt attcgtcata tcgtggcgct gcgtggcgat 360

ctgccgccgg gaagtggtaa gccagaaatg tatgcttctg acctggtgac gctgttaaaa 420

gaagtggcag atttcgatat ctccgtggcg gcgtatccgg aagttcaccc ggaagcaaaa 480

agcgctcagg cggatttgct taatctgaaa cgcaaagtgg atgccggagc caaccgcgcg 540

attactcagt tcttcttcga tgtcgaaagc tacctgcgtt ttcgtgaccg ctgtgtatcg 600

gcgggcattg atgtggaaat tattccggga attttgccgg tatctaactt taaacaggcg 660

aagaaatttg ccgatatgac caacgtgcgt attccggcgt ggatggcgca aatgttcgac 720

ggtctggatg atgatgccga aacccgcaaa ctggttggcg cgaatattgc catggatatg 780

gtgaagattt taagccgtga aggagtgaaa gatttccact tctatacgct taaccgtgct 840

gaaatgagtt acgcgatttg ccatacgctg gggggttcgac ctggtttata a 891

<210> 28
<211> 296
<212> PRT
<213> Escherichia coli

<400> 28

Met Ser Phe Phe His Ala Ser Gln Arg Asp Ala Leu Asn Gln Ser Leu
1 5 10 15

Ala Glu Val Gln Gly Gln Ile Asn Val Ser Phe Glu Phe Phe Pro Pro
20 25 30

Arg Thr Ser Glu Met Glu Gln Thr Leu Trp Asn Ser Ile Asp Arg Leu
35 40 45

Ser Ser Leu Lys Pro Lys Phe Val Ser Val Thr Tyr Gly Ala Asn Ser
50 55 60

Gly Glu Arg Asp Arg Thr His Ser Ile Ile Lys Gly Ile Lys Asp Arg
65 70 75 80

Thr Gly Leu Glu Ala Ala Pro His Leu Thr Cys Ile Asp Ala Thr Pro
85 90 95

Asp Glu Leu Arg Thr Ile Ala Arg Asp Tyr Trp Asn Asn Gly Ile Arg
100 105 110

His Ile Val Ala Leu Arg Gly Asp Leu Pro Pro Gly Ser Gly Lys Pro

115 120 125

Glu Met Tyr Ala Ser Asp Leu Val Thr Leu Leu Lys Glu Val Ala Asp
130 135 140

Phe Asp Ile Ser Val Ala Ala Tyr Pro Glu Val His Pro Glu Ala Lys
145 150 155 160

Ser Ala Gln Ala Asp Leu Leu Asn Leu Lys Arg Lys Val Asp Ala Gly
165 170 175

Ala Asn Arg Ala Ile Thr Gln Phe Phe Phe Asp Val Glu Ser Tyr Leu
180 185 190

Arg Phe Arg Asp Arg Cys Val Ser Ala Gly Ile Asp Val Glu Ile Ile
195 200 205

Pro Gly Ile Leu Pro Val Ser Asn Phe Lys Gln Ala Lys Lys Phe Ala
210 215 220

Asp Met Thr Asn Val Arg Ile Pro Ala Trp Met Ala Gln Met Phe Asp
225 230 235 240

Gly Leu Asp Asp Asp Ala Glu Thr Arg Lys Leu Val Gly Ala Asn Ile
245 250 255

Ala Met Asp Met Val Lys Ile Leu Ser Arg Glu Gly Val Lys Asp Phe
260 265 270

His Phe Tyr Thr Leu Asn Arg Ala Glu Met Ser Tyr Ala Ile Cys His
275 280 285

Thr Leu Gly Val Arg Pro Gly Leu
290 295

<210> 29
<211> 2262
<212> DNA
<213> Escherichia coli

<400> 29
atgacaatat tgaatcacac cctcggtttc cctcgcgttg gcctgcgtcg cgagctgaaa 60

aaagcgcaag aaagttattg ggcggggaac tccacgcgtg aagaactgct ggcggtaggg 120

cgtgaattgc gtgctcgtca ctgggatcaa caaaagcaag cgggtatcga cctgctgccg 180

gtgggcgatt ttgcctggta cgatcatgta ctgaccacca gtctgctgct gggtaacgtt 240

ccggcgcgtc atcagaacaa agatggttcg gtagatatcg acaccctgtt ccgtattggt 300

cgtggacgtg cgccgactgg cgaacctgcg gcggcagcgg aaatgaccaa atggtttaac 360

accaactatc actacatggt gccggagttc gttaaaggcc aacagttcaa actgacctgg 420

acgcagctgc tggacgaagt ggacgaggcg ctggcgctgg gccacaaggt gaaacctgtg 480

ctgctggggc cggttacctg gctgtggctg gggaaagtga aaggtgaaca atttgaccgc 540

ctgagcctgc tgaacgacat tctgccggtt tatcagcaag tgctggcaga actggcgaaa 600

cgcggcatcg agtgggtaca gattgatgaa cccgcgctgg tactggaact accacaggcg 660

tggctggacg catacaaacc cgcttacgac gcgctccagg gacaggtgaa actgctgctg 720

accacctatt ttgaaggcgt aacgccaaat ctcgacacga ttactgcgct gcctgttcag 780

ggtctgcatg ttgacctcgt acatggtaaa gatgacgttg ctgaactgca caagcgcctg 840

ccttctgact ggttgctgtc tgcgggtctg atcaatggtc gtaacgtctg gcgcgccgat 900

cttaccgaga aatatgcgca aattaaggac attgtcggca aacgtgattt gtgggtggca 960

tcttcctgct cgttgctgca cagccccatc gacctgagcg tggaaacgcg tcttgatgca 1020

gaagtgaaaa gctggtttgc cttcgcccta caaaaatgcc atgaactggc actgctgcgc 1080

gatgcgctga acagtggtga cacggcagct ctggcagagt ggagcgcccc gattcaggca 1140

cgtcgtcact ctacccgcgt acataatccg gcggtagaaa agcgtctggc ggcgatcacc 1200

gcccaggaca gccagcgtgc gaatgtctat gaagtgcgtg ctgaagccca gcgtgcgcgt 1260

tttaaactgc cagcgtggcc gaccaccacg attggttcct tcccgcaaac cacggaaatt 1320

cgtaccctgc gtctggattt caaaaagggc aatctcgacg ccaacaacta ccgcacgggc 1380

attgcggaac atatcaagca ggccattgtt gagcaggaac gtttgggact ggatgtgctg 1440

gtacatggcg aggccgagcg taatgacatg gtggaatact ttggcgagca cctcgacgga 1500

tttgtcttta cgcaaaacgg ttgggtacag agctacggtt cccgctgcgt gaagccaccg 1560

attgtcattg gtgacattag ccgcccggca ccgattaccg tggagtgggc gaagtatgcg 1620

caatcgctga ccgacaaacc ggtgaaaggg atgctgacgg ggccggtgac catactctgc 1680

tggtcgttcc cgcgtgaaga tgtcagccgt gaaaccatcg ccaaacagat tgcgctggcg 1740

ctgcgtgatg aagtggccga tctggaagcc gctggaattg gcatcatcca gattgacgaa 1800

ccggcgctgc gcgaaggttt accgctgcgt cgtagcgact gggatgcgta tctccagtgg 1860

ggcgtagagg ccttccgtat caacgccgcc gtggcgaaag atgacacaca aatccacact 1920

cacatgtgtt attgcgagtt caacgacatc atggattcga ttgcggcgct ggacgcagac 1980

gtcatcacca tcgaaacctc gcgttccgac atggagttgc tggagtcgtt tgaagagttt 2040

gattatccaa atgaaatcgg tcctggcgtc tatgacattc actcgccaaa cgtaccgagc 2100

gtggaatgga ttgaagcctt gctgaagaaa gcggcaaaac gcattccggc agagcgcctg 2160

tgggtcaacc cggactgtgg cctgaaaacg cgcggctggc cagaaacccg cgcggcactg 2220

gcgaacatgg tgcaggcggc gcagaacttg cgtcgggggt aa 2262

<210> 30
<211> 753
<212> PRT
<213> Escherichia coli

<400> 30

Met Thr Ile Leu Asn His Thr Leu Gly Phe Pro Arg Val Gly Leu Arg
1 5 10 15

Arg Glu Leu Lys Lys Ala Gln Glu Ser Tyr Trp Ala Gly Asn Ser Thr
20 25 30

Arg Glu Glu Leu Leu Ala Val Gly Arg Glu Leu Arg Ala Arg His Trp
35 40 45

Asp Gln Gln Lys Gln Ala Gly Ile Asp Leu Leu Pro Val Gly Asp Phe
50 55 60

Ala Trp Tyr Asp His Val Leu Thr Thr Ser Leu Leu Leu Gly Asn Val
65 70 75 80

Pro Ala Arg His Gln Asn Lys Asp Gly Ser Val Asp Ile Asp Thr Leu
85 90 95

Phe Arg Ile Gly Arg Gly Arg Ala Pro Thr Gly Glu Pro Ala Ala Ala
100 105 110

Ala Glu Met Thr Lys Trp Phe Asn Thr Asn Tyr His Tyr Met Val Pro
115 120 125

Glu Phe Val Lys Gly Gln Gln Phe Lys Leu Thr Trp Thr Gln Leu Leu
130 135 140

Asp Glu Val Asp Glu Ala Leu Ala Leu Gly His Lys Val Lys Pro Val
145 150 155 160

Leu Leu Gly Pro Val Thr Trp Leu Trp Leu Gly Lys Val Lys Gly Glu
165 170 175

Gln Phe Asp Arg Leu Ser Leu Leu Asn Asp Ile Leu Pro Val Tyr Gln
180 185 190

Gln Val Leu Ala Glu Leu Ala Lys Arg Gly Ile Glu Trp Val Gln Ile
195 200 205

```
Asp Glu Pro Ala Leu Val Leu Glu Leu Pro Gln Ala Trp Leu Asp Ala
    210                 215                 220

Tyr Lys Pro Ala Tyr Asp Ala Leu Gln Gly Gln Val Lys Leu Leu Leu
225                 230                 235                 240

Thr Thr Tyr Phe Glu Gly Val Thr Pro Asn Leu Asp Thr Ile Thr Ala
                245                 250                 255

Leu Pro Val Gln Gly Leu His Val Asp Leu Val His Gly Lys Asp Asp
            260                 265                 270

Val Ala Glu Leu His Lys Arg Leu Pro Ser Asp Trp Leu Leu Ser Ala
        275                 280                 285

Gly Leu Ile Asn Gly Arg Asn Val Trp Arg Ala Asp Leu Thr Glu Lys
    290                 295                 300

Tyr Ala Gln Ile Lys Asp Ile Val Gly Lys Arg Asp Leu Trp Val Ala
305                 310                 315                 320

Ser Ser Cys Ser Leu Leu His Ser Pro Ile Asp Leu Ser Val Glu Thr
                325                 330                 335

Arg Leu Asp Ala Glu Val Lys Ser Trp Phe Ala Phe Ala Leu Gln Lys
            340                 345                 350

Cys His Glu Leu Ala Leu Leu Arg Asp Ala Leu Asn Ser Gly Asp Thr
        355                 360                 365

Ala Ala Leu Ala Glu Trp Ser Ala Pro Ile Gln Ala Arg Arg His Ser
    370                 375                 380

Thr Arg Val His Asn Pro Ala Val Glu Lys Arg Leu Ala Ala Ile Thr
385                 390                 395                 400

Ala Gln Asp Ser Gln Arg Ala Asn Val Tyr Glu Val Arg Ala Glu Ala
                405                 410                 415

Gln Arg Ala Arg Phe Lys Leu Pro Ala Trp Pro Thr Thr Thr Ile Gly
            420                 425                 430

Ser Phe Pro Gln Thr Thr Glu Ile Arg Thr Leu Arg Leu Asp Phe Lys
        435                 440                 445

Lys Gly Asn Leu Asp Ala Asn Asn Tyr Arg Thr Gly Ile Ala Glu His
    450                 455                 460
```

```
Ile Lys Gln Ala Ile Val Glu Gln Glu Arg Leu Gly Leu Asp Val Leu
465                 470                 475                 480

Val His Gly Glu Ala Glu Arg Asn Asp Met Val Glu Tyr Phe Gly Glu
                485                 490                 495

His Leu Asp Gly Phe Val Phe Thr Gln Asn Gly Trp Val Gln Ser Tyr
            500                 505                 510

Gly Ser Arg Cys Val Lys Pro Pro Ile Val Ile Gly Asp Ile Ser Arg
        515                 520                 525

Pro Ala Pro Ile Thr Val Glu Trp Ala Lys Tyr Ala Gln Ser Leu Thr
    530                 535                 540

Asp Lys Pro Val Lys Gly Met Leu Thr Gly Pro Val Thr Ile Leu Cys
545                 550                 555                 560

Trp Ser Phe Pro Arg Glu Asp Val Ser Arg Glu Thr Ile Ala Lys Gln
                565                 570                 575

Ile Ala Leu Ala Leu Arg Asp Glu Val Ala Asp Leu Glu Ala Ala Gly
            580                 585                 590

Ile Gly Ile Ile Gln Ile Asp Glu Pro Ala Leu Arg Glu Gly Leu Pro
        595                 600                 605

Leu Arg Arg Ser Asp Trp Asp Ala Tyr Leu Gln Trp Gly Val Glu Ala
    610                 615                 620

Phe Arg Ile Asn Ala Ala Val Ala Lys Asp Asp Thr Gln Ile His Thr
625                 630                 635                 640

His Met Cys Tyr Cys Glu Phe Asn Asp Ile Met Asp Ser Ile Ala Ala
                645                 650                 655

Leu Asp Ala Asp Val Ile Thr Ile Glu Thr Ser Arg Ser Asp Met Glu
            660                 665                 670

Leu Leu Glu Ser Phe Glu Glu Phe Asp Tyr Pro Asn Glu Ile Gly Pro
        675                 680                 685

Gly Val Tyr Asp Ile His Ser Pro Asn Val Pro Ser Val Glu Trp Ile
    690                 695                 700

Glu Ala Leu Leu Lys Lys Ala Ala Lys Arg Ile Pro Ala Glu Arg Leu
705                 710                 715                 720
```

Trp Val Asn Pro Asp Cys Gly Leu Lys Thr Arg Gly Trp Pro Glu Thr
725 730 735

Arg Ala Ala Leu Ala Asn Met Val Gln Ala Ala Gln Asn Leu Arg Arg
740 745 750

Gly

<210> 31
<211> 531
<212> DNA
<213> Escherichia coli

<400> 31
atggctatca ctggcatctt tttcggcagc gacaccggta ataccgaaaa tatcgcaaaa 60

atgattcaaa aacagcttgg taaagacgtt gccgatgtcc atgacattgc aaaaagcagc 120

aaagaagatc tggaagctta tgacattctg ctgctgggca tcccaacctg gtattacggc 180

gaagcgcagt gtgactggga tgacttcttc ccgactctcg aagagattga tttcaacggc 240

aaactggttg cgctgtttgg ttgtggtgac caggaagatt acgccgaata tttctgcgac 300

gcattgggca ccatccgcga catcattgaa ccgcgcggtg caaccatcgt tggtcactgg 360

ccaactgcgg gctatcattt cgaagcatca aaaggtctgg cagatgacga ccactttgtc 420

ggtctggcta tcgacgaaga ccgtcagccg gaactgaccg ctgaacgtgt agaaaaatgg 480

gttaaacaga tttctgaaga gttgcatctc gacgaaattc tcaatgcctg a 531

<210> 32
<211> 176
<212> PRT
<213> Escherichia coli

<400> 32

Met Ala Ile Thr Gly Ile Phe Phe Gly Ser Asp Thr Gly Asn Thr Glu
1 5 10 15

Asn Ile Ala Lys Met Ile Gln Lys Gln Leu Gly Lys Asp Val Ala Asp
20 25 30

Val His Asp Ile Ala Lys Ser Ser Lys Glu Asp Leu Glu Ala Tyr Asp
35 40 45

Ile Leu Leu Leu Gly Ile Pro Thr Trp Tyr Tyr Gly Glu Ala Gln Cys
50 55 60

Asp Trp Asp Asp Phe Phe Pro Thr Leu Glu Glu Ile Asp Phe Asn Gly

65 70 75 80

Lys Leu Val Ala Leu Phe Gly Cys Gly Asp Gln Glu Asp Tyr Ala Glu
85 90 95

Tyr Phe Cys Asp Ala Leu Gly Thr Ile Arg Asp Ile Ile Glu Pro Arg
100 105 110

Gly Ala Thr Ile Val Gly His Trp Pro Thr Ala Gly Tyr His Phe Glu
115 120 125

Ala Ser Lys Gly Leu Ala Asp Asp Asp His Phe Val Gly Leu Ala Ile
130 135 140

Asp Glu Asp Arg Gln Pro Glu Leu Thr Ala Glu Arg Val Glu Lys Trp
145 150 155 160

Val Lys Gln Ile Ser Glu Glu Leu His Leu Asp Glu Ile Leu Asn Ala
165 170 175

<210> 33
<211> 747
<212> DNA
<213> Escherichia coli

<400> 33
atggctgatt gggtaacagg caaagtcact aaagtgcaga actggaccga cgccctgttt 60

agtctcaccg ttcacgcccc cgtgcttccg tttaccgccg ggcaatttac caagcttggc 120

cttgaaatcg acggcgaacg cgtccagcgc gcctactcct atgtaaactc gcccgataat 180

cccgatctgg agttttacct ggtcaccgtc cccgatggca aattaagccc acgactggcg 240

gcactgaaac caggcgatga agtgcaggtg gttagcgaag cggcaggatt ctttgtgctc 300

gatgaagtgc cgcactgcga aacgctatgg atgctggcaa ccggtacagc gattggccct 360

tatttatcga ttctgcaact aggtaaagat ttagatcgct tcaaaaatct ggtcctggtg 420

cacgccgcac gttatgccgc cgacttaagc tatttgccac tgatgcagga actggaaaaa 480

cgctacgaag gaaaactgcg cattcagacg gtggtcagtc gggaaacggc agcggggtcg 540

ctcaccggac ggataccggc attaattgaa agtggggaac tggaaagcac gattggcctg 600

ccgatgaata aagaaaccag ccatgtgatg ctgtgcggca atccacagat ggtgcgcgat 660

acacaacagt tgctgaaaga gacccggcag atgacgaaac atttacgtcg ccgaccgggc 720

catatgacag cggagcatta ctggtaa 747

<210> 34
<211> 248
<212> PRT

<213> Escherichia coli

<400> 34

Met Ala Asp Trp Val Thr Gly Lys Val Thr Lys Val Gln Asn Trp Thr
1 5 10 15

Asp Ala Leu Phe Ser Leu Thr Val His Ala Pro Val Leu Pro Phe Thr
20 25 30

Ala Gly Gln Phe Thr Lys Leu Gly Leu Glu Ile Asp Gly Glu Arg Val
35 40 45

Gln Arg Ala Tyr Ser Tyr Val Asn Ser Pro Asp Asn Pro Asp Leu Glu
50 55 60

Phe Tyr Leu Val Thr Val Pro Asp Gly Lys Leu Ser Pro Arg Leu Ala
65 70 75 80

Ala Leu Lys Pro Gly Asp Glu Val Gln Val Val Ser Glu Ala Ala Gly
85 90 95

Phe Phe Val Leu Asp Glu Val Pro His Cys Glu Thr Leu Trp Met Leu
100 105 110

Ala Thr Gly Thr Ala Ile Gly Pro Tyr Leu Ser Ile Leu Gln Leu Gly
115 120 125

Lys Asp Leu Asp Arg Phe Lys Asn Leu Val Leu Val His Ala Ala Arg
130 135 140

Tyr Ala Ala Asp Leu Ser Tyr Leu Pro Leu Met Gln Glu Leu Glu Lys
145 150 155 160

Arg Tyr Glu Gly Lys Leu Arg Ile Gln Thr Val Val Ser Arg Glu Thr
165 170 175

Ala Ala Gly Ser Leu Thr Gly Arg Ile Pro Ala Leu Ile Glu Ser Gly
180 185 190

Glu Leu Glu Ser Thr Ile Gly Leu Pro Met Asn Lys Glu Thr Ser His
195 200 205

Val Met Leu Cys Gly Asn Pro Gln Met Val Arg Asp Thr Gln Gln Leu
210 215 220

Leu Lys Glu Thr Arg Gln Met Thr Lys His Leu Arg Arg Arg Pro Gly
225 230 235 240

His Met Thr Ala Glu His Tyr Trp
245

<210> 35
<211> 1155
<212> DNA
<213> Escherichia coli

<400> 35
atggcaaaac accttttac gtccgagtcc gtctctgaag ggcatcctga caaaattgct 60

gaccaaattt ctgatgccgt tttagacgcg atcctcgaac aggatccgaa agcacgcgtt 120

gcttgcgaaa cctacgtaaa aaccggcatg gttttagttg gcggcgaaat caccaccagc 180

gcctgggtag acatcgaaga gatcacccgt aacaccgttc gcgaaattgg ctatgtgcat 240

tccgacatgg gctttgacgc taactcctgt gcggttctga gcgctatcgg caaacagtct 300

cctgacatca accagggcgt tgaccgtgcc gatccgctgg aacagggcgc gggtgaccag 360

ggtctgatgt ttggctacgc aactaatgaa accgacgtgc tgatgccagc acctatcacc 420

tatgcacacc gtctggtaca gcgtcaggct gaagtgcgta aaaacggcac tctgccgtgg 480

ctgcgcccgg acgcgaaaag ccaggtgact tttcagtatg acgacggcaa aatcgttggt 540

atcgatgctg tcgtgctttc cactcagcac tctgaagaga tcgaccagaa atcgctgcaa 600

gaagcggtaa tggaagagat catcaagcca attctgcccg ctgaatggct gacttctgcc 660

accaaattct tcatcaaccc gaccggtcgt ttcgttatcg gtggcccaat gggtgactgc 720

ggtctgactg gtcgtaaaat tatcgttgat acctacggcg gcatggcgcg tcacggtggc 780

ggtgcattct ctggtaaaga tccatcaaaa gtggaccgtt ccgcagccta cgcagcacgt 840

tatgtcgcga aaaacatcgt tgctgctggc ctggccgatc gttgtgaaat tcaggtttcc 900

tacgcaatcg gcgtggctga accgacctcc atcatggtag aaactttcgg tactgagaaa 960

gtgccttctg aacaactgac cctgctggta cgtgagttct tcgacctgcg cccatacggt 1020

ctgattcaga tgctggatct gctgcacccg atctacaaag aaaccgcagc atacggtcac 1080

tttggtcgtg aacatttccc gtgggaaaaa accgacaaag cgcagctgct gcgcgatgct 1140

gccggtctga agtaa 1155

<210> 36
<211> 384
<212> PRT
<213> Escherichia coli

<400> 36

Met Ala Lys His Leu Phe Thr Ser Glu Ser Val Ser Glu Gly His Pro
1 5 10 15

```
Asp Lys Ile Ala Asp Gln Ile Ser Asp Ala Val Leu Asp Ala Ile Leu
            20                  25                  30

Glu Gln Asp Pro Lys Ala Arg Val Ala Cys Glu Thr Tyr Val Lys Thr
        35                  40                  45

Gly Met Val Leu Val Gly Gly Glu Ile Thr Thr Ser Ala Trp Val Asp
    50                  55                  60

Ile Glu Glu Ile Thr Arg Asn Thr Val Arg Glu Ile Gly Tyr Val His
65                  70                  75                  80

Ser Asp Met Gly Phe Asp Ala Asn Ser Cys Ala Val Leu Ser Ala Ile
                85                  90                  95

Gly Lys Gln Ser Pro Asp Ile Asn Gln Gly Val Asp Arg Ala Asp Pro
            100                 105                 110

Leu Glu Gln Gly Ala Gly Asp Gln Gly Leu Met Phe Gly Tyr Ala Thr
        115                 120                 125

Asn Glu Thr Asp Val Leu Met Pro Ala Pro Ile Thr Tyr Ala His Arg
    130                 135                 140

Leu Val Gln Arg Gln Ala Glu Val Arg Lys Asn Gly Thr Leu Pro Trp
145                 150                 155                 160

Leu Arg Pro Asp Ala Lys Ser Gln Val Thr Phe Gln Tyr Asp Asp Gly
                165                 170                 175

Lys Ile Val Gly Ile Asp Ala Val Val Leu Ser Thr Gln His Ser Glu
            180                 185                 190

Glu Ile Asp Gln Lys Ser Leu Gln Glu Ala Val Met Glu Glu Ile Ile
        195                 200                 205

Lys Pro Ile Leu Pro Ala Glu Trp Leu Thr Ser Ala Thr Lys Phe Phe
    210                 215                 220

Ile Asn Pro Thr Gly Arg Phe Val Ile Gly Gly Pro Met Gly Asp Cys
225                 230                 235                 240

Gly Leu Thr Gly Arg Lys Ile Ile Val Asp Thr Tyr Gly Gly Met Ala
                245                 250                 255

Arg His Gly Gly Gly Ala Phe Ser Gly Lys Asp Pro Ser Lys Val Asp
            260                 265                 270
```

```
Arg Ser Ala Ala Tyr Ala Ala Arg Tyr Val Ala Lys Asn Ile Val Ala
        275                 280                 285

Ala Gly Leu Ala Asp Arg Cys Glu Ile Gln Val Ser Tyr Ala Ile Gly
    290                 295                 300

Val Ala Glu Pro Thr Ser Ile Met Val Glu Thr Phe Gly Thr Glu Lys
305                 310                 315                 320

Val Pro Ser Glu Gln Leu Thr Leu Leu Val Arg Glu Phe Phe Asp Leu
                325                 330                 335

Arg Pro Tyr Gly Leu Ile Gln Met Leu Asp Leu Leu His Pro Ile Tyr
            340                 345                 350

Lys Glu Thr Ala Ala Tyr Gly His Phe Gly Arg Glu His Phe Pro Trp
        355                 360                 365

Glu Lys Thr Asp Lys Ala Gln Leu Leu Arg Asp Ala Ala Gly Leu Lys
    370                 375                 380


<210>  37
<211>  1155
<212>  DNA
<213>  Escherichia coli

<400>  37
atggcaaaac acctttttac gtccgagtcc gtctctgaag ggcatcctga caaaattgct      60

gaccaaattt ctgatgccgt tttagacgcg atcctcgaac aggatccgaa agcacgcgtt     120

gcttgcgaaa cctacgtaaa aaccggcatg gttttagttg gcggcgaaat caccaccagc     180

gcctgggtag acatcgaaga gatcacccgt aacaccgttc gcgaaattgg ctatgtgcat     240

tccgacatgg gctttgacgc taactcctgt gcggttctga gcgctatcgg caaacagtct     300

cctgacatca accagggcgt tgaccgtgcc gatccgctgg aacagggcgc gggtgaccag     360

ggtctgatgt ttggctacgc aactaatgaa accgacgtgc tgatgccagc acctatcacc     420

tatgcacacc gtctggtaca gcgtcaggct gaagtgcgta aaaacggcac tctgccgtgg     480

ctgcgcccgg acgcgaaaag ccaggtgact tttcagtatg acgacggcaa aatcgttggt     540

atcgatgctg tcgtgctttc cactcagcac tctgaagaga tcgaccagaa atcgctgcaa     600

gaagcggtaa tggaagagat catcaagcca attctgcccg ctgaatggct gacttctgcc     660

accaaattct tcatcaaccc gatcggtcgt ttcgttatcg gtggcccaat gggtgactgc     720

ggtctgactg gtcgtaaaat tatcgttgat acctacggcg gcatggcgcg tcacggtggc     780

ggtgcattct ctggtaaaga tccatcaaaa gtggaccgtt ccgcagccta cgcagcacgt     840
```

```
tatgtcgcga aaaacatcgt tgctgctggc ctggccgatc gttgtgaaat tcaggtttcc      900

tacgcaatcg gcgtggctga accgacctcc atcatggtag aaactttcgg tactgagaaa      960

gtgccttctg aacaactgac cctgctggta cgtgagttct tcgacctgcg cccatacggt     1020

ctgattcaga tgctggatct gctgcacccg atctacaaag aaaccgcagc atacggtcac     1080

tttggtcgtg aacatttccc gtgggaaaaa accgacaaag cgcagctgct gcgcgatgct     1140

gccggtctga agtaa                                                      1155


<210>  38
<211>  384
<212>  PRT
<213>  Escherichia coli

<400>  38

Met Ala Lys His Leu Phe Thr Ser Glu Ser Val Ser Glu Gly His Pro
1               5                   10                  15

Asp Lys Ile Ala Asp Gln Ile Ser Asp Ala Val Leu Asp Ala Ile Leu
            20                  25                  30

Glu Gln Asp Pro Lys Ala Arg Val Ala Cys Glu Thr Tyr Val Lys Thr
        35                  40                  45

Gly Met Val Leu Val Gly Gly Glu Ile Thr Thr Ser Ala Trp Val Asp
    50                  55                  60

Ile Glu Glu Ile Thr Arg Asn Thr Val Arg Glu Ile Gly Tyr Val His
65                  70                  75                  80

Ser Asp Met Gly Phe Asp Ala Asn Ser Cys Ala Val Leu Ser Ala Ile
                85                  90                  95

Gly Lys Gln Ser Pro Asp Ile Asn Gln Gly Val Asp Arg Ala Asp Pro
            100                 105                 110

Leu Glu Gln Gly Ala Gly Asp Gln Gly Leu Met Phe Gly Tyr Ala Thr
        115                 120                 125

Asn Glu Thr Asp Val Leu Met Pro Ala Pro Ile Thr Tyr Ala His Arg
    130                 135                 140

Leu Val Gln Arg Gln Ala Glu Val Arg Lys Asn Gly Thr Leu Pro Trp
145                 150                 155                 160

Leu Arg Pro Asp Ala Lys Ser Gln Val Thr Phe Gln Tyr Asp Asp Gly
                165                 170                 175
```

```
Lys Ile Val Gly Ile Asp Ala Val Val Leu Ser Thr Gln His Ser Glu
            180                 185                 190

Glu Ile Asp Gln Lys Ser Leu Gln Glu Ala Val Met Glu Glu Ile Ile
        195                 200                 205

Lys Pro Ile Leu Pro Ala Glu Trp Leu Thr Ser Ala Thr Lys Phe Phe
    210                 215                 220

Ile Asn Pro Ile Gly Arg Phe Val Ile Gly Gly Pro Met Gly Asp Cys
225                 230                 235                 240

Gly Leu Thr Gly Arg Lys Ile Ile Val Asp Thr Tyr Gly Gly Met Ala
                245                 250                 255

Arg His Gly Gly Gly Ala Phe Ser Gly Lys Asp Pro Ser Lys Val Asp
            260                 265                 270

Arg Ser Ala Ala Tyr Ala Ala Arg Tyr Val Ala Lys Asn Ile Val Ala
        275                 280                 285

Ala Gly Leu Ala Asp Arg Cys Glu Ile Gln Val Ser Tyr Ala Ile Gly
    290                 295                 300

Val Ala Glu Pro Thr Ser Ile Met Val Glu Thr Phe Gly Thr Glu Lys
305                 310                 315                 320

Val Pro Ser Glu Gln Leu Thr Leu Leu Val Arg Glu Phe Phe Asp Leu
                325                 330                 335

Arg Pro Tyr Gly Leu Ile Gln Met Leu Asp Leu Leu His Pro Ile Tyr
            340                 345                 350

Lys Glu Thr Ala Ala Tyr Gly His Phe Gly Arg Glu His Phe Pro Trp
        355                 360                 365

Glu Lys Thr Asp Lys Ala Gln Leu Leu Arg Asp Ala Ala Gly Leu Lys
    370                 375                 380

<210>  39
<211>  1155
<212>  DNA
<213>  Escherichia coli

<400>  39
atggcaaaac acctttttac gtccgagtcc gtctctgaag ggcatcctga caaaattgct      60

gaccaaattt ctgatgccgt tttagacgcg atcctcgaac aggatccgaa agcacgcgtt      120
```

gcttgcgaaa cctacgtaaa aaccggcatg gttttagttg gcggcgaaat caccaccagc 180

gcctgggtag acatcgaaga gatcacccgt aacaccgttc gcgaaattgg ctatgtgcat 240

tccgacatgg gctttgacgc taactcctgt gcggttctga gcgctatcgg caaacagtct 300

cctgacatca accagggcgt tgaccgtgcc gatccgctgg aacagggcgc gggtgaccag 360

ggtctgatgt ttggctacgc aactaatgaa accgacgtgc tgatgccagc acctatcacc 420

tatgcatacc gtctggtaca gcgtcaggct gaagtgcgta aaaacggcac tctgccgtgg 480

ctgcgcccgg acgcgaaaag ccaggtgact tttcagtatg acgacggcaa aatcgttggt 540

atcgatgctg tcgtgctttc cactcagcac tctgaagaga tcgaccagaa atcgctgcaa 600

gaagcggtaa tggaagagat catcaagcca attctgcccg ctgaatggct gacttctgcc 660

accaaattct tcatcaaccc gaccggtcgt ttcgttatcg gtggcccaat gggtgactgc 720

ggtctgactg gtcgtaaaat tatcgttgat acctacggcg gcatggcgcg tcacggtggc 780

ggtgcattct ctggtaaaga tccatcaaaa gtggaccgtt ccgcagccta cgcagcacgt 840

tatgtcgcga aaaacatcgt tgctgctggc ctggccgatc gttgtgaaat tcaggtttcc 900

tacgcaatcg gcgtggctga accgacctcc atcatggtag aaactttcgg tactgagaaa 960

gtgccttctg aacaactgac cctgctggta cgtgagttct tcgacctgcg cccatacggt 1020

ctgattcaga tgctggatct gctgcacccg atctacaaag aaaccgcagc atacggtcac 1080

tttggtcgtg aacatttccc gtgggaaaaa accgacaaag cgcagctgct gcgcgatgct 1140

gccggtctga agtaa 1155

<210> 40
<211> 384
<212> PRT
<213> Escherichia coli

<400> 40

Met Ala Lys His Leu Phe Thr Ser Glu Ser Val Ser Glu Gly His Pro
1 5 10 15

Asp Lys Ile Ala Asp Gln Ile Ser Asp Ala Val Leu Asp Ala Ile Leu
20 25 30

Glu Gln Asp Pro Lys Ala Arg Val Ala Cys Glu Thr Tyr Val Lys Thr
35 40 45

Gly Met Val Leu Val Gly Gly Glu Ile Thr Thr Ser Ala Trp Val Asp
50 55 60

Ile Glu Glu Ile Thr Arg Asn Thr Val Arg Glu Ile Gly Tyr Val His
65 70 75 80

```
Ser Asp Met Gly Phe Asp Ala Asn Ser Cys Ala Val Leu Ser Ala Ile
                85                  90                  95

Gly Lys Gln Ser Pro Asp Ile Asn Gln Gly Val Asp Arg Ala Asp Pro
            100                 105                 110

Leu Glu Gln Gly Ala Gly Asp Gln Gly Leu Met Phe Gly Tyr Ala Thr
        115                 120                 125

Asn Glu Thr Asp Val Leu Met Pro Ala Pro Ile Thr Tyr Ala Tyr Arg
    130                 135                 140

Leu Val Gln Arg Gln Ala Glu Val Arg Lys Asn Gly Thr Leu Pro Trp
145                 150                 155                 160

Leu Arg Pro Asp Ala Lys Ser Gln Val Thr Phe Gln Tyr Asp Asp Gly
                165                 170                 175

Lys Ile Val Gly Ile Asp Ala Val Val Leu Ser Thr Gln His Ser Glu
            180                 185                 190

Glu Ile Asp Gln Lys Ser Leu Gln Glu Ala Val Met Glu Glu Ile Ile
        195                 200                 205

Lys Pro Ile Leu Pro Ala Glu Trp Leu Thr Ser Ala Thr Lys Phe Phe
    210                 215                 220

Ile Asn Pro Thr Gly Arg Phe Val Ile Gly Gly Pro Met Gly Asp Cys
225                 230                 235                 240

Gly Leu Thr Gly Arg Lys Ile Ile Val Asp Thr Tyr Gly Gly Met Ala
                245                 250                 255

Arg His Gly Gly Gly Ala Phe Ser Gly Lys Asp Pro Ser Lys Val Asp
            260                 265                 270

Arg Ser Ala Ala Tyr Ala Ala Arg Tyr Val Ala Lys Asn Ile Val Ala
        275                 280                 285

Ala Gly Leu Ala Asp Arg Cys Glu Ile Gln Val Ser Tyr Ala Ile Gly
    290                 295                 300

Val Ala Glu Pro Thr Ser Ile Met Val Glu Thr Phe Gly Thr Glu Lys
305                 310                 315                 320

Val Pro Ser Glu Gln Leu Thr Leu Leu Val Arg Glu Phe Phe Asp Leu
                325                 330                 335
```

```
Arg Pro Tyr Gly Leu Ile Gln Met Leu Asp Leu Leu His Pro Ile Tyr
            340                 345                 350

Lys Glu Thr Ala Ala Tyr Gly His Phe Gly Arg Glu His Phe Pro Trp
        355                 360                 365

Glu Lys Thr Asp Lys Ala Gln Leu Leu Arg Asp Ala Ala Gly Leu Lys
    370                 375                 380


<210>  41
<211>  1161
<212>  DNA
<213>  Escherichia coli

<400>  41
atgacgcgta aacaggccac catcgcagtg cgtagcgggt taaatgacga cgaacagtat      60

ggttgcgttg tcccaccgat ccatctttcc agcacctata actttaccgg atttaatgaa     120

ccgcgcgcgc atgattactc gcgtcgcggc aacccaacgc gcgatgtggt tcagcgtgcg     180

ctggcagaac tggaaggtgg tgctggtgca gtacttacta ataccggcat gtccgcgatt     240

cacctggtaa cgaccgtctt tttgaaacct ggcgatctgc tggttgcgcc gcacgactgc     300

tacggcggta gctatcgcct gttcgacagt ctggcgaaac gcggttgcta tcgcgtgttg     360

tttgttgatc aaggcgatga acaggcatta cgggcagcgc tggcagaaaa acccaaactg     420

gtactggtag aaagcccaag taatccattg ttacgcgtcg tggatattgc gaaaatctgc     480

catctggcaa gggaagtcgg ggcggtgagc gtggtggata acaccttctt aagcccggca     540

ttacaaaatc cgctggcatt aggtgccgat ctggtgttgc attcatgcac gaaatatctg     600

aacggtcact cagacgtagt ggccggcgtg gtgattgcta aagacccgga cgttgtcact     660

gaactggcct ggtgggcaaa caatattggc gtgacgggcg gcgcgtttga cagctatctg     720

ctgctacgtg ggttgcgaac gctggtgccg cgtatggagc tggcgcagcg caacgcgcag     780

gcgattgtga aatacctgca aacccagccg ttggtgaaaa aactgtatca cccgtcgttg     840

ccggaaaatc aggggcatga aattgccgcg cgccagcaaa aaggctttgg cgcaatgttg     900

agttttgaac tggatggcga tgagcagacg ctgcgtcgtt tcctgggcgg gctgtcgttg     960

tttacgctgg cggaatcatt agggggagtg gaaagtttaa tctctcacgc cgcaaccatg    1020

acacatgcag gcatggcacc agaagcgcgt gctgccgccg ggatctccga gacgctgctg    1080

cgtatctcca ccggtattga agatggcgaa gatttaattg ccgacctgga aatggcttc     1140

cgggctgcaa acaaggggta a                                               1161


<210>  42
<211>  386
```

<212> PRT
<213> Escherichia coli

<400> 42

Met Thr Arg Lys Gln Ala Thr Ile Ala Val Arg Ser Gly Leu Asn Asp
1 5 10 15

Asp Glu Gln Tyr Gly Cys Val Val Pro Pro Ile His Leu Ser Ser Thr
20 25 30

Tyr Asn Phe Thr Gly Phe Asn Glu Pro Arg Ala His Asp Tyr Ser Arg
35 40 45

Arg Gly Asn Pro Thr Arg Asp Val Val Gln Arg Ala Leu Ala Glu Leu
50 55 60

Glu Gly Gly Ala Gly Ala Val Leu Thr Asn Thr Gly Met Ser Ala Ile
65 70 75 80

His Leu Val Thr Thr Val Phe Leu Lys Pro Gly Asp Leu Leu Val Ala
85 90 95

Pro His Asp Cys Tyr Gly Gly Ser Tyr Arg Leu Phe Asp Ser Leu Ala
100 105 110

Lys Arg Gly Cys Tyr Arg Val Leu Phe Val Asp Gln Gly Asp Glu Gln
115 120 125

Ala Leu Arg Ala Ala Leu Ala Glu Lys Pro Lys Leu Val Leu Val Glu
130 135 140

Ser Pro Ser Asn Pro Leu Leu Arg Val Val Asp Ile Ala Lys Ile Cys
145 150 155 160

His Leu Ala Arg Glu Val Gly Ala Val Ser Val Val Asp Asn Thr Phe
165 170 175

Leu Ser Pro Ala Leu Gln Asn Pro Leu Ala Leu Gly Ala Asp Leu Val
180 185 190

Leu His Ser Cys Thr Lys Tyr Leu Asn Gly His Ser Asp Val Val Ala
195 200 205

Gly Val Val Ile Ala Lys Asp Pro Asp Val Val Thr Glu Leu Ala Trp
210 215 220

Trp Ala Asn Asn Ile Gly Val Thr Gly Gly Ala Phe Asp Ser Tyr Leu
225 230 235 240

Leu Leu Arg Gly Leu Arg Thr Leu Val Pro Arg Met Glu Leu Ala Gln
245 250 255

Arg Asn Ala Gln Ala Ile Val Lys Tyr Leu Gln Thr Gln Pro Leu Val
260 265 270

Lys Lys Leu Tyr His Pro Ser Leu Pro Glu Asn Gln Gly His Glu Ile
275 280 285

Ala Ala Arg Gln Gln Lys Gly Phe Gly Ala Met Leu Ser Phe Glu Leu
290 295 300

Asp Gly Asp Glu Gln Thr Leu Arg Arg Phe Leu Gly Gly Leu Ser Leu
305 310 315 320

Phe Thr Leu Ala Glu Ser Leu Gly Gly Val Glu Ser Leu Ile Ser His
325 330 335

Ala Ala Thr Met Thr His Ala Gly Met Ala Pro Glu Ala Arg Ala Ala
340 345 350

Ala Gly Ile Ser Glu Thr Leu Leu Arg Ile Ser Thr Gly Ile Glu Asp
355 360 365

Gly Glu Asp Leu Ile Ala Asp Leu Glu Asn Gly Phe Arg Ala Ala Asn
370 375 380

Lys Gly
385

<210> 43
<211> 1188
<212> DNA
<213> Escherichia coli

<400> 43
atggcggaca aaaagcttga tactcaactg gtgaatgcag gacgcagcaa aaaatacact 60

ctcggcgcgg taaatagcgt gattcagcgc gcttcttcgc tggtctttga cagtgtagaa 120

gccaaaaaac acgcgacacg taatcgcgcc aatggagagt tgttctatgg acggcgcgga 180

acgttaaccc atttctcctt acaacaagcg atgtgtgaac tggaaggtgg cgcaggctgc 240

gtgctatttc cctgcggggc ggcagcggtt gctaattcca ttcttgcttt tatcgaacag 300

ggcgatcatg tgttgatgac caacaccgcc tatgaaccga gtcaggattt ctgtagcaaa 360

atcctcagca aactgggcgt aacgacatca tggtttgatc cgctgattgg tgccgatatc 420

gttaagcatc tgcagccaaa cactaaaatc gtgtttctgg aatcgccagg ctccatcacc 480

atggaagtcc acgacgttcc ggcgattgtt gccgccgtac gcagtgtggt gccggatgcc 540

atcattatga tcgacaacac ctgggcagcc ggtgtgctgt ttaaggcgct ggattttggc 600

atcgatgttt ctattcaagc cgccaccaaa tatctggttg ggcattcaga tgcgatgatt 660

ggcactgccg tgtgcaatgc ccgttgctgg gagcagctac gggaaaatgc ctatctgatg 720

ggccagatgg tcgatgccga taccgcctat ataaccagcc gtggcctgcg cacattaggt 780

gtgcgtttgc gtcaacatca tgaaagcagt ctgaaagtgg ctgaatggct ggcagaacat 840

ccgcaagttg cgcgagttaa ccaccctgct ctgcctggca gtaaaggtca cgaattctgg 900

aaacgagact ttacaggcag cagcgggcta ttttcctttg tgcttaagaa aaaactcaat 960

aatgaagagc tggcgaacta tctggataac ttcagtttat tcagcatggc ctactcgtgg 1020

ggcgggtatg aatcgttgat cctggcaaat caaccagaac atatcgccgc cattcgccca 1080

caaggcgaga tcgattttag cgggaccttg attcgcctgc atattggtct ggaagatgtc 1140

gacgatctga ttgccgatct ggacgccggt tttgcgcgaa ttgtataa 1188

<210> 44
<211> 395
<212> PRT
<213> Escherichia coli

<400> 44

Met Ala Asp Lys Lys Leu Asp Thr Gln Leu Val Asn Ala Gly Arg Ser
1 5 10 15

Lys Lys Tyr Thr Leu Gly Ala Val Asn Ser Val Ile Gln Arg Ala Ser
20 25 30

Ser Leu Val Phe Asp Ser Val Glu Ala Lys Lys His Ala Thr Arg Asn
35 40 45

Arg Ala Asn Gly Glu Leu Phe Tyr Gly Arg Arg Gly Thr Leu Thr His
50 55 60

Phe Ser Leu Gln Gln Ala Met Cys Glu Leu Glu Gly Gly Ala Gly Cys
65 70 75 80

Val Leu Phe Pro Cys Gly Ala Ala Ala Val Ala Asn Ser Ile Leu Ala
85 90 95

Phe Ile Glu Gln Gly Asp His Val Leu Met Thr Asn Thr Ala Tyr Glu
100 105 110

Pro Ser Gln Asp Phe Cys Ser Lys Ile Leu Ser Lys Leu Gly Val Thr
115 120 125

Thr Ser Trp Phe Asp Pro Leu Ile Gly Ala Asp Ile Val Lys His Leu
130 135 140

Gln Pro Asn Thr Lys Ile Val Phe Leu Glu Ser Pro Gly Ser Ile Thr
145 150 155 160

Met Glu Val His Asp Val Pro Ala Ile Val Ala Ala Val Arg Ser Val
165 170 175

Val Pro Asp Ala Ile Ile Met Ile Asp Asn Thr Trp Ala Ala Gly Val
180 185 190

Leu Phe Lys Ala Leu Asp Phe Gly Ile Asp Val Ser Ile Gln Ala Ala
195 200 205

Thr Lys Tyr Leu Val Gly His Ser Asp Ala Met Ile Gly Thr Ala Val
210 215 220

Cys Asn Ala Arg Cys Trp Glu Gln Leu Arg Glu Asn Ala Tyr Leu Met
225 230 235 240

Gly Gln Met Val Asp Ala Asp Thr Ala Tyr Ile Thr Ser Arg Gly Leu
245 250 255

Arg Thr Leu Gly Val Arg Leu Arg Gln His His Glu Ser Ser Leu Lys
260 265 270

Val Ala Glu Trp Leu Ala Glu His Pro Gln Val Ala Arg Val Asn His
275 280 285

Pro Ala Leu Pro Gly Ser Lys Gly His Glu Phe Trp Lys Arg Asp Phe
290 295 300

Thr Gly Ser Ser Gly Leu Phe Ser Phe Val Leu Lys Lys Lys Leu Asn
305 310 315 320

Asn Glu Glu Leu Ala Asn Tyr Leu Asp Asn Phe Ser Leu Phe Ser Met
325 330 335

Ala Tyr Ser Trp Gly Gly Tyr Glu Ser Leu Ile Leu Ala Asn Gln Pro
340 345 350

Glu His Ile Ala Ala Ile Arg Pro Gln Gly Glu Ile Asp Phe Ser Gly
355 360 365

Thr Leu Ile Arg Leu His Ile Gly Leu Glu Asp Val Asp Asp Leu Ile

370 375 380

Ala Asp Leu Asp Ala Gly Phe Ala Arg Ile Val
385 390 395

<210> 45
<211> 3684
<212> DNA
<213> Escherichia coli

<400> 45
atgagcagca aagtggaaca actgcgtgcg cagttaaatg aacgtattct ggtgctggac 60

ggcggtgcgg gtaccatgat ccagagttat cgactgaacg aagccgattt tcgtggtgaa 120

cgctttgccg actggccatg cgacctcaaa ggcaacaacg acctgctggt actcagtaaa 180

ccggaagtga tcgccgctat ccacaacgcc tactttgaag cgggcgcgga tatcatcgaa 240

accaacacct tcaactccac gaccattgcg atggcggatt accagatgga atccctgtcg 300

gcggaaatca actttgcggc ggcgaaactg gcgcgagctt gtgctgacga gtggaccgcg 360

cgcacgccag agaaaccgcg ctacgttgcc ggtgttctcg gcccgaccaa ccgcacggcg 420

tctatttctc cggacgtcaa cgatccggca tttcgtaata tcacttttga cgggctggtg 480

gcggcttatc gagagtccac caaagcgctg gtggaaggtg gcgcggatct gatcctgatt 540

gaaaccgttt tcgacaccct taacgccaaa gcggcggtat ttgcggtgaa aacggagttt 600

gaagcgctgg gcgttgagct gccgattatg atctccggca ccatcaccga cgcctccggg 660

cgcacgctct ccgggcagac caccgaagca ttttacaact cattgcgcca cgccgaagct 720

ctgacctttg gcctgaactg tgcgctgggg cccgatgaac tgcgccagta cgtgcaggag 780

ctgtcacgga ttgcggaatg ctacgtcacc gcgcacccga acgccgggct acccaacgcc 840

tttggtgagt acgatctcga cgccgacacg atggcaaaac agatacgtga atgggcgcaa 900

gcgggttttc tcaatatcgt cggcggctgc tgtggcacca cgccacaaca tattgcagcg 960

atgagtcgtg cagtagaagg attagcgccg cgcaaactgc cggaaattcc cgtagcctgc 1020

cgtttgtccg gcctggagcc gctgaacatt ggcgaagata gcctgtttgt gaacgtgggt 1080

gaacgcacca acgtcaccgg ttccgctaag ttcaagcgcc tgatcaaaga agagaaatac 1140

agcgaggcgc tggatgtcgc gcgtcaacag gtggaaaacg gcgcgcagat tatcgatatc 1200

aacatggatg aagggatgct cgatgccgaa gcggcgatgg tgcgttttct caatctgatt 1260

gccggtgaac cggatatcgc tcgcgtgccg attatgatcg actcctcaaa atgggacgtc 1320

attgaaaaag gtctgaagtg tatccagggc aaaggcattg ttaactctat ctcgatgaaa 1380

gagggcgtcg atgcctttat ccatcacgcg aaattgttgc gtcgctacgg tgcggcagtg 1440

gtggtaatgg cctttgacga acagggacag gccgatactc gcgcacggaa aatcgagatt 1500

tgccgtcggg cgtacaaaat cctcaccgaa gaggttggct tcccgccaga agatatcatc 1560

ttcgacccaa acatcttcgc ggtcgcaact ggcattgaag agcacaacaa ctacgcgcag 1620

gactttatcg gcgcgtgtga agacatcaaa cgcgaactgc cgcacgcgct gatttccggc 1680

ggcgtatcta acgtttcttt ctcgttccgt ggcaacgatc cggtgcgcga agccattcac 1740

gcagtgttcc tctactacgc tattcgcaat ggcatggata tggggatcgt caacgccggg 1800

caactggcga tttacgacga cctacccgct gaactgcgcg acgcggtgga agatgtgatt 1860

cttaatcgtc gcgacgatgg caccgagcgt ttactggagc ttgccgagaa atatcgcggc 1920

agcaaaaccg acgacaccgc caacgcccag caggcggagt ggcgctcgtg ggaagtgaat 1980

aaacgtctgg aatactcgct ggtcaaaggc attaccgagt ttatcgagca ggataccgaa 2040

gaagcccgcc agcaggctac gcgcccgatt gaagtgattg aaggcccgtt gatggacggc 2100

atgaatgtgg tcggcgacct gtttggcgaa gggaaaatgt tcctgccaca ggtggtcaaa 2160

tcggcgcgcg tcatgaaaca ggcggtggcc tacctcgaac cgtttattga agccagcaaa 2220

gagcagggca aaaccaacgg caagatggtg atcgccaccg tgaagggcga cgtccacgac 2280

atcggtaaaa atatcgttgg tgtggtgctg caatgtaaca actacgaaat tgtcgatctc 2340

ggcgttatgg tgcctgcgga aaaaattctc cgtaccgcta aagaagtgaa tgctgatctg 2400

attggccttt cggggcttat cacgccgtcg ctggacgaga tggttaacgt ggcgaaagag 2460

atggagcgtc agggcttcac tattccgtta ctgattggcg gcgcgacgac ctcaaaagcg 2520

cacacggcgg tgaaaatcga gcagaactac agcggcccga cggtgtatgt gcagaatgcc 2580

tcgcgtaccg ttggtgtggt ggcggcgctg ctttccgata cccagcgtga tgattttgtc 2640

gctcgtaccc gcaaggagta cgaaaccgta cgtattcagc acgggcgcaa gaaaccgcgc 2700

acaccaccgg tcacgctgga agcggcgcgc gataacgatt tcgcttttga ctggcaggct 2760

tacacgccgc cggtggcgca ccgtctcggc gtgcaggaag tcgaagccag catcgaaacg 2820

ctgcgtaatt acatcgactg gacaccgttc tttatgacct ggtcgctggc cgggaagtat 2880

ccgcgcattc tggaagatga agtggtgggc gttgaggcgc agcggctgtt taaagacgcc 2940

aacgacatgc tggataaatt aagcgccgag aaaacgctga atccgcgtgg cgtggtgggc 3000

ctgttcccgg caaaccgtgt gggcgatgac attgaaatct accgtgacga aacgcgtacc 3060

catgtgatca acgtcagcca ccatctgcgt caacagaccg aaaaaacagg cttcgctaac 3120

tactgtctcg ctgacttcgt tgcgccgaag ctttctggta aagcagatta catcggcgca 3180

tttgccgtga ctggcgggct ggaagaggac gcactggctg atgcctttga agcgcagcac 3240

gatgattaca acaaaatcat ggtgaaagcg cttgccgacc gtttagccga agcctttgcg 3300

gagtatctcc atgagcgtgt gcgtaaagtc tactggggct atgcgccgaa cgagaacctc 3360

agcaacgaag agctgatccg cgaaaactac cagggcatcc gtccggcacc gggctatccg 3420

gcctgcccgg aacatacgga aaaagccacc atctgggagc tgctggaagt ggaaaaacac 3480

actggcatga aactcacaga atctttcgcc atgtggcccg gtgcatcggt ttcgggttgg 3540

tacttcagcc acccggacag caagtactac gctgtagcac aaattcagcg cgatcaggtt 3600

gaagattatg cccgccgtaa aggtatgagc gttaccgaag ttgagcgctg gctggcaccg 3660

aatctggggt atgacgcgga ctga 3684

<210> 46
<211> 1227
<212> PRT
<213> Escherichia coli

<400> 46

Met Ser Ser Lys Val Glu Gln Leu Arg Ala Gln Leu Asn Glu Arg Ile
1 5 10 15

Leu Val Leu Asp Gly Gly Ala Gly Thr Met Ile Gln Ser Tyr Arg Leu
20 25 30

Asn Glu Ala Asp Phe Arg Gly Glu Arg Phe Ala Asp Trp Pro Cys Asp
35 40 45

Leu Lys Gly Asn Asn Asp Leu Leu Val Leu Ser Lys Pro Glu Val Ile
50 55 60

Ala Ala Ile His Asn Ala Tyr Phe Glu Ala Gly Ala Asp Ile Ile Glu
65 70 75 80

Thr Asn Thr Phe Asn Ser Thr Thr Ile Ala Met Ala Asp Tyr Gln Met
85 90 95

Glu Ser Leu Ser Ala Glu Ile Asn Phe Ala Ala Ala Lys Leu Ala Arg
100 105 110

Ala Cys Ala Asp Glu Trp Thr Ala Arg Thr Pro Glu Lys Pro Arg Tyr
115 120 125

Val Ala Gly Val Leu Gly Pro Thr Asn Arg Thr Ala Ser Ile Ser Pro
130 135 140

Asp Val Asn Asp Pro Ala Phe Arg Asn Ile Thr Phe Asp Gly Leu Val
145 150 155 160

Ala Ala Tyr Arg Glu Ser Thr Lys Ala Leu Val Glu Gly Gly Ala Asp
165 170 175

Leu Ile Leu Ile Glu Thr Val Phe Asp Thr Leu Asn Ala Lys Ala Ala
180 185 190

Val Phe Ala Val Lys Thr Glu Phe Glu Ala Leu Gly Val Glu Leu Pro
195 200 205

Ile Met Ile Ser Gly Thr Ile Thr Asp Ala Ser Gly Arg Thr Leu Ser
210 215 220

Gly Gln Thr Thr Glu Ala Phe Tyr Asn Ser Leu Arg His Ala Glu Ala
225 230 235 240

Leu Thr Phe Gly Leu Asn Cys Ala Leu Gly Pro Asp Glu Leu Arg Gln
245 250 255

Tyr Val Gln Glu Leu Ser Arg Ile Ala Glu Cys Tyr Val Thr Ala His
260 265 270

Pro Asn Ala Gly Leu Pro Asn Ala Phe Gly Glu Tyr Asp Leu Asp Ala
275 280 285

Asp Thr Met Ala Lys Gln Ile Arg Glu Trp Ala Gln Ala Gly Phe Leu
290 295 300

Asn Ile Val Gly Gly Cys Cys Gly Thr Thr Pro Gln His Ile Ala Ala
305 310 315 320

Met Ser Arg Ala Val Glu Gly Leu Ala Pro Arg Lys Leu Pro Glu Ile
325 330 335

Pro Val Ala Cys Arg Leu Ser Gly Leu Glu Pro Leu Asn Ile Gly Glu
340 345 350

Asp Ser Leu Phe Val Asn Val Gly Glu Arg Thr Asn Val Thr Gly Ser
355 360 365

Ala Lys Phe Lys Arg Leu Ile Lys Glu Glu Lys Tyr Ser Glu Ala Leu
370 375 380

Asp Val Ala Arg Gln Gln Val Glu Asn Gly Ala Gln Ile Ile Asp Ile
385 390 395 400

Asn Met Asp Glu Gly Met Leu Asp Ala Glu Ala Ala Met Val Arg Phe
405 410 415

Leu Asn Leu Ile Ala Gly Glu Pro Asp Ile Ala Arg Val Pro Ile Met
420 425 430

```
Ile Asp Ser Ser Lys Trp Asp Val Ile Glu Lys Gly Leu Lys Cys Ile
        435                 440                 445

Gln Gly Lys Gly Ile Val Asn Ser Ile Ser Met Lys Glu Gly Val Asp
    450                 455                 460

Ala Phe Ile His His Ala Lys Leu Leu Arg Arg Tyr Gly Ala Ala Val
465                 470                 475                 480

Val Val Met Ala Phe Asp Glu Gln Gly Gln Ala Asp Thr Arg Ala Arg
                485                 490                 495

Lys Ile Glu Ile Cys Arg Arg Ala Tyr Lys Ile Leu Thr Glu Glu Val
            500                 505                 510

Gly Phe Pro Pro Glu Asp Ile Ile Phe Asp Pro Asn Ile Phe Ala Val
        515                 520                 525

Ala Thr Gly Ile Glu Glu His Asn Asn Tyr Ala Gln Asp Phe Ile Gly
    530                 535                 540

Ala Cys Glu Asp Ile Lys Arg Glu Leu Pro His Ala Leu Ile Ser Gly
545                 550                 555                 560

Gly Val Ser Asn Val Ser Phe Ser Phe Arg Gly Asn Asp Pro Val Arg
                565                 570                 575

Glu Ala Ile His Ala Val Phe Leu Tyr Tyr Ala Ile Arg Asn Gly Met
            580                 585                 590

Asp Met Gly Ile Val Asn Ala Gly Gln Leu Ala Ile Tyr Asp Asp Leu
        595                 600                 605

Pro Ala Glu Leu Arg Asp Ala Val Glu Asp Val Ile Leu Asn Arg Arg
    610                 615                 620

Asp Asp Gly Thr Glu Arg Leu Leu Glu Leu Ala Glu Lys Tyr Arg Gly
625                 630                 635                 640

Ser Lys Thr Asp Asp Thr Ala Asn Ala Gln Gln Ala Glu Trp Arg Ser
                645                 650                 655

Trp Glu Val Asn Lys Arg Leu Glu Tyr Ser Leu Val Lys Gly Ile Thr
            660                 665                 670

Glu Phe Ile Glu Gln Asp Thr Glu Glu Ala Arg Gln Gln Ala Thr Arg
        675                 680                 685
```

```
Pro Ile Glu Val Ile Glu Gly Pro Leu Met Asp Gly Met Asn Val Val
    690                 695                 700

Gly Asp Leu Phe Gly Glu Gly Lys Met Phe Leu Pro Gln Val Val Lys
705                 710                 715                 720

Ser Ala Arg Val Met Lys Gln Ala Val Ala Tyr Leu Glu Pro Phe Ile
                725                 730                 735

Glu Ala Ser Lys Glu Gln Gly Lys Thr Asn Gly Lys Met Val Ile Ala
            740                 745                 750

Thr Val Lys Gly Asp Val His Asp Ile Gly Lys Asn Ile Val Gly Val
        755                 760                 765

Val Leu Gln Cys Asn Asn Tyr Glu Ile Val Asp Leu Gly Val Met Val
    770                 775                 780

Pro Ala Glu Lys Ile Leu Arg Thr Ala Lys Glu Val Asn Ala Asp Leu
785                 790                 795                 800

Ile Gly Leu Ser Gly Leu Ile Thr Pro Ser Leu Asp Glu Met Val Asn
                805                 810                 815

Val Ala Lys Glu Met Glu Arg Gln Gly Phe Thr Ile Pro Leu Leu Ile
            820                 825                 830

Gly Gly Ala Thr Thr Ser Lys Ala His Thr Ala Val Lys Ile Glu Gln
        835                 840                 845

Asn Tyr Ser Gly Pro Thr Val Tyr Val Gln Asn Ala Ser Arg Thr Val
    850                 855                 860

Gly Val Val Ala Ala Leu Leu Ser Asp Thr Gln Arg Asp Asp Phe Val
865                 870                 875                 880

Ala Arg Thr Arg Lys Glu Tyr Glu Thr Val Arg Ile Gln His Gly Arg
                885                 890                 895

Lys Lys Pro Arg Thr Pro Pro Val Thr Leu Glu Ala Ala Arg Asp Asn
            900                 905                 910

Asp Phe Ala Phe Asp Trp Gln Ala Tyr Thr Pro Pro Val Ala His Arg
        915                 920                 925

Leu Gly Val Gln Glu Val Glu Ala Ser Ile Glu Thr Leu Arg Asn Tyr
```

930 935 940

Ile Asp Trp Thr Pro Phe Phe Met Thr Trp Ser Leu Ala Gly Lys Tyr
945 950 955 960

Pro Arg Ile Leu Glu Asp Glu Val Val Gly Val Glu Ala Gln Arg Leu
965 970 975

Phe Lys Asp Ala Asn Asp Met Leu Asp Lys Leu Ser Ala Glu Lys Thr
980 985 990

Leu Asn Pro Arg Gly Val Val Gly Leu Phe Pro Ala Asn Arg Val Gly
995 1000 1005

Asp Asp Ile Glu Ile Tyr Arg Asp Glu Thr Arg Thr His Val Ile
1010 1015 1020

Asn Val Ser His His Leu Arg Gln Gln Thr Glu Lys Thr Gly Phe
1025 1030 1035

Ala Asn Tyr Cys Leu Ala Asp Phe Val Ala Pro Lys Leu Ser Gly
1040 1045 1050

Lys Ala Asp Tyr Ile Gly Ala Phe Ala Val Thr Gly Gly Leu Glu
1055 1060 1065

Glu Asp Ala Leu Ala Asp Ala Phe Glu Ala Gln His Asp Asp Tyr
1070 1075 1080

Asn Lys Ile Met Val Lys Ala Leu Ala Asp Arg Leu Ala Glu Ala
1085 1090 1095

Phe Ala Glu Tyr Leu His Glu Arg Val Arg Lys Val Tyr Trp Gly
1100 1105 1110

Tyr Ala Pro Asn Glu Asn Leu Ser Asn Glu Glu Leu Ile Arg Glu
1115 1120 1125

Asn Tyr Gln Gly Ile Arg Pro Ala Pro Gly Tyr Pro Ala Cys Pro
1130 1135 1140

Glu His Thr Glu Lys Ala Thr Ile Trp Glu Leu Leu Glu Val Glu
1145 1150 1155

Lys His Thr Gly Met Lys Leu Thr Glu Ser Phe Ala Met Trp Pro
1160 1165 1170

Gly Ala Ser Val Ser Gly Trp Tyr Phe Ser His Pro Asp Ser Lys
1175 1180 1185

Tyr Tyr Ala Val Ala Gln Ile Gln Arg Asp Gln Val Glu Asp Tyr
1190 1195 1200

Ala Arg Arg Lys Gly Met Ser Val Thr Glu Val Glu Arg Trp Leu
1205 1210 1215

Ala Pro Asn Leu Gly Tyr Asp Ala Asp
1220 1225

<210> 47
<211> 1104
<212> DNA
<213> Escherichia coli

<400> 47
atgaaaaatg ttggttttat cggctggcgc ggtatggtcg gctccgttct catgcaacgc 60

atggttgaag agcgcgactt cgacgccatt cgccctgtct tcttttctac ttctcagctt 120

ggccaggctg cgccgtcttt tggcggaacc actggcacac ttcaggatgc ctttgatctg 180

gaggcgctaa aggccctcga tatcattgtg acctgtcagg gcggcgatta taccaacgaa 240

atctatccaa agcttcgtga aagcggatgg caaggttact ggattgacgc agcatcgtct 300

ctgcgcatga aagatgacgc catcatcatt cttgaccccg tcaatcagga cgtcattacc 360

gacggattaa ataatggcat caggactttt gttggcggta actgtaccgt aagcctgatg 420

ttgatgtcgt tgggtggttt attcgccaat gatcttgttg attgggtgtc cgttgcaacc 480

taccaggccg cttccggcgg tggtgcgcga catatgcgtg agttattaac ccagatgggc 540

catctgtatg gccatgtggc agatgaactc gcgaccccgt cctctgctat tctcgatatc 600

gaacgcaaag tcacaacctt aacccgtagc ggtgagctgc cggtggataa ctttggcgtg 660

ccgctggcgg gtagcctgat tccgtggatc gacaaacagc tcgataacgg tcagagccgc 720

gaagagtgga aggggcaggc ggaaaccaac aagatcctca acacatcttc cgtaattccg 780

gtagatggtt tatgtgtgcg tgtcggggca ttgcgctgcc acagccaggc attcactatt 840

aaattgaaaa aagatgtgtc tattccgacc gtggaagaac tgctggctgc gcacaatccg 900

tgggcgaaag tcgttccgaa cgatcgggaa atcactatgc gtgagctaac cccagctgcc 960

gttaccggca cgctgaccac gccggtaggc cgcctgcgta agctgaatat gggaccagag 1020

ttcctgtcag cctttaccgt gggcgaccag ctgctgtggg gggccgcgga gccgctgcgt 1080

cggatgcttc gtcaactggc gtaa 1104

<210> 48
<211> 367

<212> PRT
<213> Escherichia coli

<400> 48

Met Lys Asn Val Gly Phe Ile Gly Trp Arg Gly Met Val Gly Ser Val
1               5                   10                  15

Leu Met Gln Arg Met Val Glu Glu Arg Asp Phe Asp Ala Ile Arg Pro
            20                  25                  30

Val Phe Phe Ser Thr Ser Gln Leu Gly Gln Ala Ala Pro Ser Phe Gly
        35                  40                  45

Gly Thr Thr Gly Thr Leu Gln Asp Ala Phe Asp Leu Glu Ala Leu Lys
    50                  55                  60

Ala Leu Asp Ile Ile Val Thr Cys Gln Gly Gly Asp Tyr Thr Asn Glu
65                  70                  75                  80

Ile Tyr Pro Lys Leu Arg Glu Ser Gly Trp Gln Gly Tyr Trp Ile Asp
                85                  90                  95

Ala Ala Ser Ser Leu Arg Met Lys Asp Asp Ala Ile Ile Ile Leu Asp
            100                 105                 110

Pro Val Asn Gln Asp Val Ile Thr Asp Gly Leu Asn Asn Gly Ile Arg
        115                 120                 125

Thr Phe Val Gly Gly Asn Cys Thr Val Ser Leu Met Leu Met Ser Leu
    130                 135                 140

Gly Gly Leu Phe Ala Asn Asp Leu Val Asp Trp Val Ser Val Ala Thr
145                 150                 155                 160

Tyr Gln Ala Ala Ser Gly Gly Gly Ala Arg His Met Arg Glu Leu Leu
                165                 170                 175

Thr Gln Met Gly His Leu Tyr Gly His Val Ala Asp Glu Leu Ala Thr
            180                 185                 190

Pro Ser Ser Ala Ile Leu Asp Ile Glu Arg Lys Val Thr Thr Leu Thr
        195                 200                 205

Arg Ser Gly Glu Leu Pro Val Asp Asn Phe Gly Val Pro Leu Ala Gly
    210                 215                 220

Ser Leu Ile Pro Trp Ile Asp Lys Gln Leu Asp Asn Gly Gln Ser Arg
225                 230                 235                 240

Glu Glu Trp Lys Gly Gln Ala Glu Thr Asn Lys Ile Leu Asn Thr Ser
245 250 255

Ser Val Ile Pro Val Asp Gly Leu Cys Val Arg Val Gly Ala Leu Arg
260 265 270

Cys His Ser Gln Ala Phe Thr Ile Lys Leu Lys Lys Asp Val Ser Ile
275 280 285

Pro Thr Val Glu Glu Leu Leu Ala Ala His Asn Pro Trp Ala Lys Val
290 295 300

Val Pro Asn Asp Arg Glu Ile Thr Met Arg Glu Leu Thr Pro Ala Ala
305 310 315 320

Val Thr Gly Thr Leu Thr Thr Pro Val Gly Arg Leu Arg Lys Leu Asn
325 330 335

Met Gly Pro Glu Phe Leu Ser Ala Phe Thr Val Gly Asp Gln Leu Leu
340 345 350

Trp Gly Ala Ala Glu Pro Leu Arg Arg Met Leu Arg Gln Leu Ala
355 360 365

<210> 49
<211> 1350
<212> DNA
<213> Escherichia coli

<400> 49
atgtctgaaa ttgttgtctc caaatttggc ggtaccagcg tagctgattt tgacgccatg 60

aaccgcagcg ctgatattgt gctttctgat gccaacgtgc gtttagttgt cctctcggct 120

tctgctggta tcactaatct gctggtcgct ttagctgaag gactggaacc tggcgagcga 180

ttcgaaaaac tcgacgctat ccgcaacatc cagtttgcca ttctggaacg tctgcgttac 240

ccgaacgtta tccgtgaaga gattgaacgt ctgctggaga acattactgt tctggcagaa 300

gcggcggcgc tggcaacgtc tccggcgctg acagatgagc tggtcagcca cggcgagctg 360

atgtcgaccc tgctgtttgt tgagatcctg cgcgaacgcg atgttcaggc acagtggttt 420

gatgtacgta aagtgatgcg taccaacgac cgatttggtc gtgcagagcc agatatagcc 480

gcgctggcgg aactggccgc gctgcagctg ctcccacgtc tcaatgaagg cttagtgatc 540

acccagggat ttatcggtag cgaaaataaa ggtcgtacaa cgacgcttgg ccgtggaggc 600

agcgattata cggcagcctt gctggcggag gctttacacg catctcgtgt tgatatctgg 660

accgacgtcc cgggcatcta caccaccgat ccacgcgtag tttccgcagc aaaacgcatt 720

gatgaaatcg cgtttgccga agcggcagag atggcaactt ttggtgcaaa agtactgcat 780

ccggcaacgt tgctacccgc agtacgcagc gatatcccgg tctttgtcgg ctccagcaaa 840

gacccacgcg caggtggtac gctggtgtgc aataaaactg aaaatccgcc gctgttccgc 900

gctctggcgc ttcgtcgcaa tcagactctg ctcactttgc acagcctgaa tatgctgcat 960

tctcgcggtt tcctcgcgga agttttcggc atcctcgcgc ggcataatat ttcggtagac 1020

ttaatcacca cgtcagaagt gagcgtggca ttaacccttg ataccaccgg ttcaacctcc 1080

actggcgata cgttgctgac gcaatctctg ctgatggagc tttccgcact gtgtcgggtg 1140

gaggtggaag aaggtctggc gctggtcgcg ttgattggca atgacctgtc aaaagcctgc 1200

ggcgttggca aagaggtatt cggcgtactg gaaccgttca acattcgcat gatttgttat 1260

ggcgcatcca gccataacct gtgcttcctg gtgcccggcg aagatgccga gcaggtggtg 1320

caaaaactgc atagtaattt gtttgagtaa 1350

<210> 50
<211> 449
<212> PRT
<213> Escherichia coli

<400> 50

Met Ser Glu Ile Val Val Ser Lys Phe Gly Gly Thr Ser Val Ala Asp
1 5 10 15

Phe Asp Ala Met Asn Arg Ser Ala Asp Ile Val Leu Ser Asp Ala Asn
20 25 30

Val Arg Leu Val Val Leu Ser Ala Ser Ala Gly Ile Thr Asn Leu Leu
35 40 45

Val Ala Leu Ala Glu Gly Leu Glu Pro Gly Glu Arg Phe Glu Lys Leu
50 55 60

Asp Ala Ile Arg Asn Ile Gln Phe Ala Ile Leu Glu Arg Leu Arg Tyr
65 70 75 80

Pro Asn Val Ile Arg Glu Glu Ile Glu Arg Leu Leu Glu Asn Ile Thr
85 90 95

Val Leu Ala Glu Ala Ala Ala Leu Ala Thr Ser Pro Ala Leu Thr Asp
100 105 110

Glu Leu Val Ser His Gly Glu Leu Met Ser Thr Leu Leu Phe Val Glu
115 120 125

```
Ile Leu Arg Glu Arg Asp Val Gln Ala Gln Trp Phe Asp Val Arg Lys
    130                 135                 140

Val Met Arg Thr Asn Asp Arg Phe Gly Arg Ala Glu Pro Asp Ile Ala
145                 150                 155                 160

Ala Leu Ala Glu Leu Ala Ala Leu Gln Leu Leu Pro Arg Leu Asn Glu
                165                 170                 175

Gly Leu Val Ile Thr Gln Gly Phe Ile Gly Ser Glu Asn Lys Gly Arg
            180                 185                 190

Thr Thr Thr Leu Gly Arg Gly Gly Ser Asp Tyr Thr Ala Ala Leu Leu
        195                 200                 205

Ala Glu Ala Leu His Ala Ser Arg Val Asp Ile Trp Thr Asp Val Pro
    210                 215                 220

Gly Ile Tyr Thr Thr Asp Pro Arg Val Val Ser Ala Ala Lys Arg Ile
225                 230                 235                 240

Asp Glu Ile Ala Phe Ala Glu Ala Ala Glu Met Ala Thr Phe Gly Ala
                245                 250                 255

Lys Val Leu His Pro Ala Thr Leu Leu Pro Ala Val Arg Ser Asp Ile
            260                 265                 270

Pro Val Phe Val Gly Ser Ser Lys Asp Pro Arg Ala Gly Gly Thr Leu
        275                 280                 285

Val Cys Asn Lys Thr Glu Asn Pro Pro Leu Phe Arg Ala Leu Ala Leu
    290                 295                 300

Arg Arg Asn Gln Thr Leu Leu Thr Leu His Ser Leu Asn Met Leu His
305                 310                 315                 320

Ser Arg Gly Phe Leu Ala Glu Val Phe Gly Ile Leu Ala Arg His Asn
                325                 330                 335

Ile Ser Val Asp Leu Ile Thr Thr Ser Glu Val Ser Val Ala Leu Thr
            340                 345                 350

Leu Asp Thr Thr Gly Ser Thr Ser Thr Gly Asp Thr Leu Leu Thr Gln
        355                 360                 365

Ser Leu Leu Met Glu Leu Ser Ala Leu Cys Arg Val Glu Val Glu Glu
    370                 375                 380
```

Gly Leu Ala Leu Val Ala Leu Ile Gly Asn Asp Leu Ser Lys Ala Cys
385 390 395 400

Gly Val Gly Lys Glu Val Phe Gly Val Leu Glu Pro Phe Asn Ile Arg
405 410 415

Met Ile Cys Tyr Gly Ala Ser Ser His Asn Leu Cys Phe Leu Val Pro
420 425 430

Gly Glu Asp Ala Glu Gln Val Val Gln Lys Leu His Ser Asn Leu Phe
435 440 445

Glu

<210> 51
<211> 2433
<212> DNA
<213> Escherichia coli

<400> 51
atgagtgtga ttgcgcaggc aggggcgaaa ggtcgtcagc tgcataaatt tggtggcagt 60

agtctggctg atgtgaagtg ttatttgcgt gtcgcgggca ttatggcgga gtactctcag 120

cctgacgata tgatggtggt ttccgccgcc ggtagcacca ctaaccagtt gattaactgg 180

ttgaaactaa gccagaccga tcgtctctct gcgcatcagg ttcaacaaac gctgcgtcgc 240

tatcagtgcg atctgattag cggtctgcta cccgctgaag aagccgatag cctcattagc 300

gcttttgtca gcgaccttga gcgcctggcg gcgctgctcg acagcggtat taacgacgca 360

gtgtatgcgg aagtggtggg ccacggggaa gtatggtcgg cacgtctgat gtctgcggta 420

cttaatcaac aagggctgcc agcggcctgg cttgatgccc gcgagttttt acgcgctgaa 480

cgcgccgcac aaccgcaggt tgatgaaggg ctttcttacc cgttgctgca acagctgctg 540

gtgcaacatc cgggcaaacg tctggtggtg accggattta tcagccgcaa caacgccggt 600

gaaacggtgc tgctggggcg taacggttcc gactattccg cgacacaaat cggtgcgctg 660

gcgggtgttt ctcgcgtaac catctggagc gacgtcgccg ggtatacag tgccgacccg 720

cgtaaagtga aagatgcctg cctgctgccg ttgctgcgtc tggatgaggc cagcgaactg 780

gcgcgcctgg cggctcccgt tcttcacgcc cgtactttac agccggtttc tggcagcgaa 840

atcgacctgc aactgcgctg tagctacacg ccggatcaag gttccacgcg cattgaacgc 900

gtgctggcct ccggtactgg tgcgcgtatt gtcaccagcc acgatgatgt ctgtttgatt 960

gagtttcagg tgcccgccag tcaggatttc aaactggcgc ataaagagat cgaccaaatc 1020

ctgaaacgcg cgcaggtacg cccgctggcg gttggcgtac ataacgatcg ccagttgctg 1080

caattttgct acacctcaga agtggccgac agtgcgctga aaatcctcga cgaagcggga 1140

ttacctggcg aactgcgcct gcgtcagggg ctggcgctgg tggcgatggt cggtgcaggc 1200

gtcacccgta acccgctgca ttgccaccgc ttctggcagc aactgaaagg ccagccggtc 1260

gaatttacct ggcagtccga tgacggcatc agcctggtgg cagtactgcg caccggcccg 1320

accgaaagcc tgattcaggg gctgcatcag tccgtcttcc gcgcagaaaa acgcatcggc 1380

ctggtattgt tcggtaaggg caatatcggt tcccgttggc tggaactgtt cgcccgtgag 1440

cagagcacgc tttcggcacg taccggcttt gagtttgtgc tggcaggtgt ggtggacagc 1500

cgccgcagcc tgttgagcta tgacgggctg gacgccagcc gcgcgttagc cttcttcaac 1560

gatgaagcgg ttgagcagga tgaagagtcg ttgttcctgt ggatgcgcgc ccatccgtat 1620

gatgatttag tggtgctgga cgttaccgcc agccagcagc ttgctgatca gtatcttgat 1680

ttcgccagcc acggtttcca cgttatcagc gccaacaaac tggcgggagc cagcgacagc 1740

aataaatatc gccagatcca cgacgccttc gaaaaaaccg ggcgtcactg gctgtacaat 1800

gccaccgtcg gtgcgggctt gccgatcaac cacaccgtgc gcgatctgat cgacagcggc 1860

gatactattt tgtcgatcag cgggatcttc tccggcacgc tctcctggct gttcctgcaa 1920

ttcgacggta gcgtgccgtt taccgagctg gtggatcagg cgtggcagca gggcttaacc 1980

gaacctgacc cgcgtgacga tctctctggc aaagacgtga tgcgcaagct ggtgattctg 2040

gcgcgtgaag caggttacaa catcgaaccg gatcaggtac gtgtggaatc gctggtgcct 2100

gctcattgcg aaggcggcag catcgaccat ttctttgaaa atggcgatga actgaacgag 2160

cagatggtgc aacggctgga agcggcccgc gaaatggggc tggtgctgcg ctacgtggcg 2220

cgtttcgatg ccaacggtaa agcgcgtgta ggcgtggaag cggtgcgtga agatcatccg 2280

ttggcatcac tgctgccgtg cgataacgtc tttgccatcg aaagccgctg gtatcgcgat 2340

aaccctctgg tgatccgcgg acctggcgct gggcgcgacg tcaccgccgg ggcgattcag 2400

tcggatatca accggctggc acagttgttg taa 2433

<210> 52
<211> 810
<212> PRT
<213> Escherichia coli

<400> 52

Met Ser Val Ile Ala Gln Ala Gly Ala Lys Gly Arg Gln Leu His Lys
1 5 10 15

Phe Gly Gly Ser Ser Leu Ala Asp Val Lys Cys Tyr Leu Arg Val Ala
20 25 30

Gly Ile Met Ala Glu Tyr Ser Gln Pro Asp Asp Met Met Val Val Ser

```
        35                  40                  45

Ala Ala Gly Ser Thr Thr Asn Gln Leu Ile Asn Trp Leu Lys Leu Ser
    50                  55                  60

Gln Thr Asp Arg Leu Ser Ala His Gln Val Gln Gln Thr Leu Arg Arg
65                  70                  75                  80

Tyr Gln Cys Asp Leu Ile Ser Gly Leu Leu Pro Ala Glu Glu Ala Asp
                85                  90                  95

Ser Leu Ile Ser Ala Phe Val Ser Asp Leu Glu Arg Leu Ala Ala Leu
            100                 105                 110

Leu Asp Ser Gly Ile Asn Asp Ala Val Tyr Ala Glu Val Val Gly His
        115                 120                 125

Gly Glu Val Trp Ser Ala Arg Leu Met Ser Ala Val Leu Asn Gln Gln
    130                 135                 140

Gly Leu Pro Ala Ala Trp Leu Asp Ala Arg Glu Phe Leu Arg Ala Glu
145                 150                 155                 160

Arg Ala Ala Gln Pro Gln Val Asp Glu Gly Leu Ser Tyr Pro Leu Leu
                165                 170                 175

Gln Gln Leu Leu Val Gln His Pro Gly Lys Arg Leu Val Val Thr Gly
            180                 185                 190

Phe Ile Ser Arg Asn Asn Ala Gly Glu Thr Val Leu Leu Gly Arg Asn
        195                 200                 205

Gly Ser Asp Tyr Ser Ala Thr Gln Ile Gly Ala Leu Ala Gly Val Ser
    210                 215                 220

Arg Val Thr Ile Trp Ser Asp Val Ala Gly Val Tyr Ser Ala Asp Pro
225                 230                 235                 240

Arg Lys Val Lys Asp Ala Cys Leu Leu Pro Leu Leu Arg Leu Asp Glu
                245                 250                 255

Ala Ser Glu Leu Ala Arg Leu Ala Ala Pro Val Leu His Ala Arg Thr
            260                 265                 270

Leu Gln Pro Val Ser Gly Ser Glu Ile Asp Leu Gln Leu Arg Cys Ser
        275                 280                 285
```

```
Tyr Thr Pro Asp Gln Gly Ser Thr Arg Ile Glu Arg Val Leu Ala Ser
    290                 295                 300

Gly Thr Gly Ala Arg Ile Val Thr Ser His Asp Asp Val Cys Leu Ile
305                 310                 315                 320

Glu Phe Gln Val Pro Ala Ser Gln Asp Phe Lys Leu Ala His Lys Glu
                325                 330                 335

Ile Asp Gln Ile Leu Lys Arg Ala Gln Val Arg Pro Leu Ala Val Gly
            340                 345                 350

Val His Asn Asp Arg Gln Leu Leu Gln Phe Cys Tyr Thr Ser Glu Val
        355                 360                 365

Ala Asp Ser Ala Leu Lys Ile Leu Asp Glu Ala Gly Leu Pro Gly Glu
    370                 375                 380

Leu Arg Leu Arg Gln Gly Leu Ala Leu Val Ala Met Val Gly Ala Gly
385                 390                 395                 400

Val Thr Arg Asn Pro Leu His Cys His Arg Phe Trp Gln Gln Leu Lys
                405                 410                 415

Gly Gln Pro Val Glu Phe Thr Trp Gln Ser Asp Asp Gly Ile Ser Leu
            420                 425                 430

Val Ala Val Leu Arg Thr Gly Pro Thr Glu Ser Leu Ile Gln Gly Leu
        435                 440                 445

His Gln Ser Val Phe Arg Ala Glu Lys Arg Ile Gly Leu Val Leu Phe
    450                 455                 460

Gly Lys Gly Asn Ile Gly Ser Arg Trp Leu Glu Leu Phe Ala Arg Glu
465                 470                 475                 480

Gln Ser Thr Leu Ser Ala Arg Thr Gly Phe Glu Phe Val Leu Ala Gly
                485                 490                 495

Val Val Asp Ser Arg Arg Ser Leu Leu Ser Tyr Asp Gly Leu Asp Ala
            500                 505                 510

Ser Arg Ala Leu Ala Phe Phe Asn Asp Glu Ala Val Glu Gln Asp Glu
        515                 520                 525

Glu Ser Leu Phe Leu Trp Met Arg Ala His Pro Tyr Asp Asp Leu Val
    530                 535                 540
```

```
Val Leu Asp Val Thr Ala Ser Gln Gln Leu Ala Asp Gln Tyr Leu Asp
545                 550                 555                 560

Phe Ala Ser His Gly Phe His Val Ile Ser Ala Asn Lys Leu Ala Gly
                565                 570                 575

Ala Ser Asp Ser Asn Lys Tyr Arg Gln Ile His Asp Ala Phe Glu Lys
            580                 585                 590

Thr Gly Arg His Trp Leu Tyr Asn Ala Thr Val Gly Ala Gly Leu Pro
        595                 600                 605

Ile Asn His Thr Val Arg Asp Leu Ile Asp Ser Gly Asp Thr Ile Leu
    610                 615                 620

Ser Ile Ser Gly Ile Phe Ser Gly Thr Leu Ser Trp Leu Phe Leu Gln
625                 630                 635                 640

Phe Asp Gly Ser Val Pro Phe Thr Glu Leu Val Asp Gln Ala Trp Gln
                645                 650                 655

Gln Gly Leu Thr Glu Pro Asp Pro Arg Asp Asp Leu Ser Gly Lys Asp
            660                 665                 670

Val Met Arg Lys Leu Val Ile Leu Ala Arg Glu Ala Gly Tyr Asn Ile
        675                 680                 685

Glu Pro Asp Gln Val Arg Val Glu Ser Leu Val Pro Ala His Cys Glu
    690                 695                 700

Gly Gly Ser Ile Asp His Phe Phe Glu Asn Gly Asp Glu Leu Asn Glu
705                 710                 715                 720

Gln Met Val Gln Arg Leu Glu Ala Ala Arg Glu Met Gly Leu Val Leu
                725                 730                 735

Arg Tyr Val Ala Arg Phe Asp Ala Asn Gly Lys Ala Arg Val Gly Val
            740                 745                 750

Glu Ala Val Arg Glu Asp His Pro Leu Ala Ser Leu Leu Pro Cys Asp
        755                 760                 765

Asn Val Phe Ala Ile Glu Ser Arg Trp Tyr Arg Asp Asn Pro Leu Val
    770                 775                 780

Ile Arg Gly Pro Gly Ala Gly Arg Asp Val Thr Ala Gly Ala Ile Gln
785                 790                 795                 800
```

```
Ser Asp Ile Asn Arg Leu Ala Gln Leu Leu
                805                 810


<210>  53
<211>  1191
<212>  DNA
<213>  Escherichia coli

<400>  53
atgtttgaga acattaccgc cgctcctgcc gacccgattc tgggcctggc cgatctgttt      60

cgtgccgatg aacgtcccgg caaaattaac ctcgggattg gtgtctataa agatgagacg     120

ggcaaaaccc cggtactgac cagcgtgaaa aaggctgaac agtatctgct cgaaaatgaa     180

accaccaaaa attacctcgg cattgacggc atccctgaat ttggtcgctg cactcaggaa     240

ctgctgtttg gtaaaggtag cgccctgatc aatgacaaac gtgctcgcac ggcacagact     300

ccgggggggca ctggcgcact acgcgtggct gccgatttcc tggcaaaaaa taccagcgtt    360

aagcgtgtgt gggtgagcaa cccaagctgg ccgaaccata agagcgtctt taactctgca     420

ggtctggaag ttcgtgaata cgcttattat gatgcggaaa atcacactct tgacttcgat     480

gcactgatta acagcctgaa tgaagctcag gctggcgacg tagtgctgtt ccatggctgc     540

tgccataacc caaccggtat cgaccctacg ctggaacaat ggcaaacact ggcacaactc     600

tccgttgaga aaggctggtt accgctgttt gacttcgctt accagggttt tgcccgtggt     660

ctggaagaag atgctgaagg actgcgcgct ttcgcggcta tgcataaaga gctgattgtt     720

gccagttcct actctaaaaa ctttggcctg tacaacgagc gtgttggcgc ttgtactctg     780

gttgctgccg acagtgaaac cgttgatcgc gcattcagcc aaatgaaagc ggcgattcgc     840

gctaactact ctaacccacc agcacacggc gcttctgttg ttgccaccat cctgagcaac     900

gatgcgttac gtgcgatttg ggaacaagag ctgactgata tgcgccagcg tattcagcgt     960

atgcgtcagt tgttcgtcaa tacgctgcag gaaaaaggcg caaaccgcga cttcagcttt    1020

atcatcaaac agaacggcat gttctccttc agtggcctga caaaagaaca agtgctgcgt    1080

ctgcgcgaag agtttggcgt atatgcggtt gcttctggtc gcgtaaatgt ggccgggatg    1140

acaccagata acatggctcc gctgtgcgaa gcgattgtgg cagtgctgta a             1191


<210>  54
<211>  396
<212>  PRT
<213>  Escherichia coli

<400>  54

Met Phe Glu Asn Ile Thr Ala Ala Pro Ala Asp Pro Ile Leu Gly Leu
1               5                   10                  15
```

```
Ala Asp Leu Phe Arg Ala Asp Glu Arg Pro Gly Lys Ile Asn Leu Gly
            20                  25                  30

Ile Gly Val Tyr Lys Asp Glu Thr Gly Lys Thr Pro Val Leu Thr Ser
        35                  40                  45

Val Lys Lys Ala Glu Gln Tyr Leu Leu Glu Asn Glu Thr Thr Lys Asn
    50                  55                  60

Tyr Leu Gly Ile Asp Gly Ile Pro Glu Phe Gly Arg Cys Thr Gln Glu
65                  70                  75                  80

Leu Leu Phe Gly Lys Gly Ser Ala Leu Ile Asn Asp Lys Arg Ala Arg
                85                  90                  95

Thr Ala Gln Thr Pro Gly Gly Thr Gly Ala Leu Arg Val Ala Ala Asp
            100                 105                 110

Phe Leu Ala Lys Asn Thr Ser Val Lys Arg Val Trp Val Ser Asn Pro
        115                 120                 125

Ser Trp Pro Asn His Lys Ser Val Phe Asn Ser Ala Gly Leu Glu Val
    130                 135                 140

Arg Glu Tyr Ala Tyr Tyr Asp Ala Glu Asn His Thr Leu Asp Phe Asp
145                 150                 155                 160

Ala Leu Ile Asn Ser Leu Asn Glu Ala Gln Ala Gly Asp Val Val Leu
                165                 170                 175

Phe His Gly Cys Cys His Asn Pro Thr Gly Ile Asp Pro Thr Leu Glu
            180                 185                 190

Gln Trp Gln Thr Leu Ala Gln Leu Ser Val Glu Lys Gly Trp Leu Pro
        195                 200                 205

Leu Phe Asp Phe Ala Tyr Gln Gly Phe Ala Arg Gly Leu Glu Glu Asp
    210                 215                 220

Ala Glu Gly Leu Arg Ala Phe Ala Ala Met His Lys Glu Leu Ile Val
225                 230                 235                 240

Ala Ser Ser Tyr Ser Lys Asn Phe Gly Leu Tyr Asn Glu Arg Val Gly
                245                 250                 255

Ala Cys Thr Leu Val Ala Ala Asp Ser Glu Thr Val Asp Arg Ala Phe
            260                 265                 270
```

```
Ser Gln Met Lys Ala Ala Ile Arg Ala Asn Tyr Ser Asn Pro Pro Ala
        275                 280                 285

His Gly Ala Ser Val Val Ala Thr Ile Leu Ser Asn Asp Ala Leu Arg
    290                 295                 300

Ala Ile Trp Glu Gln Glu Leu Thr Asp Met Arg Gln Arg Ile Gln Arg
305                 310                 315                 320

Met Arg Gln Leu Phe Val Asn Thr Leu Gln Glu Lys Gly Ala Asn Arg
                325                 330                 335

Asp Phe Ser Phe Ile Ile Lys Gln Asn Gly Met Phe Ser Phe Ser Gly
            340                 345                 350

Leu Thr Lys Glu Gln Val Leu Arg Leu Arg Glu Glu Phe Gly Val Tyr
        355                 360                 365

Ala Val Ala Ser Gly Arg Val Asn Val Ala Gly Met Thr Pro Asp Asn
    370                 375                 380

Met Ala Pro Leu Cys Glu Ala Ile Val Ala Val Leu
385                 390                 395
```

<210> 55
<211> 822
<212> DNA
<213> Escherichia coli

<400> 55

```
atgtcgtgtg aagaactgga aattgtctgg aacaatatta aagccgaagc cagaacgctg      60

gcggactgtg agccaatgct ggccagtttt taccacgcga cgctactcaa gcacgaaaac     120

cttggcagtg cactgagcta catgctggcg aacaagctgt catcgccaat tatgcctgct     180

attgctatcc gtgaagtggt ggaagaagcc tacgccgctg acccggaaat gatcgcctct     240

gcggcctgtg atattcaggc ggtgcgtacc cgcgacccgg cagtcgataa atactcaacc     300

ccgttgttat acctgaaggg ttttcatgcc ttgcaggcct atcgcatcgg tcactggttg     360

tggaatcagg ggcgtcgcgc actggcaatc tttctgcaaa accaggtttc tgtgacgttc     420

caggtcgata ttcacccggc agcaaaaatt ggtcgcggta tcatgcttga ccacgcgaca     480

ggcatcgtcg ttggtgaaac ggcggtgatt gaaaacgacg tatcgattct gcaatctgtg     540

acgcttggcg gtacgggtaa atctggtggt gaccgtcacc cgaaaattcg tgaaggtgtg     600

atgattggcg cgggcgcgaa aatcctcggc aatattgaag ttgggcgcgg cgcgaagatt     660

ggcgcaggtt ccgtggtgct gcaaccggtg ccgccgcata ccaccgccgc tggcgttccg     720
```

gctcgtattg tcggtaaacc agacagcgat aagccatcaa tggatatgga ccagcatttc 780

aacggtatta accatacatt tgagtatggg gatgggatct aa 822

<210> 56
<211> 273
<212> PRT
<213> Escherichia coli

<400> 56

Met Ser Cys Glu Glu Leu Glu Ile Val Trp Asn Asn Ile Lys Ala Glu
1 5 10 15

Ala Arg Thr Leu Ala Asp Cys Glu Pro Met Leu Ala Ser Phe Tyr His
20 25 30

Ala Thr Leu Leu Lys His Glu Asn Leu Gly Ser Ala Leu Ser Tyr Met
35 40 45

Leu Ala Asn Lys Leu Ser Ser Pro Ile Met Pro Ala Ile Ala Ile Arg
50 55 60

Glu Val Val Glu Glu Ala Tyr Ala Ala Asp Pro Glu Met Ile Ala Ser
65 70 75 80

Ala Ala Cys Asp Ile Gln Ala Val Arg Thr Arg Asp Pro Ala Val Asp
85 90 95

Lys Tyr Ser Thr Pro Leu Leu Tyr Leu Lys Gly Phe His Ala Leu Gln
100 105 110

Ala Tyr Arg Ile Gly His Trp Leu Trp Asn Gln Gly Arg Arg Ala Leu
115 120 125

Ala Ile Phe Leu Gln Asn Gln Val Ser Val Thr Phe Gln Val Asp Ile
130 135 140

His Pro Ala Ala Lys Ile Gly Arg Gly Ile Met Leu Asp His Ala Thr
145 150 155 160

Gly Ile Val Val Gly Glu Thr Ala Val Ile Glu Asn Asp Val Ser Ile
165 170 175

Leu Gln Ser Val Thr Leu Gly Gly Thr Gly Lys Ser Gly Gly Asp Arg
180 185 190

His Pro Lys Ile Arg Glu Gly Val Met Ile Gly Ala Gly Ala Lys Ile
195 200 205

Leu Gly Asn Ile Glu Val Gly Arg Gly Ala Lys Ile Gly Ala Gly Ser
    210                 215                 220

Val Val Leu Gln Pro Val Pro Pro His Thr Thr Ala Ala Gly Val Pro
225                 230                 235                 240

Ala Arg Ile Val Gly Lys Pro Asp Ser Asp Lys Pro Ser Met Asp Met
                245                 250                 255

Asp Gln His Phe Asn Gly Ile Asn His Thr Phe Glu Tyr Gly Asp Gly
            260                 265                 270

Ile

<210> 57
<211> 1017
<212> DNA
<213> Escherichia coli

<400> 57
atggccgtta acttactgaa aaagaactca ctcgcgctgg tcgcttctct gctgctggcg 60

ggccatgtac aggcaacgga actgctgaac agttcttatg acgtctcccg cgagctgttt 120

gccgccctga atccgccgtt tgagcaacaa tgggcaaaag ataacggcgg cgacaaactg 180

acgataaaac aatctcatgc cgggtcatca aaacaggcgc tggcgatttt acagggctta 240

aaagccgacg ttgtcactta taaccaggtg accgacgtac aaatcctgca cgataaaggc 300

aagctgatcc cggccgactg gcagtcgcgc ctgccgaata atagctcgcc gttctactcc 360

accatgggct tcctggtgcg taagggtaac ccgaagaata tccacgattg gaacgacctg 420

gtgcgctccg acgtgaagct gattttcccg aacccgaaaa cgtcgggtaa cgcgcgttat 480

acctatctgg cggcatgggg cgcagcggat aaagctgacg gtggtgacaa aggcaaaacc 540

gaacagttta tgacccagtt cctgaaaaac gttgaagtgt tcgatactgg cggtcgtggc 600

gcgaccacca cttttgccga gcgcggcctg ggcgatgtgc tgattagctt cgaatcggaa 660

gtgaacaaca tccgtaaaca gtatgaagcg cagggctttg aagtggtgat tccgaaaacc 720

aacattctgg cggaattccc ggtggcgtgg gttgataaaa acgtgcaggc caacggtacg 780

gaaaaagccg ccaaagccta tctgaactgg ctctatagcc cgcaggcgca aaccatcatc 840

accgactatt actaccgcgt gaataacccg gaggtgatgg acaaactgaa agacaaattc 900

ccgcagaccg agctgttccg cgtggaagac aaatttggct cctggccgga agtgatgaaa 960

acccacttca ccagcggcgg cgagttagac aagctgttag cggcggggcg taactga 1017

<210> 58
<211> 338
<212> PRT
<213> Escherichia coli

<400> 58

Met Ala Val Asn Leu Leu Lys Lys Asn Ser Leu Ala Leu Val Ala Ser
1 5 10 15

Leu Leu Leu Ala Gly His Val Gln Ala Thr Glu Leu Leu Asn Ser Ser
20 25 30

Tyr Asp Val Ser Arg Glu Leu Phe Ala Ala Leu Asn Pro Pro Phe Glu
35 40 45

Gln Gln Trp Ala Lys Asp Asn Gly Gly Asp Lys Leu Thr Ile Lys Gln
50 55 60

Ser His Ala Gly Ser Ser Lys Gln Ala Leu Ala Ile Leu Gln Gly Leu
65 70 75 80

Lys Ala Asp Val Val Thr Tyr Asn Gln Val Thr Asp Val Gln Ile Leu
85 90 95

His Asp Lys Gly Lys Leu Ile Pro Ala Asp Trp Gln Ser Arg Leu Pro
100 105 110

Asn Asn Ser Ser Pro Phe Tyr Ser Thr Met Gly Phe Leu Val Arg Lys
115 120 125

Gly Asn Pro Lys Asn Ile His Asp Trp Asn Asp Leu Val Arg Ser Asp
130 135 140

Val Lys Leu Ile Phe Pro Asn Pro Lys Thr Ser Gly Asn Ala Arg Tyr
145 150 155 160

Thr Tyr Leu Ala Ala Trp Gly Ala Ala Asp Lys Ala Asp Gly Gly Asp
165 170 175

Lys Gly Lys Thr Glu Gln Phe Met Thr Gln Phe Leu Lys Asn Val Glu
180 185 190

Val Phe Asp Thr Gly Gly Arg Gly Ala Thr Thr Thr Phe Ala Glu Arg
195 200 205

Gly Leu Gly Asp Val Leu Ile Ser Phe Glu Ser Glu Val Asn Asn Ile
210 215 220

Arg Lys Gln Tyr Glu Ala Gln Gly Phe Glu Val Val Ile Pro Lys Thr
225 230 235 240

Asn Ile Leu Ala Glu Phe Pro Val Ala Trp Val Asp Lys Asn Val Gln
245 250 255

Ala Asn Gly Thr Glu Lys Ala Ala Lys Ala Tyr Leu Asn Trp Leu Tyr
260 265 270

Ser Pro Gln Ala Gln Thr Ile Ile Thr Asp Tyr Tyr Tyr Arg Val Asn
275 280 285

Asn Pro Glu Val Met Asp Lys Leu Lys Asp Lys Phe Pro Gln Thr Glu
290 295 300

Leu Phe Arg Val Glu Asp Lys Phe Gly Ser Trp Pro Glu Val Met Lys
305 310 315 320

Thr His Phe Thr Ser Gly Gly Glu Leu Asp Lys Leu Leu Ala Ala Gly
325 330 335

Arg Asn

<210> 59
<211> 834
<212> DNA
<213> Escherichia coli

<400> 59
atgtttgctg tctcctccag acgcgtgctg ccgggcttta ccttaagcct cggcaccagt 60

ctgctgtttg tgtgcctgat tttgctgctg ccgctctccg cgctggtgat gcaactggcc 120

cagatgagct gggcgcagta ctgggaggtg atcaccaacc cgcaggtggt cgcggcctac 180

aaagtaacgc tgctgtcggc gtttgtggca tcgattttta acggcgtttt cggtctgctg 240

atggcgtgga tcctaacccg ctatcgcttc ccaggccgca cgctgcttga tgcgctgatg 300

gatttaccct ttgcgctgcc aacggctgtc gccggtttaa cgctggcctc gctcttttcc 360

gtaaacggtt tttacggtga atggctggcg aagtttgata tcaaagtcac ctatacatgg 420

ctggggattg cggtggctat ggcctttacc agcattccgt ttgtggtgcg taccgtgcag 480

ccggtgctgg aagagttagg cccggaatat gaagaagcgg cggaaacgct tggtgcaacg 540

cgctggcaga gtttctgcaa agtggtgctg ccggagcttt ctccggcgct ggtggcgggc 600

gtggcgctgt cgtttacccg tagtcttggt gaatttggcg cggtgatttt tatcgccgga 660

aatatcgcgt ggaagacgga agtgacgtcg ctgatgattt ttgtgcgctt acaggagttt 720

gattacccgg cagcgagcgc gattgcttcg gtgatcctcg cggcatctct gctgctgctg 780

ttctcaatta acactctgca aagtcgcttt ggtcggcgtg tggtaggtca ttaa 834

<210> 60
<211> 277
<212> PRT
<213> Escherichia coli

<400> 60

```
Met Phe Ala Val Ser Ser Arg Arg Val Leu Pro Gly Phe Thr Leu Ser
1               5                   10                  15

Leu Gly Thr Ser Leu Leu Phe Val Cys Leu Ile Leu Leu Leu Pro Leu
            20                  25                  30

Ser Ala Leu Val Met Gln Leu Ala Gln Met Ser Trp Ala Gln Tyr Trp
        35                  40                  45

Glu Val Ile Thr Asn Pro Gln Val Val Ala Ala Tyr Lys Val Thr Leu
    50                  55                  60

Leu Ser Ala Phe Val Ala Ser Ile Phe Asn Gly Val Phe Gly Leu Leu
65                  70                  75                  80

Met Ala Trp Ile Leu Thr Arg Tyr Arg Phe Pro Gly Arg Thr Leu Leu
                85                  90                  95

Asp Ala Leu Met Asp Leu Pro Phe Ala Leu Pro Thr Ala Val Ala Gly
            100                 105                 110

Leu Thr Leu Ala Ser Leu Phe Ser Val Asn Gly Phe Tyr Gly Glu Trp
        115                 120                 125

Leu Ala Lys Phe Asp Ile Lys Val Thr Tyr Thr Trp Leu Gly Ile Ala
    130                 135                 140

Val Ala Met Ala Phe Thr Ser Ile Pro Phe Val Val Arg Thr Val Gln
145                 150                 155                 160

Pro Val Leu Glu Glu Leu Gly Pro Glu Tyr Glu Glu Ala Ala Glu Thr
                165                 170                 175

Leu Gly Ala Thr Arg Trp Gln Ser Phe Cys Lys Val Val Leu Pro Glu
            180                 185                 190

Leu Ser Pro Ala Leu Val Ala Gly Val Ala Leu Ser Phe Thr Arg Ser
        195                 200                 205
```

Leu Gly Glu Phe Gly Ala Val Ile Phe Ile Ala Gly Asn Ile Ala Trp
    210                 215                 220

Lys Thr Glu Val Thr Ser Leu Met Ile Phe Val Arg Leu Gln Glu Phe
225                 230                 235                 240

Asp Tyr Pro Ala Ala Ser Ala Ile Ala Ser Val Ile Leu Ala Ala Ser
                245                 250                 255

Leu Leu Leu Leu Phe Ser Ile Asn Thr Leu Gln Ser Arg Phe Gly Arg
            260                 265                 270

Arg Val Val Gly His
        275

<210> 61
<211> 876
<212> DNA
<213> Escherichia coli

<400> 61
atggcggaag ttacccaatt gaagcgttat gacgcgcgcc cgattaactg gggcaaatgg 60

tttctgattg gcatcgggat gctggtttcg gcgttcatcc tgctggtgcc gatgatttac 120

atcttcgtgc aggcattcag caaggggctg atgccggttt tacagaatct ggccgatccg 180

gacatgctgc acgccatctg gctgacggtg atgatcgcgc tgattgccgt accggtaaac 240

ctggtgttcg gcattctgct ggcctggctg gtgacgcgct ttaacttccc tggacgccag 300

ttactgctga cgctactgga cattccgttt gccgtatcgc cggtggttgc cggtctggtg 360

tatttgctgt tctacggctc taacggcccg ctcggcggtt ggctcgacga gcataacctg 420

caaattatgt tctcctggcc gggaatggtg ctggtcacca tcttcgtgac gtgtccgttt 480

gtggtgcgcg aactggtgcc ggtgatgtta agccagggca gccaggaaga cgaagcggcg 540

attttgcttg gcgcgtccgg ctggcagatg ttccgtcgcg tcacattacc gaacatccgc 600

tgggcgctgc tttatggcgt ggtgttgacc aacgcccgcg caattggcga gtttggcgcg 660

gtgtcggtgg tttccggctc gattcgcggc gaaaccctgt cgctgccgtt acagattgaa 720

ttgctggagc aggactacaa caccgtcggc tcctttaccg ctgcggcgct gttaacgctg 780

atggcgatta tcaccctgtt tttaaaaagt atgttgcagt ggcgcctgga gaatcaggaa 840

aaacgcgcac agcaggagga acatcatgag cattga 876

<210> 62
<211> 291
<212> PRT
<213> Escherichia coli

<400> 62

```
Met Ala Glu Val Thr Gln Leu Lys Arg Tyr Asp Ala Arg Pro Ile Asn
1               5                   10                  15

Trp Gly Lys Trp Phe Leu Ile Gly Ile Gly Met Leu Val Ser Ala Phe
            20                  25                  30

Ile Leu Leu Val Pro Met Ile Tyr Ile Phe Val Gln Ala Phe Ser Lys
        35                  40                  45

Gly Leu Met Pro Val Leu Gln Asn Leu Ala Asp Pro Asp Met Leu His
    50                  55                  60

Ala Ile Trp Leu Thr Val Met Ile Ala Leu Ile Ala Val Pro Val Asn
65                  70                  75                  80

Leu Val Phe Gly Ile Leu Leu Ala Trp Leu Val Thr Arg Phe Asn Phe
                85                  90                  95

Pro Gly Arg Gln Leu Leu Leu Thr Leu Leu Asp Ile Pro Phe Ala Val
            100                 105                 110

Ser Pro Val Val Ala Gly Leu Val Tyr Leu Leu Phe Tyr Gly Ser Asn
        115                 120                 125

Gly Pro Leu Gly Gly Trp Leu Asp Glu His Asn Leu Gln Ile Met Phe
    130                 135                 140

Ser Trp Pro Gly Met Val Leu Val Thr Ile Phe Val Thr Cys Pro Phe
145                 150                 155                 160

Val Val Arg Glu Leu Val Pro Val Met Leu Ser Gln Gly Ser Gln Glu
                165                 170                 175

Asp Glu Ala Ala Ile Leu Leu Gly Ala Ser Gly Trp Gln Met Phe Arg
            180                 185                 190

Arg Val Thr Leu Pro Asn Ile Arg Trp Ala Leu Leu Tyr Gly Val Val
        195                 200                 205

Leu Thr Asn Ala Arg Ala Ile Gly Glu Phe Gly Ala Val Ser Val Val
    210                 215                 220

Ser Gly Ser Ile Arg Gly Glu Thr Leu Ser Leu Pro Leu Gln Ile Glu
225                 230                 235                 240

Leu Leu Glu Gln Asp Tyr Asn Thr Val Gly Ser Phe Thr Ala Ala Ala
                245                 250                 255
```

Leu Leu Thr Leu Met Ala Ile Ile Thr Leu Phe Leu Lys Ser Met Leu
260 265 270

Gln Trp Arg Leu Glu Asn Gln Glu Lys Arg Ala Gln Gln Glu Glu His
275 280 285

His Glu His
290

<210> 63
<211> 1098
<212> DNA
<213> Escherichia coli

<400> 63
atgagcattg agattgccaa tattaagaag tcgtttggtc gcacccaggt gctgaacgat 60

atctcactgg atattccttc aggtcagatg gtcgcgttgc tggggccgtc cggttccggg 120

aaaaccacgc tgctgcgcat tatcgccggg ctggagcatc aaaccagcgg gcatattcgc 180

ttccacggca ccgacgtgag ccgcctgcac gcacgtgatc gtaaagtcgg tttcgtgttc 240

cagcattacg cgctgttccg ccatatgacg gtgttcgaca atatcgcttt tggcctgacg 300

gtgctgccgc gtcgcgagcg cccgaatgcc gcagccatca aagcgaaagt gacaaaattg 360

ctggaaatgg tccagcttgc ccatctggcg gatcgttatc cggcgcagct ttccggcggc 420

cagaaacagc gcgtggcgct ggcgcgcgcg ctggctgtgg aaccgcaaat tctgctgctt 480

gatgaaccgt ttggcgcgct ggatgcgcag gtgcgtaaag agctgcgtcg ctggctgcgt 540

caactccatg aagaactaaa attcaccagc gtttttgtga cccacgatca ggaagaagcg 600

accgaagtag ctgatcgtgt agttgtgatg agccagggca atattgaaca ggctgacgcg 660

ccggatcagg tatggcgcga accggcgacc cgttttgtgc tcgaatttat gggcgaagtg 720

aaccgcctgc agggaaccat tcgcggcggg cagttccatg ttggcgcgca tcgctggccg 780

ctgggctaca cacctgcgta tcaggggccg gtggatctct tcctgcgccc ttgggaagtg 840

gatatcagcc gccgtaccag cctcgattcg ccgctgccgg tacaggtact ggaagccagc 900

ccgaaaggtc actacaccca attagtggtg cagccgctgg ggtggtacaa cgaaccgctg 960

acggtcgtga tgcatggcga cgatgccccg cagcgtggcg agcgtttatt cgttggtctg 1020

caacatgcgc ggctgtataa cggcgacgag cgtatcgaaa cccgcgatga ggaacttgct 1080

ctcgcacaaa gcgcctga 1098

<210> 64
<211> 365
<212> PRT
<213> Escherichia coli

<400> 64

Met Ser Ile Glu Ile Ala Asn Ile Lys Lys Ser Phe Gly Arg Thr Gln
1 5 10 15

Val Leu Asn Asp Ile Ser Leu Asp Ile Pro Ser Gly Gln Met Val Ala
20 25 30

Leu Leu Gly Pro Ser Gly Ser Gly Lys Thr Thr Leu Leu Arg Ile Ile
35 40 45

Ala Gly Leu Glu His Gln Thr Ser Gly His Ile Arg Phe His Gly Thr
50 55 60

Asp Val Ser Arg Leu His Ala Arg Asp Arg Lys Val Gly Phe Val Phe
65 70 75 80

Gln His Tyr Ala Leu Phe Arg His Met Thr Val Phe Asp Asn Ile Ala
85 90 95

Phe Gly Leu Thr Val Leu Pro Arg Arg Glu Arg Pro Asn Ala Ala Ala
100 105 110

Ile Lys Ala Lys Val Thr Lys Leu Leu Glu Met Val Gln Leu Ala His
115 120 125

Leu Ala Asp Arg Tyr Pro Ala Gln Leu Ser Gly Gly Gln Lys Gln Arg
130 135 140

Val Ala Leu Ala Arg Ala Leu Ala Val Glu Pro Gln Ile Leu Leu Leu
145 150 155 160

Asp Glu Pro Phe Gly Ala Leu Asp Ala Gln Val Arg Lys Glu Leu Arg
165 170 175

Arg Trp Leu Arg Gln Leu His Glu Glu Leu Lys Phe Thr Ser Val Phe
180 185 190

Val Thr His Asp Gln Glu Glu Ala Thr Glu Val Ala Asp Arg Val Val
195 200 205

Val Met Ser Gln Gly Asn Ile Glu Gln Ala Asp Ala Pro Asp Gln Val
210 215 220

Trp Arg Glu Pro Ala Thr Arg Phe Val Leu Glu Phe Met Gly Glu Val
225 230 235 240

Asn Arg Leu Gln Gly Thr Ile Arg Gly Gly Gln Phe His Val Gly Ala
245 250 255

His Arg Trp Pro Leu Gly Tyr Thr Pro Ala Tyr Gln Gly Pro Val Asp
260 265 270

Leu Phe Leu Arg Pro Trp Glu Val Asp Ile Ser Arg Arg Thr Ser Leu
275 280 285

Asp Ser Pro Leu Pro Val Gln Val Leu Glu Ala Ser Pro Lys Gly His
290 295 300

Tyr Thr Gln Leu Val Val Gln Pro Leu Gly Trp Tyr Asn Glu Pro Leu
305 310 315 320

Thr Val Val Met His Gly Asp Asp Ala Pro Gln Arg Gly Glu Arg Leu
325 330 335

Phe Val Gly Leu Gln His Ala Arg Leu Tyr Asn Gly Asp Glu Arg Ile
340 345 350

Glu Thr Arg Asp Glu Glu Leu Ala Leu Ala Gln Ser Ala
355 360 365

<210> 65
<211> 912
<212> DNA
<213> Escherichia coli

<400> 65
gtgagtacat tagaacaaac aataggcaat acgcctctgg tgaagttgca gcgaatgggg 60

ccggataacg gcagtgaagt gtggttaaaa ctggaaggca ataacccggc aggttcggtg 120

aaagatcgtg cggcactttc gatgatcgtc gaggcggaaa agcgcgggga aattaaaccg 180

ggtgatgtct taatcgaagc caccagtggt aacaccggca ttgcgctggc aatgattgcc 240

gcgctgaaag gctatcgcat gaaattgctg atgcccgaca acatgagcca ggaacgccgt 300

gcggcgatgc gtgcttatgg tgcggaactg attcttgtca ccaaagagca gggcatggaa 360

ggtgcgcgcg atctggcgct ggagatggcg aatcgtggcg aaggaaagct gctcgatcag 420

ttcaataatc ccgataaccc ttatgcgcat tacaccacca ctgggccgga aatctggcag 480

caaaccggcg ggcgcatcac tcattttgtc tccagcatgg ggacgaccgg cactatcacc 540

ggcgtctcac gctttatgcg cgaacaatcc aaaccggtga ccattgtcgg cctgcaaccg 600

gaagagggca gcagcattcc cggcattcgc cgctggccta cggaatatct gccggggatt 660

ttcaacgctt ctctggtgga tgaggtgctg gatattcatc agcgcgatgc ggaaaacacc 720

atgcgcgaac tggcggtgcg ggaaggaata ttctgtggcg tcagctccgg cggcgcggtt 780

gccggagcac tgcgggtggc aaaagctaac cctgacgcgg tggtggtggc gatcatctgc 840

gatcgtggcg atcgctacct ttctaccggg gtgtttgggg aagagcattt tagccagggg 900

gcggggattt aa 912

<210> 66
<211> 303
<212> PRT
<213> Escherichia coli

<400> 66

Met Ser Thr Leu Glu Gln Thr Ile Gly Asn Thr Pro Leu Val Lys Leu
1 5 10 15

Gln Arg Met Gly Pro Asp Asn Gly Ser Glu Val Trp Leu Lys Leu Glu
20 25 30

Gly Asn Asn Pro Ala Gly Ser Val Lys Asp Arg Ala Ala Leu Ser Met
35 40 45

Ile Val Glu Ala Glu Lys Arg Gly Glu Ile Lys Pro Gly Asp Val Leu
50 55 60

Ile Glu Ala Thr Ser Gly Asn Thr Gly Ile Ala Leu Ala Met Ile Ala
65 70 75 80

Ala Leu Lys Gly Tyr Arg Met Lys Leu Leu Met Pro Asp Asn Met Ser
85 90 95

Gln Glu Arg Arg Ala Ala Met Arg Ala Tyr Gly Ala Glu Leu Ile Leu
100 105 110

Val Thr Lys Glu Gln Gly Met Glu Gly Ala Arg Asp Leu Ala Leu Glu
115 120 125

Met Ala Asn Arg Gly Glu Gly Lys Leu Leu Asp Gln Phe Asn Asn Pro
130 135 140

Asp Asn Pro Tyr Ala His Tyr Thr Thr Thr Gly Pro Glu Ile Trp Gln
145 150 155 160

Gln Thr Gly Gly Arg Ile Thr His Phe Val Ser Ser Met Gly Thr Thr
165 170 175

Gly Thr Ile Thr Gly Val Ser Arg Phe Met Arg Glu Gln Ser Lys Pro
180 185 190

Val Thr Ile Val Gly Leu Gln Pro Glu Glu Gly Ser Ser Ile Pro Gly
195 200 205

Ile Arg Arg Trp Pro Thr Glu Tyr Leu Pro Gly Ile Phe Asn Ala Ser
210 215 220

Leu Val Asp Glu Val Leu Asp Ile His Gln Arg Asp Ala Glu Asn Thr
225 230 235 240

Met Arg Glu Leu Ala Val Arg Glu Gly Ile Phe Cys Gly Val Ser Ser
245 250 255

Gly Gly Ala Val Ala Gly Ala Leu Arg Val Ala Lys Ala Asn Pro Asp
260 265 270

Ala Val Val Val Ala Ile Ile Cys Asp Arg Gly Asp Arg Tyr Leu Ser
275 280 285

Thr Gly Val Phe Gly Glu Glu His Phe Ser Gln Gly Ala Gly Ile
290 295 300

<210> 67
<211> 1800
<212> DNA
<213> Escherichia coli

<400> 67
atgacgacac aggtcccacc ttccgcgttg cttccgttga acccggagca actggcacgc 60

cttcaggcgg ccacgaccga tttaactccc acccagcttg cctgggtttc tggctatttc 120

tggggcgtac tcaatcagca gcctgctgcg cttgcagcga cgccagcgcc agccgcagaa 180

atgccgggta taactattat ctccgcctcg caaaccggca atgcgcgccg ggttgctgaa 240

gcattacgtg atgatttatt agcagcaaaa ctgaacgtta agctggtgaa cgcgggcgac 300

tataaattca aacaaatcgc cagcgaaaaa ctgctcatcg tagtgacgtc aacgcaaggg 360

gaaggggaac cgccggaaga agccgtcgcg ctgcataagt tcctgttctc caaaaaagcg 420

ccaaagctgg aaaacaccgc gtttgccgtg tttagcctcg gcgatagctc ttatgaattt 480

ttctgccagt ccgggaaaga tttcgacagc aagctggcgg aactgggtgg tgaacgcctg 540

ctcgaccgtg tcgatgccga tgttgaatac caggctgctg ccagcgagtg gcgcgcccgc 600

gtggttgatg cgcttaaatc gcgtgcgcct gtcgcggcac cttcgcaatc cgtcgctact 660

ggcgcggtaa atgaaatcca caccagcccg tacagcaaag acgcgccgct ggtggctagc 720

ctctctgtta accagaaaat taccgggcgt aactctgaaa aagacgttcg ccatatcgaa 780

attgacttag gtgactcggg catgcgttac cagccgggtg acgcgctggg cgtctggtat 840

cagaacgatc cggcactggt gaaagaactt gtcgaactgc tgtggctgaa aggcgatgaa 900

cctgtcaccg tcgagggcaa aacgttgcct ctgaacgaag cgctacagtg gcacttcgaa 960

ctgaccgtca acaccgccaa cattgttgag aattacgcca cgcttacccg cagtgaaaca 1020

ctgctgccgc tggtgggcga taaagcgaag ttacagcatt acgccgcgac gacgccgatt 1080

gttgacatgg tgcgtttctc cccggcacag cttgatgccg aagcgctaat taatctgctg 1140

cgcccgctga cgccgcgtct gtattccatc gcctcctcgc aggcggaagt cgagaacgaa 1200

gtacacgtca ccgttggtgt ggtgcgttac gacgtggaag gccgcgcccg tgccggtggt 1260

gcctccagct tcctcgctga ccgcgtggaa gaagagggcg aagtccgcgt atttatcgaa 1320

cataacgata acttccgcct gccagccaat ccagaaaccc cggtgattat gattggccca 1380

ggcaccggta ttgcgccgtt ccgcgccttt atgcagcaac gcgccgccga cgaagcgcca 1440

ggtaaaaact ggctgttctt tggtaatccg cactttacgg aagacttcct gtaccaggtg 1500

gagtggcagc gctacgtcaa agatggcgtg ctgacacgta tcgatcttgc ctggtcgcgc 1560

gatcaaaaag aaaaagttta cgtacaagac aaactgcgcg aacagggcgc ggagctgtgg 1620

cgctggatca atgatggtgc ccacatttat gtctgcggcg acgctaatcg catggcgaaa 1680

gacgttgagc aggcacttct ggaagtgatt gccgaatttg gtggcatgga caccgaagcg 1740

gcggatgaat ttttaagtga gctgcgcgta gagcgccgtt atcagcgaga tgtctactaa 1800

<210> 68
<211> 599
<212> PRT
<213> Escherichia coli

<400> 68

Met Thr Thr Gln Val Pro Pro Ser Ala Leu Leu Pro Leu Asn Pro Glu
1 5 10 15

Gln Leu Ala Arg Leu Gln Ala Ala Thr Thr Asp Leu Thr Pro Thr Gln
20 25 30

Leu Ala Trp Val Ser Gly Tyr Phe Trp Gly Val Leu Asn Gln Gln Pro
35 40 45

Ala Ala Leu Ala Ala Thr Pro Ala Pro Ala Ala Glu Met Pro Gly Ile
50 55 60

Thr Ile Ile Ser Ala Ser Gln Thr Gly Asn Ala Arg Arg Val Ala Glu
65 70 75 80

Ala Leu Arg Asp Asp Leu Leu Ala Ala Lys Leu Asn Val Lys Leu Val
85 90 95

```
Asn Ala Gly Asp Tyr Lys Phe Lys Gln Ile Ala Ser Glu Lys Leu Leu
            100                 105                 110

Ile Val Val Thr Ser Thr Gln Gly Glu Gly Glu Pro Pro Glu Glu Ala
        115                 120                 125

Val Ala Leu His Lys Phe Leu Phe Ser Lys Lys Ala Pro Lys Leu Glu
    130                 135                 140

Asn Thr Ala Phe Ala Val Phe Ser Leu Gly Asp Ser Ser Tyr Glu Phe
145                 150                 155                 160

Phe Cys Gln Ser Gly Lys Asp Phe Asp Ser Lys Leu Ala Glu Leu Gly
                165                 170                 175

Gly Glu Arg Leu Leu Asp Arg Val Asp Ala Asp Val Glu Tyr Gln Ala
            180                 185                 190

Ala Ala Ser Glu Trp Arg Ala Arg Val Val Asp Ala Leu Lys Ser Arg
        195                 200                 205

Ala Pro Val Ala Ala Pro Ser Gln Ser Val Ala Thr Gly Ala Val Asn
    210                 215                 220

Glu Ile His Thr Ser Pro Tyr Ser Lys Asp Ala Pro Leu Val Ala Ser
225                 230                 235                 240

Leu Ser Val Asn Gln Lys Ile Thr Gly Arg Asn Ser Glu Lys Asp Val
                245                 250                 255

Arg His Ile Glu Ile Asp Leu Gly Asp Ser Gly Met Arg Tyr Gln Pro
            260                 265                 270

Gly Asp Ala Leu Gly Val Trp Tyr Gln Asn Asp Pro Ala Leu Val Lys
        275                 280                 285

Glu Leu Val Glu Leu Leu Trp Leu Lys Gly Asp Glu Pro Val Thr Val
    290                 295                 300

Glu Gly Lys Thr Leu Pro Leu Asn Glu Ala Leu Gln Trp His Phe Glu
305                 310                 315                 320

Leu Thr Val Asn Thr Ala Asn Ile Val Glu Asn Tyr Ala Thr Leu Thr
                325                 330                 335

Arg Ser Glu Thr Leu Leu Pro Leu Val Gly Asp Lys Ala Lys Leu Gln
            340                 345                 350
```

```
His Tyr Ala Ala Thr Thr Pro Ile Val Asp Met Val Arg Phe Ser Pro
        355                 360                 365

Ala Gln Leu Asp Ala Glu Ala Leu Ile Asn Leu Leu Arg Pro Leu Thr
    370                 375                 380

Pro Arg Leu Tyr Ser Ile Ala Ser Ser Gln Ala Glu Val Glu Asn Glu
385                 390                 395                 400

Val His Val Thr Val Gly Val Val Arg Tyr Asp Val Glu Gly Arg Ala
                405                 410                 415

Arg Ala Gly Gly Ala Ser Ser Phe Leu Ala Asp Arg Val Glu Glu Glu
            420                 425                 430

Gly Glu Val Arg Val Phe Ile Glu His Asn Asp Asn Phe Arg Leu Pro
        435                 440                 445

Ala Asn Pro Glu Thr Pro Val Ile Met Ile Gly Pro Gly Thr Gly Ile
    450                 455                 460

Ala Pro Phe Arg Ala Phe Met Gln Gln Arg Ala Ala Asp Glu Ala Pro
465                 470                 475                 480

Gly Lys Asn Trp Leu Phe Phe Gly Asn Pro His Phe Thr Glu Asp Phe
                485                 490                 495

Leu Tyr Gln Val Glu Trp Gln Arg Tyr Val Lys Asp Gly Val Leu Thr
            500                 505                 510

Arg Ile Asp Leu Ala Trp Ser Arg Asp Gln Lys Glu Lys Val Tyr Val
        515                 520                 525

Gln Asp Lys Leu Arg Glu Gln Gly Ala Glu Leu Trp Arg Trp Ile Asn
    530                 535                 540

Asp Gly Ala His Ile Tyr Val Cys Gly Asp Ala Asn Arg Met Ala Lys
545                 550                 555                 560

Asp Val Glu Gln Ala Leu Leu Glu Val Ile Ala Glu Phe Gly Gly Met
                565                 570                 575

Asp Thr Glu Ala Ala Asp Glu Phe Leu Ser Glu Leu Arg Val Glu Arg
            580                 585                 590

Arg Tyr Gln Arg Asp Val Tyr
        595
```

<210> 69
<211> 1713
<212> DNA
<213> Escherichia coli

<400> 69
atgagcgaaa aacatccagg gcctttagtg gtcgaaggaa aactgacaga cgccgagcgc 60

atgaagcatg aaagcaacta cctgcgcggc accattgcgg aagatttaaa cgacggtctg 120

accggcggct ttaagggcga caacttcctg ctgattcgct tccacggcat gtatcagcag 180

gatgaccgcg acatccgcgc cgaacgtgct gaacagaagc tggagccgcg ccacgcgatg 240

ctgcttcgct gtcgtctgcc gggtggggtg attaccacta aacagtggca ggcgatcgac 300

aaatttgccg gtgaaaacac catctatggc agcattcgcc tgaccaaccg ccagacgttt 360

cagttccacg gcattctgaa aaagaacgtc aaaccggtgc accagatgct gcactcggtc 420

ggtcttgatg cgctggcgac agctaacgac atgaaccgta acgtactctg cacctcgaac 480

ccttacgagt cgcagctgca cgcggaagcg tacgagtggg cgaagaagat ttctgagcat 540

ctgttgcctc gtacccgcgc gtatgcggag atctggctcg accaggaaaa agtcgccact 600

actgatgaag aaccgatcct cggccagacc tacctgccgc gtaaattcaa aaccacggta 660

gtgatcccgc cacagaacga tatcgatctg cacgccaacg acatgaactt cgtggcgatc 720

gccgaaaacg gcaagctggt gggctttaac ctgttggtgg gcggtgggct ttccatcgaa 780

cacggcaaca agaaaaccta cgcccgcacc gcgagtgagt ttggctatct gccgctggag 840

catacgctgg cggtggccga agccgtcgtg acaactcagc gtgactgggg taaccgaacc 900

gatcgtaaaa atgccaaaac caaatacacg ctggagcgcg tgggggttga gacgtttaaa 960

gcggaagtgg agcgtcgcgc ggggatcaaa tttgaaccga tccgtccata tgagttcacc 1020

ggacgaggcg atcgtattgg ctgggttaag ggcattgatg ataactggca cctgacgctg 1080

tttatcgaaa atggtcgcat ccttgattat ccggcgcgtc cgctgaaaac cggcctgctg 1140

gagatcgcga agatccacaa aggcgatttc cgcattacgg cgaaccagaa tctgatcatc 1200

gccggtgtac cggaaagcga gaaagcgaag atcgagaaga tcgccaaaga gagcgggtta 1260

atgaatgccg tcacgccgca gcgtgaaaac tcgatggctt gcgtgtcatt cccgacttgc 1320

ccgctggcga tggcggaagc agagcgtttc ctgccgtctt ttatcgacaa catcgataat 1380

ttaatggcga aacatggtgt cagcgatgag catatcgtga tgcgtgtaac aggctgcccg 1440

aacggttgtg gtcgcgcgat gctggcggaa gtgggcctgg tgggtaaagc gccgggtcgc 1500

tacaacctgc atcttggcgg caaccgcatt gggacacgta tcccacggat gtataaagaa 1560

aacatcaccg agccggaaat cctggcgtcg cttgatgaac tgatagggcg ctgggcgaaa 1620

gagcgcgaag cgggtgaagg cttcggcgac tttacggtgc gtgcgggcat cattcgcccg 1680

gtgctcgatc cggcgcgtga tttgtgggat taa 1713

<210> 70
<211> 570
<212> PRT
<213> Escherichia coli

<400> 70

Met Ser Glu Lys His Pro Gly Pro Leu Val Val Glu Gly Lys Leu Thr
1 5 10 15

Asp Ala Glu Arg Met Lys His Glu Ser Asn Tyr Leu Arg Gly Thr Ile
20 25 30

Ala Glu Asp Leu Asn Asp Gly Leu Thr Gly Gly Phe Lys Gly Asp Asn
35 40 45

Phe Leu Leu Ile Arg Phe His Gly Met Tyr Gln Gln Asp Asp Arg Asp
50 55 60

Ile Arg Ala Glu Arg Ala Glu Gln Lys Leu Glu Pro Arg His Ala Met
65 70 75 80

Leu Leu Arg Cys Arg Leu Pro Gly Gly Val Ile Thr Thr Lys Gln Trp
85 90 95

Gln Ala Ile Asp Lys Phe Ala Gly Glu Asn Thr Ile Tyr Gly Ser Ile
100 105 110

Arg Leu Thr Asn Arg Gln Thr Phe Gln Phe His Gly Ile Leu Lys Lys
115 120 125

Asn Val Lys Pro Val His Gln Met Leu His Ser Val Gly Leu Asp Ala
130 135 140

Leu Ala Thr Ala Asn Asp Met Asn Arg Asn Val Leu Cys Thr Ser Asn
145 150 155 160

Pro Tyr Glu Ser Gln Leu His Ala Glu Ala Tyr Glu Trp Ala Lys Lys
165 170 175

Ile Ser Glu His Leu Leu Pro Arg Thr Arg Ala Tyr Ala Glu Ile Trp
180 185 190

Leu Asp Gln Glu Lys Val Ala Thr Thr Asp Glu Glu Pro Ile Leu Gly
195 200 205

Gln Thr Tyr Leu Pro Arg Lys Phe Lys Thr Thr Val Val Ile Pro Pro
    210                 215                 220

Gln Asn Asp Ile Asp Leu His Ala Asn Asp Met Asn Phe Val Ala Ile
225                 230                 235                 240

Ala Glu Asn Gly Lys Leu Val Gly Phe Asn Leu Leu Val Gly Gly Gly
                245                 250                 255

Leu Ser Ile Glu His Gly Asn Lys Lys Thr Tyr Ala Arg Thr Ala Ser
            260                 265                 270

Glu Phe Gly Tyr Leu Pro Leu Glu His Thr Leu Ala Val Ala Glu Ala
        275                 280                 285

Val Val Thr Thr Gln Arg Asp Trp Gly Asn Arg Thr Asp Arg Lys Asn
    290                 295                 300

Ala Lys Thr Lys Tyr Thr Leu Glu Arg Val Gly Val Glu Thr Phe Lys
305                 310                 315                 320

Ala Glu Val Glu Arg Arg Ala Gly Ile Lys Phe Glu Pro Ile Arg Pro
                325                 330                 335

Tyr Glu Phe Thr Gly Arg Gly Asp Arg Ile Gly Trp Val Lys Gly Ile
            340                 345                 350

Asp Asp Asn Trp His Leu Thr Leu Phe Ile Glu Asn Gly Arg Ile Leu
        355                 360                 365

Asp Tyr Pro Ala Arg Pro Leu Lys Thr Gly Leu Leu Glu Ile Ala Lys
    370                 375                 380

Ile His Lys Gly Asp Phe Arg Ile Thr Ala Asn Gln Asn Leu Ile Ile
385                 390                 395                 400

Ala Gly Val Pro Glu Ser Glu Lys Ala Lys Ile Glu Lys Ile Ala Lys
                405                 410                 415

Glu Ser Gly Leu Met Asn Ala Val Thr Pro Gln Arg Glu Asn Ser Met
            420                 425                 430

Ala Cys Val Ser Phe Pro Thr Cys Pro Leu Ala Met Ala Glu Ala Glu
        435                 440                 445

Arg Phe Leu Pro Ser Phe Ile Asp Asn Ile Asp Asn Leu Met Ala Lys
    450                 455                 460

His Gly Val Ser Asp Glu His Ile Val Met Arg Val Thr Gly Cys Pro
465 470 475 480

Asn Gly Cys Gly Arg Ala Met Leu Ala Glu Val Gly Leu Val Gly Lys
485 490 495

Ala Pro Gly Arg Tyr Asn Leu His Leu Gly Gly Asn Arg Ile Gly Thr
500 505 510

Arg Ile Pro Arg Met Tyr Lys Glu Asn Ile Thr Glu Pro Glu Ile Leu
515 520 525

Ala Ser Leu Asp Glu Leu Ile Gly Arg Trp Ala Lys Glu Arg Glu Ala
530 535 540

Gly Glu Gly Phe Gly Asp Phe Thr Val Arg Ala Gly Ile Ile Arg Pro
545 550 555 560

Val Leu Asp Pro Ala Arg Asp Leu Trp Asp
565 570

<210> 71
<211> 735
<212> DNA
<213> Escherichia coli

<400> 71
atgtccaaac tcgatctaaa cgccctgaac gaactgccga aggtagatcg cattctggcg 60

ctggcggaaa ctaacgccga actggaaaaa ctggacgctg aaggccgcgt agcctgggcg 120

ctggataatc tgcccggtga atatgtactt tcttccagct ttggcattca ggcggcggtg 180

agcctgcatc tggtgaatca aattcgcccg gatattccgg tgatcctcac cgatacgggt 240

tacttgttcc cggaaaccta ccgctttatt gacgagttaa cggacaaact caagctcaac 300

ctgaaagtgt accgtgctac cgaaagcgca gcctggcagg aagcacgcta cggaaaactg 360

tgggagcagg gcgttgaagg cattgaaaag tacaatgaca tcaacaaagt cgaaccgatg 420

aaccgggctc tgaaagaact gaatgcgcaa acctggtttg ctggcctgcg ccgtgaacaa 480

tccggcagtc gtgccaattt accggtgctg gcaattcagc gtggcgtatt taaagtgctg 540

ccgattatcg actgggataa ccgaactatt tatcagtacc tgcaaaaaca tggcctgaaa 600

tatcacccat tatgggatga aggatattta tcggtgggcg atacccatac aacccgtaaa 660

tgggaacccg gcatggcgga agaagaaacg cgtttctttg gcttaaaaag ggaatgtggg 720

ttacacgaag ggtaa 735

<210> 72

<211> 244
<212> PRT
<213> Escherichia coli

<400> 72

Met Ser Lys Leu Asp Leu Asn Ala Leu Asn Glu Leu Pro Lys Val Asp
1 5 10 15

Arg Ile Leu Ala Leu Ala Glu Thr Asn Ala Glu Leu Glu Lys Leu Asp
20 25 30

Ala Glu Gly Arg Val Ala Trp Ala Leu Asp Asn Leu Pro Gly Glu Tyr
35 40 45

Val Leu Ser Ser Ser Phe Gly Ile Gln Ala Ala Val Ser Leu His Leu
50 55 60

Val Asn Gln Ile Arg Pro Asp Ile Pro Val Ile Leu Thr Asp Thr Gly
65 70 75 80

Tyr Leu Phe Pro Glu Thr Tyr Arg Phe Ile Asp Glu Leu Thr Asp Lys
85 90 95

Leu Lys Leu Asn Leu Lys Val Tyr Arg Ala Thr Glu Ser Ala Ala Trp
100 105 110

Gln Glu Ala Arg Tyr Gly Lys Leu Trp Glu Gln Gly Val Glu Gly Ile
115 120 125

Glu Lys Tyr Asn Asp Ile Asn Lys Val Glu Pro Met Asn Arg Ala Leu
130 135 140

Lys Glu Leu Asn Ala Gln Thr Trp Phe Ala Gly Leu Arg Arg Glu Gln
145 150 155 160

Ser Gly Ser Arg Ala Asn Leu Pro Val Leu Ala Ile Gln Arg Gly Val
165 170 175

Phe Lys Val Leu Pro Ile Ile Asp Trp Asp Asn Arg Thr Ile Tyr Gln
180 185 190

Tyr Leu Gln Lys His Gly Leu Lys Tyr His Pro Leu Trp Asp Glu Gly
195 200 205

Tyr Leu Ser Val Gly Asp Thr His Thr Thr Arg Lys Trp Glu Pro Gly
210 215 220

Met Ala Glu Glu Glu Thr Arg Phe Phe Gly Leu Lys Arg Glu Cys Gly

225 230 235 240

Leu His Glu Gly

<210> 73
<211> 972
<212> DNA
<213> Escherichia coli

<400> 73
atgagtaaga tttttgaaga taactcgctg actatcggtc acacgccgct ggttcgcctg 60

aatcgcatcg gtaacggacg cattctggcg aaggtggaat ctcgtaaccc cagcttcagc 120

gttaagtgcc gtatcggtgc caacatgatt tgggatgccg aaaagcgcgg cgtgctgaaa 180

ccaggcgttg aactggttga accgaccagc ggtaataccg ggattgcact ggcctatgta 240

gctgccgctc gcggttacaa actcaccctg accatgccag aaaccatgag tattgaacgc 300

cgcaagctgc tgaaagcgtt aggtgcaaac ctggtgctga cggaaggtgc taaaggcatg 360

aaaggcgcaa tccaaaaagc agaagaaatt gtcgccagca atccagagaa atacctgctg 420

ctgcaacaat tcagcaatcc ggcaaaccct gaaattcacg aaaagaccac cggtccggag 480

atatgggaag ataccgacgg tcaggttgat gtatttattg ctggcgttgg gactggcggt 540

acgctgactg gcgtcagccg ctacattaaa ggcaccaaag gcaagaccga tcttatctct 600

gtcgccgttg agccaaccga ttctccagtt atcgcccagg cgctggcagg tgaagagatt 660

aaacctggcc cgcataaaat tcagggtatt ggcgctggtt ttatcccggc taacctcgat 720

ctcaagctgg tcgataaagt cattggcatc accaatgaag aagcgatttc taccgcgcgt 780

cgtctgatgg aagaagaagg tattcttgca ggtatctctt ctggagcagc tgttgccgcg 840

gcgttgaaac tacaagaaga tgaaagcttt accaacaaga atattgtggt tattctacca 900

tcatcgggtg agcgttattt aagcaccgca ttgtttgccg atctcttcac tgagaaagaa 960

ttgcaacagt aa 972

<210> 74
<211> 323
<212> PRT
<213> Escherichia coli

<400> 74

Met Ser Lys Ile Phe Glu Asp Asn Ser Leu Thr Ile Gly His Thr Pro
1 5 10 15

Leu Val Arg Leu Asn Arg Ile Gly Asn Gly Arg Ile Leu Ala Lys Val
20 25 30

```
Glu Ser Arg Asn Pro Ser Phe Ser Val Lys Cys Arg Ile Gly Ala Asn
        35                  40                  45

Met Ile Trp Asp Ala Glu Lys Arg Gly Val Leu Lys Pro Gly Val Glu
    50                  55                  60

Leu Val Glu Pro Thr Ser Gly Asn Thr Gly Ile Ala Leu Ala Tyr Val
65                  70                  75                  80

Ala Ala Ala Arg Gly Tyr Lys Leu Thr Leu Thr Met Pro Glu Thr Met
                85                  90                  95

Ser Ile Glu Arg Arg Lys Leu Leu Lys Ala Leu Gly Ala Asn Leu Val
            100                 105                 110

Leu Thr Glu Gly Ala Lys Gly Met Lys Gly Ala Ile Gln Lys Ala Glu
        115                 120                 125

Glu Ile Val Ala Ser Asn Pro Glu Lys Tyr Leu Leu Leu Gln Gln Phe
    130                 135                 140

Ser Asn Pro Ala Asn Pro Glu Ile His Glu Lys Thr Thr Gly Pro Glu
145                 150                 155                 160

Ile Trp Glu Asp Thr Asp Gly Gln Val Asp Val Phe Ile Ala Gly Val
                165                 170                 175

Gly Thr Gly Gly Thr Leu Thr Gly Val Ser Arg Tyr Ile Lys Gly Thr
            180                 185                 190

Lys Gly Lys Thr Asp Leu Ile Ser Val Ala Val Glu Pro Thr Asp Ser
        195                 200                 205

Pro Val Ile Ala Gln Ala Leu Ala Gly Glu Glu Ile Lys Pro Gly Pro
    210                 215                 220

His Lys Ile Gln Gly Ile Gly Ala Gly Phe Ile Pro Ala Asn Leu Asp
225                 230                 235                 240

Leu Lys Leu Val Asp Lys Val Ile Gly Ile Thr Asn Glu Glu Ala Ile
                245                 250                 255

Ser Thr Ala Arg Arg Leu Met Glu Glu Glu Gly Ile Leu Ala Gly Ile
            260                 265                 270

Ser Ser Gly Ala Ala Val Ala Ala Ala Leu Lys Leu Gln Glu Asp Glu
        275                 280                 285
```

Ser Phe Thr Asn Lys Asn Ile Val Val Ile Leu Pro Ser Ser Gly Glu
290 295 300

Arg Tyr Leu Ser Thr Ala Leu Phe Ala Asp Leu Phe Thr Glu Lys Glu
305 310 315 320

Leu Gln Gln

<210> 75
<211> 909
<212> DNA
<213> Escherichia coli

<400> 75
atggatcaaa tacgacttac tcacctgcgg caactggagg cggaaagcat ccacattatt 60

cgcgaggtgg cggcagaatt ctcaaatccg gtgatgctct actctatcgg taaagattcc 120

agcgtcatgc tgcatctggc gcgcaaggcg ttttatccag gtacgctgcc tttcccgttg 180

ctgcatgtcg ataccggctg gaaattccgc gagatgtatg agttccgcga tcgtactgct 240

aaagcctacg gctgcgaact gctggtgcat aaaaacccgg aaggcgtggc gatggggatt 300

aatccattcg tgcacggcag cgcgaaacat accgatatta tgaaaactga aggcctgaaa 360

caggcgctga acaaatacgg ttttgatgcc gccttcggtg gtgcgcgccg tgacgaagag 420

aaatcccgcg ctaaagagcg aatttactct ttccgtgacc gcttccatcg ctgggatccg 480

aaaaatcagc gcccggagct gtggcacaac tacaacgggc aaattaacaa aggcgaaagc 540

atccgcgtct tcccgctctc taactggacc gagcaggata tctggcaata catctggctg 600

gaaaatatcg acattgttcc gctatatctc gctgcggaac gtccggttct ggaacgcgac 660

ggtatgttga tgatgattga tgacaaccgt atcgacctgc aaccgggcga agtgattaaa 720

aaacggatgg tgcgtttccg tacgctgggc tgctggccgc tgaccggtgc ggtggagtca 780

aatgcacaaa cactgccgga aatcatcgaa gagatgctgg tttccaccac cagtgaacgt 840

cagggccgcg tgattgaccg cgaccaggcg gggtctatgg agctgaaaaa acgtcagggg 900

tatttttaa 909

<210> 76
<211> 302
<212> PRT
<213> Escherichia coli

<400> 76

Met Asp Gln Ile Arg Leu Thr His Leu Arg Gln Leu Glu Ala Glu Ser
1 5 10 15

```
Ile His Ile Ile Arg Glu Val Ala Ala Glu Phe Ser Asn Pro Val Met
            20                  25                  30

Leu Tyr Ser Ile Gly Lys Asp Ser Ser Val Met Leu His Leu Ala Arg
        35                  40                  45

Lys Ala Phe Tyr Pro Gly Thr Leu Pro Phe Pro Leu Leu His Val Asp
    50                  55                  60

Thr Gly Trp Lys Phe Arg Glu Met Tyr Glu Phe Arg Asp Arg Thr Ala
65                  70                  75                  80

Lys Ala Tyr Gly Cys Glu Leu Leu Val His Lys Asn Pro Glu Gly Val
                85                  90                  95

Ala Met Gly Ile Asn Pro Phe Val His Gly Ser Ala Lys His Thr Asp
            100                 105                 110

Ile Met Lys Thr Glu Gly Leu Lys Gln Ala Leu Asn Lys Tyr Gly Phe
        115                 120                 125

Asp Ala Ala Phe Gly Gly Ala Arg Arg Asp Glu Glu Lys Ser Arg Ala
    130                 135                 140

Lys Glu Arg Ile Tyr Ser Phe Arg Asp Arg Phe His Arg Trp Asp Pro
145                 150                 155                 160

Lys Asn Gln Arg Pro Glu Leu Trp His Asn Tyr Asn Gly Gln Ile Asn
                165                 170                 175

Lys Gly Glu Ser Ile Arg Val Phe Pro Leu Ser Asn Trp Thr Glu Gln
            180                 185                 190

Asp Ile Trp Gln Tyr Ile Trp Leu Glu Asn Ile Asp Ile Val Pro Leu
        195                 200                 205

Tyr Leu Ala Ala Glu Arg Pro Val Leu Glu Arg Asp Gly Met Leu Met
    210                 215                 220

Met Ile Asp Asp Asn Arg Ile Asp Leu Gln Pro Gly Glu Val Ile Lys
225                 230                 235                 240

Lys Arg Met Val Arg Phe Arg Thr Leu Gly Cys Trp Pro Leu Thr Gly
                245                 250                 255

Ala Val Glu Ser Asn Ala Gln Thr Leu Pro Glu Ile Ile Glu Glu Met
            260                 265                 270
```

Leu Val Ser Thr Thr Ser Glu Arg Gln Gly Arg Val Ile Asp Arg Asp
        275                 280                 285

Gln Ala Gly Ser Met Glu Leu Lys Lys Arg Gln Gly Tyr Phe
    290                 295                 300

<210> 77
<211> 1428
<212> DNA
<213> Escherichia coli

<400> 77
atgaacaccg cacttgcaca acaaatcgcc aatgaaggcg gcgtcgaagc ctggatgatt 60

gcgcaacaac ataaaagcct gctgcgtttt ctgacctgtg gtagcgtcga tgacggcaaa 120

agtactctga ttggtcgtct gctgcacgat acccgccaaa tctacgaaga tcagctctca 180

tcgctgcata acgacagtaa gcgtcacggc acccagggcg aaaagctgga tctggctctg 240

ctggtggacg gcctgcaagc tgagcgcgaa cagggcatca ccattgacgt ggcctaccgc 300

tatttctcta ccgagaagcg taaatttatt atcgccgaca ccccagggca cgagcagtac 360

acccgcaata tggcgactgg cgcatcgaca tgtgaactgg cgatcttact gatcgatgcc 420

cgtaaaggcg tgctcgatca aacccgtcgt cacagtttta tctccacact gttggggatc 480

aaacatctgg tcgtggcgat caacaaaatg gatctggtgg attacagtga agagacgttc 540

acccgtattc gtgaagatta tttgaccttt gccgggcagc tgccgggtaa tctggatatc 600

cgctttgtgc cgctctctgc actggaaggc gacaacgtgg catcgcaaag tgaaagtatg 660

ccgtggtaca gcggtccgac actgctcgaa gtgctggaaa ccgtggagat ccagcgagtg 720

gtggatgctc agccaatgcg cttcccggtg cagtacgtta atcgcccgaa tctcgatttt 780

cgtggttacg ccggaacgct ggcatccggt cgcgtggaag tcgggcaacg tgtcaaagtg 840

ctgccctctg gtgtggaatc aaacgtcgcg cggatcgtga cttttgatgg tgatcgcgaa 900

gaagcctttg ccggagaagc gatcaccctg gtgctgacgg atgagatcga catcagccgt 960

ggcgatctgc tgctggcggc agacgaagcg ttaccggcgg tgcagagcgc gtcggtggat 1020

gtggtatgga tggcggaaca gccgctttct ccagggcaga gttacgacat caaaattgcc 1080

ggtaagaaga cgcgcgcgcg tgttgatggc attcgctatc aggttgatat taataacctt 1140

acccagcgtg aagttgaaaa cctgccactg aatgggatcg gcctcgtgga tctcactttt 1200

gacgagccgc tggtgttaga tcgttatcaa caaaatccgg tgacgggtgg gctgattttt 1260

atcgatcgcc tgagcaatgt gaccgtgggt gccggtatgg tgcacgagcc agttagccag 1320

gcaactgctg cgccatctga attcagtgca ttcgaactgg aattgaatgc tctggttcgt 1380

cgccactttc cgcactgggg cgcgcgcgat ttgctggggg ataaataa 1428

<210> 78
<211> 475
<212> PRT
<213> Escherichia coli

<400> 78

Met Asn Thr Ala Leu Ala Gln Gln Ile Ala Asn Glu Gly Gly Val Glu
1 5 10 15

Ala Trp Met Ile Ala Gln Gln His Lys Ser Leu Leu Arg Phe Leu Thr
20 25 30

Cys Gly Ser Val Asp Asp Gly Lys Ser Thr Leu Ile Gly Arg Leu Leu
35 40 45

His Asp Thr Arg Gln Ile Tyr Glu Asp Gln Leu Ser Ser Leu His Asn
50 55 60

Asp Ser Lys Arg His Gly Thr Gln Gly Glu Lys Leu Asp Leu Ala Leu
65 70 75 80

Leu Val Asp Gly Leu Gln Ala Glu Arg Glu Gln Gly Ile Thr Ile Asp
85 90 95

Val Ala Tyr Arg Tyr Phe Ser Thr Glu Lys Arg Lys Phe Ile Ile Ala
100 105 110

Asp Thr Pro Gly His Glu Gln Tyr Thr Arg Asn Met Ala Thr Gly Ala
115 120 125

Ser Thr Cys Glu Leu Ala Ile Leu Leu Ile Asp Ala Arg Lys Gly Val
130 135 140

Leu Asp Gln Thr Arg Arg His Ser Phe Ile Ser Thr Leu Leu Gly Ile
145 150 155 160

Lys His Leu Val Val Ala Ile Asn Lys Met Asp Leu Val Asp Tyr Ser
165 170 175

Glu Glu Thr Phe Thr Arg Ile Arg Glu Asp Tyr Leu Thr Phe Ala Gly
180 185 190

Gln Leu Pro Gly Asn Leu Asp Ile Arg Phe Val Pro Leu Ser Ala Leu
195 200 205

Glu Gly Asp Asn Val Ala Ser Gln Ser Glu Ser Met Pro Trp Tyr Ser
210 215 220

```
Gly Pro Thr Leu Leu Glu Val Leu Glu Thr Val Glu Ile Gln Arg Val
225                 230                 235                 240

Val Asp Ala Gln Pro Met Arg Phe Pro Val Gln Tyr Val Asn Arg Pro
                245                 250                 255

Asn Leu Asp Phe Arg Gly Tyr Ala Gly Thr Leu Ala Ser Gly Arg Val
            260                 265                 270

Glu Val Gly Gln Arg Val Lys Val Leu Pro Ser Gly Val Glu Ser Asn
        275                 280                 285

Val Ala Arg Ile Val Thr Phe Asp Gly Asp Arg Glu Glu Ala Phe Ala
    290                 295                 300

Gly Glu Ala Ile Thr Leu Val Leu Thr Asp Glu Ile Asp Ile Ser Arg
305                 310                 315                 320

Gly Asp Leu Leu Leu Ala Ala Asp Glu Ala Leu Pro Ala Val Gln Ser
                325                 330                 335

Ala Ser Val Asp Val Val Trp Met Ala Glu Gln Pro Leu Ser Pro Gly
            340                 345                 350

Gln Ser Tyr Asp Ile Lys Ile Ala Gly Lys Lys Thr Arg Ala Arg Val
        355                 360                 365

Asp Gly Ile Arg Tyr Gln Val Asp Ile Asn Asn Leu Thr Gln Arg Glu
    370                 375                 380

Val Glu Asn Leu Pro Leu Asn Gly Ile Gly Leu Val Asp Leu Thr Phe
385                 390                 395                 400

Asp Glu Pro Leu Val Leu Asp Arg Tyr Gln Gln Asn Pro Val Thr Gly
                405                 410                 415

Gly Leu Ile Phe Ile Asp Arg Leu Ser Asn Val Thr Val Gly Ala Gly
            420                 425                 430

Met Val His Glu Pro Val Ser Gln Ala Thr Ala Ala Pro Ser Glu Phe
        435                 440                 445

Ser Ala Phe Glu Leu Glu Leu Asn Ala Leu Val Arg Arg His Phe Pro
    450                 455                 460

His Trp Gly Ala Arg Asp Leu Leu Gly Asp Lys
465                 470                 475
```

<210> 79
<211> 606
<212> DNA
<213> Escherichia coli

<400> 79
atggcgctgc atgacgaaaa cgtcgtctgg catagccatc cggtcactgt gcaacaacgc 60

gagctacacc acggtcatcg tggtgtagtg ctgtggttta ccggcctctc cgggtccggt 120

aaatcaacgg tcgccggggc gctggaggag gcgttacata aactcggcgt cagtacgtat 180

ctgctggatg gcgacaatgt tcgccacgga ttatgcagcg atctcggttt tagcgatgcc 240

gatcgtaaag agaatatccg tcgcgtcggt gaagtggcga atttgatggt tgaagccgga 300

ctggtggtgc tgaccgcatt tatctcgcca caccgcgccg aacgccagat ggttcgcgaa 360

cgcgtaggag aagggcgctt tatcgaagtg tttgtcgata cgccgctggc gatttgcgaa 420

gcccgcgatc ccaaaggctt atataagaaa gcgcgtgccg gtgaactgcg caactttacg 480

ggaatagatt ccgtttacga agcgcctgaa tcggcagaaa ttcatctcaa tggtgaacaa 540

ttagtaacaa atttggtaca gcaattatta gatctgttga gacagaacga tattatcaga 600

tcctga 606

<210> 80
<211> 201
<212> PRT
<213> Escherichia coli

<400> 80

Met Ala Leu His Asp Glu Asn Val Val Trp His Ser His Pro Val Thr
1               5                   10                  15

Val Gln Gln Arg Glu Leu His His Gly His Arg Gly Val Val Leu Trp
            20                  25                  30

Phe Thr Gly Leu Ser Gly Ser Gly Lys Ser Thr Val Ala Gly Ala Leu
        35                  40                  45

Glu Glu Ala Leu His Lys Leu Gly Val Ser Thr Tyr Leu Leu Asp Gly
    50                  55                  60

Asp Asn Val Arg His Gly Leu Cys Ser Asp Leu Gly Phe Ser Asp Ala
65                  70                  75                  80

Asp Arg Lys Glu Asn Ile Arg Arg Val Gly Glu Val Ala Asn Leu Met
                85                  90                  95

Val Glu Ala Gly Leu Val Val Leu Thr Ala Phe Ile Ser Pro His Arg

100 105 110

Ala Glu Arg Gln Met Val Arg Glu Arg Val Gly Glu Gly Arg Phe Ile
115 120 125

Glu Val Phe Val Asp Thr Pro Leu Ala Ile Cys Glu Ala Arg Asp Pro
130 135 140

Lys Gly Leu Tyr Lys Lys Ala Arg Ala Gly Glu Leu Arg Asn Phe Thr
145 150 155 160

Gly Ile Asp Ser Val Tyr Glu Ala Pro Glu Ser Ala Glu Ile His Leu
165 170 175

Asn Gly Glu Gln Leu Val Thr Asn Leu Val Gln Gln Leu Leu Asp Leu
180 185 190

Leu Arg Gln Asn Asp Ile Ile Arg Ser
195 200

<210> 81
<211> 990
<212> DNA
<213> Escherichia coli

<400> 81
atgaacaagt ggggcgtagg gttaacattt ttgctggcgg caaccagcgt tatggcaaag 60

gatattcagc ttcttaacgt ttcatatgat ccaacgcgcg aattgtacga acagtacaac 120

aaggcattca gcgcccactg gaaacagcaa actggtgata acgtggtgat tcgtcagtca 180

cacggtggct caggtaaaca agcgacgtcg gtaatcaacg gtattgaagc tgatgttgtc 240

acgctggctc tggcctatga cgtggacgca attgcggaac gcgggcggat tgataaagag 300

tggatcaaac gtctgccgga taactccgca ccgtacactt ccaccattgt tttcctggta 360

cgtaagggaa atccgaagca gatccatgac tggaacgatc tgattaaacc gggtgtttcg 420

gtgatcacgc ctaatccgaa aagctctggt ggcgcgcgct ggaactacct ggcagcctgg 480

ggctacgcgc tgcatcacaa caacaacgat caggcaaaag cacaggattt tgttcgggca 540

ctgtataaaa acgtcgaagt tctggattct ggcgcgcgtg gctccactaa cacttttgtc 600

gagcgcggaa ttggcgatgt actgattgcc tgggaaaacg aagctctgct ggcagcgaat 660

gaactgggga aagataaatt cgaaatcgtc acgccgagtg agtctatcct cgcagagcca 720

accgtgtcgg tggtcgataa agtggtcgag aaaaaaggta ctaaagaggt ggcggaagcc 780

tacctgaaat atctctactc gccagaaggt caggaaattg ccgcgaaaaa ctactaccgt 840

ccgcgcgacg ctgaggtggc gaaaaagtac gaaaatgcgt ttccaaagct gaagttattc 900

accattgatg aagagttcgg cggctggacg aaagcgcaaa aagagcattt tgctaacggc 960

ggtacgttcg atcagatcag caaacgctga 990

<210> 82
<211> 329
<212> PRT
<213> Escherichia coli

<400> 82

Met Asn Lys Trp Gly Val Gly Leu Thr Phe Leu Leu Ala Ala Thr Ser
1 5 10 15

Val Met Ala Lys Asp Ile Gln Leu Leu Asn Val Ser Tyr Asp Pro Thr
20 25 30

Arg Glu Leu Tyr Glu Gln Tyr Asn Lys Ala Phe Ser Ala His Trp Lys
35 40 45

Gln Gln Thr Gly Asp Asn Val Val Ile Arg Gln Ser His Gly Gly Ser
50 55 60

Gly Lys Gln Ala Thr Ser Val Ile Asn Gly Ile Glu Ala Asp Val Val
65 70 75 80

Thr Leu Ala Leu Ala Tyr Asp Val Asp Ala Ile Ala Glu Arg Gly Arg
85 90 95

Ile Asp Lys Glu Trp Ile Lys Arg Leu Pro Asp Asn Ser Ala Pro Tyr
100 105 110

Thr Ser Thr Ile Val Phe Leu Val Arg Lys Gly Asn Pro Lys Gln Ile
115 120 125

His Asp Trp Asn Asp Leu Ile Lys Pro Gly Val Ser Val Ile Thr Pro
130 135 140

Asn Pro Lys Ser Ser Gly Gly Ala Arg Trp Asn Tyr Leu Ala Ala Trp
145 150 155 160

Gly Tyr Ala Leu His His Asn Asn Asn Asp Gln Ala Lys Ala Gln Asp
165 170 175

Phe Val Arg Ala Leu Tyr Lys Asn Val Glu Val Leu Asp Ser Gly Ala
180 185 190

Arg Gly Ser Thr Asn Thr Phe Val Glu Arg Gly Ile Gly Asp Val Leu
195 200 205

Ile Ala Trp Glu Asn Glu Ala Leu Leu Ala Ala Asn Glu Leu Gly Lys
    210                 215                 220

Asp Lys Phe Glu Ile Val Thr Pro Ser Glu Ser Ile Leu Ala Glu Pro
225                 230                 235                 240

Thr Val Ser Val Val Asp Lys Val Val Glu Lys Lys Gly Thr Lys Glu
                245                 250                 255

Val Ala Glu Ala Tyr Leu Lys Tyr Leu Tyr Ser Pro Glu Gly Gln Glu
            260                 265                 270

Ile Ala Ala Lys Asn Tyr Tyr Arg Pro Arg Asp Ala Glu Val Ala Lys
        275                 280                 285

Lys Tyr Glu Asn Ala Phe Pro Lys Leu Lys Leu Phe Thr Ile Asp Glu
    290                 295                 300

Glu Phe Gly Gly Trp Thr Lys Ala Gln Lys Glu His Phe Ala Asn Gly
305                 310                 315                 320

Gly Thr Phe Asp Gln Ile Ser Lys Arg
                325

<210> 83
<211> 246
<212> DNA
<213> Escherichia coli

<400> 83
atgcgcatta ctatttacac tcgtaacgat tgcgttcagt gccacgccac caaacgggcg 60

atggaaaacc ggggctttga ttttgaaatg attaatgtcg atcgcgttcc tgaagcggca 120

gaagcgttgc gtgctcaggg ctttcgtcag ttgccggtag tgattgctgg cgatcttagc 180

tggtctggtt tccgtccgga catgattaac cgtctgcatc cagcgccaca cgcggccagt 240

gcatga 246

<210> 84
<211> 81
<212> PRT
<213> Escherichia coli

<400> 84

Met Arg Ile Thr Ile Tyr Thr Arg Asn Asp Cys Val Gln Cys His Ala
1               5                   10                  15

Thr Lys Arg Ala Met Glu Asn Arg Gly Phe Asp Phe Glu Met Ile Asn
            20                  25                  30

Val Asp Arg Val Pro Glu Ala Ala Glu Ala Leu Arg Ala Gln Gly Phe
35 40 45

Arg Gln Leu Pro Val Val Ile Ala Gly Asp Leu Ser Trp Ser Gly Phe
50 55 60

Arg Pro Asp Met Ile Asn Arg Leu His Pro Ala Pro His Ala Ala Ser
65 70 75 80

Ala

<210> 85
<211> 258
<212> DNA
<213> Escherichia coli

<400> 85
atgcaaaccg ttatttttgg tcgttcgggt tgcccttact gtgtgcgtgc aaaagatctg 60

gctgagaaat tgagcaatga acgcgatgat tttcagtatc agtatgtaga tattcgtgcg 120

gaagggatca ctaaagaaga tctacaacaa aaggcaggta aacccgtaga aaccgtgccg 180

cagatttttg tcgatcagca acatatcggc ggctataccg attttgctgc atgggtgaaa 240

gaaaatctgg acgcctga 258

<210> 86
<211> 85
<212> PRT
<213> Escherichia coli

<400> 86

Met Gln Thr Val Ile Phe Gly Arg Ser Gly Cys Pro Tyr Cys Val Arg
1 5 10 15

Ala Lys Asp Leu Ala Glu Lys Leu Ser Asn Glu Arg Asp Asp Phe Gln
20 25 30

Tyr Gln Tyr Val Asp Ile Arg Ala Glu Gly Ile Thr Lys Glu Asp Leu
35 40 45

Gln Gln Lys Ala Gly Lys Pro Val Glu Thr Val Pro Gln Ile Phe Val
50 55 60

Asp Gln Gln His Ile Gly Gly Tyr Thr Asp Phe Ala Ala Trp Val Lys
65 70 75 80

Glu Asn Leu Asp Ala

<210> 87
<211> 1353
<212> DNA
<213> Escherichia coli

<400> 87
atgactaaac actatgatta catcgccatc ggcggcggca gcggcggtat cgcctccatc 60

aaccgcgcgg ctatgtacgg ccagaaatgt gcgctgattg aagccaaaga gctgggcggc 120

acctgcgtaa atgttggctg tgtgccgaaa aaagtgatgt ggcacgcggc gcaaatccgt 180

gaagcgatcc atatgtacgg cccggattat ggttttgata ccactatcaa taaattcaac 240

tgggaaacgt tgatcgccag ccgtaccgcc tatatcgacc gtattcatac ttcctatgaa 300

aacgtgctcg gtaaaaataa cgttgatgta atcaaaggct ttgcccgctt cgttgatgcc 360

aaaacgctgg aggtaaacgg cgaaaccatc acggccgatc atattctgat cgccacaggc 420

ggtcgtccga gccacccgga tattccgggc gtggaatacg gtattgattc tgatggcttc 480

ttcgcccttc ctgctttgcc agagcgcgtg gcggttgttg gcgcgggtta catcgccgtt 540

gagctggcgg gcgtgattaa cggcctcggc gcgaaaacgc atctgtttgt gcgtaaacat 600

gcgccgctgc gcagcttcga cccgatgatt tccgaaacgc tggtcgaagt gatgaacgcc 660

gaaggcccgc agctgcacac caacgccatc ccgaaagcgg tagtgaaaaa taccgatggt 720

agcctgacgc tggagctgga agatggtcgc agtgaaacgg tggattgcct gatttgggcg 780

attggtcgcg agcctgccaa tgacaacatc aacctggaag ccgctggcgt taaaactaac 840

gaaaaaggct atatcgtcgt cgataaatat caaaacacca atattgaagg tatttacgcg 900

gtgggcgata acacgggtgc agtggagctg acaccggtgg cagttgcagc gggtcgccgt 960

ctctctgaac gcctgtttaa taacaagccg gatgagcatc tggattacag caacattccg 1020

accgtggtct tcagccatcc gccgattggt actgttggtt taacggaacc gcaggcgcgc 1080

gagcagtatg gcgacgatca ggtgaaagtg tataaatcct ctttcaccgc gatgtatacc 1140

gccgtcacca ctcaccgcca gccgtgccgc atgaagctgg tgtgcgttgg atcggaagag 1200

aagattgtcg gtattcacgg cattggcttt ggtatggacg aaatgttgca gggcttcgcg 1260

gtggcgctga agatgggggc aaccaaaaaa gacttcgaca ataccgtcgc cattcaccca 1320

acggcggcag aagagttcgt gacaatgcgt taa 1353

<210> 88
<211> 450
<212> PRT
<213> Escherichia coli

<400> 88

```
Met Thr Lys His Tyr Asp Tyr Ile Ala Ile Gly Gly Gly Ser Gly Gly
1               5                   10                  15

Ile Ala Ser Ile Asn Arg Ala Ala Met Tyr Gly Gln Lys Cys Ala Leu
            20                  25                  30

Ile Glu Ala Lys Glu Leu Gly Gly Thr Cys Val Asn Val Gly Cys Val
        35                  40                  45

Pro Lys Lys Val Met Trp His Ala Ala Gln Ile Arg Glu Ala Ile His
    50                  55                  60

Met Tyr Gly Pro Asp Tyr Gly Phe Asp Thr Thr Ile Asn Lys Phe Asn
65                  70                  75                  80

Trp Glu Thr Leu Ile Ala Ser Arg Thr Ala Tyr Ile Asp Arg Ile His
                85                  90                  95

Thr Ser Tyr Glu Asn Val Leu Gly Lys Asn Asn Val Asp Val Ile Lys
            100                 105                 110

Gly Phe Ala Arg Phe Val Asp Ala Lys Thr Leu Glu Val Asn Gly Glu
        115                 120                 125

Thr Ile Thr Ala Asp His Ile Leu Ile Ala Thr Gly Gly Arg Pro Ser
    130                 135                 140

His Pro Asp Ile Pro Gly Val Glu Tyr Gly Ile Asp Ser Asp Gly Phe
145                 150                 155                 160

Phe Ala Leu Pro Ala Leu Pro Glu Arg Val Ala Val Val Gly Ala Gly
                165                 170                 175

Tyr Ile Ala Val Glu Leu Ala Gly Val Ile Asn Gly Leu Gly Ala Lys
            180                 185                 190

Thr His Leu Phe Val Arg Lys His Ala Pro Leu Arg Ser Phe Asp Pro
        195                 200                 205

Met Ile Ser Glu Thr Leu Val Glu Val Met Asn Ala Glu Gly Pro Gln
    210                 215                 220

Leu His Thr Asn Ala Ile Pro Lys Ala Val Val Lys Asn Thr Asp Gly
225                 230                 235                 240

Ser Leu Thr Leu Glu Leu Glu Asp Gly Arg Ser Glu Thr Val Asp Cys
                245                 250                 255
```

Leu Ile Trp Ala Ile Gly Arg Glu Pro Ala Asn Asp Asn Ile Asn Leu
260 265 270

Glu Ala Ala Gly Val Lys Thr Asn Glu Lys Gly Tyr Ile Val Val Asp
275 280 285

Lys Tyr Gln Asn Thr Asn Ile Glu Gly Ile Tyr Ala Val Gly Asp Asn
290 295 300

Thr Gly Ala Val Glu Leu Thr Pro Val Ala Val Ala Ala Gly Arg Arg
305 310 315 320

Leu Ser Glu Arg Leu Phe Asn Asn Lys Pro Asp Glu His Leu Asp Tyr
325 330 335

Ser Asn Ile Pro Thr Val Val Phe Ser His Pro Pro Ile Gly Thr Val
340 345 350

Gly Leu Thr Glu Pro Gln Ala Arg Glu Gln Tyr Gly Asp Asp Gln Val
355 360 365

Lys Val Tyr Lys Ser Ser Phe Thr Ala Met Tyr Thr Ala Val Thr Thr
370 375 380

His Arg Gln Pro Cys Arg Met Lys Leu Val Cys Val Gly Ser Glu Glu
385 390 395 400

Lys Ile Val Gly Ile His Gly Ile Gly Phe Gly Met Asp Glu Met Leu
405 410 415

Gln Gly Phe Ala Val Ala Leu Lys Met Gly Ala Thr Lys Lys Asp Phe
420 425 430

Asp Asn Thr Val Ala Ile His Pro Thr Ala Ala Glu Glu Phe Val Thr
435 440 445

Met Arg
450

<210> 89
<211> 648
<212> DNA
<213> Escherichia coli

<400> 89
gtgaagctat acatttacga tcactgccct tactgcctca aagcccgcat gattttcggc 60

ctgaaaaata tccccgtcga attacatgtt ctgctcaacg acgacgcaga aacacccacc 120

cggatggtcg gtcaaaaaca ggttcccatt ctgcaaaaag atgacagccg ctatatgcca 180

gaaagcatgg acatcgttca ctatgtcgat aaactcgacg gcaaaccgtt actgaccggc 240

aaacgttccc ctgccattga agagtggctg cgcaaggtca atggctacgc caacaaactg 300

ctgttgccgc gttttgccaa atcggcattt gatgagtttt ctactcccgc cgcgcgcaaa 360

tatttcgtcg acaagaaaga ggccagcgcg ggtaattttg ccgacctgct ggcccactct 420

gacggtctga ttaagaatat cagcgatgat ttacgtgcgc tggacaaact gatcgtcaaa 480

ccgaacgccg tgaatggcga actttcggaa gatgatattc agctattccc gctactgcgt 540

aatctgacgc tggtagccgg aattaactgg ccaagccgcg ttgctgatta ccgcgataat 600

atggcgaaac agacacaaat caatttgtta tcatcaatgg cgatttaa 648

<210> 90
<211> 215
<212> PRT
<213> Escherichia coli

<400> 90

Met Lys Leu Tyr Ile Tyr Asp His Cys Pro Tyr Cys Leu Lys Ala Arg
1 5 10 15

Met Ile Phe Gly Leu Lys Asn Ile Pro Val Glu Leu His Val Leu Leu
20 25 30

Asn Asp Asp Ala Glu Thr Pro Thr Arg Met Val Gly Gln Lys Gln Val
35 40 45

Pro Ile Leu Gln Lys Asp Asp Ser Arg Tyr Met Pro Glu Ser Met Asp
50 55 60

Ile Val His Tyr Val Asp Lys Leu Asp Gly Lys Pro Leu Leu Thr Gly
65 70 75 80

Lys Arg Ser Pro Ala Ile Glu Glu Trp Leu Arg Lys Val Asn Gly Tyr
85 90 95

Ala Asn Lys Leu Leu Leu Pro Arg Phe Ala Lys Ser Ala Phe Asp Glu
100 105 110

Phe Ser Thr Pro Ala Ala Arg Lys Tyr Phe Val Asp Lys Lys Glu Ala
115 120 125

Ser Ala Gly Asn Phe Ala Asp Leu Leu Ala His Ser Asp Gly Leu Ile
130 135 140

Lys Asn Ile Ser Asp Asp Leu Arg Ala Leu Asp Lys Leu Ile Val Lys

145 150 155 160

Pro Asn Ala Val Asn Gly Glu Leu Ser Glu Asp Asp Ile Gln Leu Phe
165 170 175

Pro Leu Leu Arg Asn Leu Thr Leu Val Ala Gly Ile Asn Trp Pro Ser
180 185 190

Arg Val Ala Asp Tyr Arg Asp Asn Met Ala Lys Gln Thr Gln Ile Asn
195 200 205

Leu Leu Ser Ser Met Ala Ile
210 215

<210> 91
<211> 1374
<212> DNA
<213> Escherichia coli

<400> 91
gtggatcatt tgcctatatt ttgccaatta cgcgatcgcg actgtctgat tgtcggcggt 60

ggtgatgtcg cggaacgcaa agcaaggttg ctgttagacg caggcgctcg cttaacggtg 120

aatgcattag cgtttattcc acagttcacc gcatgggcag atgcaggcat gttaaccctc 180

gtcgaagggc catttgatga aagccttctc gacacctgct ggctggcgat tgcagcgacg 240

gatgatgacg cgcttaacca gcgcgtcagc gaagccgctg aagctcgtcg catcttctgt 300

aacgtggtcg atgcgccgaa agccgccagc tttattatgc cgtcgattat tgaccgctca 360

ccgctcatgg tagcggtctc ctctggcggc acctctccgg ttctggcacg cctgttgcgc 420

gaaaaacttg aatcactgct gccgttacat ctgggccagg tagcgaaata cgccgggcaa 480

ttacgcgggc gagtgaaaca acagttcgcc acgatgggtg agcgtcgccg tttctgggag 540

aaattgttcg ttaacgaccg cctggcgcag tcgctggcaa acaacgatca gaaagccatt 600

actgaaacga ccgaacagtt aatcaacgaa ccgctcgacc atcgcggtga agtggtgctg 660

gttggtgcag gtccgggcga tgccgggctg ctgacactga aaggactgca acaaattcag 720

caggcagatg tggtggtcta cgaccgtctg gtttctgacg atattatgaa tctggtacgc 780

cgcgatgcgg accgtgtttt cgtcggcaaa cgcgcgggat accactgcgt accccaggaa 840

gagattaacc agatcctgct gcgggaagcg caaaaaggca aacgcgtggt gcggctgaaa 900

ggtggcgatc cgtttatttt tggccgtggt ggcgaagagc tggaaacact gtgcaacgcg 960

ggtattccgt tctcggtggt tccgggtatt accgcagctt ctggttgctc tgcctattcg 1020

ggtattccac tcacgcatcg cgattatgcc cagagcgtac gcttaattac cggacactta 1080

aaaaccggtg gcgagctgga ctgggaaaac ctggcggcag aaaaacagac gctggtgttc 1140

tatatggggt tgaatcaggc cgcgactatt cagcaaaagc tgattgaaca cggaatgcca 1200

ggcgaaatgc cggtggcaat tgtcgaaaac ggtacggcag tcacgcagcg cgtgattgac 1260

ggtacgctca cacagctggg agaactggcg cagcaaatga acagtccatc gctaattatt 1320

attggtcggg ttgttggcct gcgcgataaa ctgaactggt tctccaacca ttaa 1374

<210> 92
<211> 457
<212> PRT
<213> Escherichia coli

<400> 92

Met Asp His Leu Pro Ile Phe Cys Gln Leu Arg Asp Arg Asp Cys Leu
1 5 10 15

Ile Val Gly Gly Gly Asp Val Ala Glu Arg Lys Ala Arg Leu Leu Leu
20 25 30

Asp Ala Gly Ala Arg Leu Thr Val Asn Ala Leu Ala Phe Ile Pro Gln
35 40 45

Phe Thr Ala Trp Ala Asp Ala Gly Met Leu Thr Leu Val Glu Gly Pro
50 55 60

Phe Asp Glu Ser Leu Leu Asp Thr Cys Trp Leu Ala Ile Ala Ala Thr
65 70 75 80

Asp Asp Asp Ala Leu Asn Gln Arg Val Ser Glu Ala Ala Glu Ala Arg
85 90 95

Arg Ile Phe Cys Asn Val Val Asp Ala Pro Lys Ala Ala Ser Phe Ile
100 105 110

Met Pro Ser Ile Ile Asp Arg Ser Pro Leu Met Val Ala Val Ser Ser
115 120 125

Gly Gly Thr Ser Pro Val Leu Ala Arg Leu Leu Arg Glu Lys Leu Glu
130 135 140

Ser Leu Leu Pro Leu His Leu Gly Gln Val Ala Lys Tyr Ala Gly Gln
145 150 155 160

Leu Arg Gly Arg Val Lys Gln Gln Phe Ala Thr Met Gly Glu Arg Arg
165 170 175

Arg Phe Trp Glu Lys Leu Phe Val Asn Asp Arg Leu Ala Gln Ser Leu
180 185 190

Ala Asn Asn Asp Gln Lys Ala Ile Thr Glu Thr Thr Glu Gln Leu Ile
        195                 200                 205

Asn Glu Pro Leu Asp His Arg Gly Glu Val Val Leu Val Gly Ala Gly
    210                 215                 220

Pro Gly Asp Ala Gly Leu Leu Thr Leu Lys Gly Leu Gln Gln Ile Gln
225                 230                 235                 240

Gln Ala Asp Val Val Val Tyr Asp Arg Leu Val Ser Asp Asp Ile Met
                245                 250                 255

Asn Leu Val Arg Arg Asp Ala Asp Arg Val Phe Val Gly Lys Arg Ala
            260                 265                 270

Gly Tyr His Cys Val Pro Gln Glu Glu Ile Asn Gln Ile Leu Leu Arg
        275                 280                 285

Glu Ala Gln Lys Gly Lys Arg Val Val Arg Leu Lys Gly Gly Asp Pro
    290                 295                 300

Phe Ile Phe Gly Arg Gly Gly Glu Glu Leu Glu Thr Leu Cys Asn Ala
305                 310                 315                 320

Gly Ile Pro Phe Ser Val Val Pro Gly Ile Thr Ala Ala Ser Gly Cys
                325                 330                 335

Ser Ala Tyr Ser Gly Ile Pro Leu Thr His Arg Asp Tyr Ala Gln Ser
            340                 345                 350

Val Arg Leu Ile Thr Gly His Leu Lys Thr Gly Gly Glu Leu Asp Trp
        355                 360                 365

Glu Asn Leu Ala Ala Glu Lys Gln Thr Leu Val Phe Tyr Met Gly Leu
    370                 375                 380

Asn Gln Ala Ala Thr Ile Gln Gln Lys Leu Ile Glu His Gly Met Pro
385                 390                 395                 400

Gly Glu Met Pro Val Ala Ile Val Glu Asn Gly Thr Ala Val Thr Gln
                405                 410                 415

Arg Val Ile Asp Gly Thr Leu Thr Gln Leu Gly Glu Leu Ala Gln Gln
            420                 425                 430

Met Asn Ser Pro Ser Leu Ile Ile Ile Gly Arg Val Val Gly Leu Arg
        435                 440                 445

Asp Lys Leu Asn Trp Phe Ser Asn His
    450                 455

<210> 93
<211> 975
<212> DNA
<213> Escherichia coli

<400> 93
atgaaattac aacaacttcg ctatattgtt gaggtggtca atcataacct gaatgtctca 60

tcaacagcgg aaggacttta cacatcacaa cccgggatca gtaaacaagt cagaatgctg 120

gaagacgagc taggcattca aatttttcc cgaagcggca agcacctgac gcaggtaacg 180

ccagcagggc aagaaataat tcgtatcgct cgcgaagtcc tgtcgaaagt cgatgccata 240

aaatcggttg ccggagagca cacctggccg gataaaggtt cactgtatat cgccaccacg 300

catacccagg cacgctacgc attaccaaac gtcatcaaag gctttattga gcgttatcct 360

cgcgtttctt tgcatatgca ccagggctcg ccgacacaaa ttgctgatgc cgtctctaaa 420

ggcaatgctg atttcgctat cgccacagaa gcgctgcatc tgtatgaaga tttagtgatg 480

ttaccgtgct accactggaa tcgggctatt gtagtcactc cggatcaccc gctggcaggc 540

aaaaaagcca ttaccattga agaactggcg caatatccgt tggtgacata taccttcggc 600

tttaccggac gttcagaact ggatactgcc tttaatcgcg cagggttaac gccgcgtatc 660

gttttcacgg caacggatgc tgacgtcatt aaaacttacg tccggttagg gctgggggta 720

ggggtcattg ccagcatggc ggtggatccg gtcgccgatc ccgaccttgt gcgtgttgat 780

gctcacgata tcttcagcca cagtacaacc aaaattggtt ttcgccgtag tactttcttg 840

cgcagttata tgtatgattt cattcagcgt tttgcaccgc atttaacgcg tgatgtcgtt 900

gatgcggctg tcgcattgcg ctctaatgaa gaaattgagg tcatgtttaa agatataaaa 960

ctgccggaaa aataa 975

<210> 94
<211> 324
<212> PRT
<213> Escherichia coli

<400> 94

Met Lys Leu Gln Gln Leu Arg Tyr Ile Val Glu Val Val Asn His Asn
1               5                   10                  15

Leu Asn Val Ser Ser Thr Ala Glu Gly Leu Tyr Thr Ser Gln Pro Gly
            20                  25                  30

Ile Ser Lys Gln Val Arg Met Leu Glu Asp Glu Leu Gly Ile Gln Ile

35 40 45

Phe Ser Arg Ser Gly Lys His Leu Thr Gln Val Thr Pro Ala Gly Gln
50 55 60

Glu Ile Ile Arg Ile Ala Arg Glu Val Leu Ser Lys Val Asp Ala Ile
65 70 75 80

Lys Ser Val Ala Gly Glu His Thr Trp Pro Asp Lys Gly Ser Leu Tyr
85 90 95

Ile Ala Thr Thr His Thr Gln Ala Arg Tyr Ala Leu Pro Asn Val Ile
100 105 110

Lys Gly Phe Ile Glu Arg Tyr Pro Arg Val Ser Leu His Met His Gln
115 120 125

Gly Ser Pro Thr Gln Ile Ala Asp Ala Val Ser Lys Gly Asn Ala Asp
130 135 140

Phe Ala Ile Ala Thr Glu Ala Leu His Leu Tyr Glu Asp Leu Val Met
145 150 155 160

Leu Pro Cys Tyr His Trp Asn Arg Ala Ile Val Val Thr Pro Asp His
165 170 175

Pro Leu Ala Gly Lys Lys Ala Ile Thr Ile Glu Glu Leu Ala Gln Tyr
180 185 190

Pro Leu Val Thr Tyr Thr Phe Gly Phe Thr Gly Arg Ser Glu Leu Asp
195 200 205

Thr Ala Phe Asn Arg Ala Gly Leu Thr Pro Arg Ile Val Phe Thr Ala
210 215 220

Thr Asp Ala Asp Val Ile Lys Thr Tyr Val Arg Leu Gly Leu Gly Val
225 230 235 240

Gly Val Ile Ala Ser Met Ala Val Asp Pro Val Ala Asp Pro Asp Leu
245 250 255

Val Arg Val Asp Ala His Asp Ile Phe Ser His Ser Thr Thr Lys Ile
260 265 270

Gly Phe Arg Arg Ser Thr Phe Leu Arg Ser Tyr Met Tyr Asp Phe Ile
275 280 285

Gln Arg Phe Ala Pro His Leu Thr Arg Asp Val Val Asp Ala Ala Val
290 295 300

Ala Leu Arg Ser Asn Glu Glu Ile Glu Val Met Phe Lys Asp Ile Lys
305 310 315 320

Leu Pro Glu Lys

<210> 95
<211> 975
<212> DNA
<213> Escherichia coli

<400> 95
atgaaattac aacaacttcg ctatattgtt gaggtggtca atcataacct gaatgtctca 60

tcaacagcgg aaggacttta cacatcacaa cccgggatca gtaaacaagt cagaatgctg 120

gaagacgagc taggcattca aatttttcc cgaagcggca agcacctgac gcaggtaacg 180

ccagcagggc aagaaataat tcgtatcgct cgcgaagtcc tgtcgaaagt cgatgccata 240

aaatcggttg ccggagagca cacctggccg gataaaggtt cactgtatat cgccaccacg 300

catacccagg cacgctacgc attaccaaac gtcatcaaag gctttattga gcgttatcct 360

cgcgtttctt tgcatatgca ccagggctcg ccgacacaaa ttgctgatgc cgtctctaaa 420

ggcaatgctg atttcgctat cgcccaagaa gcgctgcatc tgtatgaaga tttagtgatg 480

ttaccgtgct accactggaa tcgggctatt gtagtcactc cggatcaccc gctggcaggc 540

aaaaaagcca ttaccattga agaactggcg caatatccgt tggtgacata taccttcggc 600

tttaccggac gttcagaact ggatactgcc tttaatcgcg cagggttaac gccgcgtatc 660

gttttcacgg caacggatgc tgacgtcatt aaaacttacg tccggttagg gctgggggta 720

ggggtcattg ccagcatggc ggtggatccg gtcgccgatc ccgaccttgt gcgtgttgat 780

gctcacgata tcttcagcca cagtacaacc aaaattggtt ttcgccgtag tactttcttg 840

cgcagttata tgtatgattt cattcagcgt tttgcaccgc atttaacgcg tgatgtcgtt 900

gatgcggctg tcgcattgcg ctctaatgaa gaaattgagg tcatgtttaa agatataaaa 960

ctgccggaaa aataa 975

<210> 96
<211> 324
<212> PRT
<213> Escherichia coli

<400> 96

Met Lys Leu Gln Gln Leu Arg Tyr Ile Val Glu Val Val Asn His Asn
1 5 10 15

```
Leu Asn Val Ser Ser Thr Ala Glu Gly Leu Tyr Thr Ser Gln Pro Gly
            20                  25                  30

Ile Ser Lys Gln Val Arg Met Leu Glu Asp Glu Leu Gly Ile Gln Ile
        35                  40                  45

Phe Ser Arg Ser Gly Lys His Leu Thr Gln Val Thr Pro Ala Gly Gln
    50                  55                  60

Glu Ile Ile Arg Ile Ala Arg Glu Val Leu Ser Lys Val Asp Ala Ile
65                  70                  75                  80

Lys Ser Val Ala Gly Glu His Thr Trp Pro Asp Lys Gly Ser Leu Tyr
                85                  90                  95

Ile Ala Thr Thr His Thr Gln Ala Arg Tyr Ala Leu Pro Asn Val Ile
            100                 105                 110

Lys Gly Phe Ile Glu Arg Tyr Pro Arg Val Ser Leu His Met His Gln
        115                 120                 125

Gly Ser Pro Thr Gln Ile Ala Asp Ala Val Ser Lys Gly Asn Ala Asp
    130                 135                 140

Phe Ala Ile Ala Gln Glu Ala Leu His Leu Tyr Glu Asp Leu Val Met
145                 150                 155                 160

Leu Pro Cys Tyr His Trp Asn Arg Ala Ile Val Val Thr Pro Asp His
                165                 170                 175

Pro Leu Ala Gly Lys Lys Ala Ile Thr Ile Glu Glu Leu Ala Gln Tyr
            180                 185                 190

Pro Leu Val Thr Tyr Thr Phe Gly Phe Thr Gly Arg Ser Glu Leu Asp
        195                 200                 205

Thr Ala Phe Asn Arg Ala Gly Leu Thr Pro Arg Ile Val Phe Thr Ala
    210                 215                 220

Thr Asp Ala Asp Val Ile Lys Thr Tyr Val Arg Leu Gly Leu Gly Val
225                 230                 235                 240

Gly Val Ile Ala Ser Met Ala Val Asp Pro Val Ala Asp Pro Asp Leu
                245                 250                 255

Val Arg Val Asp Ala His Asp Ile Phe Ser His Ser Thr Thr Lys Ile
            260                 265                 270
```

Gly Phe Arg Arg Ser Thr Phe Leu Arg Ser Tyr Met Tyr Asp Phe Ile
275 280 285

Gln Arg Phe Ala Pro His Leu Thr Arg Asp Val Val Asp Ala Ala Val
290 295 300

Ala Leu Arg Ser Asn Glu Glu Ile Glu Val Met Phe Lys Asp Ile Lys
305 310 315 320

Leu Pro Glu Lys

<210> 97
<211> 975
<212> DNA
<213> Escherichia coli

<400> 97
atgaaattac aacaacttcg ctatattgtt gaggtggtca atcataacct gaatgtctca 60
tcaacagcgg aaggacttta cacatcacaa cccgggatca gtaaacaagt cagaatgctg 120
gaagacgagc taggcattca aatttttcc cgaagcggca agcacctgac gcaggtaacg 180
ccagcagggc aagaaataat tcgtatcgct cgcgaagtcc tgtcgaaagt cgatgccata 240
aaatcggttg ccggagagca cacctggccg gataaaggtt cactgtatat cgccaccacg 300
catacccagg cacgctacgc attaccaaac gtcatcaaag gctttattga gcgttatcct 360
cgcgtttctt tgcatatgca ccagggctcg ccgacacaaa ttgctgatgc cgtctctaaa 420
ggcaatgctg atttcgctat cgccccagaa gcgctgcatc tgtatgaaga tttagtgatg 480
ttaccgtgct accactggaa tcgggctatt gtagtcactc cggatcaccc gctggcaggc 540
aaaaaagcca ttaccattga agaactggcg caatatccgt tggtgacata taccttcggc 600
tttaccggac gttcagaact ggatactgcc tttaatcgcg cagggttaac gccgcgtatc 660
gttttcacgg caacggatgc tgacgtcatt aaaacttacg tccggttagg gctgggggta 720
ggggtcattg ccagcatggc ggtggatccg gtcgccgatc ccgaccttgt gcgtgttgat 780
gctcacgata tcttcagcca cagtacaacc aaaattggtt ttcgccgtag tactttcttg 840
cgcagttata tgtatgattt cattcagcgt tttgcaccgc atttaacgcg tgatgtcgtt 900
gatgcggctg tcgcattgcg ctctaatgaa gaaattgagg tcatgtttaa agatataaaa 960
ctgccggaaa aataa 975

<210> 98
<211> 324
<212> PRT
<213> Escherichia coli

<400> 98

Met Lys Leu Gln Gln Leu Arg Tyr Ile Val Glu Val Val Asn His Asn
1 5 10 15

Leu Asn Val Ser Ser Thr Ala Glu Gly Leu Tyr Thr Ser Gln Pro Gly
20 25 30

Ile Ser Lys Gln Val Arg Met Leu Glu Asp Glu Leu Gly Ile Gln Ile
35 40 45

Phe Ser Arg Ser Gly Lys His Leu Thr Gln Val Thr Pro Ala Gly Gln
50 55 60

Glu Ile Ile Arg Ile Ala Arg Glu Val Leu Ser Lys Val Asp Ala Ile
65 70 75 80

Lys Ser Val Ala Gly Glu His Thr Trp Pro Asp Lys Gly Ser Leu Tyr
85 90 95

Ile Ala Thr Thr His Thr Gln Ala Arg Tyr Ala Leu Pro Asn Val Ile
100 105 110

Lys Gly Phe Ile Glu Arg Tyr Pro Arg Val Ser Leu His Met His Gln
115 120 125

Gly Ser Pro Thr Gln Ile Ala Asp Ala Val Ser Lys Gly Asn Ala Asp
130 135 140

Phe Ala Ile Ala Pro Glu Ala Leu His Leu Tyr Glu Asp Leu Val Met
145 150 155 160

Leu Pro Cys Tyr His Trp Asn Arg Ala Ile Val Val Thr Pro Asp His
165 170 175

Pro Leu Ala Gly Lys Lys Ala Ile Thr Ile Glu Glu Leu Ala Gln Tyr
180 185 190

Pro Leu Val Thr Tyr Thr Phe Gly Phe Thr Gly Arg Ser Glu Leu Asp
195 200 205

Thr Ala Phe Asn Arg Ala Gly Leu Thr Pro Arg Ile Val Phe Thr Ala
210 215 220

Thr Asp Ala Asp Val Ile Lys Thr Tyr Val Arg Leu Gly Leu Gly Val
225 230 235 240

Gly Val Ile Ala Ser Met Ala Val Asp Pro Val Ala Asp Pro Asp Leu
245 250 255

Val Arg Val Asp Ala His Asp Ile Phe Ser His Ser Thr Thr Lys Ile
260 265 270

Gly Phe Arg Arg Ser Thr Phe Leu Arg Ser Tyr Met Tyr Asp Phe Ile
275 280 285

Gln Arg Phe Ala Pro His Leu Thr Arg Asp Val Val Asp Ala Ala Val
290 295 300

Ala Leu Arg Ser Asn Glu Glu Ile Glu Val Met Phe Lys Asp Ile Lys
305 310 315 320

Leu Pro Glu Lys

<210> 99
<211> 1095
<212> DNA
<213> Escherichia coli

<400> 99
atggcacaac agactccttt gtacgaacaa cacacgcttt gcggcgctcg catggtggat 60

ttccacggct ggatgatgcc gctgcattac ggttcgcaaa tcgacgaaca tcatgcggta 120

cgtaccgatg ccggaatgtt tgatgtgtca catatgacca tcgtcgatct tcgcggcagc 180

cgcacccggg agtttctgcg ttatctgctg gcgaacgatg tggcgaagct caccaaaagc 240

ggcaaagccc tttactcggg gatgttgaat gcctctggcg gtgtgataga tgacctcatc 300

gtctactact ttactgaaga tttcttccgc ctcgttgtta actccgccac ccgcgaaaaa 360

gacctctcct ggattaccca acacgctgaa cctttcggca tcgaaattac cgttcgtgat 420

gacctttcca tgattgccgt gcaagggccg aatgcgcagg caaaagctgc cacactgttt 480

aatgacgccc agcgtcaggc ggtggaaggg atgaaaccgt tctttggcgt gcaggcgggc 540

gatctgttta ttgccaccac tggttatacc ggtgaagcgg gctatgaaat tgcgctgccc 600

aatgaaaaag cggccgattt ctggcgtgcg ctggtggaag cgggtgttaa gccatgtggc 660

ttgggcgcgc gtgacacgct gcgtctggaa gcgggcatga atctttatgg tcaggagatg 720

gacgaaacca tctctccttt agccgccaac atgggctgga ccatcgcctg ggaaccggca 780

gatcgtgact ttatcggtcg tgaagccctg gaagtgcagc gtgagcatgg tacagaaaaa 840

ctggttggtc tggtgatgac cgaaaaaggc gtgctgcgta atgaactgcc ggtacgcttt 900

accgatgcgc agggcaacca gcatgaaggc attatcacca gcggtacttt ctccccgacg 960

ctgggttaca gcattgcgct ggcgcgcgtg ccggaaggta ttggcgaaac ggcgattgtg 1020

caaattcgca accgtgaaat gccggttaaa gtgacaaaac ctgtttttgt gcgtaacggc 1080

aaagccgtcg cgtga 1095

<210> 100
<211> 364
<212> PRT
<213> Escherichia coli

<400> 100

Met Ala Gln Gln Thr Pro Leu Tyr Glu Gln His Thr Leu Cys Gly Ala
1 5 10 15

Arg Met Val Asp Phe His Gly Trp Met Met Pro Leu His Tyr Gly Ser
20 25 30

Gln Ile Asp Glu His His Ala Val Arg Thr Asp Ala Gly Met Phe Asp
35 40 45

Val Ser His Met Thr Ile Val Asp Leu Arg Gly Ser Arg Thr Arg Glu
50 55 60

Phe Leu Arg Tyr Leu Leu Ala Asn Asp Val Ala Lys Leu Thr Lys Ser
65 70 75 80

Gly Lys Ala Leu Tyr Ser Gly Met Leu Asn Ala Ser Gly Gly Val Ile
85 90 95

Asp Asp Leu Ile Val Tyr Tyr Phe Thr Glu Asp Phe Phe Arg Leu Val
100 105 110

Val Asn Ser Ala Thr Arg Glu Lys Asp Leu Ser Trp Ile Thr Gln His
115 120 125

Ala Glu Pro Phe Gly Ile Glu Ile Thr Val Arg Asp Asp Leu Ser Met
130 135 140

Ile Ala Val Gln Gly Pro Asn Ala Gln Ala Lys Ala Ala Thr Leu Phe
145 150 155 160

Asn Asp Ala Gln Arg Gln Ala Val Glu Gly Met Lys Pro Phe Phe Gly
165 170 175

Val Gln Ala Gly Asp Leu Phe Ile Ala Thr Thr Gly Tyr Thr Gly Glu
180 185 190

Ala Gly Tyr Glu Ile Ala Leu Pro Asn Glu Lys Ala Ala Asp Phe Trp
195 200 205

```
Arg Ala Leu Val Glu Ala Gly Val Lys Pro Cys Gly Leu Gly Ala Arg
    210                 215                 220

Asp Thr Leu Arg Leu Glu Ala Gly Met Asn Leu Tyr Gly Gln Glu Met
225                 230                 235                 240

Asp Glu Thr Ile Ser Pro Leu Ala Ala Asn Met Gly Trp Thr Ile Ala
                245                 250                 255

Trp Glu Pro Ala Asp Arg Asp Phe Ile Gly Arg Glu Ala Leu Glu Val
            260                 265                 270

Gln Arg Glu His Gly Thr Glu Lys Leu Val Gly Leu Val Met Thr Glu
        275                 280                 285

Lys Gly Val Leu Arg Asn Glu Leu Pro Val Arg Phe Thr Asp Ala Gln
    290                 295                 300

Gly Asn Gln His Glu Gly Ile Ile Thr Ser Gly Thr Phe Ser Pro Thr
305                 310                 315                 320

Leu Gly Tyr Ser Ile Ala Leu Ala Arg Val Pro Glu Gly Ile Gly Glu
                325                 330                 335

Thr Ala Ile Val Gln Ile Arg Asn Arg Glu Met Pro Val Lys Val Thr
            340                 345                 350

Lys Pro Val Phe Val Arg Asn Gly Lys Ala Val Ala
        355                 360


<210>  101
<211>  390
<212>  DNA
<213>  Escherichia coli

<400>  101
atgagcaacg taccagcaga actgaaatac agcaaagaac acgaatggct gcgtaaagaa        60

gccgacggca cttacaccgt tggtattacc gaacatgctc aggagctgtt aggcgatatg       120

gtgtttgttg acctgccgga agtgggcgca acggttagcg cgggcgatga ctgcgcggtt       180

gccgaatcgg taaaagcggc gtcagacatt tatgcgccag taagcggtga aatcgtggcg       240

gtaaacgacg cactgagcga ttccccggaa ctggtgaaca gcgaaccgta tgcaggcggc       300

tggatcttta aaatcaaagc cagcgatgaa agcgaactgg aatcactgct ggatgcgacc       360

gcatacgaag cattgttaga agacgagtaa                                        390
```

<210> 102
<211> 129
<212> PRT
<213> Escherichia coli

<400> 102

Met Ser Asn Val Pro Ala Glu Leu Lys Tyr Ser Lys Glu His Glu Trp
1 5 10 15

Leu Arg Lys Glu Ala Asp Gly Thr Tyr Thr Val Gly Ile Thr Glu His
20 25 30

Ala Gln Glu Leu Leu Gly Asp Met Val Phe Val Asp Leu Pro Glu Val
35 40 45

Gly Ala Thr Val Ser Ala Gly Asp Asp Cys Ala Val Ala Glu Ser Val
50 55 60

Lys Ala Ala Ser Asp Ile Tyr Ala Pro Val Ser Gly Glu Ile Val Ala
65 70 75 80

Val Asn Asp Ala Leu Ser Asp Ser Pro Glu Leu Val Asn Ser Glu Pro
85 90 95

Tyr Ala Gly Gly Trp Ile Phe Lys Ile Lys Ala Ser Asp Glu Ser Glu
100 105 110

Leu Glu Ser Leu Leu Asp Ala Thr Ala Tyr Glu Ala Leu Leu Glu Asp
115 120 125

Glu

<210> 103
<211> 2874
<212> DNA
<213> Escherichia coli

<400> 103
atgacacaga cgttaagcca gcttgaaaac agcggcgctt ttattgaacg ccatatcgga 60

ccggacgccg cgcaacagca agaaatgctg aatgccgttg gtgcacaatc gttaaacgcg 120

ctgaccggcc agattgtgcc gaaagatatt caacttgcga caccaccgca ggttggcgca 180

ccggcgaccg aatacgccgc actggcagaa ctcaaggcta ttgccagtcg caataaacgc 240

ttcacgtctt acatcggcat gggttacacc gccgtgcagc taccgccggt tatcctgcgt 300

aacatgctgg aaaatccggg ctggtatacc gcgtacactc cgtatcaacc tgaagtctcc 360

cagggccgcc ttgaagcact gctcaacttc cagcaggtaa cgctggattt gactggactg 420

gatatggcct ctgcttctct tctggacgag gccaccgctg ccgccgaagc aatggcgatg 480

gcgaaacgcg tcagcaaact gaaaaatgcc aaccgcttct tcgtggcttc cgatgtgcat 540

ccgcaaacgc tggatgtggt ccgtactcgt gccgaaacct ttggttttga agtgattgtc 600

gatgacgcgc aaaaagtgct cgaccatcag gacgtcttcg gcgtgctgtt acagcaggta 660

ggcactaccg gtgaaattca cgactacact gcgcttatta gcgaactgaa atcacgcaaa 720

attgtggtca gcgttgccgc cgatattatg gcgctggtgc tgttaactgc gccgggtaaa 780

cagggcgcgg atattgtttt tggttcggcg caacgcttcg gcgtgccgat gggctacggt 840

ggcccacacg cggcattctt tgcggcgaaa gatgaataca aacgctcaat gccgggccgt 900

attatcggtg tatcgaaaga tgcagctggc aataccgcgc tgcgcatggc gatgcagact 960

cgcgagcaac atatccgccg tgagaaagcg aactccaaca tttgtacttc ccaggtactg 1020

ctggcaaaca tcgccagcct gtatgccgtt tatcacggcc cggttggcct gaaacgtatc 1080

gctaaccgca ttcaccgtct gaccgatatc ctggcggcgg gcctgcaaca aaaaggtctg 1140

aaactgcgcc atgcgcacta tttcgacacc ttgtgtgtgg aagtggccga caaagcgggc 1200

gtactgacgc gtgccgaagc ggctgaaatc aacctgcgta gcgatattct gaacgcggtt 1260

gggatcaccc ttgatgaaac aaccacgcgt gaaaacgtaa tgcagctttt caacgtgctg 1320

ctgggcgata accacggcct ggacatcgac acgctggaca aagacgtggc tcacgacagc 1380

cgctctatcc agcctgcgat gctgcgcgac gacgaaatcc tcacccatcc ggtgtttaat 1440

cgctaccaca gcgaaaccga aatgatgcgc tatatgcact cgctggagcg taaagatctg 1500

gcgctgaatc aggcgatgat cccgctgggt tcctgcacca tgaaactgaa cgccgccgcc 1560

gagatgatcc caatcacctg gccggaattt gccgaactgc acccgttctg cccgccggag 1620

caggccgaag gttatcagca gatgattgcg cagctggctg actggctggt gaaactgacc 1680

ggttacgacg ccgtttgtat gcagccgaac tctggcgcac agggcgaata cgcgggcctg 1740

ctggcgattc gtcattatca tgaaagccgc aacgaagggc atcgcgatat ctgcctgatc 1800

ccggcttctg cgcacggaac taaccccgct tctgcacata tggcaggaat gcaggtggtg 1860

gttgtggcgt gtgataaaaa cggcaacatc gatctgactg atctgcgcgc gaaagcggaa 1920

caggcgggcg ataacctctc ctgtatcatg gtgacttatc cttctaccca cggcgtgtat 1980

gaagaaacga tccgtgaagt gtgtgaagtc gtgcatcagt tcggcggtca ggtttacctt 2040

gatggcgcga acatgaacgc ccaggttggc atcacctcgc cgggctttat tggtgcggac 2100

gtttcacacc ttaacctaca taaaactttc tgcattccgc acggcggtgg tggtccgggt 2160

atgggaccga tcggcgtgaa agcgcatttg gcaccgtttg taccgggtca tagcgtggtg 2220

caaatcgaag gcatgttaac ccgtcagggc gcggtttctg cggcaccgtt cggtagcgcc 2280

tctatcctgc caatcagctg gatgtacatc cgcatgatgg gcgcagaagg gctgaaaaaa 2340

gcaagccagg tggcaatcct caacgccaac tatattgcca gccgcctgca ggatgccttc 2400

ccggtgctgt ataccggtcg cgacggtcgc gtggcgcacg aatgtattct cgatattcgc 2460

ccgctgaaag aagaaaccgg catcagcgag ctggatattg ccaagcgcct gatcgactac 2520

ggtttccacg cgccgacgat gtcgttcccg gtggcgggta cgctgatggt tgaaccgact 2580

gaatctgaaa gcaaagtgga actggatcgc tttatcgacg cgatgctggc tatccgcgca 2640

gaaattgacc aggtgaaagc cggtgtctgg ccgctggaag ataacccgct ggtgaacgcg 2700

ccgcacattc agagcgaact ggtcgccgag tgggcgcatc cgtacagccg tgaagttgcg 2760

gtattcccgg caggtgtggc agacaaatac tggccgacag tgaaacgtct ggatgatgtt 2820

tacggcgacc gtaacctgtt ctgctcctgc gtaccgatta gcgaatacca gtaa 2874

<210> 104
<211> 957
<212> PRT
<213> Escherichia coli

<400> 104

Met Thr Gln Thr Leu Ser Gln Leu Glu Asn Ser Gly Ala Phe Ile Glu
1 5 10 15

Arg His Ile Gly Pro Asp Ala Ala Gln Gln Gln Glu Met Leu Asn Ala
20 25 30

Val Gly Ala Gln Ser Leu Asn Ala Leu Thr Gly Gln Ile Val Pro Lys
35 40 45

Asp Ile Gln Leu Ala Thr Pro Pro Gln Val Gly Ala Pro Ala Thr Glu
50 55 60

Tyr Ala Ala Leu Ala Glu Leu Lys Ala Ile Ala Ser Arg Asn Lys Arg
65 70 75 80

Phe Thr Ser Tyr Ile Gly Met Gly Tyr Thr Ala Val Gln Leu Pro Pro
85 90 95

Val Ile Leu Arg Asn Met Leu Glu Asn Pro Gly Trp Tyr Thr Ala Tyr
100 105 110

Thr Pro Tyr Gln Pro Glu Val Ser Gln Gly Arg Leu Glu Ala Leu Leu
115 120 125

Asn Phe Gln Gln Val Thr Leu Asp Leu Thr Gly Leu Asp Met Ala Ser
130 135 140

Ala Ser Leu Leu Asp Glu Ala Thr Ala Ala Ala Glu Ala Met Ala Met
145 150 155 160

Ala Lys Arg Val Ser Lys Leu Lys Asn Ala Asn Arg Phe Phe Val Ala
165 170 175

Ser Asp Val His Pro Gln Thr Leu Asp Val Val Arg Thr Arg Ala Glu
180 185 190

Thr Phe Gly Phe Glu Val Ile Val Asp Asp Ala Gln Lys Val Leu Asp
195 200 205

His Gln Asp Val Phe Gly Val Leu Leu Gln Gln Val Gly Thr Thr Gly
210 215 220

Glu Ile His Asp Tyr Thr Ala Leu Ile Ser Glu Leu Lys Ser Arg Lys
225 230 235 240

Ile Val Val Ser Val Ala Ala Asp Ile Met Ala Leu Val Leu Leu Thr
245 250 255

Ala Pro Gly Lys Gln Gly Ala Asp Ile Val Phe Gly Ser Ala Gln Arg
260 265 270

Phe Gly Val Pro Met Gly Tyr Gly Gly Pro His Ala Ala Phe Phe Ala
275 280 285

Ala Lys Asp Glu Tyr Lys Arg Ser Met Pro Gly Arg Ile Ile Gly Val
290 295 300

Ser Lys Asp Ala Ala Gly Asn Thr Ala Leu Arg Met Ala Met Gln Thr
305 310 315 320

Arg Glu Gln His Ile Arg Arg Glu Lys Ala Asn Ser Asn Ile Cys Thr
325 330 335

Ser Gln Val Leu Leu Ala Asn Ile Ala Ser Leu Tyr Ala Val Tyr His
340 345 350

Gly Pro Val Gly Leu Lys Arg Ile Ala Asn Arg Ile His Arg Leu Thr
355 360 365

Asp Ile Leu Ala Ala Gly Leu Gln Gln Lys Gly Leu Lys Leu Arg His
370 375 380

Ala His Tyr Phe Asp Thr Leu Cys Val Glu Val Ala Asp Lys Ala Gly
385 390 395 400

```
Val Leu Thr Arg Ala Glu Ala Ala Glu Ile Asn Leu Arg Ser Asp Ile
                405                 410                 415

Leu Asn Ala Val Gly Ile Thr Leu Asp Glu Thr Thr Thr Arg Glu Asn
            420                 425                 430

Val Met Gln Leu Phe Asn Val Leu Leu Gly Asp Asn His Gly Leu Asp
        435                 440                 445

Ile Asp Thr Leu Asp Lys Asp Val Ala His Asp Ser Arg Ser Ile Gln
    450                 455                 460

Pro Ala Met Leu Arg Asp Asp Glu Ile Leu Thr His Pro Val Phe Asn
465                 470                 475                 480

Arg Tyr His Ser Glu Thr Glu Met Met Arg Tyr Met His Ser Leu Glu
                485                 490                 495

Arg Lys Asp Leu Ala Leu Asn Gln Ala Met Ile Pro Leu Gly Ser Cys
            500                 505                 510

Thr Met Lys Leu Asn Ala Ala Ala Glu Met Ile Pro Ile Thr Trp Pro
        515                 520                 525

Glu Phe Ala Glu Leu His Pro Phe Cys Pro Pro Glu Gln Ala Glu Gly
    530                 535                 540

Tyr Gln Gln Met Ile Ala Gln Leu Ala Asp Trp Leu Val Lys Leu Thr
545                 550                 555                 560

Gly Tyr Asp Ala Val Cys Met Gln Pro Asn Ser Gly Ala Gln Gly Glu
                565                 570                 575

Tyr Ala Gly Leu Leu Ala Ile Arg His Tyr His Glu Ser Arg Asn Glu
            580                 585                 590

Gly His Arg Asp Ile Cys Leu Ile Pro Ala Ser Ala His Gly Thr Asn
        595                 600                 605

Pro Ala Ser Ala His Met Ala Gly Met Gln Val Val Val Val Ala Cys
    610                 615                 620

Asp Lys Asn Gly Asn Ile Asp Leu Thr Asp Leu Arg Ala Lys Ala Glu
625                 630                 635                 640

Gln Ala Gly Asp Asn Leu Ser Cys Ile Met Val Thr Tyr Pro Ser Thr
                645                 650                 655
```

His Gly Val Tyr Glu Glu Thr Ile Arg Glu Val Cys Glu Val Val His
660 665 670

Gln Phe Gly Gly Gln Val Tyr Leu Asp Gly Ala Asn Met Asn Ala Gln
675 680 685

Val Gly Ile Thr Ser Pro Gly Phe Ile Gly Ala Asp Val Ser His Leu
690 695 700

Asn Leu His Lys Thr Phe Cys Ile Pro His Gly Gly Gly Gly Pro Gly
705 710 715 720

Met Gly Pro Ile Gly Val Lys Ala His Leu Ala Pro Phe Val Pro Gly
725 730 735

His Ser Val Val Gln Ile Glu Gly Met Leu Thr Arg Gln Gly Ala Val
740 745 750

Ser Ala Ala Pro Phe Gly Ser Ala Ser Ile Leu Pro Ile Ser Trp Met
755 760 765

Tyr Ile Arg Met Met Gly Ala Glu Gly Leu Lys Lys Ala Ser Gln Val
770 775 780

Ala Ile Leu Asn Ala Asn Tyr Ile Ala Ser Arg Leu Gln Asp Ala Phe
785 790 795 800

Pro Val Leu Tyr Thr Gly Arg Asp Gly Arg Val Ala His Glu Cys Ile
805 810 815

Leu Asp Ile Arg Pro Leu Lys Glu Glu Thr Gly Ile Ser Glu Leu Asp
820 825 830

Ile Ala Lys Arg Leu Ile Asp Tyr Gly Phe His Ala Pro Thr Met Ser
835 840 845

Phe Pro Val Ala Gly Thr Leu Met Val Glu Pro Thr Glu Ser Glu Ser
850 855 860

Lys Val Glu Leu Asp Arg Phe Ile Asp Ala Met Leu Ala Ile Arg Ala
865 870 875 880

Glu Ile Asp Gln Val Lys Ala Gly Val Trp Pro Leu Glu Asp Asn Pro
885 890 895

Leu Val Asn Ala Pro His Ile Gln Ser Glu Leu Val Ala Glu Trp Ala

900 905 910

His Pro Tyr Ser Arg Glu Val Ala Val Phe Pro Ala Gly Val Ala Asp
915 920 925

Lys Tyr Trp Pro Thr Val Lys Arg Leu Asp Asp Val Tyr Gly Asp Arg
930 935 940

Asn Leu Phe Cys Ser Cys Val Pro Ile Ser Glu Tyr Gln
945 950 955

<210> 105
<211> 1089
<212> DNA
<213> Escherichia coli

<400> 105
atggctcaaa tcttcaattt tagttctggt ccggcaatgc taccggcaga ggtgcttaaa 60

caggctcaac aggaactgcg cgactggaac ggtcttggta cgtcggtgat ggaagtgagt 120

caccgtggca aagagttcat tcaggttgca gaggaagccg agaaggattt tcgcgatctt 180

cttaatgtcc cctccaacta caaggtatta ttctgccatg gcggtggtcg cggtcagttt 240

gctgcggtac cgctgaatat tctcggtgat aaaaccaccg cagattatgt tgatgccggt 300

tactgggcgg caagtgccat taaagaagcg aaaaaatact gcacgcctaa tgtctttgac 360

gccaaagtga ctgttgatgg tctgcgcgcg gttaagccaa tgcgtgaatg gcaactctct 420

gataatgctg cttatatgca ttattgcccg aatgaaacca tcgatggtat cgccatcgac 480

gaaacgccag acttcggcgc agatgtggtg gtcgccgctg acttctcttc aaccattctt 540

tcccgtccga ttgacgtcag ccgttatggt gtaatttacg ctggcgcgca gaaaaatatc 600

ggcccggctg gcctgacaat cgtcatcgtt cgtgaagatt tgctgggcaa agcgaatatc 660

gcgtgtccgt cgattctgga ttattccatc ctcaacgata acggctccat gtttaacacg 720

ccgccgacat ttgcctggta tctatctggt ctggtcttta aatggctgaa agcgaacggc 780

ggtgtagctg aaatggataa aatcaatcag caaaaagcag aactgctata tggggtgatt 840

gataacagcg atttctaccg caatgacgtg gcgaaagcta accgttcgcg gatgaacgtg 900

ccgttccagt tggcggacag tgcgcttgac aaattgttcc ttgaagagtc ttttgctgct 960

ggccttcatg cactgaaagg tcaccgtgtg gtcggcggaa tgcgcgcttc tatttataac 1020

gccatgccgc tggaaggcgt taaagcgctg acagacttca tggttgagtt cgaacgccgt 1080

cacggttaa 1089

<210> 106
<211> 362
<212> PRT

<213> Escherichia coli

<400> 106

Met Ala Gln Ile Phe Asn Phe Ser Ser Gly Pro Ala Met Leu Pro Ala
1               5                   10                  15

Glu Val Leu Lys Gln Ala Gln Gln Glu Leu Arg Asp Trp Asn Gly Leu
            20                  25                  30

Gly Thr Ser Val Met Glu Val Ser His Arg Gly Lys Glu Phe Ile Gln
        35                  40                  45

Val Ala Glu Glu Ala Glu Lys Asp Phe Arg Asp Leu Leu Asn Val Pro
    50                  55                  60

Ser Asn Tyr Lys Val Leu Phe Cys His Gly Gly Gly Arg Gly Gln Phe
65                  70                  75                  80

Ala Ala Val Pro Leu Asn Ile Leu Gly Asp Lys Thr Thr Ala Asp Tyr
                85                  90                  95

Val Asp Ala Gly Tyr Trp Ala Ala Ser Ala Ile Lys Glu Ala Lys Lys
            100                 105                 110

Tyr Cys Thr Pro Asn Val Phe Asp Ala Lys Val Thr Val Asp Gly Leu
        115                 120                 125

Arg Ala Val Lys Pro Met Arg Glu Trp Gln Leu Ser Asp Asn Ala Ala
    130                 135                 140

Tyr Met His Tyr Cys Pro Asn Glu Thr Ile Asp Gly Ile Ala Ile Asp
145                 150                 155                 160

Glu Thr Pro Asp Phe Gly Ala Asp Val Val Val Ala Ala Asp Phe Ser
                165                 170                 175

Ser Thr Ile Leu Ser Arg Pro Ile Asp Val Ser Arg Tyr Gly Val Ile
            180                 185                 190

Tyr Ala Gly Ala Gln Lys Asn Ile Gly Pro Ala Gly Leu Thr Ile Val
        195                 200                 205

Ile Val Arg Glu Asp Leu Leu Gly Lys Ala Asn Ile Ala Cys Pro Ser
    210                 215                 220

Ile Leu Asp Tyr Ser Ile Leu Asn Asp Asn Gly Ser Met Phe Asn Thr
225                 230                 235                 240

Pro Pro Thr Phe Ala Trp Tyr Leu Ser Gly Leu Val Phe Lys Trp Leu
245 250 255

Lys Ala Asn Gly Gly Val Ala Glu Met Asp Lys Ile Asn Gln Gln Lys
260 265 270

Ala Glu Leu Leu Tyr Gly Val Ile Asp Asn Ser Asp Phe Tyr Arg Asn
275 280 285

Asp Val Ala Lys Ala Asn Arg Ser Arg Met Asn Val Pro Phe Gln Leu
290 295 300

Ala Asp Ser Ala Leu Asp Lys Leu Phe Leu Glu Glu Ser Phe Ala Ala
305 310 315 320

Gly Leu His Ala Leu Lys Gly His Arg Val Val Gly Gly Met Arg Ala
325 330 335

Ser Ile Tyr Asn Ala Met Pro Leu Glu Gly Val Lys Ala Leu Thr Asp
340 345 350

Phe Met Val Glu Phe Glu Arg Arg His Gly
355 360

<210> 107
<211> 1233
<212> DNA
<213> Escherichia coli

<400> 107
atggcaaagg tatcgctgga gaaagacaag attaagtttc tgctggtaga aggcgtgcac 60

caaaaggcgc tggaaagcct tcgtgcagct ggttacacca acatcgaatt tcacaaaggc 120

gcgctggatg atgaacaatt aaaagaatcc atccgcgatg cccacttcat cggcctgcga 180

tcccgtaccc atctgactga agacgtgatc aacgccgcag aaaaactggt cgctattggc 240

tgtttctgta tcggaacaaa ccaggttgat ctggatgcgg cggcaaagcg cgggatcccg 300

gtatttaacg caccgttctc aaatacgcgc tctgttgcgg agctggtgat tggcgaactg 360

ctgctgctat tgcgcggcgt gccggaagcc aatgctaaag cgcaccgtgg cgtgtggaac 420

aaactggcgg cgggttcttt tgaagcgcgc ggcaaaaagc tgggtatcat cggctacggt 480

catattggta cgcaattggg cattctggct gaatcgctgg gaatgtatgt ttacttttat 540

gatattgaaa ataaactgcc gctgggcaac gccactcagg tacagcatct ttctgacctg 600

ctgaatatga gcgatgtggt gagtctgcat gtaccagaga atccgtccac caaaaatatg 660

atgggcgcga aagaaatttc actaatgaag cccggctcgc tgctgattaa tgcttcgcgc 720

ggtactgtgg tggatattcc ggcgctgtgt gatgcgctgg cgagcaaaca tctggcgggg 780

gcggcaatcg acgtattccc gacggaaccg gcgaccaata gcgatccatt tacctctccg 840

ctgtgtgaat tcgacaacgt ccttctgacg ccacacattg gcggttcgac tcaggaagcg 900

caggagaata tcggcctgga agttgcgggt aaattgatca agtattctga caatggctca 960

acgctctctg cggtgaactt cccggaagtc tcgctgccac tgcacggtgg gcgtcgtctg 1020

atgcacatcc acgaaaaccg tccgggcgtg ctaactgcgc tgaacaaaat cttcgccgag 1080

cagggcgtca acatcgccgc gcaatatctg caaacttccg cccagatggg ttatgtggtt 1140

attgatattg aagccgacga agacgttgcc gaaaaagcgc tgcaggcaat gaaagctatt 1200

ccgggtacca ttcgcgcccg tctgctgtac taa 1233

<210> 108
<211> 410
<212> PRT
<213> Escherichia coli

<400> 108

Met Ala Lys Val Ser Leu Glu Lys Asp Lys Ile Lys Phe Leu Leu Val
1 5 10 15

Glu Gly Val His Gln Lys Ala Leu Glu Ser Leu Arg Ala Ala Gly Tyr
20 25 30

Thr Asn Ile Glu Phe His Lys Gly Ala Leu Asp Asp Glu Gln Leu Lys
35 40 45

Glu Ser Ile Arg Asp Ala His Phe Ile Gly Leu Arg Ser Arg Thr His
50 55 60

Leu Thr Glu Asp Val Ile Asn Ala Ala Glu Lys Leu Val Ala Ile Gly
65 70 75 80

Cys Phe Cys Ile Gly Thr Asn Gln Val Asp Leu Asp Ala Ala Ala Lys
85 90 95

Arg Gly Ile Pro Val Phe Asn Ala Pro Phe Ser Asn Thr Arg Ser Val
100 105 110

Ala Glu Leu Val Ile Gly Glu Leu Leu Leu Leu Leu Arg Gly Val Pro
115 120 125

Glu Ala Asn Ala Lys Ala His Arg Gly Val Trp Asn Lys Leu Ala Ala
130 135 140

Gly Ser Phe Glu Ala Arg Gly Lys Lys Leu Gly Ile Ile Gly Tyr Gly

145 150 155 160

His Ile Gly Thr Gln Leu Gly Ile Leu Ala Glu Ser Leu Gly Met Tyr
165 170 175

Val Tyr Phe Tyr Asp Ile Glu Asn Lys Leu Pro Leu Gly Asn Ala Thr
180 185 190

Gln Val Gln His Leu Ser Asp Leu Leu Asn Met Ser Asp Val Val Ser
195 200 205

Leu His Val Pro Glu Asn Pro Ser Thr Lys Asn Met Met Gly Ala Lys
210 215 220

Glu Ile Ser Leu Met Lys Pro Gly Ser Leu Leu Ile Asn Ala Ser Arg
225 230 235 240

Gly Thr Val Val Asp Ile Pro Ala Leu Cys Asp Ala Leu Ala Ser Lys
245 250 255

His Leu Ala Gly Ala Ala Ile Asp Val Phe Pro Thr Glu Pro Ala Thr
260 265 270

Asn Ser Asp Pro Phe Thr Ser Pro Leu Cys Glu Phe Asp Asn Val Leu
275 280 285

Leu Thr Pro His Ile Gly Gly Ser Thr Gln Glu Ala Gln Glu Asn Ile
290 295 300

Gly Leu Glu Val Ala Gly Lys Leu Ile Lys Tyr Ser Asp Asn Gly Ser
305 310 315 320

Thr Leu Ser Ala Val Asn Phe Pro Glu Val Ser Leu Pro Leu His Gly
325 330 335

Gly Arg Arg Leu Met His Ile His Glu Asn Arg Pro Gly Val Leu Thr
340 345 350

Ala Leu Asn Lys Ile Phe Ala Glu Gln Gly Val Asn Ile Ala Ala Gln
355 360 365

Tyr Leu Gln Thr Ser Ala Gln Met Gly Tyr Val Val Ile Asp Ile Glu
370 375 380

Ala Asp Glu Asp Val Ala Glu Lys Ala Leu Gln Ala Met Lys Ala Ile
385 390 395 400

Pro Gly Thr Ile Arg Ala Arg Leu Leu Tyr
405 410

<210> 109
<211> 969
<212> DNA
<213> Escherichia coli

<400> 109
atgcctaaca ttacctggtg cgacctgcct gaagatgtct ctttatggcc gggtctgcct 60

ctttcattaa gtggtgatga agtgatgcca ctggattacc acgcaggtcg tagcggctgg 120

ctgctgtatg gtcgtgggct ggataaacaa cgtctgaccc aataccagag caaactgggt 180

gcggcgatgg tgattgttgc cgcctggtgc gtggaagatt atcaggtgat tcgtctggca 240

ggttcactca ccgcacgggc tacacgcctg gcccacgaag cgcagctgga tgtcgccccg 300

ctggggaaaa tcccgcacct gcgcacgccg ggtttgctgg tgatggatat ggactccacc 360

gccatccaga ttgaatgtat tgatgaaatt gccaaactgg ccggaacggg cgagatggtg 420

gcggaagtaa ccgaacgggc gatgcgcggc gaactcgatt ttaccgccag cctgcgcagc 480

cgtgtggcga cgctgaaagg cgctgacgcc aatattctgc aacaggtgcg tgaaaatctg 540

ccgctgatgc caggcttaac gcaactggtg ctcaagctgg aaacgctggg ctggaaagtg 600

gcgattgcct ccggcggctt tactttcttt gctgaatacc tgcgcgacaa gctgcgcctg 660

accgccgtgg tagccaatga actggagatc atggacggta aatttaccgg caatgtgatc 720

ggcgacatcg tagacgcgca gtacaaagcg aaaactctga ctcgcctcgc gcaggagtat 780

gaaatcccgc tggcgcagac cgtggcgatt ggcgatggag ccaatgacct gccgatgatc 840

aaagcggcag ggctggggat tgcctaccat gccaagccaa aagtgaatga aaaggcggaa 900

gtcaccatcc gtcacgctga cctgatgggg gtattctgca tcctctcagg cagcctgaat 960

cagaagtaa 969

<210> 110
<211> 322
<212> PRT
<213> Escherichia coli

<400> 110

Met Pro Asn Ile Thr Trp Cys Asp Leu Pro Glu Asp Val Ser Leu Trp
1 5 10 15

Pro Gly Leu Pro Leu Ser Leu Ser Gly Asp Glu Val Met Pro Leu Asp
20 25 30

Tyr His Ala Gly Arg Ser Gly Trp Leu Leu Tyr Gly Arg Gly Leu Asp
35 40 45

```
Lys Gln Arg Leu Thr Gln Tyr Gln Ser Lys Leu Gly Ala Ala Met Val
    50                  55                  60

Ile Val Ala Ala Trp Cys Val Glu Asp Tyr Gln Val Ile Arg Leu Ala
65                  70                  75                  80

Gly Ser Leu Thr Ala Arg Ala Thr Arg Leu Ala His Glu Ala Gln Leu
                85                  90                  95

Asp Val Ala Pro Leu Gly Lys Ile Pro His Leu Arg Thr Pro Gly Leu
            100                 105                 110

Leu Val Met Asp Met Asp Ser Thr Ala Ile Gln Ile Glu Cys Ile Asp
        115                 120                 125

Glu Ile Ala Lys Leu Ala Gly Thr Gly Glu Met Val Ala Glu Val Thr
    130                 135                 140

Glu Arg Ala Met Arg Gly Glu Leu Asp Phe Thr Ala Ser Leu Arg Ser
145                 150                 155                 160

Arg Val Ala Thr Leu Lys Gly Ala Asp Ala Asn Ile Leu Gln Gln Val
                165                 170                 175

Arg Glu Asn Leu Pro Leu Met Pro Gly Leu Thr Gln Leu Val Leu Lys
            180                 185                 190

Leu Glu Thr Leu Gly Trp Lys Val Ala Ile Ala Ser Gly Gly Phe Thr
        195                 200                 205

Phe Phe Ala Glu Tyr Leu Arg Asp Lys Leu Arg Leu Thr Ala Val Val
    210                 215                 220

Ala Asn Glu Leu Glu Ile Met Asp Gly Lys Phe Thr Gly Asn Val Ile
225                 230                 235                 240

Gly Asp Ile Val Asp Ala Gln Tyr Lys Ala Lys Thr Leu Thr Arg Leu
                245                 250                 255

Ala Gln Glu Tyr Glu Ile Pro Leu Ala Gln Thr Val Ala Ile Gly Asp
            260                 265                 270

Gly Ala Asn Asp Leu Pro Met Ile Lys Ala Ala Gly Leu Gly Ile Ala
        275                 280                 285

Tyr His Ala Lys Pro Lys Val Asn Glu Lys Ala Glu Val Thr Ile Arg
    290                 295                 300
```

His Ala Asp Leu Met Gly Val Phe Cys Ile Leu Ser Gly Ser Leu Asn
305 310 315 320

Gln Lys

<210> 111
<211> 843
<212> DNA
<213> Escherichia coli

<400> 111
atgcattcac tccaacgtaa agttctgcgt actatttgtc cggaccaaaa aggtctgatc 60

gcacgtatta ccaatatttg ctacaagcac gagttaaata tcgtacagaa caatgaattt 120

gttgatcacc gtaccgggcg ctttttttatg cgcacggaac tggaagggat ttttaatgat 180

tccaccctgc tggcggatct cgatagcgca ttgccagaag gctccgtgcg tgagctgaat 240

cctgccggtc gtcgccggat agtgattctg gtcactaaag aagcgcattg ccttggcgat 300

ttgttgatga aagccaatta cggcggcctg gatgtcgaaa tcgcggcagt tattggtaac 360

cacgatactt tacgttctct ggttgagcgt tttgatattc cgtttgagct ggtaagccat 420

gaagggttaa cccgcaacga gcacgatcaa aagatggcgg atgccattga tgcttatcaa 480

cctgactacg tggtgctggc gaagtatatg cgggtattaa cgccggaatt tgtggcacgc 540

ttcccgaata agatcatcaa tattcaccat tcattcctgc cagcgtttat tggcgcacgt 600

ccttatcacc aggcctatga acgtggtgtg aagattattg gcgcaaccgc tcactatgtg 660

aatgacaatc tggacgaagg cccaatcatc atgcaggacg ttattcatgt cgatcatacc 720

tacacagctg aagatatgat gcgcgcaggt cgtgacgtcg agaaaaacgt cttaagtcgt 780

gcactataca aagtactggc acagcgcgtc tttgtttacg gtaatcgaac gattattctt 840

taa 843

<210> 112
<211> 280
<212> PRT
<213> Escherichia coli

<400> 112

Met His Ser Leu Gln Arg Lys Val Leu Arg Thr Ile Cys Pro Asp Gln
1 5 10 15

Lys Gly Leu Ile Ala Arg Ile Thr Asn Ile Cys Tyr Lys His Glu Leu
20 25 30

Asn Ile Val Gln Asn Asn Glu Phe Val Asp His Arg Thr Gly Arg Phe

35 40 45

Phe Met Arg Thr Glu Leu Glu Gly Ile Phe Asn Asp Ser Thr Leu Leu
50 55 60

Ala Asp Leu Asp Ser Ala Leu Pro Glu Gly Ser Val Arg Glu Leu Asn
65 70 75 80

Pro Ala Gly Arg Arg Arg Ile Val Ile Leu Val Thr Lys Glu Ala His
85 90 95

Cys Leu Gly Asp Leu Leu Met Lys Ala Asn Tyr Gly Gly Leu Asp Val
100 105 110

Glu Ile Ala Ala Val Ile Gly Asn His Asp Thr Leu Arg Ser Leu Val
115 120 125

Glu Arg Phe Asp Ile Pro Phe Glu Leu Val Ser His Glu Gly Leu Thr
130 135 140

Arg Asn Glu His Asp Gln Lys Met Ala Asp Ala Ile Asp Ala Tyr Gln
145 150 155 160

Pro Asp Tyr Val Val Leu Ala Lys Tyr Met Arg Val Leu Thr Pro Glu
165 170 175

Phe Val Ala Arg Phe Pro Asn Lys Ile Ile Asn Ile His His Ser Phe
180 185 190

Leu Pro Ala Phe Ile Gly Ala Arg Pro Tyr His Gln Ala Tyr Glu Arg
195 200 205

Gly Val Lys Ile Ile Gly Ala Thr Ala His Tyr Val Asn Asp Asn Leu
210 215 220

Asp Glu Gly Pro Ile Ile Met Gln Asp Val Ile His Val Asp His Thr
225 230 235 240

Tyr Thr Ala Glu Asp Met Met Arg Ala Gly Arg Asp Val Glu Lys Asn
245 250 255

Val Leu Ser Arg Ala Leu Tyr Lys Val Leu Ala Gln Arg Val Phe Val
260 265 270

Tyr Gly Asn Arg Thr Ile Ile Leu
275 280

<210> 113
<211> 1365
<212> DNA
<213> Escherichia coli

<400> 113
gtgattagtc tattcgacat gtttaaggtg gggattggtc cctcatcttc ccataccgta 60

gggcctatga aggcaggtaa acagttcgtc gatgatctgg tcgaaaaagg cttactggat 120

agcgttactc gcgttgccgt ggacgtttat ggttcactgt cgctgacggg taaaggccac 180

cacaccgata tcgccattat tatgggtctt gcaggtaacg aacctgccac cgtggatatc 240

gacagtattc ccggttttat tcgcgacgta gaagagcgcg aacgtctgct gctggcacag 300

ggacggcatg aagtggattt cccgcgcgac aacgggatgc gttttcataa cggcaacctg 360

ccgctgcatg aaaacggtat gcaaatccac gcctataacg gcgatgaagt cgtctacagc 420

aaaacttatt attccatcgg cggcggtttt atcgtcgatg aagaacactt tggtcaggat 480

gctgccaacg aagtaagcgt gccgtatccg ttcaaatctg ccaccgaact gctcgcgtac 540

tgtaatgaaa ccggctattc gctgtctggt ctcgctatgc agaacgaact ggcgctgcac 600

agcaagaaag agatcgacga gtatttcgcg catgtctggc aaaccatgca ggcatgtatc 660

gatcgcggga tgaacaccga aggtgtactg ccaggcccgc tgcgcgtgcc acgtcgtgcg 720

tctgccctgc gccggatgct ggtttccagc gataaactgt ctaacgatcc gatgaatgtc 780

attgactggg taaacatgtt tgcgctggca gttaacgaag aaaacgccgc cggtggtcgt 840

gtggtaactg cgccaaccaa cggtgcctgc ggtatcgttc cggcagtgct ggcttactat 900

gaccacttta ttgaatcggt cagcccggac atctataccc gttactttat ggcagcgggc 960

gcgattggtg cattgtataa aatgaacgcc tctatttccg gtgcggaagt tggttgccag 1020

ggcgaagtgg gtgttgcctg ttcaatggct gctgcgggtc ttgcagaact gctgggcggt 1080

agcccggaac aggtttgcgt ggcggcggaa attggcatgg aacacaacct tggtttaacc 1140

tgcgacccgg ttgcagggca ggttcaggtg ccgtgcattg agcgtaatgc cattgcctct 1200

gtgaaggcga ttaacgccgc gcggatggct ctgcgccgca ccagtgcacc gcgcgtctcg 1260

ctggataagg tcatcgaaac gatgtacgaa accggtaagg acatgaacgc caaataccgc 1320

gaaacctcac gcggtggtct ggcaatcaaa gtccagtgtg actaa 1365

<210> 114
<211> 454
<212> PRT
<213> Escherichia coli

<400> 114

Met Ile Ser Leu Phe Asp Met Phe Lys Val Gly Ile Gly Pro Ser Ser
1 5 10 15

```
Ser His Thr Val Gly Pro Met Lys Ala Gly Lys Gln Phe Val Asp Asp
            20                  25                  30

Leu Val Glu Lys Gly Leu Leu Asp Ser Val Thr Arg Val Ala Val Asp
        35                  40                  45

Val Tyr Gly Ser Leu Ser Leu Thr Gly Lys Gly His His Thr Asp Ile
    50                  55                  60

Ala Ile Ile Met Gly Leu Ala Gly Asn Glu Pro Ala Thr Val Asp Ile
65                  70                  75                  80

Asp Ser Ile Pro Gly Phe Ile Arg Asp Val Glu Glu Arg Glu Arg Leu
                85                  90                  95

Leu Leu Ala Gln Gly Arg His Glu Val Asp Phe Pro Arg Asp Asn Gly
            100                 105                 110

Met Arg Phe His Asn Gly Asn Leu Pro Leu His Glu Asn Gly Met Gln
        115                 120                 125

Ile His Ala Tyr Asn Gly Asp Glu Val Val Tyr Ser Lys Thr Tyr Tyr
    130                 135                 140

Ser Ile Gly Gly Gly Phe Ile Val Asp Glu Glu His Phe Gly Gln Asp
145                 150                 155                 160

Ala Ala Asn Glu Val Ser Val Pro Tyr Pro Phe Lys Ser Ala Thr Glu
                165                 170                 175

Leu Leu Ala Tyr Cys Asn Glu Thr Gly Tyr Ser Leu Ser Gly Leu Ala
            180                 185                 190

Met Gln Asn Glu Leu Ala Leu His Ser Lys Lys Glu Ile Asp Glu Tyr
        195                 200                 205

Phe Ala His Val Trp Gln Thr Met Gln Ala Cys Ile Asp Arg Gly Met
    210                 215                 220

Asn Thr Glu Gly Val Leu Pro Gly Pro Leu Arg Val Pro Arg Arg Ala
225                 230                 235                 240

Ser Ala Leu Arg Arg Met Leu Val Ser Ser Asp Lys Leu Ser Asn Asp
                245                 250                 255

Pro Met Asn Val Ile Asp Trp Val Asn Met Phe Ala Leu Ala Val Asn
            260                 265                 270
```

Glu Glu Asn Ala Ala Gly Gly Arg Val Val Thr Ala Pro Thr Asn Gly
        275                 280                 285

Ala Cys Gly Ile Val Pro Ala Val Leu Ala Tyr Tyr Asp His Phe Ile
    290                 295                 300

Glu Ser Val Ser Pro Asp Ile Tyr Thr Arg Tyr Phe Met Ala Ala Gly
305                 310                 315                 320

Ala Ile Gly Ala Leu Tyr Lys Met Asn Ala Ser Ile Ser Gly Ala Glu
                325                 330                 335

Val Gly Cys Gln Gly Glu Val Gly Val Ala Cys Ser Met Ala Ala Ala
            340                 345                 350

Gly Leu Ala Glu Leu Leu Gly Gly Ser Pro Glu Gln Val Cys Val Ala
        355                 360                 365

Ala Glu Ile Gly Met Glu His Asn Leu Gly Leu Thr Cys Asp Pro Val
    370                 375                 380

Ala Gly Gln Val Gln Val Pro Cys Ile Glu Arg Asn Ala Ile Ala Ser
385                 390                 395                 400

Val Lys Ala Ile Asn Ala Ala Arg Met Ala Leu Arg Arg Thr Ser Ala
                405                 410                 415

Pro Arg Val Ser Leu Asp Lys Val Ile Glu Thr Met Tyr Glu Thr Gly
            420                 425                 430

Lys Asp Met Asn Ala Lys Tyr Arg Glu Thr Ser Arg Gly Gly Leu Ala
        435                 440                 445

Ile Lys Val Gln Cys Asp
    450

<210> 115
<211> 1233
<212> DNA
<213> Escherichia coli

<400> 115
atggcaaagg tatcgctgga gaaagacaag attaagtttc tgctggtaga aggcgtgcac 60

caaaaggcgc tggaaagcct tcgtgcagct ggttacacca acatcgaatt tcacaaaggc 120

gcgctggatg atgaacaatt aaaagaatcc atccgcgatg cccacttcat cggcctgcga 180

tcccgtaccc atctgactga agacgtgatc aacgccgcag aaaaactggt cgctattggc 240

tgtttctgta tcggaacaaa ccaggttgat ctggatgcgg cggcaaagcg cgggatcccg 300

gtatttaacg caccgttctc aaatacgcgc tctgttgcgg agctggtgat tggcgaactg 360

ctgctgctat tgcgcggcgt gccggaagcc aatgctaaag cgcaccgtgg cgtgtggaac 420

aaactggcgg cgggttcttt tgaagcgcgc ggcaaaaagc tgggtatcat cggctacggt 480

catattggta cgcaattggg cattctggct gaatcgctgg gaatgtatgt ttacttttat 540

gatattgaaa ataaactgcc gctgggcaac gccactcagg tacagcatct ttctgacctg 600

ctgaatatga gcgatgtggt gagtctgcat gtaccagaga atccgtccac caaaaatatg 660

atgggcgcga aagaaatttc actaatgaag cccggctcgc tgctgattaa tgcttcgcgc 720

ggtactgtgg tggatattcc ggcgctgtgt gatgcgctgg cgagcaaaca tctggcgggg 780

gcggcaatcg acgtattccc gacggaaccg gcgaccaata gcgatccatt tacctctccg 840

ctgtgtgaat tcgacaacgt ccttctgacg ccacacattg gcggttcgac tcaggaagcg 900

caggagaata tcggcctgga agttgcgggt aaattgatca agtattctga caatggctca 960

acgctctctg cggtgaactt cccggaagtc tcgctgccac tgcacggtgg gcgtcgtctg 1020

atgcacatcc acgaaaaccg tccgggcgtg ctaactgcgc tgaacaaaat cttcgccgag 1080

cagggcgtcg ccatcgcggc gcaatatctg caaacttccg cccagatggg ttatgtggtt 1140

attgatattg aagccgacga agacgttgcc gaaaaagcgc tgcaggcaat gaaagctatt 1200

ccgggtacca ttcgcgcccg tctgctgtac taa 1233

<210> 116
<211> 410
<212> PRT
<213> Escherichia coli

<400> 116

Met Ala Lys Val Ser Leu Glu Lys Asp Lys Ile Lys Phe Leu Leu Val
1 5 10 15

Glu Gly Val His Gln Lys Ala Leu Glu Ser Leu Arg Ala Ala Gly Tyr
20 25 30

Thr Asn Ile Glu Phe His Lys Gly Ala Leu Asp Asp Glu Gln Leu Lys
35 40 45

Glu Ser Ile Arg Asp Ala His Phe Ile Gly Leu Arg Ser Arg Thr His
50 55 60

Leu Thr Glu Asp Val Ile Asn Ala Ala Glu Lys Leu Val Ala Ile Gly
65 70 75 80

```
Cys Phe Cys Ile Gly Thr Asn Gln Val Asp Leu Asp Ala Ala Ala Lys
                85                  90                  95

Arg Gly Ile Pro Val Phe Asn Ala Pro Phe Ser Asn Thr Arg Ser Val
            100                 105                 110

Ala Glu Leu Val Ile Gly Glu Leu Leu Leu Leu Leu Arg Gly Val Pro
        115                 120                 125

Glu Ala Asn Ala Lys Ala His Arg Gly Val Trp Asn Lys Leu Ala Ala
    130                 135                 140

Gly Ser Phe Glu Ala Arg Gly Lys Lys Leu Gly Ile Ile Gly Tyr Gly
145                 150                 155                 160

His Ile Gly Thr Gln Leu Gly Ile Leu Ala Glu Ser Leu Gly Met Tyr
                165                 170                 175

Val Tyr Phe Tyr Asp Ile Glu Asn Lys Leu Pro Leu Gly Asn Ala Thr
            180                 185                 190

Gln Val Gln His Leu Ser Asp Leu Leu Asn Met Ser Asp Val Val Ser
        195                 200                 205

Leu His Val Pro Glu Asn Pro Ser Thr Lys Asn Met Met Gly Ala Lys
    210                 215                 220

Glu Ile Ser Leu Met Lys Pro Gly Ser Leu Leu Ile Asn Ala Ser Arg
225                 230                 235                 240

Gly Thr Val Val Asp Ile Pro Ala Leu Cys Asp Ala Leu Ala Ser Lys
                245                 250                 255

His Leu Ala Gly Ala Ala Ile Asp Val Phe Pro Thr Glu Pro Ala Thr
            260                 265                 270

Asn Ser Asp Pro Phe Thr Ser Pro Leu Cys Glu Phe Asp Asn Val Leu
        275                 280                 285

Leu Thr Pro His Ile Gly Gly Ser Thr Gln Glu Ala Gln Glu Asn Ile
    290                 295                 300

Gly Leu Glu Val Ala Gly Lys Leu Ile Lys Tyr Ser Asp Asn Gly Ser
305                 310                 315                 320

Thr Leu Ser Ala Val Asn Phe Pro Glu Val Ser Leu Pro Leu His Gly
                325                 330                 335
```

Gly Arg Arg Leu Met His Ile His Glu Asn Arg Pro Gly Val Leu Thr
340 345 350

Ala Leu Asn Lys Ile Phe Ala Glu Gln Gly Val Ala Ile Ala Ala Gln
355 360 365

Tyr Leu Gln Thr Ser Ala Gln Met Gly Tyr Val Val Ile Asp Ile Glu
370 375 380

Ala Asp Glu Asp Val Ala Glu Lys Ala Leu Gln Ala Met Lys Ala Ile
385 390 395 400

Pro Gly Thr Ile Arg Ala Arg Leu Leu Tyr
405 410

<210> 117
<211> 1254
<212> DNA
<213> Escherichia coli

<400> 117
atgttaaagc gtgaaatgaa cattgccgat tatgatgccg aactgtggca ggctatggag 60

caggaaaaag tacgtcagga agagcacatc gaactgatcg cctccgaaaa ctacaccagc 120

ccgcgcgtaa tgcaggcgca gggttctcag ctgaccaaca aatatgctga aggttatccg 180

ggcaaacgct actacggcgg ttgcgagtat gttgatatcg ttgaacaact ggcgatcgat 240

cgtgcgaaag aactgttcgg cgctgactac gctaacgtcc agccgcactc cggctcccag 300

gctaactttg cggtctacac cgcgctgctg gaaccaggtg ataccgttct gggtatgaac 360

ctggcgcatg gcggtcacct gactcacggt tctccggtta acttctccgg taaactgtac 420

aacatcgttc cttacggtat cgatgctacc ggtcatatcg actacgccga tctggaaaaa 480

caagccaaag aacacaagcc gaaaatgatt atcggtggtt tctctgcata ttccggcgtg 540

gtggactggg cgaaaatgcg tgaaatcgct gacagcatcg gtgcttacct gttcgttgat 600

atggcgcacg ttgcgggcct ggttgctgct ggcgtctacc cgaacccggt tcctcatgct 660

cacgttgtta ctaccaccac tcacaaaacc ctggcgggtc cgcgcggcgg cctgatcctg 720

gcgaaaggtg gtagcgaaga gctgtacaaa aaactgaact ctgccgtttt ccctggtggt 780

cagggcggtc cgttgatgca cgtaatcgcc ggtaaagcgg ttgctctgaa agaagcgatg 840

gagcctgagt tcaaaactta ccagcagcag gtcgctaaaa acgctaaagc gatggtagaa 900

gtgttcctcg agcgcggcta caaagtggtt tccggcggca ctgataacca cctgttcctg 960

gttgatctgg ttgataaaaa cctgaccggt aaagaagcag acgccgctct gggccgtgct 1020

aacatcaccg tcaacaaaaa cagcgtaccg aacgatccga agagcccgtt tgtgacctcc 1080

ggtattcgtg taggtactcc ggcgattacc cgtcgcggct ttaaagaagc cgaagcgaaa 1140

gaactggctg gctggatgtg tgacgtgctg gacagcatca atgatgaagc cgttatcgag 1200

cgcatcaaag gtaaagttct cgacatctgc gcacgttacc cggtttacgc ataa 1254

<210> 118
<211> 417
<212> PRT
<213> Escherichia coli

<400> 118

Met Leu Lys Arg Glu Met Asn Ile Ala Asp Tyr Asp Ala Glu Leu Trp
1 5 10 15

Gln Ala Met Glu Gln Glu Lys Val Arg Gln Glu Glu His Ile Glu Leu
20 25 30

Ile Ala Ser Glu Asn Tyr Thr Ser Pro Arg Val Met Gln Ala Gln Gly
35 40 45

Ser Gln Leu Thr Asn Lys Tyr Ala Glu Gly Tyr Pro Gly Lys Arg Tyr
50 55 60

Tyr Gly Gly Cys Glu Tyr Val Asp Ile Val Glu Gln Leu Ala Ile Asp
65 70 75 80

Arg Ala Lys Glu Leu Phe Gly Ala Asp Tyr Ala Asn Val Gln Pro His
85 90 95

Ser Gly Ser Gln Ala Asn Phe Ala Val Tyr Thr Ala Leu Leu Glu Pro
100 105 110

Gly Asp Thr Val Leu Gly Met Asn Leu Ala His Gly Gly His Leu Thr
115 120 125

His Gly Ser Pro Val Asn Phe Ser Gly Lys Leu Tyr Asn Ile Val Pro
130 135 140

Tyr Gly Ile Asp Ala Thr Gly His Ile Asp Tyr Ala Asp Leu Glu Lys
145 150 155 160

Gln Ala Lys Glu His Lys Pro Lys Met Ile Ile Gly Gly Phe Ser Ala
165 170 175

Tyr Ser Gly Val Val Asp Trp Ala Lys Met Arg Glu Ile Ala Asp Ser
180 185 190

Ile Gly Ala Tyr Leu Phe Val Asp Met Ala His Val Ala Gly Leu Val

195 200 205

Ala Ala Gly Val Tyr Pro Asn Pro Val Pro His Ala His Val Val Thr
210 215 220

Thr Thr Thr His Lys Thr Leu Ala Gly Pro Arg Gly Gly Leu Ile Leu
225 230 235 240

Ala Lys Gly Gly Ser Glu Glu Leu Tyr Lys Lys Leu Asn Ser Ala Val
245 250 255

Phe Pro Gly Gly Gln Gly Gly Pro Leu Met His Val Ile Ala Gly Lys
260 265 270

Ala Val Ala Leu Lys Glu Ala Met Glu Pro Glu Phe Lys Thr Tyr Gln
275 280 285

Gln Gln Val Ala Lys Asn Ala Lys Ala Met Val Glu Val Phe Leu Glu
290 295 300

Arg Gly Tyr Lys Val Val Ser Gly Gly Thr Asp Asn His Leu Phe Leu
305 310 315 320

Val Asp Leu Val Asp Lys Asn Leu Thr Gly Lys Glu Ala Asp Ala Ala
325 330 335

Leu Gly Arg Ala Asn Ile Thr Val Asn Lys Asn Ser Val Pro Asn Asp
340 345 350

Pro Lys Ser Pro Phe Val Thr Ser Gly Ile Arg Val Gly Thr Pro Ala
355 360 365

Ile Thr Arg Arg Gly Phe Lys Glu Ala Glu Ala Lys Glu Leu Ala Gly
370 375 380

Trp Met Cys Asp Val Leu Asp Ser Ile Asn Asp Glu Ala Val Ile Glu
385 390 395 400

Arg Ile Lys Gly Lys Val Leu Asp Ile Cys Ala Arg Tyr Pro Val Tyr
405 410 415

Ala

<210> 119
<211> 1254
<212> DNA
<213> Escherichia coli

<400> 119
atgttaaagc gtgaaatgaa cattgccgat tatgatgccg aactgtggca ggctatggag 60

caggaaaaag tacgtcagga agagcacatc gaactgatcg cctccgaaaa ctacaccagc 120

ccgcgcgtaa tgcaggcgca gggttctcag ctgaccaaca aatatgctga aggttatccg 180

ggcaaacgct actacggcgg ttgcgagtat gttgatatcg ttgaacaact ggcgatcgat 240

cgtgcgaaag aactgttcgg cgctgactac gctaacgtcc agccgcactc cggctcccag 300

gctaactttg cggtctacac cgcgctgctg gaaccaggtg ataccgttct gggtatgaac 360

ctggcgcatg gcggtcacct gactcacggt tctccggtta acttctccgg taaactgtac 420

aacatcgttc cttacggtat cgatgctacc ggtcatatcg actacgccga tctggaaaaa 480

caagccaaag aacacaagcc gaaaatgatt atcggtggtt tctctgcata ttccggcgtg 540

gtggactggg cgaaaatgcg tgaaatcgct gacagcatcg gtgcttacct gttcgttgat 600

atggcgcacg ttgcgggcct ggttgctgct ggcgtctacc cgaacccggt tcctcatgct 660

cacgttgtta ctaccaccac tcacaaaacc ctggcgggtc cgaaaggagg cctgatcctg 720

gcgaaaggtg gtagcgaaga gctgtacaaa aaactgaact ctgccgtttt ccctggtggt 780

cagggcggtc cgttgatgca cgtaatcgcc ggtaaagcgg ttgctctgaa agaagcgatg 840

gagcctgagt tcaaaactta ccagcagcag gtcgctaaaa acgctaaagc gatggtagaa 900

gtgttcctcg agcgcggcta caaagtggtt tccggcggca ctgataacca cctgttcctg 960

gttgatctgg ttgataaaaa cctgaccggt aaagaagcag acgccgctct gggccgtgct 1020

aacatcaccg tcaacaaaaa cagcgtaccg aacgatccga agagcccgtt tgtgacctcc 1080

ggtattcgtg taggtactcc ggcgattacc cgtcgcggct ttaaagaagc cgaagcgaaa 1140

gaactggctg gctggatgtg tgacgtgctg gacagcatca atgatgaagc cgttatcgag 1200

cgcatcaaag gtaaagttct cgacatctgc gcacgttacc cggtttacgc ataa 1254

<210> 120
<211> 417
<212> PRT
<213> Escherichia coli

<400> 120

Met Leu Lys Arg Glu Met Asn Ile Ala Asp Tyr Asp Ala Glu Leu Trp
1 5 10 15

Gln Ala Met Glu Gln Glu Lys Val Arg Gln Glu Glu His Ile Glu Leu
20 25 30

Ile Ala Ser Glu Asn Tyr Thr Ser Pro Arg Val Met Gln Ala Gln Gly
35 40 45

```
Ser Gln Leu Thr Asn Lys Tyr Ala Glu Gly Tyr Pro Gly Lys Arg Tyr
    50                  55                  60

Tyr Gly Gly Cys Glu Tyr Val Asp Ile Val Glu Gln Leu Ala Ile Asp
65                  70                  75                  80

Arg Ala Lys Glu Leu Phe Gly Ala Asp Tyr Ala Asn Val Gln Pro His
                85                  90                  95

Ser Gly Ser Gln Ala Asn Phe Ala Val Tyr Thr Ala Leu Leu Glu Pro
            100                 105                 110

Gly Asp Thr Val Leu Gly Met Asn Leu Ala His Gly Gly His Leu Thr
        115                 120                 125

His Gly Ser Pro Val Asn Phe Ser Gly Lys Leu Tyr Asn Ile Val Pro
    130                 135                 140

Tyr Gly Ile Asp Ala Thr Gly His Ile Asp Tyr Ala Asp Leu Glu Lys
145                 150                 155                 160

Gln Ala Lys Glu His Lys Pro Lys Met Ile Ile Gly Gly Phe Ser Ala
                165                 170                 175

Tyr Ser Gly Val Val Asp Trp Ala Lys Met Arg Glu Ile Ala Asp Ser
            180                 185                 190

Ile Gly Ala Tyr Leu Phe Val Asp Met Ala His Val Ala Gly Leu Val
        195                 200                 205

Ala Ala Gly Val Tyr Pro Asn Pro Val Pro His Ala His Val Val Thr
    210                 215                 220

Thr Thr Thr His Lys Thr Leu Ala Gly Pro Lys Gly Gly Leu Ile Leu
225                 230                 235                 240

Ala Lys Gly Gly Ser Glu Glu Leu Tyr Lys Lys Leu Asn Ser Ala Val
                245                 250                 255

Phe Pro Gly Gly Gln Gly Gly Pro Leu Met His Val Ile Ala Gly Lys
            260                 265                 270

Ala Val Ala Leu Lys Glu Ala Met Glu Pro Glu Phe Lys Thr Tyr Gln
        275                 280                 285

Gln Gln Val Ala Lys Asn Ala Lys Ala Met Val Glu Val Phe Leu Glu
    290                 295                 300
```

Arg Gly Tyr Lys Val Val Ser Gly Gly Thr Asp Asn His Leu Phe Leu
305 310 315 320

Val Asp Leu Val Asp Lys Asn Leu Thr Gly Lys Glu Ala Asp Ala Ala
325 330 335

Leu Gly Arg Ala Asn Ile Thr Val Asn Lys Asn Ser Val Pro Asn Asp
340 345 350

Pro Lys Ser Pro Phe Val Thr Ser Gly Ile Arg Val Gly Thr Pro Ala
355 360 365

Ile Thr Arg Arg Gly Phe Lys Glu Ala Glu Ala Lys Glu Leu Ala Gly
370 375 380

Trp Met Cys Asp Val Leu Asp Ser Ile Asn Asp Glu Ala Val Ile Glu
385 390 395 400

Arg Ile Lys Gly Lys Val Leu Asp Ile Cys Ala Arg Tyr Pro Val Tyr
405 410 415

Ala

<210> 121
<211> 1425
<212> DNA
<213> Escherichia coli

<400> 121
atgagtactg aaatcaaaac tcaggtcgtg gtacttgggg caggccccgc aggttactcc 60
gctgccttcc gttgcgctga tttaggtctg gaaaccgtaa tcgtagaacg ttacaacacc 120
cttggcggtg tttgcctgaa cgtcggctgt atcccttcta aagcactgct gcacgtagca 180
aaagttatcg aagaagccaa agcgctggct gaacacggta tcgtcttcgg cgaaccgaaa 240
accgatatcg acaagattcg tacctggaaa gagaaagtga tcaatcagct gaccggtggt 300
ctggctggta tggcgaaagg ccgcaaagtc aaagtggtca acggtctggg taaattcacc 360
ggggctaaca ccctggaagt tgaaggtgag aacggcaaaa ccgtgatcaa cttcgacaac 420
gcgatcattg cagcgggttc tcgcccgatc caactgccgt ttattccgca tgaagatccg 480
cgtatctggg actccactga cgcgctggaa ctgaaagaag taccagaacg cctgctggta 540
atgggtggcg gtatcatcgg tctggaaatg ggcaccgttt accacgcgct gggttcacag 600
attgacgtgg ttgaaatgtt cgaccaggtt atcccggcag ctgacaaaga catcgttaaa 660
gtcttcacca agcgtatcag caagaaattc aacctgatgc tggaaaccaa agttaccgcc 720

```
gttgaagcga aagaagacgg catttatgtg acgatggaag gcaaaaaagc acccgctgaa      780

ccgcagcgtt acgacgccgt gctggtagcg attggtcgtg tgccgaacgg taaaaacctc      840

gacgcaggca aagcaggcgt ggaagttgac gaccgtggtt tcatccgcgt tgacaaacag      900

ctgcgtacca acgtaccgca catctttgct atcggcgata tcgtcggtca accgatgctg      960

gcacacaaag gtgttcacga aggtcacgtt gccgctgaag ttatcgccgg taagaaacac     1020

tacttcgatc cgaaagttat cccgtccatc gcctataccg aaccagaagt tgcatgggtg     1080

ggtctgactg agaaagaagc gaaagagaaa ggcatcagct atgaaaccgc caccttcccg     1140

tgggctgctt ctggtcgtgc tatcgcttcc gactgcgcag acggtatgac caagctgatt     1200

ttcgacaaag aatctcaccg tgtgatcggt ggtgcgattg tcggtactaa cggcggcgag     1260

ctgctgggtg aaatcggcct ggcaatcgaa atgggttgtg atgctgaaga catcgcactg     1320

accatccacg cgcacccgac tctgcacgag tctgtgggcc tggcggcaga agtgttcgaa     1380

ggtagcatta ccgacctgcc gaacccgaaa gcgaagaaga agtaa                     1425
```

```
<210>  122
<211>  474
<212>  PRT
<213>  Escherichia coli

<400>  122

Met Ser Thr Glu Ile Lys Thr Gln Val Val Val Leu Gly Ala Gly Pro
1               5                   10                  15

Ala Gly Tyr Ser Ala Ala Phe Arg Cys Ala Asp Leu Gly Leu Glu Thr
            20                  25                  30

Val Ile Val Glu Arg Tyr Asn Thr Leu Gly Gly Val Cys Leu Asn Val
        35                  40                  45

Gly Cys Ile Pro Ser Lys Ala Leu Leu His Val Ala Lys Val Ile Glu
    50                  55                  60

Glu Ala Lys Ala Leu Ala Glu His Gly Ile Val Phe Gly Glu Pro Lys
65                  70                  75                  80

Thr Asp Ile Asp Lys Ile Arg Thr Trp Lys Glu Lys Val Ile Asn Gln
                85                  90                  95

Leu Thr Gly Gly Leu Ala Gly Met Ala Lys Gly Arg Lys Val Lys Val
            100                 105                 110

Val Asn Gly Leu Gly Lys Phe Thr Gly Ala Asn Thr Leu Glu Val Glu
        115                 120                 125
```

Gly Glu Asn Gly Lys Thr Val Ile Asn Phe Asp Asn Ala Ile Ile Ala
    130                 135                 140

Ala Gly Ser Arg Pro Ile Gln Leu Pro Phe Ile Pro His Glu Asp Pro
145                 150                 155                 160

Arg Ile Trp Asp Ser Thr Asp Ala Leu Glu Leu Lys Glu Val Pro Glu
                165                 170                 175

Arg Leu Leu Val Met Gly Gly Gly Ile Ile Gly Leu Glu Met Gly Thr
            180                 185                 190

Val Tyr His Ala Leu Gly Ser Gln Ile Asp Val Val Glu Met Phe Asp
        195                 200                 205

Gln Val Ile Pro Ala Ala Asp Lys Asp Ile Val Lys Val Phe Thr Lys
    210                 215                 220

Arg Ile Ser Lys Lys Phe Asn Leu Met Leu Glu Thr Lys Val Thr Ala
225                 230                 235                 240

Val Glu Ala Lys Glu Asp Gly Ile Tyr Val Thr Met Glu Gly Lys Lys
                245                 250                 255

Ala Pro Ala Glu Pro Gln Arg Tyr Asp Ala Val Leu Val Ala Ile Gly
            260                 265                 270

Arg Val Pro Asn Gly Lys Asn Leu Asp Ala Gly Lys Ala Gly Val Glu
        275                 280                 285

Val Asp Asp Arg Gly Phe Ile Arg Val Asp Lys Gln Leu Arg Thr Asn
    290                 295                 300

Val Pro His Ile Phe Ala Ile Gly Asp Ile Val Gly Gln Pro Met Leu
305                 310                 315                 320

Ala His Lys Gly Val His Glu Gly His Val Ala Ala Glu Val Ile Ala
                325                 330                 335

Gly Lys Lys His Tyr Phe Asp Pro Lys Val Ile Pro Ser Ile Ala Tyr
            340                 345                 350

Thr Glu Pro Glu Val Ala Trp Val Gly Leu Thr Glu Lys Glu Ala Lys
        355                 360                 365

Glu Lys Gly Ile Ser Tyr Glu Thr Ala Thr Phe Pro Trp Ala Ala Ser

370 375 380

Gly Arg Ala Ile Ala Ser Asp Cys Ala Asp Gly Met Thr Lys Leu Ile
385 390 395 400

Phe Asp Lys Glu Ser His Arg Val Ile Gly Gly Ala Ile Val Gly Thr
405 410 415

Asn Gly Gly Glu Leu Leu Gly Glu Ile Gly Leu Ala Ile Glu Met Gly
420 425 430

Cys Asp Ala Glu Asp Ile Ala Leu Thr Ile His Ala His Pro Thr Leu
435 440 445

His Glu Ser Val Gly Leu Ala Ala Glu Val Phe Glu Gly Ser Ile Thr
450 455 460

Asp Leu Pro Asn Pro Lys Ala Lys Lys Lys
465 470

<210> 123
<211> 1425
<212> DNA
<213> Escherichia coli

<400> 123
atgagtactg aaatcaaaac tcaggtcgtg gtacttgggg caggccccgc aggttactcc 60
gctgccttcc gttgcgctga tttaggtctg gaaaccgtaa tcgtagaacg ttacaacacc 120
cttggcggtg tttgcctgaa cgtcggctgt atcccttcta aagtactgct gcacgtagca 180
aaagttatcg aagaagccaa agcgctggct gaacacggta tcgtcttcgg cgaaccgaaa 240
accgatatcg acaagattcg tacctggaaa gagaaagtga tcaatcagct gaccggtggt 300
ctggctggta tggcgaaagg ccgcaaagtc aaagtggtca acggtctggg taaattcacc 360
ggggctaaca ccctggaagt tgaaggtgag aacggcaaaa ccgtgatcaa cttcgacaac 420
gcgatcattg cagcgggttc tcgcccgatc caactgccgt ttattccgca tgaagatccg 480
cgtatctggg actccactga cgcgctggaa ctgaaagaag taccagaacg cctgctggta 540
atgggtggcg gtatcatcgg tctggaaatg ggcaccgttt accacgcgct gggttcacag 600
attgacgtgg ttgaaatgtt cgaccaggtt atcccggcag ctgacaaaga catcgttaaa 660
gtcttcacca agcgtatcag caagaaattc aacctgatgc tggaaaccaa agttaccgcc 720
gttgaagcga aagaagacgg catttatgtg acgatggaag gcaaaaaagc acccgctgaa 780
ccgcagcgtt acgacgccgt gctggtagcg attggtcgtg tgccgaacgg taaaaacctc 840
gacgcaggca aagcaggcgt ggaagttgac gaccgtggtt tcatccgcgt tgacaaacag 900

ctgcgtacca acgtaccgca catctttgct atcggcgata tcgtcggtca accgatgctg 960

gcacacaaag gtgttcacga aggtcacgtt gccgctgaag ttatcgccgg taagaaacac 1020

tacttcgatc cgaaagttat cccgtccatc gcctataccg aaccagaagt tgcatgggtg 1080

ggtctgactg agaaagaagc gaaagagaaa ggcatcagct atgaaaccgc caccttcccg 1140

tgggctgctt ctggtcgtgc tatcgcttcc gactgcgcag acggtatgac caagctgatt 1200

ttcgacaaag aatctcaccg tgtgatcggt ggtgcgattg tcggtactaa cggcggcgag 1260

ctgctgggtg aaatcggcct ggcaatcgaa atgggttgtg atgctgaaga catcgcactg 1320

accatccacg cgcacccgac tctgcacgag tctgtgggcc tggcggcaga agtgttcgaa 1380

ggtagcatta ccgacctgcc gaacccgaaa gcgaagaaga agtaa 1425

<210> 124
<211> 474
<212> PRT
<213> Escherichia coli

<400> 124

Met Ser Thr Glu Ile Lys Thr Gln Val Val Val Leu Gly Ala Gly Pro
1 5 10 15

Ala Gly Tyr Ser Ala Ala Phe Arg Cys Ala Asp Leu Gly Leu Glu Thr
20 25 30

Val Ile Val Glu Arg Tyr Asn Thr Leu Gly Gly Val Cys Leu Asn Val
35 40 45

Gly Cys Ile Pro Ser Lys Val Leu Leu His Val Ala Lys Val Ile Glu
50 55 60

Glu Ala Lys Ala Leu Ala Glu His Gly Ile Val Phe Gly Glu Pro Lys
65 70 75 80

Thr Asp Ile Asp Lys Ile Arg Thr Trp Lys Glu Lys Val Ile Asn Gln
85 90 95

Leu Thr Gly Gly Leu Ala Gly Met Ala Lys Gly Arg Lys Val Lys Val
100 105 110

Val Asn Gly Leu Gly Lys Phe Thr Gly Ala Asn Thr Leu Glu Val Glu
115 120 125

Gly Glu Asn Gly Lys Thr Val Ile Asn Phe Asp Asn Ala Ile Ile Ala
130 135 140

Ala Gly Ser Arg Pro Ile Gln Leu Pro Phe Ile Pro His Glu Asp Pro

145 150 155 160

Arg Ile Trp Asp Ser Thr Asp Ala Leu Glu Leu Lys Glu Val Pro Glu
165 170 175

Arg Leu Leu Val Met Gly Gly Gly Ile Ile Gly Leu Glu Met Gly Thr
180 185 190

Val Tyr His Ala Leu Gly Ser Gln Ile Asp Val Val Glu Met Phe Asp
195 200 205

Gln Val Ile Pro Ala Ala Asp Lys Asp Ile Val Lys Val Phe Thr Lys
210 215 220

Arg Ile Ser Lys Lys Phe Asn Leu Met Leu Glu Thr Lys Val Thr Ala
225 230 235 240

Val Glu Ala Lys Glu Asp Gly Ile Tyr Val Thr Met Glu Gly Lys Lys
245 250 255

Ala Pro Ala Glu Pro Gln Arg Tyr Asp Ala Val Leu Val Ala Ile Gly
260 265 270

Arg Val Pro Asn Gly Lys Asn Leu Asp Ala Gly Lys Ala Gly Val Glu
275 280 285

Val Asp Asp Arg Gly Phe Ile Arg Val Asp Lys Gln Leu Arg Thr Asn
290 295 300

Val Pro His Ile Phe Ala Ile Gly Asp Ile Val Gly Gln Pro Met Leu
305 310 315 320

Ala His Lys Gly Val His Glu Gly His Val Ala Ala Glu Val Ile Ala
325 330 335

Gly Lys Lys His Tyr Phe Asp Pro Lys Val Ile Pro Ser Ile Ala Tyr
340 345 350

Thr Glu Pro Glu Val Ala Trp Val Gly Leu Thr Glu Lys Glu Ala Lys
355 360 365

Glu Lys Gly Ile Ser Tyr Glu Thr Ala Thr Phe Pro Trp Ala Ala Ser
370 375 380

Gly Arg Ala Ile Ala Ser Asp Cys Ala Asp Gly Met Thr Lys Leu Ile
385 390 395 400

Phe Asp Lys Glu Ser His Arg Val Ile Gly Gly Ala Ile Val Gly Thr
405 410 415

Asn Gly Gly Glu Leu Leu Gly Glu Ile Gly Leu Ala Ile Glu Met Gly
420 425 430

Cys Asp Ala Glu Asp Ile Ala Leu Thr Ile His Ala His Pro Thr Leu
435 440 445

His Glu Ser Val Gly Leu Ala Ala Glu Val Phe Glu Gly Ser Ile Thr
450 455 460

Asp Leu Pro Asn Pro Lys Ala Lys Lys Lys
465 470

<210> 125
<211> 1368
<212> DNA
<213> Escherichia coli

<400> 125
atgattagcg tattcgatat tttcaaaatc ggcattggcc cttccagttc tcataccgtt 60

ggaccaatga aagcgggtaa acaatttacc gacgatctga ttgcccgtaa cctgcttaaa 120

gacgtgaccc gcgtggtggt tgacgtgtac ggctcgctct ctctgaccgg taaaggccac 180

cacactgata tcgccattat tatgggcctg gcgggtaacc tgccggatac cgtggatatc 240

gattccatcc ccagttttat tcaggatgtg aatactcatg gtcgcctgat gctggcaaac 300

ggtcagcatg aagtggagtt cccggttgat cagtgcatga acttccacgc cgacaacctt 360

tctctgcatg aaaacggtat gcgcattacc gcgctggcgg gcgataaagt cgtttacagc 420

cagacttact actctattgg cggtggcttt atcgttgatg aagagcattt tggccagcag 480

gatagcgcac cggttgaagt tccttatccg tacagttcag cagccgatct gcaaaaacat 540

tgtcaggaaa ccgggctgtc actctctggc ctgatgatga aaaacgagct ggcgctgcac 600

agcaaagaag agctggaaca gcacctggcg aacgtctggg aagtcatgcg cggcggtatt 660

gagcgcggta tttccaccga aggcgtgttg cctggcaaac tgcgcgttcc acgccgtgct 720

gcggcactac gccggatgct ggtcagccag gataaaacca ccactgaccc gatggcggtt 780

gttgactgga tcaacatgtt tgcactggca gtgaacgaag agaacgctgc tggcggtcgc 840

gtggtgactg cgccgactaa cggtgcgtgc gggattatcc cggcagttct ggcgtactac 900

gacaagttta tccgcgaagt gaacgctaac tcactggctc gttacctgct ggtagccagc 960

gccattggtt ctctttataa gatgaacgcg tcgatttctg gtgctgaagt gggttgccag 1020

ggtgaagttg gcgtggcgtg ctcaatggcg gcggctggtc tggcagaact attaggcgca 1080

agcccggcgc aggtgtgcat cgcggcggaa atcgccatgg agcacaacct cggtctgacg 1140

tgtgacccgg tcgccggaca ggtacaggtg ccatgcatcg agcgtaacgc cattgcggca 1200

gtaaaagcgg tgaacgccgc acgtatggcg ctgcgccgta ccagcgagcc gcgcgtctgc 1260

ctcgataaag ttatcgaaac catgtacgaa acaggtaaag atatgaacgc caagtaccgc 1320

gaaacctctc gcggcggcct ggcaatgaag atcgttgcct gcgattaa 1368

<210> 126
<211> 455
<212> PRT
<213> Escherichia coli

<400> 126

Met Ile Ser Val Phe Asp Ile Phe Lys Ile Gly Ile Gly Pro Ser Ser
1 5 10 15

Ser His Thr Val Gly Pro Met Lys Ala Gly Lys Gln Phe Thr Asp Asp
20 25 30

Leu Ile Ala Arg Asn Leu Leu Lys Asp Val Thr Arg Val Val Val Asp
35 40 45

Val Tyr Gly Ser Leu Ser Leu Thr Gly Lys Gly His His Thr Asp Ile
50 55 60

Ala Ile Ile Met Gly Leu Ala Gly Asn Leu Pro Asp Thr Val Asp Ile
65 70 75 80

Asp Ser Ile Pro Ser Phe Ile Gln Asp Val Asn Thr His Gly Arg Leu
85 90 95

Met Leu Ala Asn Gly Gln His Glu Val Glu Phe Pro Val Asp Gln Cys
100 105 110

Met Asn Phe His Ala Asp Asn Leu Ser Leu His Glu Asn Gly Met Arg
115 120 125

Ile Thr Ala Leu Ala Gly Asp Lys Val Val Tyr Ser Gln Thr Tyr Tyr
130 135 140

Ser Ile Gly Gly Gly Phe Ile Val Asp Glu Glu His Phe Gly Gln Gln
145 150 155 160

Asp Ser Ala Pro Val Glu Val Pro Tyr Pro Tyr Ser Ser Ala Ala Asp
165 170 175

Leu Gln Lys His Cys Gln Glu Thr Gly Leu Ser Leu Ser Gly Leu Met
180 185 190

Met Lys Asn Glu Leu Ala Leu His Ser Lys Glu Glu Leu Glu Gln His
195 200 205

Leu Ala Asn Val Trp Glu Val Met Arg Gly Gly Ile Glu Arg Gly Ile
210 215 220

Ser Thr Glu Gly Val Leu Pro Gly Lys Leu Arg Val Pro Arg Arg Ala
225 230 235 240

Ala Ala Leu Arg Arg Met Leu Val Ser Gln Asp Lys Thr Thr Thr Asp
245 250 255

Pro Met Ala Val Val Asp Trp Ile Asn Met Phe Ala Leu Ala Val Asn
260 265 270

Glu Glu Asn Ala Ala Gly Gly Arg Val Val Thr Ala Pro Thr Asn Gly
275 280 285

Ala Cys Gly Ile Ile Pro Ala Val Leu Ala Tyr Tyr Asp Lys Phe Ile
290 295 300

Arg Glu Val Asn Ala Asn Ser Leu Ala Arg Tyr Leu Leu Val Ala Ser
305 310 315 320

Ala Ile Gly Ser Leu Tyr Lys Met Asn Ala Ser Ile Ser Gly Ala Glu
325 330 335

Val Gly Cys Gln Gly Glu Val Gly Val Ala Cys Ser Met Ala Ala Ala
340 345 350

Gly Leu Ala Glu Leu Leu Gly Ala Ser Pro Ala Gln Val Cys Ile Ala
355 360 365

Ala Glu Ile Ala Met Glu His Asn Leu Gly Leu Thr Cys Asp Pro Val
370 375 380

Ala Gly Gln Val Gln Val Pro Cys Ile Glu Arg Asn Ala Ile Ala Ala
385 390 395 400

Val Lys Ala Val Asn Ala Ala Arg Met Ala Leu Arg Arg Thr Ser Glu
405 410 415

Pro Arg Val Cys Leu Asp Lys Val Ile Glu Thr Met Tyr Glu Thr Gly
420 425 430

Lys Asp Met Asn Ala Lys Tyr Arg Glu Thr Ser Arg Gly Gly Leu Ala

435 440 445

Met Lys Ile Val Ala Cys Asp
450 455

<210> 127
<211> 1845
<212> DNA
<213> Escherichia coli

<400> 127
atgattaaaa aagcttcgct gctgacggcg tgttccgtca cggcattttc cgcttgggca 60
caggatacca gcccggatac tctcgtcgtt actgctaacc gttttgaaca gccgcgcagc 120
actgtgcttg caccaaccac cgttgtgacc cgtcaggata tcgaccgctg gcagtcgacc 180
tcggtcaatg atgtgctgcg ccgtcttccg ggcgtcgata tcacccaaaa cggcggttca 240
ggtcagctct catctatttt tattcgcggt acaaatgcca gtcatgtgtt ggtgttaatt 300
gatggcgtac gcctgaatct ggcgggggtg agtggttctg ccgaccttag ccagttccct 360
attgcgcttg tccagcgtgt tgaatatatc cgtgggccgc gctccgctgt ttatggttcc 420
gatgcaatag gcggggtggt gaatatcatc acgacgcgcg atgaacccgg aacggaaatt 480
tcagcagggt ggggaagcaa tagttatcag aactatgatg tctctacgca gcaacaactg 540
ggggataaga cacgggtaac gctgttgggc gattatgccc atactcatgg ttatgatgtt 600
gttgcctatg gtaataccgg aacgcaagcg cagacagata acgatggttt tttaagtaaa 660
acgctttatg gcgcgctgga gcataacttt actgatgcct ggagcggctt tgtgcgcggc 720
tatggctatg ataaccgtac caattatgac gcgtattatt ctcccggttc accgttgctc 780
gatacccgta aactctatag ccaaagttgg gacgccgggc tgcgctataa cggcgaactg 840
attaaatcac aactcattac cagctatagc catagcaaag attacaacta cgatccccat 900
tatggtcgtt atgattcgtc ggcgacgctc gatgagatga agcaatacac cgtccagtgg 960
gcaaacaatg tcatcgttgg tcacggtagt attggtgcgg gtgtcgactg gcagaaacag 1020
actacgacgc cgggtacagg ttatgttgag gatggatatg atcaacgtaa taccggcatc 1080
tatctgaccg ggctgcaaca agtcggcgat tttacctttg aaggcgcagc acgcagtgac 1140
gataactcac agtttggtcg tcatggaacc tggcaaacca gcgccggttg ggaattcatc 1200
gaaggttatc gcttcattgc ttcctacggg acatcttata aggcaccaaa tctggggcaa 1260
ctgtatggct tctacggaaa tccgaatctg gacccggaga aaagcaaaca gtgggaaggc 1320
gcgtttgaag gcttaaccgc tggggtgaac tggcgtattt ccggatatcg taacgatgtc 1380
agtgacttga tcgattatga tgatcacacc ctgaaatatt acaacgaagg gaaagcgcgg 1440
attaagggcg tcgaggcgac cgccaatttt gataccggac cactgacgca tactgtgagt 1500

```
tatgattatg tcgatgcgcg caatgcgatt accgacacgc cgttgttacg ccgtgctaaa      1560

cagcaggtga aataccagct cgactggcag ttgtatgact tcgactgggg tattacttat      1620

cagtatttag gcactcgcta tgataaggat tactcatctt atccttatca aaccgttaaa      1680

atgggcggtg tgagcttgtg ggatcttgcg gttgcgtatc cggtcacctc tcacctgaca      1740

gttcgtggta aaatagccaa cctgttcgac aaagattatg agacagtcta tggctaccaa      1800

actgcaggac gggaatacac cttgtctggc agctacacct tctga                      1845
```

<210> 128
<211> 614
<212> PRT
<213> Escherichia coli

<400> 128

```
Met Ile Lys Lys Ala Ser Leu Leu Thr Ala Cys Ser Val Thr Ala Phe
1               5                   10                  15

Ser Ala Trp Ala Gln Asp Thr Ser Pro Asp Thr Leu Val Val Thr Ala
            20                  25                  30

Asn Arg Phe Glu Gln Pro Arg Ser Thr Val Leu Ala Pro Thr Thr Val
        35                  40                  45

Val Thr Arg Gln Asp Ile Asp Arg Trp Gln Ser Thr Ser Val Asn Asp
    50                  55                  60

Val Leu Arg Arg Leu Pro Gly Val Asp Ile Thr Gln Asn Gly Gly Ser
65                  70                  75                  80

Gly Gln Leu Ser Ser Ile Phe Ile Arg Gly Thr Asn Ala Ser His Val
                85                  90                  95

Leu Val Leu Ile Asp Gly Val Arg Leu Asn Leu Ala Gly Val Ser Gly
            100                 105                 110

Ser Ala Asp Leu Ser Gln Phe Pro Ile Ala Leu Val Gln Arg Val Glu
        115                 120                 125

Tyr Ile Arg Gly Pro Arg Ser Ala Val Tyr Gly Ser Asp Ala Ile Gly
    130                 135                 140

Gly Val Val Asn Ile Ile Thr Thr Arg Asp Glu Pro Gly Thr Glu Ile
145                 150                 155                 160

Ser Ala Gly Trp Gly Ser Asn Ser Tyr Gln Asn Tyr Asp Val Ser Thr
                165                 170                 175
```

Gln Gln Gln Leu Gly Asp Lys Thr Arg Val Thr Leu Leu Gly Asp Tyr
180 185 190

Ala His Thr His Gly Tyr Asp Val Val Ala Tyr Gly Asn Thr Gly Thr
195 200 205

Gln Ala Gln Thr Asp Asn Asp Gly Phe Leu Ser Lys Thr Leu Tyr Gly
210 215 220

Ala Leu Glu His Asn Phe Thr Asp Ala Trp Ser Gly Phe Val Arg Gly
225 230 235 240

Tyr Gly Tyr Asp Asn Arg Thr Asn Tyr Asp Ala Tyr Tyr Ser Pro Gly
245 250 255

Ser Pro Leu Leu Asp Thr Arg Lys Leu Tyr Ser Gln Ser Trp Asp Ala
260 265 270

Gly Leu Arg Tyr Asn Gly Glu Leu Ile Lys Ser Gln Leu Ile Thr Ser
275 280 285

Tyr Ser His Ser Lys Asp Tyr Asn Tyr Asp Pro His Tyr Gly Arg Tyr
290 295 300

Asp Ser Ser Ala Thr Leu Asp Glu Met Lys Gln Tyr Thr Val Gln Trp
305 310 315 320

Ala Asn Asn Val Ile Val Gly His Gly Ser Ile Gly Ala Gly Val Asp
325 330 335

Trp Gln Lys Gln Thr Thr Thr Pro Gly Thr Gly Tyr Val Glu Asp Gly
340 345 350

Tyr Asp Gln Arg Asn Thr Gly Ile Tyr Leu Thr Gly Leu Gln Gln Val
355 360 365

Gly Asp Phe Thr Phe Glu Gly Ala Ala Arg Ser Asp Asp Asn Ser Gln
370 375 380

Phe Gly Arg His Gly Thr Trp Gln Thr Ser Ala Gly Trp Glu Phe Ile
385 390 395 400

Glu Gly Tyr Arg Phe Ile Ala Ser Tyr Gly Thr Ser Tyr Lys Ala Pro
405 410 415

Asn Leu Gly Gln Leu Tyr Gly Phe Tyr Gly Asn Pro Asn Leu Asp Pro
420 425 430

Glu Lys Ser Lys Gln Trp Glu Gly Ala Phe Glu Gly Leu Thr Ala Gly
435 440 445

Val Asn Trp Arg Ile Ser Gly Tyr Arg Asn Asp Val Ser Asp Leu Ile
450 455 460

Asp Tyr Asp Asp His Thr Leu Lys Tyr Tyr Asn Glu Gly Lys Ala Arg
465 470 475 480

Ile Lys Gly Val Glu Ala Thr Ala Asn Phe Asp Thr Gly Pro Leu Thr
485 490 495

His Thr Val Ser Tyr Asp Tyr Val Asp Ala Arg Asn Ala Ile Thr Asp
500 505 510

Thr Pro Leu Leu Arg Arg Ala Lys Gln Gln Val Lys Tyr Gln Leu Asp
515 520 525

Trp Gln Leu Tyr Asp Phe Asp Trp Gly Ile Thr Tyr Gln Tyr Leu Gly
530 535 540

Thr Arg Tyr Asp Lys Asp Tyr Ser Ser Tyr Pro Tyr Gln Thr Val Lys
545 550 555 560

Met Gly Gly Val Ser Leu Trp Asp Leu Ala Val Ala Tyr Pro Val Thr
565 570 575

Ser His Leu Thr Val Arg Gly Lys Ile Ala Asn Leu Phe Asp Lys Asp
580 585 590

Tyr Glu Thr Val Tyr Gly Tyr Gln Thr Ala Gly Arg Glu Tyr Thr Leu
595 600 605

Ser Gly Ser Tyr Thr Phe
610

<210> 129
<211> 981
<212> DNA
<213> Escherichia coli

<400> 129
atgctgacac ttgcccgcca acaacagcga caaaatattc gctggttatt atgcctgtca 60

gttttgatgc tgctggcgct tctcttaagc ctttgcgccg gtgaacaatg gatctcgcca 120

ggtgactggt ttactcctcg tggcgaactg ttcgtctggc aaattcgcct gccacgtacg 180

ctggctgtat tgctggttgg tgcggcgctg gctatatccg gcgctgtaat gcaggcgttg 240

tttgaaaatc ctctggcaga acctggacta cttggcgtct ctaacggcgc aggcgtgggg 300

cttatcgccg cggtattgct tgggcaaggg caactcccca actgggcgct agggctgtgt 360

gcgattgctg gcgcgcttat catcacttta atactcttac gtttcgcccg tcgtcatctt 420

tcgaccagtc ggttattgct ggctggcgtt gcattaggga ttatctgtag cgcactaatg 480

acgtgggcta tctacttttc cacctcagtt gatttgcgtc agctgatgta ctggatgatg 540

ggcggttttg gcggcgtaga ctggcggcaa agctggctga tgctggcatt gatccccgtg 600

ttgttgtgga tctgttgtca gtccaggccg atgaatatgt tagcacttgg cgagatctcg 660

gcgcggcaac tgggtttacc cctgtggttc tggcgcaatg tgctggtggc agcgaccggc 720

tggatggttg gcgtcagtgt ggcgctggcg ggtgctatcg gctttattgg tctggtgatc 780

ccccatattc tccggttgtg tggtttaacc gatcatcgcg tattacttcc cggctgcgcg 840

ctggcagggg cgagcgcatt gctgctggcc gatattgtag cgcgcctggc attagctgcc 900

gcagagctgc ctattggcgt ggtcaccgca acgttaggtg cgccggtgtt tatctggtta 960

ttgttaaaag caggacgtta g 981

<210> 130
<211> 326
<212> PRT
<213> Escherichia coli

<400> 130

Met Leu Thr Leu Ala Arg Gln Gln Gln Arg Gln Asn Ile Arg Trp Leu
1 5 10 15

Leu Cys Leu Ser Val Leu Met Leu Leu Ala Leu Leu Leu Ser Leu Cys
20 25 30

Ala Gly Glu Gln Trp Ile Ser Pro Gly Asp Trp Phe Thr Pro Arg Gly
35 40 45

Glu Leu Phe Val Trp Gln Ile Arg Leu Pro Arg Thr Leu Ala Val Leu
50 55 60

Leu Val Gly Ala Ala Leu Ala Ile Ser Gly Ala Val Met Gln Ala Leu
65 70 75 80

Phe Glu Asn Pro Leu Ala Glu Pro Gly Leu Leu Gly Val Ser Asn Gly
85 90 95

Ala Gly Val Gly Leu Ile Ala Ala Val Leu Leu Gly Gln Gly Gln Leu
100 105 110

Pro Asn Trp Ala Leu Gly Leu Cys Ala Ile Ala Gly Ala Leu Ile Ile
        115                 120                 125

Thr Leu Ile Leu Leu Arg Phe Ala Arg Arg His Leu Ser Thr Ser Arg
    130                 135                 140

Leu Leu Leu Ala Gly Val Ala Leu Gly Ile Ile Cys Ser Ala Leu Met
145                 150                 155                 160

Thr Trp Ala Ile Tyr Phe Ser Thr Ser Val Asp Leu Arg Gln Leu Met
                165                 170                 175

Tyr Trp Met Met Gly Gly Phe Gly Gly Val Asp Trp Arg Gln Ser Trp
            180                 185                 190

Leu Met Leu Ala Leu Ile Pro Val Leu Leu Trp Ile Cys Cys Gln Ser
        195                 200                 205

Arg Pro Met Asn Met Leu Ala Leu Gly Glu Ile Ser Ala Arg Gln Leu
    210                 215                 220

Gly Leu Pro Leu Trp Phe Trp Arg Asn Val Leu Val Ala Ala Thr Gly
225                 230                 235                 240

Trp Met Val Gly Val Ser Val Ala Leu Ala Gly Ala Ile Gly Phe Ile
                245                 250                 255

Gly Leu Val Ile Pro His Ile Leu Arg Leu Cys Gly Leu Thr Asp His
            260                 265                 270

Arg Val Leu Leu Pro Gly Cys Ala Leu Ala Gly Ala Ser Ala Leu Leu
        275                 280                 285

Leu Ala Asp Ile Val Ala Arg Leu Ala Leu Ala Ala Ala Glu Leu Pro
    290                 295                 300

Ile Gly Val Val Thr Ala Thr Leu Gly Ala Pro Val Phe Ile Trp Leu
305                 310                 315                 320

Leu Leu Lys Ala Gly Arg
                325

<210>  131
<211>  750
<212>  DNA
<213>  Escherichia coli

<400>  131
atgtctattg tgatgcagtt acaagatgtt gcggaatcta cccgcctggg gccgctttct        60

ggcgaggttc gggctgggga gatcctgcac ctggtggggc cgaatggcgc gggtaagagt 120

accttactgg cgcgaatggc cggaatgacc agcggtaagg gaagcattca gttcgcgggg 180

caaccactgg aagcatggtc cgcaacaaaa ctcgcgctgc atcgcgccta tctttcacaa 240

cagcagacgc cgccgtttgc aacgccggtc tggcactacc tgacactgca tcagcacgat 300

aaaacgcgta ccgaactact gaatgatgtc gcaggggcgc tggctcttga tgacaaactc 360

ggacgtagca ccaatcaact ttccggcggt gaatggcaac gcgtacgtct tgctgcggtg 420

gtgttgcaaa tcacaccaca agccaatccc gcaggccaat tgctgcttct tgatgagccg 480

atgaacagtc ttgatgttgc gcaacaaagt gcgttagaca aaattctgag cgcgctgtgt 540

cagcaaggac tggcgattgt gatgagcagt cacgatctca accacacatt gcgtcatgcg 600

catcgggcgt ggttgctaaa aggtggaaaa atgctggcca gtggacgcag ggaagaggtg 660

ctcacgccgc caaatctggc gcaggcctat gggatgaatt ttcgccgtct ggatatcgaa 720

ggtcacagaa tgctgatttc gaccatctga 750

<210> 132
<211> 249
<212> PRT
<213> Escherichia coli

<400> 132

Met Ser Ile Val Met Gln Leu Gln Asp Val Ala Glu Ser Thr Arg Leu
1 5 10 15

Gly Pro Leu Ser Gly Glu Val Arg Ala Gly Glu Ile Leu His Leu Val
20 25 30

Gly Pro Asn Gly Ala Gly Lys Ser Thr Leu Leu Ala Arg Met Ala Gly
35 40 45

Met Thr Ser Gly Lys Gly Ser Ile Gln Phe Ala Gly Gln Pro Leu Glu
50 55 60

Ala Trp Ser Ala Thr Lys Leu Ala Leu His Arg Ala Tyr Leu Ser Gln
65 70 75 80

Gln Gln Thr Pro Pro Phe Ala Thr Pro Val Trp His Tyr Leu Thr Leu
85 90 95

His Gln His Asp Lys Thr Arg Thr Glu Leu Leu Asn Asp Val Ala Gly
100 105 110

Ala Leu Ala Leu Asp Asp Lys Leu Gly Arg Ser Thr Asn Gln Leu Ser
115 120 125

Gly Gly Glu Trp Gln Arg Val Arg Leu Ala Ala Val Val Leu Gln Ile
130 135 140

Thr Pro Gln Ala Asn Pro Ala Gly Gln Leu Leu Leu Leu Asp Glu Pro
145 150 155 160

Met Asn Ser Leu Asp Val Ala Gln Gln Ser Ala Leu Asp Lys Ile Leu
165 170 175

Ser Ala Leu Cys Gln Gln Gly Leu Ala Ile Val Met Ser Ser His Asp
180 185 190

Leu Asn His Thr Leu Arg His Ala His Arg Ala Trp Leu Leu Lys Gly
195 200 205

Gly Lys Met Leu Ala Ser Gly Arg Arg Glu Glu Val Leu Thr Pro Pro
210 215 220

Asn Leu Ala Gln Ala Tyr Gly Met Asn Phe Arg Arg Leu Asp Ile Glu
225 230 235 240

Gly His Arg Met Leu Ile Ser Thr Ile
245

<210> 133
<211> 801
<212> DNA
<213> Escherichia coli

<400> 133
atggctaagt cactgttcag ggcgctggtc gccctgtctt ttcttgcgcc actgtggctc 60

aacgccgcgc cgcgcgtcat cacgctttct cccgccaaca ctgaacttgc ctttgccgcc 120

gggatcacgc cggttggggt cagcagctat tccgactatc ctccacaagc gcaaaagatt 180

gagcaggttt ccacctggca ggggatgaat ctggaacgca ttgtcgcgct gaaacccgat 240

ctggtgattg cctggcgtgg aggtaatgcc gagcggcagg ttgaccagct ggcttcgctg 300

ggaataaaag tgatgtgggt cgatgcgaca agcattgaac aaattgccaa tgcgttacgt 360

caactggccc cctggagtcc gcaaccagac aaggccgaac aagccgcgca atccctgctg 420

gatcagtacg cgcaattgaa agcgcaatat gctgataaac ctaaaaaacg tgtttttctg 480

caattcggca ttaatccgcc atttaccagt ggaaaagagt cgattcagaa ccaggtactc 540

gaagtttgtg gcggagaaaa catctttaaa gacagccggg ttccctggcc gcaagttagc 600

cgcgaacagg tgttagcacg ctcgccacag gcgattgtca ttacaggcgg accggaccaa 660

attcctaaaa tcaaacaata ctggggtgaa cagctcaaaa ttcccgttat tcctctcacg 720

agtgactggt ttgaacgtgc aagcccacgt attatcctcg ctgcacaaca gctctgtaat 780

gcgctttcac aggtagatta g 801

<210> 134
<211> 266
<212> PRT
<213> Escherichia coli

<400> 134

Met Ala Lys Ser Leu Phe Arg Ala Leu Val Ala Leu Ser Phe Leu Ala
1 5 10 15

Pro Leu Trp Leu Asn Ala Ala Pro Arg Val Ile Thr Leu Ser Pro Ala
20 25 30

Asn Thr Glu Leu Ala Phe Ala Ala Gly Ile Thr Pro Val Gly Val Ser
35 40 45

Ser Tyr Ser Asp Tyr Pro Pro Gln Ala Gln Lys Ile Glu Gln Val Ser
50 55 60

Thr Trp Gln Gly Met Asn Leu Glu Arg Ile Val Ala Leu Lys Pro Asp
65 70 75 80

Leu Val Ile Ala Trp Arg Gly Gly Asn Ala Glu Arg Gln Val Asp Gln
85 90 95

Leu Ala Ser Leu Gly Ile Lys Val Met Trp Val Asp Ala Thr Ser Ile
100 105 110

Glu Gln Ile Ala Asn Ala Leu Arg Gln Leu Ala Pro Trp Ser Pro Gln
115 120 125

Pro Asp Lys Ala Glu Gln Ala Ala Gln Ser Leu Leu Asp Gln Tyr Ala
130 135 140

Gln Leu Lys Ala Gln Tyr Ala Asp Lys Pro Lys Lys Arg Val Phe Leu
145 150 155 160

Gln Phe Gly Ile Asn Pro Pro Phe Thr Ser Gly Lys Glu Ser Ile Gln
165 170 175

Asn Gln Val Leu Glu Val Cys Gly Gly Glu Asn Ile Phe Lys Asp Ser
180 185 190

Arg Val Pro Trp Pro Gln Val Ser Arg Glu Gln Val Leu Ala Arg Ser
195 200 205

Pro Gln Ala Ile Val Ile Thr Gly Gly Pro Asp Gln Ile Pro Lys Ile
    210                 215                 220

Lys Gln Tyr Trp Gly Glu Gln Leu Lys Ile Pro Val Ile Pro Leu Thr
225                 230                 235                 240

Ser Asp Trp Phe Glu Arg Ala Ser Pro Arg Ile Ile Leu Ala Ala Gln
                245                 250                 255

Gln Leu Cys Asn Ala Leu Ser Gln Val Asp
            260                 265

<210> 135
<211> 1197
<212> DNA
<213> Escherichia coli

<400> 135
atgcgtggag aattttatca gcagttaacc aacgatctgg aaaccgcacg ggcggaaggg 60

ttgtttaaag aagagcgcat tattacgtct gcgcagcaag cagatatcac tgtggctgat 120

ggaagccacg tcattaactt ttgtgccaac aactatctcg ggctggcgaa tcatcctgat 180

ctgattgcgg cggcaaaggc gggaatggat tctcacggtt tcggcatggc ttcggtgcgt 240

tttatttgcg gcactcagga cagccataaa gagcttgaac aaaaactggc ggccttcctg 300

gggatggaag atgcgattct ctactcttcc tgctttgatg ctaacggtgg cctgtttgaa 360

acgcttctgg gtgcggaaga cgccattatc tccgacgcac tgaaccacgc gtctattatt 420

gatggtgtgc gtctgtgcaa agctaaacgc tatcgctatg ccaacaacga tatgcaggag 480

ctggaagcac gtctgaaaga agcgcgtgaa gccggtgcgc gtcatgtgct gatcgccacc 540

gatggtgtgt tctcaatgga cggcgtgatt gccaacctga agggcgtttg cgatctggca 600

gataaatatg atgccctggt gatggtagac gactcccacg cggtcggttt tgtcggtgaa 660

aatggtcgtg gttcccatga atactgcgat gtgatgggcc gggtcgatat tatcaccggt 720

acgcttggta aagcgctggg cggggcttct ggtggttata ccgcggcgcg caaagaagtg 780

gttgagtggc tgcgccagcg ttctcgtccg tacctgttct ccaactcgct ggcaccggcc 840

attgttgccg cgtccatcaa agtactggag atggtcgaag cgggcagcga actgcgtgac 900

cgtctgtggg cgaacgcgcg tcagttccgt gagcaaatgt cggcggcggg ctttaccctg 960

gcgggagccg atcacgccat tattccggtc atgcttggtg atgcggtagt ggcgcagaaa 1020

tttgcccgtg agctgcaaaa agagggcatt tacgttaccg gtttcttcta tccggtcgtt 1080

ccgaaaggtc aggcgcgtat tcgtacccag atgtctgcgg cgcatacccc tgagcaaatt 1140

acgcgtgcag tagaagcatt tacgcgtatt ggtaaacaac tgggcgttat cgcctga 1197

<210> 136
<211> 398
<212> PRT
<213> Escherichia coli

<400> 136

```
Met Arg Gly Glu Phe Tyr Gln Gln Leu Thr Asn Asp Leu Glu Thr Ala
1               5                   10                  15

Arg Ala Glu Gly Leu Phe Lys Glu Glu Arg Ile Ile Thr Ser Ala Gln
            20                  25                  30

Gln Ala Asp Ile Thr Val Ala Asp Gly Ser His Val Ile Asn Phe Cys
        35                  40                  45

Ala Asn Asn Tyr Leu Gly Leu Ala Asn His Pro Asp Leu Ile Ala Ala
    50                  55                  60

Ala Lys Ala Gly Met Asp Ser His Gly Phe Gly Met Ala Ser Val Arg
65                  70                  75                  80

Phe Ile Cys Gly Thr Gln Asp Ser His Lys Glu Leu Glu Gln Lys Leu
                85                  90                  95

Ala Ala Phe Leu Gly Met Glu Asp Ala Ile Leu Tyr Ser Ser Cys Phe
            100                 105                 110

Asp Ala Asn Gly Gly Leu Phe Glu Thr Leu Leu Gly Ala Glu Asp Ala
        115                 120                 125

Ile Ile Ser Asp Ala Leu Asn His Ala Ser Ile Ile Asp Gly Val Arg
    130                 135                 140

Leu Cys Lys Ala Lys Arg Tyr Arg Tyr Ala Asn Asn Asp Met Gln Glu
145                 150                 155                 160

Leu Glu Ala Arg Leu Lys Glu Ala Arg Glu Ala Gly Ala Arg His Val
                165                 170                 175

Leu Ile Ala Thr Asp Gly Val Phe Ser Met Asp Gly Val Ile Ala Asn
            180                 185                 190

Leu Lys Gly Val Cys Asp Leu Ala Asp Lys Tyr Asp Ala Leu Val Met
        195                 200                 205

Val Asp Asp Ser His Ala Val Gly Phe Val Gly Glu Asn Gly Arg Gly
    210                 215                 220
```

Ser His Glu Tyr Cys Asp Val Met Gly Arg Val Asp Ile Ile Thr Gly
225 230 235 240

Thr Leu Gly Lys Ala Leu Gly Gly Ala Ser Gly Gly Tyr Thr Ala Ala
245 250 255

Arg Lys Glu Val Val Glu Trp Leu Arg Gln Arg Ser Arg Pro Tyr Leu
260 265 270

Phe Ser Asn Ser Leu Ala Pro Ala Ile Val Ala Ala Ser Ile Lys Val
275 280 285

Leu Glu Met Val Glu Ala Gly Ser Glu Leu Arg Asp Arg Leu Trp Ala
290 295 300

Asn Ala Arg Gln Phe Arg Glu Gln Met Ser Ala Ala Gly Phe Thr Leu
305 310 315 320

Ala Gly Ala Asp His Ala Ile Ile Pro Val Met Leu Gly Asp Ala Val
325 330 335

Val Ala Gln Lys Phe Ala Arg Glu Leu Gln Lys Glu Gly Ile Tyr Val
340 345 350

Thr Gly Phe Phe Tyr Pro Val Val Pro Lys Gly Gln Ala Arg Ile Arg
355 360 365

Thr Gln Met Ser Ala Ala His Thr Pro Glu Gln Ile Thr Arg Ala Val
370 375 380

Glu Ala Phe Thr Arg Ile Gly Lys Gln Leu Gly Val Ile Ala
385 390 395

<210> 137
<211> 1026
<212> DNA
<213> Escherichia coli

<400> 137
atgaaagcgt tatccaaact gaaagcggaa gagggcatct ggatgaccga cgttcctgta 60

ccggaactcg ggcataacga tctgctgatt aaaatccgta aaacagccat ctgcgggact 120

gacgttcaca tctataactg ggatgagtgg tcgcaaaaaa ccatcccggt gccgatggtc 180

gtgggccatg aatatgtcgg tgaagtggta ggtattggtc aggaagtgaa aggcttcaag 240

atcggcgatc gcgtttctgg cgaaggccat atcacctgtg gtcattgccg caactgtcgt 300

ggtggtcgta cccatttgtg ccgcaacacg ataggcgttg gtgttaatcg cccgggctgc 360

tttgccgaat atctggtgat cccggcattc aacgccttca aaatccccga caatatttcc 420

gatgacttag ccgcaatttt tgatcccttc ggtaacgccg tgcataccgc gctgtcgttt 480

gatctggtgg gcgaagatgt gctggtttct ggtgcaggcc cgattggtat tatggcagcg 540

gcggtggcga aacacgttgg tgcacgcaat gtggtgatca ctgatgttaa cgaataccgc 600

cttgagctgg cgcgtaaaat gggtatcacc cgtgcggtta acgtcgccaa agaaaatctc 660

aatgacgtga tggcggagtt aggcatgacc gaaggttttg atgtcggtct ggaaatgtcc 720

ggtgcgccgc cagcgtttcg taccatgctt gacaccatga atcacggcgg ccgtattgcg 780

atgctgggta ttccgccgtc tgatatgtct atcgactgga ccaaagtgat ctttaaaggc 840

ttgttcatta aaggtattta cggtcgtgag atgtttgaaa cctggtacaa gatggcggcg 900

ctgattcagt ctggcctcga tctttcgccg atcattaccc atcgtttctc tatcgatgat 960

ttccagaagg gctttgacgc tatgcgttcg ggccagtccg ggaaagttat tctgagctgg 1020

gattaa 1026

<210> 138
<211> 341
<212> PRT
<213> Escherichia coli

<400> 138

Met Lys Ala Leu Ser Lys Leu Lys Ala Glu Glu Gly Ile Trp Met Thr
1 5 10 15

Asp Val Pro Val Pro Glu Leu Gly His Asn Asp Leu Leu Ile Lys Ile
20 25 30

Arg Lys Thr Ala Ile Cys Gly Thr Asp Val His Ile Tyr Asn Trp Asp
35 40 45

Glu Trp Ser Gln Lys Thr Ile Pro Val Pro Met Val Val Gly His Glu
50 55 60

Tyr Val Gly Glu Val Val Gly Ile Gly Gln Glu Val Lys Gly Phe Lys
65 70 75 80

Ile Gly Asp Arg Val Ser Gly Glu Gly His Ile Thr Cys Gly His Cys
85 90 95

Arg Asn Cys Arg Gly Gly Arg Thr His Leu Cys Arg Asn Thr Ile Gly
100 105 110

Val Gly Val Asn Arg Pro Gly Cys Phe Ala Glu Tyr Leu Val Ile Pro
115 120 125

```
Ala Phe Asn Ala Phe Lys Ile Pro Asp Asn Ile Ser Asp Asp Leu Ala
    130                 135                 140

Ala Ile Phe Asp Pro Phe Gly Asn Ala Val His Thr Ala Leu Ser Phe
145                 150                 155                 160

Asp Leu Val Gly Glu Asp Val Leu Val Ser Gly Ala Gly Pro Ile Gly
                165                 170                 175

Ile Met Ala Ala Ala Val Ala Lys His Val Gly Ala Arg Asn Val Val
            180                 185                 190

Ile Thr Asp Val Asn Glu Tyr Arg Leu Glu Leu Ala Arg Lys Met Gly
        195                 200                 205

Ile Thr Arg Ala Val Asn Val Ala Lys Glu Asn Leu Asn Asp Val Met
    210                 215                 220

Ala Glu Leu Gly Met Thr Glu Gly Phe Asp Val Gly Leu Glu Met Ser
225                 230                 235                 240

Gly Ala Pro Pro Ala Phe Arg Thr Met Leu Asp Thr Met Asn His Gly
                245                 250                 255

Gly Arg Ile Ala Met Leu Gly Ile Pro Pro Ser Asp Met Ser Ile Asp
            260                 265                 270

Trp Thr Lys Val Ile Phe Lys Gly Leu Phe Ile Lys Gly Ile Tyr Gly
        275                 280                 285

Arg Glu Met Phe Glu Thr Trp Tyr Lys Met Ala Ala Leu Ile Gln Ser
    290                 295                 300

Gly Leu Asp Leu Ser Pro Ile Ile Thr His Arg Phe Ser Ile Asp Asp
305                 310                 315                 320

Phe Gln Lys Gly Phe Asp Ala Met Arg Ser Gly Gln Ser Gly Lys Val
                325                 330                 335

Ile Leu Ser Trp Asp
            340
```

<210>  139
<211>  1002
<212>  DNA
<213>  Escherichia coli

<400> 139
atgattgatt tacgcagtga taccgttacc cgaccaagcc gcgccatgct cgaagcgatg 60

atggccgccc cggttgggga cgacgtttac ggagacgacc ctaccgttaa tgctctgcag 120

gactacgcag cagagctttc cggtaaagaa gccgccattt ttctgcctac cggcactcag 180

gccaacctgg tcgctctgct cagtcactgc gaacgcggcg aagagtatat tgtcggtcag 240

gccgcgcata actatctgtt tgaagccggt ggcgcggcgg tgctgggcag tattcaaccg 300

caacccatag acgcggctgc cgacggcacg ctaccgctgg ataaagtggc gatgaaaatc 360

aaacccgacg atatccattt cgcccgcacc aaattactca gtctggaaaa cacccacaac 420

ggcaaagtgt tgccgcggga atacctgaaa gaagcatggg aatttacccg cgagcgcaat 480

ctggcgctgc atgttgacgg tgcgcgcatc tttaatgccg tggtggctta cggctgcgaa 540

ctgaaagaga tcacgcaata ttgtgattcg ttcaccattt gcctgtcgaa aggtcttggg 600

acgccagtcg gttcattact cgtcggtaat cgtgattaca ttaaacgtgc cattcgctgg 660

cggaaaatga caggtggcgg gatgcgccag tccggcattc tggctgccgc cgggatatat 720

gccctgaaaa ataacgttgc gcgcttgcag gaagaccacg acaacgctgc ctggatggcg 780

gagcagctgc gtgaagcagg cgcggatgtg atgcgtcagg acaccaatat gctgtttgtt 840

cgcgtcgggg aagaaaatgc tgccgcgtta ggcgaataca tgaaagcgag aaacgtgctg 900

attaacgcct cgccgattgt ccgcctggtg acgcatcttg acgtctcgcg cgaacaactg 960

gcggaagtcg ccgcccactg gcgtgcattc ctggcgcgtt aa 1002

<210> 140
<211> 333
<212> PRT
<213> Escherichia coli

<400> 140

Met Ile Asp Leu Arg Ser Asp Thr Val Thr Arg Pro Ser Arg Ala Met
1 5 10 15

Leu Glu Ala Met Met Ala Ala Pro Val Gly Asp Asp Val Tyr Gly Asp
20 25 30

Asp Pro Thr Val Asn Ala Leu Gln Asp Tyr Ala Ala Glu Leu Ser Gly
35 40 45

Lys Glu Ala Ala Ile Phe Leu Pro Thr Gly Thr Gln Ala Asn Leu Val
50 55 60

Ala Leu Leu Ser His Cys Glu Arg Gly Glu Glu Tyr Ile Val Gly Gln
65 70 75 80

```
Ala Ala His Asn Tyr Leu Phe Glu Ala Gly Gly Ala Ala Val Leu Gly
                85                  90                  95

Ser Ile Gln Pro Gln Pro Ile Asp Ala Ala Ala Asp Gly Thr Leu Pro
            100                 105                 110

Leu Asp Lys Val Ala Met Lys Ile Lys Pro Asp Asp Ile His Phe Ala
        115                 120                 125

Arg Thr Lys Leu Leu Ser Leu Glu Asn Thr His Asn Gly Lys Val Leu
    130                 135                 140

Pro Arg Glu Tyr Leu Lys Glu Ala Trp Glu Phe Thr Arg Glu Arg Asn
145                 150                 155                 160

Leu Ala Leu His Val Asp Gly Ala Arg Ile Phe Asn Ala Val Val Ala
                165                 170                 175

Tyr Gly Cys Glu Leu Lys Glu Ile Thr Gln Tyr Cys Asp Ser Phe Thr
            180                 185                 190

Ile Cys Leu Ser Lys Gly Leu Gly Thr Pro Val Gly Ser Leu Leu Val
        195                 200                 205

Gly Asn Arg Asp Tyr Ile Lys Arg Ala Ile Arg Trp Arg Lys Met Thr
    210                 215                 220

Gly Gly Gly Met Arg Gln Ser Gly Ile Leu Ala Ala Ala Gly Ile Tyr
225                 230                 235                 240

Ala Leu Lys Asn Asn Val Ala Arg Leu Gln Glu Asp His Asp Asn Ala
                245                 250                 255

Ala Trp Met Ala Glu Gln Leu Arg Glu Ala Gly Ala Asp Val Met Arg
            260                 265                 270

Gln Asp Thr Asn Met Leu Phe Val Arg Val Gly Glu Glu Asn Ala Ala
        275                 280                 285

Ala Leu Gly Glu Tyr Met Lys Ala Arg Asn Val Leu Ile Asn Ala Ser
    290                 295                 300

Pro Ile Val Arg Leu Val Thr His Leu Asp Val Ser Arg Glu Gln Leu
305                 310                 315                 320

Ala Glu Val Ala Ala His Trp Arg Ala Phe Leu Ala Arg
                325                 330
```

<210> 141
<211> 1443
<212> DNA
<213> Escherichia coli

<400> 141
atgtccagaa ggcttcgcag aacaaaaatc gttaccacgt taggcccagc aacagatcgc 60

gataataatc ttgaaaaagt tatcgcggcg ggtgccaacg ttgtacgtat gaacttttct 120

cacggctcgc ctgaagatca caaaatgcgc gcggataaag ttcgtgagat tgccgcaaaa 180

ctggggcgtc atgtggctat tctgggtgac ctccaggggc ccaaaatccg tgtatccacc 240

tttaaagaag gcaaagtttt cctcaatatt ggggataaat tcctgctcga cgccaacctg 300

ggtaaaggtg aaggcgacaa agaaaaagtc ggtatcgact acaaaggcct gcctgctgac 360

gtcgtgcctg gtgacatcct gctgctggac gatggtcgcg tccagttaaa agtactggaa 420

gttcagggca tgaaagtgtt caccgaagtc accgtcggtg gtcccctctc caacaataaa 480

ggtatcaaca aacttggcgg cggtttgtcg gctgaagcgc tgaccgaaaa agacaaagca 540

gacattaaga ctgcggcgtt gattggcgta gattacctgg ctgtctcctt cccacgctgt 600

ggcgaagatc tgaactatgc ccgtcgcctg gcacgcgatg caggatgtga tgcgaaaatt 660

gttgccaagg ttgaacgtgc ggaagccgtt tgcagccagg atgcaatgga tgacatcatc 720

ctcgcctctg acgtggtaat ggttgcacgt ggcgacctcg gtgtggaaat tggcgacccg 780

gaactggtcg gcattcagaa agcgttgatc cgtcgtgcgc gtcagctaaa ccgagcggta 840

atcacggcga cccagatgat ggagtcaatg attactaacc cgatgccgac gcgtgcagaa 900

gtcatggacg tagcaaacgc cgttctggat ggtactgacg ctgtgatgct gtctgcagaa 960

actgccgctg ggcagtatcc gtcagaaacc gttgcagcca tggcgcgcgt ttgcctgggt 1020

gcggaaaaaa tcccgagcat caacgtttct aaacaccgtc tggacgttca gttcgacaat 1080

gtggaagaag ctattgccat gtcagcaatg tacgcagcta accacctgaa aggcgttacg 1140

gcgatcatca ccatgaccga atcgggtcgt accgcgctga tgacctcccg tatcagctct 1200

ggtctgccaa ttttcgccat gtcgcgccat gaacgtacgc tgaacctgac tgctctctat 1260

cgtggcgtta cgccggtgca ctttgatagc gctaatgacg gcgtagcagc tgccagcgaa 1320

gcggttaatc tgctgcgcga taaaggttac ttgatgtctg gtgacctggt gattgtcacc 1380

cagggcgacg tgatgagtac cgtgggttct actaatacca cgcgtatttt aacggtagag 1440

taa 1443

<210> 142
<211> 480
<212> PRT
<213> Escherichia coli

<400>  142

```
Met Ser Arg Arg Leu Arg Arg Thr Lys Ile Val Thr Thr Leu Gly Pro
1               5                   10                  15

Ala Thr Asp Arg Asp Asn Asn Leu Glu Lys Val Ile Ala Ala Gly Ala
            20                  25                  30

Asn Val Val Arg Met Asn Phe Ser His Gly Ser Pro Glu Asp His Lys
        35                  40                  45

Met Arg Ala Asp Lys Val Arg Glu Ile Ala Ala Lys Leu Gly Arg His
    50                  55                  60

Val Ala Ile Leu Gly Asp Leu Gln Gly Pro Lys Ile Arg Val Ser Thr
65                  70                  75                  80

Phe Lys Glu Gly Lys Val Phe Leu Asn Ile Gly Asp Lys Phe Leu Leu
                85                  90                  95

Asp Ala Asn Leu Gly Lys Gly Glu Gly Asp Lys Glu Lys Val Gly Ile
            100                 105                 110

Asp Tyr Lys Gly Leu Pro Ala Asp Val Val Pro Gly Asp Ile Leu Leu
        115                 120                 125

Leu Asp Asp Gly Arg Val Gln Leu Lys Val Leu Glu Val Gln Gly Met
    130                 135                 140

Lys Val Phe Thr Glu Val Thr Val Gly Gly Pro Leu Ser Asn Asn Lys
145                 150                 155                 160

Gly Ile Asn Lys Leu Gly Gly Gly Leu Ser Ala Glu Ala Leu Thr Glu
                165                 170                 175

Lys Asp Lys Ala Asp Ile Lys Thr Ala Ala Leu Ile Gly Val Asp Tyr
            180                 185                 190

Leu Ala Val Ser Phe Pro Arg Cys Gly Glu Asp Leu Asn Tyr Ala Arg
        195                 200                 205

Arg Leu Ala Arg Asp Ala Gly Cys Asp Ala Lys Ile Val Ala Lys Val
    210                 215                 220

Glu Arg Ala Glu Ala Val Cys Ser Gln Asp Ala Met Asp Asp Ile Ile
225                 230                 235                 240

Leu Ala Ser Asp Val Val Met Val Ala Arg Gly Asp Leu Gly Val Glu
```

245 250 255

Ile Gly Asp Pro Glu Leu Val Gly Ile Gln Lys Ala Leu Ile Arg Arg
260 265 270

Ala Arg Gln Leu Asn Arg Ala Val Ile Thr Ala Thr Gln Met Met Glu
275 280 285

Ser Met Ile Thr Asn Pro Met Pro Thr Arg Ala Glu Val Met Asp Val
290 295 300

Ala Asn Ala Val Leu Asp Gly Thr Asp Ala Val Met Leu Ser Ala Glu
305 310 315 320

Thr Ala Ala Gly Gln Tyr Pro Ser Glu Thr Val Ala Ala Met Ala Arg
325 330 335

Val Cys Leu Gly Ala Glu Lys Ile Pro Ser Ile Asn Val Ser Lys His
340 345 350

Arg Leu Asp Val Gln Phe Asp Asn Val Glu Glu Ala Ile Ala Met Ser
355 360 365

Ala Met Tyr Ala Ala Asn His Leu Lys Gly Val Thr Ala Ile Ile Thr
370 375 380

Met Thr Glu Ser Gly Arg Thr Ala Leu Met Thr Ser Arg Ile Ser Ser
385 390 395 400

Gly Leu Pro Ile Phe Ala Met Ser Arg His Glu Arg Thr Leu Asn Leu
405 410 415

Thr Ala Leu Tyr Arg Gly Val Thr Pro Val His Phe Asp Ser Ala Asn
420 425 430

Asp Gly Val Ala Ala Ala Ser Glu Ala Val Asn Leu Leu Arg Asp Lys
435 440 445

Gly Tyr Leu Met Ser Gly Asp Leu Val Ile Val Thr Gln Gly Asp Val
450 455 460

Met Ser Thr Val Gly Ser Thr Asn Thr Thr Arg Ile Leu Thr Val Glu
465 470 475 480

<210> 143
<211> 1413
<212> DNA
<213> Escherichia coli

<400> 143
atgaaaaaga ccaaaattgt ttgcaccatc ggaccgaaaa ccgaatctga agagatgtta 60

gctaaaatgc tggacgctgg catgaacgtt atgcgtctga acttctctca tggtgactat 120

gcagaacacg gtcagcgcat tcagaatctg cgcaacgtga tgagcaaaac tggtaaaacc 180

gccgctatcc tgcttgatac caaaggtccg gaaatccgca ccatgaaact ggaaggcggt 240

aacgacgttt ctctgaaagc tggtcagacc tttactttca ccactgataa atctgttatc 300

ggcaacagcg aaatggttgc ggtaacgtat gaaggtttca ctactgacct gtctgttggc 360

aacaccgtac tggttgacga tggtctgatc ggtatggaag ttaccgccat tgaaggtaac 420

aaagttatct gtaaagtgct gaacaacggt gacctgggcg aaaacaaagg tgtgaacctg 480

cctggcgttt ccattgctct gccagcactg gctgaaaaag acaaacagga cctgatcttt 540

ggttgcgaac aaggcgtaga ctttgttgct gcttccttta ttcgtaagcg ttctgacgtt 600

atcgaaatcc gtgagcacct gaaagcgcac ggcggcgaaa acatccacat catctccaaa 660

atcgaaaacc aggaaggcct caacaacttc gacgaaatcc tcgaagcctc tgacggcatc 720

atggttgcgc gtggcgacct gggtgtagaa atcccggtag aagaagttat cttcgcccag 780

aagatgatga tcgaaaaatg tatccgtgca cgtaaagtcg ttatcactgc gacccagatg 840

ctggattcca tgatcaaaaa cccacgcccg actcgcgcag aagccggtga cgttgcaaac 900

gccatcctcg acggtactga cgcagtgatg ctgtctggtg aatccgcaaa aggtaaatac 960

ccgctggaag cggtttctat catggcgacc atctgcgaac gtaccgaccg cgtgatgaac 1020

agccgtctcg agttcaacaa tgacaaccgt aaactgcgca ttaccgaagc ggtatgccgt 1080

ggtgccgttg aaactgctga aaaactggat gctccgctga tcgtggttgc tactcagggc 1140

ggtaaatctg ctcgcgcagt acgtaaatac ttcccggatg ccaccatcct ggcactgacc 1200

accaacgaaa aaacggctca tcagttggta ctgagcaaag gcgttgtgcc gcagcttgtt 1260

aaagagatca cttctactga tgatttctac cgtctgggta aagaactggc tctgcagagc 1320

ggtctggcac acaaaggtga cgttgtagtt atggtttctg gtgcactggt accgagcggc 1380

actactaaca ccgcatctgt tcacgtcctg taa 1413

<210> 144
<211> 470
<212> PRT
<213> Escherichia coli

<400> 144

Met Lys Lys Thr Lys Ile Val Cys Thr Ile Gly Pro Lys Thr Glu Ser
1               5                   10                  15

Glu Glu Met Leu Ala Lys Met Leu Asp Ala Gly Met Asn Val Met Arg

20 25 30

Leu Asn Phe Ser His Gly Asp Tyr Ala Glu His Gly Gln Arg Ile Gln
35 40 45

Asn Leu Arg Asn Val Met Ser Lys Thr Gly Lys Thr Ala Ala Ile Leu
50 55 60

Leu Asp Thr Lys Gly Pro Glu Ile Arg Thr Met Lys Leu Glu Gly Gly
65 70 75 80

Asn Asp Val Ser Leu Lys Ala Gly Gln Thr Phe Thr Phe Thr Thr Asp
85 90 95

Lys Ser Val Ile Gly Asn Ser Glu Met Val Ala Val Thr Tyr Glu Gly
100 105 110

Phe Thr Thr Asp Leu Ser Val Gly Asn Thr Val Leu Val Asp Asp Gly
115 120 125

Leu Ile Gly Met Glu Val Thr Ala Ile Glu Gly Asn Lys Val Ile Cys
130 135 140

Lys Val Leu Asn Asn Gly Asp Leu Gly Glu Asn Lys Gly Val Asn Leu
145 150 155 160

Pro Gly Val Ser Ile Ala Leu Pro Ala Leu Ala Glu Lys Asp Lys Gln
165 170 175

Asp Leu Ile Phe Gly Cys Glu Gln Gly Val Asp Phe Val Ala Ala Ser
180 185 190

Phe Ile Arg Lys Arg Ser Asp Val Ile Glu Ile Arg Glu His Leu Lys
195 200 205

Ala His Gly Gly Glu Asn Ile His Ile Ile Ser Lys Ile Glu Asn Gln
210 215 220

Glu Gly Leu Asn Asn Phe Asp Glu Ile Leu Glu Ala Ser Asp Gly Ile
225 230 235 240

Met Val Ala Arg Gly Asp Leu Gly Val Glu Ile Pro Val Glu Glu Val
245 250 255

Ile Phe Ala Gln Lys Met Met Ile Glu Lys Cys Ile Arg Ala Arg Lys
260 265 270

```
Val Val Ile Thr Ala Thr Gln Met Leu Asp Ser Met Ile Lys Asn Pro
        275                 280                 285

Arg Pro Thr Arg Ala Glu Ala Gly Asp Val Ala Asn Ala Ile Leu Asp
    290                 295                 300

Gly Thr Asp Ala Val Met Leu Ser Gly Glu Ser Ala Lys Gly Lys Tyr
305                 310                 315                 320

Pro Leu Glu Ala Val Ser Ile Met Ala Thr Ile Cys Glu Arg Thr Asp
                325                 330                 335

Arg Val Met Asn Ser Arg Leu Glu Phe Asn Asn Asp Asn Arg Lys Leu
            340                 345                 350

Arg Ile Thr Glu Ala Val Cys Arg Gly Ala Val Glu Thr Ala Glu Lys
        355                 360                 365

Leu Asp Ala Pro Leu Ile Val Val Ala Thr Gln Gly Gly Lys Ser Ala
    370                 375                 380

Arg Ala Val Arg Lys Tyr Phe Pro Asp Ala Thr Ile Leu Ala Leu Thr
385                 390                 395                 400

Thr Asn Glu Lys Thr Ala His Gln Leu Val Leu Ser Lys Gly Val Val
                405                 410                 415

Pro Gln Leu Val Lys Glu Ile Thr Ser Thr Asp Asp Phe Tyr Arg Leu
            420                 425                 430

Gly Lys Glu Leu Ala Leu Gln Ser Gly Leu Ala His Lys Gly Asp Val
        435                 440                 445

Val Val Met Val Ser Gly Ala Leu Val Pro Ser Gly Thr Thr Asn Thr
    450                 455                 460

Ala Ser Val His Val Leu
465                 470


<210>  145
<211>  3114
<212>  DNA
<213>  Rhizobium etli

<400>  145
atgcgccagc ttggcaacaa ggtcgcagcg cgcaacctgg cgatctcggt cggcgtaccg      60

gtcgtgccgg cgaccgagcc actgccggac gatatggccg aagtggcgaa gatggcggcg     120

gcgatcggct atcccgtcat gctgaaggca tcctggggcg gcggcggtcg cggcatgcgc     180
```

gtcattcgtt ccgaggccga cctcgccaag gaagtgacgg aagccaagcg cgaggcgatg 240

gcggccttcg gcaaggacga ggtctatctc gaaaaactgg tcgagcgcgc ccgccacgtc 300

gaaagccaga tcctcggcga cacccacggc aatgtcgtgc atctcttcga gcgcgactgt 360

tccgttcagc gccgcaatca gaaggtcgtc gagcgcgcgc ccgcacccta tctttcggaa 420

gcgcagcgcc aggaactcgc cgcctattcg ctgaagatcg caggggcgac caactatatc 480

ggcgccggca ccgtcgaata tctgatggat gccgataccg gcaaatttta cttcatcgaa 540

gtcaatccgc gcatccaggt cgagcacacg gtgaccgaag tcgtcaccgg catcgatatc 600

gtcaaggcgc agatccacat cctggacggc gccgcgatcg gcacgccgca atccggcgtg 660

ccgaaccagg aagacatccg tctcaacggt cacgccctgc agtgccgcgt gacgacggaa 720

gatccggagc acaacttcat tccggattac ggccgcatca ccgcctatcg ctcggcttcc 780

ggcttcggca tccggcttga cggcggcacc tcttattccg gcgccatcat cacccgctat 840

tacgatccgc tgctcgtcaa ggtcacggcc tgggcgccga acccgctgga agccatttcc 900

cgcatggacc gggcgctgcg cgaattccgc atccgtggcg tcgccaccaa cctgaccttc 960

ctcgaagcga tcatcggcca tccgaaattc cgcgacaaca gctacaccac ccgcttcatc 1020

gacacgacgc cggagctctt ccagcaggtc aagcgccagg accgcgcgac gaagcttctg 1080

acctatctcg ccgacgtcac cgtcaatggc catcccgagg ccaaggacag gccgaagccc 1140

ctcgagaatg ccgccaggcc ggtggtgccc tatgccaatg gcaacggggt gaaggacggc 1200

accaagcagc tgctcgatac gctcggcccg aaaaaattcg gcgaatggat gcgcaatgag 1260

aagcgcgtgc ttctgaccga caccacgatg cgcgacggcc accagtcgct gctcgcaacc 1320

cgcatgcgta cctatgacat cgccaggatc gccggcacct attcgcatgc gctgccgaac 1380

ctcttgtcgc tcgaatgctg ggcggcgccc accttcgacg tctcgatgcg cttcctcacc 1440

gaagatccgt gggagcggct ggcgctgatc cgagaggggg cgccgaacct gctcctgcag 1500

atgctgctgc gcggcgccaa tggcgtcggt tacaccaact atcccgacaa tgtcgtcaaa 1560

tacttcgtcc gccaggcggc caaaggcggc atcgatctct tccgcgtctt cgactgcctg 1620

aactgggtcg agaatatgcg ggtgtcgatg gatgcgattg ccgaggagaa caagctctgc 1680

gaggcggcga tctgctacac cggcgatatc ctcaattccg cccgcccgaa atacgacttg 1740

aaatattaca ccaaccttgc cgtcgagctt gagaaggccg gcgcccatat cattgcggtc 1800

aaggatatgg cgggccttct gaagccggct gctgccaagg ttctgttcaa ggcgctgcgt 1860

gaagcaaccg gcctgccgat ccatttccac acgcatgaca cctcgggcat tgcggcggca 1920

acggttcttg ccgccgtcga agccggtgtc gatgccgtcg atgcggcgat ggatgcgctc 1980

tccggcaaca cctcgcaacc ctgtctcggc tcgatcgtcg aggcgctctc cggctccgag 2040

cgcgatcccg gcctcgatcc ggcatggatc cgccgcatct ccttctattg ggaagcggtg 2100

cgcaaccagt atgccgcctt cgaaagcgac ctcaagggac cggcatcgga agtctatctg 2160

catgaaatgc cgggcggcca gttcaccaac ctcaaggagc aggcccgctc gctggggctg 2220

gaaacccgct ggcaccaggt ggcgcaggcc tatgccgacg ccaaccagat gttcggcgat 2280

atcgtcaagg tgacgccatc ctccaaggtc gtcggcgaca tggcgctgat gatggtctcc 2340

caggacctga ccgtcgccga tgtcgtcagc cccgaccgcg aagtctcctt cccggaatcg 2400

gtcgtctcga tgctgaaggg cgatctcggc cagcctccgt ctggatggcc ggaagcgctg 2460

cagaagaaag cattgaaggg cgaaaagccc tatacggtgc gccccggctc gctgctcaag 2520

gaagccgatc tcgatgcgga acgcaaagtc atcgagaaga agcttgagcg cgaggtcagc 2580

gacttcgaat tcgcttccta tctgatgtat ccgaaggtct tcaccgactt tgcgcttgcc 2640

tccgatacct acggtccggt ttcggtgctg ccgacgcccg cctattttta cgggttggcg 2700

gacggcgagg agctgttcgc cgacatcgag aagggcaaga cgctcgtcat cgtcaatcag 2760

gcggtgagcg ccaccgacag ccagggcatg gtcactgtct tcttcgagct caacggccag 2820

ccgcgccgta tcaaggtgcc cgatcgggcc cacggggcga cgggagccgc cgtgcgccgc 2880

aaggccgaac ccggcaatgc cgcccatgtc ggtgcgccga tgccgggcgt catcagccgt 2940

gtctttgtct cttcaggcca ggccgtcaat gccggcgacg tgctcgtctc catcgaggcc 3000

atgaagatgg aaaccgcgat ccatgcggaa aaggacggca ccattgccga agtgctggtc 3060

aaggccggcg atcagatcga tgccaaggac ctgctggcgg tttacggcgg atga 3114

<210> 146
<211> 1037
<212> PRT
<213> Rhizobium etli

<400> 146

Met Arg Gln Leu Gly Asn Lys Val Ala Ala Arg Asn Leu Ala Ile Ser
1 5 10 15

Val Gly Val Pro Val Val Pro Ala Thr Glu Pro Leu Pro Asp Asp Met
20 25 30

Ala Glu Val Ala Lys Met Ala Ala Ala Ile Gly Tyr Pro Val Met Leu
35 40 45

Lys Ala Ser Trp Gly Gly Gly Gly Arg Gly Met Arg Val Ile Arg Ser
50 55 60

Glu Ala Asp Leu Ala Lys Glu Val Thr Glu Ala Lys Arg Glu Ala Met
65 70 75 80

Ala Ala Phe Gly Lys Asp Glu Val Tyr Leu Glu Lys Leu Val Glu Arg
85 90 95

Ala Arg His Val Glu Ser Gln Ile Leu Gly Asp Thr His Gly Asn Val
100 105 110

Val His Leu Phe Glu Arg Asp Cys Ser Val Gln Arg Arg Asn Gln Lys
115 120 125

Val Val Glu Arg Ala Pro Ala Pro Tyr Leu Ser Glu Ala Gln Arg Gln
130 135 140

Glu Leu Ala Ala Tyr Ser Leu Lys Ile Ala Gly Ala Thr Asn Tyr Ile
145 150 155 160

Gly Ala Gly Thr Val Glu Tyr Leu Met Asp Ala Asp Thr Gly Lys Phe
165 170 175

Tyr Phe Ile Glu Val Asn Pro Arg Ile Gln Val Glu His Thr Val Thr
180 185 190

Glu Val Val Thr Gly Ile Asp Ile Val Lys Ala Gln Ile His Ile Leu
195 200 205

Asp Gly Ala Ala Ile Gly Thr Pro Gln Ser Gly Val Pro Asn Gln Glu
210 215 220

Asp Ile Arg Leu Asn Gly His Ala Leu Gln Cys Arg Val Thr Thr Glu
225 230 235 240

Asp Pro Glu His Asn Phe Ile Pro Asp Tyr Gly Arg Ile Thr Ala Tyr
245 250 255

Arg Ser Ala Ser Gly Phe Gly Ile Arg Leu Asp Gly Gly Thr Ser Tyr
260 265 270

Ser Gly Ala Ile Ile Thr Arg Tyr Tyr Asp Pro Leu Leu Val Lys Val
275 280 285

Thr Ala Trp Ala Pro Asn Pro Leu Glu Ala Ile Ser Arg Met Asp Arg
290 295 300

Ala Leu Arg Glu Phe Arg Ile Arg Gly Val Ala Thr Asn Leu Thr Phe
305 310 315 320

Leu Glu Ala Ile Ile Gly His Pro Lys Phe Arg Asp Asn Ser Tyr Thr
325 330 335

Thr Arg Phe Ile Asp Thr Thr Pro Glu Leu Phe Gln Gln Val Lys Arg
340 345 350

Gln Asp Arg Ala Thr Lys Leu Leu Thr Tyr Leu Ala Asp Val Thr Val
355 360 365

Asn Gly His Pro Glu Ala Lys Asp Arg Pro Lys Pro Leu Glu Asn Ala
370 375 380

Ala Arg Pro Val Val Pro Tyr Ala Asn Gly Asn Gly Val Lys Asp Gly
385 390 395 400

Thr Lys Gln Leu Leu Asp Thr Leu Gly Pro Lys Lys Phe Gly Glu Trp
405 410 415

Met Arg Asn Glu Lys Arg Val Leu Leu Thr Asp Thr Thr Met Arg Asp
420 425 430

Gly His Gln Ser Leu Leu Ala Thr Arg Met Arg Thr Tyr Asp Ile Ala
435 440 445

Arg Ile Ala Gly Thr Tyr Ser His Ala Leu Pro Asn Leu Leu Ser Leu
450 455 460

Glu Cys Trp Gly Gly Ala Thr Phe Asp Val Ser Met Arg Phe Leu Thr
465 470 475 480

Glu Asp Pro Trp Glu Arg Leu Ala Leu Ile Arg Glu Gly Ala Pro Asn
485 490 495

Leu Leu Leu Gln Met Leu Leu Arg Gly Ala Asn Gly Val Gly Tyr Thr
500 505 510

Asn Tyr Pro Asp Asn Val Val Lys Tyr Phe Val Arg Gln Ala Ala Lys
515 520 525

Gly Gly Ile Asp Leu Phe Arg Val Phe Asp Cys Leu Asn Trp Val Glu
530 535 540

Asn Met Arg Val Ser Met Asp Ala Ile Ala Glu Glu Asn Lys Leu Cys
545 550 555 560

Glu Ala Ala Ile Cys Tyr Thr Gly Asp Ile Leu Asn Ser Ala Arg Pro
565 570 575

Lys Tyr Asp Leu Lys Tyr Tyr Thr Asn Leu Ala Val Glu Leu Glu Lys

580 585 590

Ala Gly Ala His Ile Ile Ala Val Lys Asp Met Ala Gly Leu Leu Lys
595 600 605

Pro Ala Ala Ala Lys Val Leu Phe Lys Ala Leu Arg Glu Ala Thr Gly
610 615 620

Leu Pro Ile His Phe His Thr His Asp Thr Ser Gly Ile Ala Ala Ala
625 630 635 640

Thr Val Leu Ala Ala Val Glu Ala Gly Val Asp Ala Val Asp Ala Ala
645 650 655

Met Asp Ala Leu Ser Gly Asn Thr Ser Gln Pro Cys Leu Gly Ser Ile
660 665 670

Val Glu Ala Leu Ser Gly Ser Glu Arg Asp Pro Gly Leu Asp Pro Ala
675 680 685

Trp Ile Arg Arg Ile Ser Phe Tyr Trp Glu Ala Val Arg Asn Gln Tyr
690 695 700

Ala Ala Phe Glu Ser Asp Leu Lys Gly Pro Ala Ser Glu Val Tyr Leu
705 710 715 720

His Glu Met Pro Gly Gly Gln Phe Thr Asn Leu Lys Glu Gln Ala Arg
725 730 735

Ser Leu Gly Leu Glu Thr Arg Trp His Gln Val Ala Gln Ala Tyr Ala
740 745 750

Asp Ala Asn Gln Met Phe Gly Asp Ile Val Lys Val Thr Pro Ser Ser
755 760 765

Lys Val Val Gly Asp Met Ala Leu Met Met Val Ser Gln Asp Leu Thr
770 775 780

Val Ala Asp Val Val Ser Pro Asp Arg Glu Val Ser Phe Pro Glu Ser
785 790 795 800

Val Val Ser Met Leu Lys Gly Asp Leu Gly Gln Pro Pro Ser Gly Trp
805 810 815

Pro Glu Ala Leu Gln Lys Lys Ala Leu Lys Gly Glu Lys Pro Tyr Thr
820 825 830

Val Arg Pro Gly Ser Leu Leu Lys Glu Ala Asp Leu Asp Ala Glu Arg
835 840 845

Lys Val Ile Glu Lys Lys Leu Glu Arg Glu Val Ser Asp Phe Glu Phe
850 855 860

Ala Ser Tyr Leu Met Tyr Pro Lys Val Phe Thr Asp Phe Ala Leu Ala
865 870 875 880

Ser Asp Thr Tyr Gly Pro Val Ser Val Leu Pro Thr Pro Ala Tyr Phe
885 890 895

Tyr Gly Leu Ala Asp Gly Glu Glu Leu Phe Ala Asp Ile Glu Lys Gly
900 905 910

Lys Thr Leu Val Ile Val Asn Gln Ala Val Ser Ala Thr Asp Ser Gln
915 920 925

Gly Met Val Thr Val Phe Phe Glu Leu Asn Gly Gln Pro Arg Arg Ile
930 935 940

Lys Val Pro Asp Arg Ala His Gly Ala Thr Gly Ala Ala Val Arg Arg
945 950 955 960

Lys Ala Glu Pro Gly Asn Ala Ala His Val Gly Ala Pro Met Pro Gly
965 970 975

Val Ile Ser Arg Val Phe Val Ser Ser Gly Gln Ala Val Asn Ala Gly
980 985 990

Asp Val Leu Val Ser Ile Glu Ala Met Lys Met Glu Thr Ala Ile His
995 1000 1005

Ala Glu Lys Asp Gly Thr Ile Ala Glu Val Leu Val Lys Ala Gly
1010 1015 1020

Asp Gln Ile Asp Ala Lys Asp Leu Leu Ala Val Tyr Gly Gly
1025 1030 1035

<210> 147
<211> 1284
<212> DNA
<213> Escherichia coli

<400> 147
atggctgata caaaagcaaa actcaccctc aacggggata cagctgttga actggatgtg 60

ctgaaaggca cgctgggtca agatgttatt gatatccgta ctctcggttc aaaaggtgtg 120

ttcacctttg acccaggctt cacttcaacc gcatcctgcg aatctaaaat tacttttatt 180

gatggtgatg aaggtatttt gctgcaccgc ggtttcccga tcgatcagct ggcgaccgat 240
tctaactacc tggaagtttg ttacatcctg ctgaatggtg aaaaaccgac tcaggaacag 300
tatgacgaat ttaaaactac ggtgacccgt cataccatga tccacgagca gattacccgt 360
ctgttccatg ctttccgtcg cgactcgcat ccaatggcag tcatgtgtgg tattaccggc 420
gcgctggcgg cgttctatca cgactcgctg gatgttaaca atcctcgtca ccgtgaaatt 480
gccgcgttcc gcctgctgtc gaaaatgccg accatggccg cgatgtgtta caagtattcc 540
attggtcagc catttgttta cccgcgcaac gatctctcct acgccggtaa cttcctgaat 600
atgatgttct ccacgccgtg cgaaccgtat gaagttaatc cgattctgga acgtgctatg 660
gaccgtattc tgatcctgca cgctgaccat gaacagaacg cctctacctc caccgtgcgt 720
accgctggct cttcgggtgc gaacccgttt gcctgtatcg cagcaggtat tgcttcactg 780
tggggacctg cgcacggcgg tgctaacgaa gcggcgctga aaatgctgga agaaatcagc 840
tccgttaaac acattccgga atttgttcgt cgtgcgaaag acaaaaatga ttctttccgc 900
ctgatgggct tcggtcaccg cgtgtacaaa aattacgacc cgcgcgccac cgtaatgcgt 960
gaaacctgcc atgaagtgct gaaagagctg ggcacgaagg atgacctgct ggaagtggct 1020
atggagctgg aaaacatcgc gctgaacgac ccgtacttta tcgagaagaa actgtacccg 1080
aacgtcgatt tctactctgg tatcatcctg aaagcgatgg gtattccgtc ttccatgttc 1140
accgtcattt tcgcaatggc acgtaccgtt ggctggatcg cccactggag cgaaatgcac 1200
agtgacggta tgaagattgc ccgtccgcgt cagctgtata caggatatga aaaacgcgac 1260
tttaaaagcg atatcaagcg ttaa 1284

<210> 148
<211> 427
<212> PRT
<213> Escherichia coli

<400> 148

Met Ala Asp Thr Lys Ala Lys Leu Thr Leu Asn Gly Asp Thr Ala Val
1 5 10 15

Glu Leu Asp Val Leu Lys Gly Thr Leu Gly Gln Asp Val Ile Asp Ile
20 25 30

Arg Thr Leu Gly Ser Lys Gly Val Phe Thr Phe Asp Pro Gly Phe Thr
35 40 45

Ser Thr Ala Ser Cys Glu Ser Lys Ile Thr Phe Ile Asp Gly Asp Glu
50 55 60

Gly Ile Leu Leu His Arg Gly Phe Pro Ile Asp Gln Leu Ala Thr Asp
65 70 75 80

Ser Asn Tyr Leu Glu Val Cys Tyr Ile Leu Leu Asn Gly Glu Lys Pro
85 90 95

Thr Gln Glu Gln Tyr Asp Glu Phe Lys Thr Thr Val Thr Arg His Thr
100 105 110

Met Ile His Glu Gln Ile Thr Arg Leu Phe His Ala Phe Arg Arg Asp
115 120 125

Ser His Pro Met Ala Val Met Cys Gly Ile Thr Gly Ala Leu Ala Ala
130 135 140

Phe Tyr His Asp Ser Leu Asp Val Asn Asn Pro Arg His Arg Glu Ile
145 150 155 160

Ala Ala Phe Arg Leu Leu Ser Lys Met Pro Thr Met Ala Ala Met Cys
165 170 175

Tyr Lys Tyr Ser Ile Gly Gln Pro Phe Val Tyr Pro Arg Asn Asp Leu
180 185 190

Ser Tyr Ala Gly Asn Phe Leu Asn Met Met Phe Ser Thr Pro Cys Glu
195 200 205

Pro Tyr Glu Val Asn Pro Ile Leu Glu Arg Ala Met Asp Arg Ile Leu
210 215 220

Ile Leu His Ala Asp His Glu Gln Asn Ala Ser Thr Ser Thr Val Arg
225 230 235 240

Thr Ala Gly Ser Ser Gly Ala Asn Pro Phe Ala Cys Ile Ala Ala Gly
245 250 255

Ile Ala Ser Leu Trp Gly Pro Ala His Gly Gly Ala Asn Glu Ala Ala
260 265 270

Leu Lys Met Leu Glu Glu Ile Ser Ser Val Lys His Ile Pro Glu Phe
275 280 285

Val Arg Arg Ala Lys Asp Lys Asn Asp Ser Phe Arg Leu Met Gly Phe
290 295 300

Gly His Arg Val Tyr Lys Asn Tyr Asp Pro Arg Ala Thr Val Met Arg
305 310 315 320

```
Glu Thr Cys His Glu Val Leu Lys Glu Leu Gly Thr Lys Asp Asp Leu
                325                 330                 335

Leu Glu Val Ala Met Glu Leu Glu Asn Ile Ala Leu Asn Asp Pro Tyr
            340                 345                 350

Phe Ile Glu Lys Lys Leu Tyr Pro Asn Val Asp Phe Tyr Ser Gly Ile
        355                 360                 365

Ile Leu Lys Ala Met Gly Ile Pro Ser Ser Met Phe Thr Val Ile Phe
    370                 375                 380

Ala Met Ala Arg Thr Val Gly Trp Ile Ala His Trp Ser Glu Met His
385                 390                 395                 400

Ser Asp Gly Met Lys Ile Ala Arg Pro Arg Gln Leu Tyr Thr Gly Tyr
                405                 410                 415

Glu Lys Arg Asp Phe Lys Ser Asp Ile Lys Arg
            420                 425
```

<210> 149
<211> 1167
<212> DNA
<213> Escherichia coli

<400> 149

```
atgaacttac atgaatatca ggcaaaacaa ctttttgccc gctatggctt accagcaccg      60
gtgggttatg cctgtactac tccgcgcgaa gcagaagaag ccgcttcaaa aatcggtgcc     120
ggtccgtggg tagtgaaatg tcaggttcac gctggtggcc gcggtaaagc gggcggtgtg     180
aaagttgtaa acagcaaaga agacatccgt gcttttgcag aaaactggct gggcaagcgt     240
ctggtaacgt atcaaacaga tgccaatggc caaccggtta accagattct ggttgaagca     300
gcgaccgata tcgctaaaga gctgtatctc ggtgccgttg ttgaccgtag ttcccgtcgt     360
gtggtcttta tggcctccac cgaaggcggc gtggaaatcg aaaaagtggc ggaagaaact     420
ccgcacctga tccataaagt tgcgcttgat ccgctgactg gcccgatgcc gtatcaggga     480
cgcgagctgg cgttcaaact gggtctggaa ggtaaactgg ttcagcagtt caccaaaatc     540
ttcatgggcc tggcgaccat ttttctggag cgcgacctgg cgttgatcga aatcaacccg     600
ctggtcatca ccaaacaggg cgatctgatt tgcctcgacg gcaaactggg cgctgacggc     660
aacgcactgt tccgccagcc tgatctgcgc gaaatgcgtg accagtcgca ggaagatccg     720
cgtgaagcac aggctgcaca gtgggaactg aactacgttg cgctggacgg taacatcggt     780
tgtatggtta acggcgcagg tctggcgatg ggtacgatgg acatcgttaa actgcacggc     840
```

ggcgaaccgg ctaacttcct tgacgttggc ggcggcgcaa ccaaagaacg tgtaaccgaa 900

gcgttcaaaa tcatcctctc tgacgacaaa gtgaaagccg ttctggttaa catcttcggc 960

ggtatcgttc gttgcgacct gatcgctgac ggtatcatcg gcgcggtagc agaagtgggt 1020

gttaacgtac cggtcgtggt acgtctggaa ggtaacaacg ccgaactcgg cgcgaagaaa 1080

ctggctgaca gcggcctgaa tattattgca gcaaaaggtc tgacggatgc agctcagcag 1140

gttgttgccg cagtggaggg gaaataa 1167

<210> 150
<211> 388
<212> PRT
<213> Escherichia coli

<400> 150

Met Asn Leu His Glu Tyr Gln Ala Lys Gln Leu Phe Ala Arg Tyr Gly
1 5 10 15

Leu Pro Ala Pro Val Gly Tyr Ala Cys Thr Thr Pro Arg Glu Ala Glu
20 25 30

Glu Ala Ala Ser Lys Ile Gly Ala Gly Pro Trp Val Val Lys Cys Gln
35 40 45

Val His Ala Gly Gly Arg Gly Lys Ala Gly Gly Val Lys Val Val Asn
50 55 60

Ser Lys Glu Asp Ile Arg Ala Phe Ala Glu Asn Trp Leu Gly Lys Arg
65 70 75 80

Leu Val Thr Tyr Gln Thr Asp Ala Asn Gly Gln Pro Val Asn Gln Ile
85 90 95

Leu Val Glu Ala Ala Thr Asp Ile Ala Lys Glu Leu Tyr Leu Gly Ala
100 105 110

Val Val Asp Arg Ser Ser Arg Arg Val Val Phe Met Ala Ser Thr Glu
115 120 125

Gly Gly Val Glu Ile Glu Lys Val Ala Glu Glu Thr Pro His Leu Ile
130 135 140

His Lys Val Ala Leu Asp Pro Leu Thr Gly Pro Met Pro Tyr Gln Gly
145 150 155 160

Arg Glu Leu Ala Phe Lys Leu Gly Leu Glu Gly Lys Leu Val Gln Gln
165 170 175

Phe Thr Lys Ile Phe Met Gly Leu Ala Thr Ile Phe Leu Glu Arg Asp
180 185 190

Leu Ala Leu Ile Glu Ile Asn Pro Leu Val Ile Thr Lys Gln Gly Asp
195 200 205

Leu Ile Cys Leu Asp Gly Lys Leu Gly Ala Asp Gly Asn Ala Leu Phe
210 215 220

Arg Gln Pro Asp Leu Arg Glu Met Arg Asp Gln Ser Gln Glu Asp Pro
225 230 235 240

Arg Glu Ala Gln Ala Ala Gln Trp Glu Leu Asn Tyr Val Ala Leu Asp
245 250 255

Gly Asn Ile Gly Cys Met Val Asn Gly Ala Gly Leu Ala Met Gly Thr
260 265 270

Met Asp Ile Val Lys Leu His Gly Gly Glu Pro Ala Asn Phe Leu Asp
275 280 285

Val Gly Gly Gly Ala Thr Lys Glu Arg Val Thr Glu Ala Phe Lys Ile
290 295 300

Ile Leu Ser Asp Asp Lys Val Lys Ala Val Leu Val Asn Ile Phe Gly
305 310 315 320

Gly Ile Val Arg Cys Asp Leu Ile Ala Asp Gly Ile Ile Gly Ala Val
325 330 335

Ala Glu Val Gly Val Asn Val Pro Val Val Val Arg Leu Glu Gly Asn
340 345 350

Asn Ala Glu Leu Gly Ala Lys Lys Leu Ala Asp Ser Gly Leu Asn Ile
355 360 365

Ile Ala Ala Lys Gly Leu Thr Asp Ala Ala Gln Gln Val Val Ala Ala
370 375 380

Val Glu Gly Lys
385

<210> 151
<211> 870
<212> DNA
<213> Escherichia coli

<400> 151

atgtccattt taatcgataa aaacaccaag gttatctgcc agggctttac cggtagccag 60

gggactttcc actcagaaca ggccattgca tacggcacta aaatggttgg cggcgtaacc 120

ccaggtaaag gcggcaccac ccacctcggc ctgccggtgt tcaacaccgt gcgtgaagcc 180

gttgctgcca ctggcgctac cgcttctgtt atctacgtac cagcaccgtt ctgcaaagac 240

tccattctgg aagccatcga cgcaggcatc aaactgatta tcaccatcac tgaaggcatc 300

ccgacgctgg atatgctgac cgtgaaagtg aagctggatg aagcaggcgt tcgtatgatc 360

ggcccgaact gcccaggcgt tatcactccg ggtgaatgca aaatcggtat ccagcctggt 420

cacattcaca aaccgggtaa agtgggtatc gtttcccgtt ccggtacact gacctatgaa 480

gcggttaaac agaccacgga ttacggtttc ggtcagtcga cctgtgtcgg tatcggcggt 540

gacccgatcc cgggctctaa ctttatcgac attctcgaaa tgttcgaaaa agatccgcag 600

accgaagcga tcgtgatgat cggtgagatc ggcggtagcg ctgaagaaga agcagctgcg 660

tacatcaaag agcacgttac caagccagtt gtgggttaca tcgctggtgt gactgcgccg 720

aaaggcaaac gtatgggcca cgcgggtgcc atcattgccg gtgggaaagg gactgcggat 780

gagaaattcg ctgctctgga agccgcaggc gtgaaaaccg ttcgcagcct ggcggatatc 840

ggtgaagcac tgaaaactgt tctgaaataa 870

<210> 152
<211> 289
<212> PRT
<213> Escherichia coli

<400> 152

Met Ser Ile Leu Ile Asp Lys Asn Thr Lys Val Ile Cys Gln Gly Phe
1 5 10 15

Thr Gly Ser Gln Gly Thr Phe His Ser Glu Gln Ala Ile Ala Tyr Gly
20 25 30

Thr Lys Met Val Gly Gly Val Thr Pro Gly Lys Gly Gly Thr Thr His
35 40 45

Leu Gly Leu Pro Val Phe Asn Thr Val Arg Glu Ala Val Ala Ala Thr
50 55 60

Gly Ala Thr Ala Ser Val Ile Tyr Val Pro Ala Pro Phe Cys Lys Asp
65 70 75 80

Ser Ile Leu Glu Ala Ile Asp Ala Gly Ile Lys Leu Ile Ile Thr Ile
85 90 95

Thr Glu Gly Ile Pro Thr Leu Asp Met Leu Thr Val Lys Val Lys Leu

100 105 110

Asp Glu Ala Gly Val Arg Met Ile Gly Pro Asn Cys Pro Gly Val Ile
115 120 125

Thr Pro Gly Glu Cys Lys Ile Gly Ile Gln Pro Gly His Ile His Lys
130 135 140

Pro Gly Lys Val Gly Ile Val Ser Arg Ser Gly Thr Leu Thr Tyr Glu
145 150 155 160

Ala Val Lys Gln Thr Thr Asp Tyr Gly Phe Gly Gln Ser Thr Cys Val
165 170 175

Gly Ile Gly Gly Asp Pro Ile Pro Gly Ser Asn Phe Ile Asp Ile Leu
180 185 190

Glu Met Phe Glu Lys Asp Pro Gln Thr Glu Ala Ile Val Met Ile Gly
195 200 205

Glu Ile Gly Gly Ser Ala Glu Glu Glu Ala Ala Ala Tyr Ile Lys Glu
210 215 220

His Val Thr Lys Pro Val Val Gly Tyr Ile Ala Gly Val Thr Ala Pro
225 230 235 240

Lys Gly Lys Arg Met Gly His Ala Gly Ala Ile Ile Ala Gly Gly Lys
245 250 255

Gly Thr Ala Asp Glu Lys Phe Ala Ala Leu Glu Ala Ala Gly Val Lys
260 265 270

Thr Val Arg Ser Leu Ala Asp Ile Gly Glu Ala Leu Lys Thr Val Leu
275 280 285

Lys

<210> 153
<211> 1719
<212> DNA
<213> Escherichia coli

<400> 153
atgaaacaaa cggttgcagc ttatatcgcc aaaacactcg aatcggcagg ggtgaaacgc 60

atctggggag tcacaggcga ctctctgaac ggtcttagtg acagtcttaa tcgcatgggc 120

accatcgagt ggatgtccac ccgccacgaa gaagtggcgg cctttgccgc tggcgctgaa 180

gcacaactta gcggagaact ggcggtctgc gccggatcgt gcggccccgg caacctgcac 240

ttaatcaacg gcctgttcga ttgccaccgc aatcacgttc cggtactggc gattgccgct 300

catattccct ccagcgaaat tggcagcggc tatttccagg aaacccaccc acaagagcta 360

ttccgcgaat gtagtcacta ttgcgagctg gtttccagcc cggagcagat cccacaagta 420

ctggcgattg ccatgcgcaa agcggtgctt aaccgtggcg tttcggttgt cgtgttacca 480

ggcgacgtgg cgttaaaacc tgcgccagaa ggggcaacca tgcactggta tcatgcgcca 540

caaccagtcg tgacgccgga agaagaagag ttacgcaaac tggcgcaact gctgcgttat 600

tccagcaata tcgccctgat gtgtggcagc ggctgcgcgg gggcgcataa agagttagtt 660

gagtttgccg ggaaaattaa agcgcctatt gttcatgccc tgcgcggtaa agaacatgtc 720

gaatacgata atccgtatga tgttggaatg accgggttaa tcggcttctc gtcaggtttc 780

cataccatga tgaacgccga cacgttagtg ctactcggca cgcaatttcc ctaccgcgcc 840

ttctacccga ccgatgccaa aatcattcag attgatatca acccagccag catcggcgct 900

cacagcaagg tggatatggc actggtcggc gatatcaagt cgactctgcg tgcattgctt 960

ccattggtgg aagaaaaagc cgatcgcaag tttctggata aagcgctgga agattaccgc 1020

gacgcccgca aagggctgga cgatttagct aaaccgagcg agaaagccat tcacccgcaa 1080

tatctggcgc agcaaattag tcattttgcc gccgatgacg ctattttcac ctgtgacgtt 1140

ggtacgccaa cggtgtgggc ggcacgttat ctaaaaatga acggcaagcg tcgcctgtta 1200

ggttcgttta accacggttc gatggctaac gccatgccgc aggcgctggg tgcgcaggcg 1260

acagagccag aacgtcaggt ggtcgccatg tgcggcgatg gcggttttag catgttgatg 1320

ggcgatttcc tctcagtagt gcagatgaaa ctgccagtga aaattgtcgt ctttaacaac 1380

agcgtgctgg gctttgtggc gatggagatg aaagctggtg gctatttgac tgacggcacc 1440

gaactacacg acacaaactt tgcccgcatt gccgaagcgt gcggcattac gggtatccgt 1500

gtagaaaaag cgtctgaagt tgatgaagcc ctgcaacgcg ccttctccat cgacggtccg 1560

gtgttggtgg atgtggtggt cgccaaagaa gagttagcca ttccaccgca gatcaaactc 1620

gaacaggcca aaggtttcag cctgtatatg ctgcgcgcaa tcatcagcgg acgcggtgat 1680

gaagtgatcg aactggcgaa aacaaactgg ctaaggtaa 1719

<210> 154
<211> 572
<212> PRT
<213> Escherichia coli

<400> 154

Met Lys Gln Thr Val Ala Ala Tyr Ile Ala Lys Thr Leu Glu Ser Ala
1 5 10 15

Gly Val Lys Arg Ile Trp Gly Val Thr Gly Asp Ser Leu Asn Gly Leu
20 25 30

Ser Asp Ser Leu Asn Arg Met Gly Thr Ile Glu Trp Met Ser Thr Arg
35 40 45

His Glu Glu Val Ala Ala Phe Ala Ala Gly Ala Glu Ala Gln Leu Ser
50 55 60

Gly Glu Leu Ala Val Cys Ala Gly Ser Cys Gly Pro Gly Asn Leu His
65 70 75 80

Leu Ile Asn Gly Leu Phe Asp Cys His Arg Asn His Val Pro Val Leu
85 90 95

Ala Ile Ala Ala His Ile Pro Ser Ser Glu Ile Gly Ser Gly Tyr Phe
100 105 110

Gln Glu Thr His Pro Gln Glu Leu Phe Arg Glu Cys Ser His Tyr Cys
115 120 125

Glu Leu Val Ser Ser Pro Glu Gln Ile Pro Gln Val Leu Ala Ile Ala
130 135 140

Met Arg Lys Ala Val Leu Asn Arg Gly Val Ser Val Val Val Leu Pro
145 150 155 160

Gly Asp Val Ala Leu Lys Pro Ala Pro Glu Gly Ala Thr Met His Trp
165 170 175

Tyr His Ala Pro Gln Pro Val Val Thr Pro Glu Glu Glu Glu Leu Arg
180 185 190

Lys Leu Ala Gln Leu Leu Arg Tyr Ser Ser Asn Ile Ala Leu Met Cys
195 200 205

Gly Ser Gly Cys Ala Gly Ala His Lys Glu Leu Val Glu Phe Ala Gly
210 215 220

Lys Ile Lys Ala Pro Ile Val His Ala Leu Arg Gly Lys Glu His Val
225 230 235 240

Glu Tyr Asp Asn Pro Tyr Asp Val Gly Met Thr Gly Leu Ile Gly Phe
245 250 255

Ser Ser Gly Phe His Thr Met Met Asn Ala Asp Thr Leu Val Leu Leu
260 265 270

Gly Thr Gln Phe Pro Tyr Arg Ala Phe Tyr Pro Thr Asp Ala Lys Ile
275 280 285

Ile Gln Ile Asp Ile Asn Pro Ala Ser Ile Gly Ala His Ser Lys Val
290 295 300

Asp Met Ala Leu Val Gly Asp Ile Lys Ser Thr Leu Arg Ala Leu Leu
305 310 315 320

Pro Leu Val Glu Glu Lys Ala Asp Arg Lys Phe Leu Asp Lys Ala Leu
325 330 335

Glu Asp Tyr Arg Asp Ala Arg Lys Gly Leu Asp Asp Leu Ala Lys Pro
340 345 350

Ser Glu Lys Ala Ile His Pro Gln Tyr Leu Ala Gln Gln Ile Ser His
355 360 365

Phe Ala Ala Asp Asp Ala Ile Phe Thr Cys Asp Val Gly Thr Pro Thr
370 375 380

Val Trp Ala Ala Arg Tyr Leu Lys Met Asn Gly Lys Arg Arg Leu Leu
385 390 395 400

Gly Ser Phe Asn His Gly Ser Met Ala Asn Ala Met Pro Gln Ala Leu
405 410 415

Gly Ala Gln Ala Thr Glu Pro Glu Arg Gln Val Val Ala Met Cys Gly
420 425 430

Asp Gly Gly Phe Ser Met Leu Met Gly Asp Phe Leu Ser Val Val Gln
435 440 445

Met Lys Leu Pro Val Lys Ile Val Val Phe Asn Asn Ser Val Leu Gly
450 455 460

Phe Val Ala Met Glu Met Lys Ala Gly Gly Tyr Leu Thr Asp Gly Thr
465 470 475 480

Glu Leu His Asp Thr Asn Phe Ala Arg Ile Ala Glu Ala Cys Gly Ile
485 490 495

Thr Gly Ile Arg Val Glu Lys Ala Ser Glu Val Asp Glu Ala Leu Gln
500 505 510

Arg Ala Phe Ser Ile Asp Gly Pro Val Leu Val Asp Val Val Val Ala

```
        515                 520                 525

Lys Glu Glu Leu Ala Ile Pro Pro Gln Ile Lys Leu Glu Gln Ala Lys
    530                 535                 540

Gly Phe Ser Leu Tyr Met Leu Arg Ala Ile Ile Ser Gly Arg Gly Asp
545                 550                 555                 560

Glu Val Ile Glu Leu Ala Lys Thr Asn Trp Leu Arg
                565                 570


<210>  155
<211>  1203
<212>  DNA
<213>  Escherichia coli

<400>  155
atgtcgagta agttagtact ggttctgaac tgcggtagtt cttcactgaa atttgccatc      60

atcgatgcag taaatggtga agagtacctt tctggtttag ccgaatgttt ccacctgccc     120

gaagcacgta tcaaatggaa aatggacggc aataaacagg aagcggcttt aggtgcaggc     180

gccgctcaca gcgaagcgct caactttatc gttaatacta ttctggcaca aaaaccagaa     240

ctgtctgcgc agctgactgc tatcggtcac cgtatcgtac acggcggcga aaagtatacc     300

agctccgtag tgatcgatga gtctgttatt cagggtatca aagatgcagc ttcttttgca     360

ccgctgcaca acccggctca cctgatcggt atcgaagaag ctctgaaatc tttcccacag     420

ctgaaagaca aaaacgttgc tgtatttgac accgcgttcc accagactat gccggaagag     480

tcttacctct acgccctgcc ttacaacctg tacaaagagc acggcatccg tcgttacggc     540

gcgcacggca ccagccactt ctatgtaacc caggaagcgg caaaaatgct gaacaaaccg     600

gtagaagaac tgaacatcat cacctgccac ctgggcaacg gtggttccgt ttctgctatc     660

cgcaacggta aatgcgttga cacctctatg ggcctgaccc cgctggaagg tctggtcatg     720

ggtacccgtt ctggtgatat cgatccggcg atcatcttcc acctgcacga caccctgggc     780

atgagcgttg acgcaatcaa caaactgctg accaaagagt ctggcctgct gggtctgacc     840

gaagtgacca gcgactgccg ctatgttgaa gacaactacg cgacgaaaga agacgcgaag     900

cgcgcaatgg acgtttactg ccaccgcctg gcgaaataca tcggtgccta cactgcgctg     960

atggatggtc gtctggacgc tgttgtattc actggtggta tcggtgaaaa tgccgcaatg    1020

gttcgtgaac tgtctctggg caaactgggc gtgctgggct ttgaagttga tcatgaacgc    1080

aacctggctg cacgtttcgg caaatctggt ttcatcaaca aagaaggtac ccgtcctgcg    1140

gtggttatcc caaccaacga agaactggtt atcgcgcaag acgcgagccg cctgactgcc    1200

tga                                                                  1203
```

<210> 156
<211> 400
<212> PRT
<213> Escherichia coli

<400> 156

Met Ser Ser Lys Leu Val Leu Val Leu Asn Cys Gly Ser Ser Ser Leu
1 5 10 15

Lys Phe Ala Ile Ile Asp Ala Val Asn Gly Glu Glu Tyr Leu Ser Gly
20 25 30

Leu Ala Glu Cys Phe His Leu Pro Glu Ala Arg Ile Lys Trp Lys Met
35 40 45

Asp Gly Asn Lys Gln Glu Ala Ala Leu Gly Ala Gly Ala Ala His Ser
50 55 60

Glu Ala Leu Asn Phe Ile Val Asn Thr Ile Leu Ala Gln Lys Pro Glu
65 70 75 80

Leu Ser Ala Gln Leu Thr Ala Ile Gly His Arg Ile Val His Gly Gly
85 90 95

Glu Lys Tyr Thr Ser Ser Val Val Ile Asp Glu Ser Val Ile Gln Gly
100 105 110

Ile Lys Asp Ala Ala Ser Phe Ala Pro Leu His Asn Pro Ala His Leu
115 120 125

Ile Gly Ile Glu Glu Ala Leu Lys Ser Phe Pro Gln Leu Lys Asp Lys
130 135 140

Asn Val Ala Val Phe Asp Thr Ala Phe His Gln Thr Met Pro Glu Glu
145 150 155 160

Ser Tyr Leu Tyr Ala Leu Pro Tyr Asn Leu Tyr Lys Glu His Gly Ile
165 170 175

Arg Arg Tyr Gly Ala His Gly Thr Ser His Phe Tyr Val Thr Gln Glu
180 185 190

Ala Ala Lys Met Leu Asn Lys Pro Val Glu Glu Leu Asn Ile Ile Thr
195 200 205

Cys His Leu Gly Asn Gly Gly Ser Val Ser Ala Ile Arg Asn Gly Lys
210 215 220

Cys Val Asp Thr Ser Met Gly Leu Thr Pro Leu Glu Gly Leu Val Met
225 230 235 240

Gly Thr Arg Ser Gly Asp Ile Asp Pro Ala Ile Ile Phe His Leu His
245 250 255

Asp Thr Leu Gly Met Ser Val Asp Ala Ile Asn Lys Leu Leu Thr Lys
260 265 270

Glu Ser Gly Leu Leu Gly Leu Thr Glu Val Thr Ser Asp Cys Arg Tyr
275 280 285

Val Glu Asp Asn Tyr Ala Thr Lys Glu Asp Ala Lys Arg Ala Met Asp
290 295 300

Val Tyr Cys His Arg Leu Ala Lys Tyr Ile Gly Ala Tyr Thr Ala Leu
305 310 315 320

Met Asp Gly Arg Leu Asp Ala Val Val Phe Thr Gly Gly Ile Gly Glu
325 330 335

Asn Ala Ala Met Val Arg Glu Leu Ser Leu Gly Lys Leu Gly Val Leu
340 345 350

Gly Phe Glu Val Asp His Glu Arg Asn Leu Ala Ala Arg Phe Gly Lys
355 360 365

Ser Gly Phe Ile Asn Lys Glu Gly Thr Arg Pro Ala Val Val Ile Pro
370 375 380

Thr Asn Glu Glu Leu Val Ile Ala Gln Asp Ala Ser Arg Leu Thr Ala
385 390 395 400

<210> 157
<211> 2145
<212> DNA
<213> Escherichia coli

<400> 157
gtgtcccgta ttattatgct gatccctacc ggaaccagcg tcggtctgac cagcgtcagc 60

cttggcgtga tccgtgcaat ggaacgcaaa ggcgttcgtc tgagcgtttt caaacctatc 120

gctcagccgc gtaccggtgg cgatgcgccc gatcagacta cgactatcgt gcgtgcgaac 180

tcttccacca cgacggccgc tgaaccgctg aaaatgagct acgttgaagg tctgctttcc 240

agcaatcaga aagatgtgct gatggaagag atcgtcgcaa actaccacgc taacaccaaa 300

gacgctgaag tcgttctggt tgaaggtctg gtcccgacac gtaagcacca gtttgcccag 360

tctctgaact acgaaatcgc taaaacgctg aatgcggaaa tcgtcttcgt tatgtctcag 420

ggcactgaca ccccggaaca gctgaaagag cgtatcgaac tgacccgcaa cagcttcggc 480

ggtgccaaaa acaccaacat caccggcgtt atcgttaaca aactgaacgc accggttgat 540

gaacagggtc gtactcgccc ggatctgtcc gagattttcg acgactcttc caaagctaaa 600

gtaaacaatg ttgatccggc gaagctgcaa gaatccagcc cgctgccggt tctcggcgct 660

gtgccgtgga gctttgacct gatcgcgact cgtgcgatcg atatggctcg ccacctgaat 720

gcgaccatca tcaacgaagg cgacatcaat actcgccgcg ttaaatccgt cactttctgc 780

gcacgcagca ttccgcacat gctggagcac ttccgtgccg gttctctgct ggtgacttcc 840

gcagaccgtc ctgacgtgct ggtggccgct tgcctggcag ccatgaacgg cgtagaaatc 900

ggtgccctgc tgctgactgg cggttacgaa atggacgcgc gcatttctaa actgtgcgaa 960

cgtgctttcg ctaccggcct gccggtattt atggtgaaca ccaacacctg gcagacctct 1020

ctgagcctgc agagcttcaa cctggaagtt ccggttgacg atcacgaacg tatcgagaaa 1080

gttcaggaat acgttgctaa ctacatcaac gctgactgga tcgaatctct gactgccact 1140

tctgagcgca gccgtcgtct gtctccgcct gcgttccgtt atcagctgac tgaacttgcg 1200

cgcaaagcgg gcaaacgtat cgtactgccg gaaggtgacg aaccgcgtac cgttaaagca 1260

gccgctatct gtgctgaacg tggtatcgca acttgcgtac tgctgggtaa tccggcagag 1320

atcaaccgtg ttgcagcgtc tcagggtgta gaactgggtg cagggattga aatcgttgat 1380

ccagaagtgg ttcgcgaaag ctatgttggt cgtctggtcg aactgcgtaa gaacaaaggc 1440

atgaccgaaa ccgttgcccg cgaacagctg gaagacaacg tggtgctcgg tacgctgatg 1500

ctggaacagg atgaagttga tggtctggtt tccggtgctg ttcacactac cgcaaacacc 1560

atccgtccgc cgctgcagct gatcaaaact gcaccgggca gctccctggt atcttccgtg 1620

ttcttcatgc tgctgccgga acaggtttac gtttacggtg actgtgcgat caacccggat 1680

ccgaccgctg aacagctggc agaaatcgcg attcagtccg ctgattccgc tgcggccttc 1740

ggtatcgaac cgcgcgttgc tatgctctcc tactccaccg gtacttctgg tgcaggtagc 1800

gacgtagaaa aagttcgcga agcaactcgt ctggcgcagg aaaaacgtcc tgacctgatg 1860

atcgacggtc cgctgcagta cgacgctgcg gtaatggctg acgttgcgaa atccaaagcg 1920

ccgaactctc cggttgcagg tcgcgctacc gtgttcatct tcccggatct gaacaccggt 1980

aacaccacct acaaagcggt acagcgttct gccgacctga tctccatcgg gccgatgctg 2040

cagggtatgc gcaagccggt taacgacctg tcccgtggcg cactggttga cgatatcgtc 2100

tacaccatcg cgctgactgc gattcagtct gcacagcagc agtaa 2145

<210> 158
<211> 714

<212> PRT
<213> Escherichia coli

<400> 158

Met Ser Arg Ile Ile Met Leu Ile Pro Thr Gly Thr Ser Val Gly Leu
1               5                   10                  15

Thr Ser Val Ser Leu Gly Val Ile Arg Ala Met Glu Arg Lys Gly Val
            20                  25                  30

Arg Leu Ser Val Phe Lys Pro Ile Ala Gln Pro Arg Thr Gly Gly Asp
        35                  40                  45

Ala Pro Asp Gln Thr Thr Thr Ile Val Arg Ala Asn Ser Ser Thr Thr
    50                  55                  60

Thr Ala Ala Glu Pro Leu Lys Met Ser Tyr Val Glu Gly Leu Leu Ser
65                  70                  75                  80

Ser Asn Gln Lys Asp Val Leu Met Glu Glu Ile Val Ala Asn Tyr His
                85                  90                  95

Ala Asn Thr Lys Asp Ala Glu Val Val Leu Val Glu Gly Leu Val Pro
            100                 105                 110

Thr Arg Lys His Gln Phe Ala Gln Ser Leu Asn Tyr Glu Ile Ala Lys
        115                 120                 125

Thr Leu Asn Ala Glu Ile Val Phe Val Met Ser Gln Gly Thr Asp Thr
    130                 135                 140

Pro Glu Gln Leu Lys Glu Arg Ile Glu Leu Thr Arg Asn Ser Phe Gly
145                 150                 155                 160

Gly Ala Lys Asn Thr Asn Ile Thr Gly Val Ile Val Asn Lys Leu Asn
                165                 170                 175

Ala Pro Val Asp Glu Gln Gly Arg Thr Arg Pro Asp Leu Ser Glu Ile
            180                 185                 190

Phe Asp Asp Ser Ser Lys Ala Lys Val Asn Asn Val Asp Pro Ala Lys
        195                 200                 205

Leu Gln Glu Ser Ser Pro Leu Pro Val Leu Gly Ala Val Pro Trp Ser
    210                 215                 220

Phe Asp Leu Ile Ala Thr Arg Ala Ile Asp Met Ala Arg His Leu Asn
225                 230                 235                 240

Ala Thr Ile Ile Asn Glu Gly Asp Ile Asn Thr Arg Arg Val Lys Ser
245 250 255

Val Thr Phe Cys Ala Arg Ser Ile Pro His Met Leu Glu His Phe Arg
260 265 270

Ala Gly Ser Leu Leu Val Thr Ser Ala Asp Arg Pro Asp Val Leu Val
275 280 285

Ala Ala Cys Leu Ala Ala Met Asn Gly Val Glu Ile Gly Ala Leu Leu
290 295 300

Leu Thr Gly Gly Tyr Glu Met Asp Ala Arg Ile Ser Lys Leu Cys Glu
305 310 315 320

Arg Ala Phe Ala Thr Gly Leu Pro Val Phe Met Val Asn Thr Asn Thr
325 330 335

Trp Gln Thr Ser Leu Ser Leu Gln Ser Phe Asn Leu Glu Val Pro Val
340 345 350

Asp Asp His Glu Arg Ile Glu Lys Val Gln Glu Tyr Val Ala Asn Tyr
355 360 365

Ile Asn Ala Asp Trp Ile Glu Ser Leu Thr Ala Thr Ser Glu Arg Ser
370 375 380

Arg Arg Leu Ser Pro Pro Ala Phe Arg Tyr Gln Leu Thr Glu Leu Ala
385 390 395 400

Arg Lys Ala Gly Lys Arg Ile Val Leu Pro Glu Gly Asp Glu Pro Arg
405 410 415

Thr Val Lys Ala Ala Ala Ile Cys Ala Glu Arg Gly Ile Ala Thr Cys
420 425 430

Val Leu Leu Gly Asn Pro Ala Glu Ile Asn Arg Val Ala Ala Ser Gln
435 440 445

Gly Val Glu Leu Gly Ala Gly Ile Glu Ile Val Asp Pro Glu Val Val
450 455 460

Arg Glu Ser Tyr Val Gly Arg Leu Val Glu Leu Arg Lys Asn Lys Gly
465 470 475 480

Met Thr Glu Thr Val Ala Arg Glu Gln Leu Glu Asp Asn Val Val Leu

485 490 495

Gly Thr Leu Met Leu Glu Gln Asp Glu Val Asp Gly Leu Val Ser Gly
500 505 510

Ala Val His Thr Thr Ala Asn Thr Ile Arg Pro Pro Leu Gln Leu Ile
515 520 525

Lys Thr Ala Pro Gly Ser Ser Leu Val Ser Ser Val Phe Phe Met Leu
530 535 540

Leu Pro Glu Gln Val Tyr Val Tyr Gly Asp Cys Ala Ile Asn Pro Asp
545 550 555 560

Pro Thr Ala Glu Gln Leu Ala Glu Ile Ala Ile Gln Ser Ala Asp Ser
565 570 575

Ala Ala Ala Phe Gly Ile Glu Pro Arg Val Ala Met Leu Ser Tyr Ser
580 585 590

Thr Gly Thr Ser Gly Ala Gly Ser Asp Val Glu Lys Val Arg Glu Ala
595 600 605

Thr Arg Leu Ala Gln Glu Lys Arg Pro Asp Leu Met Ile Asp Gly Pro
610 615 620

Leu Gln Tyr Asp Ala Ala Val Met Ala Asp Val Ala Lys Ser Lys Ala
625 630 635 640

Pro Asn Ser Pro Val Ala Gly Arg Ala Thr Val Phe Ile Phe Pro Asp
645 650 655

Leu Asn Thr Gly Asn Thr Thr Tyr Lys Ala Val Gln Arg Ser Ala Asp
660 665 670

Leu Ile Ser Ile Gly Pro Met Leu Gln Gly Met Arg Lys Pro Val Asn
675 680 685

Asp Leu Ser Arg Gly Ala Leu Val Asp Asp Ile Val Tyr Thr Ile Ala
690 695 700

Leu Thr Ala Ile Gln Ser Ala Gln Gln Gln
705 710

<210> 159
<211> 2664
<212> DNA
<213> Escherichia coli

<400> 159
atgtcagaac gtttcccaaa tgacgtggat ccgatcgaaa ctcgcgactg gctccaggcg 60

atcgaatcgg tcatccgtga agaaggtgtt gagcgtgctc agtatctgat cgaccaactg 120

cttgctgaag cccgcaaagg cggtgtaaac gtagccgcag gcacaggtat cagcaactac 180

atcaacacca tccccgttga agaacaaccg gagtatccgg gtaatctgga actggaacgc 240

cgtattcgtt cagctatccg ctggaacgcc atcatgacgg tgctgcgtgc gtcgaaaaaa 300

gacctcgaac tgggcggcca tatggcgtcc ttccagtctt ccgcaaccat ttatgatgtg 360

tgctttaacc acttcttccg tgcacgcaac gagcaggatg gcggcgacct ggtttacttc 420

cagggccaca tctccccggg cgtgtacgct cgtgctttcc tggaaggtcg tctgactcag 480

gagcagctgg ataacttccg tcaggaagtt cacggcaatg gcctctcttc ctatccgcac 540

ccgaaactga tgccggaatt ctggcagttc ccgaccgtat ctatgggtct gggtccgatt 600

ggtgctattt accaggctaa attcctgaaa tatctggaac accgtggcct gaaagatacc 660

tctaaacaaa ccgtttacgc gttcctcggt gacggtgaaa tggacgaacc ggaatccaaa 720

ggtgcgatca ccatcgctac ccgtgaaaaa ctggataacc tggtcttcgt tatcaactgt 780

aacctgcagc gtcttgacgg cccggtcacc ggtaacggca agatcatcaa cgaactggaa 840

ggcatcttcg aaggtgctgg ctggaacgtg atcaaagtga tgtggggtag ccgttgggat 900

gaactgctgc gtaaggatac cagcggtaaa ctgatccagc tgatgaacga aaccgttgac 960

ggcgactacc agaccttcaa atcgaaagat ggtgcgtacg ttcgtgaaca cttcttcggt 1020

aaatatcctg aaaccgcagc actggttgca gactggactg acgagcagat ctgggcactg 1080

aaccgtggtg gtcacgatcc gaagaaaatc tacgctgcat tcaagaaagc gcaggaaacc 1140

aaaggcaaag cgacagtaat ccttgctcat accattaaag gttacggcat gggcgacgcg 1200

gctgaaggta aaaacatcgc gcaccaggtt aagaaaatga acatggacgg tgtgcgtcat 1260

atccgcgacc gtttcaatgt gccggtgtct gatgcagata tcgaaaaact gccgtacatc 1320

accttcccgg aaggttctga agagcatacc tatctgcacg ctcagcgtca gaaactgcac 1380

ggttatctgc caagccgtca gccgaacttc accgagaagc ttgagctgcc gagcctgcaa 1440

gacttcggcg cgctgttgga agagcagagc aaagagatct ctaccactat cgctttcgtt 1500

cgtgctctga acgtgatgct gaagaacaag tcgatcaaag atcgtctggt accgatcatc 1560

gccgacgaag cgcgtacttt cggtatggaa ggtctgttcc gtcagattgg tatttacagc 1620

ccgaacggtc agcagtacac cccgcaggac cgcgagcagg ttgcttacta taaagaagac 1680

gagaaaggtc agattctgca ggaagggatc aacgagctgg gcgcaggttg ttcctggctg 1740

gcagcggcga cctcttacag caccaacaat ctgccgatga tcccgttcta catctattac 1800

tcgatgttcg gcttccagcg tattggcgat ctgtgctggg cggctggcga ccagcaagcg 1860

cgtggcttcc tgatcggcgg tacttccggt cgtaccaccc tgaacggcga aggtctgcag 1920

cacgaagatg gtcacagcca cattcagtcg ctgactatcc cgaactgtat ctcttacgac 1980

ccggcttacg cttacgaagt tgctgtcatc atgcatgacg gtctggagcg tatgtacggt 2040

gaaaaacaag agaacgttta ctactacatc actacgctga acgaaaacta ccacatgccg 2100

gcaatgccgg aaggtgctga ggaaggtatc cgtaaaggta tctacaaact cgaaactatt 2160

gaaggtagca aaggtaaagt tcagctgctc ggctccggtt ctatcctgcg tcacgtccgt 2220

gaagcagctg agatcctggc gaaagattac ggcgtaggtt ctgacgttta tagcgtgacc 2280

tccttcaccg agctggcgcg tgatggtcag gattgtgaac gctggaacat gctgcacccg 2340

ctggaaactc cgcgcgttcc gtatatcgct caggtgatga acgacgctcc ggcagtggca 2400

tctaccgact atatgaaact gttcgctgag caggtccgta cttacgtacc ggctgacgac 2460

taccgcgtac tgggtactga tggcttcggt cgttccgaca gccgtgagaa cctgcgtcac 2520

cacttcgaag ttgatgcttc ttatgtcgtg gttgcggcgc tgggcgaact ggctaaacgt 2580

ggcgaaatcg ataagaaagt ggttgctgac gcaatcgcca aattcaacat cgatgcagat 2640

aaagttaacc cgcgtctggc gtaa 2664

<210> 160
<211> 887
<212> PRT
<213> Escherichia coli

<400> 160

Met Ser Glu Arg Phe Pro Asn Asp Val Asp Pro Ile Glu Thr Arg Asp
1 5 10 15

Trp Leu Gln Ala Ile Glu Ser Val Ile Arg Glu Glu Gly Val Glu Arg
20 25 30

Ala Gln Tyr Leu Ile Asp Gln Leu Leu Ala Glu Ala Arg Lys Gly Gly
35 40 45

Val Asn Val Ala Ala Gly Thr Gly Ile Ser Asn Tyr Ile Asn Thr Ile
50 55 60

Pro Val Glu Glu Gln Pro Glu Tyr Pro Gly Asn Leu Glu Leu Glu Arg
65 70 75 80

Arg Ile Arg Ser Ala Ile Arg Trp Asn Ala Ile Met Thr Val Leu Arg
85 90 95

Ala Ser Lys Lys Asp Leu Glu Leu Gly Gly His Met Ala Ser Phe Gln
100 105 110

Ser Ser Ala Thr Ile Tyr Asp Val Cys Phe Asn His Phe Phe Arg Ala
115 120 125

Arg Asn Glu Gln Asp Gly Gly Asp Leu Val Tyr Phe Gln Gly His Ile
130 135 140

Ser Pro Gly Val Tyr Ala Arg Ala Phe Leu Glu Gly Arg Leu Thr Gln
145 150 155 160

Glu Gln Leu Asp Asn Phe Arg Gln Glu Val His Gly Asn Gly Leu Ser
165 170 175

Ser Tyr Pro His Pro Lys Leu Met Pro Glu Phe Trp Gln Phe Pro Thr
180 185 190

Val Ser Met Gly Leu Gly Pro Ile Gly Ala Ile Tyr Gln Ala Lys Phe
195 200 205

Leu Lys Tyr Leu Glu His Arg Gly Leu Lys Asp Thr Ser Lys Gln Thr
210 215 220

Val Tyr Ala Phe Leu Gly Asp Gly Glu Met Asp Glu Pro Glu Ser Lys
225 230 235 240

Gly Ala Ile Thr Ile Ala Thr Arg Glu Lys Leu Asp Asn Leu Val Phe
245 250 255

Val Ile Asn Cys Asn Leu Gln Arg Leu Asp Gly Pro Val Thr Gly Asn
260 265 270

Gly Lys Ile Ile Asn Glu Leu Glu Gly Ile Phe Glu Gly Ala Gly Trp
275 280 285

Asn Val Ile Lys Val Met Trp Gly Ser Arg Trp Asp Glu Leu Leu Arg
290 295 300

Lys Asp Thr Ser Gly Lys Leu Ile Gln Leu Met Asn Glu Thr Val Asp
305 310 315 320

Gly Asp Tyr Gln Thr Phe Lys Ser Lys Asp Gly Ala Tyr Val Arg Glu
325 330 335

His Phe Phe Gly Lys Tyr Pro Glu Thr Ala Ala Leu Val Ala Asp Trp
340 345 350

Thr Asp Glu Gln Ile Trp Ala Leu Asn Arg Gly Gly His Asp Pro Lys

```
            355                 360                 365

Lys Ile Tyr Ala Ala Phe Lys Lys Ala Gln Glu Thr Lys Gly Lys Ala
        370                 375                 380

Thr Val Ile Leu Ala His Thr Ile Lys Gly Tyr Gly Met Gly Asp Ala
385                 390                 395                 400

Ala Glu Gly Lys Asn Ile Ala His Gln Val Lys Lys Met Asn Met Asp
                405                 410                 415

Gly Val Arg His Ile Arg Asp Arg Phe Asn Val Pro Val Ser Asp Ala
            420                 425                 430

Asp Ile Glu Lys Leu Pro Tyr Ile Thr Phe Pro Glu Gly Ser Glu Glu
        435                 440                 445

His Thr Tyr Leu His Ala Gln Arg Gln Lys Leu His Gly Tyr Leu Pro
    450                 455                 460

Ser Arg Gln Pro Asn Phe Thr Glu Lys Leu Glu Leu Pro Ser Leu Gln
465                 470                 475                 480

Asp Phe Gly Ala Leu Leu Glu Glu Gln Ser Lys Glu Ile Ser Thr Thr
                485                 490                 495

Ile Ala Phe Val Arg Ala Leu Asn Val Met Leu Lys Asn Lys Ser Ile
            500                 505                 510

Lys Asp Arg Leu Val Pro Ile Ile Ala Asp Glu Ala Arg Thr Phe Gly
        515                 520                 525

Met Glu Gly Leu Phe Arg Gln Ile Gly Ile Tyr Ser Pro Asn Gly Gln
    530                 535                 540

Gln Tyr Thr Pro Gln Asp Arg Glu Gln Val Ala Tyr Tyr Lys Glu Asp
545                 550                 555                 560

Glu Lys Gly Gln Ile Leu Gln Glu Gly Ile Asn Glu Leu Gly Ala Gly
                565                 570                 575

Cys Ser Trp Leu Ala Ala Ala Thr Ser Tyr Ser Thr Asn Asn Leu Pro
            580                 585                 590

Met Ile Pro Phe Tyr Ile Tyr Tyr Ser Met Phe Gly Phe Gln Arg Ile
        595                 600                 605
```

```
Gly Asp Leu Cys Trp Ala Ala Gly Asp Gln Gln Ala Arg Gly Phe Leu
    610                 615                 620

Ile Gly Gly Thr Ser Gly Arg Thr Thr Leu Asn Gly Glu Gly Leu Gln
625                 630                 635                 640

His Glu Asp Gly His Ser His Ile Gln Ser Leu Thr Ile Pro Asn Cys
                645                 650                 655

Ile Ser Tyr Asp Pro Ala Tyr Ala Tyr Glu Val Ala Val Ile Met His
            660                 665                 670

Asp Gly Leu Glu Arg Met Tyr Gly Glu Lys Gln Glu Asn Val Tyr Tyr
        675                 680                 685

Tyr Ile Thr Thr Leu Asn Glu Asn Tyr His Met Pro Ala Met Pro Glu
    690                 695                 700

Gly Ala Glu Glu Gly Ile Arg Lys Gly Ile Tyr Lys Leu Glu Thr Ile
705                 710                 715                 720

Glu Gly Ser Lys Gly Lys Val Gln Leu Leu Gly Ser Gly Ser Ile Leu
                725                 730                 735

Arg His Val Arg Glu Ala Ala Glu Ile Leu Ala Lys Asp Tyr Gly Val
            740                 745                 750

Gly Ser Asp Val Tyr Ser Val Thr Ser Phe Thr Glu Leu Ala Arg Asp
        755                 760                 765

Gly Gln Asp Cys Glu Arg Trp Asn Met Leu His Pro Leu Glu Thr Pro
    770                 775                 780

Arg Val Pro Tyr Ile Ala Gln Val Met Asn Asp Ala Pro Ala Val Ala
785                 790                 795                 800

Ser Thr Asp Tyr Met Lys Leu Phe Ala Glu Gln Val Arg Thr Tyr Val
                805                 810                 815

Pro Ala Asp Asp Tyr Arg Val Leu Gly Thr Asp Gly Phe Gly Arg Ser
            820                 825                 830

Asp Ser Arg Glu Asn Leu Arg His His Phe Glu Val Asp Ala Ser Tyr
        835                 840                 845

Val Val Val Ala Ala Leu Gly Glu Leu Ala Lys Arg Gly Glu Ile Asp
    850                 855                 860
```

Lys Lys Val Val Ala Asp Ala Ile Ala Lys Phe Asn Ile Asp Ala Asp
865                 870                 875                 880

Lys Val Asn Pro Arg Leu Ala
                885

<210> 161
<211> 1893
<212> DNA
<213> Escherichia coli

<400> 161
atggctatcg aaatcaaagt accggacatc ggggctgatg aagttgaaat caccgagatc 60

ctggtcaaag tgggcgacaa agttgaagcc gaacagtcgc tgatcaccgt agaaggcgac 120

aaagcctcta tggaagttcc gtctccgcag gcgggtatcg ttaaagagat caaagtctct 180

gttggcgata aaacccagac cggcgcactg attatgattt tcgattccgc cgacggtgca 240

gcagacgctg cacctgctca ggcagaagag aagaaagaag cagctccggc agcagcacca 300

gcggctgcgg cggcaaaaga cgttaacgtt ccggatatcg gcagcgacga agttgaagtg 360

accgaaatcc tggtgaaagt tggcgataaa gttgaagctg aacagtcgct gatcaccgta 420

gaaggcgaca aggcttctat ggaagttccg gctccgtttg ctggcaccgt gaaagagatc 480

aaagtgaacg tgggtgacaa agtgtctacc ggctcgctga ttatggtctt cgaagtcgcg 540

ggtgaagcag gcgcggcagc tccggccgct aaacaggaag cagctccggc agcggcccct 600

gcaccagcgg ctggcgtgaa agaagttaac gttccggata tcggcggtga cgaagttgaa 660

gtgactgaag tgatggtgaa agtgggcgac aaagttgccg ctgaacagtc actgatcacc 720

gtagaaggcg acaaagcttc tatggaagtt ccggcgccgt ttgcaggcgt cgtgaaggaa 780

ctgaaagtca acgttggcga taaagtgaaa actggctcgc tgattatgat cttcgaagtt 840

gaaggcgcag cgcctgcggc agctcctgcg aaacaggaag cggcagcgcc ggcaccggca 900

gcaaaagctg aagccccggc agcagcacca gctgcgaaag cggaaggcaa atctgaattt 960

gctgaaaacg acgcttatgt tcacgcgact ccgctgatcc gccgtctggc acgcgagttt 1020

ggtgttaacc ttgcgaaagt gaagggcact ggccgtaaag gtcgtatcct gcgcgaagac 1080

gttcaggctt acgtgaaaga agctatcaaa cgtgcagaag cagctccggc agcgactggc 1140

ggtggtatcc ctggcatgct gccgtggccg aaggtggact tcagcaagtt tggtgaaatc 1200

gaagaagtgg aactgggccg catccagaaa atctctggtg cgaacctgag ccgtaactgg 1260

gtaatgatcc cgcatgttac tcacttcgac aaaaccgata tcaccgagtt ggaagcgttc 1320

cgtaaacagc agaacgaaga agcggcgaaa cgtaagctgg atgtgaagat caccccggtt 1380

gtcttcatca tgaaagccgt tgctgcagct cttgagcaga tgcctcgctt caatagttcg 1440

ctgtcggaag acggtcagcg tctgaccctg aagaaataca tcaacatcgg tgtggcggtg 1500

gataccccga acggtctggt tgttccggta ttcaaagacg tcaacaagaa aggcatcatc 1560

gagctgtctc gcgagctgat gactatttct aagaaagcgc gtgacggtaa gctgactgcg 1620

ggcgaaatgc agggcggttg cttcaccatc tccagcatcg gcggcctggg tactacccac 1680

ttcgcgccga ttgtgaacgc gccggaagtg gctatcctcg gcgtttccaa gtccgcgatg 1740

gagccggtgt ggaatggtaa agagttcgtg ccgcgtctga tgctgccgat ttctctctcc 1800

ttcgaccacc gcgtgatcga cggtgctgat ggtgcccgtt tcattaccat cattaacaac 1860

acgctgtctg acattcgccg tctggtgatg taa 1893

<210> 162
<211> 630
<212> PRT
<213> Escherichia coli

<400> 162

Met Ala Ile Glu Ile Lys Val Pro Asp Ile Gly Ala Asp Glu Val Glu
1 5 10 15

Ile Thr Glu Ile Leu Val Lys Val Gly Asp Lys Val Glu Ala Glu Gln
20 25 30

Ser Leu Ile Thr Val Glu Gly Asp Lys Ala Ser Met Glu Val Pro Ser
35 40 45

Pro Gln Ala Gly Ile Val Lys Glu Ile Lys Val Ser Val Gly Asp Lys
50 55 60

Thr Gln Thr Gly Ala Leu Ile Met Ile Phe Asp Ser Ala Asp Gly Ala
65 70 75 80

Ala Asp Ala Ala Pro Ala Gln Ala Glu Glu Lys Lys Glu Ala Ala Pro
85 90 95

Ala Ala Ala Pro Ala Ala Ala Ala Ala Lys Asp Val Asn Val Pro Asp
100 105 110

Ile Gly Ser Asp Glu Val Glu Val Thr Glu Ile Leu Val Lys Val Gly
115 120 125

Asp Lys Val Glu Ala Glu Gln Ser Leu Ile Thr Val Glu Gly Asp Lys
130 135 140

Ala Ser Met Glu Val Pro Ala Pro Phe Ala Gly Thr Val Lys Glu Ile
145 150 155 160

```
Lys Val Asn Val Gly Asp Lys Val Ser Thr Gly Ser Leu Ile Met Val
                165                 170                 175

Phe Glu Val Ala Gly Glu Ala Gly Ala Ala Ala Pro Ala Ala Lys Gln
            180                 185                 190

Glu Ala Ala Pro Ala Ala Ala Pro Ala Pro Ala Ala Gly Val Lys Glu
        195                 200                 205

Val Asn Val Pro Asp Ile Gly Gly Asp Glu Val Glu Val Thr Glu Val
    210                 215                 220

Met Val Lys Val Gly Asp Lys Val Ala Ala Glu Gln Ser Leu Ile Thr
225                 230                 235                 240

Val Glu Gly Asp Lys Ala Ser Met Glu Val Pro Ala Pro Phe Ala Gly
                245                 250                 255

Val Val Lys Glu Leu Lys Val Asn Val Gly Asp Lys Val Lys Thr Gly
            260                 265                 270

Ser Leu Ile Met Ile Phe Glu Val Glu Gly Ala Ala Pro Ala Ala Ala
        275                 280                 285

Pro Ala Lys Gln Glu Ala Ala Ala Pro Ala Pro Ala Ala Lys Ala Glu
    290                 295                 300

Ala Pro Ala Ala Ala Pro Ala Ala Lys Ala Glu Gly Lys Ser Glu Phe
305                 310                 315                 320

Ala Glu Asn Asp Ala Tyr Val His Ala Thr Pro Leu Ile Arg Arg Leu
                325                 330                 335

Ala Arg Glu Phe Gly Val Asn Leu Ala Lys Val Lys Gly Thr Gly Arg
            340                 345                 350

Lys Gly Arg Ile Leu Arg Glu Asp Val Gln Ala Tyr Val Lys Glu Ala
        355                 360                 365

Ile Lys Arg Ala Glu Ala Ala Pro Ala Ala Thr Gly Gly Gly Ile Pro
    370                 375                 380

Gly Met Leu Pro Trp Pro Lys Val Asp Phe Ser Lys Phe Gly Glu Ile
385                 390                 395                 400

Glu Glu Val Glu Leu Gly Arg Ile Gln Lys Ile Ser Gly Ala Asn Leu
                405                 410                 415
```

Ser Arg Asn Trp Val Met Ile Pro His Val Thr His Phe Asp Lys Thr
420 425 430

Asp Ile Thr Glu Leu Glu Ala Phe Arg Lys Gln Gln Asn Glu Glu Ala
435 440 445

Ala Lys Arg Lys Leu Asp Val Lys Ile Thr Pro Val Val Phe Ile Met
450 455 460

Lys Ala Val Ala Ala Ala Leu Glu Gln Met Pro Arg Phe Asn Ser Ser
465 470 475 480

Leu Ser Glu Asp Gly Gln Arg Leu Thr Leu Lys Lys Tyr Ile Asn Ile
485 490 495

Gly Val Ala Val Asp Thr Pro Asn Gly Leu Val Val Pro Val Phe Lys
500 505 510

Asp Val Asn Lys Lys Gly Ile Ile Glu Leu Ser Arg Glu Leu Met Thr
515 520 525

Ile Ser Lys Lys Ala Arg Asp Gly Lys Leu Thr Ala Gly Glu Met Gln
530 535 540

Gly Gly Cys Phe Thr Ile Ser Ser Ile Gly Gly Leu Gly Thr Thr His
545 550 555 560

Phe Ala Pro Ile Val Asn Ala Pro Glu Val Ala Ile Leu Gly Val Ser
565 570 575

Lys Ser Ala Met Glu Pro Val Trp Asn Gly Lys Glu Phe Val Pro Arg
580 585 590

Leu Met Leu Pro Ile Ser Leu Ser Phe Asp His Arg Val Ile Asp Gly
595 600 605

Ala Asp Gly Ala Arg Phe Ile Thr Ile Ile Asn Asn Thr Leu Ser Asp
610 615 620

Ile Arg Arg Leu Val Met
625 630

<210> 163
<211> 1437
<212> DNA
<213> Escherichia coli

<400> 163
atgtcaaaca acattcgtat cgaagaagat ctgttgggta ccagggaagt tccagctgat 60

gcctactatg gtgttcacac tctgagagcg attgaaaact tctatatcag caacaacaaa 120

atcagtgata ttcctgaatt tgttcgcggt atggtaatgg ttaaaaaagc cgcagctatg 180

gcaaacaaag agctgcaaac cattcctaaa agtgtagcga atgccatcat tgccgcatgt 240

gatgaagtcc tgaacaacgg aaaatgcatg gatcagttcc cggtagacgt ctaccagggc 300

ggcgcaggta cttccgtaaa catgaacacc aacgaagtgc tggccaatat cggtctggaa 360

ctgatgggtc accaaaaagg tgaatatcag tacctgaacc cgaacgacca tgttaacaaa 420

tgtcagtcca ctaacgacgc ctacccgacc ggtttccgta tcgcagttta ctcttccctg 480

attaagctgg tagatgcgat taaccaactg cgtgaaggct ttgaacgtaa agctgtcgaa 540

ttccaggaca tcctgaaaat gggtcgtacc cagctgcagg acgcagtacc gatgaccctc 600

ggtcaggaat tccgcgcttt cagcatcctg ctgaaagaag aagtgaaaaa catccaacgt 660

accgctgaac tgctgctgga agttaacctt ggtgcaacag caatcggtac tggtctgaac 720

acgccgaaag agtactctcc gctggcagtg aaaaaactgg ctgaagttac tggcttccca 780

tgcgtaccgg ctgaagacct gatcgaagcg acctctgact gcggcgctta tgttatggtt 840

cacggcgcgc tgaaacgcct ggctgtgaag atgtccaaaa tctgtaacga cctgcgcttg 900

ctctcttcag gcccacgtgc cggcctgaac gagatcaacc tgccggaact gcaggcgggc 960

tcttccatca tgccagctaa agtaaacccg gttgttccgg aagtggttaa ccaggtatgc 1020

ttcaaagtca tcggtaacga caccactgtt accatggcag cagaagcagg tcagctgcag 1080

ttgaacgtta tggagccggt cattggccag gccatgttcg aatccgttca cattctgacc 1140

aacgcttgct acaacctgct ggaaaaatgc attaacggca tcactgctaa caaagaagtg 1200

tgcgaaggtt acgtttacaa ctctatcggt atcgttactt acctgaaccc gttcatcggt 1260

caccacaacg gtgacatcgt gggtaaaatc tgtgccgaaa ccggtaagag tgtacgtgaa 1320

gtcgttctgg aacgcggtct gttgactgaa gcggaacttg acgatatttt ctccgtacag 1380

aatctgatgc acccggctta caaagcaaaa cgctatactg atgaaagcga acagtaa 1437

<210> 164
<211> 478
<212> PRT
<213> Escherichia coli

<400> 164

Met Ser Asn Asn Ile Arg Ile Glu Glu Asp Leu Leu Gly Thr Arg Glu
1 5 10 15

Val Pro Ala Asp Ala Tyr Tyr Gly Val His Thr Leu Arg Ala Ile Glu
20 25 30

```
Asn Phe Tyr Ile Ser Asn Asn Lys Ile Ser Asp Ile Pro Glu Phe Val
        35                  40                  45

Arg Gly Met Val Met Val Lys Lys Ala Ala Ala Met Ala Asn Lys Glu
    50                  55                  60

Leu Gln Thr Ile Pro Lys Ser Val Ala Asn Ala Ile Ile Ala Ala Cys
65                  70                  75                  80

Asp Glu Val Leu Asn Asn Gly Lys Cys Met Asp Gln Phe Pro Val Asp
                85                  90                  95

Val Tyr Gln Gly Gly Ala Gly Thr Ser Val Asn Met Asn Thr Asn Glu
            100                 105                 110

Val Leu Ala Asn Ile Gly Leu Glu Leu Met Gly His Gln Lys Gly Glu
        115                 120                 125

Tyr Gln Tyr Leu Asn Pro Asn Asp His Val Asn Lys Cys Gln Ser Thr
    130                 135                 140

Asn Asp Ala Tyr Pro Thr Gly Phe Arg Ile Ala Val Tyr Ser Ser Leu
145                 150                 155                 160

Ile Lys Leu Val Asp Ala Ile Asn Gln Leu Arg Glu Gly Phe Glu Arg
                165                 170                 175

Lys Ala Val Glu Phe Gln Asp Ile Leu Lys Met Gly Arg Thr Gln Leu
            180                 185                 190

Gln Asp Ala Val Pro Met Thr Leu Gly Gln Glu Phe Arg Ala Phe Ser
        195                 200                 205

Ile Leu Leu Lys Glu Glu Val Lys Asn Ile Gln Arg Thr Ala Glu Leu
    210                 215                 220

Leu Leu Glu Val Asn Leu Gly Ala Thr Ala Ile Gly Thr Gly Leu Asn
225                 230                 235                 240

Thr Pro Lys Glu Tyr Ser Pro Leu Ala Val Lys Lys Leu Ala Glu Val
                245                 250                 255

Thr Gly Phe Pro Cys Val Pro Ala Glu Asp Leu Ile Glu Ala Thr Ser
            260                 265                 270

Asp Cys Gly Ala Tyr Val Met Val His Gly Ala Leu Lys Arg Leu Ala
```

275 280 285

Val Lys Met Ser Lys Ile Cys Asn Asp Leu Arg Leu Leu Ser Ser Gly
290 295 300

Pro Arg Ala Gly Leu Asn Glu Ile Asn Leu Pro Glu Leu Gln Ala Gly
305 310 315 320

Ser Ser Ile Met Pro Ala Lys Val Asn Pro Val Val Pro Glu Val Val
325 330 335

Asn Gln Val Cys Phe Lys Val Ile Gly Asn Asp Thr Thr Val Thr Met
340 345 350

Ala Ala Glu Ala Gly Gln Leu Gln Leu Asn Val Met Glu Pro Val Ile
355 360 365

Gly Gln Ala Met Phe Glu Ser Val His Ile Leu Thr Asn Ala Cys Tyr
370 375 380

Asn Leu Leu Glu Lys Cys Ile Asn Gly Ile Thr Ala Asn Lys Glu Val
385 390 395 400

Cys Glu Gly Tyr Val Tyr Asn Ser Ile Gly Ile Val Thr Tyr Leu Asn
405 410 415

Pro Phe Ile Gly His His Asn Gly Asp Ile Val Gly Lys Ile Cys Ala
420 425 430

Glu Thr Gly Lys Ser Val Arg Glu Val Val Leu Glu Arg Gly Leu Leu
435 440 445

Thr Glu Ala Glu Leu Asp Asp Ile Phe Ser Val Gln Asn Leu Met His
450 455 460

Pro Ala Tyr Lys Ala Lys Arg Tyr Thr Asp Glu Ser Glu Gln
465 470 475

<210> 165
<211> 2652
<212> DNA
<213> Escherichia coli

<400> 165
atgaacgaac aatattccgc attgcgtagt aatgtcagta tgctcggcaa agtgctggga 60

gaaaccatca aggatgcgtt gggagaacac attcttgaac gcgtagaaac tatccgtaag 120

ttgtcgaaat cttcacgcgc tggcaatgat gctaaccgcc aggagttgct caccacctta 180

caaaatttgt cgaacgacga gctgctgccc gttgcgcgtg cgtttagtca gttcctgaac 240
ctggccaaca ccgccgagca ataccacagc atttcgccga aaggcgaagc tgccagcaac 300
ccggaagtga tcgcccgcac cctgcgtaaa ctgaaaaacc agccggaact gagcgaagac 360
accatcaaaa aagcagtgga atcgctgtcg ctggaactgg tcctcacggc tcacccaacc 420
gaaattaccc gtcgtacact gatccacaaa atggtggaag tgaacgcctg tttaaaacag 480
ctcgataaca aagatatcgc tgactacgaa cacaaccagc tgatgcgtcg cctgcgccag 540
ttgatcgccc agtcatggca taccgatgaa atccgtaagc tgcgtccaag cccggtagat 600
gaagccaaat ggggctttgc cgtagtggaa aacagcctgt ggcaaggcgt accaaattac 660
ctgcgcgaac tgaacgaaca actggaagag aacctcggct acaaactgcc cgtcgaattt 720
gttccggtcc gttttacttc gtggatgggc ggcgaccgcg acggcaaccc gaacgtcact 780
gccgatatca cccgccacgt cctgctactc agccgctgga aagccaccga tttgttcctg 840
aaagatattc aggtgctggt ttctgaactg tcgatggttg aagcgacccc tgaactgctg 900
gcgctggttg gcgaagaagg tgccgcagaa ccgtatcgct atctgatgaa aaacctgcgt 960
tctcgcctga tggcgacaca ggcatggctg gaagcgcgcc tgaaaggcga agaactgcca 1020
aaaccagaag gcctgctgac acaaaacgaa gaactgtggg aaccgctcta cgcttgctac 1080
cagtcacttc aggcgtgtgg catgggtatt atcgccaacg gcgatctgct cgacaccctg 1140
cgccgcgtga aatgtttcgg cgtaccgctg gtccgtattg atatccgtca ggagagcacg 1200
cgtcataccg aagcgctggg cgagctgacc cgctacctcg gtatcggcga ctacgaaagc 1260
tggtcagagg ccgacaaaca ggcgttcctg atccgcgaac tgaactccaa acgtccgctt 1320
ctgccgcgca actggcaacc aagcgccgaa acgcgcgaag tgctcgatac ctgccaggtg 1380
attgccgaag caccgcaagg ctccattgcc gcctacgtga tctcgatggc gaaaacgccg 1440
tccgacgtac tggctgtcca cctgctgctg aaagaagcgg gtatcgggtt tgcgatgccg 1500
gttgctccgc tgtttgaaac cctcgatgat ctgaacaacg ccaacgatgt catgacccag 1560
ctgctcaata ttgactggta tcgtggcctg attcagggca aacagatggt gatgattggc 1620
tattccgact cagcaaaaga tgcgggagtg atggcagctt cctgggcgca atatcaggca 1680
caggatgcat taatcaaaac ctgcgaaaaa gcgggtattg agctgacgtt gttccacggt 1740
cgcggcggtt ccattggtcg cggcggcgca cctgctcatg cggcgctgct gtcacaaccg 1800
ccaggaagcc tgaaaggcgg cctgcgcgta accgaacagg gcgagatgat ccgctttaaa 1860
tatggtctgc cagaaatcac cgtcagcagc ctgtcgcttt ataccggggc gattctggaa 1920
gccaacctgc tgccaccgcc ggagccgaaa gagagctggc gtcgcattat ggatgaactg 1980
tcagtcatct cctgcgatgt ctaccgcggc tacgtacgtg aaaacaaaga ttttgtgcct 2040
tacttccgct ccgctacgcc ggaacaagaa ctgggcaaac tgccgttggg ttcacgtccg 2100

gcgaaacgtc gcccaaccgg cggcgtcgag tcactacgcg ccattccgtg gatcttcgcc 2160

tggacgcaaa accgtctgat gctccccgcc tggctgggtg caggtacggc gctgcaaaaa 2220

gtggtcgaag acggcaaaca gagcgagctg gaggctatgt gccgcgattg gccattcttc 2280

tcgacgcgtc tcggcatgct ggagatggtc ttcgccaaag cagacctgtg gctggcggaa 2340

tactatgacc aacgcctggt agacaaagca ctgtggccgt taggtaaaga gttacgcaac 2400

ctgcaagaag aagacatcaa agtggtgctg gcgattgcca acgattccca tctgatggcc 2460

gatctgccgt ggattgcaga gtctattcag ctacggaata tttacaccga cccgctgaac 2520

gtattgcagg ccgagttgct gcaccgctcc cgccaggcag aaaaagaagg ccaggaaccg 2580

gatcctcgcg tcgaacaagc gttaatggtc actattgccg ggattgcggc aggtatgcgt 2640

aataccggct aa 2652

<210> 166
<211> 883
<212> PRT
<213> Escherichia coli

<400> 166

Met Asn Glu Gln Tyr Ser Ala Leu Arg Ser Asn Val Ser Met Leu Gly
1 5 10 15

Lys Val Leu Gly Glu Thr Ile Lys Asp Ala Leu Gly Glu His Ile Leu
20 25 30

Glu Arg Val Glu Thr Ile Arg Lys Leu Ser Lys Ser Ser Arg Ala Gly
35 40 45

Asn Asp Ala Asn Arg Gln Glu Leu Leu Thr Thr Leu Gln Asn Leu Ser
50 55 60

Asn Asp Glu Leu Leu Pro Val Ala Arg Ala Phe Ser Gln Phe Leu Asn
65 70 75 80

Leu Ala Asn Thr Ala Glu Gln Tyr His Ser Ile Ser Pro Lys Gly Glu
85 90 95

Ala Ala Ser Asn Pro Glu Val Ile Ala Arg Thr Leu Arg Lys Leu Lys
100 105 110

Asn Gln Pro Glu Leu Ser Glu Asp Thr Ile Lys Lys Ala Val Glu Ser
115 120 125

Leu Ser Leu Glu Leu Val Leu Thr Ala His Pro Thr Glu Ile Thr Arg
130 135 140

Arg Thr Leu Ile His Lys Met Val Glu Val Asn Ala Cys Leu Lys Gln
145 150 155 160

Leu Asp Asn Lys Asp Ile Ala Asp Tyr Glu His Asn Gln Leu Met Arg
165 170 175

Arg Leu Arg Gln Leu Ile Ala Gln Ser Trp His Thr Asp Glu Ile Arg
180 185 190

Lys Leu Arg Pro Ser Pro Val Asp Glu Ala Lys Trp Gly Phe Ala Val
195 200 205

Val Glu Asn Ser Leu Trp Gln Gly Val Pro Asn Tyr Leu Arg Glu Leu
210 215 220

Asn Glu Gln Leu Glu Glu Asn Leu Gly Tyr Lys Leu Pro Val Glu Phe
225 230 235 240

Val Pro Val Arg Phe Thr Ser Trp Met Gly Gly Asp Arg Asp Gly Asn
245 250 255

Pro Asn Val Thr Ala Asp Ile Thr Arg His Val Leu Leu Leu Ser Arg
260 265 270

Trp Lys Ala Thr Asp Leu Phe Leu Lys Asp Ile Gln Val Leu Val Ser
275 280 285

Glu Leu Ser Met Val Glu Ala Thr Pro Glu Leu Leu Ala Leu Val Gly
290 295 300

Glu Glu Gly Ala Ala Glu Pro Tyr Arg Tyr Leu Met Lys Asn Leu Arg
305 310 315 320

Ser Arg Leu Met Ala Thr Gln Ala Trp Leu Glu Ala Arg Leu Lys Gly
325 330 335

Glu Glu Leu Pro Lys Pro Glu Gly Leu Leu Thr Gln Asn Glu Glu Leu
340 345 350

Trp Glu Pro Leu Tyr Ala Cys Tyr Gln Ser Leu Gln Ala Cys Gly Met
355 360 365

Gly Ile Ile Ala Asn Gly Asp Leu Leu Asp Thr Leu Arg Arg Val Lys
370 375 380

Cys Phe Gly Val Pro Leu Val Arg Ile Asp Ile Arg Gln Glu Ser Thr

385 390 395 400

Arg His Thr Glu Ala Leu Gly Glu Leu Thr Arg Tyr Leu Gly Ile Gly
405 410 415

Asp Tyr Glu Ser Trp Ser Glu Ala Asp Lys Gln Ala Phe Leu Ile Arg
420 425 430

Glu Leu Asn Ser Lys Arg Pro Leu Leu Pro Arg Asn Trp Gln Pro Ser
435 440 445

Ala Glu Thr Arg Glu Val Leu Asp Thr Cys Gln Val Ile Ala Glu Ala
450 455 460

Pro Gln Gly Ser Ile Ala Ala Tyr Val Ile Ser Met Ala Lys Thr Pro
465 470 475 480

Ser Asp Val Leu Ala Val His Leu Leu Leu Lys Glu Ala Gly Ile Gly
485 490 495

Phe Ala Met Pro Val Ala Pro Leu Phe Glu Thr Leu Asp Asp Leu Asn
500 505 510

Asn Ala Asn Asp Val Met Thr Gln Leu Leu Asn Ile Asp Trp Tyr Arg
515 520 525

Gly Leu Ile Gln Gly Lys Gln Met Val Met Ile Gly Tyr Ser Asp Ser
530 535 540

Ala Lys Asp Ala Gly Val Met Ala Ala Ser Trp Ala Gln Tyr Gln Ala
545 550 555 560

Gln Asp Ala Leu Ile Lys Thr Cys Glu Lys Ala Gly Ile Glu Leu Thr
565 570 575

Leu Phe His Gly Arg Gly Gly Ser Ile Gly Arg Gly Gly Ala Pro Ala
580 585 590

His Ala Ala Leu Leu Ser Gln Pro Pro Gly Ser Leu Lys Gly Gly Leu
595 600 605

Arg Val Thr Glu Gln Gly Glu Met Ile Arg Phe Lys Tyr Gly Leu Pro
610 615 620

Glu Ile Thr Val Ser Ser Leu Ser Leu Tyr Thr Gly Ala Ile Leu Glu
625 630 635 640

```
Ala Asn Leu Leu Pro Pro Pro Glu Pro Lys Glu Ser Trp Arg Arg Ile
                645                 650                 655

Met Asp Glu Leu Ser Val Ile Ser Cys Asp Val Tyr Arg Gly Tyr Val
            660                 665                 670

Arg Glu Asn Lys Asp Phe Val Pro Tyr Phe Arg Ser Ala Thr Pro Glu
        675                 680                 685

Gln Glu Leu Gly Lys Leu Pro Leu Gly Ser Arg Pro Ala Lys Arg Arg
    690                 695                 700

Pro Thr Gly Gly Val Glu Ser Leu Arg Ala Ile Pro Trp Ile Phe Ala
705                 710                 715                 720

Trp Thr Gln Asn Arg Leu Met Leu Pro Ala Trp Leu Gly Ala Gly Thr
                725                 730                 735

Ala Leu Gln Lys Val Val Glu Asp Gly Lys Gln Ser Glu Leu Glu Ala
            740                 745                 750

Met Cys Arg Asp Trp Pro Phe Phe Ser Thr Arg Leu Gly Met Leu Glu
        755                 760                 765

Met Val Phe Ala Lys Ala Asp Leu Trp Leu Ala Glu Tyr Tyr Asp Gln
    770                 775                 780

Arg Leu Val Asp Lys Ala Leu Trp Pro Leu Gly Lys Glu Leu Arg Asn
785                 790                 795                 800

Leu Gln Glu Glu Asp Ile Lys Val Val Leu Ala Ile Ala Asn Asp Ser
                805                 810                 815

His Leu Met Ala Asp Leu Pro Trp Ile Ala Glu Ser Ile Gln Leu Arg
            820                 825                 830

Asn Ile Tyr Thr Asp Pro Leu Asn Val Leu Gln Ala Glu Leu Leu His
        835                 840                 845

Arg Ser Arg Gln Ala Glu Lys Glu Gly Gln Glu Pro Asp Pro Arg Val
    850                 855                 860

Glu Gln Ala Leu Met Val Thr Ile Ala Gly Ile Ala Ala Gly Met Arg
865                 870                 875                 880

Asn Thr Gly
```

<210> 167
<211> 1623
<212> DNA
<213> Escherichia coli

<400> 167
atgcgcgtta acaatggttt gaccccgcaa gaactcgagg cttatggtat cagtgacgta 60

catgatatcg tttacaaccc aagctacgac ctgctgtatc aggaagagct cgatccgagc 120

ctgacaggtt atgagcgcgg ggtgttaact aatctgggtg ccgttgccgt cgataccggg 180

atcttcaccg gtcgttcacc aaaagataag tatatcgtcc gtgacgatac cactcgcgat 240

actttctggt gggcagacaa aggcaaaggt aagaacgaca acaaacctct ctctccggaa 300

acctggcagc atctgaaagg cctggtgacc aggcagcttt ccggcaaacg tctgttcgtt 360

gtcgacgctt tctgtggtgc gaacccggat actcgtcttt ccgtccgttt catcaccgaa 420

gtggcctggc aggcgcattt tgtcaaaaac atgtttattc gcccgagcga tgaagaactg 480

gcaggtttca aaccagactt tatcgttatg aacggcgcga agtgcactaa cccgcagtgg 540

aaagaacagg gtctcaactc cgaaaacttc gtggcgttta acctgaccga gcgcatgcag 600

ctgattggcg gcacctggta cggcggcgaa atgaagaaag ggatgttctc gatgatgaac 660

tacctgctgc cgctgaaagg tatcgcttct atgcactgct ccgccaacgt tggtgagaaa 720

ggcgatgttg cggtgttctt cggcctttcc ggcaccggta aaaccaccct ttccaccgac 780

ccgaaacgtc gcctgattgg cgatgacgaa cacggctggg acgatgacgg cgtgtttaac 840

ttcgaaggcg gctgctacgc aaaaactatc aagctgtcga aagaagcgga acctgaaatc 900

tacaacgcta tccgtcgtga tgcgttgctg gaaaacgtca ccgtgcgtga agatggcact 960

atcgactttg atgatggttc aaaaaccgag aacacccgcg tttcttatcc gatctatcac 1020

atcgataaca ttgttaagcc ggtttccaaa gcgggccacg cgactaaggt tatcttcctg 1080

actgctgatg ctttcggcgt gttgccgccg gtttctcgcc tgactgccga tcaaacccag 1140

tatcacttcc tctctggctt caccgccaaa ctggccggta ctgagcgtgg catcaccgaa 1200

ccgacgccaa ccttctccgc ttgcttcggc gcggcattcc tgtcgctgca cccgactcag 1260

tacgcagaag tgctggtgaa acgtatgcag gcggcgggcg cgcaggctta tctggttaac 1320

actggctgga acggcactgg caaacgtatc tcgattaaag atacccgcgc cattatcgac 1380

gccatcctca acggttcgct ggataatgca gaaaccttca ctctgccgat gtttaacctg 1440

gcgatcccaa ccgaactgcc gggcgtagac acgaagattc tcgatccgcg taacacctac 1500

gcttctccgg aacagtggca ggaaaaagcc gaaaccctgg cgaaactgtt tatcgacaac 1560

ttcgataaat acaccgacac ccctgcgggt gccgcgctgg tagcggctgg tccgaaactg 1620

taa 1623

<210> 168
<211> 540
<212> PRT
<213> Escherichia coli

<400> 168

Met Arg Val Asn Asn Gly Leu Thr Pro Gln Glu Leu Glu Ala Tyr Gly
1 5 10 15

Ile Ser Asp Val His Asp Ile Val Tyr Asn Pro Ser Tyr Asp Leu Leu
20 25 30

Tyr Gln Glu Glu Leu Asp Pro Ser Leu Thr Gly Tyr Glu Arg Gly Val
35 40 45

Leu Thr Asn Leu Gly Ala Val Ala Val Asp Thr Gly Ile Phe Thr Gly
50 55 60

Arg Ser Pro Lys Asp Lys Tyr Ile Val Arg Asp Asp Thr Thr Arg Asp
65 70 75 80

Thr Phe Trp Trp Ala Asp Lys Gly Lys Gly Lys Asn Asp Asn Lys Pro
85 90 95

Leu Ser Pro Glu Thr Trp Gln His Leu Lys Gly Leu Val Thr Arg Gln
100 105 110

Leu Ser Gly Lys Arg Leu Phe Val Val Asp Ala Phe Cys Gly Ala Asn
115 120 125

Pro Asp Thr Arg Leu Ser Val Arg Phe Ile Thr Glu Val Ala Trp Gln
130 135 140

Ala His Phe Val Lys Asn Met Phe Ile Arg Pro Ser Asp Glu Glu Leu
145 150 155 160

Ala Gly Phe Lys Pro Asp Phe Ile Val Met Asn Gly Ala Lys Cys Thr
165 170 175

Asn Pro Gln Trp Lys Glu Gln Gly Leu Asn Ser Glu Asn Phe Val Ala
180 185 190

Phe Asn Leu Thr Glu Arg Met Gln Leu Ile Gly Gly Thr Trp Tyr Gly
195 200 205

Gly Glu Met Lys Lys Gly Met Phe Ser Met Met Asn Tyr Leu Leu Pro
210 215 220

Leu Lys Gly Ile Ala Ser Met His Cys Ser Ala Asn Val Gly Glu Lys
225 230 235 240

Gly Asp Val Ala Val Phe Phe Gly Leu Ser Gly Thr Gly Lys Thr Thr
245 250 255

Leu Ser Thr Asp Pro Lys Arg Arg Leu Ile Gly Asp Asp Glu His Gly
260 265 270

Trp Asp Asp Asp Gly Val Phe Asn Phe Glu Gly Gly Cys Tyr Ala Lys
275 280 285

Thr Ile Lys Leu Ser Lys Glu Ala Glu Pro Glu Ile Tyr Asn Ala Ile
290 295 300

Arg Arg Asp Ala Leu Leu Glu Asn Val Thr Val Arg Glu Asp Gly Thr
305 310 315 320

Ile Asp Phe Asp Asp Gly Ser Lys Thr Glu Asn Thr Arg Val Ser Tyr
325 330 335

Pro Ile Tyr His Ile Asp Asn Ile Val Lys Pro Val Ser Lys Ala Gly
340 345 350

His Ala Thr Lys Val Ile Phe Leu Thr Ala Asp Ala Phe Gly Val Leu
355 360 365

Pro Pro Val Ser Arg Leu Thr Ala Asp Gln Thr Gln Tyr His Phe Leu
370 375 380

Ser Gly Phe Thr Ala Lys Leu Ala Gly Thr Glu Arg Gly Ile Thr Glu
385 390 395 400

Pro Thr Pro Thr Phe Ser Ala Cys Phe Gly Ala Ala Phe Leu Ser Leu
405 410 415

His Pro Thr Gln Tyr Ala Glu Val Leu Val Lys Arg Met Gln Ala Ala
420 425 430

Gly Ala Gln Ala Tyr Leu Val Asn Thr Gly Trp Asn Gly Thr Gly Lys
435 440 445

Arg Ile Ser Ile Lys Asp Thr Arg Ala Ile Ile Asp Ala Ile Leu Asn
450 455 460

Gly Ser Leu Asp Asn Ala Glu Thr Phe Thr Leu Pro Met Phe Asn Leu
465 470 475 480

Ala Ile Pro Thr Glu Leu Pro Gly Val Asp Thr Lys Ile Leu Asp Pro
485 490 495

Arg Asn Thr Tyr Ala Ser Pro Glu Gln Trp Gln Glu Lys Ala Glu Thr
500 505 510

Leu Ala Lys Leu Phe Ile Asp Asn Phe Asp Lys Tyr Thr Asp Thr Pro
515 520 525

Ala Gly Ala Ala Leu Val Ala Ala Gly Pro Lys Leu
530 535 540

<210> 169
<211> 1221
<212> DNA
<213> Escherichia coli

<400> 169
gtggttgctg aaaaccagcc tgggcacatt gatcaaataa agcagaccaa cgcgggcgcg 60

gtttatcgcc tgattgatca gcttggtcca gtctcgcgta tcgatctttc ccgtctggcg 120

caactggctc ctgccagtat cactaaaatt gtccgtgaga tgctcgaagc acacctggtg 180

caagagctgg aaatcaaaga agcggggaac cgtggccgtc cggcggtggg gctggtggtt 240

gaaactgaag cctggcacta tctttctctg cgcattagtc gcggggagat tttccttgct 300

ctgcgcgatc tgagcagcaa actggtggtg gaagagtcgc aggaactggc gttaaaagat 360

gacttgccat tgctggatcg tattatttcc catatcgatc agtttttat ccgccaccag 420

aaaaaacttg agcgtctaac ttcgattgcc ataaccttgc cgggaattat tgatacggaa 480

aatggtattg tacatcgcat gccgttctac gaggatgtaa aagagatgcc gctcggcgag 540

gcgctggagc agcataccgg cgttccggtt tatattcagc atgatatcag cgcatggacg 600

atggcagagg ccttgtttgg tgcctcacgc ggggcgcgcg atgtgattca ggtggttatc 660

gatcacaacg tgggggcggg cgtcattacc gatggtcatc tgctacacgc aggcagcagt 720

agtctcgtgg aaataggcca cacacaggtc gacccgtatg ggaaacgctg ttattgcggg 780

aatcacggct gcctcgaaac catcgccagc gtggacagta ttcttgagct ggcacagctg 840

cgtcttaatc aatccatgag ctcgatgtta catggacaac cgttaaccgt ggactcattg 900

tgtcaggcgg cattgcgcgg cgatctactg gcaaaagaca tcattaccgg ggtgggcgcg 960

catgtcgggc gcattcttgc catcatggtg aatttattta acccacaaaa aatactgatt 1020

ggctcaccgt taagtaaagc ggcagatatc ctcttcccgg tcatctcaga cagcatccgt 1080

cagcaggccc ttcctgcgta tagtcagcac atcagcgttg agagtactca gttttctaac 1140

cagggcacga tggcaggcgc tgcactggta aaagacgcga tgtataacgg ttctttgttg 1200

attcgtctgt tgcagggtta a 1221

<210> 170
<211> 406
<212> PRT
<213> Escherichia coli

<400> 170

Met Val Ala Glu Asn Gln Pro Gly His Ile Asp Gln Ile Lys Gln Thr
1               5                   10                  15

Asn Ala Gly Ala Val Tyr Arg Leu Ile Asp Gln Leu Gly Pro Val Ser
            20                  25                  30

Arg Ile Asp Leu Ser Arg Leu Ala Gln Leu Ala Pro Ala Ser Ile Thr
        35                  40                  45

Lys Ile Val Arg Glu Met Leu Glu Ala His Leu Val Gln Glu Leu Glu
    50                  55                  60

Ile Lys Glu Ala Gly Asn Arg Gly Arg Pro Ala Val Gly Leu Val Val
65                  70                  75                  80

Glu Thr Glu Ala Trp His Tyr Leu Ser Leu Arg Ile Ser Arg Gly Glu
                85                  90                  95

Ile Phe Leu Ala Leu Arg Asp Leu Ser Ser Lys Leu Val Val Glu Glu
            100                 105                 110

Ser Gln Glu Leu Ala Leu Lys Asp Asp Leu Pro Leu Leu Asp Arg Ile
        115                 120                 125

Ile Ser His Ile Asp Gln Phe Phe Ile Arg His Gln Lys Lys Leu Glu
    130                 135                 140

Arg Leu Thr Ser Ile Ala Ile Thr Leu Pro Gly Ile Ile Asp Thr Glu
145                 150                 155                 160

Asn Gly Ile Val His Arg Met Pro Phe Tyr Glu Asp Val Lys Glu Met
                165                 170                 175

Pro Leu Gly Glu Ala Leu Glu Gln His Thr Gly Val Pro Val Tyr Ile
            180                 185                 190

Gln His Asp Ile Ser Ala Trp Thr Met Ala Glu Ala Leu Phe Gly Ala
        195                 200                 205

Ser Arg Gly Ala Arg Asp Val Ile Gln Val Val Ile Asp His Asn Val
210 215 220

Gly Ala Gly Val Ile Thr Asp Gly His Leu Leu His Ala Gly Ser Ser
225 230 235 240

Ser Leu Val Glu Ile Gly His Thr Gln Val Asp Pro Tyr Gly Lys Arg
245 250 255

Cys Tyr Cys Gly Asn His Gly Cys Leu Glu Thr Ile Ala Ser Val Asp
260 265 270

Ser Ile Leu Glu Leu Ala Gln Leu Arg Leu Asn Gln Ser Met Ser Ser
275 280 285

Met Leu His Gly Gln Pro Leu Thr Val Asp Ser Leu Cys Gln Ala Ala
290 295 300

Leu Arg Gly Asp Leu Leu Ala Lys Asp Ile Ile Thr Gly Val Gly Ala
305 310 315 320

His Val Gly Arg Ile Leu Ala Ile Met Val Asn Leu Phe Asn Pro Gln
325 330 335

Lys Ile Leu Ile Gly Ser Pro Leu Ser Lys Ala Ala Asp Ile Leu Phe
340 345 350

Pro Val Ile Ser Asp Ser Ile Arg Gln Gln Ala Leu Pro Ala Tyr Ser
355 360 365

Gln His Ile Ser Val Glu Ser Thr Gln Phe Ser Asn Gln Gly Thr Met
370 375 380

Ala Gly Ala Ala Leu Val Lys Asp Ala Met Tyr Asn Gly Ser Leu Leu
385 390 395 400

Ile Arg Leu Leu Gln Gly
405

<210> 171
<211> 1434
<212> DNA
<213> Escherichia coli

<400> 171
atgtttaaga atgcatttgc taacctgcaa aaggtcggta aatcgctgat gctgccggta 60

tccgtactgc ctatcgcagg tattctgctg ggcgtcggtt ccgcgaattt cagctggctg 120

cccgccgttg tatcgcatgt tatggcagaa gcaggcggtt ccgtctttgc aaacatgcca 180

ctgatttttg cgatcggtgt cgccctcggc tttaccaata acgatggcgt atccgcgctg 240

gccgcagttg ttgcctatgg catcatggtt aaaaccatgg ccgtggttgc gccactggta 300

ctgcatttac ctgctgaaga aatcgcctct aaacacctgg cggatactgg cgtactcgga 360

gggattatct ccggtgcgat cgcagcgtac atgtttaacc gtttctaccg tattaagctg 420

cctgagtatc ttggcttctt tgccggtaaa cgctttgtgc cgatcatttc tggcctggct 480

gccatcttta ctggcgttgt gctgtccttc atttggccgc cgattggttc tgcaatccag 540

accttctctc agtgggctgc ttaccagaac ccggtagttg cgtttggcat ttacggtttc 600

atcgaacgtt gcctggtacc gtttggtctg caccacatct ggaacgtacc tttccagatg 660

cagattggtg aatacaccaa cgcagcaggt caggttttcc acggcgacat tccgcgttat 720

atggcgggtg acccgactgc gggtaaactg tctggtggct tcctgttcaa aatgtacggt 780

ctgccagctg ccgcaattgc tatctggcac tctgctaaac cagaaaaccg cgcgaaagtg 840

ggcggtatta tgatctccgc ggcgctgacc tcgttcctga ccggtatcac cgagccgatc 900

gagttctcct tcatgttcgt tgcgccgatc ctgtacatca tccacgcgat tctggcaggc 960

ctggcattcc caatctgtat tcttctgggg atgcgtgacg gtacgtcgtt ctcgcacggt 1020

ctgatcgact tcatcgttct gtctggtaac agcagcaaac tgtggctgtt cccgatcgtc 1080

ggtatcggtt atgcgattgt ttactacacc atcttccgcg tgctgattaa agcactggat 1140

ctgaaaacgc cgggtcgtga agacgcgact gaagatgcaa aagcgacagg taccagcgaa 1200

atggcaccgg ctctggttgc tgcatttggt ggtaaagaaa acattactaa cctcgacgca 1260

tgtattaccc gtctgcgcgt cagcgttgct gatgtgtcta aagtggatca ggccggcctg 1320

aagaaactgg gcgcagcggg cgtagtggtt gctggttctg gtgttcaggc gattttcggt 1380

actaaatccg ataacctgaa aaccgagatg gatgagtaca tccgtaacca ctaa 1434

<210> 172
<211> 477
<212> PRT
<213> Escherichia coli

<400> 172

Met Phe Lys Asn Ala Phe Ala Asn Leu Gln Lys Val Gly Lys Ser Leu
1 5 10 15

Met Leu Pro Val Ser Val Leu Pro Ile Ala Gly Ile Leu Leu Gly Val
20 25 30

Gly Ser Ala Asn Phe Ser Trp Leu Pro Ala Val Val Ser His Val Met
35 40 45

```
Ala Glu Ala Gly Gly Ser Val Phe Ala Asn Met Pro Leu Ile Phe Ala
    50                  55                  60

Ile Gly Val Ala Leu Gly Phe Thr Asn Asn Asp Gly Val Ser Ala Leu
65                  70                  75                  80

Ala Ala Val Val Ala Tyr Gly Ile Met Val Lys Thr Met Ala Val Val
                85                  90                  95

Ala Pro Leu Val Leu His Leu Pro Ala Glu Glu Ile Ala Ser Lys His
            100                 105                 110

Leu Ala Asp Thr Gly Val Leu Gly Gly Ile Ile Ser Gly Ala Ile Ala
        115                 120                 125

Ala Tyr Met Phe Asn Arg Phe Tyr Arg Ile Lys Leu Pro Glu Tyr Leu
    130                 135                 140

Gly Phe Phe Ala Gly Lys Arg Phe Val Pro Ile Ile Ser Gly Leu Ala
145                 150                 155                 160

Ala Ile Phe Thr Gly Val Val Leu Ser Phe Ile Trp Pro Pro Ile Gly
                165                 170                 175

Ser Ala Ile Gln Thr Phe Ser Gln Trp Ala Ala Tyr Gln Asn Pro Val
            180                 185                 190

Val Ala Phe Gly Ile Tyr Gly Phe Ile Glu Arg Cys Leu Val Pro Phe
        195                 200                 205

Gly Leu His His Ile Trp Asn Val Pro Phe Gln Met Gln Ile Gly Glu
    210                 215                 220

Tyr Thr Asn Ala Ala Gly Gln Val Phe His Gly Asp Ile Pro Arg Tyr
225                 230                 235                 240

Met Ala Gly Asp Pro Thr Ala Gly Lys Leu Ser Gly Gly Phe Leu Phe
                245                 250                 255

Lys Met Tyr Gly Leu Pro Ala Ala Ala Ile Ala Ile Trp His Ser Ala
            260                 265                 270

Lys Pro Glu Asn Arg Ala Lys Val Gly Gly Ile Met Ile Ser Ala Ala
        275                 280                 285

Leu Thr Ser Phe Leu Thr Gly Ile Thr Glu Pro Ile Glu Phe Ser Phe
    290                 295                 300
```

Met Phe Val Ala Pro Ile Leu Tyr Ile Ile His Ala Ile Leu Ala Gly
305 310 315 320

Leu Ala Phe Pro Ile Cys Ile Leu Leu Gly Met Arg Asp Gly Thr Ser
325 330 335

Phe Ser His Gly Leu Ile Asp Phe Ile Val Leu Ser Gly Asn Ser Ser
340 345 350

Lys Leu Trp Leu Phe Pro Ile Val Gly Ile Gly Tyr Ala Ile Val Tyr
355 360 365

Tyr Thr Ile Phe Arg Val Leu Ile Lys Ala Leu Asp Leu Lys Thr Pro
370 375 380

Gly Arg Glu Asp Ala Thr Glu Asp Ala Lys Ala Thr Gly Thr Ser Glu
385 390 395 400

Met Ala Pro Ala Leu Val Ala Ala Phe Gly Gly Lys Glu Asn Ile Thr
405 410 415

Asn Leu Asp Ala Cys Ile Thr Arg Leu Arg Val Ser Val Ala Asp Val
420 425 430

Ser Lys Val Asp Gln Ala Gly Leu Lys Lys Leu Gly Ala Ala Gly Val
435 440 445

Val Val Ala Gly Ser Gly Val Gln Ala Ile Phe Gly Thr Lys Ser Asp
450 455 460

Asn Leu Lys Thr Glu Met Asp Glu Tyr Ile Arg Asn His
465 470 475

<210> 173
<211> 258
<212> DNA
<213> Escherichia coli

<400> 173
atgttccagc aagaagttac cattaccgct ccgaacggtc tgcacacccg ccctgctgcc 60

cagtttgtaa aagaagctaa gggcttcact tctgaaatta ctgtgacttc caacggcaaa 120

agcgccagcg cgaaaagcct gtttaaactg cagactctgg gcctgactca aggtaccgtt 180

gtgactatct ccgcagaagg cgaagacgag cagaaagcgg ttgaacatct ggttaaactg 240

atggcggaac tcgagtaa 258

<210> 174

<211> 85
<212> PRT
<213> Escherichia coli

<400> 174

Met Phe Gln Gln Glu Val Thr Ile Thr Ala Pro Asn Gly Leu His Thr
1               5                   10                  15

Arg Pro Ala Ala Gln Phe Val Lys Glu Ala Lys Gly Phe Thr Ser Glu
            20                  25                  30

Ile Thr Val Thr Ser Asn Gly Lys Ser Ala Ser Ala Lys Ser Leu Phe
        35                  40                  45

Lys Leu Gln Thr Leu Gly Leu Thr Gln Gly Thr Val Val Thr Ile Ser
    50                  55                  60

Ala Glu Gly Glu Asp Glu Gln Lys Ala Val Glu His Leu Val Lys Leu
65                  70                  75                  80

Met Ala Glu Leu Glu
                85

<210> 175
<211> 1728
<212> DNA
<213> Escherichia coli

<400> 175
atgatttcag gcattttagc atccccgggt atcgctttcg gtaaagctct gcttctgaaa 60

gaagacgaaa ttgtcattga ccggaaaaaa atttctgccg accaggttga tcaggaagtt 120

gaacgttttc tgagcggtcg tgccaaggca tcagcccagc tggaaacgat caaaacgaaa 180

gctggtgaaa cgttcggtga agaaaaagaa gccatctttg aagggcatat tatgctgctc 240

gaagatgagg agctggagca ggaaatcata gccctgatta aagataagca catgacagct 300

gacgcagctg ctcatgaagt tatcgaaggt caggcttctg ccctggaaga gctggatgat 360

gaatacctga aagaacgtgc ggctgacgta cgtgatatcg gtaagcgcct gctgcgcaac 420

atcctgggcc tgaagattat cgacctgagc gccattcagg atgaagtcat tctggttgcc 480

gctgacctga cgccgtccga aaccgcacag ctgaacctga agaaggtgct gggtttcatc 540

accgacgcgg gtggccgtac ttcccacacc tctatcatgg cgcgttctct ggaactacct 600

gctatcgtgg gtaccggtag cgtcacctct caggtgaaaa atgacgacta tctgattctg 660

gatgccgtaa ataatcaggt ttacgtcaat ccaaccaacg aagttattga taaaatgcgc 720

gctgttcagg agcaagtggc ttctgaaaaa gcagagcttg ctaaactgaa agatctgcca 780

gctattacgc tggacggtca ccaggtagaa gtatgcgcta acattggtac ggttcgtgac 840

gttgaaggtg cagagcgtaa cggcgctgaa ggcgttggtc tgtatcgtac tgagttcctg 900

ttcatggacc gcgacgcact gcccactgaa gaagaacagt ttgctgctta caaagcagtg 960

gctgaagcgt gtggctcgca agcggttatc gttcgtacca tggacatcgg cggcgacaaa 1020

gagctgccat acatgaactt cccgaaagaa gagaacccgt tcctcggctg gcgcgctatc 1080

cgtatcgcga tggatcgtag agagatcctg cgcgatcagc tccgcgctat cctgcgtgcc 1140

tcggctttcg gtaaattgcg cattatgttc ccgatgatca tctctgttga agaagtgcgt 1200

gcactgcgca aagagatcga aatctacaaa caggaactgc gcgacgaagg taaagcgttt 1260

gacgagtcaa ttgaaatcgg cgtaatggtg gaaacaccgg ctgccgcaac aattgcacgt 1320

catttagcca aagaagttga tttctttagt atcggcacca atgatttaac gcagtacact 1380

ctggcagttg accgtggtaa tgatatgatt tcacaccttt accagccaat gtcaccgtcc 1440

gtgctgaact tgatcaagca agttattgat gcttctcatg ctgaaggcaa atggactggc 1500

atgtgtggtg agcttgctgg cgatgaacgt gctacacttc tgttgctggg gatgggtctg 1560

gacgaattct ctatgagcgc catttctatc ccgcgcatta agaagattat ccgtaacacg 1620

aacttcgaag atgcgaaggt gttagcagag caggctcttg ctcaaccgac aacggacgag 1680

ttaatgacgc tggttaacaa gttcattgaa gaaaaaacaa tctgctaa 1728

<210> 176
<211> 575
<212> PRT
<213> Escherichia coli

<400> 176

Met Ile Ser Gly Ile Leu Ala Ser Pro Gly Ile Ala Phe Gly Lys Ala
1 5 10 15

Leu Leu Leu Lys Glu Asp Glu Ile Val Ile Asp Arg Lys Lys Ile Ser
20 25 30

Ala Asp Gln Val Asp Gln Glu Val Glu Arg Phe Leu Ser Gly Arg Ala
35 40 45

Lys Ala Ser Ala Gln Leu Glu Thr Ile Lys Thr Lys Ala Gly Glu Thr
50 55 60

Phe Gly Glu Glu Lys Glu Ala Ile Phe Glu Gly His Ile Met Leu Leu
65 70 75 80

Glu Asp Glu Glu Leu Glu Gln Glu Ile Ile Ala Leu Ile Lys Asp Lys
85 90 95

```
His Met Thr Ala Asp Ala Ala Ala His Glu Val Ile Glu Gly Gln Ala
            100                 105                 110

Ser Ala Leu Glu Glu Leu Asp Asp Glu Tyr Leu Lys Glu Arg Ala Ala
        115                 120                 125

Asp Val Arg Asp Ile Gly Lys Arg Leu Leu Arg Asn Ile Leu Gly Leu
    130                 135                 140

Lys Ile Ile Asp Leu Ser Ala Ile Gln Asp Glu Val Ile Leu Val Ala
145                 150                 155                 160

Ala Asp Leu Thr Pro Ser Glu Thr Ala Gln Leu Asn Leu Lys Lys Val
                165                 170                 175

Leu Gly Phe Ile Thr Asp Ala Gly Gly Arg Thr Ser His Thr Ser Ile
            180                 185                 190

Met Ala Arg Ser Leu Glu Leu Pro Ala Ile Val Gly Thr Gly Ser Val
        195                 200                 205

Thr Ser Gln Val Lys Asn Asp Asp Tyr Leu Ile Leu Asp Ala Val Asn
    210                 215                 220

Asn Gln Val Tyr Val Asn Pro Thr Asn Glu Val Ile Asp Lys Met Arg
225                 230                 235                 240

Ala Val Gln Glu Gln Val Ala Ser Glu Lys Ala Glu Leu Ala Lys Leu
                245                 250                 255

Lys Asp Leu Pro Ala Ile Thr Leu Asp Gly His Gln Val Glu Val Cys
            260                 265                 270

Ala Asn Ile Gly Thr Val Arg Asp Val Glu Gly Ala Glu Arg Asn Gly
        275                 280                 285

Ala Glu Gly Val Gly Leu Tyr Arg Thr Glu Phe Leu Phe Met Asp Arg
    290                 295                 300

Asp Ala Leu Pro Thr Glu Glu Glu Gln Phe Ala Ala Tyr Lys Ala Val
305                 310                 315                 320

Ala Glu Ala Cys Gly Ser Gln Ala Val Ile Val Arg Thr Met Asp Ile
                325                 330                 335

Gly Gly Asp Lys Glu Leu Pro Tyr Met Asn Phe Pro Lys Glu Glu Asn
            340                 345                 350
```

Pro Phe Leu Gly Trp Arg Ala Ile Arg Ile Ala Met Asp Arg Arg Glu
355 360 365

Ile Leu Arg Asp Gln Leu Arg Ala Ile Leu Arg Ala Ser Ala Phe Gly
370 375 380

Lys Leu Arg Ile Met Phe Pro Met Ile Ile Ser Val Glu Glu Val Arg
385 390 395 400

Ala Leu Arg Lys Glu Ile Glu Ile Tyr Lys Gln Glu Leu Arg Asp Glu
405 410 415

Gly Lys Ala Phe Asp Glu Ser Ile Glu Ile Gly Val Met Val Glu Thr
420 425 430

Pro Ala Ala Ala Thr Ile Ala Arg His Leu Ala Lys Glu Val Asp Phe
435 440 445

Phe Ser Ile Gly Thr Asn Asp Leu Thr Gln Tyr Thr Leu Ala Val Asp
450 455 460

Arg Gly Asn Asp Met Ile Ser His Leu Tyr Gln Pro Met Ser Pro Ser
465 470 475 480

Val Leu Asn Leu Ile Lys Gln Val Ile Asp Ala Ser His Ala Glu Gly
485 490 495

Lys Trp Thr Gly Met Cys Gly Glu Leu Ala Gly Asp Glu Arg Ala Thr
500 505 510

Leu Leu Leu Leu Gly Met Gly Leu Asp Glu Phe Ser Met Ser Ala Ile
515 520 525

Ser Ile Pro Arg Ile Lys Lys Ile Ile Arg Asn Thr Asn Phe Glu Asp
530 535 540

Ala Lys Val Leu Ala Glu Gln Ala Leu Ala Gln Pro Thr Thr Asp Glu
545 550 555 560

Leu Met Thr Leu Val Asn Lys Phe Ile Glu Glu Lys Thr Ile Cys
565 570 575

<210> 177
<211> 510
<212> DNA
<213> Escherichia coli

<400> 177

atgggtttgt tcgataaact gaaatctctg gtttccgacg acaagaagga taccggaact 60

attgagatca ttgctccgct ctctggcgag atcgtcaata tcgaagacgt gccggatgtc 120

gtttttgcgg aaaaaatcgt tggtgatggt attgctatca aaccaacggg taacaaaatg 180

gtcgcgccag tagacggcac cattggtaaa atctttgaaa ccaaccacgc attctctatc 240

gaatctgata gcggcgttga actgttcgtc cacttcggta tcgacaccgt tgaactgaaa 300

ggcgaaggct tcaagcgtat tgctgaagaa ggtcagcgcg tgaaagttgg cgatactgtc 360

attgaatttg atctgccgct gctggaagag aaagccaagt ctaccctgac tccggttgtt 420

atctccaaca tggacgaaat caaagaactg atcaaactgt ccggtagcgt aaccgtgggt 480

gaaaccccgg ttatccgcat caagaagtaa 510

<210> 178
<211> 169
<212> PRT
<213> Escherichia coli

<400> 178

Met Gly Leu Phe Asp Lys Leu Lys Ser Leu Val Ser Asp Asp Lys Lys
1 5 10 15

Asp Thr Gly Thr Ile Glu Ile Ile Ala Pro Leu Ser Gly Glu Ile Val
20 25 30

Asn Ile Glu Asp Val Pro Asp Val Val Phe Ala Glu Lys Ile Val Gly
35 40 45

Asp Gly Ile Ala Ile Lys Pro Thr Gly Asn Lys Met Val Ala Pro Val
50 55 60

Asp Gly Thr Ile Gly Lys Ile Phe Glu Thr Asn His Ala Phe Ser Ile
65 70 75 80

Glu Ser Asp Ser Gly Val Glu Leu Phe Val His Phe Gly Ile Asp Thr
85 90 95

Val Glu Leu Lys Gly Glu Gly Phe Lys Arg Ile Ala Glu Glu Gly Gln
100 105 110

Arg Val Lys Val Gly Asp Thr Val Ile Glu Phe Asp Leu Pro Leu Leu
115 120 125

Glu Glu Lys Ala Lys Ser Thr Leu Thr Pro Val Val Ile Ser Asn Met
130 135 140

Asp Glu Ile Lys Glu Leu Ile Lys Leu Ser Gly Ser Val Thr Val Gly

145 150 155 160

Glu Thr Pro Val Ile Arg Ile Lys Lys
165

<210> 179
<211> 227
<212> DNA
<213> Escherichia coli

<400> 179
gatgaagcaa gggggtgccc catgcgtcag ttttatcagc actattttac cgcgacagcg 60

aagttgtgct ggttgcgttg gttaagcgtc ccacaacgat taaccatgct tgaaggactg 120

atgcagtggg atgaccgcaa ttctgaaagt tgacttgcct gcatcatgtg tgactgagta 180

ttggtgtaaa atcacccgcc agcagattat acctgctggt ttttttt 227

<210> 180
<211> 132
<212> DNA
<213> Escherichia coli

<400> 180
atgcgtcagt tttatcagca ctattttacc gcgacagcga agttgtgctg gttgcgttgg 60

ttaagcgtcc cacaacgatt aaccatgctt gaaggactga tgcagtggga tgaccgcaat 120

tctgaaagtt ga 132

<210> 181
<211> 43
<212> PRT
<213> Escherichia coli

<400> 181

Met Arg Gln Phe Tyr Gln His Tyr Phe Thr Ala Thr Ala Lys Leu Cys
1 5 10 15

Trp Leu Arg Trp Leu Ser Val Pro Gln Arg Leu Thr Met Leu Glu Gly
20 25 30

Leu Met Gln Trp Asp Asp Arg Asn Ser Glu Ser
35 40

<210> 182
<211> 1656
<212> DNA
<213> Escherichia coli

<400> 182
atgccatctg ctcgtctgca acaacagttc atccgcctgt ggcaatgctg cgagggtaaa 60

tcgcaggaca caacgctcaa cgaactggca gcgttattga gctgctcgcg tcgtcatatg 120

cgcaccctgc tcaacaccat gcaggatcgc ggctggctga cgtgggaagc ggaagtcggg 180

cgcggtaaac gctcgcgtct gacattcctc tataccgggc tggcgcttca gcaacagcgg 240

gcggaagacc tgctggagca ggatcgtatc gatcaactgg tgcagttggt tggcgacaaa 300

gcgactgtgc ggcaaatgct ggtttctcat ctgggccgca gcttccgcca ggggcggcac 360

atcctgcgcg tgctctacta tcgtccgttg cgtaatctgc tacctggcag cgcattgcgc 420

cgttccgaaa cccatatcgc ccggcaaatc ttcagttcgc taacgcgcat aaatgaggaa 480

aatggggaac tggaagcaga catcgcccac cactggcagc aaatttcacc gcttcactgg 540

cgtttctttt tgcgtccagg agtccatttt caccatggtc gtgaactgga aatggacgat 600

gtgatcgcct ctttaaaacg aatcaatacg ctgccgctct attcgcatat tgctgacatt 660

gtgtcgccga cgccctggac gctggatatc catctcacgc aaccggaccg ctggttaccg 720

ttactgctgg ggcaagttcc ggcgatgatc ctgccgcgcg aatgggaaac cctcagtaac 780

tttgccagcc atcccatcgg caccggtccg tatgcggtga ttcgcaacag caccaatcaa 840

ctgaaaattc aggcattcga tgacttcttc ggttaccggg cattaatcga cgaagttaac 900

gtctgggttc tgccggaaat tgccgacgag ccagccggag ggctgatgct aaaaggtcca 960

cagggcgagg aaaaagagat tgaaagccgc ctggaggaag gttgctacta tttactgttc 1020

gacagccgca cccatcgcgg ggcgaatcag caagtcaggg actgggtaag ctatgtgctt 1080

tctccaacta atctggtcta tttcgctgag gaacagtacc agcaactgtg gttcccggct 1140

tatggactgc tcccccgttg gcaccatgcc cgcaccataa agagcgaaaa accggctggc 1200

ctggaaagcc tcaccctaac cttttatcag gatcacagtg agcatcgggt gattgccggg 1260

atcatgcagc agattctggc aagtcaccag gtcacgctga aaatcaaaga gatcgactac 1320

gatcagtggc atacaggaga gatcgaaagt gatatctggc taaacagcgc caactttacc 1380

ctgccgctgg acttctctgt tttcgcacat ttatgcgaag tgccactgct acaacattgc 1440

attcccattg actggcaagc cgacgctgct cgctggcgca atggcgagat gaatctggcg 1500

aactggtgcc agcaactggt cgccagcaaa gcgatggtgc cattattgca ccactggctg 1560

atcattcagg ggcaacgcag tatgcgcggc ctgcgcatga ataccctcgg ctggttcgat 1620

tttaaatcag cgtggtttgc gccaccggat ccatga 1656

<210> 183
<211> 551
<212> PRT
<213> Escherichia coli

<400> 183

Met Pro Ser Ala Arg Leu Gln Gln Gln Phe Ile Arg Leu Trp Gln Cys
1 5 10 15

```
Cys Glu Gly Lys Ser Gln Asp Thr Thr Leu Asn Glu Leu Ala Ala Leu
            20                  25                  30

Leu Ser Cys Ser Arg Arg His Met Arg Thr Leu Leu Asn Thr Met Gln
        35                  40                  45

Asp Arg Gly Trp Leu Thr Trp Glu Ala Glu Val Gly Arg Gly Lys Arg
    50                  55                  60

Ser Arg Leu Thr Phe Leu Tyr Thr Gly Leu Ala Leu Gln Gln Gln Arg
65                  70                  75                  80

Ala Glu Asp Leu Leu Glu Gln Asp Arg Ile Asp Gln Leu Val Gln Leu
                85                  90                  95

Val Gly Asp Lys Ala Thr Val Arg Gln Met Leu Val Ser His Leu Gly
            100                 105                 110

Arg Ser Phe Arg Gln Gly Arg His Ile Leu Arg Val Leu Tyr Tyr Arg
        115                 120                 125

Pro Leu Arg Asn Leu Leu Pro Gly Ser Ala Leu Arg Arg Ser Glu Thr
    130                 135                 140

His Ile Ala Arg Gln Ile Phe Ser Ser Leu Thr Arg Ile Asn Glu Glu
145                 150                 155                 160

Asn Gly Glu Leu Glu Ala Asp Ile Ala His His Trp Gln Gln Ile Ser
                165                 170                 175

Pro Leu His Trp Arg Phe Phe Leu Arg Pro Gly Val His Phe His His
            180                 185                 190

Gly Arg Glu Leu Glu Met Asp Asp Val Ile Ala Ser Leu Lys Arg Ile
        195                 200                 205

Asn Thr Leu Pro Leu Tyr Ser His Ile Ala Asp Ile Val Ser Pro Thr
    210                 215                 220

Pro Trp Thr Leu Asp Ile His Leu Thr Gln Pro Asp Arg Trp Leu Pro
225                 230                 235                 240

Leu Leu Leu Gly Gln Val Pro Ala Met Ile Leu Pro Arg Glu Trp Glu
                245                 250                 255

Thr Leu Ser Asn Phe Ala Ser His Pro Ile Gly Thr Gly Pro Tyr Ala
```

260 265 270

Val Ile Arg Asn Ser Thr Asn Gln Leu Lys Ile Gln Ala Phe Asp Asp
275 280 285

Phe Phe Gly Tyr Arg Ala Leu Ile Asp Glu Val Asn Val Trp Val Leu
290 295 300

Pro Glu Ile Ala Asp Glu Pro Ala Gly Gly Leu Met Leu Lys Gly Pro
305 310 315 320

Gln Gly Glu Glu Lys Glu Ile Glu Ser Arg Leu Glu Glu Gly Cys Tyr
325 330 335

Tyr Leu Leu Phe Asp Ser Arg Thr His Arg Gly Ala Asn Gln Gln Val
340 345 350

Arg Asp Trp Val Ser Tyr Val Leu Ser Pro Thr Asn Leu Val Tyr Phe
355 360 365

Ala Glu Glu Gln Tyr Gln Gln Leu Trp Phe Pro Ala Tyr Gly Leu Leu
370 375 380

Pro Arg Trp His His Ala Arg Thr Ile Lys Ser Glu Lys Pro Ala Gly
385 390 395 400

Leu Glu Ser Leu Thr Leu Thr Phe Tyr Gln Asp His Ser Glu His Arg
405 410 415

Val Ile Ala Gly Ile Met Gln Gln Ile Leu Ala Ser His Gln Val Thr
420 425 430

Leu Lys Ile Lys Glu Ile Asp Tyr Asp Gln Trp His Thr Gly Glu Ile
435 440 445

Glu Ser Asp Ile Trp Leu Asn Ser Ala Asn Phe Thr Leu Pro Leu Asp
450 455 460

Phe Ser Val Phe Ala His Leu Cys Glu Val Pro Leu Leu Gln His Cys
465 470 475 480

Ile Pro Ile Asp Trp Gln Ala Asp Ala Ala Arg Trp Arg Asn Gly Glu
485 490 495

Met Asn Leu Ala Asn Trp Cys Gln Gln Leu Val Ala Ser Lys Ala Met
500 505 510

Val Pro Leu Leu His His Trp Leu Ile Ile Gln Gly Gln Arg Ser Met
515 520 525

Arg Gly Leu Arg Met Asn Thr Leu Gly Trp Phe Asp Phe Lys Ser Ala
530 535 540

Trp Phe Ala Pro Pro Asp Pro
545 550

<210> 184
<211> 1395
<212> DNA
<213> Escherichia coli

<400> 184
atgcctgacg ctaaaaaaca ggggcggtca aacaaggcaa tgacgttttt cgtctgcttc 60

cttgccgctc tggcgggatt actctttggc ctggatatcg gtgtaattgc tggcgcactg 120

ccgtttattg cagatgaatt ccagattact tcgcacacgc aagaatgggt cgtaagctcc 180

atgatgttcg gtgcggcagt cggtgcggtg ggcagcggct ggctctcctt taaactcggg 240

cgcaaaaaga gcctgatgat cggcgcaatt ttgtttgttg ccggttcgct gttctctgcg 300

gctgcgccaa acgttgaagt actgattctt tcccgcgttc tactggggct ggcggtgggt 360

gtggcctctt ataccgcacc gctgtacctc tctgaaattg cgccggaaaa aattcgtggc 420

agtatgatct cgatgtatca gttgatgatc actatcggga tcctcggtgc ttatctttct 480

gataccgcct tcagctacac cggtgcatgg cgctggatgc tgggtgtgat tatcatcccg 540

gcaattttgc tgctgattgg tgtcttcttc ctgccagaca gcccacgttg gtttgccgcc 600

aaacgccgtt ttgttgatgc cgaacgcgtg ctgctacgcc tgcgtgacac cagcgcggaa 660

gcgaaacgcg aactggatga aatccgtgaa agtttgcagg ttaaacagag tggctgggcg 720

ctgtttaaag agaacagcaa cttccgccgc gcggtgttcc ttggcgtact gttgcaggta 780

atgcagcaat tcaccgggat gaacgtcatc atgtattacg cgccgaaaat cttcgaactg 840

gcgggttata ccaacactac cgagcaaatg tgggggaccg tgattgtcgg cctgaccaac 900

gtacttgcca cctttatcgc aatcggcctt gttgaccgct ggggacgtaa accaacgcta 960

acgctgggct tcctggtgat ggctgctggc atgggcgtac tcggtacaat gatgcatatc 1020

ggtattcact ctccgtcggc gcagtatttc gccatcgcca tgctgctgat gtttattgtc 1080

ggttttgcca tgagtgccgg tccgctgatt tgggtactgt gctccgaaat tcagccgctg 1140

aaaggccgcg attttggcat cacctgctcc actgccacca actggattgc caacatgatc 1200

gttggcgcaa cgttcctgac catgctcaac acgctgggta acgccaacac cttctgggtg 1260

tatgcggctc tgaacgtact gtttatcctg ctgacattgt ggctggtacc ggaaaccaaa 1320

cacgtttcgc tggaacatat tgaacgtaat ctgatgaaag gtcgtaaact gcgcgaaata 1380

ggcgctcacg attaa 1395

<210> 185
<211> 464
<212> PRT
<213> Escherichia coli

<400> 185

Met Pro Asp Ala Lys Lys Gln Gly Arg Ser Asn Lys Ala Met Thr Phe
1 5 10 15

Phe Val Cys Phe Leu Ala Ala Leu Ala Gly Leu Leu Phe Gly Leu Asp
20 25 30

Ile Gly Val Ile Ala Gly Ala Leu Pro Phe Ile Ala Asp Glu Phe Gln
35 40 45

Ile Thr Ser His Thr Gln Glu Trp Val Val Ser Ser Met Met Phe Gly
50 55 60

Ala Ala Val Gly Ala Val Gly Ser Gly Trp Leu Ser Phe Lys Leu Gly
65 70 75 80

Arg Lys Lys Ser Leu Met Ile Gly Ala Ile Leu Phe Val Ala Gly Ser
85 90 95

Leu Phe Ser Ala Ala Ala Pro Asn Val Glu Val Leu Ile Leu Ser Arg
100 105 110

Val Leu Leu Gly Leu Ala Val Gly Val Ala Ser Tyr Thr Ala Pro Leu
115 120 125

Tyr Leu Ser Glu Ile Ala Pro Glu Lys Ile Arg Gly Ser Met Ile Ser
130 135 140

Met Tyr Gln Leu Met Ile Thr Ile Gly Ile Leu Gly Ala Tyr Leu Ser
145 150 155 160

Asp Thr Ala Phe Ser Tyr Thr Gly Ala Trp Arg Trp Met Leu Gly Val
165 170 175

Ile Ile Ile Pro Ala Ile Leu Leu Leu Ile Gly Val Phe Phe Leu Pro
180 185 190

Asp Ser Pro Arg Trp Phe Ala Ala Lys Arg Arg Phe Val Asp Ala Glu
195 200 205

```
Arg Val Leu Leu Arg Leu Arg Asp Thr Ser Ala Glu Ala Lys Arg Glu
    210                 215                 220

Leu Asp Glu Ile Arg Glu Ser Leu Gln Val Lys Gln Ser Gly Trp Ala
225                 230                 235                 240

Leu Phe Lys Glu Asn Ser Asn Phe Arg Arg Ala Val Phe Leu Gly Val
                245                 250                 255

Leu Leu Gln Val Met Gln Gln Phe Thr Gly Met Asn Val Ile Met Tyr
            260                 265                 270

Tyr Ala Pro Lys Ile Phe Glu Leu Ala Gly Tyr Thr Asn Thr Thr Glu
        275                 280                 285

Gln Met Trp Gly Thr Val Ile Val Gly Leu Thr Asn Val Leu Ala Thr
    290                 295                 300

Phe Ile Ala Ile Gly Leu Val Asp Arg Trp Gly Arg Lys Pro Thr Leu
305                 310                 315                 320

Thr Leu Gly Phe Leu Val Met Ala Ala Gly Met Gly Val Leu Gly Thr
                325                 330                 335

Met Met His Ile Gly Ile His Ser Pro Ser Ala Gln Tyr Phe Ala Ile
            340                 345                 350

Ala Met Leu Leu Met Phe Ile Val Gly Phe Ala Met Ser Ala Gly Pro
        355                 360                 365

Leu Ile Trp Val Leu Cys Ser Glu Ile Gln Pro Leu Lys Gly Arg Asp
    370                 375                 380

Phe Gly Ile Thr Cys Ser Thr Ala Thr Asn Trp Ile Ala Asn Met Ile
385                 390                 395                 400

Val Gly Ala Thr Phe Leu Thr Met Leu Asn Thr Leu Gly Asn Ala Asn
                405                 410                 415

Thr Phe Trp Val Tyr Ala Ala Leu Asn Val Leu Phe Ile Leu Leu Thr
            420                 425                 430

Leu Trp Leu Val Pro Glu Thr Lys His Val Ser Leu Glu His Ile Glu
        435                 440                 445

Arg Asn Leu Met Lys Gly Arg Lys Leu Arg Glu Ile Gly Ala His Asp
    450                 455                 460
```

<210> 186
<211> 966
<212> DNA
<213> Escherichia coli

<400> 186
atgacaaagt atgcattagt cggtgatgtg ggcggcacca acgcacgtct tgctctgtgt 60

gatattgcca gtggtgaaat ctcgcaggct aagacctatt cagggcttga ttaccccagc 120

ctcgaagcgg tcattcgcgt ttatcttgaa gaacataagg tcgaggtgaa agacggctgt 180

attgccatcg cttgcccaat taccggtgac tgggtggcga tgaccaacca tacctgggcg 240

ttctcaattg ccgaaatgaa aaagaatctc ggttttagcc atctggaaat tattaacgat 300

tttaccgctg tatcgatggc gatcccgatg ctgaaaaaag agcatctgat tcagtttggt 360

ggcgcagaac cggtcgaagg taagcctatt gcggtttacg gtgccggaac ggggcttggg 420

gttgcgcatc tggtccatgt cgataagcgt tgggtaagct tgccaggcga aggcggtcac 480

gttgattttg cgccgaatag tgaagaagag gccattatcc tcgaaatatt gcgtgcggaa 540

attggtcatg tttcggcgga gcgcgtgctt tctggccctg ggctggtgaa tttgtatcgc 600

gcaattgtga aagctgacaa ccgcctgcca gaaaatctca agccaaaaga tattaccgaa 660

cgcgcgctgg ctgacagctg caccgattgc cgccgcgcat tgtcgctgtt ttgcgtcatt 720

atgggccgtt ttggcggcaa tctggcgctc aatctcggga catttggcgg cgtgtttatt 780

gcgggcggta tcgtgccgcg cttccttgag ttcttcaaag cctccggttt ccgtgccgca 840

tttgaagata aagggcgctt taaagaatat gtccatgata ttccggtgta tctcatcgtc 900

catgacaatc cgggccttct cggttccggt gcacatttac gccagacctt aggtcacatt 960

ctgtaa 966

<210> 187
<211> 321
<212> PRT
<213> Escherichia coli

<400> 187

Met Thr Lys Tyr Ala Leu Val Gly Asp Val Gly Gly Thr Asn Ala Arg
1               5                   10                  15

Leu Ala Leu Cys Asp Ile Ala Ser Gly Glu Ile Ser Gln Ala Lys Thr
            20                  25                  30

Tyr Ser Gly Leu Asp Tyr Pro Ser Leu Glu Ala Val Ile Arg Val Tyr
        35                  40                  45

Leu Glu Glu His Lys Val Glu Val Lys Asp Gly Cys Ile Ala Ile Ala
    50                  55                  60

Cys Pro Ile Thr Gly Asp Trp Val Ala Met Thr Asn His Thr Trp Ala
65 70 75 80

Phe Ser Ile Ala Glu Met Lys Lys Asn Leu Gly Phe Ser His Leu Glu
85 90 95

Ile Ile Asn Asp Phe Thr Ala Val Ser Met Ala Ile Pro Met Leu Lys
100 105 110

Lys Glu His Leu Ile Gln Phe Gly Gly Ala Glu Pro Val Glu Gly Lys
115 120 125

Pro Ile Ala Val Tyr Gly Ala Gly Thr Gly Leu Gly Val Ala His Leu
130 135 140

Val His Val Asp Lys Arg Trp Val Ser Leu Pro Gly Glu Gly Gly His
145 150 155 160

Val Asp Phe Ala Pro Asn Ser Glu Glu Glu Ala Ile Ile Leu Glu Ile
165 170 175

Leu Arg Ala Glu Ile Gly His Val Ser Ala Glu Arg Val Leu Ser Gly
180 185 190

Pro Gly Leu Val Asn Leu Tyr Arg Ala Ile Val Lys Ala Asp Asn Arg
195 200 205

Leu Pro Glu Asn Leu Lys Pro Lys Asp Ile Thr Glu Arg Ala Leu Ala
210 215 220

Asp Ser Cys Thr Asp Cys Arg Arg Ala Leu Ser Leu Phe Cys Val Ile
225 230 235 240

Met Gly Arg Phe Gly Gly Asn Leu Ala Leu Asn Leu Gly Thr Phe Gly
245 250 255

Gly Val Phe Ile Ala Gly Gly Ile Val Pro Arg Phe Leu Glu Phe Phe
260 265 270

Lys Ala Ser Gly Phe Arg Ala Ala Phe Glu Asp Lys Gly Arg Phe Lys
275 280 285

Glu Tyr Val His Asp Ile Pro Val Tyr Leu Ile Val His Asp Asn Pro
290 295 300

Gly Leu Leu Gly Ser Gly Ala His Leu Arg Gln Thr Leu Gly His Ile

305 310 315 320

Leu

<210> 188
<211> 801
<212> DNA
<213> Escherichia coli

<400> 188
atgtaccagg ttgttgcgtc tgatttagat ggcacgttac tttctcccga ccatacgtta 60

tccccttacg ccaaagaaac tctgaagctg ctcaccgcgc gcggcatcaa ctttgtgttt 120

gcgaccggtc gtcaccacgt tgatgtgggg caaattcgcg ataatctgga gattaagtct 180

tacatgatta cctccaatgg tgcgcgcgtt cacgatctgg atggtaatct gatttttgct 240

cataacctgg atcgcgacat tgccagcgat ctgtttggcg tagtcaacga caatccggac 300

atcattacta acgtttatcg cgacgacgaa tggtttatga atcgccatcg cccggaagag 360

atgcgctttt ttaaagaagc ggtgttccaa tatgcgctgt atgagcctgg attactggag 420

ccggaaggcg tcagcaaagt gttcttcacc tgcgattccc atgaacaact gctgccgctg 480

gagcaggcga ttaacgctcg ttggggcgat cgcgtcaacg tcagtttctc taccttaacc 540

tgtctggaag tgatggcggg cggcgtttca aaaggccatg cgctggaagc ggtggcgaag 600

aaactgggct acagcctgaa ggattgtatt gcgtttggtg acgggatgaa cgacgccgaa 660

atgctgtcga tggcggggaa aggctgcatt atgggcagtg cgcaccagcg tctgaaagac 720

cttcatcccg agctggaagt gattggtact aatgccgacg acgcggtgcc gcattatctg 780

cgtaaactct atttatcgta a 801

<210> 189
<211> 266
<212> PRT
<213> Escherichia coli

<400> 189

Met Tyr Gln Val Val Ala Ser Asp Leu Asp Gly Thr Leu Leu Ser Pro
1 5 10 15

Asp His Thr Leu Ser Pro Tyr Ala Lys Glu Thr Leu Lys Leu Leu Thr
20 25 30

Ala Arg Gly Ile Asn Phe Val Phe Ala Thr Gly Arg His His Val Asp
35 40 45

Val Gly Gln Ile Arg Asp Asn Leu Glu Ile Lys Ser Tyr Met Ile Thr
50 55 60

Ser Asn Gly Ala Arg Val His Asp Leu Asp Gly Asn Leu Ile Phe Ala
65 70 75 80

His Asn Leu Asp Arg Asp Ile Ala Ser Asp Leu Phe Gly Val Val Asn
85 90 95

Asp Asn Pro Asp Ile Ile Thr Asn Val Tyr Arg Asp Asp Glu Trp Phe
100 105 110

Met Asn Arg His Arg Pro Glu Glu Met Arg Phe Phe Lys Glu Ala Val
115 120 125

Phe Gln Tyr Ala Leu Tyr Glu Pro Gly Leu Leu Glu Pro Glu Gly Val
130 135 140

Ser Lys Val Phe Phe Thr Cys Asp Ser His Glu Gln Leu Leu Pro Leu
145 150 155 160

Glu Gln Ala Ile Asn Ala Arg Trp Gly Asp Arg Val Asn Val Ser Phe
165 170 175

Ser Thr Leu Thr Cys Leu Glu Val Met Ala Gly Gly Val Ser Lys Gly
180 185 190

His Ala Leu Glu Ala Val Ala Lys Lys Leu Gly Tyr Ser Leu Lys Asp
195 200 205

Cys Ile Ala Phe Gly Asp Gly Met Asn Asp Ala Glu Met Leu Ser Met
210 215 220

Ala Gly Lys Gly Cys Ile Met Gly Ser Ala His Gln Arg Leu Lys Asp
225 230 235 240

Leu His Pro Glu Leu Glu Val Ile Gly Thr Asn Ala Asp Asp Ala Val
245 250 255

Pro His Tyr Leu Arg Lys Leu Tyr Leu Ser
260 265

<210> 190
<211> 1533
<212> DNA
<213> Escherichia coli

<400> 190
atgcgaattg gcataccaag agaacggtta accaatgaaa cccgtgttgc agcaacgcca 60

aaaacagtgg aacagctgct gaaactgggt tttaccgtcg cggtagagag cggcgcgggt 120

caactggcaa gttttgacga taaagcgttt gtgcaagcgg gcgctgaaat tgtagaaggg 180

aatagcgtct ggcagtcaga gatcattctg aaggtcaatg cgccgttaga tgatgaaatt 240

gcgttactga atcctgggac aacgctggtg agttttatct ggcctgcgca gaatccggaa 300

ttaatgcaaa aacttgcgga acgtaacgtg accgtgatgg cgatggactc tgtgccgcgt 360

atctcacgcg cacaatcgct ggacgcacta agctcgatgg cgaacatcgc cggttatcgc 420

gccattgttg aagcggcaca tgaatttggg cgcttcttta ccgggcaaat tactgcggcc 480

gggaaagtgc caccggcaaa agtgatggtg attggtgcgg gtgttgcagg tctggccgcc 540

attggcgcag caaacagtct cggcgcgatt gtgcgtgcat tcgacacccg cccggaagtg 600

aaagaacaag ttcaaagtat gggcgcggaa ttcctcgagc tggattttaa agaggaagct 660

ggcagcggcg atggctatgc caaagtgatg tcggacgcgt tcatcaaagc ggaaatggaa 720

ctctttgccg cccaggcaaa agaggtcgat atcattgtca ccaccgcgct tattccaggc 780

aaaccagcgc cgaagctaat tacccgtgaa atggttgact ccatgaaggc gggcagtgtg 840

attgtcgacc tggcagccca aaacggcggc aactgtgaat acaccgtgcc gggtgaaatc 900

ttcactacgg aaaatggtgt caaagtgatt ggttataccg atcttccggg ccgtctgccg 960

acgcaatcct cacagcttta cggcacaaac ctcgttaatc tgctgaaact gttgtgcaaa 1020

gagaaagacg gcaatatcac tgttgatttt gatgatgtgg tgattcgcgg cgtgaccgtg 1080

atccgtgcgg gcgaaattac ctggccggca ccgccgattc aggtatcagc tcagccgcag 1140

gcggcacaaa aagcggcacc ggaagtgaaa actgaggaaa aatgtacctg ctcaccgtgg 1200

cgtaaatacg cgttgatggc gctggcaatc attcttttg gctggatggc aagcgttgcg 1260

ccgaaagaat tccttgggca cttcaccgtt ttcgcgctgg cctgcgttgt cggttattac 1320

gtggtgtgga atgtatcgca cgcgctgcat acaccgttga tgtcggtcac caacgcgatt 1380

tcagggatta ttgttgtcgg agcactgttg cagattggcc agggcggctg ggttagcttc 1440

cttagtttta tcgcggtgct tatagccagc attaatattt tcggtggctt caccgtgact 1500

cagcgcatgc tgaaaatgtt ccgcaaaaat taa 1533

<210> 191
<211> 510
<212> PRT
<213> Escherichia coli

<400> 191

Met Arg Ile Gly Ile Pro Arg Glu Arg Leu Thr Asn Glu Thr Arg Val
1 5 10 15

Ala Ala Thr Pro Lys Thr Val Glu Gln Leu Leu Lys Leu Gly Phe Thr
20 25 30

Val Ala Val Glu Ser Gly Ala Gly Gln Leu Ala Ser Phe Asp Asp Lys
35 40 45

Ala Phe Val Gln Ala Gly Ala Glu Ile Val Glu Gly Asn Ser Val Trp
50 55 60

Gln Ser Glu Ile Ile Leu Lys Val Asn Ala Pro Leu Asp Asp Glu Ile
65 70 75 80

Ala Leu Leu Asn Pro Gly Thr Thr Leu Val Ser Phe Ile Trp Pro Ala
85 90 95

Gln Asn Pro Glu Leu Met Gln Lys Leu Ala Glu Arg Asn Val Thr Val
100 105 110

Met Ala Met Asp Ser Val Pro Arg Ile Ser Arg Ala Gln Ser Leu Asp
115 120 125

Ala Leu Ser Ser Met Ala Asn Ile Ala Gly Tyr Arg Ala Ile Val Glu
130 135 140

Ala Ala His Glu Phe Gly Arg Phe Phe Thr Gly Gln Ile Thr Ala Ala
145 150 155 160

Gly Lys Val Pro Pro Ala Lys Val Met Val Ile Gly Ala Gly Val Ala
165 170 175

Gly Leu Ala Ala Ile Gly Ala Ala Asn Ser Leu Gly Ala Ile Val Arg
180 185 190

Ala Phe Asp Thr Arg Pro Glu Val Lys Glu Gln Val Gln Ser Met Gly
195 200 205

Ala Glu Phe Leu Glu Leu Asp Phe Lys Glu Glu Ala Gly Ser Gly Asp
210 215 220

Gly Tyr Ala Lys Val Met Ser Asp Ala Phe Ile Lys Ala Glu Met Glu
225 230 235 240

Leu Phe Ala Ala Gln Ala Lys Glu Val Asp Ile Ile Val Thr Thr Ala
245 250 255

Leu Ile Pro Gly Lys Pro Ala Pro Lys Leu Ile Thr Arg Glu Met Val
260 265 270

Asp Ser Met Lys Ala Gly Ser Val Ile Val Asp Leu Ala Ala Gln Asn

275 280 285

Gly Gly Asn Cys Glu Tyr Thr Val Pro Gly Glu Ile Phe Thr Thr Glu
290 295 300

Asn Gly Val Lys Val Ile Gly Tyr Thr Asp Leu Pro Gly Arg Leu Pro
305 310 315 320

Thr Gln Ser Ser Gln Leu Tyr Gly Thr Asn Leu Val Asn Leu Leu Lys
325 330 335

Leu Leu Cys Lys Glu Lys Asp Gly Asn Ile Thr Val Asp Phe Asp Asp
340 345 350

Val Val Ile Arg Gly Val Thr Val Ile Arg Ala Gly Glu Ile Thr Trp
355 360 365

Pro Ala Pro Pro Ile Gln Val Ser Ala Gln Pro Gln Ala Ala Gln Lys
370 375 380

Ala Ala Pro Glu Val Lys Thr Glu Glu Lys Cys Thr Cys Ser Pro Trp
385 390 395 400

Arg Lys Tyr Ala Leu Met Ala Leu Ala Ile Ile Leu Phe Gly Trp Met
405 410 415

Ala Ser Val Ala Pro Lys Glu Phe Leu Gly His Phe Thr Val Phe Ala
420 425 430

Leu Ala Cys Val Val Gly Tyr Tyr Val Val Trp Asn Val Ser His Ala
435 440 445

Leu His Thr Pro Leu Met Ser Val Thr Asn Ala Ile Ser Gly Ile Ile
450 455 460

Val Val Gly Ala Leu Leu Gln Ile Gly Gln Gly Gly Trp Val Ser Phe
465 470 475 480

Leu Ser Phe Ile Ala Val Leu Ile Ala Ser Ile Asn Ile Phe Gly Gly
485 490 495

Phe Thr Val Thr Gln Arg Met Leu Lys Met Phe Arg Lys Asn
500 505 510

<210> 192
<211> 1389
<212> DNA
<213> Escherichia coli

<400> 192
atgtctggag gattagttac agctgcatac attgttgccg cgatcctgtt tatcttcagt 60

ctggccggtc tttcgaaaca tgaaacgtct cgccagggta acaacttcgg tatcgccggg 120

atggcgattg cgttaatcgc aaccattttt ggaccggata cgggtaatgt tggctggatc 180

ttgctggcga tggtcattgg tggggcaatt ggtatccgtc tggcgaagaa agttgaaatg 240

accgaaatgc cagaactggt ggcgatcctg catagcttcg tgggtctggc ggcagtgctg 300

gttggcttta acagctatct gcatcatgac gcgggaatgg caccgattct ggtcaatatt 360

cacctgacgg aagtgttcct cggtatcttc atcggggcgg taacgttcac gggttcggtg 420

gtggcgttcg gcaaactgtg tggcaagatt tcgtctaaac cattgatgct gccaaaccgt 480

cacaaaatga acctggcggc tctggtcgtt tccttcctgc tgctgattgt atttgttcgc 540

acggacagcg tcggcctgca agtgctggca ttgctgataa tgaccgcaat tgcgctggta 600

ttcggctggc atttagtcgc ctccatcggt ggtgcagata tgccagtggt ggtgtcgatg 660

ctgaactcgt actccggctg ggcggctgcg gctgcgggct ttatgctcag caacgacctg 720

ctgattgtga ccggtgcgct ggtcggttct tcgggggcta tcctttctta cattatgtgt 780

aaggcgatga accgttcctt tatcagcgtt attgcgggtg gtttcggcac cgacggctct 840

tctactggcg atgatcagga agtgggtgag caccgcgaaa tcaccgcaga agagacagcg 900

gaactgctga aaaactccca ttcagtgatc attactccgg ggtacggcat ggcagtcgcg 960

caggcgcaat atcctgtcgc tgaaattact gagaaattgc gcgctcgtgg tattaatgtg 1020

cgtttcggta tccacccggt cgcggggcgt ttgcctggac atatgaacgt attgctggct 1080

gaagcaaaag taccgtatga catcgtgctg gaaatggacg agatcaatga tgactttgct 1140

gataccgata ccgtactggt gattggtgct aacgatacgg ttaacccggc ggcgcaggat 1200

gatccgaaga gtccgattgc tggtatgcct gtgctggaag tgtggaaagc gcagaacgtg 1260

attgtcttta aacgttcgat gaacactggc tatgctggtg tgcaaaaccc gctgttcttc 1320

aaggaaaaca cccacatgct gtttggtgac gccaaagcca gcgtggatgc aatcctgaaa 1380

gctctgtaa 1389

<210> 193
<211> 462
<212> PRT
<213> Escherichia coli

<400> 193

Met Ser Gly Gly Leu Val Thr Ala Ala Tyr Ile Val Ala Ala Ile Leu
1 5 10 15

Phe Ile Phe Ser Leu Ala Gly Leu Ser Lys His Glu Thr Ser Arg Gln

```
            20                  25                  30

Gly Asn Asn Phe Gly Ile Ala Gly Met Ala Ile Ala Leu Ile Ala Thr
        35                  40                  45

Ile Phe Gly Pro Asp Thr Gly Asn Val Gly Trp Ile Leu Leu Ala Met
    50                  55                  60

Val Ile Gly Gly Ala Ile Gly Ile Arg Leu Ala Lys Lys Val Glu Met
65                  70                  75                  80

Thr Glu Met Pro Glu Leu Val Ala Ile Leu His Ser Phe Val Gly Leu
                85                  90                  95

Ala Ala Val Leu Val Gly Phe Asn Ser Tyr Leu His His Asp Ala Gly
            100                 105                 110

Met Ala Pro Ile Leu Val Asn Ile His Leu Thr Glu Val Phe Leu Gly
        115                 120                 125

Ile Phe Ile Gly Ala Val Thr Phe Thr Gly Ser Val Val Ala Phe Gly
    130                 135                 140

Lys Leu Cys Gly Lys Ile Ser Ser Lys Pro Leu Met Leu Pro Asn Arg
145                 150                 155                 160

His Lys Met Asn Leu Ala Ala Leu Val Val Ser Phe Leu Leu Leu Ile
                165                 170                 175

Val Phe Val Arg Thr Asp Ser Val Gly Leu Gln Val Leu Ala Leu Leu
            180                 185                 190

Ile Met Thr Ala Ile Ala Leu Val Phe Gly Trp His Leu Val Ala Ser
        195                 200                 205

Ile Gly Gly Ala Asp Met Pro Val Val Val Ser Met Leu Asn Ser Tyr
    210                 215                 220

Ser Gly Trp Ala Ala Ala Ala Ala Gly Phe Met Leu Ser Asn Asp Leu
225                 230                 235                 240

Leu Ile Val Thr Gly Ala Leu Val Gly Ser Ser Gly Ala Ile Leu Ser
                245                 250                 255

Tyr Ile Met Cys Lys Ala Met Asn Arg Ser Phe Ile Ser Val Ile Ala
            260                 265                 270
```

Gly Gly Phe Gly Thr Asp Gly Ser Ser Thr Gly Asp Asp Gln Glu Val
275 280 285

Gly Glu His Arg Glu Ile Thr Ala Glu Glu Thr Ala Glu Leu Leu Lys
290 295 300

Asn Ser His Ser Val Ile Ile Thr Pro Gly Tyr Gly Met Ala Val Ala
305 310 315 320

Gln Ala Gln Tyr Pro Val Ala Glu Ile Thr Glu Lys Leu Arg Ala Arg
325 330 335

Gly Ile Asn Val Arg Phe Gly Ile His Pro Val Ala Gly Arg Leu Pro
340 345 350

Gly His Met Asn Val Leu Leu Ala Glu Ala Lys Val Pro Tyr Asp Ile
355 360 365

Val Leu Glu Met Asp Glu Ile Asn Asp Asp Phe Ala Asp Thr Asp Thr
370 375 380

Val Leu Val Ile Gly Ala Asn Asp Thr Val Asn Pro Ala Ala Gln Asp
385 390 395 400

Asp Pro Lys Ser Pro Ile Ala Gly Met Pro Val Leu Glu Val Trp Lys
405 410 415

Ala Gln Asn Val Ile Val Phe Lys Arg Ser Met Asn Thr Gly Tyr Ala
420 425 430

Gly Val Gln Asn Pro Leu Phe Phe Lys Glu Asn Thr His Met Leu Phe
435 440 445

Gly Asp Ala Lys Ala Ser Val Asp Ala Ile Leu Lys Ala Leu
450 455 460

<210> 194
<211> 1401
<212> DNA
<213> Escherichia coli

<400> 194
atgccacatt cctacgatta cgatgccata gtaataggtt ccggccccgg cggcgaaggc 60

gctgcaatgg gcctggttaa gcaaggtgcg cgcgtcgcag ttatcgagcg ttatcaaaat 120

gttggcggcg gttgcaccca ctggggcacc atcccgtcga aagctctccg tcacgccgtc 180

agccgcatta tagaattcaa tcaaaaccca ctttacagcg accattcccg actgctccgc 240

tcttcttttg ccgatatcct taaccatgcc gataacgtga ttaatcaaca aacgcgcatg 300

cgtcagggat tttacgaacg taatcactgt gaaatattgc agggaaacgc tcgctttgtt 360

gacgagcata cgttggcgct ggattgcccg gacggcagcg ttgaaacact aaccgctgaa 420

aaatttgtta ttgcctgcgg ctctcgtcca tatcatccaa cagatgttga tttcacccat 480

ccacgcattt acgacagcga ctcaattctc agcatgcacc acgaaccgcg ccatgtactt 540

atctatggtg ctggagtgat cggctgtgaa tatgcgtcga tcttccgcgg tatggatgta 600

aaagtggatc tgatcaacac ccgcgatcgc ctgctggcat ttctcgatca agagatgtca 660

gattctctct cctatcactt ctggaacagt ggcgtagtga ttcgtcacaa cgaagagtac 720

gagaagatcg aaggctgtga cgatggtgtg atcatgcatc tgaagtcggg taaaaaactg 780

aaagctgact gcctgctcta tgccaacggt cgcaccggta ataccgattc gctggcgtta 840

cagaacattg ggctagaaac tgacagccgc ggacagctga aggtcaacag catgtatcag 900

accgcacagc cacacgttta cgcggtgggc gacgtgattg gttatccgag cctggcgtcg 960

gcggcctatg accaggggcg cattgccgcg caggcgctgg taaaaggcga agccaccgca 1020

catctgattg aagatatccc taccggtatt tacaccatcc cggaaatcag ctctgtgggc 1080

aaaaccgaac agcagctgac cgcaatgaaa gtgccatatg aagtgggccg cgcccagttt 1140

aaacatctgg cacgcgcaca aatcgtcggc atgaacgtgg gcacgctgaa aattttgttc 1200

catcgggaaa caaaagagat tctgggtatt cactgctttg gcgagcgcgc tgccgaaatt 1260

attcatatcg gtcaggcgat tatggaacag aaaggtggcg gcaacactat tgagtacttc 1320

gtcaacacca cctttaacta cccgacgatg gcggaagcct atcgggtagc tgcgttaaac 1380

ggtttaaacc gcctgtttta a 1401

<210> 195
<211> 466
<212> PRT
<213> Escherichia coli

<400> 195

Met Pro His Ser Tyr Asp Tyr Asp Ala Ile Val Ile Gly Ser Gly Pro
1 5 10 15

Gly Gly Glu Gly Ala Ala Met Gly Leu Val Lys Gln Gly Ala Arg Val
20 25 30

Ala Val Ile Glu Arg Tyr Gln Asn Val Gly Gly Gly Cys Thr His Trp
35 40 45

Gly Thr Ile Pro Ser Lys Ala Leu Arg His Ala Val Ser Arg Ile Ile
50 55 60

```
Glu Phe Asn Gln Asn Pro Leu Tyr Ser Asp His Ser Arg Leu Leu Arg
65                  70                  75                  80

Ser Ser Phe Ala Asp Ile Leu Asn His Ala Asp Asn Val Ile Asn Gln
                85                  90                  95

Gln Thr Arg Met Arg Gln Gly Phe Tyr Glu Arg Asn His Cys Glu Ile
            100                 105                 110

Leu Gln Gly Asn Ala Arg Phe Val Asp Glu His Thr Leu Ala Leu Asp
        115                 120                 125

Cys Pro Asp Gly Ser Val Glu Thr Leu Thr Ala Glu Lys Phe Val Ile
    130                 135                 140

Ala Cys Gly Ser Arg Pro Tyr His Pro Thr Asp Val Asp Phe Thr His
145                 150                 155                 160

Pro Arg Ile Tyr Asp Ser Asp Ser Ile Leu Ser Met His His Glu Pro
                165                 170                 175

Arg His Val Leu Ile Tyr Gly Ala Gly Val Ile Gly Cys Glu Tyr Ala
            180                 185                 190

Ser Ile Phe Arg Gly Met Asp Val Lys Val Asp Leu Ile Asn Thr Arg
        195                 200                 205

Asp Arg Leu Leu Ala Phe Leu Asp Gln Glu Met Ser Asp Ser Leu Ser
    210                 215                 220

Tyr His Phe Trp Asn Ser Gly Val Val Ile Arg His Asn Glu Glu Tyr
225                 230                 235                 240

Glu Lys Ile Glu Gly Cys Asp Asp Gly Val Ile Met His Leu Lys Ser
                245                 250                 255

Gly Lys Lys Leu Lys Ala Asp Cys Leu Leu Tyr Ala Asn Gly Arg Thr
            260                 265                 270

Gly Asn Thr Asp Ser Leu Ala Leu Gln Asn Ile Gly Leu Glu Thr Asp
        275                 280                 285

Ser Arg Gly Gln Leu Lys Val Asn Ser Met Tyr Gln Thr Ala Gln Pro
    290                 295                 300

His Val Tyr Ala Val Gly Asp Val Ile Gly Tyr Pro Ser Leu Ala Ser
305                 310                 315                 320
```

Ala Ala Tyr Asp Gln Gly Arg Ile Ala Ala Gln Ala Leu Val Lys Gly
325 330 335

Glu Ala Thr Ala His Leu Ile Glu Asp Ile Pro Thr Gly Ile Tyr Thr
340 345 350

Ile Pro Glu Ile Ser Ser Val Gly Lys Thr Glu Gln Gln Leu Thr Ala
355 360 365

Met Lys Val Pro Tyr Glu Val Gly Arg Ala Gln Phe Lys His Leu Ala
370 375 380

Arg Ala Gln Ile Val Gly Met Asn Val Gly Thr Leu Lys Ile Leu Phe
385 390 395 400

His Arg Glu Thr Lys Glu Ile Leu Gly Ile His Cys Phe Gly Glu Arg
405 410 415

Ala Ala Glu Ile Ile His Ile Gly Gln Ala Ile Met Glu Gln Lys Gly
420 425 430

Gly Gly Asn Thr Ile Glu Tyr Phe Val Asn Thr Thr Phe Asn Tyr Pro
435 440 445

Thr Met Ala Glu Ala Tyr Arg Val Ala Ala Leu Asn Gly Leu Asn Arg
450 455 460

Leu Phe
465

<210> 196
<211> 1476
<212> DNA
<213> Escherichia coli

<400> 196
atggcggtaa cgcaaacagc ccaggcctgt gacctggtca ttttcggcgc gaaaggcgac 60

cttgcgcgtc gtaaattgct gccttccctg tatcaactgg aaaaagccgg tcagctcaac 120

ccggacaccc ggattatcgg cgtagggcgt gctgactggg ataaagcggc atataccaaa 180

gttgtccgcg aggcgctcga aactttcatg aaagaaacca ttgatgaagg tttatgggac 240

accctgagtg cacgtctgga tttttgtaat ctcgatgtca atgacactgc tgcattcagc 300

cgtctcggcg cgatgctgga tcaaaaaaat cgtatcacca ttaactactt tgccatgccg 360

cccagcactt ttggcgcaat ttgcaaaggg cttggcgagg caaaactgaa tgctaaaccg 420

gcacgcgtag tcatggagaa accgctgggg acgtcgctgg cgacctcgca ggaaatcaat 480

gatcaggttg gcgaatactt cgaggagtgc caggtttacc gtatcgacca ctatcttggt 540

aaagaaacgg tgctgaacct gttggcgctg cgttttgcta actccctgtt tgtgaataac 600

tgggacaatc gcaccattga tcatgttgag attaccgtgg cagaagaagt ggggatcgaa 660

gggcgctggg gctattttga taaagccggt cagatgcgcg acatgatcca gaaccacctg 720

ctgcaaattc tttgcatgat tgcgatgtct ccgccgtctg acctgagcgc agacagcatc 780

cgcgatgaaa aagtgaaagt actgaagtct ctgcgccgca tcgaccgctc caacgtacgc 840

gaaaaaaccg tacgcgggca atatactgcg ggcttcgccc agggcaaaaa agtgccggga 900

tatctggaag aagagggcgc gaacaagagc agcaatacag aaactttcgt ggcgatccgc 960

gtcgacattg ataactggcg ctgggccggt gtgccattct acctgcgtac tggtaaacgt 1020

ctgccgacca aatgttctga agtcgtggtc tatttcaaaa cacctgaact gaatctgttt 1080

aaagaatcgt ggcaggatct gccgcagaat aaactgacta tccgtctgca acctgatgaa 1140

ggcgtggata tccaggtact gaataaagtt cctggccttg accacaaaca taacctgcaa 1200

atcaccaagc tggatctgag ctattcagaa acctttaatc agacgcatct ggcggatgcc 1260

tatgaacgtt tgctgctgga aaccatgcgt ggtattcagg cactgtttgt acgtcgcgac 1320

gaagtggaag aagcctggaa atgggtagac tccattactg aggcgtgggc gatggacaat 1380

gatgcgccga aaccgtatca ggccggaacc tggggacccg ttgcctcggt ggcgatgatt 1440

acccgtgatg gtcgttcctg gaatgagttt gagtaa 1476

<210> 197
<211> 491
<212> PRT
<213> Escherichia coli

<400> 197

Met Ala Val Thr Gln Thr Ala Gln Ala Cys Asp Leu Val Ile Phe Gly
1 5 10 15

Ala Lys Gly Asp Leu Ala Arg Arg Lys Leu Leu Pro Ser Leu Tyr Gln
20 25 30

Leu Glu Lys Ala Gly Gln Leu Asn Pro Asp Thr Arg Ile Ile Gly Val
35 40 45

Gly Arg Ala Asp Trp Asp Lys Ala Ala Tyr Thr Lys Val Val Arg Glu
50 55 60

Ala Leu Glu Thr Phe Met Lys Glu Thr Ile Asp Glu Gly Leu Trp Asp
65 70 75 80

Thr Leu Ser Ala Arg Leu Asp Phe Cys Asn Leu Asp Val Asn Asp Thr

85 90 95

Ala Ala Phe Ser Arg Leu Gly Ala Met Leu Asp Gln Lys Asn Arg Ile
100 105 110

Thr Ile Asn Tyr Phe Ala Met Pro Pro Ser Thr Phe Gly Ala Ile Cys
115 120 125

Lys Gly Leu Gly Glu Ala Lys Leu Asn Ala Lys Pro Ala Arg Val Val
130 135 140

Met Glu Lys Pro Leu Gly Thr Ser Leu Ala Thr Ser Gln Glu Ile Asn
145 150 155 160

Asp Gln Val Gly Glu Tyr Phe Glu Glu Cys Gln Val Tyr Arg Ile Asp
165 170 175

His Tyr Leu Gly Lys Glu Thr Val Leu Asn Leu Leu Ala Leu Arg Phe
180 185 190

Ala Asn Ser Leu Phe Val Asn Asn Trp Asp Asn Arg Thr Ile Asp His
195 200 205

Val Glu Ile Thr Val Ala Glu Glu Val Gly Ile Glu Gly Arg Trp Gly
210 215 220

Tyr Phe Asp Lys Ala Gly Gln Met Arg Asp Met Ile Gln Asn His Leu
225 230 235 240

Leu Gln Ile Leu Cys Met Ile Ala Met Ser Pro Pro Ser Asp Leu Ser
245 250 255

Ala Asp Ser Ile Arg Asp Glu Lys Val Lys Val Leu Lys Ser Leu Arg
260 265 270

Arg Ile Asp Arg Ser Asn Val Arg Glu Lys Thr Val Arg Gly Gln Tyr
275 280 285

Thr Ala Gly Phe Ala Gln Gly Lys Lys Val Pro Gly Tyr Leu Glu Glu
290 295 300

Glu Gly Ala Asn Lys Ser Ser Asn Thr Glu Thr Phe Val Ala Ile Arg
305 310 315 320

Val Asp Ile Asp Asn Trp Arg Trp Ala Gly Val Pro Phe Tyr Leu Arg
325 330 335

Thr Gly Lys Arg Leu Pro Thr Lys Cys Ser Glu Val Val Val Tyr Phe
340 345 350

Lys Thr Pro Glu Leu Asn Leu Phe Lys Glu Ser Trp Gln Asp Leu Pro
355 360 365

Gln Asn Lys Leu Thr Ile Arg Leu Gln Pro Asp Glu Gly Val Asp Ile
370 375 380

Gln Val Leu Asn Lys Val Pro Gly Leu Asp His Lys His Asn Leu Gln
385 390 395 400

Ile Thr Lys Leu Asp Leu Ser Tyr Ser Glu Thr Phe Asn Gln Thr His
405 410 415

Leu Ala Asp Ala Tyr Glu Arg Leu Leu Leu Glu Thr Met Arg Gly Ile
420 425 430

Gln Ala Leu Phe Val Arg Arg Asp Glu Val Glu Glu Ala Trp Lys Trp
435 440 445

Val Asp Ser Ile Thr Glu Ala Trp Ala Met Asp Asn Asp Ala Pro Lys
450 455 460

Pro Tyr Gln Ala Gly Thr Trp Gly Pro Val Ala Ser Val Ala Met Ile
465 470 475 480

Thr Arg Asp Gly Arg Ser Trp Asn Glu Phe Glu
485 490

<210> 198
<211> 1992
<212> DNA
<213> Escherichia coli

<400> 198
atgtcctcac gtaaagagct tgccaatgct attcgtgcgc tgagcatgga cgcagtacag 60

aaagccaaat ccggtcaccc gggtgcccct atgggtatgg ctgacattgc cgaagtcctg 120

tggcgtgatt tcctgaaaca caacccgcag aatccgtcct gggctgaccg tgaccgcttc 180

gtgctgtcca acggccacgg ctccatgctg atctacagcc tgctgcacct caccggttac 240

gatctgccga tggaagaact gaaaaacttc cgtcagctgc actctaaaac tccgggtcac 300

ccggaagtgg gttacaccgc tggtgtggaa accaccaccg gtccgctggg tcagggtatt 360

gccaacgcag tcggtatggc gattgcagaa aaaacgctgg cggcgcagtt taaccgtccg 420

ggccacgaca ttgtcgacca ctacacctac gccttcatgg gcgacggctg catgatggaa 480

ggcatctccc acgaagtttg ctctctggcg ggtacgctga agctgggtaa actgattgca 540

ttctacgatg acaacggtat ttctatcgat ggtcacgttg aaggctggtt caccgacgac 600

accgcaatgc gtttcgaagc ttacggctgg cacgttattc gcgacatcga cggtcatgac 660

gcggcatcta tcaaacgcgc agtagaagaa gcgcgcgcag tgactgacaa accttccctg 720

ctgatgtgca aaaccatcat cggtttcggt tccccgaaca aagccggtac ccacgactcc 780

cacggtgcgc cgctgggcga cgctgaaatt gccctgaccc gcgaacaact gggctggaaa 840

tatgcgccgt tcgaaatccc gtctgaaatc tatgctcagt gggatgcgaa agaagcaggc 900

caggcgaaag aatccgcatg gaacgagaaa ttcgctgctt acgcgaaagc ttatccgcag 960

gaagccgctg aatttacccg ccgtatgaaa ggcgaaatgc cgtctgactt cgacgctaaa 1020

gcgaaagagt tcatcgctaa actgcaggct aatccggcga aaatcgccag ccgtaaagcg 1080

tctcagaatg ctatcgaagc gttcggtccg ctgttgccgg aattcctcgg cggttctgct 1140

gacctggcgc cgtctaacct gaccctgtgg tctggttcta aagcaatcaa cgaagatgct 1200

gcgggtaact acatccacta cggtgttcgc gagttcggta tgaccgcgat tgctaacggt 1260

atctccctgc acggtggctt cctgccgtac acctccacct tcctgatgtt cgtggaatac 1320

gcacgtaacg ccgtacgtat ggctgcgctg atgaaacagc gtcaggtgat ggtttacacc 1380

cacgactcca tcggtctggg cgaagacggc ccgactcacc agccggttga gcaggtcgct 1440

tctctgcgcg taaccccgaa catgtctaca tggcgtccgt gtgaccaggt tgaatccgcg 1500

gtcgcgtgga aatacggtgt tgagcgtcag gacggcccga ccgcactgat cctctcccgt 1560

cagaacctgg cgcagcagga acgaactgaa gagcaactgg caaacatcgc gcgcggtggt 1620

tatgtgctga aagactgcgc cggtcagccg gaactgattt tcatcgctac cggttcagaa 1680

gttgaactgg ctgttgctgc ctacgaaaaa ctgactgccg aaggcgtgaa agcgcgcgtg 1740

gtgtccatgc cgtctaccga cgcatttgac aagcaggatg ctgcttaccg tgaatccgta 1800

ctgccgaaag cggttactgc acgcgttgct gtagaagcgg gtattgctga ctactggtac 1860

aagtatgttg gcctgaacgg tgctatcgtc ggtatgacca ccttcggtga atctgctccg 1920

gcagagctgc tgtttgaaga gttcggcttc actgttgata acgttgttgc gaaagcaaaa 1980

gaactgctgt aa 1992

<210> 199
<211> 663
<212> PRT
<213> Escherichia coli

<400> 199

Met Ser Ser Arg Lys Glu Leu Ala Asn Ala Ile Arg Ala Leu Ser Met
1 5 10 15

```
Asp Ala Val Gln Lys Ala Lys Ser Gly His Pro Gly Ala Pro Met Gly
            20                  25                  30

Met Ala Asp Ile Ala Glu Val Leu Trp Arg Asp Phe Leu Lys His Asn
        35                  40                  45

Pro Gln Asn Pro Ser Trp Ala Asp Arg Asp Arg Phe Val Leu Ser Asn
    50                  55                  60

Gly His Gly Ser Met Leu Ile Tyr Ser Leu Leu His Leu Thr Gly Tyr
65                  70                  75                  80

Asp Leu Pro Met Glu Glu Leu Lys Asn Phe Arg Gln Leu His Ser Lys
                85                  90                  95

Thr Pro Gly His Pro Glu Val Gly Tyr Thr Ala Gly Val Glu Thr Thr
            100                 105                 110

Thr Gly Pro Leu Gly Gln Gly Ile Ala Asn Ala Val Gly Met Ala Ile
        115                 120                 125

Ala Glu Lys Thr Leu Ala Ala Gln Phe Asn Arg Pro Gly His Asp Ile
    130                 135                 140

Val Asp His Tyr Thr Tyr Ala Phe Met Gly Asp Gly Cys Met Met Glu
145                 150                 155                 160

Gly Ile Ser His Glu Val Cys Ser Leu Ala Gly Thr Leu Lys Leu Gly
                165                 170                 175

Lys Leu Ile Ala Phe Tyr Asp Asp Asn Gly Ile Ser Ile Asp Gly His
            180                 185                 190

Val Glu Gly Trp Phe Thr Asp Asp Thr Ala Met Arg Phe Glu Ala Tyr
        195                 200                 205

Gly Trp His Val Ile Arg Asp Ile Asp Gly His Asp Ala Ala Ser Ile
    210                 215                 220

Lys Arg Ala Val Glu Glu Ala Arg Ala Val Thr Asp Lys Pro Ser Leu
225                 230                 235                 240

Leu Met Cys Lys Thr Ile Ile Gly Phe Gly Ser Pro Asn Lys Ala Gly
                245                 250                 255

Thr His Asp Ser His Gly Ala Pro Leu Gly Asp Ala Glu Ile Ala Leu
            260                 265                 270
```

```
Thr Arg Glu Gln Leu Gly Trp Lys Tyr Ala Pro Phe Glu Ile Pro Ser
        275                 280                 285

Glu Ile Tyr Ala Gln Trp Asp Ala Lys Glu Ala Gly Gln Ala Lys Glu
    290                 295                 300

Ser Ala Trp Asn Glu Lys Phe Ala Ala Tyr Ala Lys Ala Tyr Pro Gln
305                 310                 315                 320

Glu Ala Ala Glu Phe Thr Arg Arg Met Lys Gly Glu Met Pro Ser Asp
                325                 330                 335

Phe Asp Ala Lys Ala Lys Glu Phe Ile Ala Lys Leu Gln Ala Asn Pro
            340                 345                 350

Ala Lys Ile Ala Ser Arg Lys Ala Ser Gln Asn Ala Ile Glu Ala Phe
        355                 360                 365

Gly Pro Leu Leu Pro Glu Phe Leu Gly Gly Ser Ala Asp Leu Ala Pro
    370                 375                 380

Ser Asn Leu Thr Leu Trp Ser Gly Ser Lys Ala Ile Asn Glu Asp Ala
385                 390                 395                 400

Ala Gly Asn Tyr Ile His Tyr Gly Val Arg Glu Phe Gly Met Thr Ala
                405                 410                 415

Ile Ala Asn Gly Ile Ser Leu His Gly Gly Phe Leu Pro Tyr Thr Ser
            420                 425                 430

Thr Phe Leu Met Phe Val Glu Tyr Ala Arg Asn Ala Val Arg Met Ala
        435                 440                 445

Ala Leu Met Lys Gln Arg Gln Val Met Val Tyr Thr His Asp Ser Ile
    450                 455                 460

Gly Leu Gly Glu Asp Gly Pro Thr His Gln Pro Val Glu Gln Val Ala
465                 470                 475                 480

Ser Leu Arg Val Thr Pro Asn Met Ser Thr Trp Arg Pro Cys Asp Gln
                485                 490                 495

Val Glu Ser Ala Val Ala Trp Lys Tyr Gly Val Glu Arg Gln Asp Gly
            500                 505                 510

Pro Thr Ala Leu Ile Leu Ser Arg Gln Asn Leu Ala Gln Gln Glu Arg
        515                 520                 525
```

Thr Glu Glu Gln Leu Ala Asn Ile Ala Arg Gly Gly Tyr Val Leu Lys
530 535 540

Asp Cys Ala Gly Gln Pro Glu Leu Ile Phe Ile Ala Thr Gly Ser Glu
545 550 555 560

Val Glu Leu Ala Val Ala Ala Tyr Glu Lys Leu Thr Ala Glu Gly Val
565 570 575

Lys Ala Arg Val Val Ser Met Pro Ser Thr Asp Ala Phe Asp Lys Gln
580 585 590

Asp Ala Ala Tyr Arg Glu Ser Val Leu Pro Lys Ala Val Thr Ala Arg
595 600 605

Val Ala Val Glu Ala Gly Ile Ala Asp Tyr Trp Tyr Lys Tyr Val Gly
610 615 620

Leu Asn Gly Ala Ile Val Gly Met Thr Thr Phe Gly Glu Ser Ala Pro
625 630 635 640

Ala Glu Leu Leu Phe Glu Glu Phe Gly Phe Thr Val Asp Asn Val Val
645 650 655

Ala Lys Ala Lys Glu Leu Leu
660

<210> 200
<211> 2004
<212> DNA
<213> Escherichia coli

<400> 200
atgtcccgaa aagaccttgc caatgcgatt cgcgcactca gtatggatgc ggtacaaaaa 60

gccaactctg gtcatcccgg cgcgccgatg ggcatggctg atattgccga agtgctgtgg 120

aacgattttc ttaaacataa ccctaccgac ccaacctggt atgatcgcga ccgctttatt 180

ctttccaacg gtcacgcgtc gatgctgctc tacagtttgc tacatctgac cggttacgac 240

ctgccgctgg aagaactgaa gaacttccgt cagttgcatt cgaaaacccc aggccacccg 300

gagattggct atacgccagg cgttgaaacc accaccggcc cgcttggaca aggtttggcg 360

aacgccgtcg ggctggcgat agcagagcgt acactggcgg cgcagtttaa ccagccagac 420

catgagatcg tcgatcactt cacctatgtg tttatgggcg acggctgcct gatggaaggt 480

atttcccacg aagtctgttc gctggcaggc acgctgggac tgggcaagct gattggtttt 540

tacgatcaca acggtatttc catcgacggt gaaacagaag gctggtttac cgacgatacg 600

gcaaaacgtt ttgaagccta tcactggcat gtgatccatg aaatcgacgg tcacgatccg 660

caggcggtga aggaagcgat ccttgaagcg caaagcgtga aagataagcc gtcgctgatt 720

atctgccgta cggtgattgg ctttggttcg ccgaataaag caggtaagga agaggcgcac 780

ggcgcaccac tgggggaaga agaagtggcg ctggcacggc aaaaactggg ctggcaccat 840

ccgccatttg agatccctaa agagatttat cacgcctggg atgcccgtga aaaaggcgaa 900

aaagcgcagc agagctggaa tgagaagttt gccgcctata aaaaggctca tccgcaactg 960

gcagaagagt ttacccgacg gatgagcggt ggtttaccga aggactggga gaaaacgact 1020

cagaaatata tcaatgagtt acaggcaaat ccggcgaaaa tcgctacccg taaggcttcg 1080

caaaatacgc ttaacgctta cgggccgatg ctgcctgagt tgctcggcgg ttcggcggat 1140

ctggctccca gcaacctgac catctggaaa ggttctgttt cgctgaagga agatccagcg 1200

ggcaactaca ttcactacgg ggtgcgtgaa tttggcatga ccgctatcgc caacggcatc 1260

gcgcaccacg gcggctttgt gccgtatacc gcgacgttcc tgatgtttgt tgaatacgcc 1320

cgtaacgccg cgcggatggc ggcactgatg aaagcgcggc agattatggt ttatacccac 1380

gactcaattg gcctgggcga agatggtccg acgcaccagg ctgttgagca actggccagc 1440

ctgcgcttaa cgccaaattt cagcacctgg cgaccgtgcg atcaggtgga agcggcggtg 1500

ggctggaagc tggcggttga gcgccacaac ggaccgacgg cactgatcct ctcaaggcag 1560

aatctggccc aggtggaacg tacgccggat caggttaaag agattgctcg tggcggttat 1620

gtgctgaaag acagcggcgg taagccagat attattctga ttgccaccgg ttcagagatg 1680

gaaattaccc tgcaagcggc agagaaatta gcaggagaag gtcgcaatgt acgcgtagtt 1740

tccctgccct cgaccgatat tttcgacgcc caggatgagg aatatcggga gtcggtgttg 1800

ccttctaacg ttgcggctcg cgtggcggtg gaagcaggta ttgccgatta ctggtacaag 1860

tatgttggtc tgaaaggggc aattgtcggg atgacgggtt acggggaatc tgctccggcg 1920

gataagctgt tcccgttctt tggctttacc gccgagaata ttgtggcaaa agcgcataag 1980

gtgctgggag tgaaaggtgc ctga 2004

<210> 201
<211> 667
<212> PRT
<213> Escherichia coli

<400> 201

Met Ser Arg Lys Asp Leu Ala Asn Ala Ile Arg Ala Leu Ser Met Asp
1               5                   10                  15

Ala Val Gln Lys Ala Asn Ser Gly His Pro Gly Ala Pro Met Gly Met
            20                  25                  30

Ala Asp Ile Ala Glu Val Leu Trp Asn Asp Phe Leu Lys His Asn Pro
35 40 45

Thr Asp Pro Thr Trp Tyr Asp Arg Asp Arg Phe Ile Leu Ser Asn Gly
50 55 60

His Ala Ser Met Leu Leu Tyr Ser Leu Leu His Leu Thr Gly Tyr Asp
65 70 75 80

Leu Pro Leu Glu Glu Leu Lys Asn Phe Arg Gln Leu His Ser Lys Thr
85 90 95

Pro Gly His Pro Glu Ile Gly Tyr Thr Pro Gly Val Glu Thr Thr Thr
100 105 110

Gly Pro Leu Gly Gln Gly Leu Ala Asn Ala Val Gly Leu Ala Ile Ala
115 120 125

Glu Arg Thr Leu Ala Ala Gln Phe Asn Gln Pro Asp His Glu Ile Val
130 135 140

Asp His Phe Thr Tyr Val Phe Met Gly Asp Gly Cys Leu Met Glu Gly
145 150 155 160

Ile Ser His Glu Val Cys Ser Leu Ala Gly Thr Leu Gly Leu Gly Lys
165 170 175

Leu Ile Gly Phe Tyr Asp His Asn Gly Ile Ser Ile Asp Gly Glu Thr
180 185 190

Glu Gly Trp Phe Thr Asp Asp Thr Ala Lys Arg Phe Glu Ala Tyr His
195 200 205

Trp His Val Ile His Glu Ile Asp Gly His Asp Pro Gln Ala Val Lys
210 215 220

Glu Ala Ile Leu Glu Ala Gln Ser Val Lys Asp Lys Pro Ser Leu Ile
225 230 235 240

Ile Cys Arg Thr Val Ile Gly Phe Gly Ser Pro Asn Lys Ala Gly Lys
245 250 255

Glu Glu Ala His Gly Ala Pro Leu Gly Glu Glu Glu Val Ala Leu Ala
260 265 270

Arg Gln Lys Leu Gly Trp His His Pro Pro Phe Glu Ile Pro Lys Glu

275 280 285

Ile Tyr His Ala Trp Asp Ala Arg Glu Lys Gly Glu Lys Ala Gln Gln
290 295 300

Ser Trp Asn Glu Lys Phe Ala Ala Tyr Lys Lys Ala His Pro Gln Leu
305 310 315 320

Ala Glu Glu Phe Thr Arg Arg Met Ser Gly Gly Leu Pro Lys Asp Trp
325 330 335

Glu Lys Thr Thr Gln Lys Tyr Ile Asn Glu Leu Gln Ala Asn Pro Ala
340 345 350

Lys Ile Ala Thr Arg Lys Ala Ser Gln Asn Thr Leu Asn Ala Tyr Gly
355 360 365

Pro Met Leu Pro Glu Leu Leu Gly Gly Ser Ala Asp Leu Ala Pro Ser
370 375 380

Asn Leu Thr Ile Trp Lys Gly Ser Val Ser Leu Lys Glu Asp Pro Ala
385 390 395 400

Gly Asn Tyr Ile His Tyr Gly Val Arg Glu Phe Gly Met Thr Ala Ile
405 410 415

Ala Asn Gly Ile Ala His His Gly Gly Phe Val Pro Tyr Thr Ala Thr
420 425 430

Phe Leu Met Phe Val Glu Tyr Ala Arg Asn Ala Ala Arg Met Ala Ala
435 440 445

Leu Met Lys Ala Arg Gln Ile Met Val Tyr Thr His Asp Ser Ile Gly
450 455 460

Leu Gly Glu Asp Gly Pro Thr His Gln Ala Val Glu Gln Leu Ala Ser
465 470 475 480

Leu Arg Leu Thr Pro Asn Phe Ser Thr Trp Arg Pro Cys Asp Gln Val
485 490 495

Glu Ala Ala Val Gly Trp Lys Leu Ala Val Glu Arg His Asn Gly Pro
500 505 510

Thr Ala Leu Ile Leu Ser Arg Gln Asn Leu Ala Gln Val Glu Arg Thr
515 520 525

Pro Asp Gln Val Lys Glu Ile Ala Arg Gly Gly Tyr Val Leu Lys Asp
    530                 535                 540

Ser Gly Gly Lys Pro Asp Ile Ile Leu Ile Ala Thr Gly Ser Glu Met
545                 550                 555                 560

Glu Ile Thr Leu Gln Ala Ala Glu Lys Leu Ala Gly Glu Gly Arg Asn
                565                 570                 575

Val Arg Val Val Ser Leu Pro Ser Thr Asp Ile Phe Asp Ala Gln Asp
            580                 585                 590

Glu Glu Tyr Arg Glu Ser Val Leu Pro Ser Asn Val Ala Ala Arg Val
        595                 600                 605

Ala Val Glu Ala Gly Ile Ala Asp Tyr Trp Tyr Lys Tyr Val Gly Leu
    610                 615                 620

Lys Gly Ala Ile Val Gly Met Thr Gly Tyr Gly Glu Ser Ala Pro Ala
625                 630                 635                 640

Asp Lys Leu Phe Pro Phe Phe Gly Phe Thr Ala Glu Asn Ile Val Ala
                645                 650                 655

Lys Ala His Lys Val Leu Gly Val Lys Gly Ala
            660                 665

<210> 202
<211> 1407
<212> DNA
<213> Escherichia coli

<400> 202
atgtccaagc aacagatcgg cgtagtcggt atggcagtga tgggacgcaa ccttgcgctc 60

aacatcgaaa gccgtggtta taccgtctct attttcaacc gttcccgtga gaagacggaa 120

gaagtgattg ccgaaaatcc aggcaagaaa ctggttcctt actatacggt gaaagagttt 180

gtcgaatctc tggaaacgcc tcgtcgcatc ctgttaatgg tgaaagcagg tgcaggcacg 240

gatgctgcta ttgattccct caaaccatat ctcgataaag gagacatcat cattgatggt 300

ggtaacacct tcttccagga cactattcgt cgtaatcgtg agctttcagc agagggcttt 360

aacttcatcg gtaccggtgt ttctggcggt gaagaggggg cgctgaaagg tccttctatt 420

atgcctggtg gccagaaaga agcctatgaa ttggtagcac cgatcctgac caaaatcgcc 480

gccgtagctg aagacggtga accatgcgtt acctatattg gtgccgatgg cgcaggtcac 540

tatgtgaaga tggttcacaa cggtattgaa tacggcgata tgcagctgat tgctgaagcc 600

tattctctgc ttaaaggtgg cctgaacctc accaacgaag aactggcgca gacctttacc 660

gagtggaata acggtgaact gagcagttac ctgatcgaca tcaccaaaga tatcttcacc 720

aaaaaagatg aagacggtaa ctacctggtt gatgtgatcc tggatgaagc ggctaacaaa 780

ggtaccggta aatggaccag ccagagcgcg ctggatctcg gcgaaccgct gtcgctgatt 840

accgagtctg tgtttgcacg ttatatctct tctctgaaag atcagcgtgt tgccgcatct 900

aaagttctct ctggtccgca agcacagcca gcaggcgaca aggctgagtt catcgaaaaa 960

gttcgtcgtg cgctgtatct gggcaaaatc gtttcttacg cccagggctt ctctcagctg 1020

cgtgctgcgt ctgaagagta caactgggat ctgaactacg gcgaaatcgc gaagattttc 1080

cgtgctggct gcatcatccg tgcgcagttc ctgcagaaaa tcaccgatgc ttatgccgaa 1140

aatccacaga tcgctaacct gttgctggct ccgtacttca agcaaattgc cgatgactac 1200

cagcaggcgc tgcgtgatgt cgttgcttat gcagtacaga acggtattcc ggttccgacc 1260

ttctccgcag cggttgccta ttacgacagc taccgtgctg ctgttctgcc tgcgaacctg 1320

atccaggcac agcgtgacta ttttggtgcg catacttata agcgtattga taaagaaggt 1380

gtgttccata ccgaatggct ggattaa 1407

<210> 203
<211> 468
<212> PRT
<213> Escherichia coli

<400> 203

Met Ser Lys Gln Gln Ile Gly Val Val Gly Met Ala Val Met Gly Arg
1 5 10 15

Asn Leu Ala Leu Asn Ile Glu Ser Arg Gly Tyr Thr Val Ser Ile Phe
20 25 30

Asn Arg Ser Arg Glu Lys Thr Glu Glu Val Ile Ala Glu Asn Pro Gly
35 40 45

Lys Lys Leu Val Pro Tyr Tyr Thr Val Lys Glu Phe Val Glu Ser Leu
50 55 60

Glu Thr Pro Arg Arg Ile Leu Leu Met Val Lys Ala Gly Ala Gly Thr
65 70 75 80

Asp Ala Ala Ile Asp Ser Leu Lys Pro Tyr Leu Asp Lys Gly Asp Ile
85 90 95

Ile Ile Asp Gly Gly Asn Thr Phe Phe Gln Asp Thr Ile Arg Arg Asn
100 105 110

```
Arg Glu Leu Ser Ala Glu Gly Phe Asn Phe Ile Gly Thr Gly Val Ser
        115                 120                 125

Gly Gly Glu Glu Gly Ala Leu Lys Gly Pro Ser Ile Met Pro Gly Gly
    130                 135                 140

Gln Lys Glu Ala Tyr Glu Leu Val Ala Pro Ile Leu Thr Lys Ile Ala
145                 150                 155                 160

Ala Val Ala Glu Asp Gly Glu Pro Cys Val Thr Tyr Ile Gly Ala Asp
                165                 170                 175

Gly Ala Gly His Tyr Val Lys Met Val His Asn Gly Ile Glu Tyr Gly
            180                 185                 190

Asp Met Gln Leu Ile Ala Glu Ala Tyr Ser Leu Leu Lys Gly Gly Leu
        195                 200                 205

Asn Leu Thr Asn Glu Glu Leu Ala Gln Thr Phe Thr Glu Trp Asn Asn
    210                 215                 220

Gly Glu Leu Ser Ser Tyr Leu Ile Asp Ile Thr Lys Asp Ile Phe Thr
225                 230                 235                 240

Lys Lys Asp Glu Asp Gly Asn Tyr Leu Val Asp Val Ile Leu Asp Glu
                245                 250                 255

Ala Ala Asn Lys Gly Thr Gly Lys Trp Thr Ser Gln Ser Ala Leu Asp
            260                 265                 270

Leu Gly Glu Pro Leu Ser Leu Ile Thr Glu Ser Val Phe Ala Arg Tyr
        275                 280                 285

Ile Ser Ser Leu Lys Asp Gln Arg Val Ala Ala Ser Lys Val Leu Ser
    290                 295                 300

Gly Pro Gln Ala Gln Pro Ala Gly Asp Lys Ala Glu Phe Ile Glu Lys
305                 310                 315                 320

Val Arg Arg Ala Leu Tyr Leu Gly Lys Ile Val Ser Tyr Ala Gln Gly
                325                 330                 335

Phe Ser Gln Leu Arg Ala Ala Ser Glu Glu Tyr Asn Trp Asp Leu Asn
            340                 345                 350

Tyr Gly Glu Ile Ala Lys Ile Phe Arg Ala Gly Cys Ile Ile Arg Ala
        355                 360                 365
```

Gln Phe Leu Gln Lys Ile Thr Asp Ala Tyr Ala Glu Asn Pro Gln Ile
370 375 380

Ala Asn Leu Leu Leu Ala Pro Tyr Phe Lys Gln Ile Ala Asp Asp Tyr
385 390 395 400

Gln Gln Ala Leu Arg Asp Val Val Ala Tyr Ala Val Gln Asn Gly Ile
405 410 415

Pro Val Pro Thr Phe Ser Ala Ala Val Ala Tyr Tyr Asp Ser Tyr Arg
420 425 430

Ala Ala Val Leu Pro Ala Asn Leu Ile Gln Ala Gln Arg Asp Tyr Phe
435 440 445

Gly Ala His Thr Tyr Lys Arg Ile Asp Lys Glu Gly Val Phe His Thr
450 455 460

Glu Trp Leu Asp
465

<210> 204
<211> 1434
<212> DNA
<213> Streptococcus mutans

<400> 204
atggcaatga caaaacaata taaaaattat gtcaatggcg agtggaagct ttcagaaaat 60

gaaattaaaa tctacgaacc agccagtgga gctgaattgg gttcagttcc agcaatgagt 120

actgaagaag tagattatgt ttatgcttca gccaagaaag ctcaaccagc ttggcgagca 180

ctttcataca tagaacgtgc tgcctacctt cataaggtag cagatatttt gatgcgtgat 240

aaagaaaaaa taggtgctat tctttccaaa gaggttgcta aaggttataa atcagcagtc 300

agcgaagttg ttcgtactgc agaaatcatt aattatgcag ctgaagaagg tcttcgtatg 360

gaaggtgaag tccttgaagg cggcagtttt gaagcagcca gcaagaaaaa aattgccgtt 420

gttcgtcgtg aaccagtagg tcttgtatta gctatttcac catttaacta ccctgttaac 480

ttggcaggtt cgaaaattgc accggctctt attgcgggaa atgttattgc ttttaaacca 540

ccgacgcaag gatcaatctc agggctctta cttgctgaag catttgctga agctggactt 600

cctgcaggtg tctttaatac cattacaggt cgtggttctg aaattggaga ctatattgta 660

gaacatcaag ccgttaactt tatcaatttc actggttcaa caggaattgg cgaacgtatt 720

ggcaaaatgg ctggtatgcg tccgattatg cttgaactcg gtggaaaaga ttcagccatc 780

gttcttgaag atgcggacct tgaattgact gctaaaaata ttattgcagg tgcttttggt 840

tattcaggtc aacgctgtac agcagttaaa cgtgttcttg tgatggaaag tgttgctgat 900

gaactggtcg aaaaaatccg tgaaaaagtt cttgcattaa caattggtaa tccagaagac 960

gatgcagata ttacaccgtt gattgataca aaatcagctg attatgtaga aggtcttatt 1020

aatgatgcca atgataaagg agccactgcc cttactgaaa tcaaacgtga aggtaatctt 1080

atctgtccaa tcctctttga taaggtaacg acagatatgc gtcttgcttg ggaagaacca 1140

tttggtcctg ttcttccgat cattcgtgtg acatctgtag aagaagccat tgaaatttct 1200

aacaaatcgg aatatggact tcaggcttct atctttacaa atgatttccc acgcgctttt 1260

ggtattgctg agcagcttga agttggtaca gttcatatca ataataagac acagcgcggc 1320

acggacaact tcccattctt aggggctaaa aaatcaggtg caggtattca aggggtaaaa 1380

tattctattg aagctatgac aactgttaaa tccgtcgtat ttgatatcaa ataa 1434

<210> 205
<211> 477
<212> PRT
<213> Streptococcus mutans

<400> 205

Met Ala Met Thr Lys Gln Tyr Lys Asn Tyr Val Asn Gly Glu Trp Lys
1 5 10 15

Leu Ser Glu Asn Glu Ile Lys Ile Tyr Glu Pro Ala Ser Gly Ala Glu
20 25 30

Leu Gly Ser Val Pro Ala Met Ser Thr Glu Glu Val Asp Tyr Val Tyr
35 40 45

Ala Ser Ala Lys Lys Ala Gln Pro Ala Trp Arg Ala Leu Ser Tyr Ile
50 55 60

Glu Arg Ala Ala Tyr Leu His Lys Val Ala Asp Ile Leu Met Arg Asp
65 70 75 80

Lys Glu Lys Ile Gly Ala Ile Leu Ser Lys Glu Val Ala Lys Gly Tyr
85 90 95

Lys Ser Ala Val Ser Glu Val Val Arg Thr Ala Glu Ile Ile Asn Tyr
100 105 110

Ala Ala Glu Glu Gly Leu Arg Met Glu Gly Glu Val Leu Glu Gly Gly
115 120 125

Ser Phe Glu Ala Ala Ser Lys Lys Lys Ile Ala Val Val Arg Arg Glu
130 135 140

Pro Val Gly Leu Val Leu Ala Ile Ser Pro Phe Asn Tyr Pro Val Asn
145 150 155 160

Leu Ala Gly Ser Lys Ile Ala Pro Ala Leu Ile Ala Gly Asn Val Ile
165 170 175

Ala Phe Lys Pro Pro Thr Gln Gly Ser Ile Ser Gly Leu Leu Leu Ala
180 185 190

Glu Ala Phe Ala Glu Ala Gly Leu Pro Ala Gly Val Phe Asn Thr Ile
195 200 205

Thr Gly Arg Gly Ser Glu Ile Gly Asp Tyr Ile Val Glu His Gln Ala
210 215 220

Val Asn Phe Ile Asn Phe Thr Gly Ser Thr Gly Ile Gly Glu Arg Ile
225 230 235 240

Gly Lys Met Ala Gly Met Arg Pro Ile Met Leu Glu Leu Gly Gly Lys
245 250 255

Asp Ser Ala Ile Val Leu Glu Asp Ala Asp Leu Glu Leu Thr Ala Lys
260 265 270

Asn Ile Ile Ala Gly Ala Phe Gly Tyr Ser Gly Gln Arg Cys Thr Ala
275 280 285

Val Lys Arg Val Leu Val Met Glu Ser Val Ala Asp Glu Leu Val Glu
290 295 300

Lys Ile Arg Glu Lys Val Leu Ala Leu Thr Ile Gly Asn Pro Glu Asp
305 310 315 320

Asp Ala Asp Ile Thr Pro Leu Ile Asp Thr Lys Ser Ala Asp Tyr Val
325 330 335

Glu Gly Leu Ile Asn Asp Ala Asn Asp Lys Gly Ala Thr Ala Leu Thr
340 345 350

Glu Ile Lys Arg Glu Gly Asn Leu Ile Cys Pro Ile Leu Phe Asp Lys
355 360 365

Val Thr Thr Asp Met Arg Leu Ala Trp Glu Glu Pro Phe Gly Pro Val
370 375 380

Leu Pro Ile Ile Arg Val Thr Ser Val Glu Glu Ala Ile Glu Ile Ser
385 390 395 400

Asn Lys Ser Glu Tyr Gly Leu Gln Ala Ser Ile Phe Thr Asn Asp Phe
405 410 415

Pro Arg Ala Phe Gly Ile Ala Glu Gln Leu Glu Val Gly Thr Val His
420 425 430

Ile Asn Asn Lys Thr Gln Arg Gly Thr Asp Asn Phe Pro Phe Leu Gly
435 440 445

Ala Lys Lys Ser Gly Ala Gly Ile Gln Gly Val Lys Tyr Ser Ile Glu
450 455 460

Ala Met Thr Thr Val Lys Ser Val Val Phe Asp Ile Lys
465 470 475

<210> 206
<211> 1344
<212> DNA
<213> Escherichia coli

<400> 206
atggatcaga catattctct ggagtcattc ctcaaccatg tccaaaagcg cgacccgaat 60

caaaccgagt tcgcgcaagc cgttcgtgaa gtaatgacca cactctggcc ttttcttgaa 120

caaaatccaa aatatcgcca gatgtcatta ctggagcgtc tggttgaacc ggagcgcgtg 180

atccagtttc gcgtggtatg ggttgatgat cgcaaccaga tacaggtcaa ccgtgcatgg 240

cgtgtgcagt tcagctctgc catcggcccg tacaaaggcg gtatgcgctt ccatccgtca 300

gttaaccttt ccattctcaa attcctcggc tttgaacaaa ccttcaaaaa tgccctgact 360

actctgccga tgggcggtgg taaaggcggc agcgatttcg atccgaaagg aaaaagcgaa 420

ggtgaagtga tgcgtttttg ccaggcgctg atgactgaac tgtatcgcca cctgggcgcg 480

gataccgacg ttccggcagg tgatatcggg gttggtggtc gtgaagtcgg ctttatggcg 540

gggatgatga aaaagctctc caacaatacc gcctgcgtct tcaccggtaa gggcctttca 600

tttggcggca gtcttattcg cccggaagct accggctacg gtctggttta tttcacagaa 660

gcaatgctaa aacgccacgg tatgggtttt gaagggatgc gcgtttccgt ttctggctcc 720

ggcaacgtcg cccagtacgc tatcgaaaaa gcgatggaat ttggtgctcg tgtgatcact 780

gcgtcagact ccagcggcac tgtagttgat gaaagcggat tcacgaaaga gaaactggca 840

cgtcttatcg aaatcaaagc cagccgcgat ggtcgagtgg cagattacgc caaagaattt 900

ggtctggtct atctcgaagg ccaacagccg tggtctctac cggttgatat cgccctgcct 960

tgcgccaccc agaatgaact ggatgttgac gccgcgcatc agcttatcgc taatggcgtt 1020

aaagccgtcg ccgaaggggc aaatatgccg accaccatcg aagcgactga actgttccag 1080

caggcaggcg tactatttgc accgggtaaa gcggctaatg ctggtggcgt cgctacatcg 1140

ggcctggaaa tggcacaaaa cgctgcgcgc ctgggctgga aagccgagaa agttgacgca 1200

cgtttgcatc acatcatgct ggatatccac catgcctgtg ttgagcatgg tggtgaaggt 1260

gagcaaacca actacgtgca gggcgcgaac attgccggtt ttgtgaaggt tgccgatgcg 1320

atgctggcgc agggtgtgat ttaa 1344

<210> 207
<211> 447
<212> PRT
<213> Escherichia coli

<400> 207

Met Asp Gln Thr Tyr Ser Leu Glu Ser Phe Leu Asn His Val Gln Lys
1 5 10 15

Arg Asp Pro Asn Gln Thr Glu Phe Ala Gln Ala Val Arg Glu Val Met
20 25 30

Thr Thr Leu Trp Pro Phe Leu Glu Gln Asn Pro Lys Tyr Arg Gln Met
35 40 45

Ser Leu Leu Glu Arg Leu Val Glu Pro Glu Arg Val Ile Gln Phe Arg
50 55 60

Val Val Trp Val Asp Asp Arg Asn Gln Ile Gln Val Asn Arg Ala Trp
65 70 75 80

Arg Val Gln Phe Ser Ser Ala Ile Gly Pro Tyr Lys Gly Gly Met Arg
85 90 95

Phe His Pro Ser Val Asn Leu Ser Ile Leu Lys Phe Leu Gly Phe Glu
100 105 110

Gln Thr Phe Lys Asn Ala Leu Thr Thr Leu Pro Met Gly Gly Gly Lys
115 120 125

Gly Gly Ser Asp Phe Asp Pro Lys Gly Lys Ser Glu Gly Glu Val Met
130 135 140

Arg Phe Cys Gln Ala Leu Met Thr Glu Leu Tyr Arg His Leu Gly Ala
145 150 155 160

Asp Thr Asp Val Pro Ala Gly Asp Ile Gly Val Gly Gly Arg Glu Val
165 170 175

Gly Phe Met Ala Gly Met Met Lys Lys Leu Ser Asn Asn Thr Ala Cys
180 185 190

Val Phe Thr Gly Lys Gly Leu Ser Phe Gly Gly Ser Leu Ile Arg Pro
195 200 205

Glu Ala Thr Gly Tyr Gly Leu Val Tyr Phe Thr Glu Ala Met Leu Lys
210 215 220

Arg His Gly Met Gly Phe Glu Gly Met Arg Val Ser Val Ser Gly Ser
225 230 235 240

Gly Asn Val Ala Gln Tyr Ala Ile Glu Lys Ala Met Glu Phe Gly Ala
245 250 255

Arg Val Ile Thr Ala Ser Asp Ser Ser Gly Thr Val Val Asp Glu Ser
260 265 270

Gly Phe Thr Lys Glu Lys Leu Ala Arg Leu Ile Glu Ile Lys Ala Ser
275 280 285

Arg Asp Gly Arg Val Ala Asp Tyr Ala Lys Glu Phe Gly Leu Val Tyr
290 295 300

Leu Glu Gly Gln Gln Pro Trp Ser Leu Pro Val Asp Ile Ala Leu Pro
305 310 315 320

Cys Ala Thr Gln Asn Glu Leu Asp Val Asp Ala Ala His Gln Leu Ile
325 330 335

Ala Asn Gly Val Lys Ala Val Ala Glu Gly Ala Asn Met Pro Thr Thr
340 345 350

Ile Glu Ala Thr Glu Leu Phe Gln Gln Ala Gly Val Leu Phe Ala Pro
355 360 365

Gly Lys Ala Ala Asn Ala Gly Gly Val Ala Thr Ser Gly Leu Glu Met
370 375 380

Ala Gln Asn Ala Ala Arg Leu Gly Trp Lys Ala Glu Lys Val Asp Ala
385 390 395 400

Arg Leu His His Ile Met Leu Asp Ile His His Ala Cys Val Glu His
405 410 415

Gly Gly Glu Gly Glu Gln Thr Asn Tyr Val Gln Gly Ala Asn Ile Ala
420 425 430

```
Gly Phe Val Lys Val Ala Asp Ala Met Leu Ala Gln Gly Val Ile
        435                 440                 445


<210>  208
<211>  1548
<212>  DNA
<213>  Escherichia coli

<400>  208
atgacggacc atacaatgaa gaaaaacccc gtaagtatac cacacaccgt ctggtacgcc      60

gacgatatcc gccgcggaga acgcgaggcg gcagatgtgc tggggctcac actctatgag     120

ctgatgcttc gcgctggcga ggccgcattc caggtgtgtc gttcggcgta tcctgacgcc     180

cgccactggc tggtgctgtg cggtcatggt aataacggcg gcgatggcta cgtggtcgcg     240

cgactggcca aagcggtcgg cattgaggtc acgttgttgg cccaggagag cgacaaaccg     300

ttgccggaag aggccgcgct ggcacgcgaa gcatggttaa acgcgggtgg cgagatccat     360

gcttcgaata ttgtctggcc cgaatcggta gatctgattg ttgatgcgct gctcggtacc     420

ggtttgcggc aagcgccccg cgaatccatt agccagttaa tcgaccacgc taattcccat     480

cctgcgccga ttgtggcggt tgatatccct tccggcctgc tggctgaaac tggcgctacg     540

ccaggcgcgg tgatcaacgc cgatcacacc atcactttta ttgcgctgaa accaggcttg     600

ctcactggaa aagcgcggga tgttaccgga caactgcatt ttgactcact ggggctggat     660

agttggctgg caggtcagga gacgaaaatt cagcggtttt cagcagaaca actttctcac     720

tggctaaaac cgcgtcgccc gacttcgcat aaaggcgatc acgggcggct ggtaattatc     780

ggtggcgatc acggcacggc gggggctatt cgtatgacgg gggaagcggc gctgcgtgct     840

ggtgctggtt tagtccgagt actgacccgc agtgaaaaca ttgcgccgct gctgactgca     900

cgaccggaat tgatggtgca tgaactgacg atggactctc ttaccgaaag cctggaatgg     960

gccgatgtgg tggtgattgg tcccggtctg ggccagcaag agtgggggaa aaaagcactg    1020

caaaaagttg agaattttcg caaaccgatg ttgtgggatg ccgatgcatt gaacctgctg    1080

gcaatcaatc ccgataagcg tcacaatcgc gtgatcacgc cgcatcctgg cgaggccgca    1140

cggttgttag gctgttccgt cgctgaaatt gaaagtgacc gcttacattg cgccaaacgt    1200

ctggtacaac gttatggcgg cgtagcggtg ctgaaaggtg ccggaaccgt ggtcgccgcc    1260

catcctgacg ctttaggcat tattgatgcc ggaaatgcag gcatggcgag cggcggcatg    1320

ggcgatgtgc tctctggtat tattggcgca ttgcttgggc aaaaactgtc gccgtatgat    1380

gcagcctgtg caggctgtgt cgcgcacggt gcggcagctg acgtactggc ggcgcgtttt    1440

ggaacgcgcg ggatgctggc aaccgatctc ttttccacgc tacagcgtat tgttaacccg    1500

gaagtgactg ataaaaacca tgatgaatcg agtaattccg ctccctga                 1548
```

<210> 209
<211> 515
<212> PRT
<213> Escherichia coli

<400> 209

Met Thr Asp His Thr Met Lys Lys Asn Pro Val Ser Ile Pro His Thr
1 5 10 15

Val Trp Tyr Ala Asp Asp Ile Arg Arg Gly Glu Arg Glu Ala Ala Asp
20 25 30

Val Leu Gly Leu Thr Leu Tyr Glu Leu Met Leu Arg Ala Gly Glu Ala
35 40 45

Ala Phe Gln Val Cys Arg Ser Ala Tyr Pro Asp Ala Arg His Trp Leu
50 55 60

Val Leu Cys Gly His Gly Asn Asn Gly Gly Asp Gly Tyr Val Val Ala
65 70 75 80

Arg Leu Ala Lys Ala Val Gly Ile Glu Val Thr Leu Leu Ala Gln Glu
85 90 95

Ser Asp Lys Pro Leu Pro Glu Glu Ala Ala Leu Ala Arg Glu Ala Trp
100 105 110

Leu Asn Ala Gly Gly Glu Ile His Ala Ser Asn Ile Val Trp Pro Glu
115 120 125

Ser Val Asp Leu Ile Val Asp Ala Leu Leu Gly Thr Gly Leu Arg Gln
130 135 140

Ala Pro Arg Glu Ser Ile Ser Gln Leu Ile Asp His Ala Asn Ser His
145 150 155 160

Pro Ala Pro Ile Val Ala Val Asp Ile Pro Ser Gly Leu Leu Ala Glu
165 170 175

Thr Gly Ala Thr Pro Gly Ala Val Ile Asn Ala Asp His Thr Ile Thr
180 185 190

Phe Ile Ala Leu Lys Pro Gly Leu Leu Thr Gly Lys Ala Arg Asp Val
195 200 205

Thr Gly Gln Leu His Phe Asp Ser Leu Gly Leu Asp Ser Trp Leu Ala
210 215 220

```
Gly Gln Glu Thr Lys Ile Gln Arg Phe Ser Ala Glu Gln Leu Ser His
225                 230                 235                 240

Trp Leu Lys Pro Arg Arg Pro Thr Ser His Lys Gly Asp His Gly Arg
                245                 250                 255

Leu Val Ile Ile Gly Gly Asp His Gly Thr Ala Gly Ala Ile Arg Met
            260                 265                 270

Thr Gly Glu Ala Ala Leu Arg Ala Gly Ala Gly Leu Val Arg Val Leu
        275                 280                 285

Thr Arg Ser Glu Asn Ile Ala Pro Leu Leu Thr Ala Arg Pro Glu Leu
    290                 295                 300

Met Val His Glu Leu Thr Met Asp Ser Leu Thr Glu Ser Leu Glu Trp
305                 310                 315                 320

Ala Asp Val Val Val Ile Gly Pro Gly Leu Gly Gln Gln Glu Trp Gly
                325                 330                 335

Lys Lys Ala Leu Gln Lys Val Glu Asn Phe Arg Lys Pro Met Leu Trp
            340                 345                 350

Asp Ala Asp Ala Leu Asn Leu Leu Ala Ile Asn Pro Asp Lys Arg His
        355                 360                 365

Asn Arg Val Ile Thr Pro His Pro Gly Glu Ala Ala Arg Leu Leu Gly
    370                 375                 380

Cys Ser Val Ala Glu Ile Glu Ser Asp Arg Leu His Cys Ala Lys Arg
385                 390                 395                 400

Leu Val Gln Arg Tyr Gly Gly Val Ala Val Leu Lys Gly Ala Gly Thr
                405                 410                 415

Val Val Ala Ala His Pro Asp Ala Leu Gly Ile Ile Asp Ala Gly Asn
            420                 425                 430

Ala Gly Met Ala Ser Gly Gly Met Gly Asp Val Leu Ser Gly Ile Ile
        435                 440                 445

Gly Ala Leu Leu Gly Gln Lys Leu Ser Pro Tyr Asp Ala Ala Cys Ala
    450                 455                 460

Gly Cys Val Ala His Gly Ala Ala Ala Asp Val Leu Ala Ala Arg Phe
465                 470                 475                 480
```

Gly Thr Arg Gly Met Leu Ala Thr Asp Leu Phe Ser Thr Leu Gln Arg
485 490 495

Ile Val Asn Pro Glu Val Thr Asp Lys Asn His Asp Glu Ser Ser Asn
500 505 510

Ser Ala Pro
515

<210> 210
<211> 879
<212> DNA
<213> Escherichia coli

<400> 210
atgaataatc atttcaagtg tattggcatt gtgggacacc cacggcaccc cactgcactg 60

acaacacatg aaatgctcta ccgctggctg tgcacaaaag gttacgaggt catcgttgag 120

caacaaatcg ctcacgaact gcaactgaag aatgtgaaaa ctggcacgct cgcggagatt 180

gggcaactag ctgatctcgc ggtagtcgtt ggtggcgacg gtaatatgct gggcgcggca 240

cgcacactcg cccgttacga tattaaagtt attggaatca accgtggcaa cctgggtttc 300

ctgactgacc ttgaccccga taacgcccag caacagttag ccgatgtgct ggaaggccac 360

tacatcagcg agaaacgttt tttgctggaa gcgcaagtct gtcagcaaga ttgccagaaa 420

cgcatcagca ccgcgataaa tgaagtggtg cttcatccag gcaaagtggc gcatatgatt 480

gagttcgaag tgtatatcga cgagatcttt gcgttttctc agcgatctga tggactaatt 540

atttcgacgc caacaggctc caccgcctat tccctctctg caggcggtcc tattctgacc 600

ccctctctgg atgcgattac cctggtgccc atgttcccgc atacgttgtc agcacgacca 660

ctggtcataa acagcagcag cacgatccgt ctgcgttttt cgcatcgccg taacgacctg 720

gaaatcagtt gcgacagcca gatagcactg ccgattcagg aaggtgaaga tgtcctgatt 780

cgtcgctgtg attaccatct gaatctgatt catccgaaag attacagtta tttcaacaca 840

ttaagcacca agctcggctg gtcaaaaaaa ttattctaa 879

<210> 211
<211> 292
<212> PRT
<213> Escherichia coli

<400> 211

Met Asn Asn His Phe Lys Cys Ile Gly Ile Val Gly His Pro Arg His
1 5 10 15

Pro Thr Ala Leu Thr Thr His Glu Met Leu Tyr Arg Trp Leu Cys Thr

20 25 30

Lys Gly Tyr Glu Val Ile Val Glu Gln Gln Ile Ala His Glu Leu Gln
35 40 45

Leu Lys Asn Val Lys Thr Gly Thr Leu Ala Glu Ile Gly Gln Leu Ala
50 55 60

Asp Leu Ala Val Val Val Gly Gly Asp Gly Asn Met Leu Gly Ala Ala
65 70 75 80

Arg Thr Leu Ala Arg Tyr Asp Ile Lys Val Ile Gly Ile Asn Arg Gly
85 90 95

Asn Leu Gly Phe Leu Thr Asp Leu Asp Pro Asp Asn Ala Gln Gln Gln
100 105 110

Leu Ala Asp Val Leu Glu Gly His Tyr Ile Ser Glu Lys Arg Phe Leu
115 120 125

Leu Glu Ala Gln Val Cys Gln Gln Asp Cys Gln Lys Arg Ile Ser Thr
130 135 140

Ala Ile Asn Glu Val Val Leu His Pro Gly Lys Val Ala His Met Ile
145 150 155 160

Glu Phe Glu Val Tyr Ile Asp Glu Ile Phe Ala Phe Ser Gln Arg Ser
165 170 175

Asp Gly Leu Ile Ile Ser Thr Pro Thr Gly Ser Thr Ala Tyr Ser Leu
180 185 190

Ser Ala Gly Gly Pro Ile Leu Thr Pro Ser Leu Asp Ala Ile Thr Leu
195 200 205

Val Pro Met Phe Pro His Thr Leu Ser Ala Arg Pro Leu Val Ile Asn
210 215 220

Ser Ser Ser Thr Ile Arg Leu Arg Phe Ser His Arg Arg Asn Asp Leu
225 230 235 240

Glu Ile Ser Cys Asp Ser Gln Ile Ala Leu Pro Ile Gln Glu Gly Glu
245 250 255

Asp Val Leu Ile Arg Arg Cys Asp Tyr His Leu Asn Leu Ile His Pro
260 265 270

Lys Asp Tyr Ser Tyr Phe Asn Thr Leu Ser Thr Lys Leu Gly Trp Ser
        275                 280                 285

Lys Lys Leu Phe
    290

<210> 212
<211> 738
<212> DNA
<213> Escherichia coli

<400> 212
atggaaagcc ctactccaca gcctgctcct ggttcggcga ccttcatgga aggatgcaaa 60

gacagtttac cgattgttat tagttatatt ccggtggcct ttgcgttcgg tctgaatgcg 120

acccgtctgg gattctctcc tctcgaaagc gtttttttct cctgcatcat ttatgcaggc 180

gcgagccagt tcgtcattac cgcgatgctg gcagccggga gtagtttgtg gattgctgca 240

ctgaccgtca tggcaatgga tgttcgccat gtgttgtatg gcccgtcact gcgtagccgt 300

attattcagc gtctgcaaaa atcgaaaacc gccctgtggg cgtttggcct gacggatgag 360

gtttttgccg ccgcaaccgc aaaactggta cgcaataatc gccgctggag cgagaactgg 420

atgatcggca ttgccttcag ttcatggtca tcgtgggtat ttggtacggt aataggggca 480

ttctccggca gcggcttgct gcaaggttat cccgccgttg aagctgcatt aggttttatg 540

cttccggcac tctttatgag tttcctgctc gcctctttcc agcgcaaaca atctctttgc 600

gttaccgcag cgttagttgg tgcccttgca ggcgtaacgc tattttctat tcccgtcgcc 660

attctggcag gcattgtctg tggctgcctc actgcgttaa tccaggcatt ctggcaagga 720

gcgcccgatg agctatga 738

<210> 213
<211> 245
<212> PRT
<213> Escherichia coli

<400> 213

Met Glu Ser Pro Thr Pro Gln Pro Ala Pro Gly Ser Ala Thr Phe Met
1               5                   10                  15

Glu Gly Cys Lys Asp Ser Leu Pro Ile Val Ile Ser Tyr Ile Pro Val
            20                  25                  30

Ala Phe Ala Phe Gly Leu Asn Ala Thr Arg Leu Gly Phe Ser Pro Leu
        35                  40                  45

Glu Ser Val Phe Phe Ser Cys Ile Ile Tyr Ala Gly Ala Ser Gln Phe
    50                  55                  60

Val Ile Thr Ala Met Leu Ala Ala Gly Ser Ser Leu Trp Ile Ala Ala
65 70 75 80

Leu Thr Val Met Ala Met Asp Val Arg His Val Leu Tyr Gly Pro Ser
85 90 95

Leu Arg Ser Arg Ile Ile Gln Arg Leu Gln Lys Ser Lys Thr Ala Leu
100 105 110

Trp Ala Phe Gly Leu Thr Asp Glu Val Phe Ala Ala Ala Thr Ala Lys
115 120 125

Leu Val Arg Asn Asn Arg Arg Trp Ser Glu Asn Trp Met Ile Gly Ile
130 135 140

Ala Phe Ser Ser Trp Ser Ser Trp Val Phe Gly Thr Val Ile Gly Ala
145 150 155 160

Phe Ser Gly Ser Gly Leu Leu Gln Gly Tyr Pro Ala Val Glu Ala Ala
165 170 175

Leu Gly Phe Met Leu Pro Ala Leu Phe Met Ser Phe Leu Leu Ala Ser
180 185 190

Phe Gln Arg Lys Gln Ser Leu Cys Val Thr Ala Ala Leu Val Gly Ala
195 200 205

Leu Ala Gly Val Thr Leu Phe Ser Ile Pro Val Ala Ile Leu Ala Gly
210 215 220

Ile Val Cys Gly Cys Leu Thr Ala Leu Ile Gln Ala Phe Trp Gln Gly
225 230 235 240

Ala Pro Asp Glu Leu
245

<210> 214
<211> 336
<212> DNA
<213> Escherichia coli

<400> 214
atgagctatg aggttctgct gcttgggtta ctagttggcg tggcgaatta ttgcttccgc 60

tatttgccgc tgcgcctgcg tgtgggtaat gcccgcccaa ccaaacgtgg cgcggtaggt 120

attttgctcg acaccattgg catcgcctcg atatgcgctc tgctggttgt ctctaccgca 180

ccagaagtga tgcacgatac acgccgtttc gtgcccacgc tggtcggctt cgcggtactg 240

ggtgccagtt tctataaaac acgcagcatt atcatcccaa cactgcttag tgcgctggcc 300

tatgggctcg cctggaaagt gatggcgatt atataa 336

<210> 215
<211> 111
<212> PRT
<213> Escherichia coli

<400> 215

Met Ser Tyr Glu Val Leu Leu Leu Gly Leu Leu Val Gly Val Ala Asn
1 5 10 15

Tyr Cys Phe Arg Tyr Leu Pro Leu Arg Leu Arg Val Gly Asn Ala Arg
20 25 30

Pro Thr Lys Arg Gly Ala Val Gly Ile Leu Leu Asp Thr Ile Gly Ile
35 40 45

Ala Ser Ile Cys Ala Leu Leu Val Val Ser Thr Ala Pro Glu Val Met
50 55 60

His Asp Thr Arg Arg Phe Val Pro Thr Leu Val Gly Phe Ala Val Leu
65 70 75 80

Gly Ala Ser Phe Tyr Lys Thr Arg Ser Ile Ile Ile Pro Thr Leu Leu
85 90 95

Ser Ala Leu Ala Tyr Gly Leu Ala Trp Lys Val Met Ala Ile Ile
100 105 110

<210> 216
<211> 1032
<212> DNA
<213> Escherichia coli

<400> 216
atgataaaac tttcgaatat caccaaagtg ttccaccagg gcacccgcac catccaggcg 60

ttgaacaacg tcagcctgca tgtgccagct ggacaaattt atggcgttat cggtgcctca 120

ggcgcgggta agagtacgct tatacgttgt gtaaacctgc tggagcgccc aaccgagggt 180

agcgtgctgg tcgatggcca ggaactgacc acgctgtcag aatccgagtt gaccaaagct 240

cgccgccaga ttggtatgat tttccagcat tttaacctgc tctcttcgcg tactgttttt 300

ggcaacgtgg ctctgccgct ggagctggac aacacaccga aagacgaggt caaacgtcgc 360

gtgacggaat tgctgtcatt ggttggtctt ggcgataagc atgatagcta cccgtcgaat 420

ctttccggtg ggcagaaaca acgtgtggca attgcccgtg cgttagccag caatcccaaa 480

gtattgctgt gtgatgaagc caccagcgcg ctggacccgg caacgacacg ttctattctc 540

gaactgctga aagacatcaa ccgccgtctg gggttgacga ttctgttgat cacccacgaa 600

atggacgttg tgaagcgcat ttgtgattgc gtggcggtca tcagcaatgg agaactgatc 660

gagcaggaca cggtaagtga agtgttctcg catccgaaaa cgccgctggc gcagaagttt 720

attcagtcga ccctgcatct ggatatcccg gaagattacc aggaacgtct gcaagcggag 780

ccatttactg actgcgtgcc gatgctgcgt ctggagttta ccggtcaatc ggtcgatgcc 840

ccactgcttt ctgaaaccgc gcgtcgtttc aacgtcaaca acaacattat tagcgcgcag 900

atggattacg ccggtggcgt taagttcggc atcatgctga ctgaaatgca cggcacacaa 960

caagatacgc aagccgccat tgcctggctg caggaacacc atgtaaaagt agaggtactg 1020

ggttatgtct ga 1032

<210> 217
<211> 343
<212> PRT
<213> Escherichia coli

<400> 217

Met Ile Lys Leu Ser Asn Ile Thr Lys Val Phe His Gln Gly Thr Arg
1 5 10 15

Thr Ile Gln Ala Leu Asn Asn Val Ser Leu His Val Pro Ala Gly Gln
20 25 30

Ile Tyr Gly Val Ile Gly Ala Ser Gly Ala Gly Lys Ser Thr Leu Ile
35 40 45

Arg Cys Val Asn Leu Leu Glu Arg Pro Thr Glu Gly Ser Val Leu Val
50 55 60

Asp Gly Gln Glu Leu Thr Thr Leu Ser Glu Ser Glu Leu Thr Lys Ala
65 70 75 80

Arg Arg Gln Ile Gly Met Ile Phe Gln His Phe Asn Leu Leu Ser Ser
85 90 95

Arg Thr Val Phe Gly Asn Val Ala Leu Pro Leu Glu Leu Asp Asn Thr
100 105 110

Pro Lys Asp Glu Val Lys Arg Arg Val Thr Glu Leu Leu Ser Leu Val
115 120 125

Gly Leu Gly Asp Lys His Asp Ser Tyr Pro Ser Asn Leu Ser Gly Gly
130 135 140

Gln Lys Gln Arg Val Ala Ile Ala Arg Ala Leu Ala Ser Asn Pro Lys
145 150 155 160

Val Leu Leu Cys Asp Glu Ala Thr Ser Ala Leu Asp Pro Ala Thr Thr
165 170 175

Arg Ser Ile Leu Glu Leu Leu Lys Asp Ile Asn Arg Arg Leu Gly Leu
180 185 190

Thr Ile Leu Leu Ile Thr His Glu Met Asp Val Val Lys Arg Ile Cys
195 200 205

Asp Cys Val Ala Val Ile Ser Asn Gly Glu Leu Ile Glu Gln Asp Thr
210 215 220

Val Ser Glu Val Phe Ser His Pro Lys Thr Pro Leu Ala Gln Lys Phe
225 230 235 240

Ile Gln Ser Thr Leu His Leu Asp Ile Pro Glu Asp Tyr Gln Glu Arg
245 250 255

Leu Gln Ala Glu Pro Phe Thr Asp Cys Val Pro Met Leu Arg Leu Glu
260 265 270

Phe Thr Gly Gln Ser Val Asp Ala Pro Leu Leu Ser Glu Thr Ala Arg
275 280 285

Arg Phe Asn Val Asn Asn Asn Ile Ile Ser Ala Gln Met Asp Tyr Ala
290 295 300

Gly Gly Val Lys Phe Gly Ile Met Leu Thr Glu Met His Gly Thr Gln
305 310 315 320

Gln Asp Thr Gln Ala Ala Ile Ala Trp Leu Gln Glu His His Val Lys
325 330 335

Val Glu Val Leu Gly Tyr Val
340

<210> 218
<211> 654
<212> DNA
<213> Escherichia coli

<400> 218
atgtctgagc cgatgatgtg gctgctggtt cgtggcgtat gggaaacgct ggcaatgacc 60

ttcgtatccg gttttttgg ctttgtgatt ggtctgccgg ttggcgttct gctttatgtc 120

acgcgtccgg ggcaaattat tgctaacgcg aagctgtatc gtaccgtttc tgcgattgtg 180

aacattttcc gttccatccc gttcattatc ttgcttgtat ggatgattcc gtttacccgc 240

gttattgtcg gtacatcgat tggtttgcag gcagcgattg ttccgttaac cgttggtgca 300

gcaccgttta ttgcccgtat ggtcgagaac gctctgctgg agatcccaac cgggttaatt 360

gaagcttccc gcgcaatggg tgccacgccg atgcagatcg tccgtaaggt gctgttaccg 420

gaagcgctgc cgggtctggt gaatgcggca actatcaccc tgattaccct ggtcggttat 480

tccgcgatgg gtggtgcagt cggtgccggt ggtttaggtc agattggcta tcagtatggc 540

tacatcggct ataacgcgac ggtgatgaat acggtactgg tattgctggt cattctggtt 600

tatttaattc agttcgcagg cgaccgcatc gtccgggctg tcactcgcaa gtaa 654

<210> 219
<211> 217
<212> PRT
<213> Escherichia coli

<400> 219

Met Ser Glu Pro Met Met Trp Leu Leu Val Arg Gly Val Trp Glu Thr
1 5 10 15

Leu Ala Met Thr Phe Val Ser Gly Phe Phe Gly Phe Val Ile Gly Leu
20 25 30

Pro Val Gly Val Leu Leu Tyr Val Thr Arg Pro Gly Gln Ile Ile Ala
35 40 45

Asn Ala Lys Leu Tyr Arg Thr Val Ser Ala Ile Val Asn Ile Phe Arg
50 55 60

Ser Ile Pro Phe Ile Ile Leu Leu Val Trp Met Ile Pro Phe Thr Arg
65 70 75 80

Val Ile Val Gly Thr Ser Ile Gly Leu Gln Ala Ala Ile Val Pro Leu
85 90 95

Thr Val Gly Ala Ala Pro Phe Ile Ala Arg Met Val Glu Asn Ala Leu
100 105 110

Leu Glu Ile Pro Thr Gly Leu Ile Glu Ala Ser Arg Ala Met Gly Ala
115 120 125

Thr Pro Met Gln Ile Val Arg Lys Val Leu Leu Pro Glu Ala Leu Pro
130 135 140

Gly Leu Val Asn Ala Ala Thr Ile Thr Leu Ile Thr Leu Val Gly Tyr
145 150 155 160

```
Ser Ala Met Gly Gly Ala Val Gly Ala Gly Gly Leu Gly Gln Ile Gly
                165                 170                 175

Tyr Gln Tyr Gly Tyr Ile Gly Tyr Asn Ala Thr Val Met Asn Thr Val
            180                 185                 190

Leu Val Leu Leu Val Ile Leu Val Tyr Leu Ile Gln Phe Ala Gly Asp
        195                 200                 205

Arg Ile Val Arg Ala Val Thr Arg Lys
    210                 215


<210>  220
<211>  816
<212>  DNA
<213>  Escherichia coli

<400>  220
atggcgttca aattcaaaac ctttgcggca gtgggagccc tgatcggatc actggcactg      60

gtaggctgcg gtcaggatga aaaagatcca aaccacatta aagtcggcgt gattgttggt     120

gccgaacagc aggttgcaga agtcgcgcag aaagttgcga aagacaaata tggcctggac     180

gttgagctgg taaccttcaa cgactatgtt ctgccaaacg aagcattgag caaaggcgat     240

atcgacgcca acgccttcca gcataaaccg taccttgatc agcaactgaa agatcgtggc     300

tacaaactgg tcgcagtagg caacactttt gtttatccga ttgctggtta ctccaagaaa     360

atcaaatcac tggatgaact gcaggatggt tcgcaggttg ccgtgccaaa cgacccaact     420

aaccttggtc gttcactgct gctgctgcaa aaagtgggct tgatcaaact gaaagatggc     480

gttggcctgc tgccgaccgt tcttgatgtt gttgagaacc ccaaaaatct gaaaattgtt     540

gaactggaag caccgcaact gccgcgttct ctggacgacg cgcaaatcgc tctggcagtt     600

atcaatacca cctatgccag ccagattggc ctgactccgg cgaaagacgg tatctttgtt     660

gaagataaag agtccccgta cgtaaacctg atcgtgacgc gtgaagataa caaagacgcc     720

gagaacgtga agaaattcgt ccaggcttat cagtctgacg aagtttacga agcagcaaac     780

aaagtgttta acggcggagc tgttaaaggc tggtaa                               816


<210>  221
<211>  271
<212>  PRT
<213>  Escherichia coli

<400>  221

Met Ala Phe Lys Phe Lys Thr Phe Ala Ala Val Gly Ala Leu Ile Gly
1               5                   10                  15
```

Ser Leu Ala Leu Val Gly Cys Gly Gln Asp Glu Lys Asp Pro Asn His
20 25 30

Ile Lys Val Gly Val Ile Val Gly Ala Glu Gln Gln Val Ala Glu Val
35 40 45

Ala Gln Lys Val Ala Lys Asp Lys Tyr Gly Leu Asp Val Glu Leu Val
50 55 60

Thr Phe Asn Asp Tyr Val Leu Pro Asn Glu Ala Leu Ser Lys Gly Asp
65 70 75 80

Ile Asp Ala Asn Ala Phe Gln His Lys Pro Tyr Leu Asp Gln Gln Leu
85 90 95

Lys Asp Arg Gly Tyr Lys Leu Val Ala Val Gly Asn Thr Phe Val Tyr
100 105 110

Pro Ile Ala Gly Tyr Ser Lys Lys Ile Lys Ser Leu Asp Glu Leu Gln
115 120 125

Asp Gly Ser Gln Val Ala Val Pro Asn Asp Pro Thr Asn Leu Gly Arg
130 135 140

Ser Leu Leu Leu Leu Gln Lys Val Gly Leu Ile Lys Leu Lys Asp Gly
145 150 155 160

Val Gly Leu Leu Pro Thr Val Leu Asp Val Val Glu Asn Pro Lys Asn
165 170 175

Leu Lys Ile Val Glu Leu Glu Ala Pro Gln Leu Pro Arg Ser Leu Asp
180 185 190

Asp Ala Gln Ile Ala Leu Ala Val Ile Asn Thr Thr Tyr Ala Ser Gln
195 200 205

Ile Gly Leu Thr Pro Ala Lys Asp Gly Ile Phe Val Glu Asp Lys Glu
210 215 220

Ser Pro Tyr Val Asn Leu Ile Val Thr Arg Glu Asp Asn Lys Asp Ala
225 230 235 240

Glu Asn Val Lys Lys Phe Val Gln Ala Tyr Gln Ser Asp Glu Val Tyr
245 250 255

Glu Ala Ala Asn Lys Val Phe Asn Gly Gly Ala Val Lys Gly Trp
260 265 270

<210> 222
<211> 1503
<212> DNA
<213> Escherichia coli

<400> 222
atgctgaaaa ggaaaaaagt aaaaccgatt acccttcgtg atgtcaccat tattgatgac 60

ggtaaactgc gtaaagccat taccgcagca tcactgggta atgcaatgga atggttcgat 120

tttggtgttt atggttttgt tgcttacgca ttaggtaaag tttttttccc gggggctgac 180

cccagcgtgc agatggttgc tgcacttgcc actttctccg ttccctttct gattcgaccg 240

cttggcggac tcttctttgg tatgttgggc gataaatatg gtcgccagaa gatcctcgct 300

atcactattg tgattatgtc gatcagtacg ttctgtattg gcttaatacc gtcctacgac 360

acgattggta tttgggcacc gattctgctg ttgatctgta agatggcaca aggtttctcg 420

gtcggcggtg aatataccgg ggcgtcgata tttgttgcgg aatactcccc tgaccgtaaa 480

cgtggcttta tgggcagctg gctggacttc ggttctattg ccgggtttgt gctgggtgcg 540

ggcgtggtgg tgttaatttc gaccattgtc ggcgaagcga acttcctcga ttggggctgg 600

cgtattccgt tctttatcgc tctgccgtta gggattatcg ggctttacct gcgccatgcg 660

ctggaagaga ctccggcgtt ccagcagcat gtcgataaac tggaacaggg cgaccgtgaa 720

ggtttgcagg atggcccgaa agtctcgttt aaagagattg ccactaaata ctggcgcagc 780

ctgttgacat gtattggtct ggtaattgcc accaacgtga cttactacat gttgctgacc 840

tatatgccga gttatttgtc gcataacctg cattactccg aagaccacgg ggtgctgatt 900

attatcgcca ttatgatcgg tatgctgttt gtccagccgg tgatgggctt gctgagtgac 960

cgttttggcc gtcgtccgtt tgtgctactt ggtagtgttg ccctgtttgt gttggcgatc 1020

ccggcgttta ttctgattaa cagtaacgtc atcggcctga tttttgccgg gttactgatg 1080

ctggcggtga tccttaactg ctttacgggc gttatggctt ctaccttgcc agcgatgttc 1140

ccgacgcata tccgttacag cgcgctggcg gcggcattta atatttcggt gctggttgcc 1200

ggtctgacgc caacgctggc ggcctggctg gtcgaaagct cgcagaatct gatgatgcct 1260

gcctattacc tgatggtagt ggcggtggtt ggtttaatca ccggcgtaac catgaaagag 1320

acggcaaatc gtccgttgaa aggtgcgaca ccggcggcgt cagatataca ggaagcgaag 1380

gaaattctcg tcgagcatta cgataatatc gagcagaaaa tcgatgatat tgaccacgag 1440

attgccgatt tgcaggcgaa acgtacccgc ctggtgcagc aacatccgcg aattgatgaa 1500

taa 1503

<210> 223
<211> 500

<212> PRT
<213> Escherichia coli

<400> 223

Met Leu Lys Arg Lys Lys Val Lys Pro Ile Thr Leu Arg Asp Val Thr
1 5 10 15

Ile Ile Asp Asp Gly Lys Leu Arg Lys Ala Ile Thr Ala Ala Ser Leu
20 25 30

Gly Asn Ala Met Glu Trp Phe Asp Phe Gly Val Tyr Gly Phe Val Ala
35 40 45

Tyr Ala Leu Gly Lys Val Phe Phe Pro Gly Ala Asp Pro Ser Val Gln
50 55 60

Met Val Ala Ala Leu Ala Thr Phe Ser Val Pro Phe Leu Ile Arg Pro
65 70 75 80

Leu Gly Gly Leu Phe Phe Gly Met Leu Gly Asp Lys Tyr Gly Arg Gln
85 90 95

Lys Ile Leu Ala Ile Thr Ile Val Ile Met Ser Ile Ser Thr Phe Cys
100 105 110

Ile Gly Leu Ile Pro Ser Tyr Asp Thr Ile Gly Ile Trp Ala Pro Ile
115 120 125

Leu Leu Leu Ile Cys Lys Met Ala Gln Gly Phe Ser Val Gly Gly Glu
130 135 140

Tyr Thr Gly Ala Ser Ile Phe Val Ala Glu Tyr Ser Pro Asp Arg Lys
145 150 155 160

Arg Gly Phe Met Gly Ser Trp Leu Asp Phe Gly Ser Ile Ala Gly Phe
165 170 175

Val Leu Gly Ala Gly Val Val Val Leu Ile Ser Thr Ile Val Gly Glu
180 185 190

Ala Asn Phe Leu Asp Trp Gly Trp Arg Ile Pro Phe Phe Ile Ala Leu
195 200 205

Pro Leu Gly Ile Ile Gly Leu Tyr Leu Arg His Ala Leu Glu Glu Thr
210 215 220

Pro Ala Phe Gln Gln His Val Asp Lys Leu Glu Gln Gly Asp Arg Glu
225 230 235 240

```
Gly Leu Gln Asp Gly Pro Lys Val Ser Phe Lys Glu Ile Ala Thr Lys
                245                 250                 255

Tyr Trp Arg Ser Leu Leu Thr Cys Ile Gly Leu Val Ile Ala Thr Asn
            260                 265                 270

Val Thr Tyr Tyr Met Leu Leu Thr Tyr Met Pro Ser Tyr Leu Ser His
        275                 280                 285

Asn Leu His Tyr Ser Glu Asp His Gly Val Leu Ile Ile Ile Ala Ile
    290                 295                 300

Met Ile Gly Met Leu Phe Val Gln Pro Val Met Gly Leu Leu Ser Asp
305                 310                 315                 320

Arg Phe Gly Arg Arg Pro Phe Val Leu Leu Gly Ser Val Ala Leu Phe
                325                 330                 335

Val Leu Ala Ile Pro Ala Phe Ile Leu Ile Asn Ser Asn Val Ile Gly
            340                 345                 350

Leu Ile Phe Ala Gly Leu Leu Met Leu Ala Val Ile Leu Asn Cys Phe
        355                 360                 365

Thr Gly Val Met Ala Ser Thr Leu Pro Ala Met Phe Pro Thr His Ile
    370                 375                 380

Arg Tyr Ser Ala Leu Ala Ala Ala Phe Asn Ile Ser Val Leu Val Ala
385                 390                 395                 400

Gly Leu Thr Pro Thr Leu Ala Ala Trp Leu Val Glu Ser Ser Gln Asn
                405                 410                 415

Leu Met Met Pro Ala Tyr Tyr Leu Met Val Val Ala Val Val Gly Leu
            420                 425                 430

Ile Thr Gly Val Thr Met Lys Glu Thr Ala Asn Arg Pro Leu Lys Gly
        435                 440                 445

Ala Thr Pro Ala Ala Ser Asp Ile Gln Glu Ala Lys Glu Ile Leu Val
    450                 455                 460

Glu His Tyr Asp Asn Ile Glu Gln Lys Ile Asp Asp Ile Asp His Glu
465                 470                 475                 480

Ile Ala Asp Leu Gln Ala Lys Arg Thr Arg Leu Val Gln Gln His Pro
```

485 490 495

Arg Ile Asp Glu
500

<210> 224
<211> 1257
<212> DNA
<213> Escherichia coli

<400> 224
atgagtggac tcaaacaaga actggggctg gcccagggca ttggcctgct atcgacgtca 60

ttattaggca ctggcgtgtt tgccgttcct gcgttagctg cgctggtagc gggcaataac 120

agcctgtggg cgtggcccgt tttgattatc ttagtgttcc cgattgcgat tgtgtttgcg 180

attctgggtc gccactatcc cagcgcaggc ggcgtcgcgc acttcgtcgg tatggcgttt 240

ggttcgcggc ttgagcgagt caccggctgg ctgtttttat cggtcattcc cgtgggtttg 300

cctgccgcac tacaaattgc cgccgggttc ggccaggcga tgtttggctg gcatagctgg 360

caactgttgt tggcagaact cggtacgctg gcgctggtgt ggtatatcgg tactcgcggt 420

gccagttcca gtgctaatct acaaaccgtt attgccggac ttatcgtcgc gctgattgtc 480

gctatctggt gggcgggcga tatcaaacct gcgaatatcc cctttccggc acctggtaat 540

atcgaactta ccgggttatt tgctgcgtta tcagtgatgt tctggtgttt tgtcggtctg 600

gaggcatttg cccatctcgc ctcggaattt aaaaatccag agcgtgattt tcctcgtgct 660

ttgatgattg gtctgctgct ggcaggatta gtctactggg gctgtacggt agtcgtctta 720

cacttcgacg cctatggtga aaaaatggcg gcggcagcat cgcttccaaa aattgtagtg 780

cagttgttcg gtgtaggagc gttatggatt gcctgcgtga ttggctatct ggcctgcttt 840

gccagtctca acatttatat acagagcttc gcccgcctgg tctggtcgca ggcgcaacat 900

aatcctgacc actacctggc acgcctctct tctcgccata tcccgaataa tgccctcaat 960

gcggtgctcg gctgctgtgt ggtgagcact ttggtgattc atgctttaga gatcaatctg 1020

gacgctctta ttatttatgc caatggcatc tttattatga tttatctgtt atgcatgctg 1080

gcaggctgta aattattgca aggacgttat cgactactgg cggtggttgg cgggctgtta 1140

tgcgttctgt tactggcaat ggtcggctgg aaaagtctct atgcgctgat catgctggcg 1200

gggttatggc tgttgctgcc aaaacgaaaa acgccggaaa atggcataac cacataa 1257

<210> 225
<211> 418
<212> PRT
<213> Escherichia coli

<400> 225

```
Met Ser Gly Leu Lys Gln Glu Leu Gly Leu Ala Gln Gly Ile Gly Leu
1               5                   10                  15

Leu Ser Thr Ser Leu Leu Gly Thr Gly Val Phe Ala Val Pro Ala Leu
            20                  25                  30

Ala Ala Leu Val Ala Gly Asn Asn Ser Leu Trp Ala Trp Pro Val Leu
        35                  40                  45

Ile Ile Leu Val Phe Pro Ile Ala Ile Val Phe Ala Ile Leu Gly Arg
    50                  55                  60

His Tyr Pro Ser Ala Gly Gly Val Ala His Phe Val Gly Met Ala Phe
65                  70                  75                  80

Gly Ser Arg Leu Glu Arg Val Thr Gly Trp Leu Phe Leu Ser Val Ile
                85                  90                  95

Pro Val Gly Leu Pro Ala Ala Leu Gln Ile Ala Ala Gly Phe Gly Gln
            100                 105                 110

Ala Met Phe Gly Trp His Ser Trp Gln Leu Leu Leu Ala Glu Leu Gly
        115                 120                 125

Thr Leu Ala Leu Val Trp Tyr Ile Gly Thr Arg Gly Ala Ser Ser Ser
    130                 135                 140

Ala Asn Leu Gln Thr Val Ile Ala Gly Leu Ile Val Ala Leu Ile Val
145                 150                 155                 160

Ala Ile Trp Trp Ala Gly Asp Ile Lys Pro Ala Asn Ile Pro Phe Pro
                165                 170                 175

Ala Pro Gly Asn Ile Glu Leu Thr Gly Leu Phe Ala Ala Leu Ser Val
            180                 185                 190

Met Phe Trp Cys Phe Val Gly Leu Glu Ala Phe Ala His Leu Ala Ser
        195                 200                 205

Glu Phe Lys Asn Pro Glu Arg Asp Phe Pro Arg Ala Leu Met Ile Gly
    210                 215                 220

Leu Leu Leu Ala Gly Leu Val Tyr Trp Gly Cys Thr Val Val Val Leu
225                 230                 235                 240

His Phe Asp Ala Tyr Gly Glu Lys Met Ala Ala Ala Ala Ser Leu Pro
                245                 250                 255
```

Lys Ile Val Val Gln Leu Phe Gly Val Gly Ala Leu Trp Ile Ala Cys
260 265 270

Val Ile Gly Tyr Leu Ala Cys Phe Ala Ser Leu Asn Ile Tyr Ile Gln
275 280 285

Ser Phe Ala Arg Leu Val Trp Ser Gln Ala Gln His Asn Pro Asp His
290 295 300

Tyr Leu Ala Arg Leu Ser Ser Arg His Ile Pro Asn Asn Ala Leu Asn
305 310 315 320

Ala Val Leu Gly Cys Cys Val Val Ser Thr Leu Val Ile His Ala Leu
325 330 335

Glu Ile Asn Leu Asp Ala Leu Ile Ile Tyr Ala Asn Gly Ile Phe Ile
340 345 350

Met Ile Tyr Leu Leu Cys Met Leu Ala Gly Cys Lys Leu Leu Gln Gly
355 360 365

Arg Tyr Arg Leu Leu Ala Val Val Gly Gly Leu Leu Cys Val Leu Leu
370 375 380

Leu Ala Met Val Gly Trp Lys Ser Leu Tyr Ala Leu Ile Met Leu Ala
385 390 395 400

Gly Leu Trp Leu Leu Leu Pro Lys Arg Lys Thr Pro Glu Asn Gly Ile
405 410 415

Thr Thr

<210> 226
<211> 519
<212> DNA
<213> Escherichia coli

<400> 226
atgtccatcc gttttgcccg caaagccgac tgtgctgcca ttgcggaaat ttataaccac 60

gccgtgttgt atacggcggc tatctggaat gaccaaacgg tggatgctga taaccgcatt 120

gcctggtttg aagcgcggac tttagcaggt tatccagtgc tggtgagcga ggaaaacggc 180

gtagtgacgg gatatgcctc gtttggcgac tggcgtagtt tcgatggttt tcgccatacc 240

gtggaacatt cggtttatgt ccatcccgat catcagggca aaggtctggg gcgtaaattg 300

ttaagccgat tgattgatga agcgcgggat tgcgggaagc atgtcatggt cgccgggatc 360

gaatcgcaaa atcaggcctc gctgcatctc caccagtcgc tgggatttgt cgtcaccgcg 420

caaatgccgc aggtaggcac taaatttggt cgttggctgg atctgacatt tatgcagttg 480

caactcgacg agcgcactga accggacgcg attggatga 519

<210> 227
<211> 172
<212> PRT
<213> Escherichia coli

<400> 227

Met Ser Ile Arg Phe Ala Arg Lys Ala Asp Cys Ala Ala Ile Ala Glu
1 5 10 15

Ile Tyr Asn His Ala Val Leu Tyr Thr Ala Ala Ile Trp Asn Asp Gln
20 25 30

Thr Val Asp Ala Asp Asn Arg Ile Ala Trp Phe Glu Ala Arg Thr Leu
35 40 45

Ala Gly Tyr Pro Val Leu Val Ser Glu Glu Asn Gly Val Val Thr Gly
50 55 60

Tyr Ala Ser Phe Gly Asp Trp Arg Ser Phe Asp Gly Phe Arg His Thr
65 70 75 80

Val Glu His Ser Val Tyr Val His Pro Asp His Gln Gly Lys Gly Leu
85 90 95

Gly Arg Lys Leu Leu Ser Arg Leu Ile Asp Glu Ala Arg Asp Cys Gly
100 105 110

Lys His Val Met Val Ala Gly Ile Glu Ser Gln Asn Gln Ala Ser Leu
115 120 125

His Leu His Gln Ser Leu Gly Phe Val Val Thr Ala Gln Met Pro Gln
130 135 140

Val Gly Thr Lys Phe Gly Arg Trp Leu Asp Leu Thr Phe Met Gln Leu
145 150 155 160

Gln Leu Asp Glu Arg Thr Glu Pro Asp Ala Ile Gly
165 170

<210> 228
<211> 1332
<212> DNA
<213> Escherichia coli

<400> 228
gtggtaaagg aacgtaaaac cgagttggtc gagggattcc gccattcggt tccctatatc 60

aatacccacc ggggaaaaac gtttgtcatc atgctcggcg gtgaagccat tgagcatgag 120

aatttctcca gtatcgttaa tgatatcggg ttgttgcaca gcctcggcat ccgtctggtg 180

gtggtctatg gcgcacgtcc gcagatcgac gcaaatctgg ctgcgcatca ccacgaaccg 240

ctgtatcaca agaatatacg tgtgaccgac gccaaaacac tggaactggt gaagcaggct 300

gcgggaacat tgcaactgga tattactgct cgcctgtcga tgagtctcaa taacacgccg 360

ctgcagggcg cgcatatcaa cgtcgtcagt ggcaatttta ttattgccca gccgctgggc 420

gtcgatgacg gcgtggatta ctgccatagc gggcgtatcc ggcggattga tgaagacgcg 480

atccatcgtc aactggacag cggtgcaata gtgctaatgg ggccggtcgc tgtttcagtc 540

actggcgaga gctttaacct gacctcggaa gagattgcca ctcaactggc catcaaactg 600

aaagctgaaa agatgattgg tttttgctct tcccagggcg tcactaatga cgacggtgat 660

attgtctccg aacttttccc taacgaagcg caagcgcggg tagaagccca ggaagagaaa 720

ggcgattaca actccggtac ggtgcgcttt ttgcgtggcg cagtgaaagc ctgccgcagc 780

ggcgtgcgtc gctgtcattt aatcagttat caggaagatg gcgcgctgtt gcaagagttg 840

ttctcacgcg acggtatcgg tacgcagatt gtgatggaaa gcgccgagca gattcgtcgc 900

gcaacaatca acgatattgg cggtattctg gagttgattc gcccactgga gcagcaaggt 960

attctggtac gccgttctcg cgagcagctg gagatggaaa tcgacaaatt caccattatt 1020

cagcgcgata acacgactat tgcctgcgcc gcgctctatc cgttcccgga agagaagatt 1080

ggggaaatgg cctgtgtggc agttcacccg gattaccgca gttcatcaag ggtgaagtt 1140

ctgctggaac gcattgccgc tcaggcgaag cagagcggct taagcaaatt gtttgtgctg 1200

accacgcgca gtattcactg gttccaggaa cgtggattta ccccagtgga tattgattta 1260

ctgcccgaga gcaaaaagca gttgtacaac taccagcgta aatccaaagt gttgatggcg 1320

gatttagggt aa 1332

<210> 229
<211> 443
<212> PRT
<213> Escherichia coli

<400> 229

Met Val Lys Glu Arg Lys Thr Glu Leu Val Glu Gly Phe Arg His Ser
1               5                   10                  15

Val Pro Tyr Ile Asn Thr His Arg Gly Lys Thr Phe Val Ile Met Leu
            20                  25                  30

Gly Gly Glu Ala Ile Glu His Glu Asn Phe Ser Ser Ile Val Asn Asp
35 40 45

Ile Gly Leu Leu His Ser Leu Gly Ile Arg Leu Val Val Val Tyr Gly
50 55 60

Ala Arg Pro Gln Ile Asp Ala Asn Leu Ala Ala His His His Glu Pro
65 70 75 80

Leu Tyr His Lys Asn Ile Arg Val Thr Asp Ala Lys Thr Leu Glu Leu
85 90 95

Val Lys Gln Ala Ala Gly Thr Leu Gln Leu Asp Ile Thr Ala Arg Leu
100 105 110

Ser Met Ser Leu Asn Asn Thr Pro Leu Gln Gly Ala His Ile Asn Val
115 120 125

Val Ser Gly Asn Phe Ile Ile Ala Gln Pro Leu Gly Val Asp Asp Gly
130 135 140

Val Asp Tyr Cys His Ser Gly Arg Ile Arg Arg Ile Asp Glu Asp Ala
145 150 155 160

Ile His Arg Gln Leu Asp Ser Gly Ala Ile Val Leu Met Gly Pro Val
165 170 175

Ala Val Ser Val Thr Gly Glu Ser Phe Asn Leu Thr Ser Glu Glu Ile
180 185 190

Ala Thr Gln Leu Ala Ile Lys Leu Lys Ala Glu Lys Met Ile Gly Phe
195 200 205

Cys Ser Ser Gln Gly Val Thr Asn Asp Asp Gly Asp Ile Val Ser Glu
210 215 220

Leu Phe Pro Asn Glu Ala Gln Ala Arg Val Glu Ala Gln Glu Glu Lys
225 230 235 240

Gly Asp Tyr Asn Ser Gly Thr Val Arg Phe Leu Arg Gly Ala Val Lys
245 250 255

Ala Cys Arg Ser Gly Val Arg Arg Cys His Leu Ile Ser Tyr Gln Glu
260 265 270

Asp Gly Ala Leu Leu Gln Glu Leu Phe Ser Arg Asp Gly Ile Gly Thr
275 280 285

Gln Ile Val Met Glu Ser Ala Glu Gln Ile Arg Arg Ala Thr Ile Asn
290 295 300

Asp Ile Gly Gly Ile Leu Glu Leu Ile Arg Pro Leu Glu Gln Gln Gly
305 310 315 320

Ile Leu Val Arg Arg Ser Arg Glu Gln Leu Glu Met Glu Ile Asp Lys
325 330 335

Phe Thr Ile Ile Gln Arg Asp Asn Thr Thr Ile Ala Cys Ala Ala Leu
340 345 350

Tyr Pro Phe Pro Glu Glu Lys Ile Gly Glu Met Ala Cys Val Ala Val
355 360 365

His Pro Asp Tyr Arg Ser Ser Ser Arg Gly Glu Val Leu Leu Glu Arg
370 375 380

Ile Ala Ala Gln Ala Lys Gln Ser Gly Leu Ser Lys Leu Phe Val Leu
385 390 395 400

Thr Thr Arg Ser Ile His Trp Phe Gln Glu Arg Gly Phe Thr Pro Val
405 410 415

Asp Ile Asp Leu Leu Pro Glu Ser Lys Lys Gln Leu Tyr Asn Tyr Gln
420 425 430

Arg Lys Ser Lys Val Leu Met Ala Asp Leu Gly
435 440

<210> 230
<211> 1545
<212> DNA
<213> Escherichia coli

<400> 230
atggctgact cgcaacccct gtccggtgct ccggaaggtg ccgaatattt aagagcagtg 60

ctgcgcgcgc cggtttacga ggcggcgcag gttacgccgc tacaaaaaat ggaaaaactg 120

tcgtcgcgtc ttgataacgt cattctggtg aagcgcgaag atcgccagcc agtgcacagc 180

tttaagctgc gcggcgcata cgccatgatg gcgggcctga cggaagaaca gaaagcgcac 240

ggcgtgatca ctgcttctgc gggtaaccac gcgcagggcg tcgcgttttc ttctgcgcgg 300

ttaggcgtga aggccctgat cgttatgcca accgccaccg ccgacatcaa agtcgacgcg 360

gtgcgcggct tcggcggcga agtgctgctc cacggcgcga actttgatga agcgaaagcc 420

aaagcgatcg aactgtcaca gcagcagggg ttcacctggg tgccgccgtt cgaccatccg 480

atggtgattg ccgggcaagg cacgctggcg ctggaactgc tccagcagga cgcccatctc 540

gaccgcgtat ttgtgccagt cggcggcggc ggtctggctg ctggcgtggc ggtgctgatc 600

aaacaactga tgccgcaaat caaagtgatc gccgtagaag cggaagactc cgcctgcctg 660

aaagcagcgc tggatgcggg tcatccggtt gatctgccgc gcgtagggct atttgctgaa 720

ggcgtagcgg taaaacgcat cggtgacgaa accttccgtt tatgccagga gtatctcgac 780

gacatcatca ccgtcgatag cgatgcgatc tgtgcggcga tgaaggattt attcgaagat 840

gtgcgcgcgg tggcggaacc ctctggcgcg ctggcgctgg cgggaatgaa aaaatatatc 900

gccctgcaca acattcgcgg cgaacggctg gcgcatattc tttccggtgc caacgtgaac 960

ttccacggcc tgcgctacgt ctcagaacgc tgcgaactgg gcgaacagcg tgaagcgttg 1020

ttggcggtga ccattccgga agaaaaaggc agcttcctca aattctgcca actgcttggc 1080

gggcgttcgg tcaccgagtt caactaccgt tttgccgatg ccaaaaacgc ctgcatcttt 1140

gtcggtgtgc gcctgagccg cggcctcgaa gagcgcaaag aaattttgca gatgctcaac 1200

gacggcggct acagcgtggt tgatctctcc gacgacgaaa tggcgaagct acacgtgcgc 1260

tatatggtcg gcggacgtcc atcgcatccg ttgcaggaac gcctctacag cttcgaattc 1320

ccggaatcac cgggcgcgct gctgcgcttc ctcaacacgc tgggtacgta ctggaacatt 1380

tctttgttcc actatcgcag ccatggcacc gactacgggc gcgtactggc ggcgttcgaa 1440

cttggcgacc atgaaccgga tttcgaaacc cggctgaatg agctgggcta cgattgccac 1500

gacgaaacca ataacccggc gttcaggttc tttttggcgg gttag 1545

<210> 231
<211> 514
<212> PRT
<213> Escherichia coli

<400> 231

Met Ala Asp Ser Gln Pro Leu Ser Gly Ala Pro Glu Gly Ala Glu Tyr
1 5 10 15

Leu Arg Ala Val Leu Arg Ala Pro Val Tyr Glu Ala Ala Gln Val Thr
20 25 30

Pro Leu Gln Lys Met Glu Lys Leu Ser Ser Arg Leu Asp Asn Val Ile
35 40 45

Leu Val Lys Arg Glu Asp Arg Gln Pro Val His Ser Phe Lys Leu Arg
50 55 60

Gly Ala Tyr Ala Met Met Ala Gly Leu Thr Glu Glu Gln Lys Ala His
65 70 75 80

```
Gly Val Ile Thr Ala Ser Ala Gly Asn His Ala Gln Gly Val Ala Phe
                85                  90                  95

Ser Ser Ala Arg Leu Gly Val Lys Ala Leu Ile Val Met Pro Thr Ala
            100                 105                 110

Thr Ala Asp Ile Lys Val Asp Ala Val Arg Gly Phe Gly Gly Glu Val
        115                 120                 125

Leu Leu His Gly Ala Asn Phe Asp Glu Ala Lys Ala Lys Ala Ile Glu
    130                 135                 140

Leu Ser Gln Gln Gln Gly Phe Thr Trp Val Pro Pro Phe Asp His Pro
145                 150                 155                 160

Met Val Ile Ala Gly Gln Gly Thr Leu Ala Leu Glu Leu Leu Gln Gln
                165                 170                 175

Asp Ala His Leu Asp Arg Val Phe Val Pro Val Gly Gly Gly Gly Leu
            180                 185                 190

Ala Ala Gly Val Ala Val Leu Ile Lys Gln Leu Met Pro Gln Ile Lys
        195                 200                 205

Val Ile Ala Val Glu Ala Glu Asp Ser Ala Cys Leu Lys Ala Ala Leu
    210                 215                 220

Asp Ala Gly His Pro Val Asp Leu Pro Arg Val Gly Leu Phe Ala Glu
225                 230                 235                 240

Gly Val Ala Val Lys Arg Ile Gly Asp Glu Thr Phe Arg Leu Cys Gln
                245                 250                 255

Glu Tyr Leu Asp Asp Ile Ile Thr Val Asp Ser Asp Ala Ile Cys Ala
            260                 265                 270

Ala Met Lys Asp Leu Phe Glu Asp Val Arg Ala Val Ala Glu Pro Ser
        275                 280                 285

Gly Ala Leu Ala Leu Ala Gly Met Lys Lys Tyr Ile Ala Leu His Asn
    290                 295                 300

Ile Arg Gly Glu Arg Leu Ala His Ile Leu Ser Gly Ala Asn Val Asn
305                 310                 315                 320

Phe His Gly Leu Arg Tyr Val Ser Glu Arg Cys Glu Leu Gly Glu Gln
                325                 330                 335
```

Arg Glu Ala Leu Leu Ala Val Thr Ile Pro Glu Glu Lys Gly Ser Phe
340 345 350

Leu Lys Phe Cys Gln Leu Leu Gly Gly Arg Ser Val Thr Glu Phe Asn
355 360 365

Tyr Arg Phe Ala Asp Ala Lys Asn Ala Cys Ile Phe Val Gly Val Arg
370 375 380

Leu Ser Arg Gly Leu Glu Glu Arg Lys Glu Ile Leu Gln Met Leu Asn
385 390 395 400

Asp Gly Gly Tyr Ser Val Val Asp Leu Ser Asp Asp Glu Met Ala Lys
405 410 415

Leu His Val Arg Tyr Met Val Gly Gly Arg Pro Ser His Pro Leu Gln
420 425 430

Glu Arg Leu Tyr Ser Phe Glu Phe Pro Glu Ser Pro Gly Ala Leu Leu
435 440 445

Arg Phe Leu Asn Thr Leu Gly Thr Tyr Trp Asn Ile Ser Leu Phe His
450 455 460

Tyr Arg Ser His Gly Thr Asp Tyr Gly Arg Val Leu Ala Ala Phe Glu
465 470 475 480

Leu Gly Asp His Glu Pro Asp Phe Glu Thr Arg Leu Asn Glu Leu Gly
485 490 495

Tyr Asp Cys His Asp Glu Thr Asn Asn Pro Ala Phe Arg Phe Phe Leu
500 505 510

Ala Gly

<210> 232
<211> 933
<212> DNA
<213> Escherichia coli

<400> 232
atggttaaag tttatgcccc ggcttccagt gccaatatga gcgtcgggtt tgatgtgctc 60

ggggcggcgg tgacacctgt tgatggtgca ttgctcggag atgtagtcac ggttgaggcg 120

gcagagacat tcagtctcaa caacctcgga cgctttgccg ataagctgcc gtcagaacca 180

cgggaaaata tcgtttatca gtgctgggag cgtttttgcc aggaactggg taagcaaatt 240

ccagtggcga tgaccctgga aaagaatatg ccgatcggtt cgggcttagg ctccagtgcc 300

tgttcggtgg tcgcggcgct gatggcgatg aatgaacact gcggcaagcc gcttaatgac 360

actcgtttgc tggctttgat gggcgagctg gaaggccgta tctccggcag cattcattac 420

gacaacgtgg caccgtgttt tctcggtggt atgcagttga tgatcgaaga aaacgacatc 480

atcagccagc aagtgccagg gtttgatgag tggctgtggg tgctggcgta tccggggatt 540

aaagtctcga cggcagaagc cagggctatt ttaccggcgc agtatcgccg ccaggattgc 600

attgcgcacg ggcgacatct ggcaggcttc attcacgcct gctattcccg tcagcctgag 660

cttgccgcga agctgatgaa agatgttatc gctgaaccct accgtgaacg gttactgcca 720

ggcttccggc aggcgcggca ggcggtcgcg gaaatcggcg cggtagcgag cggtatctcc 780

ggctccggcc cgaccttgtt cgctctgtgt gacaagccgg aaaccgccca gcgcgttgcc 840

gactggttgg gtaagaacta cctgcaaaat caggaaggtt ttgttcatat ttgccggctg 900

gatacggcgg gcgcacgagt actggaaaac taa 933

<210> 233
<211> 310
<212> PRT
<213> Escherichia coli

<400> 233

Met Val Lys Val Tyr Ala Pro Ala Ser Ser Ala Asn Met Ser Val Gly
1 5 10 15

Phe Asp Val Leu Gly Ala Ala Val Thr Pro Val Asp Gly Ala Leu Leu
20 25 30

Gly Asp Val Val Thr Val Glu Ala Ala Glu Thr Phe Ser Leu Asn Asn
35 40 45

Leu Gly Arg Phe Ala Asp Lys Leu Pro Ser Glu Pro Arg Glu Asn Ile
50 55 60

Val Tyr Gln Cys Trp Glu Arg Phe Cys Gln Glu Leu Gly Lys Gln Ile
65 70 75 80

Pro Val Ala Met Thr Leu Glu Lys Asn Met Pro Ile Gly Ser Gly Leu
85 90 95

Gly Ser Ser Ala Cys Ser Val Val Ala Ala Leu Met Ala Met Asn Glu
100 105 110

His Cys Gly Lys Pro Leu Asn Asp Thr Arg Leu Leu Ala Leu Met Gly
115 120 125

Glu Leu Glu Gly Arg Ile Ser Gly Ser Ile His Tyr Asp Asn Val Ala
130 135 140

Pro Cys Phe Leu Gly Gly Met Gln Leu Met Ile Glu Glu Asn Asp Ile
145 150 155 160

Ile Ser Gln Gln Val Pro Gly Phe Asp Glu Trp Leu Trp Val Leu Ala
165 170 175

Tyr Pro Gly Ile Lys Val Ser Thr Ala Glu Ala Arg Ala Ile Leu Pro
180 185 190

Ala Gln Tyr Arg Arg Gln Asp Cys Ile Ala His Gly Arg His Leu Ala
195 200 205

Gly Phe Ile His Ala Cys Tyr Ser Arg Gln Pro Glu Leu Ala Ala Lys
210 215 220

Leu Met Lys Asp Val Ile Ala Glu Pro Tyr Arg Glu Arg Leu Leu Pro
225 230 235 240

Gly Phe Arg Gln Ala Arg Gln Ala Val Ala Glu Ile Gly Ala Val Ala
245 250 255

Ser Gly Ile Ser Gly Ser Gly Pro Thr Leu Phe Ala Leu Cys Asp Lys
260 265 270

Pro Glu Thr Ala Gln Arg Val Ala Asp Trp Leu Gly Lys Asn Tyr Leu
275 280 285

Gln Asn Gln Glu Gly Phe Val His Ile Cys Arg Leu Asp Thr Ala Gly
290 295 300

Ala Arg Val Leu Glu Asn
305 310

<210> 234
<211> 1287
<212> DNA
<213> Escherichia coli

<400> 234
atgaaactct acaatctgaa agatcacaac gagcaggtca gctttgcgca agccgtaacc 60

caggggttgg gcaaaaatca ggggctgttt tttccgcacg acctgccgga attcagcctg 120

actgaaattg atgagatgct gaagctggat tttgtcaccc gcagtgcgaa gatcctctcg 180

gcgtttattg gtgatgaaat cccacaggaa atcctggaag agcgcgtgcg cgcggcgttt 240

gccttcccgg ctccggtcgc caatgttgaa agcgatgtcg gttgtctgga attgttccac 300

gggccaacgc tggcatttaa agatttcggc ggtcgcttta tggcacaaat gctgacccat 360

attgcgggtg ataagccagt gaccattctg accgcgacct ccggtgatac cggagcggca 420

gtggctcatg ctttctacgg tttaccgaat gtgaaagtgg ttatcctcta tccacgaggc 480

aaaatcagtc cactgcaaga aaaactgttc tgtacattgg gcggcaatat cgaaactgtt 540

gccatcgacg gcgatttcga tgcctgtcag gcgctggtga agcaggcgtt tgatgatgaa 600

gaactgaaag tggcgctagg gttaaactcg gctaactcga ttaacatcag ccgtttgctg 660

gcgcagattt gctactactt tgaagctgtt gcgcagctgc cgcaggagac gcgcaaccag 720

ctggttgtct cggtgccaag cggaaacttc ggcgatttga cggcgggtct gctggcgaag 780

tcactcggtc tgccggtgaa acgttttatt gctgcgacca acgtgaacga taccgtgcca 840

cgtttcctgc acgacggtca gtggtcaccc aaagcgactc aggcgacgtt atccaacgcg 900

atggacgtga gtcagccgaa caactggccg cgtgtggaag agttgttccg ccgcaaaatc 960

tggcaactga aagagctggg ttatgcagcc gtggatgatg aaaccacgca acagacaatg 1020

cgtgagttaa aagaactggg ctacacttcg gagccgcacg ctgccgtagc ttatcgtgcg 1080

ctgcgtgatc agttgaatcc aggcgaatat ggcttgttcc tcggcaccgc gcatccggcg 1140

aaatttaaag agagcgtgga agcgattctc ggtgaaacgt tggatctgcc aaaagagctg 1200

gcagaacgtg ctgatttacc cttgctttca cataatctgc ccgccgattt tgctgcgttg 1260

cgtaaattga tgatgaatca tcagtaa 1287

<210> 235
<211> 428
<212> PRT
<213> Escherichia coli

<400> 235

Met Lys Leu Tyr Asn Leu Lys Asp His Asn Glu Gln Val Ser Phe Ala
1 5 10 15

Gln Ala Val Thr Gln Gly Leu Gly Lys Asn Gln Gly Leu Phe Phe Pro
20 25 30

His Asp Leu Pro Glu Phe Ser Leu Thr Glu Ile Asp Glu Met Leu Lys
35 40 45

Leu Asp Phe Val Thr Arg Ser Ala Lys Ile Leu Ser Ala Phe Ile Gly
50 55 60

Asp Glu Ile Pro Gln Glu Ile Leu Glu Glu Arg Val Arg Ala Ala Phe
65 70 75 80

Ala Phe Pro Ala Pro Val Ala Asn Val Glu Ser Asp Val Gly Cys Leu
85 90 95

Glu Leu Phe His Gly Pro Thr Leu Ala Phe Lys Asp Phe Gly Gly Arg
100 105 110

Phe Met Ala Gln Met Leu Thr His Ile Ala Gly Asp Lys Pro Val Thr
115 120 125

Ile Leu Thr Ala Thr Ser Gly Asp Thr Gly Ala Ala Val Ala His Ala
130 135 140

Phe Tyr Gly Leu Pro Asn Val Lys Val Val Ile Leu Tyr Pro Arg Gly
145 150 155 160

Lys Ile Ser Pro Leu Gln Glu Lys Leu Phe Cys Thr Leu Gly Gly Asn
165 170 175

Ile Glu Thr Val Ala Ile Asp Gly Asp Phe Asp Ala Cys Gln Ala Leu
180 185 190

Val Lys Gln Ala Phe Asp Asp Glu Glu Leu Lys Val Ala Leu Gly Leu
195 200 205

Asn Ser Ala Asn Ser Ile Asn Ile Ser Arg Leu Leu Ala Gln Ile Cys
210 215 220

Tyr Tyr Phe Glu Ala Val Ala Gln Leu Pro Gln Glu Thr Arg Asn Gln
225 230 235 240

Leu Val Val Ser Val Pro Ser Gly Asn Phe Gly Asp Leu Thr Ala Gly
245 250 255

Leu Leu Ala Lys Ser Leu Gly Leu Pro Val Lys Arg Phe Ile Ala Ala
260 265 270

Thr Asn Val Asn Asp Thr Val Pro Arg Phe Leu His Asp Gly Gln Trp
275 280 285

Ser Pro Lys Ala Thr Gln Ala Thr Leu Ser Asn Ala Met Asp Val Ser
290 295 300

Gln Pro Asn Asn Trp Pro Arg Val Glu Glu Leu Phe Arg Arg Lys Ile
305 310 315 320

Trp Gln Leu Lys Glu Leu Gly Tyr Ala Ala Val Asp Asp Glu Thr Thr

325 330 335

Gln Gln Thr Met Arg Glu Leu Lys Glu Leu Gly Tyr Thr Ser Glu Pro
340 345 350

His Ala Ala Val Ala Tyr Arg Ala Leu Arg Asp Gln Leu Asn Pro Gly
355 360 365

Glu Tyr Gly Leu Phe Leu Gly Thr Ala His Pro Ala Lys Phe Lys Glu
370 375 380

Ser Val Glu Ala Ile Leu Gly Glu Thr Leu Asp Leu Pro Lys Glu Leu
385 390 395 400

Ala Glu Arg Ala Asp Leu Pro Leu Leu Ser His Asn Leu Pro Ala Asp
405 410 415

Phe Ala Ala Leu Arg Lys Leu Met Met Asn His Gln
420 425

<210> 236
<211> 1365
<212> DNA
<213> Escherichia coli

<400> 236
atgattagtg cattcgatat tttcaaaatt gggattggtc cctccagttc gcataccgtg 60

gggccaatga atgccggaaa aagttttatt gatcggctgg aaagtagcgg cttattaacc 120

gcgacgagcc atattgtggt cgatctgtac gggtcgttgt cactgacggg caaaggccat 180

gccacggatg tcgccatcat catgggactg gcaggaaaca gtccgcagga tgttgtcatt 240

gatgagatcc ctgcatttat agagttagta acgcgcagcg ggcggctgcc agtggcatct 300

ggtgcgcata ttgttgattt tcctgtagca aagaacatta tcttccatcc cgaaatgttg 360

cctcgccatg agaacggaat gcggatcact gcctggaagg gacaggaaga gctattaagt 420

aaaacctatt actctgtcgg cggcgggttt attgtcgaag aagaacactt cggcctgtcg 480

cacgatgtcg aaacgtccgt accttacgat ttccactcag caggtgaact gctgaaaatg 540

tgtgattaca acggcctgtc tatatctggt ctgatgatgc acaacgagct agcgctgcgc 600

agcaaagcgg aaattgacgc cggttttgcc cgtatctggc aagtgatgca tgacggtatt 660

gaacgtggga tgaacactga aggcgtgctg cctggtccgc tcaatgtgcc gcgccgtgcc 720

gtagcgctgc gtcgtcagct ggtttccagc gataacatct ctaacgatcc gatgaatgtc 780

atcgactgga tcaacatgta cgcgctggcg gttagtgaag aaaacgcagc tggcgggcgc 840

gtggtaacgg caccgactaa cggtgcgtgc ggcattattc cggcagtact ggcttattac 900

gataagttcc gtcgtccggt aaacgagcgg tcaattgccc gctattttct ggccgcgggg 960

gctattggcg cgctgtataa aatgaacgcc tccatctctg gcgcggaagt cggctgtcag 1020

ggggagattg gcgtggcctg ttcaatggcg gcggcagggt taactgaact actgggcggc 1080

agtccggcgc aggtatgcaa tgcggcggaa atcgcgatgg agcataacct tgggctgacc 1140

tgcgatccgg ttgccggaca ggtacaaatc ccgtgcattg aacgtaatgc cattaatgcc 1200

gtgaaagcag taaacgccgc gcggatggcg atgcgccgca cctcggcacc gcgtgtttca 1260

ctcgataaag tgatcgagac gatgtatgaa accggcaaag atatgaacga taaataccgc 1320

gaaacatcac gcggaggact ggccattaaa gtggtctgcg gctga 1365

<210> 237
<211> 454
<212> PRT
<213> Escherichia coli

<400> 237

Met Ile Ser Ala Phe Asp Ile Phe Lys Ile Gly Ile Gly Pro Ser Ser
1 5 10 15

Ser His Thr Val Gly Pro Met Asn Ala Gly Lys Ser Phe Ile Asp Arg
20 25 30

Leu Glu Ser Ser Gly Leu Leu Thr Ala Thr Ser His Ile Val Val Asp
35 40 45

Leu Tyr Gly Ser Leu Ser Leu Thr Gly Lys Gly His Ala Thr Asp Val
50 55 60

Ala Ile Ile Met Gly Leu Ala Gly Asn Ser Pro Gln Asp Val Val Ile
65 70 75 80

Asp Glu Ile Pro Ala Phe Ile Glu Leu Val Thr Arg Ser Gly Arg Leu
85 90 95

Pro Val Ala Ser Gly Ala His Ile Val Asp Phe Pro Val Ala Lys Asn
100 105 110

Ile Ile Phe His Pro Glu Met Leu Pro Arg His Glu Asn Gly Met Arg
115 120 125

Ile Thr Ala Trp Lys Gly Gln Glu Glu Leu Leu Ser Lys Thr Tyr Tyr
130 135 140

Ser Val Gly Gly Gly Phe Ile Val Glu Glu Glu His Phe Gly Leu Ser
145 150 155 160

```
His Asp Val Glu Thr Ser Val Pro Tyr Asp Phe His Ser Ala Gly Glu
                165                 170                 175

Leu Leu Lys Met Cys Asp Tyr Asn Gly Leu Ser Ile Ser Gly Leu Met
            180                 185                 190

Met His Asn Glu Leu Ala Leu Arg Ser Lys Ala Glu Ile Asp Ala Gly
        195                 200                 205

Phe Ala Arg Ile Trp Gln Val Met His Asp Gly Ile Glu Arg Gly Met
    210                 215                 220

Asn Thr Glu Gly Val Leu Pro Gly Pro Leu Asn Val Pro Arg Arg Ala
225                 230                 235                 240

Val Ala Leu Arg Arg Gln Leu Val Ser Ser Asp Asn Ile Ser Asn Asp
                245                 250                 255

Pro Met Asn Val Ile Asp Trp Ile Asn Met Tyr Ala Leu Ala Val Ser
            260                 265                 270

Glu Glu Asn Ala Ala Gly Gly Arg Val Val Thr Ala Pro Thr Asn Gly
        275                 280                 285

Ala Cys Gly Ile Ile Pro Ala Val Leu Ala Tyr Tyr Asp Lys Phe Arg
    290                 295                 300

Arg Pro Val Asn Glu Arg Ser Ile Ala Arg Tyr Phe Leu Ala Ala Gly
305                 310                 315                 320

Ala Ile Gly Ala Leu Tyr Lys Met Asn Ala Ser Ile Ser Gly Ala Glu
                325                 330                 335

Val Gly Cys Gln Gly Glu Ile Gly Val Ala Cys Ser Met Ala Ala Ala
            340                 345                 350

Gly Leu Thr Glu Leu Leu Gly Gly Ser Pro Ala Gln Val Cys Asn Ala
        355                 360                 365

Ala Glu Ile Ala Met Glu His Asn Leu Gly Leu Thr Cys Asp Pro Val
    370                 375                 380

Ala Gly Gln Val Gln Ile Pro Cys Ile Glu Arg Asn Ala Ile Asn Ala
385                 390                 395                 400

Val Lys Ala Val Asn Ala Ala Arg Met Ala Met Arg Arg Thr Ser Ala
                405                 410                 415
```

Pro Arg Val Ser Leu Asp Lys Val Ile Glu Thr Met Tyr Glu Thr Gly
420 425 430

Lys Asp Met Asn Asp Lys Tyr Arg Glu Thr Ser Arg Gly Gly Leu Ala
435 440 445

Ile Lys Val Val Cys Gly
450

<210> 238
<211> 1287
<212> DNA
<213> Escherichia coli

<400> 238
atgaagatag cgacgataaa aactgggctt gcttcactgg cgatgcttcc gggactggta 60

atggctgcac ctgcggtggc cgataaagcc gacaatgcgt ttatgatgat ttgtactgcg 120

ctggtgctgt ttatgactat tccggggatt gccctgtttt acggtgggtt gattcgcggc 180

aaaaacgtgc tgtcgatgct gacgcaggtg acggtgacat ttgcactggt ctgtattctc 240

tgggtggttt acggttactc gctggcgttt ggtgagggca acaacttctt cggcaacatt 300

aactggttga tgctgaaaaa catcgaactg acggcggtga tgggcagcat ttatcagtat 360

atccacgtgg cgtttcaggg atcgtttgcc tgcattaccg tcggcttgat agttggggcg 420

ctggcggaac gaatccgctt ctcagctgtg ttgattttcg tggtggtatg gctgacgctc 480

tcttacattc cgattgcgca tatggtgtgg ggcggtggtt tgctggcttc tcacggtgcg 540

ctggatttcg cgggtggcac cgtggtgcac attaacgccg caatcgccgg tctggtgggc 600

gcgtatctga taggaaaacg cgtgggcttc ggtaaagagg cgtttaaacc gcacaacctg 660

ccgatggtct tcaccgggac tgccattctc tatatcggtt ggtttggctt taacgccggg 720

tcagcgggca cggcgaatga aatcgcggca ctggcatttg tgaatactgt ggtcgcaacg 780

gcggcggcaa ttcttggctg gatcttcggt gaatgggcgc tgcgtggtaa gccttcactg 840

ctgggggcgt gttctggcgc gattgccggt ctggtcggcg tgacgccagc ctgcggctac 900

attggggttg gcggcgcgtt gattatcggc gtggtagctg gtctggcggg cttgtggggc 960

gttaccatgc tcaaacgctt gctgcgggtg gatgatccct gcgatgtctt cggtgtgcac 1020

ggcgtttgtg gcattgtcgg ctgtatcatg accgggattt ttgccgccag ctcgctgggc 1080

ggcgtgggct tcgctgaagg tgtgacgatg ggccatcagt tgctggtaca gctggaaagc 1140

atcgccatta cgatcgtctg gtccggtgtt gtggcattta tcggctacaa attggcggat 1200

ctgacggttg gtctgcgtgt accggaagag caggagcgag aagggctgga tgtcaacagc 1260

cacggcgaga atgcctataa cgcgtaa 1287

<210>  239
<211>  428
<212>  PRT
<213>  Escherichia coli

<400>  239

Met Lys Ile Ala Thr Ile Lys Thr Gly Leu Ala Ser Leu Ala Met Leu
1               5                   10                  15

Pro Gly Leu Val Met Ala Ala Pro Ala Val Ala Asp Lys Ala Asp Asn
            20                  25                  30

Ala Phe Met Met Ile Cys Thr Ala Leu Val Leu Phe Met Thr Ile Pro
        35                  40                  45

Gly Ile Ala Leu Phe Tyr Gly Gly Leu Ile Arg Gly Lys Asn Val Leu
    50                  55                  60

Ser Met Leu Thr Gln Val Thr Val Thr Phe Ala Leu Val Cys Ile Leu
65                  70                  75                  80

Trp Val Val Tyr Gly Tyr Ser Leu Ala Phe Gly Glu Gly Asn Asn Phe
                85                  90                  95

Phe Gly Asn Ile Asn Trp Leu Met Leu Lys Asn Ile Glu Leu Thr Ala
            100                 105                 110

Val Met Gly Ser Ile Tyr Gln Tyr Ile His Val Ala Phe Gln Gly Ser
        115                 120                 125

Phe Ala Cys Ile Thr Val Gly Leu Ile Val Gly Ala Leu Ala Glu Arg
    130                 135                 140

Ile Arg Phe Ser Ala Val Leu Ile Phe Val Val Val Trp Leu Thr Leu
145                 150                 155                 160

Ser Tyr Ile Pro Ile Ala His Met Val Trp Gly Gly Gly Leu Leu Ala
                165                 170                 175

Ser His Gly Ala Leu Asp Phe Ala Gly Gly Thr Val Val His Ile Asn
            180                 185                 190

Ala Ala Ile Ala Gly Leu Val Gly Ala Tyr Leu Ile Gly Lys Arg Val
        195                 200                 205

Gly Phe Gly Lys Glu Ala Phe Lys Pro His Asn Leu Pro Met Val Phe
    210                 215                 220

Thr Gly Thr Ala Ile Leu Tyr Ile Gly Trp Phe Gly Phe Asn Ala Gly
225 230 235 240

Ser Ala Gly Thr Ala Asn Glu Ile Ala Ala Leu Ala Phe Val Asn Thr
245 250 255

Val Val Ala Thr Ala Ala Ala Ile Leu Gly Trp Ile Phe Gly Glu Trp
260 265 270

Ala Leu Arg Gly Lys Pro Ser Leu Leu Gly Ala Cys Ser Gly Ala Ile
275 280 285

Ala Gly Leu Val Gly Val Thr Pro Ala Cys Gly Tyr Ile Gly Val Gly
290 295 300

Gly Ala Leu Ile Ile Gly Val Val Ala Gly Leu Ala Gly Leu Trp Gly
305 310 315 320

Val Thr Met Leu Lys Arg Leu Leu Arg Val Asp Asp Pro Cys Asp Val
325 330 335

Phe Gly Val His Gly Val Cys Gly Ile Val Gly Cys Ile Met Thr Gly
340 345 350

Ile Phe Ala Ala Ser Ser Leu Gly Gly Val Gly Phe Ala Glu Gly Val
355 360 365

Thr Met Gly His Gln Leu Leu Val Gln Leu Glu Ser Ile Ala Ile Thr
370 375 380

Ile Val Trp Ser Gly Val Val Ala Phe Ile Gly Tyr Lys Leu Ala Asp
385 390 395 400

Leu Thr Val Gly Leu Arg Val Pro Glu Glu Gln Glu Arg Glu Gly Leu
405 410 415

Asp Val Asn Ser His Gly Glu Asn Ala Tyr Asn Ala
420 425

<210> 240
<211> 50
<212> DNA
<213> Escherichia coli

<400> 240
caataaatac aaaaaatggg acggcacgca ccgtcccatt tacgagacag 50

<210> 241
<211> 50
<212> DNA
<213> Escherichia coli

<400> 241
gttacaggta agcgatgccg aactggcggc gcgtcgtgaa gcgcaggacg 50

<210> 242
<211> 50
<212> DNA
<213> Escherichia coli

<400> 242
caaacgcgaa ccgaacaata agaagcacaa catcacgagg aatcaccatg 50

<210> 243
<211> 50
<212> DNA
<213> Escherichia coli

<400> 243
cgtattgctg ttgcgggtta agtgcgcgct gatgccctca ccccgactct 50

<210> 244
<211> 50
<212> DNA
<213> Escherichia coli

<400> 244
gcgcaaaccg ggcggggttt ttcgtttaag cacctcccgg aaagtcggcc 50

<210> 245
<211> 50
<212> DNA
<213> Escherichia coli

<400> 245
agcgtctgga gcagatgata agccaaatcg ataagctgga agatgtcgtg 50

<210> 246
<211> 50
<212> DNA
<213> Escherichia coli

<400> 246
caattgctta agcaagatcg gacggttaat gtgttttaca catttttttcc 50

<210> 247
<211> 50
<212> DNA
<213> Escherichia coli

<400> 247
gcgaaaattg tggaggttgc tcgctctggt gtggtcggac tttcgcgcgg 50

<210> 248
<211> 50
<212> DNA
<213> Escherichia coli

<400> 248
ctcccaaatc gggggccctt ttttattgat aacaaaaagg caacactatg 50

<210> 249
<211> 50
<212> DNA
<213> Escherichia coli

<400> 249
gtgcctgttg atccaacctg atgaaaaggt gccggatgat gtgaatcatc 50

<210> 250
<211> 50
<212> DNA
<213> Escherichia coli

<400> 250
cggcggtgac acctgttgat ggtgcattgc tcggagatgt agtcacggtt 50

<210> 251
<211> 50
<212> DNA
<213> Escherichia coli

<400> 251
gcagaacgtg ctgatttacc cttgctttca cataatctgc ccgccgattt 50

<210> 252
<211> 34
<212> DNA
<213> T7 phage

<400> 252
tcacactggc tcaccttcgg gtgggccttt ctgc 34

<210> 253
<211> 66
<212> DNA
<213> artificiel

<400> 253
gagctgttgg cgattaatca tccggctcgt atattgtgtg gggttgcatg tactggagga 60

cagacc 66

<210> 254
<211> 50
<212> DNA
<213> Escherichia coli

<400> 254
gtgtctttgc tgatctgcta cgtaccctct catggaagtt aggagtctga 50

<210> 255
<211> 50
<212> DNA
<213> Escherichia coli

<400> 255
atggttaaag tttatgcccc ggcttccagt gccaatatga gcgtcgggtt 50

<210> 256
<211> 1645
<212> DNA
<213> Escherichia coli

<400> 256
atgaatggcg cacagtgggt ggtacatgcg ttgcgggcac agggtgtgaa caccgttttc 60

ggttatccgg gtggcgcaat tatgccggtt tacgatgcat tgtatgacgg cggcgtggag 120

cacttgctat gccgacatga gcagggtgcg gcaatggcgg ctatcggtta tgctcgtgct 180

accggcaaaa ctggcgtatg tatcgccacg tctggtccgg gcgcaaccaa cctgataacc 240

gggcttgcgg acgcactgtt agattccatc cctgttgttg ccatcaccgg tcaagtgtcc 300

gcaccgttta tcggcactga cgcatttcag gaagtggatg tcctgggatt gtcgttagcc 360

tgtaccaagc acagctttct ggtgcagtcg ctggaagagt tgccgcgcat catggctgaa 420

gcattcgacg ttgcctgctc aggtcgtcct ggtccggttc tggtcgatat cccaaaagat 480

atccagttag ccagcggtga cctggaaccg tggttcacca ccgttgaaaa cgaagtgact 540

ttcccacatg ccgaagttga gcaagcgcgc cagatgctgg caaaagcgca aaaaccgatg 600

ctgtacgttg gcggtggcgt gggtatggcg caggcagttc cggctttgcg tgaatttctc 660

gctgccacaa aaatgcctgc cacctgtacg ctgaaagggc tgggcgcagt agaagcagat 720

tatccgtact atctgggcat gctggggatg cacggcacca aagcggcaaa cttcgcggtg 780

caggagtgtg acctgctgat cgccgtgggc gcacgttttg atgaccgggt gaccggcaaa 840

ctgaacacct tcgcgccaca cgccagtgtt atccatatgg atatcgaccc ggcagaaatg 900

aacaagctgc gtcaggcaca tgtggcatta caaggtgatt taaatgctct gttaccagca 960

ttacagcagc cgttaaatca atgactggca gcaacactgc gcgcagctgc gtgatgaaca 1020

ttcctggcgt tacgaccatc ccggtgacgc tatctacgcg ccgttgttgt taaaacaact 1080

gtcggatcgt aaacctgcgg attgcgtcgt gaccacagat gtggggcagc accagatgtg 1140

ggctgcgcag cacatcgccc acactcgccc ggaaaatttc atcacctcca gcggtttagg 1200

taccatgggt tttggtttac cggcggcggt tggcgcacaa gtcgcgcgac cgaacgatac 1260

cgttgtctgt atctccggtg acggctcttt catgatgaat gtgcaagagc tgggcaccgt 1320

aaaacgcaag cagttaccgt tgaaaatcgt cttactcgat aaccaacggt tagggatggt 1380

tcgacaatgg cagcaactgt tttttcagga acgatacagc gaaaccaccc ttactgataa 1440

ccccgatttc ctcatgttag ccagcgcctt cggcatccat ggccaacaca tcacccggaa 1500

agaccaggtt gaagcggcac tcgacaccat gctgaacagt gatgggccat acctgcttca 1560

tgtctcaatc gacgaacttg agaacgtctg gccgctggtg ccgcctggcg ccagtaattc 1620

agaaatgttg gagaaattat catga 1645

<210> 257
<211> 984
<212> DNA
<213> Escherichia coli

<400> 257
atgaatggcg cacagtgggt ggtacatgcg ttgcgggcac agggtgtgaa caccgttttc 60

ggttatccgg gtggcgcaat tatgccggtt tacgatgcat tgtatgacgg cggcgtggag 120

cacttgctat gccgacatga gcagggtgcg gcaatggcgg ctatcggtta tgctcgtgct 180

accggcaaaa ctggcgtatg tatcgccacg tctggtccgg gcgcaaccaa cctgataacc 240

gggcttgcgg acgcactgtt agattccatc cctgttgttg ccatcaccgg tcaagtgtcc 300

gcaccgttta tcggcactga cgcatttcag gaagtggatg tcctgggatt gtcgttagcc 360

tgtaccaagc acagctttct ggtgcagtcg ctggaagagt tgccgcgcat catggctgaa 420

gcattcgacg ttgcctgctc aggtcgtcct ggtccggttc tggtcgatat cccaaaagat 480

atccagttag ccagcggtga cctggaaccg tggttcacca ccgttgaaaa cgaagtgact 540

ttcccacatg ccgaagttga gcaagcgcgc cagatgctgg caaaagcgca aaaaccgatg 600

ctgtacgttg gcggtggcgt gggtatggcg caggcagttc cggctttgcg tgaatttctc 660

gctgccacaa aaatgcctgc cacctgtacg ctgaaagggc tgggcgcagt agaagcagat 720

tatccgtact atctgggcat gctggggatg cacggcacca aagcggcaaa cttcgcggtg 780

caggagtgtg acctgctgat cgccgtgggc gcacgttttg atgaccgggt gaccggcaaa 840

ctgaacacct tcgcgccaca cgccagtgtt atccatatgg atatcgaccc ggcagaaatg 900

aacaagctgc gtcaggcaca tgtggcatta caaggtgatt taaatgctct gttaccagca 960

ttacagcagc cgttaaatca atga 984

<210> 258
<211> 582
<212> DNA
<213> Escherichia coli

<400> 258
ttgttgttaa aacaactgtc ggatcgtaaa cctgcggatt gcgtcgtgac cacagatgtg 60

gggcagcacc agatgtgggc tgcgcagcac atcgcccaca ctcgcccgga aaatttcatc 120

acctccagcg gtttaggtac catgggtttt ggtttaccgg cggcggttgg cgcacaagtc 180

gcgcgaccga acgataccgt tgtctgtatc tccggtgacg gctctttcat gatgaatgtg 240

caagagctgg gcaccgtaaa acgcaagcag ttaccgttga aaatcgtctt actcgataac 300

caacggttag ggatggttcg acaatggcag caactgtttt ttcaggaacg atacagcgaa 360

accaccctta ctgataaccc cgatttcctc atgttagcca gcgccttcgg catccatggc 420

caacacatca cccggaaaga ccaggttgaa gcggcactcg acaccatgct gaacagtgat 480

gggccatacc tgcttcatgt ctcaatcgac gaacttgaga acgtctggcc gctggtgccg 540

cctggcgcca gtaattcaga aatgttggag aaattatcat ga 582

<210> 259
<211> 327
<212> PRT
<213> Escherichia coli

<400> 259

Met Asn Gly Ala Gln Trp Val Val His Ala Leu Arg Ala Gln Gly Val
1 5 10 15

Asn Thr Val Phe Gly Tyr Pro Gly Gly Ala Ile Met Pro Val Tyr Asp
20 25 30

Ala Leu Tyr Asp Gly Gly Val Glu His Leu Leu Cys Arg His Glu Gln
35 40 45

Gly Ala Ala Met Ala Ala Ile Gly Tyr Ala Arg Ala Thr Gly Lys Thr
50 55 60

Gly Val Cys Ile Ala Thr Ser Gly Pro Gly Ala Thr Asn Leu Ile Thr
65 70 75 80

Gly Leu Ala Asp Ala Leu Leu Asp Ser Ile Pro Val Val Ala Ile Thr
85 90 95

Gly Gln Val Ser Ala Pro Phe Ile Gly Thr Asp Ala Phe Gln Glu Val
100 105 110

Asp Val Leu Gly Leu Ser Leu Ala Cys Thr Lys His Ser Phe Leu Val
115 120 125

Gln Ser Leu Glu Glu Leu Pro Arg Ile Met Ala Glu Ala Phe Asp Val
130 135 140

Ala Cys Ser Gly Arg Pro Gly Pro Val Leu Val Asp Ile Pro Lys Asp
145 150 155 160

Ile Gln Leu Ala Ser Gly Asp Leu Glu Pro Trp Phe Thr Thr Val Glu
165 170 175

Asn Glu Val Thr Phe Pro His Ala Glu Val Glu Gln Ala Arg Gln Met
180 185 190

Leu Ala Lys Ala Gln Lys Pro Met Leu Tyr Val Gly Gly Gly Val Gly
195 200 205

Met Ala Gln Ala Val Pro Ala Leu Arg Glu Phe Leu Ala Ala Thr Lys
210 215 220

Met Pro Ala Thr Cys Thr Leu Lys Gly Leu Gly Ala Val Glu Ala Asp
225 230 235 240

Tyr Pro Tyr Tyr Leu Gly Met Leu Gly Met His Gly Thr Lys Ala Ala
245 250 255

Asn Phe Ala Val Gln Glu Cys Asp Leu Leu Ile Ala Val Gly Ala Arg
260 265 270

Phe Asp Asp Arg Val Thr Gly Lys Leu Asn Thr Phe Ala Pro His Ala
275 280 285

Ser Val Ile His Met Asp Ile Asp Pro Ala Glu Met Asn Lys Leu Arg
290 295 300

Gln Ala His Val Ala Leu Gln Gly Asp Leu Asn Ala Leu Leu Pro Ala
305 310 315 320

Leu Gln Gln Pro Leu Asn Gln
325

<210> 260
<211> 193
<212> PRT
<213> Escherichia coli

<400> 260

Met Leu Leu Lys Gln Leu Ser Asp Arg Lys Pro Ala Asp Cys Val Val
1 5 10 15

Thr Thr Asp Val Gly Gln His Gln Met Trp Ala Ala Gln His Ile Ala
20 25 30

His Thr Arg Pro Glu Asn Phe Ile Thr Ser Ser Gly Leu Gly Thr Met
35 40 45

```
Gly Phe Gly Leu Pro Ala Ala Val Gly Ala Gln Val Ala Arg Pro Asn
    50                  55                  60

Asp Thr Val Val Cys Ile Ser Gly Asp Gly Ser Phe Met Met Asn Val
65                  70                  75                  80

Gln Glu Leu Gly Thr Val Lys Arg Lys Gln Leu Pro Leu Lys Ile Val
                85                  90                  95

Leu Leu Asp Asn Gln Arg Leu Gly Met Val Arg Gln Trp Gln Gln Leu
            100                 105                 110

Phe Phe Gln Glu Arg Tyr Ser Glu Thr Thr Leu Thr Asp Asn Pro Asp
        115                 120                 125

Phe Leu Met Leu Ala Ser Ala Phe Gly Ile His Gly Gln His Ile Thr
    130                 135                 140

Arg Lys Asp Gln Val Glu Ala Ala Leu Asp Thr Met Leu Asn Ser Asp
145                 150                 155                 160

Gly Pro Tyr Leu Leu His Val Ser Ile Asp Glu Leu Glu Asn Val Trp
                165                 170                 175

Pro Leu Val Pro Pro Gly Ala Ser Asn Ser Glu Met Leu Glu Lys Leu
            180                 185                 190

Ser


<210>  261
<211>  264
<212>  DNA
<213>  Escherichia coli

<400>  261
atgatgcaac atcaggtcaa tgtatcggct cgcttcaatc cagaaacctt agaacgtgtt        60

ttacgcgtgg tgcgtcatcg tggtttccac gtctgctcaa tgaatatggc cgccgccagc       120

gatgcacaaa atataaatat cgaattgacc gttgccagcc cacggtcggt cgacttactg       180

tttagtcagt taaataaact ggtggacgtc gcacacgttg ccatctgcca gagcacaacc       240

acatcacaac aaatccgcgc ctga                                              264


<210>  262
<211>  87
<212>  PRT
<213>  Escherichia coli

<400>  262
```

Met Met Gln His Gln Val Asn Val Ser Ala Arg Phe Asn Pro Glu Thr
1 5 10 15

Leu Glu Arg Val Leu Arg Val Val Arg His Arg Gly Phe His Val Cys
20 25 30

Ser Met Asn Met Ala Ala Ala Ser Asp Ala Gln Asn Ile Asn Ile Glu
35 40 45

Leu Thr Val Ala Ser Pro Arg Ser Val Asp Leu Leu Phe Ser Gln Leu
50 55 60

Asn Lys Leu Val Asp Val Ala His Val Ala Ile Cys Gln Ser Thr Thr
65 70 75 80

Thr Ser Gln Gln Ile Arg Ala
85

<210> 263
<211> 1305
<212> DNA
<213> Escherichia coli

<400> 263
ttgactacgc cattgaaaaa gattgtgatt gtcggcggcg gtgctggtgg gctggaaatg 60

gcaacacagc tggggcataa gctgggacgc aagaaaaaag ccaaaattac gctggtcgat 120

cgtaaccaca gccacctgtg gaaaccgctg ctgcacgaag tggcgactgg ctcgcttgat 180

gaaggcgtcg atgcgttgag ctatctggcc catgcgcgca atcatggttt ccagttccag 240

ctgggttccg tcattgatat tgatcgtgaa gcgaaaacaa tcactattgc agaactgcgc 300

gacgagaaag gtgaactgct ggttccggaa cgtaaaatcg cctatgacac cctggtaatg 360

gcgctgggta gcacctctaa cgatttcaat acgccaggtg tcaaagagaa ctgcattttc 420

ctcgataacc cgcaccaggc gcgtcgcttc caccaggaga tgctgaattt gttcctgaaa 480

tactccgcca acctgggcgc gaatggcaaa gtgaacattg cgattgtcgg cggcggcgcg 540

acgggtgtag aactctccgc tgaattgcac aacgcggtca agcaactgca cagctacggt 600

tacaaaggcc tgaccaacga agccctgaac gtaacgctgg tagaagcggg agaacgtatt 660

ttgcctgcgt taccgccacg tatctctgct gcggcccaca acgagctaac gaaacttggc 720

gttcgcgtgc tgacgcaaac catggtcacc agtgctgatg aaggcggcct gcacactaaa 780

gatggcgaat atattgaggc tgatctgatg gtatgggcag ccgggatcaa agcgccagac 840

ttcctgaaag atatcggtgg tcttgaaact aaccgtatca accagctggt ggtggaaccg 900

acgctgcaaa ccacccgcga tccagacatt tacgctattg gcgactgcgc gtcatgcccg 960

cgtccggaag ggggctttgt tccgccgcgt gctcaggctg cacaccagat ggcgacttgc 1020

gcaatgaaca acattctggc gcagatgaac ggtaagccgc tgaaaaatta tcagtataaa 1080

gatcatggtt cgctggtatc gctgtcgaac ttctccaccg tcggtagcct gatgggtaac 1140

ctgacgcgcg gctcaatgat gattgaagga cgaattgcgc gctttgtata tatctcgcta 1200

taccgaatgc atcagattgc gctgcatggt tactttaaaa ccggattaat gatgctggtg 1260

gggagtatta accgcgttat ccgtccgcgt ttgaagttgc attaa 1305

<210> 264
<211> 434
<212> PRT
<213> Escherichia coli

<400> 264

Met Thr Thr Pro Leu Lys Lys Ile Val Ile Val Gly Gly Gly Ala Gly
1               5                   10                  15

Gly Leu Glu Met Ala Thr Gln Leu Gly His Lys Leu Gly Arg Lys Lys
            20                  25                  30

Lys Ala Lys Ile Thr Leu Val Asp Arg Asn His Ser His Leu Trp Lys
        35                  40                  45

Pro Leu Leu His Glu Val Ala Thr Gly Ser Leu Asp Glu Gly Val Asp
    50                  55                  60

Ala Leu Ser Tyr Leu Ala His Ala Arg Asn His Gly Phe Gln Phe Gln
65                  70                  75                  80

Leu Gly Ser Val Ile Asp Ile Asp Arg Glu Ala Lys Thr Ile Thr Ile
                85                  90                  95

Ala Glu Leu Arg Asp Glu Lys Gly Glu Leu Leu Val Pro Glu Arg Lys
            100                 105                 110

Ile Ala Tyr Asp Thr Leu Val Met Ala Leu Gly Ser Thr Ser Asn Asp
        115                 120                 125

Phe Asn Thr Pro Gly Val Lys Glu Asn Cys Ile Phe Leu Asp Asn Pro
    130                 135                 140

His Gln Ala Arg Arg Phe His Gln Glu Met Leu Asn Leu Phe Leu Lys
145                 150                 155                 160

Tyr Ser Ala Asn Leu Gly Ala Asn Gly Lys Val Asn Ile Ala Ile Val
                165                 170                 175

```
Gly Gly Gly Ala Thr Gly Val Glu Leu Ser Ala Glu Leu His Asn Ala
            180                 185                 190

Val Lys Gln Leu His Ser Tyr Gly Tyr Lys Gly Leu Thr Asn Glu Ala
        195                 200                 205

Leu Asn Val Thr Leu Val Glu Ala Gly Glu Arg Ile Leu Pro Ala Leu
    210                 215                 220

Pro Pro Arg Ile Ser Ala Ala Ala His Asn Glu Leu Thr Lys Leu Gly
225                 230                 235                 240

Val Arg Val Leu Thr Gln Thr Met Val Thr Ser Ala Asp Glu Gly Gly
                245                 250                 255

Leu His Thr Lys Asp Gly Glu Tyr Ile Glu Ala Asp Leu Met Val Trp
            260                 265                 270

Ala Ala Gly Ile Lys Ala Pro Asp Phe Leu Lys Asp Ile Gly Gly Leu
        275                 280                 285

Glu Thr Asn Arg Ile Asn Gln Leu Val Val Glu Pro Thr Leu Gln Thr
    290                 295                 300

Thr Arg Asp Pro Asp Ile Tyr Ala Ile Gly Asp Cys Ala Ser Cys Pro
305                 310                 315                 320

Arg Pro Glu Gly Gly Phe Val Pro Pro Arg Ala Gln Ala Ala His Gln
                325                 330                 335

Met Ala Thr Cys Ala Met Asn Asn Ile Leu Ala Gln Met Asn Gly Lys
            340                 345                 350

Pro Leu Lys Asn Tyr Gln Tyr Lys Asp His Gly Ser Leu Val Ser Leu
        355                 360                 365

Ser Asn Phe Ser Thr Val Gly Ser Leu Met Gly Asn Leu Thr Arg Gly
    370                 375                 380

Ser Met Met Ile Glu Gly Arg Ile Ala Arg Phe Val Tyr Ile Ser Leu
385                 390                 395                 400

Tyr Arg Met His Gln Ile Ala Leu His Gly Tyr Phe Lys Thr Gly Leu
                405                 410                 415

Met Met Leu Val Gly Ser Ile Asn Arg Val Ile Arg Pro Arg Leu Lys
            420                 425                 430
```

Leu His

<210> 265
<211> 1569
<212> DNA
<213> Escherichia coli

<400> 265
atgttagata tagtcgaact gtcgcgctta cagtttgcct tgaccgcgat gtaccacttc 60

ctttttgtgc cactgacgct cggtatggcg ttcctgctgg ccattatgga aacggtctac 120

gtcctctccg gcaaacagat ttataaagat atgaccaagt tctggggcaa gttgtttggt 180

atcaacttcg ctctgggtgt ggctaccggt ctgaccatgg agttccagtt cgggactaac 240

tggtcttact attcccacta tgtaggggat atcttcggtg cgccgctggc aatcgaaggt 300

ctgatggcct tcttcctcga atccaccttt gtaggtctgt tcttcttcgg ttgggatcgt 360

ctgggtaaag ttcagcatat gtgtgtcacc tggctggtgg cgctcggttc aaacctgtcc 420

gcactgtgga ttctggttgc gaacggctgg atgcaaaacc caatcgcgtc cgatttcaac 480

tttgaaacta tgcgtatgga gatggtgagc ttctccgagc tggtgcttaa cccggttgct 540

caggtgaaat tcgttcacac tgtagcgtct ggttatgtga ctggcgcgat gttcatcctc 600

ggtatcagcg catggtatat gctgaaaggt cgtgacttcg ccttcgctaa acgctccttt 660

gctatcgctg ccagcttcgg tatggctgct gttctgtctg ttattgttct gggtgatgaa 720

tccggctacg aaatgggcga cgtgcagaaa accaaactgg ctgctattga agccgagtgg 780

gaaacgcaac ctgcgcctgc tgcctttact ctgttcggca ttcctgatca ggaagaggag 840

acgaacaaat ttgcgattca gatcccttac gcactgggca tcattgcaac gcgttccgtg 900

gataccccgg ttatcggcct gaaagagctg atggtgcagc atgaagaacg cattcgtaac 960

gggatgaagg cgtactctct gctcgaacaa ctgcgttctg gttctaccga ccaggcggtt 1020

cgtgaccagt tcaatagcat gaagaaagac ctcggttacg gtctgctgct gaaacgctat 1080

acgccaaacg tggctgatgc gactgaagcg cagattcaac aggcaaccaa agactccatc 1140

ccgcgtgtag cgccgctgta ctttgcgttc cgtatcatgg tggcgtgtgg cttcctgctt 1200

ctggcaatca tcgcgctctc tttctggagt gtcatccgca accgcattgg cgagaaaaaa 1260

tggcttctgc gcgccgcgct gtacggtatt ccgctgccgt ggattgctgt agaagcgggc 1320

tggttcgtgg ctgaatatgg ccgccaaccg tgggctatcg gtgaagtgct gccgacagct 1380

gtggcgaact cgtcactgac cgcaggcgat ctcatcttct caatggtgct gatttgcggc 1440

ctgtataccc tgttcctggt ggcagaattg ttcttaatgt tcaagtttgc acgcctcggc 1500

ccaagcagcc tgaaaaccgg tcgctatcac tttgagcagt cttccacgac tactcagccg 1560

gcacgctaa 1569

<210> 266
<211> 522
<212> PRT
<213> Escherichia coli

<400> 266

Met Leu Asp Ile Val Glu Leu Ser Arg Leu Gln Phe Ala Leu Thr Ala
1 5 10 15

Met Tyr His Phe Leu Phe Val Pro Leu Thr Leu Gly Met Ala Phe Leu
20 25 30

Leu Ala Ile Met Glu Thr Val Tyr Val Leu Ser Gly Lys Gln Ile Tyr
35 40 45

Lys Asp Met Thr Lys Phe Trp Gly Lys Leu Phe Gly Ile Asn Phe Ala
50 55 60

Leu Gly Val Ala Thr Gly Leu Thr Met Glu Phe Gln Phe Gly Thr Asn
65 70 75 80

Trp Ser Tyr Tyr Ser His Tyr Val Gly Asp Ile Phe Gly Ala Pro Leu
85 90 95

Ala Ile Glu Gly Leu Met Ala Phe Phe Leu Glu Ser Thr Phe Val Gly
100 105 110

Leu Phe Phe Phe Gly Trp Asp Arg Leu Gly Lys Val Gln His Met Cys
115 120 125

Val Thr Trp Leu Val Ala Leu Gly Ser Asn Leu Ser Ala Leu Trp Ile
130 135 140

Leu Val Ala Asn Gly Trp Met Gln Asn Pro Ile Ala Ser Asp Phe Asn
145 150 155 160

Phe Glu Thr Met Arg Met Glu Met Val Ser Phe Ser Glu Leu Val Leu
165 170 175

Asn Pro Val Ala Gln Val Lys Phe Val His Thr Val Ala Ser Gly Tyr
180 185 190

Val Thr Gly Ala Met Phe Ile Leu Gly Ile Ser Ala Trp Tyr Met Leu
195 200 205

Lys Gly Arg Asp Phe Ala Phe Ala Lys Arg Ser Phe Ala Ile Ala Ala
    210                 215                 220

Ser Phe Gly Met Ala Ala Val Leu Ser Val Ile Val Leu Gly Asp Glu
225                 230                 235                 240

Ser Gly Tyr Glu Met Gly Asp Val Gln Lys Thr Lys Leu Ala Ala Ile
                245                 250                 255

Glu Ala Glu Trp Glu Thr Gln Pro Ala Pro Ala Ala Phe Thr Leu Phe
            260                 265                 270

Gly Ile Pro Asp Gln Glu Glu Glu Thr Asn Lys Phe Ala Ile Gln Ile
        275                 280                 285

Pro Tyr Ala Leu Gly Ile Ile Ala Thr Arg Ser Val Asp Thr Pro Val
    290                 295                 300

Ile Gly Leu Lys Glu Leu Met Val Gln His Glu Glu Arg Ile Arg Asn
305                 310                 315                 320

Gly Met Lys Ala Tyr Ser Leu Leu Glu Gln Leu Arg Ser Gly Ser Thr
                325                 330                 335

Asp Gln Ala Val Arg Asp Gln Phe Asn Ser Met Lys Lys Asp Leu Gly
            340                 345                 350

Tyr Gly Leu Leu Leu Lys Arg Tyr Thr Pro Asn Val Ala Asp Ala Thr
        355                 360                 365

Glu Ala Gln Ile Gln Gln Ala Thr Lys Asp Ser Ile Pro Arg Val Ala
    370                 375                 380

Pro Leu Tyr Phe Ala Phe Arg Ile Met Val Ala Cys Gly Phe Leu Leu
385                 390                 395                 400

Leu Ala Ile Ile Ala Leu Ser Phe Trp Ser Val Ile Arg Asn Arg Ile
                405                 410                 415

Gly Glu Lys Lys Trp Leu Leu Arg Ala Ala Leu Tyr Gly Ile Pro Leu
            420                 425                 430

Pro Trp Ile Ala Val Glu Ala Gly Trp Phe Val Ala Glu Tyr Gly Arg
        435                 440                 445

Gln Pro Trp Ala Ile Gly Glu Val Leu Pro Thr Ala Val Ala Asn Ser
    450                 455                 460

Ser Leu Thr Ala Gly Asp Leu Ile Phe Ser Met Val Leu Ile Cys Gly
465 470 475 480

Leu Tyr Thr Leu Phe Leu Val Ala Glu Leu Phe Leu Met Phe Lys Phe
485 490 495

Ala Arg Leu Gly Pro Ser Ser Leu Lys Thr Gly Arg Tyr His Phe Glu
500 505 510

Gln Ser Ser Thr Thr Thr Gln Pro Ala Arg
515 520

<210> 267
<211> 1140
<212> DNA
<213> Escherichia coli

<400> 267
atgatcgatt atgaagtatt gcgttttatc tggtggctgc tggttggcgt tctgctgatt 60

ggttttgcag tcactgacgg tttcgacatg gggtgggca tgctcacccg tttcctcggt 120

cgtaacgaca ccgagcgtcg aattatgatt aactccattg caccacactg ggacggtaac 180

caggtttggc tgatcaccgc gggcggcgca ctctttgctg cctggccgat ggtctatgcc 240

gctgcgttct ccggcttcta tgtggcgatg atcctcgtgc tggcgtcttt gttcttccgt 300

ccggtcggtt ttgactaccg ctccaagatt gaagaaaccc gctggcgtaa catgtgggac 360

tggggcatct tcattggtag cttcgttccg ccgctggtaa ttggtgtagc gttcggtaac 420

ctgttgcagg gcgtaccgtt caacgttgat gaatatctgc gtctgtacta caccggtaac 480

ttcttccagt tgcttaaccc gttcggcctg ctggcaggcg tggtgagcgt agggatgatc 540

attactcagg gcgcaaccta tctgcaaatg cgtaccgtgg gcgaactgca cctgcgtacc 600

cgtgcaacgg ctcaggtggc tgcgctggtg acactggtct gtttcgcact ggctggcgta 660

tgggtgatgt acggtatcga tggttatgtc gtgaaatcga caatggacca ttacgcagcc 720

tctaacccac tgaataaaga agtggttcgt gaagctggcg catggctggt taacttcaac 780

aacacgccaa ttctgtgggc tattccggca ctgggtgtgg ttctgccgct gctgaccatc 840

ctgactgcac gtatggataa agccgcgtgg gcgtttgtgt tctcctccct gacgctggcc 900

tgcatcatcc tgacagccgg tatcgcaatg ttcccgtttg tgatgccgtc cagcaccatg 960

atgaacgcaa gtctgacaat gtgggatgca acttccagcc agctgacgct taacgtcatg 1020

acctgggttg cggtggttct ggtaccgatc attctgctct acaccgcctg gtgttactgg 1080

aaaatgttcg gtcgtatcac caaagaagat attgaacgta acacccactc tctgtactaa 1140

<210> 268

```
<211>  379
<212>  PRT
<213>  Escherichia coli

<400>  268

Met Ile Asp Tyr Glu Val Leu Arg Phe Ile Trp Trp Leu Leu Val Gly
1               5                   10                  15

Val Leu Leu Ile Gly Phe Ala Val Thr Asp Gly Phe Asp Met Gly Val
            20                  25                  30

Gly Met Leu Thr Arg Phe Leu Gly Arg Asn Asp Thr Glu Arg Arg Ile
        35                  40                  45

Met Ile Asn Ser Ile Ala Pro His Trp Asp Gly Asn Gln Val Trp Leu
    50                  55                  60

Ile Thr Ala Gly Gly Ala Leu Phe Ala Ala Trp Pro Met Val Tyr Ala
65                  70                  75                  80

Ala Ala Phe Ser Gly Phe Tyr Val Ala Met Ile Leu Val Leu Ala Ser
                85                  90                  95

Leu Phe Phe Arg Pro Val Gly Phe Asp Tyr Arg Ser Lys Ile Glu Glu
            100                 105                 110

Thr Arg Trp Arg Asn Met Trp Asp Trp Gly Ile Phe Ile Gly Ser Phe
        115                 120                 125

Val Pro Pro Leu Val Ile Gly Val Ala Phe Gly Asn Leu Leu Gln Gly
    130                 135                 140

Val Pro Phe Asn Val Asp Glu Tyr Leu Arg Leu Tyr Tyr Thr Gly Asn
145                 150                 155                 160

Phe Phe Gln Leu Leu Asn Pro Phe Gly Leu Leu Ala Gly Val Val Ser
                165                 170                 175

Val Gly Met Ile Ile Thr Gln Gly Ala Thr Tyr Leu Gln Met Arg Thr
            180                 185                 190

Val Gly Glu Leu His Leu Arg Thr Arg Ala Thr Ala Gln Val Ala Ala
        195                 200                 205

Leu Val Thr Leu Val Cys Phe Ala Leu Ala Gly Val Trp Val Met Tyr
    210                 215                 220

Gly Ile Asp Gly Tyr Val Val Lys Ser Thr Met Asp His Tyr Ala Ala
```

225 230 235 240

Ser Asn Pro Leu Asn Lys Glu Val Val Arg Glu Ala Gly Ala Trp Leu
245 250 255

Val Asn Phe Asn Asn Thr Pro Ile Leu Trp Ala Ile Pro Ala Leu Gly
260 265 270

Val Val Leu Pro Leu Leu Thr Ile Leu Thr Ala Arg Met Asp Lys Ala
275 280 285

Ala Trp Ala Phe Val Phe Ser Ser Leu Thr Leu Ala Cys Ile Ile Leu
290 295 300

Thr Ala Gly Ile Ala Met Phe Pro Phe Val Met Pro Ser Ser Thr Met
305 310 315 320

Met Asn Ala Ser Leu Thr Met Trp Asp Ala Thr Ser Ser Gln Leu Thr
325 330 335

Leu Asn Val Met Thr Trp Val Ala Val Val Leu Val Pro Ile Ile Leu
340 345 350

Leu Tyr Thr Ala Trp Cys Tyr Trp Lys Met Phe Gly Arg Ile Thr Lys
355 360 365

Glu Asp Ile Glu Arg Asn Thr His Ser Leu Tyr
370 375

<210> 269
<211> 114
<212> DNA
<213> Escherichia coli

<400> 269
atgtggtatt tcgcatggat tctgggaacg cttcttgcct gttcgtttgg ggtaatcacc 60

gcgctggcgc ttgaacacgt cgaatcaggc aaagccggtc aagaagacat ctga 114

<210> 270
<211> 37
<212> PRT
<213> Escherichia coli

<400> 270

Met Trp Tyr Phe Ala Trp Ile Leu Gly Thr Leu Leu Ala Cys Ser Phe
1 5 10 15

Gly Val Ile Thr Ala Leu Ala Leu Glu His Val Glu Ser Gly Lys Ala
20 25 30

Gly Gln Glu Asp Ile
35

<210> 271
<211> 948
<212> DNA
<213> Escherichia coli

<400> 271
atgagactca ggaaatacaa taaaagtttg ggatggttgt cattatttgc aggcactgta 60

ttgctcagtg gctgtaattc tgcgctgtta gatcccaaag gacagattgg tctggagcaa 120

cgttcactga tactgacggc atttggcctg atgttgattg tcgttattcc cgcaatcttg 180

atggctgttg gtttcgcctg gaagtaccgt gcgagcaata aagatgctaa gtacagcccg 240

aactggtcac actccaataa agtggaagct gtggtctgga cggtacctat cttaatcatc 300

atcttccttg cagtactgac ctggaaaacc actcacgctc ttgagcctag caagccgctg 360

gcacacgacg agaagcccat taccatcgaa gtggtttcca tggactggaa atggttcttc 420

atctacccgg aacagggcat tgctaccgtg aatgaaatcg ctttcccggc gaacactccg 480

gtgtacttca aagtgacctc caactccgtg atgaactcct tcttcattcc gcgtctgggt 540

agccagattt atgccatggc cggtatgcag actcgcctgc atctgatcgc caacgaaccc 600

ggcacttatg acggtatctc cgccagctac agcggcccgg gcttctcagg catgaagttc 660

aaagctattg caacaccgga tcgcgccgca ttcgaccagt gggtcgcaaa agcgaagcag 720

tcgccgaaca ccatgtctga catggctgcg ttcgaaaaac tggccgcgcc tagcgaatac 780

aaccaggtgg aatatttctc caacgtgaaa ccagacttgt ttgccgatgt aattaacaag 840

tttatggctc acggtaagag catggacatg acccagccag aaggtgagca cagcgcacac 900

gaaggtatgg aaggcatgga catgagccac gcggaatccg cccattaa 948

<210> 272
<211> 315
<212> PRT
<213> Escherichia coli

<400> 272

Met Arg Leu Arg Lys Tyr Asn Lys Ser Leu Gly Trp Leu Ser Leu Phe
1 5 10 15

Ala Gly Thr Val Leu Leu Ser Gly Cys Asn Ser Ala Leu Leu Asp Pro
20 25 30

Lys Gly Gln Ile Gly Leu Glu Gln Arg Ser Leu Ile Leu Thr Ala Phe
35 40 45

```
Gly Leu Met Leu Ile Val Val Ile Pro Ala Ile Leu Met Ala Val Gly
    50                  55                  60

Phe Ala Trp Lys Tyr Arg Ala Ser Asn Lys Asp Ala Lys Tyr Ser Pro
65                  70                  75                  80

Asn Trp Ser His Ser Asn Lys Val Glu Ala Val Val Trp Thr Val Pro
                85                  90                  95

Ile Leu Ile Ile Ile Phe Leu Ala Val Leu Thr Trp Lys Thr Thr His
            100                 105                 110

Ala Leu Glu Pro Ser Lys Pro Leu Ala His Asp Glu Lys Pro Ile Thr
        115                 120                 125

Ile Glu Val Val Ser Met Asp Trp Lys Trp Phe Phe Ile Tyr Pro Glu
    130                 135                 140

Gln Gly Ile Ala Thr Val Asn Glu Ile Ala Phe Pro Ala Asn Thr Pro
145                 150                 155                 160

Val Tyr Phe Lys Val Thr Ser Asn Ser Val Met Asn Ser Phe Phe Ile
                165                 170                 175

Pro Arg Leu Gly Ser Gln Ile Tyr Ala Met Ala Gly Met Gln Thr Arg
            180                 185                 190

Leu His Leu Ile Ala Asn Glu Pro Gly Thr Tyr Asp Gly Ile Ser Ala
        195                 200                 205

Ser Tyr Ser Gly Pro Gly Phe Ser Gly Met Lys Phe Lys Ala Ile Ala
    210                 215                 220

Thr Pro Asp Arg Ala Ala Phe Asp Gln Trp Val Ala Lys Ala Lys Gln
225                 230                 235                 240

Ser Pro Asn Thr Met Ser Asp Met Ala Ala Phe Glu Lys Leu Ala Ala
                245                 250                 255

Pro Ser Glu Tyr Asn Gln Val Glu Tyr Phe Ser Asn Val Lys Pro Asp
            260                 265                 270

Leu Phe Ala Asp Val Ile Asn Lys Phe Met Ala His Gly Lys Ser Met
        275                 280                 285

Asp Met Thr Gln Pro Glu Gly Glu His Ser Ala His Glu Gly Met Glu
    290                 295                 300
```

```
Gly Met Asp Met Ser His Ala Glu Ser Ala His
305                 310                 315


<210>  273
<211>  1992
<212>  DNA
<213>  Escherichia coli

<400>  273
atgttcggaa aattatcact tgatgcagtc ccgttccatg aacctatcgt catggttacg      60

atcgctggca ttattttggg aggtctggcg ctcgttggcc tgatcactta cttcggtaag     120

tggacctacc tgtggaaaga gtggctgacc tccgtcgacc ataaacgcct cggtatcatg     180

tatatcatcg tggcgattgt gatgttgctg cgtggttttg ctgacgccat tatgatgcgt     240

agccagcagg ctcttgcctc ggcgggcgaa gcgggcttcc tgccacctca ccactacgat     300

cagatcttta ccgcgcacgg cgtgattatg atcttcttcg tggcgatgcc tttcgttatc     360

ggtctgatga acctggtggt tccgctgcag atcggcgcgc gtgacgttgc gttcccgttc     420

ctcaacaact taagcttctg gtttaccgtt gttggtgtga ttctggttaa cgtttctctc     480

ggcgtgggcg aatttgcgca gaccggctgg ctggcctatc caccgctatc gggaatagag     540

tacagtccgg gagtcggtgt cgattactgg atatggagtc tccagctatc cggtataggt     600

acgacgctta ccggtatcaa cttcttcgtt accattctga agatgcgcgc accgggcatg     660

accatgttca agatgccagt atttacctgg gcatcactgt gcgcgaacgt actgattatt     720

gcttccttcc caattctgac ggttaccgtc gcgttgttga ccctggatcg ctatctgggc     780

acccatttct ttaccaacga tatgggtggc aacatgatga tgtacatcaa cctgatttgg     840

gcctggggcc acccggaagt ttacatcctg atcctgcctg ttttcggtgt gttctccgaa     900

attgcggcaa ccttctcgcg taaacgtctg tttggttata cctcgctggt atgggcaacc     960

gtctgtatca ccgtgctgtc gttcatcgtt tggctgcacc acttctttac gatgggtgcg    1020

ggcgcgaacg taaacgcctt ctttggtatc accacaatga ttatcgccat cccgaccggg    1080

gtgaagatct tcaactggct gttcaccatg tatcagggcc gcatcgtgtt ccattctgcg    1140

atgctgtgga ccatcggttt tatcgtcacc ttctcggtgg gcgggatgac tggcgtgctg    1200

ctggccgtac cgggcgcgga cttcgttctg cataacagcc tgttcctgat tgcgcacttc    1260

cataacgtga tcatcggcgg cgtggtcttc ggctgcttcg cagggatgac ctactggtgg    1320

cctaaagcgt tcggtttcaa actgaacgaa acctggggta aacgcgcgtt ctggttctgg    1380

atcatcggct tcttcgttgc ctttatgcca ctgtatgcgc tgggcttcat gggcatgacc    1440

cgtcgtttga gccagcagat tgacccgcag ttccacacca tgctgatgat tgcagccagc    1500

ggtgcagtac tgattgcgct gggtattctc tgcctcgtta ttcagatgta cgtttctatt    1560
```

cgcgaccgcg accagaaccg tgacctgact ggcgacccgt ggggtggccg tacgctggag 1620

tgggcaacct cttccccgcc tccgttctat aactttgccg tagtgccgca cgttcacgaa 1680

cgtgatgcat tctgggaaat gaaagagaaa ggcgaagcgt ataaaaagcc tgaccactat 1740

gaagaaattc atatgccgaa aaacagcggt gcaggtatcg tcattgcagc tttctccacc 1800

atcttcggtt tcgccatgat ctggcatatc tggtggctgg cgattgttgg cttcgcaggc 1860

atgatcatca cctggatcgt gaaaagcttc gacgaggacg tggattacta cgtgccggtg 1920

gcagaaatcg aaaaactgga aaaccagcat ttcgatgaga ttactaaggc agggctgaaa 1980

aatggcaact ga 1992

<210> 274
<211> 663
<212> PRT
<213> Escherichia coli

<400> 274

Met Phe Gly Lys Leu Ser Leu Asp Ala Val Pro Phe His Glu Pro Ile
1 5 10 15

Val Met Val Thr Ile Ala Gly Ile Ile Leu Gly Gly Leu Ala Leu Val
20 25 30

Gly Leu Ile Thr Tyr Phe Gly Lys Trp Thr Tyr Leu Trp Lys Glu Trp
35 40 45

Leu Thr Ser Val Asp His Lys Arg Leu Gly Ile Met Tyr Ile Ile Val
50 55 60

Ala Ile Val Met Leu Leu Arg Gly Phe Ala Asp Ala Ile Met Met Arg
65 70 75 80

Ser Gln Gln Ala Leu Ala Ser Ala Gly Glu Ala Gly Phe Leu Pro Pro
85 90 95

His His Tyr Asp Gln Ile Phe Thr Ala His Gly Val Ile Met Ile Phe
100 105 110

Phe Val Ala Met Pro Phe Val Ile Gly Leu Met Asn Leu Val Val Pro
115 120 125

Leu Gln Ile Gly Ala Arg Asp Val Ala Phe Pro Phe Leu Asn Asn Leu
130 135 140

Ser Phe Trp Phe Thr Val Val Gly Val Ile Leu Val Asn Val Ser Leu
145 150 155 160

```
Gly Val Gly Glu Phe Ala Gln Thr Gly Trp Leu Ala Tyr Pro Pro Leu
                165                 170                 175

Ser Gly Ile Glu Tyr Ser Pro Gly Val Gly Val Asp Tyr Trp Ile Trp
            180                 185                 190

Ser Leu Gln Leu Ser Gly Ile Gly Thr Thr Leu Thr Gly Ile Asn Phe
        195                 200                 205

Phe Val Thr Ile Leu Lys Met Arg Ala Pro Gly Met Thr Met Phe Lys
    210                 215                 220

Met Pro Val Phe Thr Trp Ala Ser Leu Cys Ala Asn Val Leu Ile Ile
225                 230                 235                 240

Ala Ser Phe Pro Ile Leu Thr Val Thr Val Ala Leu Leu Thr Leu Asp
                245                 250                 255

Arg Tyr Leu Gly Thr His Phe Phe Thr Asn Asp Met Gly Gly Asn Met
            260                 265                 270

Met Met Tyr Ile Asn Leu Ile Trp Ala Trp Gly His Pro Glu Val Tyr
        275                 280                 285

Ile Leu Ile Leu Pro Val Phe Gly Val Phe Ser Glu Ile Ala Ala Thr
    290                 295                 300

Phe Ser Arg Lys Arg Leu Phe Gly Tyr Thr Ser Leu Val Trp Ala Thr
305                 310                 315                 320

Val Cys Ile Thr Val Leu Ser Phe Ile Val Trp Leu His His Phe Phe
                325                 330                 335

Thr Met Gly Ala Gly Ala Asn Val Asn Ala Phe Phe Gly Ile Thr Thr
            340                 345                 350

Met Ile Ile Ala Ile Pro Thr Gly Val Lys Ile Phe Asn Trp Leu Phe
        355                 360                 365

Thr Met Tyr Gln Gly Arg Ile Val Phe His Ser Ala Met Leu Trp Thr
    370                 375                 380

Ile Gly Phe Ile Val Thr Phe Ser Val Gly Gly Met Thr Gly Val Leu
385                 390                 395                 400

Leu Ala Val Pro Gly Ala Asp Phe Val Leu His Asn Ser Leu Phe Leu
```

405 410 415

Ile Ala His Phe His Asn Val Ile Ile Gly Gly Val Val Phe Gly Cys
420 425 430

Phe Ala Gly Met Thr Tyr Trp Trp Pro Lys Ala Phe Gly Phe Lys Leu
435 440 445

Asn Glu Thr Trp Gly Lys Arg Ala Phe Trp Phe Trp Ile Ile Gly Phe
450 455 460

Phe Val Ala Phe Met Pro Leu Tyr Ala Leu Gly Phe Met Gly Met Thr
465 470 475 480

Arg Arg Leu Ser Gln Gln Ile Asp Pro Gln Phe His Thr Met Leu Met
485 490 495

Ile Ala Ala Ser Gly Ala Val Leu Ile Ala Leu Gly Ile Leu Cys Leu
500 505 510

Val Ile Gln Met Tyr Val Ser Ile Arg Asp Arg Asp Gln Asn Arg Asp
515 520 525

Leu Thr Gly Asp Pro Trp Gly Gly Arg Thr Leu Glu Trp Ala Thr Ser
530 535 540

Ser Pro Pro Pro Phe Tyr Asn Phe Ala Val Val Pro His Val His Glu
545 550 555 560

Arg Asp Ala Phe Trp Glu Met Lys Glu Lys Gly Glu Ala Tyr Lys Lys
565 570 575

Pro Asp His Tyr Glu Glu Ile His Met Pro Lys Asn Ser Gly Ala Gly
580 585 590

Ile Val Ile Ala Ala Phe Ser Thr Ile Phe Gly Phe Ala Met Ile Trp
595 600 605

His Ile Trp Trp Leu Ala Ile Val Gly Phe Ala Gly Met Ile Ile Thr
610 615 620

Trp Ile Val Lys Ser Phe Asp Glu Asp Val Asp Tyr Tyr Val Pro Val
625 630 635 640

Ala Glu Ile Glu Lys Leu Glu Asn Gln His Phe Asp Glu Ile Thr Lys
645 650 655

Ala Gly Leu Lys Asn Gly Asn
660

<210> 275
<211> 615
<212> DNA
<213> Escherichia coli

<400> 275
atggcaactg atactttgac gcacgcgact gcccacgcgc acgaacacgg gcaccacgat 60

gcaggcggaa ccaaaatctt cggattttgg atctacctga tgagcgactg cattctgttc 120

tctatcttgt ttgctaccta tgccgttctg gtgaacggca ccgcaggcgg cccgacaggt 180

aaggacattt tcgaactgcc gttcgttctg gttgaaactt tcttgctgtt gttcagctcc 240

atcacctacg gcatggcggc tatcgccatg tacaaaaaca acaaaagcca ggttatctcc 300

tggctggcgt tgacctggtt gtttggtgcc ggatttatcg ggatggaaat ctatgaattc 360

catcacctga ttgttaacgg catgggtccg gatcgcagcg gcttcctgtc agcgttcttt 420

gcgctggtcg gcacgcacgg tctgcacgtc acttctggtc ttatctggat ggcggtgctg 480

atggtgcaaa tcgcccgtcg cggcctgacc agcactaacc gtacccgcat catgtgcctg 540

agcctgttct ggcacttcct ggatgtggtt tggatctgtg tgttcactgt tgtttatctg 600

atgggggcga tgtaa 615

<210> 276
<211> 204
<212> PRT
<213> Escherichia coli

<400> 276

Met Ala Thr Asp Thr Leu Thr His Ala Thr Ala His Ala His Glu His
1 5 10 15

Gly His His Asp Ala Gly Gly Thr Lys Ile Phe Gly Phe Trp Ile Tyr
20 25 30

Leu Met Ser Asp Cys Ile Leu Phe Ser Ile Leu Phe Ala Thr Tyr Ala
35 40 45

Val Leu Val Asn Gly Thr Ala Gly Gly Pro Thr Gly Lys Asp Ile Phe
50 55 60

Glu Leu Pro Phe Val Leu Val Glu Thr Phe Leu Leu Leu Phe Ser Ser
65 70 75 80

Ile Thr Tyr Gly Met Ala Ala Ile Ala Met Tyr Lys Asn Asn Lys Ser
85 90 95

Gln Val Ile Ser Trp Leu Ala Leu Thr Trp Leu Phe Gly Ala Gly Phe
100 105 110

Ile Gly Met Glu Ile Tyr Glu Phe His His Leu Ile Val Asn Gly Met
115 120 125

Gly Pro Asp Arg Ser Gly Phe Leu Ser Ala Phe Phe Ala Leu Val Gly
130 135 140

Thr His Gly Leu His Val Thr Ser Gly Leu Ile Trp Met Ala Val Leu
145 150 155 160

Met Val Gln Ile Ala Arg Arg Gly Leu Thr Ser Thr Asn Arg Thr Arg
165 170 175

Ile Met Cys Leu Ser Leu Phe Trp His Phe Leu Asp Val Val Trp Ile
180 185 190

Cys Val Phe Thr Val Val Tyr Leu Met Gly Ala Met
195 200

<210> 277
<211> 330
<212> DNA
<213> Escherichia coli

<400> 277
atgagtcatt ctaccgatca cagcggcgcg tcccatggca gcgtaaaaac ctacatgaca 60

ggctttatcc tgtcgatcat tctgacggtg attccgttct ggatggtgat gacaggagct 120

gcctctccgg ccgtaattct gggaacaatc ctggcaatgg cagtggtaca ggttctggtg 180

catctggtgt gcttcctgca catgaatacc aaatcagatg aaggctggaa catgacggcg 240

tttgtcttca ccgtgctaat catcgctatc ctggttgtag gctccatctg gattatgtgg 300

aacctcaact acaacatgat gatgcactaa 330

<210> 278
<211> 109
<212> PRT
<213> Escherichia coli

<400> 278

Met Ser His Ser Thr Asp His Ser Gly Ala Ser His Gly Ser Val Lys
1 5 10 15

Thr Tyr Met Thr Gly Phe Ile Leu Ser Ile Ile Leu Thr Val Ile Pro
20 25 30

Phe Trp Met Val Met Thr Gly Ala Ala Ser Pro Ala Val Ile Leu Gly
35 40 45

Thr Ile Leu Ala Met Ala Val Val Gln Val Leu Val His Leu Val Cys
50 55 60

Phe Leu His Met Asn Thr Lys Ser Asp Glu Gly Trp Asn Met Thr Ala
65 70 75 80

Phe Val Phe Thr Val Leu Ile Ile Ala Ile Leu Val Val Gly Ser Ile
85 90 95

Trp Ile Met Trp Asn Leu Asn Tyr Asn Met Met Met His
100 105

<210> 279
<211> 891
<212> DNA
<213> Escherichia coli

<400> 279
atgatgttta agcaatacct gcaagtaacg aaaccaggca tcatctttgg caacctgatc 60

tcggtgattg ggggattcct gctggcctca aagggcagca ttgattatcc cctgtttatc 120

tacacgctgg ttggggtgtc actggttgtg gcgtcgggtt gtgtgtttaa caactacatc 180

gacagggata tcgacagaaa gatggaaagg acgaagaatc gggtgctggt gaaaggcctg 240

atctctcctg ctgtctcgct ggtgtacgcc acgttgctgg gtattgctgg ctttatgctg 300

ctgtggtttg gcgcgaatcc gctggcctgc tggctggggg tgatgggctt tgtggtttat 360

gtcggcgttt atagcctgta catgaaacgc cactctgtct acggcacgtt gattggttcg 420

ctctccggcg ctgcgccgcc ggtgatcggc tactgtgcgg taaccggtga gttcgatagc 480

ggcgcagcga tcctgctggc tatcttcagc ctgtggcaga tgcctcactc ctatgccatc 540

gccattttcc gctttaagga ttaccaggcg gcaaacattc cggtattgcc agtggtaaaa 600

ggcatttcgg tggcgaagaa tcacatcacg ctgtatatca tcgcctttgc cgttgccacg 660

ctgatgctct ctcttggcgg ttacgctggg tataaatatc tggtggtcgc cgcggcggtt 720

agcgtctggt ggttaggtat ggctctgcgc ggttataaag ttgctgatga cagaatctgg 780

gcgcgcaagc tgttcggctt ctctatcatc gccatcactg ccctctcggt gatgatgtcc 840

gttgatttta tggtaccgga ctcgcatacg ctgctggctg ctgtgtggta a 891

<210> 280
<211> 296
<212> PRT
<213> Escherichia coli

<400> 280

Met Met Phe Lys Gln Tyr Leu Gln Val Thr Lys Pro Gly Ile Ile Phe
1 5 10 15

Gly Asn Leu Ile Ser Val Ile Gly Gly Phe Leu Leu Ala Ser Lys Gly
20 25 30

Ser Ile Asp Tyr Pro Leu Phe Ile Tyr Thr Leu Val Gly Val Ser Leu
35 40 45

Val Val Ala Ser Gly Cys Val Phe Asn Asn Tyr Ile Asp Arg Asp Ile
50 55 60

Asp Arg Lys Met Glu Arg Thr Lys Asn Arg Val Leu Val Lys Gly Leu
65 70 75 80

Ile Ser Pro Ala Val Ser Leu Val Tyr Ala Thr Leu Leu Gly Ile Ala
85 90 95

Gly Phe Met Leu Leu Trp Phe Gly Ala Asn Pro Leu Ala Cys Trp Leu
100 105 110

Gly Val Met Gly Phe Val Val Tyr Val Gly Val Tyr Ser Leu Tyr Met
115 120 125

Lys Arg His Ser Val Tyr Gly Thr Leu Ile Gly Ser Leu Ser Gly Ala
130 135 140

Ala Pro Pro Val Ile Gly Tyr Cys Ala Val Thr Gly Glu Phe Asp Ser
145 150 155 160

Gly Ala Ala Ile Leu Leu Ala Ile Phe Ser Leu Trp Gln Met Pro His
165 170 175

Ser Tyr Ala Ile Ala Ile Phe Arg Phe Lys Asp Tyr Gln Ala Ala Asn
180 185 190

Ile Pro Val Leu Pro Val Val Lys Gly Ile Ser Val Ala Lys Asn His
195 200 205

Ile Thr Leu Tyr Ile Ile Ala Phe Ala Val Ala Thr Leu Met Leu Ser
210 215 220

Leu Gly Gly Tyr Ala Gly Tyr Lys Tyr Leu Val Val Ala Ala Ala Val
225 230 235 240

Ser Val Trp Trp Leu Gly Met Ala Leu Arg Gly Tyr Lys Val Ala Asp
245 250 255

```
Asp Arg Ile Trp Ala Arg Lys Leu Phe Gly Phe Ser Ile Ile Ala Ile
            260                 265                 270

Thr Ala Leu Ser Val Met Met Ser Val Asp Phe Met Val Pro Asp Ser
        275                 280                 285

His Thr Leu Leu Ala Ala Val Trp
    290                 295


<210>  281
<211>  444
<212>  DNA
<213>  Escherichia coli

<400>  281
atgagtatgt caacatccac tgaagtcatc gctcatcact gggcattcgc tatctttctt      60

atcgttgcca ttggcctgtg ttgcctgatg ctggtaggcg gttggttttt aggcggtcgc     120

gcacgcgcga ggtcgaaaaa cgtgccgttt gaatccggta tcgactcggt cggctccgcc     180

cgcttacgcc tgtccgccaa gttttatctg gtggccatgt tcttcgttat cttcgacgtt     240

gaagcgctgt atctgttcgc atggtcaacc tctatccgcg aaagcggctg ggtaggcttt     300

gtggaagctg caatttttat ttttgtgtta ctggcaggtc tggtttatct ggtgcgtatt     360

ggcgcgctgg actggacgcc cgcgcgttca cgccgcgagc gtatgaaccc ggaaacgaac     420

agtatcgcta atcgtcaacg ctaa                                             444


<210>  282
<211>  147
<212>  PRT
<213>  Escherichia coli

<400>  282

Met Ser Met Ser Thr Ser Thr Glu Val Ile Ala His His Trp Ala Phe
1               5                   10                  15

Ala Ile Phe Leu Ile Val Ala Ile Gly Leu Cys Cys Leu Met Leu Val
            20                  25                  30

Gly Gly Trp Phe Leu Gly Gly Arg Ala Arg Ala Arg Ser Lys Asn Val
        35                  40                  45

Pro Phe Glu Ser Gly Ile Asp Ser Val Gly Ser Ala Arg Leu Arg Leu
    50                  55                  60

Ser Ala Lys Phe Tyr Leu Val Ala Met Phe Phe Val Ile Phe Asp Val
65                  70                  75                  80
```

Glu Ala Leu Tyr Leu Phe Ala Trp Ser Thr Ser Ile Arg Glu Ser Gly
85 90 95

Trp Val Gly Phe Val Glu Ala Ala Ile Phe Ile Phe Val Leu Leu Ala
100 105 110

Gly Leu Val Tyr Leu Val Arg Ile Gly Ala Leu Asp Trp Thr Pro Ala
115 120 125

Arg Ser Arg Arg Glu Arg Met Asn Pro Glu Thr Asn Ser Ile Ala Asn
130 135 140

Arg Gln Arg
145

<210> 283
<211> 663
<212> DNA
<213> Escherichia coli

<400> 283
atggattata cgctcacccg catagatccc aacggtgaga acgaccgtta ccccctgcaa 60

aagcaggaga tcgtaaccga ccctctggag caagaagtta acaaaaacgt gtttatgggc 120

aagctcaatg acatggttaa ctggggtcgt aaaaactcaa tttggccgta taacttcggt 180

ctttcctgct gttacgttga gatggtgact tcgtttaccg cggtgcatga cgtggcgcgt 240

tttggcgcag aagtattgcg tgcttcgccg cgtcaggctg acctgatggt ggttgcagga 300

acctgcttta ccaaaatggc accggttatt cagcgtctgt atgaccagat gctggaacca 360

aaatgggtta tctcaatggg tgcctgtgcc aactctggtg gtatgtacga tatttattcc 420

gttgtgcagg gcgtcgataa attcatcccg gttgatgtgt atatcccggg ctgcccgccg 480

cgtcctgaag cgtacatgca ggcactgatg ctgttgcagg aatctatcgg caaagaacgt 540

cgtccgctct cctgggtggt tggcgatcag ggcgtttatc gcgccaatat gcaatcagag 600

cgcgaacgca agcgcggtga acgcattgcc gtaactaacc tgcgtacacc tgacgagatt 660

taa 663

<210> 284
<211> 220
<212> PRT
<213> Escherichia coli

<400> 284

Met Asp Tyr Thr Leu Thr Arg Ile Asp Pro Asn Gly Glu Asn Asp Arg
1 5 10 15

Tyr Pro Leu Gln Lys Gln Glu Ile Val Thr Asp Pro Leu Glu Gln Glu
20 25 30

Val Asn Lys Asn Val Phe Met Gly Lys Leu Asn Asp Met Val Asn Trp
35 40 45

Gly Arg Lys Asn Ser Ile Trp Pro Tyr Asn Phe Gly Leu Ser Cys Cys
50 55 60

Tyr Val Glu Met Val Thr Ser Phe Thr Ala Val His Asp Val Ala Arg
65 70 75 80

Phe Gly Ala Glu Val Leu Arg Ala Ser Pro Arg Gln Ala Asp Leu Met
85 90 95

Val Val Ala Gly Thr Cys Phe Thr Lys Met Ala Pro Val Ile Gln Arg
100 105 110

Leu Tyr Asp Gln Met Leu Glu Pro Lys Trp Val Ile Ser Met Gly Ala
115 120 125

Cys Ala Asn Ser Gly Gly Met Tyr Asp Ile Tyr Ser Val Val Gln Gly
130 135 140

Val Asp Lys Phe Ile Pro Val Asp Val Tyr Ile Pro Gly Cys Pro Pro
145 150 155 160

Arg Pro Glu Ala Tyr Met Gln Ala Leu Met Leu Leu Gln Glu Ser Ile
165 170 175

Gly Lys Glu Arg Arg Pro Leu Ser Trp Val Val Gly Asp Gln Gly Val
180 185 190

Tyr Arg Ala Asn Met Gln Ser Glu Arg Glu Arg Lys Arg Gly Glu Arg
195 200 205

Ile Ala Val Thr Asn Leu Arg Thr Pro Asp Glu Ile
210 215 220

<210> 285
<211> 1791
<212> DNA
<213> Escherichia coli

<400> 285
atgaccgact taaccgcgca agaacccgcc tggcagaccc gcgatcatct tgatgatccg 60

gtgattggcg aactgcgcaa ccgttttggg ccggatgcct ttactgttca ggcgactcgc 120

accggggttc ccgttgtgtg gatcaagcgt gaacaattac tggaagttgg cgatttctta 180

aagaaactgc cgaaacctta cgtcatgctg tttgacttac acggcatgga cgaacgtctg 240

cgcacacacc gcgaagggtt acctgccgcg gatttttccg ttttctacca tctgatttct 300

atcgatcgta accgcgacat catgctgaag gtggcgctgg cagaaaacga cctgcacgta 360

ccgaccttca ccaaactgtt cccgaacgct aactggtatg agcgtgaaac ctgggatctg 420

tttggcatta ctttcgacgg tcacccgaac ctgcgacgca tcatgatgcc gcaaacctgg 480

aaaggtcacc cgctgcgtaa agattatccg gcgcgcgcta ccgaattctc gccgtttgag 540

ctgaccaaag ccaaacagga tctggagatg gaagccctga ccttcaaacc ggaagagtgg 600

gggatgaagc gcggcaccga aaacgaggac ttcatgttcc tcaacctcgg tccgaaccac 660

ccgtcggcgc acggggcttt ccgtatcgtt ttgcaactcg atggcgaaga gattgtcgac 720

tgcgtaccag acatcggtta ccaccaccgt ggtgcggaga aaatgggcga acgccagtcc 780

tggcacagct acattccgta tactgaccgt atcgaatacc tcggcggctg cgttaacgaa 840

atgccttacg tgctggcggt agagaaactg gccgggatca ccgtgccgga tcgcgttaac 900

gtcattcgcg ttatgctctc cgaactgttc cgcatcaaca gtcacctgct gtatatctcg 960

acctttattc aggacgtcgg cgcaatgacg ccagtgttct tcgcctttac cgatcgtcag 1020

aaaatttacg atctggtgga agcaatcact ggtttccgta tgcacccggc gtggttccgt 1080

attggcggcg tagcgcacga cctgccgcgc ggctgggatc gcctgctgcg tgagttcctc 1140

gactggatgc cgaaacgtct ggcgtcttac gagaaagcgg cgctgcaaaa caccattctg 1200

aaaggtcgtt cccagggcgt tgccgcctat ggcgcgaaag aggcgctgga gtggggcacc 1260

actggcgcgg gcctgcgtgc taccgggatc gacttcgacg tgcgtaaggc gcgtccttat 1320

tctggctatg aaaacttcga ctttgaaatc ccggtgggtg gtggcgtttc tgactgctac 1380

acccgcgtaa tgcttaaagt ggaagagctg cgccagagtc tgcgcattct tgagcagtgc 1440

ctcaacaaca tgccggaagg cccgttcaaa gcggatcacc cgctgaccac gccgccgccg 1500

aaagagcgca cgctgcaaca tatcgaaacc ctgatcaccc acttcctgca agtgtcgtgg 1560

ggtccggtga tgcctgccaa tgaatctttc cagatgattg aggcgaccaa agggatcaac 1620

agttactacc tgaccagcga cggcagcacc atgagttacc gcacccgtgt tcgtaccccg 1680

agctttgcgc atttgcagca aattccggcg gcgatccgcg gcagcctggt gtctgacctg 1740

attgtttatc tgggcagtat cgattttgtt atgtcagatg tggaccgcta a 1791

<210> 286
<211> 596
<212> PRT
<213> Escherichia coli

<400> 286

Met Thr Asp Leu Thr Ala Gln Glu Pro Ala Trp Gln Thr Arg Asp His
1               5                   10                  15

Leu Asp Asp Pro Val Ile Gly Glu Leu Arg Asn Arg Phe Gly Pro Asp
            20                  25                  30

Ala Phe Thr Val Gln Ala Thr Arg Thr Gly Val Pro Val Val Trp Ile
        35                  40                  45

Lys Arg Glu Gln Leu Leu Glu Val Gly Asp Phe Leu Lys Lys Leu Pro
    50                  55                  60

Lys Pro Tyr Val Met Leu Phe Asp Leu His Gly Met Asp Glu Arg Leu
65                  70                  75                  80

Arg Thr His Arg Glu Gly Leu Pro Ala Ala Asp Phe Ser Val Phe Tyr
                85                  90                  95

His Leu Ile Ser Ile Asp Arg Asn Arg Asp Ile Met Leu Lys Val Ala
            100                 105                 110

Leu Ala Glu Asn Asp Leu His Val Pro Thr Phe Thr Lys Leu Phe Pro
        115                 120                 125

Asn Ala Asn Trp Tyr Glu Arg Glu Thr Trp Asp Leu Phe Gly Ile Thr
    130                 135                 140

Phe Asp Gly His Pro Asn Leu Arg Arg Ile Met Met Pro Gln Thr Trp
145                 150                 155                 160

Lys Gly His Pro Leu Arg Lys Asp Tyr Pro Ala Arg Ala Thr Glu Phe
                165                 170                 175

Ser Pro Phe Glu Leu Thr Lys Ala Lys Gln Asp Leu Glu Met Glu Ala
            180                 185                 190

Leu Thr Phe Lys Pro Glu Glu Trp Gly Met Lys Arg Gly Thr Glu Asn
        195                 200                 205

Glu Asp Phe Met Phe Leu Asn Leu Gly Pro Asn His Pro Ser Ala His
    210                 215                 220

Gly Ala Phe Arg Ile Val Leu Gln Leu Asp Gly Glu Glu Ile Val Asp
225                 230                 235                 240

Cys Val Pro Asp Ile Gly Tyr His His Arg Gly Ala Glu Lys Met Gly
                245                 250                 255

Glu Arg Gln Ser Trp His Ser Tyr Ile Pro Tyr Thr Asp Arg Ile Glu
260 265 270

Tyr Leu Gly Gly Cys Val Asn Glu Met Pro Tyr Val Leu Ala Val Glu
275 280 285

Lys Leu Ala Gly Ile Thr Val Pro Asp Arg Val Asn Val Ile Arg Val
290 295 300

Met Leu Ser Glu Leu Phe Arg Ile Asn Ser His Leu Leu Tyr Ile Ser
305 310 315 320

Thr Phe Ile Gln Asp Val Gly Ala Met Thr Pro Val Phe Phe Ala Phe
325 330 335

Thr Asp Arg Gln Lys Ile Tyr Asp Leu Val Glu Ala Ile Thr Gly Phe
340 345 350

Arg Met His Pro Ala Trp Phe Arg Ile Gly Gly Val Ala His Asp Leu
355 360 365

Pro Arg Gly Trp Asp Arg Leu Leu Arg Glu Phe Leu Asp Trp Met Pro
370 375 380

Lys Arg Leu Ala Ser Tyr Glu Lys Ala Ala Leu Gln Asn Thr Ile Leu
385 390 395 400

Lys Gly Arg Ser Gln Gly Val Ala Ala Tyr Gly Ala Lys Glu Ala Leu
405 410 415

Glu Trp Gly Thr Thr Gly Ala Gly Leu Arg Ala Thr Gly Ile Asp Phe
420 425 430

Asp Val Arg Lys Ala Arg Pro Tyr Ser Gly Tyr Glu Asn Phe Asp Phe
435 440 445

Glu Ile Pro Val Gly Gly Gly Val Ser Asp Cys Tyr Thr Arg Val Met
450 455 460

Leu Lys Val Glu Glu Leu Arg Gln Ser Leu Arg Ile Leu Glu Gln Cys
465 470 475 480

Leu Asn Asn Met Pro Glu Gly Pro Phe Lys Ala Asp His Pro Leu Thr
485 490 495

Thr Pro Pro Pro Lys Glu Arg Thr Leu Gln His Ile Glu Thr Leu Ile
500 505 510

Thr His Phe Leu Gln Val Ser Trp Gly Pro Val Met Pro Ala Asn Glu
515 520 525

Ser Phe Gln Met Ile Glu Ala Thr Lys Gly Ile Asn Ser Tyr Tyr Leu
530 535 540

Thr Ser Asp Gly Ser Thr Met Ser Tyr Arg Thr Arg Val Arg Thr Pro
545 550 555 560

Ser Phe Ala His Leu Gln Gln Ile Pro Ala Ala Ile Arg Gly Ser Leu
565 570 575

Val Ser Asp Leu Ile Val Tyr Leu Gly Ser Ile Asp Phe Val Met Ser
580 585 590

Asp Val Asp Arg
595

<210> 287
<211> 501
<212> DNA
<213> Escherichia coli

<400> 287
atgcacgaga atcaacaacc acaaaccgag gcttttgagc tgagtgcggc agagcgtgaa 60

gcgatcgagc acgagatgca ccactacgaa gacccgcgtg cggcgtccat tgaagcgctg 120

aaaatcgttc agaagcagcg tggctgggtg ccggatggtg cgatccacgc gatcgccgat 180

gtgctgggta ttccggcaag cgacgtcgaa ggtgtggcaa cgttctacag tcagatcttc 240

cgccagccgg ttggtcgcca tgtgatccgt tattgtgaca gcgtggtctg tcatatcaac 300

ggttatcagg gtattcaggc ggcgctcgag aaaaagctga acatcaaacc agggcaaacg 360

acatttgatg gccgctttac gctgctgcca acttgctgcc tggggaactg tgataaaggg 420

ccaaacatga tgatcgatga ggacactcac gcgcatctga ccccggaagc gatccctgaa 480

ctgctggagc ggtataaatg a 501

<210> 288
<211> 166
<212> PRT
<213> Escherichia coli

<400> 288

Met His Glu Asn Gln Gln Pro Gln Thr Glu Ala Phe Glu Leu Ser Ala
1 5 10 15

Ala Glu Arg Glu Ala Ile Glu His Glu Met His His Tyr Glu Asp Pro

20 25 30

Arg Ala Ala Ser Ile Glu Ala Leu Lys Ile Val Gln Lys Gln Arg Gly
35 40 45

Trp Val Pro Asp Gly Ala Ile His Ala Ile Ala Asp Val Leu Gly Ile
50 55 60

Pro Ala Ser Asp Val Glu Gly Val Ala Thr Phe Tyr Ser Gln Ile Phe
65 70 75 80

Arg Gln Pro Val Gly Arg His Val Ile Arg Tyr Cys Asp Ser Val Val
85 90 95

Cys His Ile Asn Gly Tyr Gln Gly Ile Gln Ala Ala Leu Glu Lys Lys
100 105 110

Leu Asn Ile Lys Pro Gly Gln Thr Thr Phe Asp Gly Arg Phe Thr Leu
115 120 125

Leu Pro Thr Cys Cys Leu Gly Asn Cys Asp Lys Gly Pro Asn Met Met
130 135 140

Ile Asp Glu Asp Thr His Ala His Leu Thr Pro Glu Ala Ile Pro Glu
145 150 155 160

Leu Leu Glu Arg Tyr Lys
165

<210> 289
<211> 1338
<212> DNA
<213> Escherichia coli

<400> 289
atgaaaaaca ttatccgtac tcccgaaacg catccgctga cctggcgtct gcgcgatgac 60

aaacagccag tgtggctgga cgaataccgc agcaaaaacg gttacgaagg cgcgcgtaag 120

gcgctgaccg ggctgtctcc ggacgaaatc gttaatcagg taaaagacgc tggtctgaaa 180

gggcgcggcg gcgcgggctt ctcgactggc ctgaaatgga gcctgatgcc gaaagacgaa 240

tccatgaaca tccgttacct gctgtgtaat gccgatgaaa tggagccggg cacctataaa 300

gaccgcctgt tgatggagca actgccgcac ctgctggtgg aaggtatgct catctccgcg 360

tttgcgctga aagcttaccg tggctacatc ttcctgcgtg gcgaatatat cgaagcggca 420

gttaatctgc gccgtgccat tgccgaagcc accgaagcgg gtctgcttgg caaaaacatt 480

atgggaacag gtttcgattt cgaactgttc gtccataccg ggcagggcg ctacatctgc 540

ggggaagaaa cagcgttaat caactccctg gaaggacgtc gtgctaaccc acgctcgaag 600

ccacccttcc cggcaacctc cggcgcatgg ggtaaaccga cctgtgtcaa caacgtcgaa 660

accctgtgta acgttccggc gatcctcgct aacggcgtgg agtggtatca gaacatctcg 720

aaaagtaaag atgctggcac caagctgatg ggcttctccg gtcgggtgaa aaatccggga 780

ctgtgggaac tgccgttcgg caccaccgca cgcgagatcc tcgaagatta cgccggtggt 840

atgcgtgatg gtctgaaatt taaagcctgg cagccaggcg gcgcggggac tgacttcctg 900

accgaagcgc accttgatct gccgatggaa ttcgaaagta tcggtaaagc gggcagccgt 960

ctgggtacgg cgctggcgat ggcggttgac catgagatca acatggtgtc gctggtgcgt 1020

aacctggaag agtttttcgc ccgtgagtcc tgcggctggt gtacgccgtg ccgcgacggt 1080

ctgccgtgga gcgtgaaaat tctgcgtgcg ctggagcgtg gtgaaggtca gccgggcgat 1140

atcgaaacac ttgagcaact gtgtcgattc ttaggcccgg gtaaaacttt ctgtgcccac 1200

gcacctggtg cagtggagcc gttacagagc gccatcaaat atttccgcga agaatttgag 1260

gcgggaatca aacagccgtt cagcaatacc catttgatta atgggattca gccgaacctg 1320

ctgaaagagc gctggtaa 1338

<210> 290
<211> 445
<212> PRT
<213> Escherichia coli

<400> 290

Met Lys Asn Ile Ile Arg Thr Pro Glu Thr His Pro Leu Thr Trp Arg
1 5 10 15

Leu Arg Asp Asp Lys Gln Pro Val Trp Leu Asp Glu Tyr Arg Ser Lys
20 25 30

Asn Gly Tyr Glu Gly Ala Arg Lys Ala Leu Thr Gly Leu Ser Pro Asp
35 40 45

Glu Ile Val Asn Gln Val Lys Asp Ala Gly Leu Lys Gly Arg Gly Gly
50 55 60

Ala Gly Phe Ser Thr Gly Leu Lys Trp Ser Leu Met Pro Lys Asp Glu
65 70 75 80

Ser Met Asn Ile Arg Tyr Leu Leu Cys Asn Ala Asp Glu Met Glu Pro
85 90 95

Gly Thr Tyr Lys Asp Arg Leu Leu Met Glu Gln Leu Pro His Leu Leu
100 105 110

Val Glu Gly Met Leu Ile Ser Ala Phe Ala Leu Lys Ala Tyr Arg Gly
115 120 125

Tyr Ile Phe Leu Arg Gly Glu Tyr Ile Glu Ala Ala Val Asn Leu Arg
130 135 140

Arg Ala Ile Ala Glu Ala Thr Glu Ala Gly Leu Leu Gly Lys Asn Ile
145 150 155 160

Met Gly Thr Gly Phe Asp Phe Glu Leu Phe Val His Thr Gly Ala Gly
165 170 175

Arg Tyr Ile Cys Gly Glu Glu Thr Ala Leu Ile Asn Ser Leu Glu Gly
180 185 190

Arg Arg Ala Asn Pro Arg Ser Lys Pro Pro Phe Pro Ala Thr Ser Gly
195 200 205

Ala Trp Gly Lys Pro Thr Cys Val Asn Asn Val Glu Thr Leu Cys Asn
210 215 220

Val Pro Ala Ile Leu Ala Asn Gly Val Glu Trp Tyr Gln Asn Ile Ser
225 230 235 240

Lys Ser Lys Asp Ala Gly Thr Lys Leu Met Gly Phe Ser Gly Arg Val
245 250 255

Lys Asn Pro Gly Leu Trp Glu Leu Pro Phe Gly Thr Thr Ala Arg Glu
260 265 270

Ile Leu Glu Asp Tyr Ala Gly Gly Met Arg Asp Gly Leu Lys Phe Lys
275 280 285

Ala Trp Gln Pro Gly Gly Ala Gly Thr Asp Phe Leu Thr Glu Ala His
290 295 300

Leu Asp Leu Pro Met Glu Phe Glu Ser Ile Gly Lys Ala Gly Ser Arg
305 310 315 320

Leu Gly Thr Ala Leu Ala Met Ala Val Asp His Glu Ile Asn Met Val
325 330 335

Ser Leu Val Arg Asn Leu Glu Glu Phe Phe Ala Arg Glu Ser Cys Gly
340 345 350

Trp Cys Thr Pro Cys Arg Asp Gly Leu Pro Trp Ser Val Lys Ile Leu
355 360 365

Arg Ala Leu Glu Arg Gly Glu Gly Gln Pro Gly Asp Ile Glu Thr Leu
    370                 375                 380

Glu Gln Leu Cys Arg Phe Leu Gly Pro Gly Lys Thr Phe Cys Ala His
385                 390                 395                 400

Ala Pro Gly Ala Val Glu Pro Leu Gln Ser Ala Ile Lys Tyr Phe Arg
                405                 410                 415

Glu Glu Phe Glu Ala Gly Ile Lys Gln Pro Phe Ser Asn Thr His Leu
            420                 425                 430

Ile Asn Gly Ile Gln Pro Asn Leu Leu Lys Glu Arg Trp
        435                 440                 445

<210> 291
<211> 2727
<212> DNA
<213> Escherichia coli

<400> 291
atggctacaa ttcatgtaga cggcaaagaa tacgaggtca acggagcgga caacctgctg 60
gaagcttgtc tgtctctggg ccttgatatt ccttactttt gctggcatcc ggcgctggga 120
agtgtcggtg cttgccgcca gtgtgcggtg aagcaatacc aaaacgcgga agacacgcgt 180
ggtcgcctgg tgatgtcctg tatgacaccg gcttccgatg gcacctttat ttccattgac 240
gacgaagaag cgaaacagtt ccgtgaaagc gtggtcgagt ggttgatgac caaccacccg 300
cacgactgtc cggtatgtga agagggcggt aactgccatc ttcaggatat gactgtgatg 360
accggacaca gcttccgtcg ctaccgtttc accaaacgta cccaccgtaa tcaggatttg 420
gggccattca tctctcacga aatgaaccgc tgcatcgcct gctaccgctg tgtgcgttac 480
tacaaagatt acgctgacgg tacagatctg ggcgtttacg gtgcgcacga caacgtctac 540
ttcggtcgcc cggaagacgg cacgctggaa agcgaatttt ccggtaacct ggtcgaaatt 600
tgcccgaccg gcgtatttac cgacaaaacg cactccgagc gttacaaccg taaatgggat 660
atgcagtttg cgccgagcat ctgccagcaa tgttccatcg gctgtaacat cagccccggt 720
gaacgttacg gcgaactgcg tcgtatcgaa aaccgttaca acggtacggt aaaccactac 780
ttcctctgcg accgtggtcg tttcggttac ggttacgtca acctgaagga tcgtccgcgt 840
cagccagtac agcgtcgtgg cgatgatttc attaccctca acgccgaaca ggcaatgcag 900
ggcgcggcag atattctgcg tcagtcgaag aaagtgatcg gtattggttc tccgcgtgcc 960
agcgtggaaa gcaactttgc gctgcgtgaa ctggtgggcg aagaaaactt ctacaccggt 1020
atcgctcacg gtgagcagga acgtctgcaa ctggcgctga aagtgctgcg tgaaggcggc 1080

atttatactc cggctctgcg cgaaatcgaa tcttacgatg cggtactggt gctgggcgaa 1140

gacgttaccc agaccggcgc gcgcgtcgcg ctggcagtgc gtcaggctgt gaaaggtaaa 1200

gcgcgcgaaa tggcggcagc acagaaagtg gctgactggc agattgcggc aatcctcaac 1260

atcggtcaac gtgcgaagca tccgctgttt gttaccaacg ttgatgacac ccgtctggat 1320

gatatcgcgg cgtggactta ccgcgcaccg gttgaagatc aggcgcgttt aggttttgcc 1380

atcgcccatg cgctggataa ctctgcacca gcggttgacg gtatcgaacc tgagctgcaa 1440

agcaaaatcg acgtcatcgt gcaggcactg gcaggtgcga agaaaccgtt gattatctcc 1500

gggacgaacg ccggtagctt agaggtgatt caggcggcgg ctaacgtcgc gaaagccctg 1560

aaaggtcgcg gcgctgacgt cggtatcacc atgattgccc gttccgtcaa cagcatgggg 1620

ctgggcatta tgggtggcgg ttcgcttgaa gaagcgttaa ccgaactgga aaccggacgc 1680

gccgacgcgg tggtggtgtt ggaaaacgat ctgcatcgtc acgcttctgc tatccgcgtg 1740

aatgctgcgc tggctaaagc accgctggtg atggtggttg atcatcaacg cacagcgatt 1800

atggaaaacg cccatctggt actttctgct gccagctttg ctgaaagcga cggtacggtg 1860

atcaacaacg aaggccgcgc ccaacgtttc ttccaggttt acgatcctgc ttattacgac 1920

agcaaaactg tcatgctgga aagctggcgc tggttacact cgctgcacag caccctgctg 1980

agccgtgaag tggactggac gcagctcgac catgtgattg acgctgttgt ggcgaaaatc 2040

ccggaactgg caggtatcaa agatgctgcg ccggatgcga cattccgtat tcgtgggcag 2100

aaactggccc gtgaaccgca ccgttacagc ggtcgtaccg ccatgcgcgc caatatcagc 2160

gttcatgagc cgcgtcagcc gcaggatatt gacaccatgt tcaccttctc gatggaaggt 2220

aacaaccagc cgactgcgca ccgttcgcaa gtgccgtttg cctgggcgcc gggctggaac 2280

tccccgcagg cgtggaacaa attccaggac gaagtgggcg gcaaactgcg ctttggcgat 2340

ccgggcgtgc gtctgtttga aaccagcgaa aatggtctgg attacttcac cagcgtaccg 2400

gcacgcttcc agccgcagga cgggaaatgg cgtatcgcgc cgtattacca cctgtttggc 2460

agcgatgaat tgtcacagcg tgctccggtc ttccagagcc gtatgccgca gccgtacatc 2520

aaactcaacc cagcggatgc cgcgaagttg ggtgtgaacg caggtacacg cgtctccttt 2580

agttacgatg gcaacacggt cacgctgccg gttgaaatcg ccgaaggact gacggcaggg 2640

caggtgggct tgccgatggg tatgtccggc attgctccgg tgctggctgg cgcgcatctt 2700

gaggatctca aggaggcaca acaatga 2727

<210> 292
<211> 908
<212> PRT
<213> Escherichia coli

<400> 292

Met Ala Thr Ile His Val Asp Gly Lys Glu Tyr Glu Val Asn Gly Ala
1 5 10 15

Asp Asn Leu Leu Glu Ala Cys Leu Ser Leu Gly Leu Asp Ile Pro Tyr
20 25 30

Phe Cys Trp His Pro Ala Leu Gly Ser Val Gly Ala Cys Arg Gln Cys
35 40 45

Ala Val Lys Gln Tyr Gln Asn Ala Glu Asp Thr Arg Gly Arg Leu Val
50 55 60

Met Ser Cys Met Thr Pro Ala Ser Asp Gly Thr Phe Ile Ser Ile Asp
65 70 75 80

Asp Glu Glu Ala Lys Gln Phe Arg Glu Ser Val Val Glu Trp Leu Met
85 90 95

Thr Asn His Pro His Asp Cys Pro Val Cys Glu Glu Gly Gly Asn Cys
100 105 110

His Leu Gln Asp Met Thr Val Met Thr Gly His Ser Phe Arg Arg Tyr
115 120 125

Arg Phe Thr Lys Arg Thr His Arg Asn Gln Asp Leu Gly Pro Phe Ile
130 135 140

Ser His Glu Met Asn Arg Cys Ile Ala Cys Tyr Arg Cys Val Arg Tyr
145 150 155 160

Tyr Lys Asp Tyr Ala Asp Gly Thr Asp Leu Gly Val Tyr Gly Ala His
165 170 175

Asp Asn Val Tyr Phe Gly Arg Pro Glu Asp Gly Thr Leu Glu Ser Glu
180 185 190

Phe Ser Gly Asn Leu Val Glu Ile Cys Pro Thr Gly Val Phe Thr Asp
195 200 205

Lys Thr His Ser Glu Arg Tyr Asn Arg Lys Trp Asp Met Gln Phe Ala
210 215 220

Pro Ser Ile Cys Gln Gln Cys Ser Ile Gly Cys Asn Ile Ser Pro Gly
225 230 235 240

Glu Arg Tyr Gly Glu Leu Arg Arg Ile Glu Asn Arg Tyr Asn Gly Thr

245 250 255

Val Asn His Tyr Phe Leu Cys Asp Arg Gly Arg Phe Gly Tyr Gly Tyr
260 265 270

Val Asn Leu Lys Asp Arg Pro Arg Gln Pro Val Gln Arg Arg Gly Asp
275 280 285

Asp Phe Ile Thr Leu Asn Ala Glu Gln Ala Met Gln Gly Ala Ala Asp
290 295 300

Ile Leu Arg Gln Ser Lys Lys Val Ile Gly Ile Gly Ser Pro Arg Ala
305 310 315 320

Ser Val Glu Ser Asn Phe Ala Leu Arg Glu Leu Val Gly Glu Glu Asn
325 330 335

Phe Tyr Thr Gly Ile Ala His Gly Glu Gln Glu Arg Leu Gln Leu Ala
340 345 350

Leu Lys Val Leu Arg Glu Gly Gly Ile Tyr Thr Pro Ala Leu Arg Glu
355 360 365

Ile Glu Ser Tyr Asp Ala Val Leu Val Leu Gly Glu Asp Val Thr Gln
370 375 380

Thr Gly Ala Arg Val Ala Leu Ala Val Arg Gln Ala Val Lys Gly Lys
385 390 395 400

Ala Arg Glu Met Ala Ala Ala Gln Lys Val Ala Asp Trp Gln Ile Ala
405 410 415

Ala Ile Leu Asn Ile Gly Gln Arg Ala Lys His Pro Leu Phe Val Thr
420 425 430

Asn Val Asp Asp Thr Arg Leu Asp Asp Ile Ala Ala Trp Thr Tyr Arg
435 440 445

Ala Pro Val Glu Asp Gln Ala Arg Leu Gly Phe Ala Ile Ala His Ala
450 455 460

Leu Asp Asn Ser Ala Pro Ala Val Asp Gly Ile Glu Pro Glu Leu Gln
465 470 475 480

Ser Lys Ile Asp Val Ile Val Gln Ala Leu Ala Gly Ala Lys Lys Pro
485 490 495

```
Leu Ile Ile Ser Gly Thr Asn Ala Gly Ser Leu Glu Val Ile Gln Ala
            500                 505                 510

Ala Ala Asn Val Ala Lys Ala Leu Lys Gly Arg Gly Ala Asp Val Gly
        515                 520                 525

Ile Thr Met Ile Ala Arg Ser Val Asn Ser Met Gly Leu Gly Ile Met
    530                 535                 540

Gly Gly Gly Ser Leu Glu Glu Ala Leu Thr Glu Leu Glu Thr Gly Arg
545                 550                 555                 560

Ala Asp Ala Val Val Val Leu Glu Asn Asp Leu His Arg His Ala Ser
                565                 570                 575

Ala Ile Arg Val Asn Ala Ala Leu Ala Lys Ala Pro Leu Val Met Val
            580                 585                 590

Val Asp His Gln Arg Thr Ala Ile Met Glu Asn Ala His Leu Val Leu
        595                 600                 605

Ser Ala Ala Ser Phe Ala Glu Ser Asp Gly Thr Val Ile Asn Asn Glu
    610                 615                 620

Gly Arg Ala Gln Arg Phe Phe Gln Val Tyr Asp Pro Ala Tyr Tyr Asp
625                 630                 635                 640

Ser Lys Thr Val Met Leu Glu Ser Trp Arg Trp Leu His Ser Leu His
                645                 650                 655

Ser Thr Leu Leu Ser Arg Glu Val Asp Trp Thr Gln Leu Asp His Val
            660                 665                 670

Ile Asp Ala Val Val Ala Lys Ile Pro Glu Leu Ala Gly Ile Lys Asp
        675                 680                 685

Ala Ala Pro Asp Ala Thr Phe Arg Ile Arg Gly Gln Lys Leu Ala Arg
    690                 695                 700

Glu Pro His Arg Tyr Ser Gly Arg Thr Ala Met Arg Ala Asn Ile Ser
705                 710                 715                 720

Val His Glu Pro Arg Gln Pro Gln Asp Ile Asp Thr Met Phe Thr Phe
                725                 730                 735

Ser Met Glu Gly Asn Asn Gln Pro Thr Ala His Arg Ser Gln Val Pro
            740                 745                 750
```

Phe Ala Trp Ala Pro Gly Trp Asn Ser Pro Gln Ala Trp Asn Lys Phe
755 760 765

Gln Asp Glu Val Gly Gly Lys Leu Arg Phe Gly Asp Pro Gly Val Arg
770 775 780

Leu Phe Glu Thr Ser Glu Asn Gly Leu Asp Tyr Phe Thr Ser Val Pro
785 790 795 800

Ala Arg Phe Gln Pro Gln Asp Gly Lys Trp Arg Ile Ala Pro Tyr Tyr
805 810 815

His Leu Phe Gly Ser Asp Glu Leu Ser Gln Arg Ala Pro Val Phe Gln
820 825 830

Ser Arg Met Pro Gln Pro Tyr Ile Lys Leu Asn Pro Ala Asp Ala Ala
835 840 845

Lys Leu Gly Val Asn Ala Gly Thr Arg Val Ser Phe Ser Tyr Asp Gly
850 855 860

Asn Thr Val Thr Leu Pro Val Glu Ile Ala Glu Gly Leu Thr Ala Gly
865 870 875 880

Gln Val Gly Leu Pro Met Gly Met Ser Gly Ile Ala Pro Val Leu Ala
885 890 895

Gly Ala His Leu Glu Asp Leu Lys Glu Ala Gln Gln
900 905

<210> 293
<211> 978
<212> DNA
<213> Escherichia coli

<400> 293
atgagttgga tatcaccgga actgattgag atcctgctga ccatcctcaa agcggtggtg 60

atcctgctgg tggttgtcac ctgcggggca ttcatgagct ttggcgaacg tcgcctgctg 120

ggtctgttcc agaaccgtta cggacctaac cgtgttggct ggggcggttc gctccagctg 180

gttgcggaca tgatcaaaat gttctttaaa gaagactgga tcccgaaatt ctcggatcgc 240

gtcatcttta ccctggcacc gatgattgcc tttacctcgc tgctgctggc ctttgcgatt 300

gtgccagtca gtccgggttg ggtggttgcc gacctgaaca tcgggatttt gttcttcctg 360

atgatggcag gtctggcggt ttacgcggtg ctgtttgcgg gctggtcaag taacaacaaa 420

tactcgttgc tgggtgcgat gcgtgcttct gcgcagaccc tgagctacga agtgttcctc 480

gggctttcct tgatgggcgt ggtggcgcag gccggttcat tcaacatgac cgacatcgtc 540

aacagccagg cgcatgtgtg gaacgttatc ccgcaattct ttggttttat tacctttgcc 600

atcgcgggcg tggcggtatg tcaccgtcac ccgtttgacc agccggaagc cgagcaggaa 660

ctggcggatg gttaccacat tgaatattcc ggtatgaagt tcggtctgtt cttcgtgggt 720

gagtacatcg ggattgtgac catctctgca ttgatggtga cgctgttctt cggtggctgg 780

caaggcccgt tgttaccgcc attcatctgg ttcgcgctga aaaccgcgtt ctttatgatg 840

atgttcattt tgattcgtgc gtcgttaccg cgtccgcgtt atgaccaggt aatgtccttc 900

ggctggaaaa tctgcctgcc gctgacgctg atcaacttgc tggtaacggc ggctgtcatt 960

ctctggcagg cgcaataa 978

<210> 294
<211> 325
<212> PRT
<213> Escherichia coli

<400> 294

Met Ser Trp Ile Ser Pro Glu Leu Ile Glu Ile Leu Leu Thr Ile Leu
1 5 10 15

Lys Ala Val Val Ile Leu Leu Val Val Val Thr Cys Gly Ala Phe Met
20 25 30

Ser Phe Gly Glu Arg Arg Leu Leu Gly Leu Phe Gln Asn Arg Tyr Gly
35 40 45

Pro Asn Arg Val Gly Trp Gly Gly Ser Leu Gln Leu Val Ala Asp Met
50 55 60

Ile Lys Met Phe Phe Lys Glu Asp Trp Ile Pro Lys Phe Ser Asp Arg
65 70 75 80

Val Ile Phe Thr Leu Ala Pro Met Ile Ala Phe Thr Ser Leu Leu Leu
85 90 95

Ala Phe Ala Ile Val Pro Val Ser Pro Gly Trp Val Val Ala Asp Leu
100 105 110

Asn Ile Gly Ile Leu Phe Phe Leu Met Met Ala Gly Leu Ala Val Tyr
115 120 125

Ala Val Leu Phe Ala Gly Trp Ser Ser Asn Asn Lys Tyr Ser Leu Leu
130 135 140

Gly Ala Met Arg Ala Ser Ala Gln Thr Leu Ser Tyr Glu Val Phe Leu

145 150 155 160

Gly Leu Ser Leu Met Gly Val Val Ala Gln Ala Gly Ser Phe Asn Met
165 170 175

Thr Asp Ile Val Asn Ser Gln Ala His Val Trp Asn Val Ile Pro Gln
180 185 190

Phe Phe Gly Phe Ile Thr Phe Ala Ile Ala Gly Val Ala Val Cys His
195 200 205

Arg His Pro Phe Asp Gln Pro Glu Ala Glu Gln Glu Leu Ala Asp Gly
210 215 220

Tyr His Ile Glu Tyr Ser Gly Met Lys Phe Gly Leu Phe Phe Val Gly
225 230 235 240

Glu Tyr Ile Gly Ile Val Thr Ile Ser Ala Leu Met Val Thr Leu Phe
245 250 255

Phe Gly Gly Trp Gln Gly Pro Leu Leu Pro Pro Phe Ile Trp Phe Ala
260 265 270

Leu Lys Thr Ala Phe Phe Met Met Met Phe Ile Leu Ile Arg Ala Ser
275 280 285

Leu Pro Arg Pro Arg Tyr Asp Gln Val Met Ser Phe Gly Trp Lys Ile
290 295 300

Cys Leu Pro Leu Thr Leu Ile Asn Leu Leu Val Thr Ala Ala Val Ile
305 310 315 320

Leu Trp Gln Ala Gln
325

<210> 295
<211> 543
<212> DNA
<213> Escherichia coli

<400> 295
atgaccttaa aagaattgtt agtaggtttc ggcacccagg ttcgtagtat ctggatgatc 60

ggcctgcacg cgttcgccaa acgcgaaacg cgaatgtacc cggaagagcc ggtctatctg 120

ccgccccgtt atcgtggtcg tatcgttctg acccgcgacc cggacggcga agagcgttgc 180

gtagcctgta acctctgcgc ggtagcctgc ccggtcggct gtatctcgct gcaaaaagca 240

gaaaccaaag acggtcgctg gtacccggaa tttttccgca tcaacttctc acgctgcatt 300

ttctgtggtc tgtgcgaaga agcctgtccg accacggcga ttcagttaac cccggatttc 360

gaaatggggg aatacaagcg ccaggatctg gtttacgaga aagaggatct gctgatctcc 420

ggtccgggca aatacccgga atataacttc taccggatgg caggtatggc aatcgacggc 480

aaagataagg gcgaagcaga gaacgaagcc aagcctatcg acgtcaagag cctgttaccg 540

taa 543

<210> 296
<211> 180
<212> PRT
<213> Escherichia coli

<400> 296

Met Thr Leu Lys Glu Leu Leu Val Gly Phe Gly Thr Gln Val Arg Ser
1 5 10 15

Ile Trp Met Ile Gly Leu His Ala Phe Ala Lys Arg Glu Thr Arg Met
20 25 30

Tyr Pro Glu Glu Pro Val Tyr Leu Pro Pro Arg Tyr Arg Gly Arg Ile
35 40 45

Val Leu Thr Arg Asp Pro Asp Gly Glu Glu Arg Cys Val Ala Cys Asn
50 55 60

Leu Cys Ala Val Ala Cys Pro Val Gly Cys Ile Ser Leu Gln Lys Ala
65 70 75 80

Glu Thr Lys Asp Gly Arg Trp Tyr Pro Glu Phe Phe Arg Ile Asn Phe
85 90 95

Ser Arg Cys Ile Phe Cys Gly Leu Cys Glu Glu Ala Cys Pro Thr Thr
100 105 110

Ala Ile Gln Leu Thr Pro Asp Phe Glu Met Gly Glu Tyr Lys Arg Gln
115 120 125

Asp Leu Val Tyr Glu Lys Glu Asp Leu Leu Ile Ser Gly Pro Gly Lys
130 135 140

Tyr Pro Glu Tyr Asn Phe Tyr Arg Met Ala Gly Met Ala Ile Asp Gly
145 150 155 160

Lys Asp Lys Gly Glu Ala Glu Asn Glu Ala Lys Pro Ile Asp Val Lys
165 170 175

Ser Leu Leu Pro

180

<210> 297
<211> 555
<212> DNA
<213> Escherichia coli

<400> 297
atggagttcg ctttttatat ctgtggcctg atagccatac ttgcgacctt gcgagtgatc 60

acccatacca atccggtaca cgcactgctg tacctgatta tttcgctgct ggcgatttcc 120

ggggtgttct tctcactggg cgcttacttc gccggtgcgc tggaaattat cgtctacgcg 180

ggtgccatta tggtgctgtt cgtgttcgtg gtgatgatgc tcaacctggg cggttcagaa 240

atcgaacagg aacgccagtg gctgaaaccg caggtgtgga ttggtccggc aattttgtcg 300

gccatcatgc tggtggtgat tgtttacgcc atcctcggtg ttaacgatca gggtatcgac 360

ggtacgccaa tcagtgctaa agcagtgggt attacgctgt tcgggcctta cgtactggcg 420

gtggaactgg cttctatgct gctgctcgca ggtctggttg tggccttcca cgtcggtcgt 480

gaagagcgtg cgggtgaagt gctgagcaat cgtaaagacg acagcgcgaa aagaaaaacg 540

gaggagcacg catga 555

<210> 298
<211> 184
<212> PRT
<213> Escherichia coli

<400> 298

Met Glu Phe Ala Phe Tyr Ile Cys Gly Leu Ile Ala Ile Leu Ala Thr
1 5 10 15

Leu Arg Val Ile Thr His Thr Asn Pro Val His Ala Leu Leu Tyr Leu
20 25 30

Ile Ile Ser Leu Leu Ala Ile Ser Gly Val Phe Phe Ser Leu Gly Ala
35 40 45

Tyr Phe Ala Gly Ala Leu Glu Ile Ile Val Tyr Ala Gly Ala Ile Met
50 55 60

Val Leu Phe Val Phe Val Val Met Met Leu Asn Leu Gly Gly Ser Glu
65 70 75 80

Ile Glu Gln Glu Arg Gln Trp Leu Lys Pro Gln Val Trp Ile Gly Pro
85 90 95

Ala Ile Leu Ser Ala Ile Met Leu Val Val Ile Val Tyr Ala Ile Leu
100 105 110

Gly Val Asn Asp Gln Gly Ile Asp Gly Thr Pro Ile Ser Ala Lys Ala
115 120 125

Val Gly Ile Thr Leu Phe Gly Pro Tyr Val Leu Ala Val Glu Leu Ala
130 135 140

Ser Met Leu Leu Leu Ala Gly Leu Val Val Ala Phe His Val Gly Arg
145 150 155 160

Glu Glu Arg Ala Gly Glu Val Leu Ser Asn Arg Lys Asp Asp Ser Ala
165 170 175

Lys Arg Lys Thr Glu Glu His Ala
180

<210> 299
<211> 303
<212> DNA
<213> Escherichia coli

<400> 299
atgatcccct tacaacatgg actgatcctc gcggcaatct tattcgttct tggcttaacc 60

ggtctggtta tccgtcgcaa tctgctgttt atgttgattg gtctggaaat catgattaac 120

gcctccgcgc tggccttcgt ggtcgccgga agctactggg gccagaccga cggtcaggtg 180

atgtacattc tcgccatcag cctcgcggcg gcagaagcga gtatcggcct tgcgctgctg 240

ctgcaacttc accgtcgtcg ccagaacctg aacatcgatt cagtaagtga gatgcgcgga 300

tga 303

<210> 300
<211> 100
<212> PRT
<213> Escherichia coli

<400> 300

Met Ile Pro Leu Gln His Gly Leu Ile Leu Ala Ala Ile Leu Phe Val
1 5 10 15

Leu Gly Leu Thr Gly Leu Val Ile Arg Arg Asn Leu Leu Phe Met Leu
20 25 30

Ile Gly Leu Glu Ile Met Ile Asn Ala Ser Ala Leu Ala Phe Val Val
35 40 45

Ala Gly Ser Tyr Trp Gly Gln Thr Asp Gly Gln Val Met Tyr Ile Leu
50 55 60

Ala Ile Ser Leu Ala Ala Ala Glu Ala Ser Ile Gly Leu Ala Leu Leu
65 70 75 80

Leu Gln Leu His Arg Arg Arg Gln Asn Leu Asn Ile Asp Ser Val Ser
85 90 95

Glu Met Arg Gly
100

<210> 301
<211> 1842
<212> DNA
<213> Escherichia coli

<400> 301
atgaacatgc ttgccttaac cattattttg ccattgattg gcttcgtcct gctggcattc 60

tcccgtgggc gctggtctga aaacgtctcg gcgatcgtcg gcgtaggctc tgtgggcctg 120

gcggcgctgg taaccgcctt tatcggcgtt gatttcttcg ctaacggcga gcagacatac 180

agccagccgc tgtggacgtg gatgtcggta ggcgacttta acatcggttt taacctggtg 240

ctggacggcc tgtcgctgac catgctctcg gtggtcactg gtgtgggttt ccttattcac 300

atgtacgcct cctggtatat gcgcggtgaa gagggctact ctcgcttctt cgcttacacc 360

aacctgttca tcgccagcat ggtggttctg gtgcttgccg acaacctgct gctgatgtac 420

ctcggctggg aaggcgtggg cctgtgctcc tatctgctga tcgggttcta ttacaccgat 480

ccgaagaatg gcgcagcggc aatgaaagcg ttcgtcgtga cccgtgtggg tgacgtgttc 540

ctcgctttcg cactgttcat tctttacaac gaactgggca ccctgaactt ccgcgaaatg 600

gtggaactgg caccagcgca ctttgctgac ggcaataaca tgctgatgtg ggcgacgctg 660

atgctgctgg gcggtgcggt cggtaaatct gcgcagttgc cgttgcagac atggcttgcc 720

gacgcgatgg cgggcccgac gcctgtctcc gcgctgatcc acgccgcaac catggtaacc 780

gcgggtgtct acctgatcgc ccgtacccac ggcctgttcc tgatgacgcc ggaagttctg 840

catctggtgg gtattgtcgg ggcggttacg ctgctgctgg ccggttttgc cgcgctggta 900

cagaccgaca tcaaacgtgt tctcgcttac tctaccatga gccagattgg ctacatgttc 960

ctcgcgcttg gcgtgcaggc atgggatgcg gcgattttcc acttgatgac ccacgcgttc 1020

tttaaagcgc tgctgttcct ggcatccggt tccgtcattc tggcctgcca tcacgaacag 1080

aacatcttca agatgggcgg tctgcgtaaa tctattccgc tggtttatct ctgcttcctg 1140

gtgggcggcg cagcactgtc ggcactaccg ctggtcactg cgggcttctt cagtaaggat 1200

gagatcctcg cgggtgcgat ggcgaatggt catatcaatc tgatggtggc aggtctggtc 1260

ggtgcgttta tgacctcgct ctacaccttc cgtatgattt tcatcgtctt ccacggaaaa 1320

gaacaaattc acgctcacgc cgtgaaaggg gtaactcaca gcctgccgct gattgtgctg 1380

ctgatccttt ccaccttcgt tggcgcactg attgtaccgc cgctgcaggg cgtgcttccg 1440

caaacgacgg aactggcgca cggcagcatg ttgaccctgg aaattacctc tggcgtggtc 1500

gcggtggtcg gcattctgct ggcagcctgg ctgtggctgg gtaaacgtac tctggtgacc 1560

tccatcgcca acagtgcgcc gggccgtctg ctgggcacct ggtggtacaa cgcctgggga 1620

tttgactggc tgtatgacaa agtgttcgtc aagccgttcc tgggtattgc ctggttgctg 1680

aaacgcgatc cgctgaactc aatgatgaac atcccggctg tcctttcccg ctttgcaggt 1740

aaaggtctgc tgttaagtga gaacggctat ctgcgctggt atgtggcatc catgagcatc 1800

ggtgcggtcg tggtgctggc actgttgatg gtactgcgtt ga 1842

<210> 302
<211> 613
<212> PRT
<213> Escherichia coli

<400> 302

Met Asn Met Leu Ala Leu Thr Ile Ile Leu Pro Leu Ile Gly Phe Val
1 5 10 15

Leu Leu Ala Phe Ser Arg Gly Arg Trp Ser Glu Asn Val Ser Ala Ile
20 25 30

Val Gly Val Gly Ser Val Gly Leu Ala Ala Leu Val Thr Ala Phe Ile
35 40 45

Gly Val Asp Phe Phe Ala Asn Gly Glu Gln Thr Tyr Ser Gln Pro Leu
50 55 60

Trp Thr Trp Met Ser Val Gly Asp Phe Asn Ile Gly Phe Asn Leu Val
65 70 75 80

Leu Asp Gly Leu Ser Leu Thr Met Leu Ser Val Val Thr Gly Val Gly
85 90 95

Phe Leu Ile His Met Tyr Ala Ser Trp Tyr Met Arg Gly Glu Glu Gly
100 105 110

Tyr Ser Arg Phe Phe Ala Tyr Thr Asn Leu Phe Ile Ala Ser Met Val
115 120 125

Val Leu Val Leu Ala Asp Asn Leu Leu Leu Met Tyr Leu Gly Trp Glu
130 135 140

Gly Val Gly Leu Cys Ser Tyr Leu Leu Ile Gly Phe Tyr Tyr Thr Asp

145 150 155 160

Pro Lys Asn Gly Ala Ala Ala Met Lys Ala Phe Val Val Thr Arg Val
165 170 175

Gly Asp Val Phe Leu Ala Phe Ala Leu Phe Ile Leu Tyr Asn Glu Leu
180 185 190

Gly Thr Leu Asn Phe Arg Glu Met Val Glu Leu Ala Pro Ala His Phe
195 200 205

Ala Asp Gly Asn Asn Met Leu Met Trp Ala Thr Leu Met Leu Leu Gly
210 215 220

Gly Ala Val Gly Lys Ser Ala Gln Leu Pro Leu Gln Thr Trp Leu Ala
225 230 235 240

Asp Ala Met Ala Gly Pro Thr Pro Val Ser Ala Leu Ile His Ala Ala
245 250 255

Thr Met Val Thr Ala Gly Val Tyr Leu Ile Ala Arg Thr His Gly Leu
260 265 270

Phe Leu Met Thr Pro Glu Val Leu His Leu Val Gly Ile Val Gly Ala
275 280 285

Val Thr Leu Leu Leu Ala Gly Phe Ala Ala Leu Val Gln Thr Asp Ile
290 295 300

Lys Arg Val Leu Ala Tyr Ser Thr Met Ser Gln Ile Gly Tyr Met Phe
305 310 315 320

Leu Ala Leu Gly Val Gln Ala Trp Asp Ala Ala Ile Phe His Leu Met
325 330 335

Thr His Ala Phe Phe Lys Ala Leu Leu Phe Leu Ala Ser Gly Ser Val
340 345 350

Ile Leu Ala Cys His His Glu Gln Asn Ile Phe Lys Met Gly Gly Leu
355 360 365

Arg Lys Ser Ile Pro Leu Val Tyr Leu Cys Phe Leu Val Gly Gly Ala
370 375 380

Ala Leu Ser Ala Leu Pro Leu Val Thr Ala Gly Phe Phe Ser Lys Asp
385 390 395 400

Glu Ile Leu Ala Gly Ala Met Ala Asn Gly His Ile Asn Leu Met Val
405 410 415

Ala Gly Leu Val Gly Ala Phe Met Thr Ser Leu Tyr Thr Phe Arg Met
420 425 430

Ile Phe Ile Val Phe His Gly Lys Glu Gln Ile His Ala His Ala Val
435 440 445

Lys Gly Val Thr His Ser Leu Pro Leu Ile Val Leu Leu Ile Leu Ser
450 455 460

Thr Phe Val Gly Ala Leu Ile Val Pro Pro Leu Gln Gly Val Leu Pro
465 470 475 480

Gln Thr Thr Glu Leu Ala His Gly Ser Met Leu Thr Leu Glu Ile Thr
485 490 495

Ser Gly Val Val Ala Val Val Gly Ile Leu Leu Ala Ala Trp Leu Trp
500 505 510

Leu Gly Lys Arg Thr Leu Val Thr Ser Ile Ala Asn Ser Ala Pro Gly
515 520 525

Arg Leu Leu Gly Thr Trp Trp Tyr Asn Ala Trp Gly Phe Asp Trp Leu
530 535 540

Tyr Asp Lys Val Phe Val Lys Pro Phe Leu Gly Ile Ala Trp Leu Leu
545 550 555 560

Lys Arg Asp Pro Leu Asn Ser Met Met Asn Ile Pro Ala Val Leu Ser
565 570 575

Arg Phe Ala Gly Lys Gly Leu Leu Leu Ser Glu Asn Gly Tyr Leu Arg
580 585 590

Trp Tyr Val Ala Ser Met Ser Ile Gly Ala Val Val Val Leu Ala Leu
595 600 605

Leu Met Val Leu Arg
610

<210> 303
<211> 1530
<212> DNA
<213> Escherichia coli

<400> 303
atgttactac cctggctaat attaattccc tttattggcg gcttcctgtg ctggcagacc 60

gaacgctttg gcgtcaaggt gccacgctgg atcgcgctga tcaccatggg attgacgctg 120

gcgctgtcgc tgcaactgtg gttgcagggc ggttattcac tgacgcaatc cgccggaatt 180

ccgcagtggc agtctgaatt cgacatgccg tggatcccgc gttttggtat ctctattcat 240

ctcgccattg acgggctgtc gctgctgatg gtcgtgctga ccggtctgct cggtgtgctg 300

gcggtactat gttcgtggaa agagatcgaa aaatatcagg gcttcttcca cctcaacctg 360

atgtggatcc tgggcggcgt tatcggcgtg ttccttgcca tcgacatgtt cctgttcttc 420

ttcttctggg aaatgatgct ggtgccgatg tacttcctga tcgcactgtg ggggcataaa 480

gcctctgacg gtaaaacgcg tatcacggcg gcaaccaagt tcttcattta cacccaggcg 540

agtggcctgg tgatgttgat tgccatcctg gcgctggttt ttgttcacta caatgcgacc 600

ggcgtctgga ccttcaacta tgaagagctg ctgaatacgc caatgtccag tggtgtggaa 660

tacctgttaa tgctgggctt cttcatcgcc ttcgcagtca aaatgccggt ggttccgctg 720

catggctggc tgccggatgc gcactcccag gctccgaccg ccggttccgt tgacctcgcg 780

gggatcttgc tgaaaactgc cgcttacggt ttgctgcgtt tctccctgcc gctgttcccg 840

aacgcgtcgg cagagttcgc gccaattgct atgtggctgg gtgttatcgg catcttctac 900

ggtgcgtgga tggccttcgc ccagaccgat atcaaacgtc tgatcgccta cacctcggtt 960

tcccacatgg gcttcgtgct gattgctatc tacaccggca gccagttggc ctaccagggc 1020

gcggtaatcc agatgattgc gcacggtttg tcggcggcgg gtctgtttat tctttgtggt 1080

cagctttatg aacgtatcca tacccgcgac atgcgcatga tgggcggtct gtggagcaag 1140

atgaaatggc tgccagcact gtcgctgttc tttgcggtgg caacgcttgg gatgcctggc 1200

accggtaact tcgtcggcga atttatgatt ctgttcggca gcttccaggt tgtaccggtg 1260

attaccgtta tctctacctt tgggctggtc tttgcatctg tttattcgct ggcgatgtta 1320

catcgcgctt acttcggtaa agcgaaaagc cagattgcca gccaggaact gccagggatg 1380

tcgctgcgtg agctgtttat gatcctgttg ctggtggtgc tgctggtact gctgggcttc 1440

tatccgcagc cgattctgga tacctcgcac tccgcgattg gcaatatcca gcagtggttt 1500

gttaattccg ttactactac aaggccgtaa 1530

<210> 304
<211> 509
<212> PRT
<213> Escherichia coli

<400> 304

Met Leu Leu Pro Trp Leu Ile Leu Ile Pro Phe Ile Gly Gly Phe Leu
1 5 10 15

Cys Trp Gln Thr Glu Arg Phe Gly Val Lys Val Pro Arg Trp Ile Ala
20 25 30

Leu Ile Thr Met Gly Leu Thr Leu Ala Leu Ser Leu Gln Leu Trp Leu
35 40 45

Gln Gly Gly Tyr Ser Leu Thr Gln Ser Ala Gly Ile Pro Gln Trp Gln
50 55 60

Ser Glu Phe Asp Met Pro Trp Ile Pro Arg Phe Gly Ile Ser Ile His
65 70 75 80

Leu Ala Ile Asp Gly Leu Ser Leu Leu Met Val Val Leu Thr Gly Leu
85 90 95

Leu Gly Val Leu Ala Val Leu Cys Ser Trp Lys Glu Ile Glu Lys Tyr
100 105 110

Gln Gly Phe Phe His Leu Asn Leu Met Trp Ile Leu Gly Gly Val Ile
115 120 125

Gly Val Phe Leu Ala Ile Asp Met Phe Leu Phe Phe Phe Phe Trp Glu
130 135 140

Met Met Leu Val Pro Met Tyr Phe Leu Ile Ala Leu Trp Gly His Lys
145 150 155 160

Ala Ser Asp Gly Lys Thr Arg Ile Thr Ala Ala Thr Lys Phe Phe Ile
165 170 175

Tyr Thr Gln Ala Ser Gly Leu Val Met Leu Ile Ala Ile Leu Ala Leu
180 185 190

Val Phe Val His Tyr Asn Ala Thr Gly Val Trp Thr Phe Asn Tyr Glu
195 200 205

Glu Leu Leu Asn Thr Pro Met Ser Ser Gly Val Glu Tyr Leu Leu Met
210 215 220

Leu Gly Phe Phe Ile Ala Phe Ala Val Lys Met Pro Val Val Pro Leu
225 230 235 240

His Gly Trp Leu Pro Asp Ala His Ser Gln Ala Pro Thr Ala Gly Ser
245 250 255

Val Asp Leu Ala Gly Ile Leu Leu Lys Thr Ala Ala Tyr Gly Leu Leu
260 265 270

Arg Phe Ser Leu Pro Leu Phe Pro Asn Ala Ser Ala Glu Phe Ala Pro
275 280 285

Ile Ala Met Trp Leu Gly Val Ile Gly Ile Phe Tyr Gly Ala Trp Met
290 295 300

Ala Phe Ala Gln Thr Asp Ile Lys Arg Leu Ile Ala Tyr Thr Ser Val
305 310 315 320

Ser His Met Gly Phe Val Leu Ile Ala Ile Tyr Thr Gly Ser Gln Leu
325 330 335

Ala Tyr Gln Gly Ala Val Ile Gln Met Ile Ala His Gly Leu Ser Ala
340 345 350

Ala Gly Leu Phe Ile Leu Cys Gly Gln Leu Tyr Glu Arg Ile His Thr
355 360 365

Arg Asp Met Arg Met Met Gly Gly Leu Trp Ser Lys Met Lys Trp Leu
370 375 380

Pro Ala Leu Ser Leu Phe Phe Ala Val Ala Thr Leu Gly Met Pro Gly
385 390 395 400

Thr Gly Asn Phe Val Gly Glu Phe Met Ile Leu Phe Gly Ser Phe Gln
405 410 415

Val Val Pro Val Ile Thr Val Ile Ser Thr Phe Gly Leu Val Phe Ala
420 425 430

Ser Val Tyr Ser Leu Ala Met Leu His Arg Ala Tyr Phe Gly Lys Ala
435 440 445

Lys Ser Gln Ile Ala Ser Gln Glu Leu Pro Gly Met Ser Leu Arg Glu
450 455 460

Leu Phe Met Ile Leu Leu Leu Val Val Leu Leu Val Leu Leu Gly Phe
465 470 475 480

Tyr Pro Gln Pro Ile Leu Asp Thr Ser His Ser Ala Ile Gly Asn Ile
485 490 495

Gln Gln Trp Phe Val Asn Ser Val Thr Thr Thr Arg Pro
500 505

<210> 305
<211> 1458

<212> DNA
<213> Escherichia coli

<400> 305
atgacaataa ctccacaaaa cctgatcgca ctgctaccgt tgctgatcgt cggcttgacg 60

gtggtggttg tgatgctctc cattgcgtgg cgacgcaatc atttcctcaa cgctacgctc 120

tcggttattg ggcttaacgc ggcgctggtt tcgctctggt ttgttggcca ggcgggcgct 180

atggacgtta cgccgctgat gcgcgttgat ggtttcgcca tgctttacac cgggctggta 240

ttgttggcga gcctcgccac ctgtactttc gcctacccgt ggcttgaagg ctataacgac 300

aacaaggatg agttctacct gttggtgtta attgccgcgc tgggcgggat cctgctggcg 360

aatgccaacc atctggcgtc tctgttcctc ggtatcgaac tgatctcttt gccgctgttt 420

ggcctggtcg gttacgcttt ccgccagaaa cgttcactgg aagccagtat caaatacacc 480

atcctttctg ccgcagcgtc ttctttcctg ctgtttggta tggcgctggt gtatgcgcag 540

tctggcgacc tgtcgtttgt cgcgttgggt aaaaaccttg gcgacggtat gctcaacgag 600

ccgctgttgc tggcaggttt cggcctgatg attgttggcc tcggcttcaa actctctctg 660

gtgccgttcc acctgtggac gccagacgta taccagggcg cgcctgcgcc ggtttccact 720

ttcctggcga cggcgagcaa aatcgctatc ttcggtgtgg tgatgcgtct gttcctctac 780

gcaccggtgg gtgacagcga agcgattcgc gtggtgctgg cgattatcgc ctttgcctcc 840

atcatcttcg gtaacctgat ggcgctgagc cagaccaata tcaaacgtct gctcggttac 900

tcatctatct ctcacctcgg ctatctgctg gtagcgctga ttgcgctgca aaccggcgag 960

atgtcgatgg aagcggtagg ggtttacctg gccggttatc tgttcagcag cctcggcgcg 1020

ttcggcgtgg tcagcctgat gtccagcccg tatcgtggcc cggatgctga ttccctgttc 1080

tcttaccgcg gtctgttctg gcatcgtccg atcctcgcgg cagtgatgac ggtgatgatg 1140

ctgtctctgg ccggtatccc gatgacgctg ggctttatcg gtaagttcta cgtgctggcg 1200

gtcggtgtcc aggcacactt gtggtggctg gtgggtgccg tggttgtcgg ttcggcaatc 1260

ggcctctact actacctgcg cgtggcggtg agcctgtatc ttcacgcccc ggaacaaccg 1320

ggtcgcgatg caccatcaaa ctggcagtac agcgcgggcg gtatcgttgt gctgatctct 1380

gcactgttgg tactggtgct gggtgtatgg ccacaaccgc tgattagcat tgtgcgtttg 1440

gcaatgccgc tgatgtaa 1458

<210> 306
<211> 485
<212> PRT
<213> Escherichia coli

<400> 306

Met Thr Ile Thr Pro Gln Asn Leu Ile Ala Leu Leu Pro Leu Leu Ile

1 5 10 15

Val Gly Leu Thr Val Val Val Val Met Leu Ser Ile Ala Trp Arg Arg
20 25 30

Asn His Phe Leu Asn Ala Thr Leu Ser Val Ile Gly Leu Asn Ala Ala
35 40 45

Leu Val Ser Leu Trp Phe Val Gly Gln Ala Gly Ala Met Asp Val Thr
50 55 60

Pro Leu Met Arg Val Asp Gly Phe Ala Met Leu Tyr Thr Gly Leu Val
65 70 75 80

Leu Leu Ala Ser Leu Ala Thr Cys Thr Phe Ala Tyr Pro Trp Leu Glu
85 90 95

Gly Tyr Asn Asp Asn Lys Asp Glu Phe Tyr Leu Leu Val Leu Ile Ala
100 105 110

Ala Leu Gly Gly Ile Leu Leu Ala Asn Ala Asn His Leu Ala Ser Leu
115 120 125

Phe Leu Gly Ile Glu Leu Ile Ser Leu Pro Leu Phe Gly Leu Val Gly
130 135 140

Tyr Ala Phe Arg Gln Lys Arg Ser Leu Glu Ala Ser Ile Lys Tyr Thr
145 150 155 160

Ile Leu Ser Ala Ala Ala Ser Ser Phe Leu Leu Phe Gly Met Ala Leu
165 170 175

Val Tyr Ala Gln Ser Gly Asp Leu Ser Phe Val Ala Leu Gly Lys Asn
180 185 190

Leu Gly Asp Gly Met Leu Asn Glu Pro Leu Leu Leu Ala Gly Phe Gly
195 200 205

Leu Met Ile Val Gly Leu Gly Phe Lys Leu Ser Leu Val Pro Phe His
210 215 220

Leu Trp Thr Pro Asp Val Tyr Gln Gly Ala Pro Ala Pro Val Ser Thr
225 230 235 240

Phe Leu Ala Thr Ala Ser Lys Ile Ala Ile Phe Gly Val Val Met Arg
245 250 255

```
Leu Phe Leu Tyr Ala Pro Val Gly Asp Ser Glu Ala Ile Arg Val Val
            260                 265                 270

Leu Ala Ile Ile Ala Phe Ala Ser Ile Ile Phe Gly Asn Leu Met Ala
        275                 280                 285

Leu Ser Gln Thr Asn Ile Lys Arg Leu Leu Gly Tyr Ser Ser Ile Ser
    290                 295                 300

His Leu Gly Tyr Leu Leu Val Ala Leu Ile Ala Leu Gln Thr Gly Glu
305                 310                 315                 320

Met Ser Met Glu Ala Val Gly Val Tyr Leu Ala Gly Tyr Leu Phe Ser
                325                 330                 335

Ser Leu Gly Ala Phe Gly Val Val Ser Leu Met Ser Ser Pro Tyr Arg
            340                 345                 350

Gly Pro Asp Ala Asp Ser Leu Phe Ser Tyr Arg Gly Leu Phe Trp His
        355                 360                 365

Arg Pro Ile Leu Ala Ala Val Met Thr Val Met Met Leu Ser Leu Ala
    370                 375                 380

Gly Ile Pro Met Thr Leu Gly Phe Ile Gly Lys Phe Tyr Val Leu Ala
385                 390                 395                 400

Val Gly Val Gln Ala His Leu Trp Trp Leu Val Gly Ala Val Val Val
                405                 410                 415

Gly Ser Ala Ile Gly Leu Tyr Tyr Tyr Leu Arg Val Ala Val Ser Leu
            420                 425                 430

Tyr Leu His Ala Pro Glu Gln Pro Gly Arg Asp Ala Pro Ser Asn Trp
        435                 440                 445

Gln Tyr Ser Ala Gly Gly Ile Val Val Leu Ile Ser Ala Leu Leu Val
    450                 455                 460

Leu Val Leu Gly Val Trp Pro Gln Pro Leu Ile Ser Ile Val Arg Leu
465                 470                 475                 480

Ala Met Pro Leu Met
                485
```

<210> 307
<211> 930
<212> DNA

<213> Escherichia coli

<400> 307
atgaccacga agaaagctga ttacatttgg ttcaatgggg agatggttcg ctgggaagac 60
gcgaaggtgc atgtgatgtc gcacgcgctg cactatggca cttcggtttt tgaaggcatc 120
cgttgctacg actcgcacaa aggaccggtt gtattccgcc atcgtgagca tatgcagcgt 180
ctgcatgact ccgccaaaat ctatcgcttc ccggtttcgc agagcattga tgagctgatg 240
gaagcttgtc gtgacgtgat ccgcaaaaac aatctcacca gcgcctatat ccgtccgctg 300
atcttcgtcg gtgatgttgg catgggagta aacccgccag cgggatactc aaccgacgtg 360
attatcgctg ctttcccgtg gggagcgtat ctgggcgcag aagcgctgga gcaggggatc 420
gatgcgatgg tttcctcctg gaaccgcgca gcaccaaaca ccatcccgac ggcggcaaaa 480
gccggtggta actacctctc ttccctgctg gtgggtagcg aagcgcgccg ccacggttat 540
caggaaggta tcgcgctgga tgtgaacggt tatatctctg aaggcgcagg cgaaaacctg 600
tttgaagtga aagatggtgt gctgttcacc ccaccgttca cctcctccgc gctgccgggt 660
attacccgtg atgccatcat caaactggcg aaagagctgg gaattgaagt acgtgagcag 720
gtgctgtcgc gcgaatccct gtacctggcg gatgaagtgt ttatgtccgg tacggcggca 780
gaaatcacgc cagtgcgcag cgtagacggt attcaggttg gcgaaggccg ttgtggcccg 840
gttaccaaac gcattcagca agccttcttc ggcctcttca ctggcgaaac cgaagataaa 900
tggggctggt tagatcaagt taatcaataa 930

<210> 308
<211> 309
<212> PRT
<213> Escherichia coli

<400> 308

Met Thr Thr Lys Lys Ala Asp Tyr Ile Trp Phe Asn Gly Glu Met Val
1               5                   10                  15

Arg Trp Glu Asp Ala Lys Val His Val Met Ser His Ala Leu His Tyr
            20                  25                  30

Gly Thr Ser Val Phe Glu Gly Ile Arg Cys Tyr Asp Ser His Lys Gly
        35                  40                  45

Pro Val Val Phe Arg His Arg Glu His Met Gln Arg Leu His Asp Ser
    50                  55                  60

Ala Lys Ile Tyr Arg Phe Pro Val Ser Gln Ser Ile Asp Glu Leu Met
65                  70                  75                  80

```
Glu Ala Cys Arg Asp Val Ile Arg Lys Asn Asn Leu Thr Ser Ala Tyr
                85                  90                  95

Ile Arg Pro Leu Ile Phe Val Gly Asp Val Gly Met Gly Val Asn Pro
            100                 105                 110

Pro Ala Gly Tyr Ser Thr Asp Val Ile Ile Ala Ala Phe Pro Trp Gly
        115                 120                 125

Ala Tyr Leu Gly Ala Glu Ala Leu Glu Gln Gly Ile Asp Ala Met Val
    130                 135                 140

Ser Ser Trp Asn Arg Ala Ala Pro Asn Thr Ile Pro Thr Ala Ala Lys
145                 150                 155                 160

Ala Gly Gly Asn Tyr Leu Ser Ser Leu Leu Val Gly Ser Glu Ala Arg
                165                 170                 175

Arg His Gly Tyr Gln Glu Gly Ile Ala Leu Asp Val Asn Gly Tyr Ile
            180                 185                 190

Ser Glu Gly Ala Gly Glu Asn Leu Phe Glu Val Lys Asp Gly Val Leu
        195                 200                 205

Phe Thr Pro Pro Phe Thr Ser Ser Ala Leu Pro Gly Ile Thr Arg Asp
    210                 215                 220

Ala Ile Ile Lys Leu Ala Lys Glu Leu Gly Ile Glu Val Arg Glu Gln
225                 230                 235                 240

Val Leu Ser Arg Glu Ser Leu Tyr Leu Ala Asp Glu Val Phe Met Ser
                245                 250                 255

Gly Thr Ala Ala Glu Ile Thr Pro Val Arg Ser Val Asp Gly Ile Gln
            260                 265                 270

Val Gly Glu Gly Arg Cys Gly Pro Val Thr Lys Arg Ile Gln Gln Ala
        275                 280                 285

Phe Phe Gly Leu Phe Thr Gly Glu Thr Glu Asp Lys Trp Gly Trp Leu
    290                 295                 300

Asp Gln Val Asn Gln
305

<210>  309
<211>  1194
<212>  DNA
```

<213> Escherichia coli

<400> 309
gtgtttcaaa aagttgacgc ctacgctggc gacccgattc ttacgcttat ggagcgtttt 60
aaagaagacc ctcgcagcga caaagtgaat ttaagtatcg gtctgtacta caacgaagac 120
ggaattattc cacaactgca agccgtggcg gaggcggaag cgcgcctgaa tgcgcagcct 180
catggcgctt cgctttattt accgatggaa gggcttaact gctatcgcca tgccattgcg 240
ccgctgctgt ttggtgcgga ccatccggta ctgaaacaac agcgcgtagc aaccattcaa 300
acccttggcg gctccggggc attgaaagtg ggcgcggatt tcctgaaacg ctacttcccg 360
gaatcaggcg tctgggtcag cgatcctacc tgggaaaacc acgtagcaat attcgccggg 420
gctggattcg aagtgagtac ttacccctgg tatgacgaag cgactaacgg cgtgcgcttt 480
aatgacctgt tggcgacgct gaaaacatta cctgcccgca gtattgtgtt gctgcatcca 540
tgttgccaca acccaacggg tgccgatctc actaatgatc agtgggatgc ggtgattgaa 600
attctcaaag cccgcgagct tattccattc ctcgatattg cctatcaagg atttggtgcc 660
ggtatggaag aggatgccta cgctattcgc gccattgcca gcgctggatt acccgctctg 720
gtgagcaatt cgttctcgaa aattttctcc ctttacggcg agcgcgtcgg cggactttct 780
gttatgtgtg aagatgccga agccgctggc cgcgtactgg ggcaattgaa agcaacagtt 840
cgccgcaact actccagccc gccgaatttt ggtgcgcagg tggtggctgc agtgctgaat 900
gacgaggcat tgaaagccag ctggctggcg gaagtagaag agatgcgtac tcgcattctg 960
gcaatgcgtc aggaattggt gaaggtatta agcacagaga tgccagaacg caatttcgat 1020
tatctgctta atcagcgcgg catgttcagt tataccggtt taagtgccgc tcaggttgac 1080
cgactacgtg aagaatttgg tgtctatctc atcgccagcg gtcgcatgtg tgtcgccggg 1140
ttaaatacgg caaatgtaca acgtgtggca aaggcgtttg ctgcggtgat gtaa 1194

<210> 310
<211> 397
<212> PRT
<213> Escherichia coli

<400> 310

Met Phe Gln Lys Val Asp Ala Tyr Ala Gly Asp Pro Ile Leu Thr Leu
1 5 10 15

Met Glu Arg Phe Lys Glu Asp Pro Arg Ser Asp Lys Val Asn Leu Ser
20 25 30

Ile Gly Leu Tyr Tyr Asn Glu Asp Gly Ile Ile Pro Gln Leu Gln Ala
35 40 45

```
Val Ala Glu Ala Glu Ala Arg Leu Asn Ala Gln Pro His Gly Ala Ser
    50                  55                  60

Leu Tyr Leu Pro Met Glu Gly Leu Asn Cys Tyr Arg His Ala Ile Ala
65                  70                  75                  80

Pro Leu Leu Phe Gly Ala Asp His Pro Val Leu Lys Gln Gln Arg Val
                85                  90                  95

Ala Thr Ile Gln Thr Leu Gly Gly Ser Gly Ala Leu Lys Val Gly Ala
            100                 105                 110

Asp Phe Leu Lys Arg Tyr Phe Pro Glu Ser Gly Val Trp Val Ser Asp
        115                 120                 125

Pro Thr Trp Glu Asn His Val Ala Ile Phe Ala Gly Ala Gly Phe Glu
    130                 135                 140

Val Ser Thr Tyr Pro Trp Tyr Asp Glu Ala Thr Asn Gly Val Arg Phe
145                 150                 155                 160

Asn Asp Leu Leu Ala Thr Leu Lys Thr Leu Pro Ala Arg Ser Ile Val
                165                 170                 175

Leu Leu His Pro Cys Cys His Asn Pro Thr Gly Ala Asp Leu Thr Asn
            180                 185                 190

Asp Gln Trp Asp Ala Val Ile Glu Ile Leu Lys Ala Arg Glu Leu Ile
        195                 200                 205

Pro Phe Leu Asp Ile Ala Tyr Gln Gly Phe Gly Ala Gly Met Glu Glu
    210                 215                 220

Asp Ala Tyr Ala Ile Arg Ala Ile Ala Ser Ala Gly Leu Pro Ala Leu
225                 230                 235                 240

Val Ser Asn Ser Phe Ser Lys Ile Phe Ser Leu Tyr Gly Glu Arg Val
                245                 250                 255

Gly Gly Leu Ser Val Met Cys Glu Asp Ala Glu Ala Ala Gly Arg Val
            260                 265                 270

Leu Gly Gln Leu Lys Ala Thr Val Arg Arg Asn Tyr Ser Ser Pro Pro
        275                 280                 285

Asn Phe Gly Ala Gln Val Val Ala Ala Val Leu Asn Asp Glu Ala Leu
    290                 295                 300
```

Lys Ala Ser Trp Leu Ala Glu Val Glu Glu Met Arg Thr Arg Ile Leu
305 310 315 320

Ala Met Arg Gln Glu Leu Val Lys Val Leu Ser Thr Glu Met Pro Glu
325 330 335

Arg Asn Phe Asp Tyr Leu Leu Asn Gln Arg Gly Met Phe Ser Tyr Thr
340 345 350

Gly Leu Ser Ala Ala Gln Val Asp Arg Leu Arg Glu Glu Phe Gly Val
355 360 365

Tyr Leu Ile Ala Ser Gly Arg Met Cys Val Ala Gly Leu Asn Thr Ala
370 375 380

Asn Val Gln Arg Val Ala Lys Ala Phe Ala Ala Val Met
385 390 395

<210> 311
<211> 1251
<212> DNA
<213> Escherichia coli

<400> 311
atggaaagta aagtagttgt tccggcacaa ggcaagaaga tcaccctgca aaacggcaaa 60

ctcaacgttc ctgaaaatcc gattatccct tacattgaag gtgatggaat cggtgtagat 120

gtaaccccag ccatgctgaa agtggtcgac gctgcagtcg agaaagccta taaaggcgag 180

cgtaaaatct cctggatgga aatttacacc ggtgaaaaat ccacacaggt ttatggtcag 240

gacgtctggc tgcctgctga aactcttgat ctgattcgtg aatatcgcgt tgccattaaa 300

ggtccgctga ccactccggt tggtggcggt attcgctctc tgaacgttgc cctgcgccag 360

gaactggatc tctacatctg cctgcgtccg gtacgttact atcagggcac tccaagcccg 420

gttaaacacc ctgaactgac cgatatggtt atcttccgtg aaaactcgga agacatttat 480

gcgggtatcg aatggaaagc agactctgcc gacgccgaga aagtgattaa attcctgcgt 540

gaagagatgg gggtgaagaa aattcgcttc ccggaacatt gtggtatcgg tattaagccg 600

tgttcggaag aaggcaccaa acgtctggtt cgtgcagcga tcgaatacgc aattgctaac 660

gatcgtgact ctgtgactct ggtgcacaaa ggcaacatca tgaagttcac cgaaggagcg 720

tttaaagact ggggctacca gctggcgcgt gaagagtttg gcggtgaact gatcgacggt 780

ggcccgtggc tgaaagttaa aaacccgaac actggcaaag agatcgtcat taaagacgtg 840

attgctgatg cattcctgca acagatcctg ctgcgtccgg ctgaatatga tgttatcgcc 900

tgtatgaacc tgaacggtga ctacatttct gacgccctgg cagcgcaggt tggcggtatc 960

ggtatcgccc ctggtgcaaa catcggtgac gaatgcgccc tgtttgaagc cacccacggt 1020

actgcgccga aatatgccgg tcaggacaaa gtaaatcctg gctctattat tctctccgct 1080

gagatgatgc tgcgccacat gggttggacc gaagcggctg acttaattgt taaaggtatg 1140

gaaggcgcaa tcaacgcgaa aaccgtaacc tatgacttcg agcgtctgat ggatggcgct 1200

aaactgctga aatgttcaga gtttggtgac gcgatcatcg aaaacatgta a 1251

<210> 312
<211> 416
<212> PRT
<213> Escherichia coli

<400> 312

Met Glu Ser Lys Val Val Val Pro Ala Gln Gly Lys Lys Ile Thr Leu
1 5 10 15

Gln Asn Gly Lys Leu Asn Val Pro Glu Asn Pro Ile Ile Pro Tyr Ile
20 25 30

Glu Gly Asp Gly Ile Gly Val Asp Val Thr Pro Ala Met Leu Lys Val
35 40 45

Val Asp Ala Ala Val Glu Lys Ala Tyr Lys Gly Glu Arg Lys Ile Ser
50 55 60

Trp Met Glu Ile Tyr Thr Gly Glu Lys Ser Thr Gln Val Tyr Gly Gln
65 70 75 80

Asp Val Trp Leu Pro Ala Glu Thr Leu Asp Leu Ile Arg Glu Tyr Arg
85 90 95

Val Ala Ile Lys Gly Pro Leu Thr Thr Pro Val Gly Gly Gly Ile Arg
100 105 110

Ser Leu Asn Val Ala Leu Arg Gln Glu Leu Asp Leu Tyr Ile Cys Leu
115 120 125

Arg Pro Val Arg Tyr Tyr Gln Gly Thr Pro Ser Pro Val Lys His Pro
130 135 140

Glu Leu Thr Asp Met Val Ile Phe Arg Glu Asn Ser Glu Asp Ile Tyr
145 150 155 160

Ala Gly Ile Glu Trp Lys Ala Asp Ser Ala Asp Ala Glu Lys Val Ile
165 170 175

Lys Phe Leu Arg Glu Glu Met Gly Val Lys Lys Ile Arg Phe Pro Glu

180 185 190

His Cys Gly Ile Gly Ile Lys Pro Cys Ser Glu Glu Gly Thr Lys Arg
195 200 205

Leu Val Arg Ala Ala Ile Glu Tyr Ala Ile Ala Asn Asp Arg Asp Ser
210 215 220

Val Thr Leu Val His Lys Gly Asn Ile Met Lys Phe Thr Glu Gly Ala
225 230 235 240

Phe Lys Asp Trp Gly Tyr Gln Leu Ala Arg Glu Glu Phe Gly Gly Glu
245 250 255

Leu Ile Asp Gly Gly Pro Trp Leu Lys Val Lys Asn Pro Asn Thr Gly
260 265 270

Lys Glu Ile Val Ile Lys Asp Val Ile Ala Asp Ala Phe Leu Gln Gln
275 280 285

Ile Leu Leu Arg Pro Ala Glu Tyr Asp Val Ile Ala Cys Met Asn Leu
290 295 300

Asn Gly Asp Tyr Ile Ser Asp Ala Leu Ala Ala Gln Val Gly Gly Ile
305 310 315 320

Gly Ile Ala Pro Gly Ala Asn Ile Gly Asp Glu Cys Ala Leu Phe Glu
325 330 335

Ala Thr His Gly Thr Ala Pro Lys Tyr Ala Gly Gln Asp Lys Val Asn
340 345 350

Pro Gly Ser Ile Ile Leu Ser Ala Glu Met Met Leu Arg His Met Gly
355 360 365

Trp Thr Glu Ala Ala Asp Leu Ile Val Lys Gly Met Glu Gly Ala Ile
370 375 380

Asn Ala Lys Thr Val Thr Tyr Asp Phe Glu Arg Leu Met Asp Gly Ala
385 390 395 400

Lys Leu Leu Lys Cys Ser Glu Phe Gly Asp Ala Ile Ile Glu Asn Met
405 410 415

<210> 313
<211> 1305
<212> DNA
<213> Escherichia coli

<400>  313
atgaaaaccc gtacacaaca aattgaagaa ttacagaaag agtggactca accgcgttgg        60

gaaggcatta ctcgcccata cagtgcggaa gatgtggtga aattacgcgg ttcagtcaat       120

cctgaatgca cgctggcgca actgggcgca gcgaaaatgt ggcgtctgct gcacggtgag       180

tcgaaaaaag gctacatcaa cagcctcggc gcactgactg gcggtcaggc gctgcaacag       240

gcgaaagcgg gtattgaagc agtctatctg tcgggatggc aggtagcggc ggacgctaac       300

ctggcggcca gcatgtatcc ggatcagtcg ctctatccgg caaactcggt gccagctgtg       360

gtggagcgga tcaacaacac cttccgtcgt gccgatcaga tccaatggtc cgcgggcatt       420

gagccgggcg atccgcgcta tgtcgattac ttcctgccga tcgttgccga tgcggaagcc       480

ggttttggcg gtgtcctgaa tgcctttgaa ctgatgaaag cgatgattga agccggtgca       540

gcggcagttc acttcgaaga tcagctggcg tcagtgaaga aatgcggtca catgggcggc       600

aaagttttag tgccaactca ggaagctatt cagaaactgg tcgcggcgcg tctggcagct       660

gacgtgacgg gcgttccaac cctgctggtt gcccgtaccg atgctgatgc ggcggatctg       720

atcacctccg attgcgaccc gtatgacagc gaatttatta ccggcgagcg taccagtgaa       780

ggcttcttcc gtactcatgc gggcattgag caagcgatca gccgtggcct ggcgtatgcg       840

ccatatgctg acctggtctg gtgtgaaacc tccacgccgg atctggaact ggcgcgtcgc       900

tttgcacaag ctatccacgc gaaatatccg ggcaaactgc tggcttataa ctgctcgccg       960

tcgttcaact ggcagaaaaa cctcgacgac aaaactattg ccagcttcca gcagcagctg      1020

tcggatatgg gctacaagtt ccagttcatc accctggcag gtatccacag catgtggttc      1080

aacatgtttg acctggcaaa cgcctatgcc cagggcgagg gtatgaagca ctacgttgag      1140

aaagtgcagc agccggaatt tgccgccgcg aaagatggct ataccttcgt atctcaccag      1200

caggaagtgg gtacaggtta cttcgataaa gtgacgacta ttattcaggg cggcacgtct      1260

tcagtcaccg cgctgaccgg ctccactgaa gaatcgcagt tctaa                      1305

<210>  314
<211>  434
<212>  PRT
<213>  Escherichia coli

<400>  314

Met Lys Thr Arg Thr Gln Gln Ile Glu Glu Leu Gln Lys Glu Trp Thr
1               5                   10                  15

Gln Pro Arg Trp Glu Gly Ile Thr Arg Pro Tyr Ser Ala Glu Asp Val
            20                  25                  30

Val Lys Leu Arg Gly Ser Val Asn Pro Glu Cys Thr Leu Ala Gln Leu

35 40 45

Gly Ala Ala Lys Met Trp Arg Leu Leu His Gly Glu Ser Lys Lys Gly
50 55 60

Tyr Ile Asn Ser Leu Gly Ala Leu Thr Gly Gly Gln Ala Leu Gln Gln
65 70 75 80

Ala Lys Ala Gly Ile Glu Ala Val Tyr Leu Ser Gly Trp Gln Val Ala
85 90 95

Ala Asp Ala Asn Leu Ala Ala Ser Met Tyr Pro Asp Gln Ser Leu Tyr
100 105 110

Pro Ala Asn Ser Val Pro Ala Val Val Glu Arg Ile Asn Asn Thr Phe
115 120 125

Arg Arg Ala Asp Gln Ile Gln Trp Ser Ala Gly Ile Glu Pro Gly Asp
130 135 140

Pro Arg Tyr Val Asp Tyr Phe Leu Pro Ile Val Ala Asp Ala Glu Ala
145 150 155 160

Gly Phe Gly Gly Val Leu Asn Ala Phe Glu Leu Met Lys Ala Met Ile
165 170 175

Glu Ala Gly Ala Ala Ala Val His Phe Glu Asp Gln Leu Ala Ser Val
180 185 190

Lys Lys Cys Gly His Met Gly Gly Lys Val Leu Val Pro Thr Gln Glu
195 200 205

Ala Ile Gln Lys Leu Val Ala Ala Arg Leu Ala Ala Asp Val Thr Gly
210 215 220

Val Pro Thr Leu Leu Val Ala Arg Thr Asp Ala Asp Ala Ala Asp Leu
225 230 235 240

Ile Thr Ser Asp Cys Asp Pro Tyr Asp Ser Glu Phe Ile Thr Gly Glu
245 250 255

Arg Thr Ser Glu Gly Phe Phe Arg Thr His Ala Gly Ile Glu Gln Ala
260 265 270

Ile Ser Arg Gly Leu Ala Tyr Ala Pro Tyr Ala Asp Leu Val Trp Cys
275 280 285

Glu Thr Ser Thr Pro Asp Leu Glu Leu Ala Arg Arg Phe Ala Gln Ala
290 295 300

Ile His Ala Lys Tyr Pro Gly Lys Leu Leu Ala Tyr Asn Cys Ser Pro
305 310 315 320

Ser Phe Asn Trp Gln Lys Asn Leu Asp Asp Lys Thr Ile Ala Ser Phe
325 330 335

Gln Gln Gln Leu Ser Asp Met Gly Tyr Lys Phe Gln Phe Ile Thr Leu
340 345 350

Ala Gly Ile His Ser Met Trp Phe Asn Met Phe Asp Leu Ala Asn Ala
355 360 365

Tyr Ala Gln Gly Glu Gly Met Lys His Tyr Val Glu Lys Val Gln Gln
370 375 380

Pro Glu Phe Ala Ala Ala Lys Asp Gly Tyr Thr Phe Val Ser His Gln
385 390 395 400

Gln Glu Val Gly Thr Gly Tyr Phe Asp Lys Val Thr Thr Ile Ile Gln
405 410 415

Gly Gly Thr Ser Ser Val Thr Ala Leu Thr Gly Ser Thr Glu Glu Ser
420 425 430

Gln Phe

<210> 315
<211> 1602
<212> DNA
<213> Escherichia coli

<400> 315
atgactgaac aggcaacaac aaccgatgaa ctggctttca caaggccgta tggcgagcag 60

gagaagcaaa ttcttactgc cgaagcggta gaatttctga ctgagctggt gacgcatttt 120

acgccacaac gcaataaact tctggcagcg cgcattcagc agcagcaaga tattgataac 180

ggaacgttgc ctgattttat ttcggaaaca gcttccattc gcgatgctga ttggaaaatt 240

cgcgggattc ctgcggactt agaagaccgc cgcgtagaga taactggccc ggtagagcgc 300

aagatggtga tcaacgcgct caacgccaat gtgaaagtct ttatggccga tttcgaagat 360

tcactggcac cagactggaa caaagtgatc gacgggcaaa ttaacctgcg tgatgcggtt 420

aacggcacca tcagttacac caatgaagca ggcaaaattt accagctcaa gcccaatcca 480

gcggttttga tttgtcgggt acgcggtctg cacttgccgg aaaaacatgt cacctggcgt 540

ggtgaggcaa tccccggcag cctgtttgat tttgcgctct atttcttcca caactatcag 600

gcactgttgg caaagggcag tggtccctat ttctatctgc cgaaaaccca gtcctggcag 660

gaagcggcct ggtggagcga agtcttcagc tatgcagaag atcgctttaa tctgccgcgc 720

ggcaccatca aggcgacgtt gctgattgaa acgctgcccg ccgtgttcca gatggatgaa 780

atccttcacg cgctgcgtga ccatattgtt ggtctgaact gcggtcgttg ggattacatc 840

ttcagctata tcaaaacgtt gaaaaactat cccgatcgcg tcctgccaga cagacaggca 900

gtgacgatgg ataaaccatt cctgaatgct tactcacgcc tgttgattaa aacctgccat 960

aaacgcggtg cttttgcgat gggcggcatg gcggcgttta ttccgagcaa agatgaagag 1020

cacaataacc aggtgctcaa caaagtaaaa gcggataaat cgctggaagc caataacggt 1080

cacgatggca catggatcgc tcacccaggc cttgcggaca cggcaatggc ggtattcaac 1140

gacattctcg gctcccgtaa aaatcagctt gaagtgatgc gcgaacaaga cgcgccgatt 1200

actgccgatc agctgctggc accttgtgat ggtgaacgca ccgaagaagg tatgcgcgcc 1260

aacattcgcg tggctgtgca gtacatcgaa gcgtggatct ctggcaacgg ctgtgtgccg 1320

atttatggcc tgatggaaga tgcggcgacg gctgaaattt cccgtacctc gatctggcag 1380

tggatccatc atcaaaaaac gttgagcaat ggcaaaccgg tgaccaaagc cttgttccgc 1440

cagatgctgg gcgaagagat gaaagtcatt gccagcgaac tgggcgaaga acgtttctcc 1500

caggggcgtt ttgacgatgc cgcacgcttg atggaacaga tcaccacttc cgatgagtta 1560

attgatttcc tgaccctgcc aggctaccgc ctgttagcgt aa 1602

<210> 316
<211> 533
<212> PRT
<213> Escherichia coli

<400> 316

Met Thr Glu Gln Ala Thr Thr Thr Asp Glu Leu Ala Phe Thr Arg Pro
1 5 10 15

Tyr Gly Glu Gln Glu Lys Gln Ile Leu Thr Ala Glu Ala Val Glu Phe
20 25 30

Leu Thr Glu Leu Val Thr His Phe Thr Pro Gln Arg Asn Lys Leu Leu
35 40 45

Ala Ala Arg Ile Gln Gln Gln Gln Asp Ile Asp Asn Gly Thr Leu Pro
50 55 60

Asp Phe Ile Ser Glu Thr Ala Ser Ile Arg Asp Ala Asp Trp Lys Ile
65 70 75 80

```
Arg Gly Ile Pro Ala Asp Leu Glu Asp Arg Arg Val Glu Ile Thr Gly
                85                  90                  95

Pro Val Glu Arg Lys Met Val Ile Asn Ala Leu Asn Ala Asn Val Lys
            100                 105                 110

Val Phe Met Ala Asp Phe Glu Asp Ser Leu Ala Pro Asp Trp Asn Lys
        115                 120                 125

Val Ile Asp Gly Gln Ile Asn Leu Arg Asp Ala Val Asn Gly Thr Ile
    130                 135                 140

Ser Tyr Thr Asn Glu Ala Gly Lys Ile Tyr Gln Leu Lys Pro Asn Pro
145                 150                 155                 160

Ala Val Leu Ile Cys Arg Val Arg Gly Leu His Leu Pro Glu Lys His
                165                 170                 175

Val Thr Trp Arg Gly Glu Ala Ile Pro Gly Ser Leu Phe Asp Phe Ala
            180                 185                 190

Leu Tyr Phe Phe His Asn Tyr Gln Ala Leu Leu Ala Lys Gly Ser Gly
        195                 200                 205

Pro Tyr Phe Tyr Leu Pro Lys Thr Gln Ser Trp Gln Glu Ala Ala Trp
    210                 215                 220

Trp Ser Glu Val Phe Ser Tyr Ala Glu Asp Arg Phe Asn Leu Pro Arg
225                 230                 235                 240

Gly Thr Ile Lys Ala Thr Leu Leu Ile Glu Thr Leu Pro Ala Val Phe
                245                 250                 255

Gln Met Asp Glu Ile Leu His Ala Leu Arg Asp His Ile Val Gly Leu
            260                 265                 270

Asn Cys Gly Arg Trp Asp Tyr Ile Phe Ser Tyr Ile Lys Thr Leu Lys
        275                 280                 285

Asn Tyr Pro Asp Arg Val Leu Pro Asp Arg Gln Ala Val Thr Met Asp
    290                 295                 300

Lys Pro Phe Leu Asn Ala Tyr Ser Arg Leu Leu Ile Lys Thr Cys His
305                 310                 315                 320

Lys Arg Gly Ala Phe Ala Met Gly Gly Met Ala Ala Phe Ile Pro Ser
```

325 330 335

Lys Asp Glu Glu His Asn Asn Gln Val Leu Asn Lys Val Lys Ala Asp
340 345 350

Lys Ser Leu Glu Ala Asn Asn Gly His Asp Gly Thr Trp Ile Ala His
355 360 365

Pro Gly Leu Ala Asp Thr Ala Met Ala Val Phe Asn Asp Ile Leu Gly
370 375 380

Ser Arg Lys Asn Gln Leu Glu Val Met Arg Glu Gln Asp Ala Pro Ile
385 390 395 400

Thr Ala Asp Gln Leu Leu Ala Pro Cys Asp Gly Glu Arg Thr Glu Glu
405 410 415

Gly Met Arg Ala Asn Ile Arg Val Ala Val Gln Tyr Ile Glu Ala Trp
420 425 430

Ile Ser Gly Asn Gly Cys Val Pro Ile Tyr Gly Leu Met Glu Asp Ala
435 440 445

Ala Thr Ala Glu Ile Ser Arg Thr Ser Ile Trp Gln Trp Ile His His
450 455 460

Gln Lys Thr Leu Ser Asn Gly Lys Pro Val Thr Lys Ala Leu Phe Arg
465 470 475 480

Gln Met Leu Gly Glu Glu Met Lys Val Ile Ala Ser Glu Leu Gly Glu
485 490 495

Glu Arg Phe Ser Gln Gly Arg Phe Asp Asp Ala Ala Arg Leu Met Glu
500 505 510

Gln Ile Thr Thr Ser Asp Glu Leu Ile Asp Phe Leu Thr Leu Pro Gly
515 520 525

Tyr Arg Leu Leu Ala
530

<210> 317
<211> 1161
<212> DNA
<213> Escherichia coli

<400> 317
atgacaaata accctctgat tccacaaagc aaacttccac aacttggcac cactattttc 60

```
acccagatga gcgcgctggc gcagcaacac caggcgatta acctgtcgca aggctttcct      120
gattttgatg gtccgcgcta tttacaggag cggctggcgc accacgttgc acagggggca      180
aaccaatacg cgcccatgac cggcgtgcag gccttgcgcg aggcgattgc tcagaaaacg      240
gaacgtttgt atggctatca accagatgcc gatagcgata tcaccgtaac ggcaggggcg      300
acggaagcgt tatacgcggc gattaccgca ctggtgcgca atggcgatga agtgatttgt      360
tttgatccca gctatgacag ttacgccccc gccatcgcgc tttctggggg aatagtgaag      420
cgtatggcac tgcaaccacc gcattttcgc gttgactggc aggaatttgc cgcattatta      480
agcgagcgca ccagactggt gatcctcaac actccgcata accccagtgc aactgtctgg      540
cagcaggctg atttcgccgc tttgtggcag gcgatcgccg ggcacgagat tttgtcatt       600
agcgatgaag tctacgagca catcaacttt tcacaacagg gccatgccag tgtgctggcg      660
catccgcagc tgcgtgagcg ggcagtggcg gtttcttcat ttggcaagac ctatcatatg      720
accggctgga aagtgggtta ttgtgttgcg ccagcgccca tcagcgccga aattcgcaag      780
gtacatcagt atctgacctt ttcggtgaat accccggcac agctggcgct tgctgatatg      840
ctacgtgcag aacctgagca ttatcttgcg ttaccggact tttatcgcca gaagcgcgat      900
attctggtga atgctttaaa tgaaagccgg ctggagattt taccgtgtga aggtacatac      960
tttttgctgg tggattacag cgcggtttct accctggatg atgttgagtt ttgccagtgg     1020
ctgacgcagg agcacggcgt agcggcgatt ccgctgtcgg tgttttgcgc cgatcccttc     1080
ccacataaac tgattcgtct ctgttttgcc aagaaggaat cgacgttgct ggcagcagct     1140
gaacgcctgc gccagcttta g                                               1161
```